(19) Europäisches Patentamt
European Patent Office
Office européen des brevets

(11) EP 2 103 620 A1

(12) **EUROPEAN PATENT APPLICATION**
published in accordance with Art. 153(4) EPC

(43) Date of publication:
**23.09.2009 Bulletin 2009/39**

(21) Application number: **07859772.1**

(22) Date of filing: **11.12.2007**

(51) Int Cl.:
*C07D 519/00* (2006.01) *A61K 31/519* (2006.01)
*A61P 35/00* (2006.01) *A61P 43/00* (2006.01)

(86) International application number:
**PCT/JP2007/073879**

(87) International publication number:
**WO 2008/072634 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.12.2006 JP 2006335158**

(71) Applicant: **Takeda Pharmaceutical Company Limited**
**Osaka-shi,**
**Osaka 541-0045 (JP)**

(72) Inventors:
- **ISHIKAWA, Tomoyasu**
  **Tsukuba-shi**
  **Ibaraki 300-4293 (JP)**
- **BANNO, Hiroshi**
  **Tsukuba-shi**
  **Ibaraki 300-4293 (JP)**
- **KAWAKITA, Youichi**
  **Tsukuba-shi**
  **Ibaraki 300-4293 (JP)**
- **OHASHI, Tomohiro**
  **Tsukuba-shi**
  **Ibaraki 300-4293 (JP)**
- **KURASAWA, Osamu**
  **Tsukuba-shi**
  **Ibaraki 300-4293 (JP)**

(74) Representative: **Jones, Nicholas Andrew**
**Withers & Rogers LLP**
**Goldings House**
**2 Hays Lane**
**London**
**SE1 2HW (GB)**

# (54) FUSED HETEROCYCLIC COMPOUND

(57) The present invention provides a fused heterocyclic compound having a tyrosine kinase inhibitory action, which is represented by the formula:

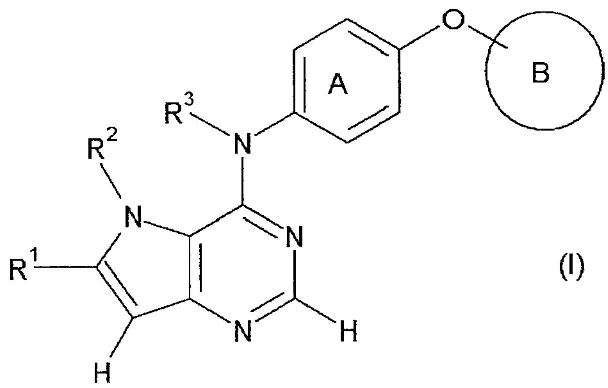

(I)

wherein
$R^1$ is a hydrogen atom, a halogen atom, or an optionally substituted group bonded via a carbon atom, a nitrogen atom or an oxygen atom;

EP 2 103 620 A1

$R^2$ is a hydrogen atom, or an optionally substituted group bonded via a carbon atom or a sulfur atom, or
$R^1$ and $R^2$, or $R^2$ and $R^3$ are optionally bonded to each other to form an optionally substituted ring structure;
$R^3$ is a hydrogen atom or an optionally substituted aliphatic hydrocarbon group, or
$R^3$ is optionally bonded to the carbon atom on ring A to form an optionally substituted ring structure;
ring A is an optionally substituted benzene ring; and ring B is
(i) an optionally substituted fused ring, or
(ii) a pyridine ring having optionally substituted carbamoyl (the pyridine ring is optionally further substituted).

## Description

### Technical Field

The present invention relates to a fused pyrimidine compound having a growth factor receptor tyrosine kinase inhibitory activity, which is useful for the prophylaxis or treatment of cancer, a production method thereof and use thereof.

### Background of the Invention

The gene of cell growth factor and growth factor receptor is called a protooncogene and plays a key role in the pathology of human tumor. The epithelial cell growth factor receptor family (erbB) includes EGFR, HER2, HER3 and HER4, which are type I receptor type tyrosine kinases. These erbB family express in various cell groups, and are deeply involved in the control of the growth and differentiation of cells and the control of suppression of cell death (apoptosis suppression). For example, high expression of EGFR and HER2, and homeostatic activation of receptors are empirically known to transform cells.

It is also known that high expression and simultaneous expression of each of these receptors are poor prognostic factors in various cancer patients.

These receptors are bound with many peptide ligands such as EGF, TGFα and the like, and binding of the ligand promotes homo- or heterodimerization of the receptors. This induces increase of kinase activity from self-phosphorylation or transphosphorylation of the receptors, and causes activation of downstream signaling pathway (MAPK, Akt) via a protein bound with a particular phosphorylated tyrosine residue. This is the mechanism of the receptor activity of the above-mentioned cell growth, differentiation, cell death suppression and the like, which is considered to be responsible for the high expression of receptor in cancer and malignant degeneration of cancer due to topical increase in the ligand concentration.

Many cancers are associated with the high expression of EGFR or HER2. For example, breast cancer (20-30%), ovarian cancer (20-40%), non-small cell lung cancer (30-60%), colorectal cancer (40-80%), prostate cancer (10-60%), urinary bladder cancer (30-60%), kidney cancer (20-40%) and the like can be mentioned. Moreover, receptor expression and prognosis are correlated, and receptor expression is a poor prognostic factor in breast cancer, non-small cell lung cancer and the like.

In recent years, clinical use of a humanized anti-HER2 antibody (Trastuzumab) against HER2 highly expressing breast cancer, clinical trial of anti-EGFR antibody and clinical trials of several low molecular weight receptor enzyme inhibitors have demonstrated a potential of these drugs against HER2 or EGFR for therapeutic drugs for cancer. While these drugs show a tumor growth inhibitory action in clinical and non-clinical trials, they are known to induce inhibition of receptor enzyme activity and suppression of downstream signaling pathway. Therefore, a compound inhibiting EGFR or HER2 kinase, or inhibiting activation of EGFR or HER2 kinase is effective as a therapeutic drug for cancer.

As a compound that inhibits receptor type tyrosine kinases represented by HER2/EGFR kinase, fused heterocyclic compounds (e.g., patent reference 1 (WO97/13771), patent reference 2 (WO98/02437), and patent reference 3 (WO00/44728)), quinazoline derivatives (e.g., patent reference 4 (WO02/02552), patent reference 5 (WO01/98277), patent reference 6 (WO03/049740) and patent reference 7 (WO03/050108)), thienopyrimidine derivatives (e.g., patent reference (WO03/053446)), aromatic azole derivatives (e.g., patent reference 9 (WO98/03648), patent reference 10 (WO01/77107) and patent reference 11 (WO03/031442)), condensed pyrimidine derivatives (e.g., patent reference 12 (WO2005/118588)) and the like are known.

As to pyrrolo[3,2-d]pyrimidine derivatives, the following compounds are known as compounds having a cell growth inhibitory activity (non-patent reference 1 (Khim.-Farm. Zh., 1982, 16, 1338-1343); non-patent reference 2 (Collect. Czech. Chem. Commun., 2003, 68, 779-791)).

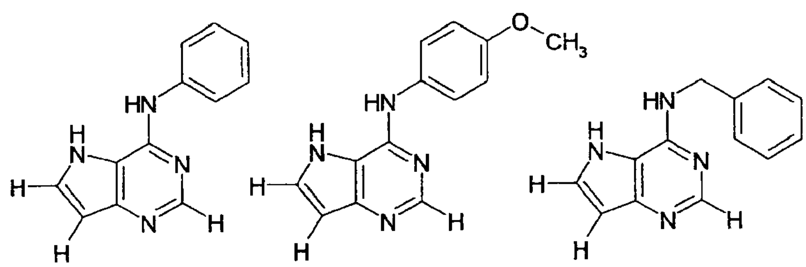

As a compound having a receptor type tyrosine kinase inhibitory activity, the following pyrrolo[3,2-d]pyrimidine derivative is known (patent reference 13 (WO96/40142) and patent reference (WO98/23613)).
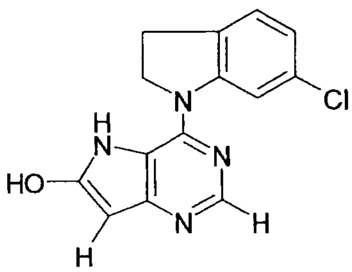

Furthermore, as to pyrazolo[4,3-d]pyrimidine derivatives, 3,5,7-trisubstituted pyrazolo[4,3-d]pyrimidine derivatives are known as compounds having a CDK inhibitory action, a cell growth inhibitory action and/or an apoptosis inducing action (e.g., patent reference 15 (EP-A-1348707)), and 3-isopropylpyrazolo[4,3-d]pyrimidine derivatives are known as compounds having a CDK1/cyclin B inhibitory activity (e.g., non-patent reference 3 (Bioorganic & Medicinal Chemistry Letters, 2003, 13, 2989-2992)). Furthermore, synthesis of 3-methylpyrazolo[4,3-d]pyrimidine derivatives has been reported (see non-patent reference 4 (The Journal of Organic Chemistry, 1956, 21, 833-836)).
[patent reference 1] WO97/13771
[patent reference 2] WO98/02437
[patent reference 3] WO00/44728
[patent reference 4] WO02/02552
[patent reference 5] WO01/98277
[patent reference 6] WO03/049740
[patent reference 7] WO03/050108
[patent reference 8] WO03/053446
[patent reference 9] WO98/03648
[patent reference 10] WO01/77107
[patent reference 11] WO03/031442
[patent reference 12] WO2005/118588
[patent reference 13] WO96/40142
[patent reference 14] WO98/23613
[patent reference 15] EP A 1348707
[non-patent reference 1] Khim. -Farm.Zh., 1982, 16, 1338-1343
[non-patent reference 2] Collect. Czech. Chem. Commun., 2003, 68, 779-791
[non-patent reference 3] Bioorganic & Medicinal Chemistry Letters, 2003, 13, 2989-2992
[non-patent reference 4] The Journal of Organic Chemistry, 1956, 21, 833-836

## Disclosure of the Invention

## Problems to be Solved by the Invention

The present invention aims at providing a compound having a superior tyrosine kinase inhibitory action, which is highly safe as a pharmaceutical product.

## Means of Solving the Problems

The present inventors have conducted intensive studies in an attempt to solve the aforementioned problems and found that the compounds represented by the following formula (I) and salts thereof have a superior tyrosine kinase inhibitory action. Further studies have resulted in the completion of the present invention.
Accordingly, the present invention relates to the following.

[1] A compound represented by the formula:

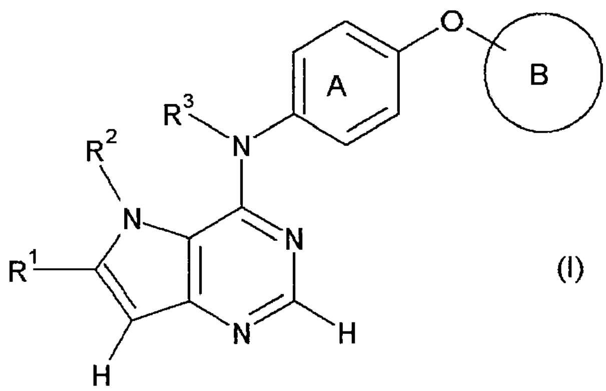

(I)

wherein

$R^1$ is a hydrogen atom, a halogen atom, or an optionally substituted group bonded via a carbon atom, a nitrogen atom or an oxygen atom;

$R^2$ is a hydrogen atom, or an optionally substituted group bonded via a carbon atom or a sulfur atom, or

$R^1$ and $R^2$, or $R^2$ and $R^3$ are optionally bonded to each other to form an optionally substituted ring structure;

$R^3$ is a hydrogen atom or an optionally substituted aliphatic hydrocarbon group, or

$R^3$ is optionally bonded to the carbon atom on ring A to form an optionally substituted ring structure;

ring A is an optionally substituted benzene ring; and

ring B is

(i) an optionally substituted fused ring, or
(ii) a pyridine ring having optionally substituted carbamoyl (the pyridine ring is optionally further substituted)

(hereinafter sometimes to be abbreviated as compound (I)), or a salt thereof,

[2] the compound of the above-mentioned [1], wherein $R^1$ is a hydrogen atom, a halogen atom or cyano,

[3] the compound of the above-mentioned [1], wherein $R^2$ is a hydrogen atom or optionally substituted alkyl,
[4] the compound of the above-mentioned [1], wherein $R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-(C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,
(f) $-NR^5-CO-$(a 5- or 6-membered heterocycle optionally substituted by 1 or 2 $C_{1-4}$ alkyl),
(g) $-NR^5-CO-(CH_2)_n-$(a 6-membered heterocycle optionally substituted by $C_{1-4}$ alkyl),
(h) $-NR^5-CO-(C_{3-6}$ cycloalkyl optionally substituted by substituent(s) selected from hydroxy and cyano),
(i) $-NR^5-CO-(C_{6-10}$ aryl optionally substituted by $C_{1-4}$ alkyl optionally substituted by 1 to 3 halogen atoms),
(j) $-NR^5-CO-NR^{5'}-C_{3-6}$ cycloalkyl,
(k) $-NR^5-SO_2-C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo,

wherein n is an integer of 1 to 4, $R^5$ and $R^{5'}$ are each a hydrogen atom or $C_{1-4}$ alkyl, and $-(CH_2)_n-$ is optionally substituted by $C_{1-4}$ alkyl,
[5] the compound of the above-mentioned [1], wherein $R^3$ is a hydrogen atom,
[6] the compound of the above-mentioned [1], wherein ring A is a benzene ring optionally substituted by 1 or 2 substituents selected from the group consisting of (1) a halogen atom and (2) $C_{1-4}$ alkyl,
[7] the compound of the above-mentioned [1], wherein ring B is

(1) a fused ring optionally substituted by substituent(s) selected from the group consisting of

(a) a halogen atom,
(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and
(i) $C_{1-6}$ alkoxy-carbonyl, or

(2) a pyridine ring having carbamoyl optionally substituted by $C_{1-6}$ alkyl (the pyridine ring is optionally further substituted by $C_{1-6}$ alkyl),

[8] the compound of the above-mentioned [1], wherein
$R^1$ is a hydrogen atom, a halogen atom or cyano;
$R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-(C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,
(f) $-NR^5-CO-$(a 5- or 6-membered heterocycle optionally substituted by 1 or 2 $C_{1-4}$ alkyl),
(g) $-NR^5-CO-(CH_2)_n-$(a 6-membered heterocycle optionally substituted by $C_{1-4}$ alkyl),
(h) $-NR^5-CO-(C_{3-6}$ cycloalkyl optionally substituted by substituent(s) selected from hydroxy and cyano),
(i) $-NR^5-CO-(C_{6-10}$ aryl optionally substituted by $C_{1-4}$ alkyl optionally substituted by 1 to 3 halogen atoms),
(j) $-NR^5-CO-NR^{5'}-C_{3-6}$ cycloalkyl,
(k) $-NR^5-SO_2-C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo,

wherein n is an integer of 1 to 4, $R^5$ and $R^{5'}$ are each a hydrogen atom or $C_{1-4}$ alkyl, and $-(CH_2)_n-$ is optionally substituted by $C_{1-4}$ alkyl;
$R^3$ is a hydrogen atom; ring A is a benzene ring optionally substituted by 1 or 2 substituents selected from the group consisting of (1) a halogen atom and (2) $C_{1-4}$ alkyl; and
ring B is

(1) a fused ring optionally substituted by substituent(s) selected from the group consisting of

(a) a halogen atom,

(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and
(i) $C_{1-6}$ alkoxy-carbonyl, or

(2) a pyridine ring having carbamoyl optionally substituted by $C_{1-6}$ alkyl (the pyridine ring is optionally further substituted by $C_{1-6}$ alkyl) ;

[9] N-{2-[4-({3-chloro-4-[(2-oxo-2,3-dihydro-1H-indol-4-yl)oxy]phenyl}amino)-5H-pyrrolo[3,2-d]pyrimidin-5-yl]ethyl}-2-(methylsulfonyl)acetamide;
4-(2-chloro-4-{[5-(2-hydroxyethyl)-5H-pyrrolo[3,2-d]pyrimidin-4-yl]amino}phenoxy)-1-methyl-1,3-dihydro-2H-indol-2-one;
2-(4-{[3-chloro-4-(1H-indol-4-yloxy)phenyl]amino}-5H-pyrrolo[3,2-d]pyrimidin-5-yl)ethanol;
N-[3-chloro-4-(1H-indol-4-yloxy)phenyl]-5-methyl-5H-pyrrolo[3,2-d]pyrimidin-4-amine;
4-(2-chloro-4-{[5-(2-hydroxyethyl)-5H-pyrrolo[3,2-d]pyrimidin-4-yl]amino}phenoxy)-1,3-dihydro-2H-indol-2-one;
N-[2-(4-{[4-(1-benzothiophen-6-yloxy)-3-chlorophenyl]amino}-5H-pyrrolo[3,2-d]pyrimidin-5-yl)ethyl]-3-hydroxy-2,2-dimethylpropanamide;
N-[2-(4-{[4-(1-benzothiophen-6-yloxy)-3-chlorophenyl]amino}-5H-pyrrolo[3,2-d]pyrimidin-5-yl)ethyl]-2-(methylsulfonyl)acetamide;
2-(4-{[4-(1-benzothiophen-4-yloxy)-3-chlorophenyl]amino}-5H-pyrrolo[3,2-d]pyrimidin-5-yl)ethanol;
N-[3-chloro-4-(1H-indazol-4-yloxy)phenyl]-5-methyl-5H-pyrrolo[3,2-d]pyrimidin-4-amine;
2-(4-{[3-chloro-4-(1H-indazol-4-yloxy)phenyl]amino}-5H-pyrrolo[3,2-d]pyrimidin-5-yl)ethanol;
N-[2-(4-{[4-(1-benzothiophen-4-yloxy)-3-chlorophenyl]amino}-5H-pyrrolo[3,2-d]pyrimidin-5-yl)ethyl]acetamide;
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide;
N-[2-(4-{[4-(1-benzothiophen-4-yloxy)-3-chlorophenyl]amino}-5H-pyrrolo[3,2-d]pyrimidin-5-yl)ethyl]acetamide;
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide;
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-3-methylbutanamide;
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-2,2-dimethylpropanamide;
N-[2-(4-{[3-chloro-4-(imidazo[1,2-a]pyridin-7-yloxy)phenyl]amino}-5H-pyrrolo[3,2-d]pyrimidin-5-yl)ethyl]-3-hydroxy-2,2-dimethylpropanamide;
N-[2-(4-{[3-chloro-4-([1,2,4]triazolo[1,5-a]pyridin-7-yloxy)phenyl]amino}-5H-pyrrolo[3,2-d]pyrimidin-5-yl)ethyl]-3-hydroxy-2,2-dimethylpropanamide;
4-(2-chloro-4-{[6-chloro-5-(2-hydroxyethyl)-5H-pyrrolo[3,2-d]pyrimidin-4-yl]amino}phenoxy)-1-methyl-1,3-dihydro-2H-indol-2-one; or
N-[2-(4-{[4-(1-benzothiophen-4-yloxy)-3-chlorophenyl]amino}-5H-pyrrolo[3,2-d]pyrimidin-5-yl)ethyl]-2-methylalaninamide or a salt thereof,

[10] a prodrug of the compound of the above-mentioned [1],

[11] a pharmaceutical agent comprising the compound of the above-mentioned [1] or a salt thereof, or a prodrug thereof,
[12] the pharmaceutical agent of the above-mentioned [11], which is a tyrosine kinase inhibitor,
[13] the pharmaceutical agent of the above-mentioned [11], which is an agent for the prophylaxis or treatment of cancer,
[14] the pharmaceutical agent of the above-mentioned [11], which is an agent for the prophylaxis or treatment of breast cancer, ovarian cancer, colorectal cancer, small intestinal cancer, gastric cancer, esophagus cancer, prostate cancer, lung cancer, pancreatic cancer or kidney cancer,
[15] a method for the prophylaxis or treatment of cancer in a mammal, which comprises administering an effective amount of the compound of the above-mentioned [1] or a salt thereof, or a prodrug thereof, to the mammal,
[16] use of the compound of the above-mentioned [1] or a salt thereof, or a prodrug thereof, for the production of

an agent for the prophylaxis or treatment of cancer, and the like.

**Effects of the Invention**

According to the present invention, a fused pyrimidine compound having a superior tyrosine kinase inhibitory action, which is low toxic and sufficiently satisfactory as a pharmaceutical product, a production method thereof and use thereof are provided.

**Detailed explanation of the Invention**

$R^1$ is a hydrogen atom, a halogen atom, or an optionally substituted group bonded via a carbon atom, a nitrogen atom or an oxygen atom.
Examples of the "halogen atom" for $R^1$ include a fluorine atom, a chlorine atom, a bromine atom and an iodine atom.
Of the "optionally substituted group bonded via a carbon atom, a nitrogen atom or an oxygen atom" for $R^1$, examples of the "optionally substituted group bonded via a carbon atom" include cyano, optionally substituted $C_{1-8}$ alkyl, optionally substituted $C_{2-8}$ alkenyl, optionally substituted $C_{2-8}$ alkynyl, optionally substituted carbamoyl, optionally substituted $C_{1-8}$ alkyl-carbonyl, optionally substituted $C_{3-8}$ cycloalkyl, optionally substituted $C_{6-18}$ aryl, optionally substituted $C_{6-18}$ aryl-$C_{1-4}$ alkyl, optionally substituted $C_{6-18}$ aryl-carbonyl, optionally substituted $C_{6-18}$ aryl-$C_{1-4}$ alkyl-carbonyl, an optionally substituted heterocyclic group, optionally substituted heterocyclyl-$C_{1-4}$ alkyl, optionally substituted heterocyclyl-carbonyl and optionally substituted heterocyclyl-$C_{1-4}$ alkyl-carbonyl.
Examples of the "$C_{1-8}$ alkyl" of the above-mentioned "optionally substituted $C_{1-8}$ alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl and the like.
The "$C_{1-8}$ alkyl" of the above-mentioned "optionally substituted $C_{1-8}$ alkyl" may have one or more (preferably 1 to 5, more preferably 1 to 3) substituents at the substitutable positions. Such substituent is selected from the group consisting of

(a) a halogen atom,
(b) oxo,
(c) optionally-halogenated $C_{1-4}$ alkyl (e.g., methyl, ethyl, propyl, isopropyl, butyl, isobutyl, sec-butyl, tert-butyl),
(d) $C_{3-8}$ cycloalkyl (e.g., cyclopropyl, cyclobutyl, cyclopentyl, cyclohexyl, cycloheptyl, cyclooctyl etc.),
(e) $-(CH_2)_m$-Q group,
(f) $-(CH_2)_m-Z^1-$($C_{1-4}$ alkyl (e.g., methyl, ethyl, propyl, isopropyl, butyl, isobutyl, sec-butyl, tert-butyl) optionally substituted by substituent(s) selected from hydroxy, a halogen atom, cyano, $C_{1-4}$ alkoxy, amino and di-$C_{1-4}$ alkylamino),
(g) $-(CH_2)_m-Z^1-$($C_{3-8}$ cycloalkyl (e.g., cyclopropyl, cyclobutyl, cyclopentyl, cyclohexyl, cycloheptyl, cyclooctyl etc.) optionally substituted by substituent(s) selected from hydroxyl and cyano),
(h) $-(CH_2)_m-Z^1-$($C_{6-10}$ aryl (e.g., phenyl etc.) optionally substituted by $C_{1-4}$ alkyl optionally substituted by halogen atom(s)),
(i) $-(CH_2)_m-Z^2-(CH_2)_n$-Q group,
(j) $-(CH_2)_mZ^2-(CH_2)_n-Z^1-C_{1-4}$ alkyl (e.g., methyl, ethyl, propyl, isopropyl, butyl, isobutyl, sec-butyl, tert-butyl),
(k) $-(CH_2)_m-Z^2-(CH_2)_n-Z^1-C_{3-8}$ cycloalkyl (e.g., cyclopropyl, cyclobutyl, cyclopentyl, cyclohexyl, cycloheptyl, cyclooctyl etc.),
(l) $-(CH_2)_m-Z^1-$(a heterocyclic group optionally substituted by substituent(s) selected from $C_{1-4}$ alkyl, hydroxy and amino (preferably a 5- to 8-membered heterocyclic group having 1 to 3 heteroatoms selected from a nitrogen atom, an oxygen atom and an optionally oxidized sulfur atom)),
(m) $-(CH_2)_m-Z^2-(CH_2)_n-$(a heterocyclic group optionally substituted by $C_{1-4}$ alkyl (preferably a 5- to 8-membered heterocyclic group having 1 to 3 heteroatoms selected from a nitrogen atom, an oxygen atom and an optionally oxidized sulfur atom)),
(n) $-(CH_2)_m-Z^2-C_{1-4}$ alkoxy (e.g., methoxy, ethoxy, propoxy, isopropoxy, butoxy, isobutoxy, sec-butoxy, tert-butoxy),
(o) $-(CH_2)_m-Z^2-(CH_2)_n-Z^1-(CH_2)_n-Z^1-C_{1-4}$ alkyl (e.g., methyl, ethyl, propyl, isopropyl, butyl, isobutyl, sec-butyl, tert-butyl), and
(p) 3- to 6-membered cyclic amino optionally substituted by substituent(s) selected from $C_{1-4}$ alkyl and oxo (hereinafter sometimes to be referred to as substituent group X). When the number of the substituents is 2 or more, the respective substituents may be the same or different.

In the above-mentioned formulas,
m is an integer of 0 to 4;
n is an integer of 1 to 4;

Q is hydroxy, carboxy, cyano, nitro, $-NR^{11}R^{12}$, $-CONR^{11}R^{12}$ or - $SO_2NR^{11}R^{12}$;
$Z^1$ is -O-, -CO-, $-C(OH)R^{13}-$, $-C(=N-OR^{13})-$, -S-, -SO-, $-SO_2-$, - $N(COR^{13})-$, $-N(CO_2R^{14})-$, $-N(SO_2R^{14})-$, -CO-O-, -O-CO-, $-CO-NR^{13}-$, - $NR^{13}-CO-$, $-NR^{13}-CO_2.$, $-NR^{13}-CO-NH-$, $-NR^{13}-SO_2-$ or $-NR^{13}-C(=NH)-NH-$; and
$Z^2$ is -O-, -CO-, $-C(OH)R^{13}-$, $-C(=N-OR^{13})-$, -S-, -SO-, $-SO_2-$, - $NR^{13}-$, $-N(COR^{13})-$, $-N(CO_2R^{14})-$, $-N(SO_2R^{14})-$, -CO-O-, -O-CO-, $-CO-NR^{13}-$, $-NR^{13}-CO-$, $-NR^{13}-CO_2-$, $-NR^{13}-CO-NH-$, $-NR^{13}-C(=NH)-NH-$, - $NR^{13}-SO_2-$ or $-SO_2-NR^{13}-$.

In addition, $-(CH_2)_m-$ and $-(CH_2)_n-$ in the above-mentioned formulas are optionally substituted by, for example, one or more (preferably 1 to 5, more preferably 1 to 3) substituents selected from halogen, optionally halogenated $C_{1-4}$ alkyl and hydroxy. When m or n is not less than 2, a subset $-CH_2CH_2-$ of $-(CH_2)_m-$ and $-(CH_2)_n-$ may be replaced by -CH=CH- or -C≡C-.

In the above-mentioned formulas, $R^{11}$ and $R^{12}$ are the same or different and each is a hydrogen atom or $C_{1-4}$ alkyl (e.g., methyl, ethyl, propyl, isopropyl, butyl, isobutyl, sec-butyl, tert-butyl), or $R^{11}$ and $R^{12}$ may be bonded to form a ring together with the nitrogen atom.

In addition, in the above-mentioned formulas, $R^{13}$ is a hydrogen atom or $C_{1-4}$ alkyl (e.g., methyl, ethyl, propyl, isopropyl, butyl, isobutyl, sec-butyl, tert-butyl), and $R^{14}$ is $C_{1-4}$ alkyl (e.g., methyl, ethyl, propyl, isopropyl, butyl, isobutyl, sec-butyl, tert-butyl).

When $R^{11}$ and $R^{12}$ are bonded to form a ring together with the nitrogen atom, Examples of the nitrogen-containing heterocyclic group include a 3- to 8-membered (preferably 5- or 6-membered) saturated or unsaturated (preferably saturated) aliphatic heterocycle such as azetidine, pyrrolidine, piperidine, homopiperidine, heptamethylenimine, morpholine, thiomorpholine, piperazine, homopiperazine and the like.

Examples of the "$C_{2-8}$ alkenyl" of the above-mentioned "optionally substituted $C_{2-8}$ alkenyl" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

The "$C_{2-8}$ alkenyl" of the above-mentioned "optionally substituted $C_{2-8}$ alkenyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

Examples of the "$C_{2-8}$ alkynyl" of the above-mentioned "optionally substituted $C_{2-8}$ alkynyl" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

The "$C_{2-8}$ alkynyl" of the above-mentioned "optionally substituted $C_{2-8}$ alkynyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

The "carbamoyl" of the above-mentioned "optionally substituted carbamoyl" may have 1 or 2 substituents at the substitutable positions. Examples of such substituent include substituents selected from substituent group X. When the number of the substituents is not less than 2, the respective substituents may be the same or different.

Examples of the "$C_{1-8}$ alkyl-carbonyl" of the above-mentioned "optionally substituted $C_{1-8}$ alkyl-carbonyl" include acetyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, neopentylcarbonyl, 1-ethylpropylcarbonyl, hexylcarbonyl, isohexylcarbonyl, 1,1-dimethylbutylcarbonyl, 2,2-dimethylbutylcarbonyl, 3,3-dimethylbutylcarbonyl, 2-ethylbutylcarbonyl, heptylcarbonyl, octylcarbonyl and the like.

The "$C_{1-8}$ alkyl-carbonyl" of the above-mentioned "optionally substituted $C_{1-8}$ alkyl-carbonyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

Examples of the "$C_{3-8}$ cycloalkyl" of the above-mentioned "optionally substituted $C_{3-8}$ cycloalkyl" include cyclopropyl, cyclobutyl, cyclopentyl, cyclohexyl, cycloheptyl, cyclooctyl and the like.

The "$C_{3-8}$ cycloalkyl" of the above-mentioned "optionally substituted $C_{3-8}$ cycloalkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl" of the above-mentioned "optionally substituted $C_{6-18}$ aryl" include phenyl, naphthyl, anthryl, phenanthryl, acenaphthyl, biphenylyl and the like.

The "$C_{6-18}$ aryl" of the above-mentioned "optionally substituted $C_{6-18}$ aryl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-$C_{1-4}$ alkyl" of the above-mentioned "optionally substituted $C_{6-18}$ aryl-$C_{1-4}$ alkyl"

include benzyl, phenethyl, phenylpropyl, naphthylmethyl, biphenylylmethyl and the like.

The "$C_{6-18}$ aryl-$C_{1-4}$ alkyl" of the above-mentioned "optionally substituted $C_{6-18}$ aryl-$C_{1-4}$ alkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-carbonyl" of the above-mentioned "optionally substituted $C_{6-18}$ aryl-carbonyl" include phenylcarbonyl, naphthylcarbonyl, anthrylcarbonyl, phenanthrylcarbonyl, acenaphthylcarbonyl, biphenylylcarbonyl and the like.

The "$C_{6-18}$ aryl-carbonyl" of the above-mentioned "optionally substituted $C_{6-18}$ aryl-carbonyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "$C_{6-18}$ aryl-$C_{1-4}$ alkyl-carbonyl" of the above-mentioned "optionally substituted $C_{6-18}$ aryl-$C_{1-4}$ alkyl-carbonyl" include benzylcarbonyl, phenethylcarbonyl, phenylpropylcarbonyl, naphthylmethylcarbonyl, biphenylylmethylcarbonyl and the like.

The "$C_{6-18}$ aryl-$C_{1-4}$ alkyl-carbonyl" of the above-mentioned "optionally substituted $C_{6-18}$ aryl-$C_{1-4}$ alkyl-carbonyl" may have one or more (preferably 1 to 5, more preferably 1 to 3) substituents at the substitutable positions. Examples of such substituent include substituents selected from the below-mentioned substituent V. When the number of the substituents is not less than 2, the respective substituents may be the same or different.

Examples of the "heterocyclic group" of the above-mentioned "optionally substituted heterocyclic group" include an aromatic heterocyclic group and a non-aromatic heterocyclic group.

Examples of the "aromatic heterocyclic group" include a 4- to 7-membered (preferably 5- or 6-membered) monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group. Examples of the fused aromatic heterocyclic group include a group derived from a fused ring wherein a ring corresponding to such 4- to 7-membered monocyclic aromatic heterocyclic group, and 1 or 2 rings selected from a 5- or 6-membered aromatic heterocycle containing 1 or 2 nitrogen atoms, a 5-membered aromatic heterocycle containing one sulfur atom and a benzene ring are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo [2,3-b]pyrazin-6-yl), pyrrolopyrimidinyl (e.g., 1H-pyrrolo[2,3-d]pyrimidin-2-yl, 1H-pyrrolo[2,3-d]pyrimidin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like; and the like.

Examples of the "non-aromatic heterocyclic group" include a 4- to 7-membered (preferably 5- or 6-membered) monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group. Examples of the fused non-aromatic heterocyclic group include a group derived from a fused ring wherein a ring corresponding to such 4- to 7-membered monocyclic non-aromatic heterocyclic group, and 1 or 2 rings selected from a 5- or 6-membered heterocycle containing 1 or 2 nitrogen atoms, a 5-membered heterocycle containing one sulfur atom and a benzene ring are condensed, and the like.

Preferable examples of the non-aromatic heterocyclic group include monocyclic non-aromatic heterocyclic groups such as oxetanyl (e.g., 2-oxetanyl, 3-oxetanyl), pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like; fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like; and the like.

The "heterocyclic group" of the above-mentioned "optionally substituted heterocyclic group" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the above-mentioned "optionally substituted heterocyclyl-$C_{1-4}$ alkyl" include a group wherein $C_{1-4}$ alkyl (e.g., methoxy, ethoxy, propoxy, isopropoxy, butoxy, isobutoxy, sec-butoxy, tert-butoxy) is substituted by the above-mentioned "optionally substituted heterocyclic group".

Examples of the above-mentioned "optionally substituted heterocyclyl-carbonyl" include a group wherein the above-mentioned "optionally substituted heterocyclic group" is bonded to carbonyl.

Examples of the above-mentioned "optionally substituted heterocyclyl-$C_{1-4}$ alkyl-carbonyl" include a group wherein the above-mentioned "optionally substituted heterocyclyl-$C_{1-4}$ alkyl" is bonded to carbonyl.

Of the "optionally substituted group bonded via a carbon atom, a nitrogen atom or an oxygen atom" for $R^1$, examples of the "optionally substituted group bonded via a nitrogen atom" include

(i) amino,
(ii) amino mono-substituted by the above-mentioned "optionally substituted group bonded via a carbon atom", and
(iii) amino di-substituted by the above-mentioned "optionally substituted group bonded via a carbon atom" and $C_{1-6}$ alkyl (e.g., methyl, ethyl, propyl, isopropyl, butyl, isobutyl, sec-butyl, tert-butyl, pentyl, propyl etc.).

Of the "optionally substituted group bonded via a carbon atom, a nitrogen atom or an oxygen atom" for $R^1$, examples of the "optionally substituted group bonded via an oxygen atom" include hydroxyl optionally substituted by the above-mentioned "optionally substituted group bonded via a carbon atom".

As $R^1$, a hydrogen atom, a halogen atom and cyano are preferable, and a hydrogen atom and a halogen atom (particularly a chlorine atom) are particularly preferable.

$R^2$ is a hydrogen atom, or an optionally substituted group bonded via a carbon atom or a sulfur atom.

Of the "optionally substituted group bonded via a carbon atom or a sulfur atom" for $R^2$, examples of the "optionally substituted group bonded via a carbon atom" include those similar to the "optionally substituted group bonded via a carbon atom" for $R^1$.

Of the "optionally substituted group bonded via a carbon atom or a sulfur atom" for $R^2$, examples of the "optionally substituted group bonded via a sulfur atom" include mercapto optionally substituted by the above-mentioned "optionally substituted group bonded via a carbon atom" wherein the sulfur atom may be oxidized.

As $R^2$, a hydrogen atom or optionally substituted alkyl is preferable. Of these,

(1) a hydrogen atom, and
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-(C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,

(f) -$NR^5$-CO-(a 5- or 6-membered heterocycle optionally substituted by 1 or 2 $C_{1-4}$ alkyl),
(g) -$NR^5$-CO-$(CH_2)_n$-(a 6-membered heterocycle optionally substituted by $C_{1-4}$ alkyl),
(h) -$NR^5$-CO-($C_{3-6}$ cycloalkyl optionally substituted by substituent(s) selected from hydroxy and cyano),
(i) -$NR^5$-CO-($C_{6-10}$ aryl optionally substituted by $C_{1-4}$ alkyl optionally substituted by 1 to 3 halogen atoms),
(j) -$NR^5$-CO-$NR^{5'}$-$C_{3-6}$ cycloalkyl,
(k) -$NR^5$-$SO_2$-$C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo,

wherein n is an integer of 1 to 4, $R^5$ and $R^{5'}$ are each a hydrogen atom or $C_{1-4}$ alkyl, and -$(CH_2)_n$- is optionally substituted by $C_{1-4}$ alkyl,
are preferable, and methyl, and ethyl substituted by hydroxy are particularly preferable.

$R^3$ is a hydrogen atom or an optionally substituted aliphatic hydrocarbon group.

Examples of the "aliphatic hydrocarbon group" of the "optionally substituted aliphatic hydrocarbon group" for $R^3$ include those similar to the "optionally substituted $C_{1-8}$ alkyl", "optionally substituted $C_{2-8}$ alkenyl", "optionally substituted $C_{2-8}$ alkynyl" and "optionally substituted $C_{3-8}$ cycloalkyl" exemplified as the "optionally substituted group bonded via a carbon atom" for $R^1$.

As $R^3$, a hydrogen atom is preferable.

Ring A is an optionally substituted benzene ring.
The "benzene ring" of the "optionally substituted benzene ring" for ring A is optionally substituted by 1 to 5 substituents selected from the group consisting of

(1) $C_{3-10}$ cycloalkyl (e.g., cyclopropyl, cyclohexyl) optionally substituted by 1 to 3 substituents selected from the group consisting of

(a) halogen;
(b) hydroxy;
(c) carboxyl;
(d) sulfo;
(e) cyano;
(f) azido;
(g) nitro;
(h) nitroso;
(i) optionally halogenated $C_{1-4}$ alkyl (e.g., methyl, ethyl, propyl, isopropyl, butyl, isobutyl, sec-butyl, tert-butyl);
(j) optionally halogenated $C_{2-4}$ alkenyl (e.g., ethenyl, 1-propenyl, 2-propenyl, 2-methyl-1-propenyl, 1-butenyl, 2-butenyl, 3-butenyl);
(k) optionally halogenated $C_{2-4}$ alkynyl (e.g., ethynyl, 1-propynyl, 2-propynyl, 1-butynyl, 2-butynyl, 3-butynyl);
(l) $C_{3-7}$ cycloalkyl (e.g., cyclopropyl, cyclobutyl, cyclopentyl, cyclohexyl, cycloheptyl);
(m) $C_{6-14}$ aryl (e.g., phenyl, naphthyl, anthryl, phenanthryl, acenaphthyl, biphenylyl);
(n) $C_{7-16}$ aralkyl (e.g., benzyl, phenethyl, phenylpropyl, naphthylmethyl, biphenylylmethyl);
(o) formyl;
(p) optionally halogenated $C_{1-6}$ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl);
(q) optionally halogenated $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(r) optionally halogenated $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl);
(s) carbamoyl;
(t) mono- or di-substituted carbamoyl by optionally halogenated $C_{1-6}$ alkyl (e.g., methyl, ethyl, propyl, isopropyl, butyl, isobutyl, sec-butyl, tert-butyl);
(u) mono- or di-$C_{6-14}$ aryl-carbamoyl (e.g., phenylcarbamoyl, naphthylcarbamoyl, anthrylcarbamoyl, phenanthrylcarbamoyl, acenaphthylcarbamoyl, biphenylylcarbamoyl);
(v) mono- or di-optionally substituted thiocarbamoyl by optionally halogenated $C_{1-6}$ alkyl (e.g., methyl, ethyl, propyl, isopropyl, butyl, isobutyl, sec-butyl, tert-butyl);

(w) mono- or di-optionally substituted ureido by optionally halogenated $C_{1-6}$ alkyl (e.g., methyl, ethyl, propyl, isopropyl, butyl, isobutyl, sec-butyl, tert-butyl);
(x) mono- or di-$C_{6-14}$ aryl-ureido (e.g., phenylureido, naphthylureido, anthrylureido, phenanthrylureido, acenaphthylureido, biphenylylureido);
(y) mono- or di-optionally substituted sulfamoyl by optionally halogenated $C_{1-6}$ alkyl (e.g., methyl, ethyl, propyl, isopropyl, butyl, isobutyl, sec-butyl, tert-butyl);
(z) optionally halogenated $C_{1-6}$ alkoxy (e.g., methoxy, ethoxy, propoxy, isopropoxy, butoxy, tert-butoxy, pentyloxy, hexyloxy);

(aa) optionally halogenated $C_{2-6}$ alkenyloxy (e.g., ethenyloxy, 1-propenyloxy, 2-propenyloxy, 2-methyl-1-propenyloxy, 1-butenyloxy, 2-butenyloxy, 3-butenyloxy);
(bb) $C_{3-10}$ cycloalkyloxy (e.g., cyclopropyloxy, cyclobutyloxy, cyclopentyloxy, cyclohexyloxy, cycloheptyloxy);
(cc) $C_{7-13}$ aralkyloxy (e.g., benzyloxy, phenethyloxy, phenylpropyloxy, naphthylmethyloxy, biphenylylmethyloxy);
(dd) $C_{6-14}$ aryloxy (e.g., phenyloxy, naphthyloxy, anthryloxy, phenanthryloxy, acenaphthyloxy, biphenylyloxy);
(ee) $C_{1-6}$ alkyl-carbonyloxy (e.g., acetyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy);
(ff) $C_{3-10}$ cycloalkyl (e.g., cyclopropyl, cyclobutyl, cyclopentyl, cyclohexyl, cycloheptyl)-$C_{1-6}$ alkyloxy (e.g., methoxy, ethoxy, propoxy, isopropoxy, butoxy, tert-butoxy, pentyloxy, hexyloxy);
(gg) $C_{1-6}$ alkylsulfonyloxy (e.g., methylsulfonyloxy, ethylsulfonyloxy, propylsulfonyloxy, isopropylsulfonyloxy, butylsulfonyloxy, isobutylsulfonyloxy, sec-butylsulfonyloxy, tert-butylsulfonyloxy);
(hh) mercapto;
(ii) optionally halogenated $C_{1-6}$ alkylthio (e.g., methylthio, ethylthio, propylthio, isopropylthio, butylthio, isobutylthio, sec-butylthio, tert-butylthio);
(jj) $C_{7-13}$ aralkylthio (e.g., benzylthio, phenethylthio, phenylpropylthio, naphthylmethylthio, biphenylylmethylthio);
(kk) $C_{6-14}$ arylthio (e.g., phenylthio, naphthylthio, anthrylthio, phenanthrylthio, acenaphthylthio, biphenylylthio);
(11) $C_{1-6}$ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl);
(mm) oxo;
(nn) $C_{1-3}$ alkylenedioxy (e.g., methylenedioxy, ethylenedioxy, propylenedioxy); and
(oo) hydroxyimino optionally substituted by $C_{1-6}$ alkyl (e.g., methyl, ethyl, propyl, isopropyl, butyl, isobutyl, sec-butyl, tert-butyl); (hereinafter sometimes to be referred to as substituent group A),

(2) $C_{6-14}$ aryl (e.g., phenyl, naphthyl) optionally substituted by 1 to 3 substituents selected from substituent group A;
(3) a heterocyclic group (the heterocyclic group is similar to the "heterocyclic group" of the "optionally substituted heterocyclic group" exemplified as the "optionally substituted group bonded via a carbon atom" for $R^1$) optionally substituted by 1 to 3 substituents selected from substituent group A;
(4) amino optionally substituted by 1 or 2 substituents selected from the group consisting of

(a) $C_{1-6}$ alkyl (e.g., methyl, ethyl, propyl, isopropyl, butyl, isobutyl, sec-butyl, tert-butyl) optionally substituted by substituent(s) selected from the group consisting of halogen, hydroxy, $C_{3-7}$ cycloalkyl (e.g., cyclopropyl, cyclobutyl, cyclopentyl, cyclohexyl, cycloheptyl), $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and $C_{1-6}$ alkoxy (e.g., methoxy, ethoxy, propoxy, isopropoxy, butoxy, tert-butoxy, pentyloxy, hexyloxy);
(b) optionally halogenated $C_{2-4}$ alkenyl (e.g., ethenyl, 1-propenyl, 2-propenyl, 2-methyl-1-propenyl, 1-butenyl, 2-butenyl, 3-butenyl);
(c) optionally halogenated $C_{2-4}$ alkynyl (e.g., ethynyl, 1-propynyl, 2-propynyl, 1-butynyl, 2-butynyl, 3-butynyl);
(d) $C_{3-7}$ cycloalkyl (e.g., cyclopropyl, cyclobutyl, cyclopentyl, cyclohexyl, cycloheptyl);
(e) $C_{6-14}$ aryl (e.g., phenyl, naphthyl, anthryl, phenanthryl, acenaphthyl, biphenylyl);
(f) $C_{7-16}$ aralkyl (e.g., benzyl, phenethyl, phenylpropyl, naphthylmethyl, biphenylylmethyl);
(g) a 4- to 7-membered (preferably 5- or 6-membered) heterocyclic group (e.g., a non-aromatic heterocyclic group such as morpholinyl etc.) containing, as a ring-constituting atom besides carbon atoms, 1 to 4 heteroatoms selected from an oxygen atom, a sulfur atom and a nitrogen atom;
(h) formyl;
(i) $C_{1-6}$ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl) optionally substituted by substituent(s) selected from the group consisting of halogen, hydroxy, $C_{3-7}$ cycloalkyl (e.g., cyclopropyl, cyclobutyl, cyclopentyl, cyclohexyl, cycloheptyl), $C_{1-6}$ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and $C_{1-6}$ alkoxy (e.g., methoxy, ethoxy, propoxy, isopropoxy, butoxy, tert-butoxy, pentyloxy, hexyloxy);

(j) $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);

(k) $C_{6-14}$ aryl-carbonyl (e.g., benzoyl);

(l) $C_{7-13}$ aralkyl-carbonyl (e.g., benzylcarbonyl, phenethylcarbonyl);

(m) $C_{3-7}$ cycloalkyl-carbonyl (e.g., cyclopropylcarbonyl, cyclobutylcarbonyl, cyclopentylcarbonyl, cyclohexylcarbonyl, cycloheptylcarbonyl);

(o) $C_{1-6}$ alkyl-carbamoyl (e.g., methylcarbamoyl, ethylcarbamoyl);

(p) $C_{6-14}$ aryl-carbamoyl (e.g., phenylcarbamoyl, 1-naphthylcarbamoyl, 2-naphthylcarbamoyl);

(q) $C_{7-13}$ aralkyl-carbamoyl (e.g., benzylcarbamoyl);

(r) $C_{1-6}$ alkylsulfonyl (e.g., methylsulfonyl, ethylsulfonyl, isopropylsulfonyl);

(s) $C_{6-14}$ arylsulfonyl (e.g., benzenesulfonyl, toluenesulfonyl, 1-naphthalenesulfonyl, 2-naphthalenesulfonyl); and

(t) $C_{7-13}$ aralkylsulfonyl (e.g., benzylsulfonyl) (hereinafter sometimes to be referred to as substituent group B);

(5) amidino;

(6) optionally formylated or halogenated $C_{1-6}$ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl);

(7) optionally halogenated $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);

(8) optionally halogenated $C_{1-6}$ alkylsulfonyl (e.g., methylsulfonyl);

(9) carbamoyl optionally substituted by 1 or 2 substituents selected from substituent group B;

(10) thiocarbamoyl optionally mono- or di-substituted by optionally halogenated $C_{1-6}$ alkyl (e.g., methyl, ethyl, propyl, isopropyl, butyl, isobutyl, sec-butyl, tert-butyl);

(11) ureido optionally substituted by 1 or 2 substituents selected from substituent group B;

(12) sulfamoyl optionally substituted by 1 or 2 substituents selected from substituent group B;

(13) carboxyl;

(14) hydroxy;

(15) $C_{1-6}$ alkoxy (e.g., methoxy, ethoxy, propoxy, isopropoxy, butoxy, tert-butoxy, pentyloxy, hexyloxy) optionally substituted by 1 to 3 substituents selected from the group consisting of halogen, carboxyl, $C_{1-6}$ alkoxy (e.g., methoxy, ethoxy, propoxy, isopropoxy, butoxy, tert-butoxy, pentyloxy, hexyloxy) and $C_{1-6}$ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);

(16) optionally halogenated $C_{2-6}$ alkenyloxy (e.g., ethenyloxy);

(17) $C_{3-10}$ cycloalkyloxy (e.g., cyclohexyloxy);

(18) $C_{7-13}$ aralkyloxy (e.g., benzyloxy);

(19) $C_{6-14}$ aryloxy (e.g., phenyloxy, naphthyloxy);

(20) $C_{1-6}$ alkyl-carbonyloxy (e.g., acetyloxy, tert-butylcarbonyloxy);

(21) mercapto;

(22) optionally halogenated $C_{1-6}$ alkylthio (e.g., methylthio, ethylthio);

(23) $C_{7-13}$ aralkylthio (e.g., benzylthio);

(24) $C_{6-14}$ arylthio (e.g., phenylthio, naphthylthio);

(25) sulfo;

(26) cyano;

(27) azido;

(28) nitro;

(29) nitroso;

(30) a halogen atom;

(31) $C_{1-6}$ alkylsulfinyl (e.g., methylsulfinyl);

(32) oxo;

(33) $C_{3-10}$ cycloalkyl-$C_{1-6}$ alkyloxy (e.g., cyclopropylmethyloxy);

(34) $C_{1-3}$ alkylenedioxy (e.g., methylenedioxy, ethylenedioxy);

(35) hydroxyimino optionally substituted by $C_{1-6}$ alkyl;

(36) $C_{1-10}$ alkyl (e.g., methyl, ethyl, propyl, isopropyl, butyl, isobutyl, sec-butyl, tert-butyl) optionally substituted by 1 to 3 substituents selected from the above-mentioned (1) - (35);

(37) $C_{2-10}$ alkenyl (e.g., ethenyl, 1-propenyl) optionally substituted by 1 to 3 substituents selected from the above-mentioned (1) - (35), and

(38) $C_{7-13}$ aralkyl (e.g., benzyl) optionally substituted by 1 to 3 substituents selected from the above-mentioned (1) - (35) (hereinafter sometimes to be referred to as substituent group V). When the number of the substituents is not less than 2, the respective substituents may be the same or different.

As ring A, a benzene ring optionally substituted by 1 or 2 substituents selected from the group consisting of (1) a halogen atom and (2) $C_{1-4}$ alkyl is preferable. Of these, a benzene ring optionally substituted by 1 or 2 substituents selected from the group consisting of a halogen atom and methyl is preferable. Particularly, a benzene ring optionally substituted by a substituent selected from the group consisting of a halogen atom and methyl is preferable.
Ring B is

(i) an optionally substituted fused ring, or
(ii) a pyridine ring having optionally substituted carbamoyl (the pyridine ring is optionally further substituted).

Examples of the "optionally substituted fused ring" for ring B include "optionally substituted fused homocycle" and "optionally substituted fused heterocycle".
Examples of the "fused homocycle" of the "optionally substituted fused homocycle" include a ring wherein two or more, the same or different rings selected from benzene, $C_{3-8}$ cycloalkane (e.g., cyclopropane, cyclobutane, cyclopentane, cyclohexane, cycloheptane, cyclooctane), $C_{3-8}$ cycloalkene (e.g., cyclopropene, cyclobutene, cyclopentene, cyclohexene, cycloheptene, cyclooctene), $C_{4-8}$ cycloalkadiene (e.g., cyclobutadiene, cyclopentadiene, cyclohexadiene, cycloheptadiene, cyclooctadiene), $C_{7-8}$ cycloalkatriene (e.g., cycloheptatriene, cyclooctatriene) and cyclooctatetraene are condensed. Specifically, naphthalene, dihydronaphthalene, tetrahydronaphthalene, hexahydronaphthalene, decahydronaphthalene, pentalene, indene, indane, azulene, heptalene and the like.
Examples of the "fused heterocycle" of the "optionally substituted fused heterocycle" include fused aromatic heterocycles such as quinoline, isoquinoline, quinazoline, quinoxaline, benzofuran, benzothiophene, benzoxazole, benzisoxazole, benzothiazole, benzimidazole, benzotriazole, indole, indazole, pyrrolopyridine, pyrrolopyrimidine, pyrrolopyrazine, imidazopyridine, imidazopyrazine, imidazopyridazine, pyrazolopyridine, pyrazolothiophene, pyrazolotriazine, triazolopyridine and the like;
fused non-aromatic heterocycles such as dihydroindole, dihydroisoindole, dihydrobenzofuran, dihydrobenzothiophene, dihydrobenzodioxine, dihydrobenzodioxepine, tetrahydrobenzofuran, tetrahydrobenzothiophene, chromene, dihydroquinoline, tetrahydroquinoline, dihydroisoquinoline, tetrahydroisoquinoline, dihydrophthalazine, benzoxazoline, benzisoxazoline, benzothiazoline, benzimidazoline, benzotriazoline, indazoline, dihydropyrrolopyridine and the like.
The "fused ring" of the "optionally substituted fused ring" for ring B may have one or more (preferably 1 to 5, more preferably 1 to 3) substituents at the substitutable positions. Examples of such substituent include substituents selected from substituent group V and $C_{2-4}$ alkylene (e.g., ethylene, propylene, trimethylene, tetramethylene). The $C_{2-4}$ alkylene may be bonded to a single carbon atom on ring B to form a spiro ring. When the number of the substituents is not less than 2, the respective substituents may be the same or different.
Examples of the "optionally substituted carbamoyl" of the "pyridine ring having optionally substituted carbamoyl" for ring B include carbamoyl optionally mono- or di-substituted by a group similar to the "optionally substituted group bonded via a carbon atom" exemplified as $R^1$.
The "pyridine ring having optionally substituted carbamoyl" for ring B is optionally further substituted. Examples of such substituent include substituents selected from substituent group V.
As ring B,

(1) a fused ring optionally substituted by substituent(s) selected from the group consisting of

(a) a halogen atom,
(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and
(i) $C_{1-6}$ alkoxy-carbonyl, and

(2) a pyridine ring having carbamoyl optionally substituted by $C_{1-6}$ alkyl (the pyridine ring is optionally further substituted by $C_{1-6}$ alkyl) are preferable.

Of these,

(1) a fused homocycle (e.g., indane, naphthalene) optionally having, as substituents, 1 or 2 substituents selected from the group consisting of (a) $C_{1-6}$ alkyl optionally substituted by halogen atom(s), (b) hydroxy and (c) oxo;
(2) a fused heterocycle (e.g., quinoline, isoquinoline, quinazoline, quinoxaline, benzoxazoline, benzisoxazoline, benzothiazoline, benzimidazoline, benzotriazoline, indole, indazole, pyrrolopyridine, dihydropyrrolopyridine, benzoxazole, benzimidazole, benzothiazole, benzisoxazole, benzisothiazole, pyrrolopyrimidine, imidazopyridazine, indazoline, pyrrolopyrazine, imidazopyridine, imidazopyrazine, pyrazolopyridine, pyrazolothiophene, pyrazolotriazine, dihydroindole, dihydroisoindole, dihydroquinoline, tetrahydroquinoline, dihydroisoquinoline, tetrahydroisoquinoline, dihydrophthalazine, dihydrobenzoxazole, benzothiophene, benzofuran, dihydrobenzothiophene, dihydrobenzofuran etc.) optionally having, as substituents, 1 to 4 substituents selected from the group consisting of (a) a halogen atom, (b) cyano, (c) $C_{1-6}$ alkyl optionally substituted by a halogen atom or $C_{3-6}$ cycloalkyl, (d) oxo, (e) $C_{2-4}$ alkylene, (f) carbamoyl, (g) $C_{1-6}$ alkyl-carbamoyl, and (h) $C_{1-6}$ alkoxy-carbonyl; and
(3) a pyridine ring having carbamoyl optionally substituted by $C_{1-8}$ alkyl (the pyridine ring is optionally further substituted by $C_{1-6}$ alkyl) ; are preferable.

Particularly,

(1) indole optionally having one $C_{1-4}$ alkyl;
(2) pyrrolopyrimidine optionally having one $C_{1-4}$ alkyl;
(3) imidazopyridine;
(4) dihydroindole optionally having 1 or 2 substituents selected from the group consisting of (a) $C_{1-4}$ alkyl optionally substituted by $C_{3-6}$ cycloalkyl, (b) a halogen atom, (c) $C_{2-4}$ alkylene and (d) oxo;
(5) dihydroisoindole optionally having 1 to 4 substituents selected from the group consisting of (a) $C_{1-4}$ alkyl, (b) a halogen atom and (c) oxo;
(6) dihydrobenzoxazole optionally having 1 or 2 substituents selected from the group consisting of $C_{1-4}$ alkyl and oxo;
(7) pyrrolopyridine;
(8) indane optionally having 1 or 2 substituents selected from (a) $C_{1-6}$ alkyl optionally substituted by 1 to 3 halogen atoms, (b) hydroxy and (c) oxo;
(9) benzothiophene;
(10) indazole optionally having 1 substituent selected from the group consisting of a halogen atom and $C_{1-4}$ alkyl;
(11) dihydrobenzofuran;
(12) quinoline optionally having 1 substituent selected from the group consisting of (a) a halogen atom and (b) $C_{1-6}$ alkyl optionally substituted by 1 to 3 halogen atoms;
(13) benzoxazole;
(14) benzofuran optionally having 1 substituent selected from the group consisting of (a) cyano and (b) carbamoyl;
(15) benzisoxazole optionally having one $C_{1-4}$ alkyl;
(16) pyrazolopyridine optionally having 1 substituent selected from the group consisting of (a) $C_{1-6}$ alkoxy-carbonyl and (b) $C_{1-6}$ alkyl-carbamoyl;
(17) benzimidazole optionally having one or two $C_{1-4}$ alkyl;
(18) triazolopyridine;
(19) naphthalene; and
(20) pyridine substituted by $C_{1-8}$ alkyl-carbamoyl and optionally further substituted by $C_{1-6}$ alkyl; are preferable.

$R^1$ and $R^2$ are optionally bonded to each other to form an optionally substituted ring structure. Examples of the "ring structure" include a saturated or unsaturated (preferably saturated) 4- to 8-membered (preferably 5- to 7-membered) heterocycle.

Examples of the "ring structure" of the "optionally substituted ring structure" formed by $R^1$ and $R^2$ bonded to each other include

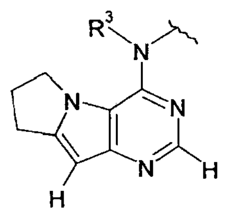,
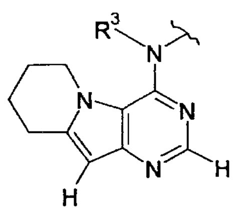,
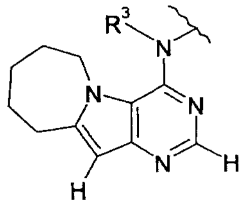,
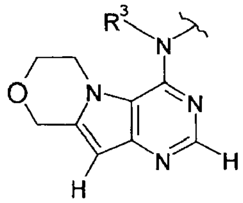

wherein each symbol is as defined above,
and the like.

$R^2$ and $R^3$ are optionally bonded to each other to form an optionally substituted ring structure. Examples of the "ring structure" include a saturated or unsaturated (preferably saturated) 4- to 8-membered (preferably 5- to 7-membered) heterocycle.

Examples of the "ring structure" of the "optionally substituted ring structure" formed by $R^2$ and $R^3$ bonded to each other include

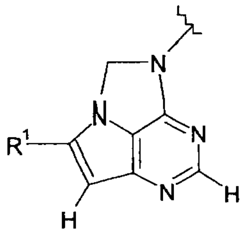,
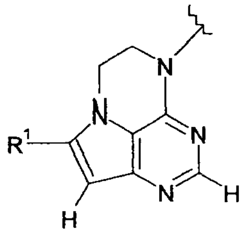,
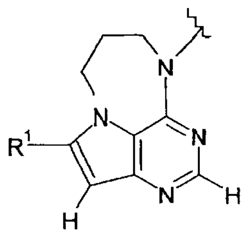

wherein each symbol is as defined above,
and the like.

The "ring structure" of the "optionally substituted ring structure" formed by $R^1$ and $R^2$, or $R^2$ and $R^3$, optionally has 1 to 5 (preferably 1 to 3, more preferably 1 or 2), the same or different substituents at any substitutable positions. Examples of the substituent include substituents selected from substituent group V. When the number of the substituents is not less than 2, the respective substituents may be the same or different.

Examples of the "ring structure" of the "optionally substituted ring structure" formed by $R^3$ bonded to the carbon atom on the adjacent benzene ring (ring A) include a saturated or unsaturated (preferably saturated) 4- to 8-membered (preferably 5- or 6-membered) nitrogen-containing heterocycle.

Specifically, the moiety of

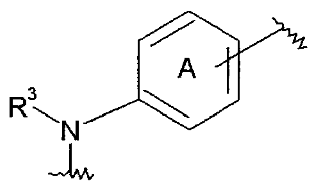

wherein each symbol is as defined above, is, for example,

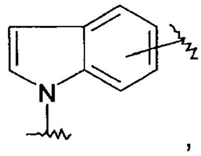, 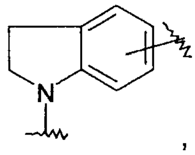, 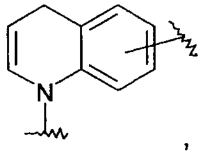, 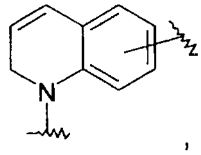, 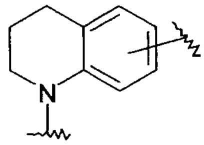

and the like.

The "ring structure" optionally has 1 to 5 (preferably 1 to 3, more preferably 1 or 2), the same or different substituents at any substitutable positions. Examples of the substituent include substituents selected from substituent group V. When the number of the substituents is not less than 2, the respective substituents may be the same or different..

Preferable compounds of compound (I) are as follows.

[Compound A]

Compound (I) wherein
$R^1$ is a hydrogen atom, a halogen atom or cyano;
$R^2$ is a hydrogen atom or optionally substituted alkyl;
$R^3$ is a hydrogen atom;
ring A is a benzene ring optionally substituted by 1 or 2 substituents selected from the group consisting of (1) a halogen atom and (2) $C_{1-4}$ alkyl; and
ring B is

(1) a fused ring optionally substituted by substituent(s) selected from the group consisting of

(a) a halogen atom,
(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and
(i) $C_{1-6}$ alkoxy-carbonyl, or

(2) a pyridine ring having carbamoyl optionally substituted by $C_{1-6}$ alkyl (the pyridine ring is optionally further substituted by $C_{1-6}$ alkyl).

[Compound B]

Compound (I) wherein
$R^1$ is a hydrogen atom, a halogen atom or cyano;
$R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-$($C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,
(f) $-NR^5-CO-$(a 5- or 6-membered heterocycle optionally substituted by 1 or 2 $C_{1-4}$ alkyl),
(g) $-NR^5-CO-(CH_2)_n-$(a 6-membered heterocycle optionally substituted by $C_{1-4}$ alkyl),
(h) $-NR^5-CO-$($C_{3-6}$ cycloalkyl optionally substituted by substituent(s) selected from hydroxy and cyano),
(i) $-NR^5-CO-$($C_{6-10}$ aryl optionally substituted by $C_{1-4}$ alkyl optionally substituted by 1 to 3 halogen atoms),

(j) $-NR^5-CO-NR^{5'}-C_{3-6}$ cycloalkyl,
(k) $-NR^5-SO_2-C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo,

wherein n is an integer of 1 to 4, $R^5$ and $R^{5'}$ are each a hydrogen atom or $C_{1-4}$ alkyl, and $-(CH_2)_n-$ is optionally substituted by $C_{1-4}$ alkyl;
$R^3$ is a hydrogen atom;
ring A is a benzene ring optionally substituted by 1 or 2 substituents selected from the group consisting of (1) a halogen atom and (2) $C_{1-4}$ alkyl; and
ring B is

(1) a fused homocycle optionally having, as substituents, 1 or 2 substituents selected from the group consisting of (a) $C_{1-6}$ alkyl optionally substituted by halogen atom(s), (b) hydroxy and (c) oxo;
(2) a fused heterocycle optionally having, as substituents, 1 to 4 substituents selected from the group consisting of (a) a halogen atom, (b) cyano, (c) $C_{1-6}$ alkyl optionally substituted by a halogen atom or $C_{3-6}$ cycloalkyl, (d) oxo, (e) $C_{2-4}$ alkylene, (f) carbamoyl, (g) $C_{1-6}$ alkyl-carbamoyl, and (h) $C_{1-6}$ alkoxy-carbonyl; or
(3) a pyridine ring having carbamoyl optionally substituted by $C_{1-8}$ alkyl (the pyridine ring is optionally further substituted by $C_{1-6}$ alkyl).

[Compound C]

Compound (I) wherein
$R^1$ is a hydrogen atom, a halogen atom or cyano;
$R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-(C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,
(f) $-NR^5-CO-$(a 5- or 6-membered heterocycle optionally substituted by 1 or 2 $C_{1-4}$ alkyl),
(g) $-NR^5-CO-(CH_2)_n-$(a 6-membered heterocycle optionally substituted by $C_{1-4}$ alkyl),
(h) $-NR^5-CO-(C_{3-6}$ cycloalkyl optionally substituted by substituent(s) selected from hydroxy and cyano),
(i) $-NR^5-CO-(C_{6-10}$ aryl optionally substituted by $C_{1-4}$ alkyl optionally substituted by 1 to 3 halogen atoms),
(j) $-NR^5-CO-NR^{5'}-C_{3-6}$ cycloalkyl,
(k) $-NR^5-SO_2-C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo,

wherein n is an integer of 1 to 4, $R^5$ and $R^{5'}$ are each a hydrogen atom or $C_{1-4}$ alkyl, and $-(CH_2)_n-$ is optionally substituted by $C_{1-4}$ alkyl;

$R^3$ is a hydrogen atom;
ring A is a benzene ring optionally substituted by 1 or 2 substituents selected from the group consisting of (1) a halogen atom and (2) $C_{1-4}$ alkyl; and

ring B is

(1) indole optionally having one $C_{1-4}$ alkyl;
(2) pyrrolopyrimidine optionally having one $C_{1-4}$ alkyl;
(3) imidazopyridine;
(4) dihydroindole optionally having 1 or 2 substituents selected from the group consisting of (a) $C_{1-4}$ alkyl optionally substituted by $C_{3-6}$ cycloalkyl, (b) a halogen atom, (c) $C_{2-4}$ alkylene and (d) oxo;
(5) dihydroisoindole optionally having 1 to 4 substituents selected from the group consisting of (a) $C_{1-4}$ alkyl, (b) a halogen atom and (c) oxo;
(6) dihydrobenzoxazole optionally having 1 or 2 substituents selected from the group consisting of $C_{1-4}$ alkyl and oxo;
(7) pyrrolopyridine;
(8) indane optionally having 1 or 2 substituents selected from

(a) $C_{1-6}$ alkyl optionally substituted by 1 to 3 halogen atoms,
(b) hydroxy and (c) oxo;

(9) benzothiophene;
(10) indazole optionally having 1 substituent selected from the group consisting of a halogen atom and $C_{1-4}$ alkyl;
(11) dihydrobenzofuran;
(12) quinoline optionally having 1 substituent selected from the group consisting of (a) a halogen atom and (b) $C_{1-6}$ alkyl optionally substituted by 1 to 3 halogen atoms;
(13) benzoxazole;
(14) benzofuran optionally having 1 substituent selected from the group consisting of (a) cyano and (b) carbamoyl;
(15) benzisoxazole optionally having one $C_{1-4}$ alkyl;
(16) pyrazolopyridine optionally having 1 substituent selected from the group consisting of (a) $C_{1-6}$ alkoxy-carbonyl and (b) $C_{1-6}$ alkyl-carbamoyl;
(17) benzimidazole optionally having one or two $C_{1-4}$ alkyl;
(18) triazolopyridine;
(19) naphthalene; or
(20) pyridine substituted by $C_{1-8}$ alkyl-carbamoyl and optionally further substituted by $C_{1-6}$ alkyl.

[Compound D]

Compound (I) which is
N-{2-[4-({3-chloro-4-[(2-oxo-2,3-dihydro-1H-indol-4-yl)oxy]phenyl}amino)-5H-pyrrolo[3,2-d]pyrimidin-5-yl]ethyl}-2-(methylsulfonyl)acetamide (Example 6);
4-(2-chloro-4-{[5-(2-hydroxyethyl)-5H-pyrrolo[3,2-d]pyrimidin-4-yl]amino}phenoxy)-1-methyl-1,3-dihydro-2H-indol-2-one (Example 8);
2-(4-{[3-chloro-4-(1H-indol-4-yloxy)phenyl]amino}-5H-pyrrolo[3,2-d]pyrimidin-5-yl)ethanol (Example 10);
N-[3-chloro-4-(1H-indol-4-yloxy)phenyl]-5-methyl-5H-pyrrolo[3,2-d]pyrimidin-4-amine (Example 22);
4-(2-chloro-4-{[5-(2-hydroxyethyl)-5H-pyrrolo[3,2-d]pyrimidin-4-yl]amino}phenoxy)-1,3-dihydro-2H-indol-2-one (Example 28);
N-[2-(4-{[4-(1-benzothiophen-6-yloxy)-3-chlorophenyl]amino}-5H-pyrrolo[3,2-d]pyrimidin-5-yl)ethyl]-3-hydroxy-2,2-dimethylpropanamide (Example 53);
N-[2-(4-{[4-(1-benzothiophen-6-yloxy)-3-chlorophenyl]amino}-5H-pyrrolo[3,2-d]pyrimidin-5-yl)ethyl]-2-(methylsulfonyl)acetamide (Example 60);
2-(4-{[4-(1-benzothiophen-4-yloxy)-3-chlorophenyl]amino}-5H-pyrrolo[3,2-d]pyrimidin-5-yl)ethanol (Example 83);
N-[3-chloro-4-(1H-indazol-4-yloxy)phenyl]-5-methyl-5H-pyrrolo[3,2-d]pyrimidin-4-amine (Example 86);
2-(4-{[3-chloro-4-(1H-indazol-4-yloxy)phenyl]amino}-5H-pyrrolo[3,2-d]pyrimidin-5-yl)ethanol (Example 95);
N-[2-(4-{[4-(1-benzothiophen-4-yloxy)-3-chlorophenyl]amino}-5H-pyrrolo[3,2-d]pyrimidin-5-yl)ethyl]acetamide (Example 99);
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide (Example 114);
N-[2-(4-{[4-(1-benzothiophen-4-yloxy)-3-chlorophenyl]amino}-5H-pyrrolo[3,2-d]pyrimidin-5-yl)ethyl]acetamide (Example 119);
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide (Example 127);
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-3-methylbutanamide (Example 148);
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-2,2-dimethylpropanamide (Example 159);
N-[2-(4-{[3-chloro-4-(imidazo[1,2-a]pyridin-7-yloxy)phenyl]amino}-5H-pyrrolo[3,2-d]pyrimidin-5-yl)ethyl]-3-hydroxy-2,2-dimethylpropanamide (Example 164);
N-[2-(4-{[3-chloro-4-([1,2,4]triazolo[1,5-a]pyridin-7-yloxy)phenyl]amino}-5H-pyrrolo[3,2-d]pyrimidin-5-yl)ethyl]-3-hydroxy-2,2-dimethylpropanamide (Example 166);
4-(2-chloro-4-{[6-chloro-5-(2-hydroxyethyl)-5H-pyrrolo[3,2-d]pyrimidin-4-yl]amino}phenoxy)-1-methyl-1,3-dihydro-2H-indol-2-one (Example 170); or
N-[2-(4-{[4-(1-benzothiophen-4-yloxy)-3-chlorophenyl]amino}-5H-pyrrolo[3,2-d]pyrimidin-5-yl)ethyl]-2-methylalaninamide (Example 207)
or a salt thereof.

Examples of the salts of compound (I) include metal salts, ammonium salts, salts with organic base, salts with inorganic acid, salts with organic acid, salts with basic or acidic amino acid and the like.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salts with organic base include salts with trimethylamine, triethylamine, pyridine, picoline, 2,6-lutidine, ethanolamine, diethanolamine, triethanolamine, tromethamine[tris(hydroxymethyl)methylamine], t-butylamine, cyclohexylamine, dicyclohexylamine, N,N'-dibenzylethylenediamine and the like.

Preferable examples of salts with inorganic acid include salts with hydrochloric acid, hydrobromic acid, nitric acid, sulfuric acid, phosphoric acid and the like.

Preferable examples of the salts with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acid include salts with arginine, lysine, ornithine and the like.

Preferable examples of the salts with acidic amino acid include salts with aspartic acid, glutamic acid and the like.

Of these, pharmaceutically acceptable salts are preferable. When a compound contains an acidic functional group, for example, inorganic salts such as alkali metal salts (e.g., sodium salt, potassium salt etc.), alkaline earth metal salts (e.g., calcium salt, magnesium salt, barium salt etc.) and the like, ammonium salt and the like can be mentioned. When a compound contains a basic functional group, for example, salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

A production method of compound (I) is described below.

The production intermediate includes salts, and as such salts, for example, those similar to the salts of compound (I) and the like can be used.

The compound obtained in each step can be used as a reaction mixture or as a crude product in the next reaction. In addition, the compound can be isolated from a reaction mixture according to a conventional method, and can be easily purified by a separation means such as recrystallization, distillation, chromatography and the like.

Schematic reaction formulas are shown in the following, wherein each symbol in the compounds is as defined above.

Compound (I) of the present invention can be produced, for example, by reacting a compound represented by the formula:

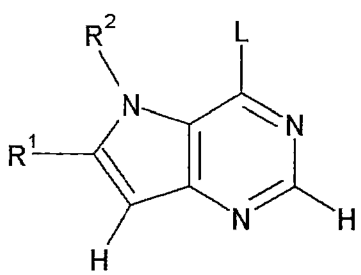

(II)

wherein L is a leaving group, and other symbols are as defined above (hereinafter sometimes to be abbreviated as compound (II)), or a salt thereof with a compound represented by the formula:

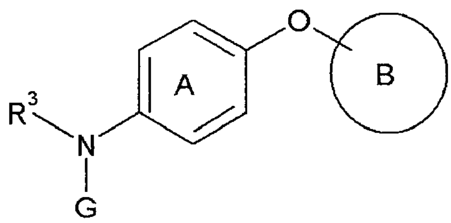

(III)

wherein G is a hydrogen atom or a metal atom, and other symbols are as defined above (hereinafter sometimes to be abbreviated as compound (III)), or a salt thereof.

G is mainly a hydrogen atom, but may be an alkali metal such as lithium, sodium, potassium, cesium and the like, or an alkaline earth metal such as magnesium, calcium and the like.

Compound (III) or a salt thereof is preferably used in an amount of 1 to 5 equivalents, preferably 1 to 2 equivalents, relative to compound (II) and the reaction is preferably carried out in a solvent. In addition, a base or an ammonium salt may be used in an amount of about 0.01 to 10 equivalents, preferably 0.1 to 2 equivalents.

In the aforementioned formula, as the leaving group for L, a halogen atom such as chlorine, bromine, iodine and the like, a group represented by the formula: $-S(O)_kR^z$ wherein k is an integer of 0, 1 or 2, and $R^z$ is a lower($C_{1-4}$) alkyl such as methyl, ethyl, propyl and the like; a $C_{6-10}$ aryl such as phenyl and tolyl; $C_{7-13}$ aralkyl such as benzyl and the like, and the like, or a group represented by the formula: $-OR^z$ wherein $R^z$ is as defined above, and the like can be used.

As the solvent in the aforementioned reaction, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used.

As the base in the aforementioned reaction, an inorganic base, an organic base and the like can be used. Specifically, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used.

As the ammonium salt in the aforementioned reaction, pyridine hydrochloride, pyridine hydrobromide, pyridinium p-toluenesulfonate, quinoline hydrochloride, isoquinoline hydrochloride, pyrimidine hydrochloride, pyrazine hydrochloride, triazine hydrochloride, trimethylamine hydrochloride, triethylamine hydrochloride, N-ethyldiisopropylamine hydrochloride and the like can be used.

The aforementioned reaction can be carried out under cooling, at room temperature or under heating (about 40 to 200°C, preferably about 40 to 160°C), and the reaction time is generally about 1 to 30 hr, preferably about 1 to 20 hr, more preferably about 1 to 10 hr.

A compound within the scope of the present invention can be also produced by applying means known *per se* to the obtained compound (I) for introduction of substituents and conversion of functional groups. For conversion of substituents, a known conventional method can be used. For example, conversion to carboxy by hydrolysis of ester, conversion to carbamoyl by amidation of carboxy, conversion to hydroxymethyl by reduction of carboxy, conversion to alcohol compound by reduction or alkylation of carbonyl, reductive amination of carbonyl, oximation of carbonyl, acylation of amino, alkylation of amino, substitution and amination of active halogen by amine, alkylation of hydroxy, substitution and amination of hydroxy and the like can be mentioned. When a reactive substituent that causes non-objective reaction is present during the introduction of substituents and conversion of functional groups, a protecting group is introduced in advance as necessary into the reactive substituent by a means known *per se*, and the protecting group is removed by a means known *per se* after the objective reaction, whereby the compound within the scope of the present invention can be also produced.

The compound (I), which is a product of the reaction, may be produced as a single compound or as a mixture.

The compound (I) thus obtained can be subjected to a means known *per se*, such as solvent extraction, concentration, neutralization, filtration, crystallization, recrystallization, column chromatography, high performance liquid chromatography and the like, whereby the objective compound can be isolated and purified at high purity from a reaction mixture.

The starting compound (III) of this production method is commercially available, or can be produced by a means known *per se*.

The starting compound (II) of this production method can be produced by, for example, a method shown by the following scheme. Here, compounds (IIa), (IIb), (IIc) and (IId) are encompassed in compound (II).

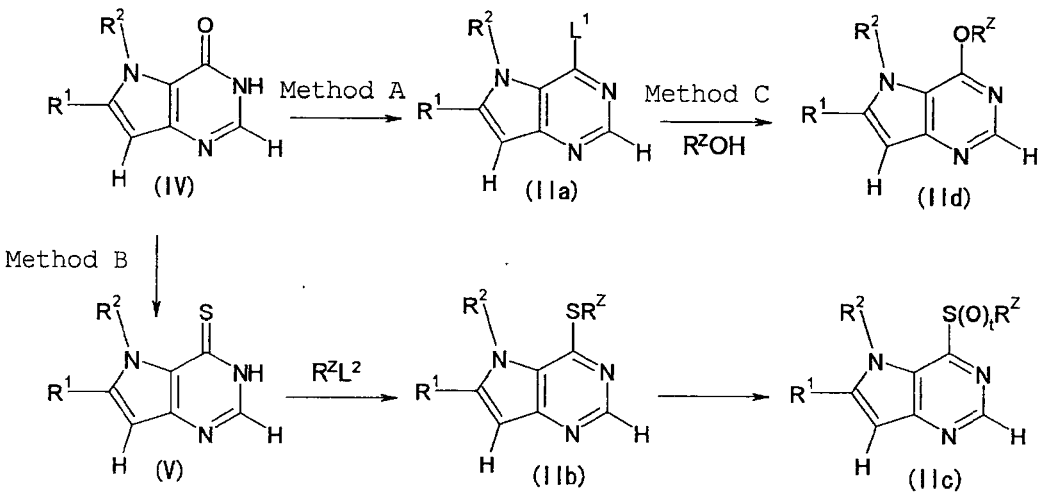


wherein $L^{1a}$ and $L^{2a}$ are halogen atoms, t is an integer of 1 or 2, and $R^z$ is as defined above.

As Method A, compound (IIa) can be produced by reacting compound (IV) with a halogenating agent. As Method B, compound (IV) is reacted with a thionating agent to give compound (V), which is then reacted with a compound represented by $R^zL^2$ in the presence of a base to give compound (IIb), which is further subjected to an oxidation reaction to give compound (IIc). As Method C, compound (IId) can be produced by reacting compound (IIa) with a compound represented by $R^zOH$ in the presence of a base.

As the halogenating agent in Method A, for example, about 1 to 100 equivalents of phosphorus oxychloride, phosphorus pentachloride, phosphorus trichloride, thionyl chloride, sulfuryl chloride, phosphorus tribromide and the like can be used. In this case, the reaction may be carried out in the presence of a base such as diethylaniline, dimethylaniline, pyridine and the like. While the reaction may be carried out without solvent, as a reaction solvent, for example, halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetonitrile, ethyl acetate and the like may be used. The reaction is carried out under cooling, at room temperature or under heating, and the reaction time is generally about 1 to 20 hr, preferably about 1 to 10 hr.

As the thionating agent used in the production step from compound (IV) to compound (V) in Method B, for example, about 1 to 5 equivalents of the Lawesson's reagent, phosphorus pentasulfide and the like can be used. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; and the like can be used. The reaction is carried out at room temperature or under heating, and the reaction time is generally about 1 to 20 hr, preferably about 1 to 10 hr.

As $R^zZ^2$ in the production step from compound (V) to compound (IIb) in Method B, for example, about 1 to 5 equivalents of methyl iodide, benzyl chloride, benzyl bromide and the like can be used, and as the base, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

As the oxidizing agent in the production step from compound (IIb) to compound (IIc) in Method B, for example, m-chloroperbenzoic acid, hydrogen peroxide, peracetic acid, t-butyl hydroperoxide, potassium peroxysulfate, potassium permanganate, sodium perborate, sodium periodate, sodium hypochlorite, halogen and the like can be used. When compound (IIc) wherein t=1 is produced, the oxidizing agent is used in about 1 to 1.5 equivalents relative to compound (IIb), and when compound (IIc) wherein t=2 is produced, it is used in about 2 to 3 equivalents relative to compound (IIb). The reaction solvent is not particularly limited as long as it does not react with the oxidizing agent and, for example,

halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane and the like; carboxylic acids such as acetic acid, trifluoroacetic acid and the like; acetonitrile, ethyl acetate, N,N-dimethylformamide, N,N-dimethylacetamide, 1-methyl-2-pyrrolidone, dimethyl sulfoxide, water or a mixed solvent thereof and the like can be used. The reaction is carried out under cooling, at room temperature or under heating, and the reaction time is generally about 1 to 20 hr, preferably about 1 to 10 hr.

As $R^zOH$ in the production step from compound (IIa) to compound (IId) in Method C, for example, about 1 to 10 equivalents of methanol, ethanol, phenol and the like can be used, and as the base, for example, sodium hydroxide, potassium hydroxide, sodium carbonate, potassium carbonate, sodium hydrogen carbonate, potassium hydrogen carbonate, triethylamine, N-ethyldiisopropylamine, pyridine, N,N-dimethylaminopyridine, sodium methoxide, sodium ethoxide, potassium t-butoxide, sodium hydride, sodium amide, diazabicycloundecene (DBU) and the like can be used. As a reaction solvent, for example, halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

Furthermore, compound (IV) can be produced by, for example, a method shown by the following formula:

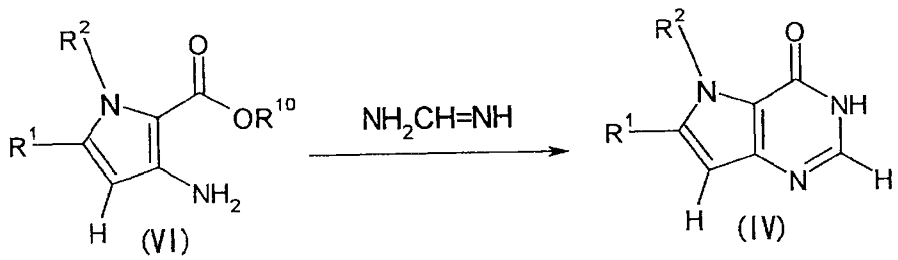


wherein $R^{10}$ is $C_{1-4}$ alkyl, and other symbols are as defined above.

That is, compound (VI) is reacted with about 1 to 4 equivalents of formamidine or a salt thereof to give compound (IV). As the reaction solvent, for example, alcohols such as methanol, ethanol, isopropanol, t-butanol and the like; halogenated hydrocarbons such as dichloromethane, chloroform, carbon tetrachloride, 1,2-dichloroethane and the like; aromatic hydrocarbons such as benzene, toluene, xylene and the like; ethers such as diethyl ether, tetrahydrofuran, dioxane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out under cooling, at room temperature or under heating, and the reaction time is generally about 1 to 20 hr, preferably about 1 to 10 hr.

Compound (II) can be also produced by, for example, a method shown by the following formula:

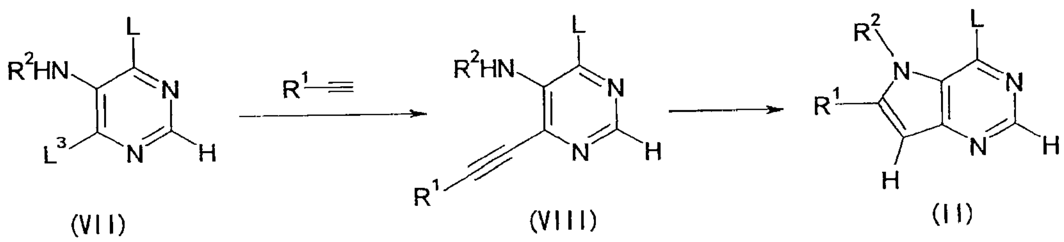

wherein $L^3$ is a halogen atom, and other symbols are as defined above.

For the production step from compound (VII) to compound (VIII) in this method, a reaction generally known as a Sonogashira reaction or a reaction analogous thereto can be carried out, and generally, compound (VIII) can be produced by reacting compound (VII) with about 1 to 3 equivalents of a compound represented by the formula:

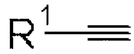

in the presence of a base, about 0.01-1 equivalent of a palladium catalyst and copper iodide. As the base, for example, triethylamine, N-ethyldiisopropylamine, diisopropylamine, pyridine, N,N-dimethylaminopyridine, diazabicycloundecene (DBU), sodium carbonate, potassium carbonate, sodium hydrogen carbonate, potassium hydrogen carbonate and the like can be used. As the palladium catalyst, for example, dichlorobis(triphenylphosphine)palladium(II), palladium on carbon, palladium(II) diacetate, bis(benzonitrile)dichloropalladium(II) and the like can be used. This reaction may be carried out in the co-presence of a tertiary phosphine compound such as triphenylphosphine, tributylphosphine and the like as a ligand. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane, 1,2-dimethoxyethane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. This reaction is carried out at room temperature or under heating, and the reaction time is generally about 1 to 50 hr, preferably about 1 to 20 hr.

For the production step from compound (VIII) to compound (II) in this method, a cyclization reaction is generally carried out in the presence of about 1 to 3 equivalents of a base or about 0.01-1 equivalent of copper iodide to give compound (II). As the base, for example, potassium t-butoxide, sodium t-butoxide, cesium t-butoxide, sodium ethoxide, potassium hydride, sodium hydride, cesium hydroxide, sodium hydroxide, potassium hydroxide, sodium carbonate, potassium carbonate, sodium hydrogen carbonate, potassium hydrogen carbonate, triethylamine, N-ethyldiisopropylamine, diisopropylamine, pyridine, N,N-dimethylaminopyridine, diazabicycloundecene (DBU) and the like can be used. As the reaction solvent, for example, halogenated hydrocarbons such as dichloromethane, chloroform, carbon tetrachloride, 1,2-dichloroethane and the like; aromatic hydrocarbons such as benzene, toluene, xylene and the like; alcohols such as methanol, ethanol, isopropanol, t-butanol and the like; ethers such as diethyl ether, tetrahydrofuran, dioxane, 1,2-dimethoxyethane and the like; acetone, acetonitrile, ethyl acetate, N,N-dimethylformamide, N,N-dimethylacetamide, 1-methyl-2-pyrrolidone, dimethyl sulfoxide, hexamethylphosphoramide, water or a mixed solvent thereof and the like can be used. The reaction is carried out at low temperature, at room temperature or under heating, and the reaction time is generally about 1 to 50 hr, preferably about 1 to 20 hr.

Depending on the kind of the substituent of starting compound (II), a starting compound (II) having a different substituent can be produced by substituent conversion from, as a starting material, a compound produced by the above-mentioned production method. For the substituent conversion, a known general method can be used. For example, conversion to carbamoyl by hydrolysis and amidation of ester, conversion to hydroxymethyl by reduction of carboxy, conversion to alcohol compound by reduction or alkylation of carbonyl, reductive amination of carbonyl, oximation of carbonyl, acylation of amino, alkylation of amino, substitution and amination of active halogen by amine, alkylation of hydroxy, substitution and amination of hydroxy and the like can be mentioned. When a reactive substituent that causes non-objective reaction is present during the introduction of substituents and conversion of functional groups, a protecting group is introduced in advance as necessary into the reactive substituent by a means known *per se*, and the protecting group is removed by a means known *per se* after the objective reaction, whereby the starting compound (II) can be also produced.

In addition, a starting material, compound (II) can be produced by, for example, a method to be used compound (II') shown in the following formula.

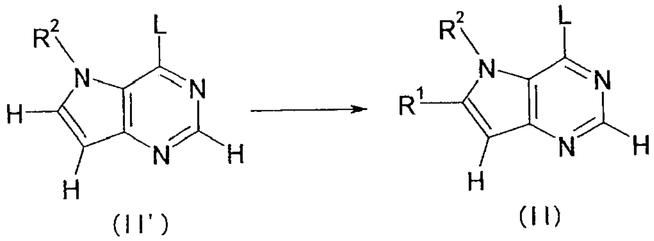

(II')  (II)

wherein each symbol is as defined above.

A step for producing compound (II) from compound (II') in this method is generally completed by withdrawing a proton from compound (II') using a base to give an anion, and reacting the anion with a cation having $R^1$. As the base, for example, n-butyllithium, s-butyllithium, t-butyllithium, lithium t-butoxide, lithium diisopropylamide and the like can be used. As a reagent for generating the cation, for example, p-toluenesulfonyl chloride, benzenesulfonyl bromide, p-toluenesulfonyl cyanide, S-(trifluoromethyl)dibenzothiophenium trifluoromethanesulfonate, N,N-dimethylformamide and the like can be used. As the reaction solvent, for example, halogenated hydrocarbons such as dichloromethane, chloroform, carbon tetrachloride, 1,2-dichloroethane and the like; ethers such as diethyl ether, tetrahydrofuran, dioxane, 1,2-dimethoxyethane and the like, a mixed solvent thereof and the like can be used. The aforementioned reaction can be carried out under cooling, preferably about not more than -20°C, and the reaction time is generally about 15 min to 50 hr, preferably about 30 min to 4 hr.

Thus-obtained compound (I) can be isolated and purified by a separation means known *per se*, such as concentration, concentration under reduced pressure, solvent extraction, crystallization, recrystallization, phase transfer, chromatography and the like.

When compound (I) is obtained as a free form, it can be converted to a desired salt by a method known *per se* or a modification thereof; conversely, when compounds (I) is obtained as a salt, it can be converted to a free form or other desired salt by a method known *per se* or a modification thereof.

When compound (I) has isomers such as optical isomer, stereoisomer, positional isomer, rotational isomer and the like, any isomers and mixtures are encompassed in the compound (I). For example, when compound (I) has an optical isomer, an optical isomer separated from a racemate is also encompassed in the compound (I). These isomers can be obtained as independent products by a synthesis means or a separation means (concentration, solvent extraction, column chromatography, recrystallization and the like) known *per se*.

The compounds (I) may be a crystal, and both a single crystal and crystal mixtures are encompassed in the compound (I). Crystals can be produced by crystallization according to crystallization methods known *per se*.

The compounds (I) may be a solvate (e.g., hydrate etc.) or a non-solvate, both of which are encompassed in the compound (I).

A compound labeled with an isotope (e.g., $^{2}H$, $^{3}H$, $^{14}C$, $^{35}S$, $^{125}I$ and the like) is also encompassed in the compound (I).

A prodrug of the compounds (I) or salts thereof (hereinafter referred to as compound (I)) means a compound which is converted to the compounds (I) with a reaction due to an enzyme, an gastric acid, etc. under the physiological condition in the living body, that is, a compound which is converted to the compounds (I) with oxidation, reduction, hydrolysis, etc. due to an enzyme; a compound which is converted to the compounds (I) by hydrolysis etc. due to gastric acid, etc. A prodrug for compounds (I) may be a compound obtained by subjecting amino in compounds (I) to an acylation, alkylation or phosphorylation (e.g., a compound obtained by subjecting amino in compounds (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting hydroxy in compounds (I) to an acylation, alkylation, phosphorylation or boration (e.g., a compound obtained by subjecting hydroxy in compounds (I) to an acetylation, palmitoylation, propanoylation, pivaloylation, succinylation, fumarylation, alanylation or dimethylaminomethylcarbonylation); a compound obtained by subjecting carboxyl in compounds (I) to an esterification or amidation (e.g., a compound obtained by subjecting carboxyl in compounds (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any one of these compounds can be produced from compounds (I) by a method known *per se*.

A prodrug for compounds (I) may also be one which is converted into compounds (I) under a physiological condition, such as those described in IYAKUHIN no KAIHATSU (Development of Pharmaceuticals), Vol. 7, Design of Molecules, p.163-198, Published by HIROKAWA SHOTEN (1990).

The compounds (I) of the present invention, or a salt thereof or a prodrug thereof (hereinafter referred to as the compound of the present invention) possess tyrosine kinase-inhibiting activity and can be used for the prophylaxis or treatment of tyrosine kinase-dependent diseases in mammals. Tyrosine kinase-dependent diseases include diseases characterized by increased cell proliferation due to abnormal tyrosine kinase enzyme activity.

Particularly, the compound of the present invention inhibits HER2 kinase and/or EGFR kinase and is therefore also useful as a therapeutic agent for suppressing the growth of HER2 and/or EGFR kinase-expressing cancer. Also, the compound of the present invention is useful as a preventive agent for preventing hormone-dependent cancer and the transition of hormone-dependent cancer to hormone-independent cancer. In addition, the compound of the present invention is useful as a pharmaceutical agent because it shows low toxicity (e.g., acute toxicity, chronic toxicity, genetic toxicity, reproductive toxicity, cardiotoxicity, drug interaction, carcinogenicity and the like), high water solubility, and is superior in stability, pharmacokinetics (absorption, distribution, metabolism, excretion and the like) and efficacy expression.

Accordingly, the compound of the present invention can be used as a safe agent for the prophylaxis or treatment of

diseases due to abnormal cell proliferation such as various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colorectal cancer (e.g., familial colorectal cancer, hereditary nonpolyposis colorectal cancer, gastrointestinal stromal tumor etc.), small intestinal cancer, colon cancer (e.g., gastrointestinal stromal tumor etc.), rectal cancer (e.g., gastrointestinal stromal tumor etc.), esophagus cancer, duodenal cancer, cancer of the tongue, cancer of pharynx (e.g., nasopharyngeal carcinoma, orocancer of pharynx, hypocancer of pharynx etc.), salivary gland cancer, brain tumor (e.g., pineal astrocytoma, pilocytic astrocytoma, diffuse astrocytoma, anaplastic astrocytoma etc.), schwannoma, non-small cell lung cancer, small cell lung cancer, liver cancer (e.g., primary liver cancer, extrahepatic bile duct cancer etc.), kidney cancer (e.g., renal cell carcinoma, transitional cell cancer of renal pelvis and ureter etc.), biliary tract cancer, endometrial cancer, endometrial carcinoma, cancer of the uterine cervix, ovarian cancer (e.g., ovarian epithelial, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), hemangioma, malignant lymphoma, malignant melanoma, thyroid cancer (e.g., medullary thyroid carcinoma etc.), parathyroid cancer, nasal cavity cancer, paranasal sinus cancer, bone tumor (e.g., osteosarcoma, Ewing's tumor, uterus sarcoma, soft tissue sarcoma etc.), vascular fibroma, retinoblastoma, penile cancer, solid cancer in childhood, Kaposi's sarcoma, Kaposi's sarcoma derived from AIDS, maxillary tumor, fibrous histiocytoma, leiomyosarcoma, rhabdomyosarcoma, leukemia (e.g., acute myeloid leukemia, acute lymphoblastic leukemia etc.) etc.), atherosclerosis, angiogenesis (e.g., angiogenesis associated with growth of solid cancer and sarcoma, angiogenesis associated with tumor metastasis, angiogenesis associated with diabetic retinopathy, etc.), viral diseases (HIV infection etc.) and the like.

Tyrosine kinase-dependent diseases further include cardiovascular diseases associated with abnormal tyrosine kinase enzyme activity. The compound of the present invention can therefore be used as an agent for prophylaxis or treatment of cardiovascular diseases such as restenosis.

The compound of the present invention is useful as an anticancer agent for the prophylaxis or treatment of cancer, especially breast cancer, ovarian cancer, colorectal cancer, gastric cancer, esophagus cancer, prostate cancer, lung cancer, pancreatic cancer and the like.

The compound of the present invention shows low toxicity and can be used as a pharmaceutical agent as it is, or as a pharmaceutical composition in admixture with a commonly known pharmaceutically acceptable carrier etc. in mammals (e.g., humans, horses, bovines, dogs, cats, rats, mice, rabbits, pigs, monkeys and the like).

In addition to the compound of the present invention, said pharmaceutical composition may contain other active ingredients, e.g., the following hormonal therapeutic agents, anticancer agents (e.g., chemotherapeutic agents, immunotherapeutic agents, or pharmaceutical agents inhibiting the action of cell growth factors or cell growth factor receptors, etc.), and the like.

As a pharmaceutical agent for mammals such as humans, the compound of the present invention can be generally administered orally in the form of, for example, tablets, capsules (including soft capsules and microcapsules), powders, granules and the like, or parenterally in the form of injections, suppositories, pellets and the like. Examples of the "parenteral administration route" include intravenous, intramuscular, subcutaneous, intra-tissue, intranasal, intradermal, instillation, intracerebral, intrarectal, intravaginal, intraperitoneal, intratumoral, administration to juxtaposition and the like of tumor, or directly to the lesion.

The dose of the compound of the present invention varies depending on the route of administration, symptoms, etc. For example, when it is administered orally as an anticancer agent to a patient (body weight 40 to 80 kg) with breast cancer or prostate cancer, its dose is, for example, 0.5 to 100 mg/kg body weight per day, preferably 1 to 50 mg/kg body weight per day, and more preferably 1 to 25 mg/kg body weight per day. This amount may be administered once or in 2 to 3 divided portions daily.

The compound of the present invention can be safely administered orally or parenterally (e.g., topical, rectal, intravenous administrations etc.) as a single agent, or a pharmaceutical composition containing a pharmacologically acceptable carrier according to a conventional method (e.g., a method described in the Japanese Pharmacopoeia etc.), such as tablet (including sugar-coated tablet, film-coated tablet), powder, granule, capsule, liquid, emulsion, suspension, injection, suppository, sustained release preparation, plaster and the like.

A combination of (1) administering an effective amount of a compound of the present invention and (2) 1 to 3 selected from the group consisting of (i) administering an effective amount of other anticancer agents, (ii) administering an effective amount of hormonal therapeutic agents and (iii) non-drug therapy can prevent and/or treat cancer more effectively. As the non-drug therapy, for example, surgery, radiotherapy, gene therapy, thermotherapy, cryotherapy, laser cauterization and the like are exemplified and two or more of these may be combined.

For example, the compound of the present invention can be used in combination with other hormonal therapeutic agents, anti-cancer agents (e.g., chemotherapeutic agent, immunotherapeutic agent (including vaccine), antibody, gene

therapeutic drug, pharmaceutical agent inhibiting action of cell growth factor and a receptor thereof, pharmaceutical agent inhibiting angiogenesis) and the like (hereinafter to be abbreviated as concomitant drug).

Although the compound of the present invention exhibits excellent anticancer action even when used as a simple agent, its effect can be enhanced by using it in combination with one or more of the concomitant drug(s) mentioned above (multi-agent co-administration).

As examples of said "hormonal therapeutic agents", there may be mentioned fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogens (e.g., tamoxifen citrate, toremifene citrate, and the like), ER down regulator (e.g., fulvestrant, and the like), human menopausal gonadotrophin, follicle stimulating hormone, pill preparations, mepitiostane, testrolactone, aminoglutethimide, LH-RH derivatives (LH-RH agonist (e.g., goserelin acetate, buserelin, leuprorelin, and the like), LH-RH antagonist), droloxifene, epitiostanol, ethinylestradiol sulfonate, aromatase inhibitors (e.g., fadrozole hydrochloride, anastrozole, retrozole, exemestane, vorozole, formestane, and the like), anti-androgens (e.g., flutamide, bicartamide, nilutamide, and the like), 5$\alpha$-reductase inhibitors (e.g., finasteride, dutasteride, epristeride, and the like), adrenocorticohormone drugs (e.g., dexamethasone, prednisolone, betamethasone, triamcinolone, and the like), androgen synthesis inhibitors (e.g., abiraterone, and the like), retinoid and drugs that retard retinoid metabolism (e.g., liarozole, and the like), etc. and LH-RH agonists (e.g., goserelin acetate, buserelin, leuprorelin) are preferable.

As examples of said "chemotherapeutic agents", there may be mentioned alkylating agents, antimetabolites, anticancer antibiotics, plant-derived anticancer agents, and the other chemotherapeutic agents.

As examples of the "alkylating agents", there may be mentioned nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and the like.

As examples of the "antimetabolites", there may be mentioned mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, and the like.

As examples of the "anticancer antibiotics", there may be mentioned actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, and the like.

As examples of the "plant-derived anticancer agents", there may be mentioned etoposide, etoposide phosphate, vinblastine sulfate, vincristine sulfate, vindesine sulfate, teniposide, paclitaxel (Taxol (trade mark)), docetaxel, vinorelbine, irinotecan, topotecan and the like.

As the "other chemotherapeutic agent", sobuzoxane and the like can be used.

As examples of said "immunotherapeutic agents (BRM)", there may be mentioned picibanil, krestin, sizofiran, lentinan, ubenimex, interferons, interleukins, macrophage colony-stimulating factor, granulocyte colony-stimulating factor, erythropoietin, lymphotoxin, *Corynebacterium parvum*, levamisole, polysaccharide K, procodazole, and the like. In addition, as the vaccine, BCG vaccine, PROVENGE, Onyvax-P, PROSTVAC-VF, GVAX, DCVax-Prostate, SAPOIMMUNE, VPM-4-001 and the like can be used.

As the "antibody", antibody to EpiCAM, antibody to PSCA, and antibody to PSMA can be used.

As examples of the "cell growth factor" in said "pharmaceutical agents inhibiting the action of cell growth factors or cell growth factor receptors", there may be mentioned any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin, TGF-$\alpha$, HB-EGF and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., acidic FGF, basic FGF, KGF (keratinocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGF$\beta$ (transforming growth factor $\beta$), HGF (hepatocyte growth factor), VEGF (vascular endothelial growth factor), and the like], and the like.

As examples of said "cell growth factor receptors", there may be mentioned any receptors capable of binding to the aforementioned cell growth factors, including EGF receptor, and a receptor belonging to the same family with that, HER2, HER3 and HER4, insulin receptor, IGF receptor, FGF receptor-1, FGF receptor-2 and the like.

As examples of said "pharmaceutical agents inhibiting the action of cell growth factor", there may be mentioned trastuzumab (Herceptin (trade mark) HER2 antibody), imatinib mesylate, ZD1839 or EGFR antibody (cetuximab (Erbitux (trade mark)) etc.), antibody against VEGF (e.g., bevacizumab (Avastin (trade mark))), VEGFR antibody, VEGFR inhibitor and EGFR inhibitor (gefitinib (Iressa (trade mark)), erlotinib (Tarceva (trade mark)) etc.).

In addition to the aforementioned drugs, L-asparaginase, aceglatone, procarbazine hydrochloride, protoporphyrin-cobalt complex salt, mercuric hematoporphyrin-sodium, topoisomerase I inhibitors (e.g., irinotecan, topotecan, and the like), topoisomerase II inhibitors (e.g., sobuzoxane, and the like), differentiation inducers (e.g., retinoid, vitamin D, and the like), angiogenesis inhibitors (e.g., thalidomide, SU11248, and the like), $\alpha$-blockers (e.g., tamsulosin hydrochloride, naftopidil, urapidil, alfuzosin, terazosin, prazosin, silodosin, and the like), serine/threonine kinase inhibitor, endothelin receptor antagonist (e.g., atrasentan, and the like), proteasome inhibitor (e.g., bortezomib, and the like), Hsp 90 inhibitor (e.g., 17-AAG, and the like), spironolactone, minoxidil, 11$\alpha$-hydroxyprogesterone, bone resorption inhibiting/ metastasis suppressing agent (e.g., zoledronic acid, alendronic acid, pamidronic acid, etidronic acid, ibandronic acid, clodronic acid) and the like can be used.

Of those mentioned above, as the concomitant drug, LH-RH agonist (e.g., goserelin acetate, buserelin, leuprorelin, and the like), HER2 antibody (trastuzumab (Herceptin (trade mark))), EGFR antibody (cetuximab (Erbitux) (trade mark) etc.), EGFR inhibitor (erlotinib (Tarceva) (trade mark), gefitinib (Iressa (trade mark)) etc.), VEGFR inhibitor or chemotherapeutic agent (paclitaxel (Taxol (trade mark) etc.) are preferable.

Particularly, trastuzumab (Herceptin (trade mark)), cetuximab (Erbitux (trade mark)), erlotinib (Tarceva (trade mark)), gefitinib (Iressa (trade mark)), paclitaxel (Taxol (trade mark)) and the like are preferable.

In combination of the compound of the present invention and the concomitant drug, the administration time of the compound of the present invention and the concomitant drug is not restricted, and the compound of the present invention and the concomitant drug can be administered to the administration subject simultaneously, or may be administered at different times. The dosage of the concomitant drug may be determined according to the dose clinically used, and can be appropriately selected depending on the administration subject, administration route, disease, combination and the like.

The administration mode of the compound of the present invention and the concomitant drug is not particularly restricted, and it is sufficient that the compound of the present invention and the concomitant drug are combined in administration. Examples of such administration mode include the following methods:

(1) The compound of the present invention and the concomitant drug are simultaneously produced to give a single preparation which is administered. (2) The compound of the present invention and the concomitant drug are separately produced to give two kinds of preparations which are administered simultaneously by the same administration route. (3) The compound of the present invention and the concomitant drug are separately produced to give two kinds of preparations which are administered by the same administration route at the different times. (4) The compound of the present invention and the concomitant drug are separately produced to give two kinds of preparations which are administered simultaneously by different administration routes. (5) The compound of the present invention and the concomitant drug are separately produced to give two kinds of preparations which are administered by different administration routes at different times (e.g., the compound of the present invention and the concomitant drug are administered in this order, or in the reverse order).

The present invention is explained in detail in the following by referring to Examples, Formulation Examples and Experimental Examples, which are not to be construed as limitative.

Example 1

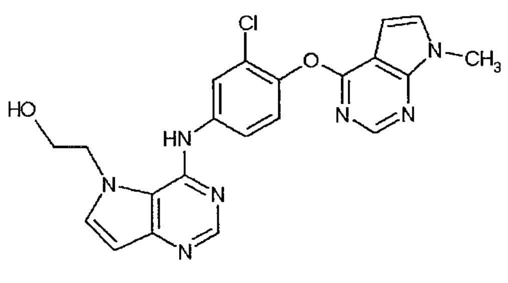

Production of 2-[4-({3-chloro-4-[(7-methyl-7H-pyrrolo[2,3-d]pyrimidin-4-yl)oxy]phenyl}amino)-5H-pyrrolo[3,2-d]pyrimidin-5-yl]ethanol

(i) Production of 3-chloro-4-[(7-methyl-7H-pyrrolo[2,3-d]pyrimidin-4-yl)oxy]aniline

4-Chloro-7H-pyrrolo[2,3-d]pyrimidine (1 g) was dissolved in N,N-dimethylformamide (7.2 mL), potassium carbonate (1.35 g) and methyl methanesulfonate (0.55 mL) were added, and the mixture was stirred at room temperature for 2 hr. The mixture was partitioned between ethyl acetate (80 mL) and water (80 mL), and the organic layer was dried over anhydrous magnesium sulfate and concentrated under reduced pressure. The residue was dissolved in N,N-dimethylformamide (10 mL), potassium carbonate (990 mg) and 4-amino-2-chlorophenol (857 mg) were added, and the mixture was stirred at 120°C for 16 hr. The mixture was partitioned between ethyl acetate (150 mL) and water (100 mL), and the organic layer was dried over anhydrous magnesium sulfate and concentrated under reduced pressure. The residue was separated and purified by silica gel column chromatography (hexane:ethyl acetate=90:10→20:80) to give the title compound (516 mg) as a pale-red powder.
$^1$H-NMR ($CDCl_3$) δ:3.75 (2H, br s), 3.88 (3H, s), 6.51 (1H, d, J= 3.0 Hz), 6.63 (1H, dd, J= 3.0 Hz, 9.0 Hz), 6.80 (1H, d, J= 3.0 Hz), 7.06 (1H, d, J= 2.0 Hz), 7.09 (1H, d, J= 3.0 Hz), 8.46 (1H, s).

(ii) Production of 2-[4-({3-chloro-4-[(7-methyl-7H-pyrrolo[2,3-d]pyrimidin-4-yl)oxy]phenyl}amino)-5H-pyrrolo[3,2-d]pyrimidin-5-yl]ethanol

3-Chloro-4-[(7-methyl-7H-pyrrolo[2,3-d]pyrimidin-4-yl)oxy]aniline (137 mg), 2-(4-chloro-5H-pyrrolo[3,2-d]pyrimidin-5-yl)ethyl benzoate (151 mg) were dissolved in 1-methyl-2-pyrrolidone (1 mL), and the solution was stirred at 140°C for 2 hr. The mixture was partitioned between ethyl acetate (80 mL) and aqueous sodium hydrogen carbonate (50 mL), and the organic layer was dried over anhydrous magnesium sulfate and concentrated under reduced pressure. The residue was separated and purified by silica gel column chromatography (hexane:ethyl acetate=90:10→0:100). The obtained oil residue was dissolved in methanol (6 mL), 1N aqueous sodium hydroxide solution (0.5 mL) was added, and the mixture was stirred at room temperature for 2 hr. 1N Hydrochloric acid (0.5 mL) was added, and the mixture was diluted with ethyl acetate (80 mL) and partitioned with saturated brine (50 mL). The organic layer was dried over anhydrous magnesium sulfate and concentrated under reduced pressure. The residue was separated and purified by silica gel column chromatography (ethyl acetate:methanol=100:0→90:10) to give the title compound (113 mg) as white crystals.
$^1$H-NMR (DMSO-$d_6$)δ: 3.84 (3H, s), 3.89 (2H, t, J= 4.5 Hz), 4.55 (2H, t, J= 4.5 Hz), 6.31 (1H, br s), 6.52 (1H, d, J= 3.0 Hz), 6.59 (1H, d, J= 3.6 Hz), 7.39 (1H, d, J= 9.0 Hz), 7.55 (1H, d, J= 3.6 Hz), 7.60 (1H, dd, J= 2.4 Hz, 9.0 Hz), 7.67 (1H, d, J= 3.0 Hz), 7.94 (1H, d, J= 2.4 Hz), 8.34 (2H, s), 9.88 (1H, br s).

Example 2

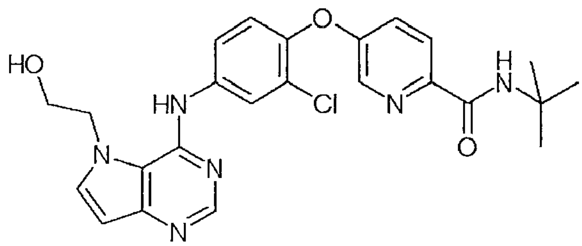

Production of N-(tert-butyl)-5-(2-chloro-4-{[5-(2-hydroxyethyl)-5H-pyrrolo[3,2-d]pyrimidin-4-yl]amino}phenoxy) pyridine-2-carboxamide

(i) Production of N-(tert-butyl)-5-hydroxypyridine-2-carboxamide

5-Hydroxypyridine-2-carboxylic acid (1.50 g) was dissolved in a mixed solvent of tetrahydrofuran (7.5 mL)/N, N-dimethylformamide (7.5 mL), tert-butylamine (1.7 mL), 1-hydroxybenzotriazole (2.20 g), triethylamine (4.5 mL) and 1-ethyl-3-(3-dimethylaminopropyl)carbodiimide hydrochloride (3.10 g) were successively added, and the mixture was stirred at room temperature for 24 hr. Water was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl

acetate=33:67→0:100→ethyl acetate:methanol=95:5) to give the title compound (1.05 g) as an orange oil.
$^1$H-NMR ($CDCl_3$) δ: 1.49 (9H, s), 7.23 (1H, dd, J = 2.6 Hz, 8.6 Hz), 7.90-8.01 (2H, m), 8.17 (1H, d, J = 2.6 Hz), 8.57 (1H, br s).

(ii) Production of N-(tert-butyl)-5-(2-chloro-4-nitrophenoxy)pyridine-2-carboxamide

A mixture of 2-chloro-1-fluoro-4-nitrobenzene (401 mg) and N-(tert-butyl)-5-hydroxypyridine-2-carboxamide (501 mg) was dissolved in N,N-dimethylformamide (4 mL), potassium carbonate (484 mg) was added, and the mixture was stirred at room temperature for 24 hr. Water was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane: ethyl acetate=95:5→67:33) to give the title compound (810 mg) as a pale-yellow oil.
$^1$H-NMR ($CDCl_3$) δ: 1.50 (9H, s), 7.02 (1H, d, J = 9.0 Hz), 7.43 (1H, dd, J = 2.8 Hz, 8.5 Hz), 7.83 (1H, br s), 8.13 (1H, dd, J = 2.8 Hz, 9.0 Hz), 8.24 (1H, d, J = 8.5 Hz), 8.31 (1H, d, J = 2.8 Hz), 8.42 (1H, d, J = 2.8 Hz).

(iii) Production of 5-(4-amino-2-chlorophenoxy)-N-(tert-butyl)pyridine-2-carboxamide

N-(tert-Butyl)-5-(2-chloro-4-nitrophenoxy)pyridine-2-carboxamide (807 mg) was dissolved in a mixed solvent of ethanol (22.5 mL)/water (2.5 mL). Reduced iron (653 mg) and calcium chloride (134 mg) were added to the mixture, and the mixture was stirred with heating under reflux for 14 hr. The reaction mixture was filtered through celite, and the filtrate was concentrated under reduced pressure. The residue was suspended in ethyl acetate, and the suspension was washed successively with water and saturated brine, dried over anhydrous magnesium sulfate, and concentrated under reduced pressure. The residue was separated and purified by silica gel column chromatography (hexane:ethyl acetate=90:10→33:67) to give the title compound (681 mg) as an orange powder.
$^1$H-NMR ($CDCl_3$) δ: 1.48 (9H, s), 3.74 (2H, s), 6. 60 (1H, dd, J = 2.7 Hz, 8.7 Hz), 6.79 (1H, d, J = 2.7 Hz), 6.94 (1H, d, J = 8.7 Hz), 7.16 (1H, dd, J = 2.7 Hz, 8.7 Hz), 7.82 (1H, br s), 8.08 (1H, d, J = 2.7 Hz), 8.19 (1H, d, J = 2.7 Hz).

(iv) Production of N-(tert-butyl)-5-(2-chloro-4-{[5-(2-hydroxyethyl)-5H-pyrrolo[3,2-d]pyrimidin-4-yl]amino}phenoxy)pyridine-2-carboxamide

A mixture of 2-(4-chloro-5H-pyrrolo[3,2-d]pyrimidin-5-yl)ethyl benzoate (100 mg) and 5-(4-amino-2-chlorophenoxy)-N-(tert-butyl)pyridine-2-carboxamide (116 mg) was dissolved in isopropyl alcohol (3 mL), pyridine hydrochloride (5 mg) was added, and the mixture was stirred at 70°C for 13 hr. The reaction mixture was cooled to room temperature, 1N aqueous sodium hydroxide solution (1.5 mL) was added and the mixture was stirred for 3 hr. Saturated aqueous ammonium chloride solution was added to the reaction mixture, and the mixture was extracted with ethyl acetate. The organic layer was dried over anhydrous magnesium sulfate and concentrated under reduced pressure. The residue was separated and purified by silica gel column chromatography (hexane:ethyl acetate=33:67→0:100), and crystallized from hexane/isopropyl alcohol to give the title compound (107 mg) as white crystals.
$^1$H-NMR ($CDCl_3$) δ: 1.48 (9H, s), 4.17 (2H, t, J = 4. 5 Hz), 4.41 (2H, t, J = 4.5 Hz), 6.20 (1H, d, J = 3.0 Hz), 7.02 (1H, d, J = 3.0 Hz), 7.12 (1H, d, J = 9.0 Hz), 7.21 (1H, dd, J = 2.8 Hz, 8.5 Hz), 7.56 (1H, dd, J = 2.5 Hz, 9.0 Hz), 7.80-7.90 (2H, m), 8.08 (1H, d, J = 8.5 Hz), 8.24-8.31 (2H, m), 9.67 (1H, s).
Example 3
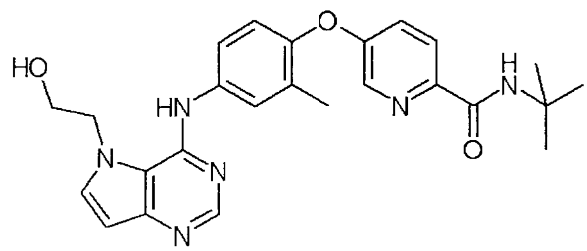

Production of N-(tert-butyl)-5-(4-{[5-(2-hydroxyethyl)-5H-pyrrolo[3,2-d]pyrimidin-4-yl]amino}-2-methylphenoxy)pyridine-2-carboxamide

(i) Production of N-(tert-butyl)-5-(2-methyl-4-nitrophenoxy)pyridine-2-carboxamide

The title compound (691 mg) was obtained as a pale-yellow oil by reaction in the same manner as in Example 2 (ii) and using 2-fluoro-5-nitrotoluene (350 mg), N-(tert-butyl)-5-hydroxypyridine-2-carboxamide (501 mg), potassium carbonate (495 mg) and N,N-dimethylformamide (4 mL).
$^1$H-NMR ($CDCl_3$) δ: 1.50 (9H, s), 2.39 (3H, s), 6.88 (1H, d, J = 9.0 Hz), 7.38 (1H, dd, J = 2.8 Hz, 8.5 Hz), 7.83 (1H, br s), 8.05 (1H, dd, J = 2.8 Hz, 9.0 Hz), 8.17-8.24 (2H, m), 8.27 (1H, d, J = 2.8 Hz).

(ii) Production of 5-(4-amino-2-methylphenoxy)-N-(tert-butyl)pyridine-2-carboxamide

The title compound (588 mg) was obtained as an orange powder by reaction in the same manner as in Example 2 (iii) and using N-(tert-butyl)-5-(2-methyl-4-nitrophenoxy)pyridine-2-carboxamide (689 mg), reduced iron (599 mg), calcium chloride (117 mg) and ethanol (18 mL)/water (2 mL).
$^1$H-NMR ($CDCl_3$) δ: 1.48 (9H, s), 2.06 (3H, s), 3.62 (2H, br s), 6.54 (1H, dd, J = 3.0 Hz, 8.4 Hz), 6.60 (1H, d, J = 3.0 Hz), 6.80 (1H, J = 8.4 Hz), 7.13 (1H, dd, J = 3.0 Hz, 8.7 Hz), 7.81 (1H, br s), 8.06 (1H, d, J = 8.7 Hz), 8.17 (1H, d, J = 3.0 Hz).

(iii) Production of N-(tert-butyl)-5-(4-{[5-(2-hydroxyethyl)-5H-pyrrolo[3,2-d]pyrimidin-4-yl]amino}-2-methylphenoxy)pyridine-2-carboxamide

The title compound (113 mg) was obtained as white crystals by reaction in the same manner as in Example 2 (iv) and using 2-(4-chloro-5H-pyrrolo[3,2-d]pyrimidin-5-yl)ethyl benzoate (101 mg), 5-(4-amino-2-methylphenoxy)-N-(tert-butyl)pyridine-2-carboxamide (108 mg), pyridine hydrochloride (5 mg), isopropyl alcohol (3 mL) and 1N aqueous sodium hydroxide solution (1.5 mL).
$^1$H-NMR ($CDCl_3$) δ: 1.48 (9H, s), 2.20 (3H, s), 4.15 (2H, t, J = 4.0 Hz), 4.39 (2H, t, J = 4.0 Hz), 6.18 (1H, d, J = 3.0 Hz), 6.98-7.02 (2H, m), 7.20 (1H, dd, J = 2.7 Hz, 8.7 Hz), 7.44-7.54 (2H, m), 7.84 (1H, s), 8.07 (1H, d, J = 8.7 Hz), 8.21-8.30 (2H, m), 9.40 (1H, s).
Example 4
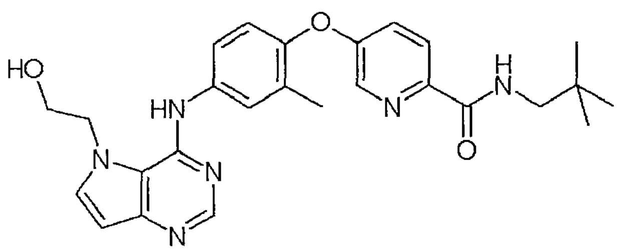

Production of N-(2,2-dimethylpropyl)-5-(4-{[5-(2-hydroxyethyl)-5H-pyrrolo[3,2-d]pyrimidin-4-yl]amino}-2-methylphenoxy)pyridine-2-carboxamide

(i) Production of N-(2,2-dimethylpropyl)-5-hydroxypyridine-2-carboxamide

The title compound (2.17 g) was obtained as a pale-yellow oil by reaction in the same manner as in Example 2 (i) and using 5-hydroxypyridine-2-carboxylic acid (1.50 g), neopentylamine (1.9 mL), tetrahydrofuran (7.5 mL)/N,N-dimethylformamide (7.5 mL), 1-hydroxybenzotriazole (2.20 g), triethylamine (4.5 mL) and 1-ethyl-3-(3-dimethylaminopropyl)carbodiimide hydrochloride (3.33 g).
$^1$H-NMR ($CDCl_3$) δ: 0.97 (9H, s), 3.22 (2H, d, J = 6.6 Hz), 7. 15 (1H, dd, J = 2.8 Hz, 8.7 Hz), 7.92 (1H, d, J = 8.7 Hz), 8.01 (1H, t, J = 6.6 Hz), 8.10 (1H, d, J = 2.8 Hz).

(ii) Production of N-(2,2-dimethylpropyl)-5-(2-methyl-4-nitrophenoxy)pyridine-2-carboxamide

The title compound (1.02 g) was obtained as a pale-yellow solid by reaction in the same manner as in Example 2 (ii) and using 2-fluoro-5-nitrotoluene (641 mg), N-(2,2-dimethylpropyl)-5-hydroxypyridine-2-carboxamide (1.05 g), potassium carbonate (871 mg) and N,N-dimethylformamide (8 mL).
$^1$H-NMR ($CDCl_3$) δ: 1.00 (9H, s), 2.40 (3H, s), 3.29 (2H, d, J = 6.8 Hz), 6.92 (1H, d, J = 9.0 Hz), 7.40 (1H, dd, J = 2.8

Hz, 8.7 Hz), 8.02 (1H, br s), 8.08 (1H, dd, J = 2.8 Hz, 9.0 Hz), 8.21 (1H, d, J = 2.8 Hz), 8.25 (1H, d, J = 8.7 Hz), 8.32 (1H, d, J = 2.8 Hz).

(iii) Production of 5-(4-amino-2-methylphenoxy)-N-(2,2-dimethylpropyl)pyridine-2-carboxamide

The title compound (878 mg) was obtained as a brown oil by reaction in the same manner as in Example 2 (iii) and using N-(2,2-dimethylpropyl)-5-(2-methyl-4-nitrophenoxy)pyridine-2-carboxamide (1.01 g), reduced iron (858 mg), calcium chloride (175 mg) and ethanol (27 mL)/water (3 mL).
$^1$H-NMR ($CDCl_3$) δ:0.98 (9H, s), 2.08 (3H, s), 3.26 (2H, d, J = 6.6 Hz), 3.63 (2H, s), 6.55 (1H, dd, J = 2.8 Hz, 8.4 Hz), 6.60 (1H, d, J = 2.8 Hz), 6.81 (1H, d, J = 8.4 Hz), 7.14 (1H, dd, J = 2.8 Hz, 8.7 Hz), 8.01 (1H, br s), 8.09 (1H, d, J = 8.7 Hz), 8.21 (1H, d, J = 2.8 Hz).

(iv) Production of N-(2,2-dimethylpropyl)-5-(4-{[5-(2-hydroxyethyl)-5H-pyrrolo[3,2-d]pyrimidin-4-yl]amino}-2-methylphenoxy)pyridine-2-carboxamide

The title compound (106 mg) was obtained as white crystals by reaction in the same manner as in Example 2 (iV) and using 2-(4-chloro-5H-pyrrolo[3,2-d]pyrimidin-5-yl)ethyl benzoate (99.4 mg), 5-(4-amino-2-methylphenoxy)-N-(2,2-dimethylpropyl)pyridine-2-carboxamide (114 mg), pyridine hydrochloride (5 mg), isopropyl alcohol (3 mL) and 1N aqueous sodium hydroxide solution (1.5 mL).
$^1$H-NMR ($CDCl_3$) δ:0.99 (9H, s), 2.21 (3H, s), 3.26 (2H, d, J = 6.4 Hz), 4.15 (2H, t, J = 4.4 Hz), 4.33-4.44 (2H, m), 6.18 (1H, d, J = 3.0 Hz), 6.93-7.04 (2H, m), 7.21 (1H, dd, J = 3.0 Hz, 8.7 Hz), 7.44-7.55 (2H, m), 8.03 (1H, t, J = 6.4 Hz), 8.10 (1H, d, J = 8.7 Hz), 8.26 (1H, s), 8.29 (1H, d, J = 3.0 Hz), 9.42 (1H, br s).
Example 5
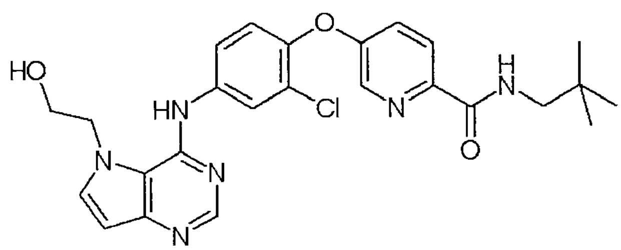

Production of 5-(2-chloro-4-{[5-(2-hydroxyethyl)-5H-pyrrolo[3,2-d]pyrimidin-4-yl]amino}phenoxy)-N-(2,2-dimethylpropyl)pyridine-2-carboxamide

(i) Production of 5-(2-chloro-4-nitrophenoxy)-N-(2,2-dimethylpropyl)pyridine-2-carboxamide

The title compound (1.19 g) was obtained as a pale-yellow oil by reaction in the same manner as in Example 2 (ii) and using 2-chloro-1-fluoro-4-nitrobenzene (724 mg), N-(2,2-dimethylpropyl)-5-hydroxypyridine-2-carboxamide (1.04 g), potassium carbonate (862 mg) and N,N-dimethylformamide (8 mL). $^1$H-NMR ($CDCl_3$) δ: 1.00 (9H, s), 3.29 (2H, d, J = 6.8 Hz), 7.07 (1H, d, J = 9.0 Hz), 7.45 (1H, dd, J = 2.8 Hz, 8.5 Hz), 8.02 (1H, br s), 8.16 (1H, dd, J = 2.8 Hz, 9.0 Hz), 8.28 (1H, d, J = 8.5 Hz), 8.36 (1H, d, J = 2.8 Hz), 8.43 (1H, d, J = 2.8 Hz).

(ii) Production of 5-(4-amino-2-chlorophenoxy)-N-(2,2-dimethylpropyl)pyridine-2-carboxamide

The title compound (1.04 g) was obtained as a brown oil by reaction in the same manner as in Example 2 (iii) and using 5-(2-chloro-4-nitrophenoxy)-N-(2,2-dimethylpropyl)pyridine-2-carboxamide (1.18 g), reduced iron (921 mg), calcium chloride (192 mg) and ethanol (36 mL)/water (4 mL).
$^1$H-NMR ($CDCl_3$) δ: 0.98 (9H, s), 3.26 (2H, d, J = 6.6 Hz), 3.76 (2H, s), 6.61 (1H, dd, J = 2.7 Hz, 8.7 Hz), 6.80 (1H, d, J = 2.7 Hz), 6.95 (1H, d, J = 8.7 Hz), 7.17 (1H, dd, J = 2.7 Hz, 8.7 Hz), 8.02 (1H, br s), 8.12 (1H, d, J = 8.7 Hz), 8.23 (1H, d, J = 2.7 Hz).

(iii) Production of 5-(2-chloro-4-{[5-(2-hydroxyethyl)-5H-pyrrolo[3,2-d]pyrimidin-4-yl]amino}phenoxy)-N-(2,2-dimethylpropyl)pyridine-2-carboxamide

The title compound (70.3 mg) was obtained as white crystals by reaction in the same manner as in Example 2 (iv) and using 2-(4-chloro-5H-pyrrolo[3,2-d]pyrimidin-5-yl)ethyl benzoate (101 mg), 5-(4-amino-2-chlorophenoxy)-N-(2,2-dimethylpropyl)pyridine-2-carboxamide (123 mg), pyridine hydrochloride (5 mg), isopropyl alcohol (3 mL) and 1N aqueous sodium hydroxide solution (1.5 mL).
$^1$H-NMR ($CDCl_3$) δ: 0.99 (9H, s), 3.26 (2H, d, J = 6.5 Hz), 4.17 (2H, t, J = 4.3 Hz), 4.41 (2H, t, J = 4.3 Hz), 6.20 (1H, d, J = 3.0 Hz), 7.02 (1H, d, J = 3.0 Hz), 7.13 (1H, d, J = 8.7 Hz), 7.22 (1H, dd, J = 2.7 Hz, 8.7 Hz), 7.57 (1H, dd, J = 2.7 Hz, 8.7 Hz), 7.85 (1H, d, J = 2.7 Hz), 8.05 (1H, t, J = 6.5 Hz), 8.11 (1H, d, J = 8.7 Hz), 8.27 (1H, s), 8.31 (1H, d, J = 2.7 Hz), 9.71 (1H, s).
Example 6
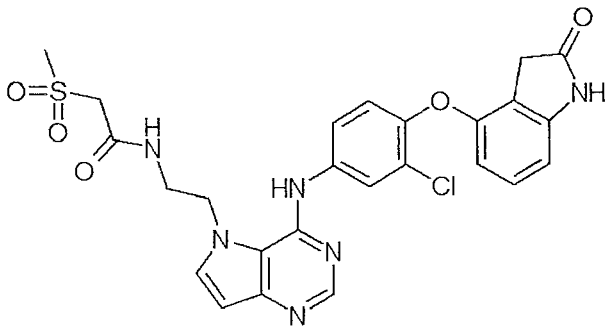

Production of N-{2-[4-({3-chloro-4-[(2-oxo-2,3-dihydro-1H-indol-4-yl)oxy]phenyl}amino)-5H-pyrrolo[3,2-d]pyrimidin-5-yl]ethyl}-2-(methylsulfonyl)acetamide

(i) Production of 4-(2-chloro-4-nitrophenoxy)-1,3-dihydro-2H-indol-2-one

The title compound (1.08 g) was obtained as a pale-orange solid by reaction in the same manner as in Example 2 (ii) and using 4-hydroxy-1,3-dihydro-2H-indol-2-one (1.80 g), 2-chloro-1-fluoro-4-nitrobenzene (2.53 g), potassium carbonate (1.84 g) and N,N-dimethylformamide (30 mL).
$^1$H-NMR ($CDCl_3$) δ: 3.42 (2H, s), 6.65 (1H, d, J = 8.3 Hz), 6.78 (1H, d, J = 8.0 Hz), 6.96 (1H, d, J = 9.1 Hz), 7.20-7.34 (1H, m), 7.88 (1H, br s), 8.09 (1H, dd, J = 2.5 Hz, 9.1 Hz), 8.40 (1H, d, J = 2.5 Hz).

(ii) Production of 4-(4-amino-2-chlorophenoxy)-1,3-dihydro-2H-indol-2-one

4-(2-Chloro-4-nitrophenoxy)-1,3-dihydro-2H-indol-2-one (502 mg) was dissolved in a mixed solvent of methanol (15 mL)/tetrahydrofuran (15 mL), 5% platinum/activated carbon (147 mg) was added, and the mixture was stirred under hydrogen atmosphere for 1 hr. The reaction mixture was filtered through celite, the filtrate was concentrated under reduced pressure, and the precipitate was collected by filtration to give the title compound (434 mg) as a white powder.
$^1$H-NMR ($CDCl_3$) δ: 3.46 (2H, s), 3.69 (2H, br s), 6.37 (1H, d, J = 8.0 Hz), 6.52-6.60 (2H, m), 6.77 (1H, d, J = 2.5 Hz), 6.91 (1H, d, J = 8.5 Hz), 7.10 (1H, t, J = 8.0 Hz), 7.66 (1H, br s).

(iii) Production of tert-butyl {2-[4-({3-chloro-4-[(2-oxo-2,3-dihydro-1H-indol-4-yl)oxy]phenyl}amino)-5H-pyrrolo[3,2-d]pyrimidin-5-yl]ethyl}carbamate

A mixture of tert-butyl [2-(4-chloro-5H-pyrrolo[3,2-d]pyrimidin-5-yl)ethyl]carbamate (398 mg) and 4-(4-amino-2-chlorophenoxy)-1,3-dihydro-2H-indol-2-one (410 mg) was dissolved in a mixed solvent of isopropyl alcohol (8 mL)/1-methyl-2-pyrrolidone (1 mL), and the solution was stirred at 80°C for 8.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (710 mg) as a white powder.
$^1$H-NMR ($CDCl_3$) δ: 1.50 (9H, s), 3.43-3.55 (2H, m), 3.50 (2H, s), 4.42-4.53 (2H, m), 5.12 (1H, t, J = 5.6 Hz), 6.50 (1H,

d, J = 8.0 Hz), 6.57-6.65 (2H, m), 7.05 (1H, d, J = 8.8 Hz), 7.13 (1H, t, J = 8.0 Hz), 7.18 (1H, d, J = 3.3 Hz), 7.73 (1H, s), 7.88 (1H, dd, J = 2.5 Hz, 8.8 Hz), 8.00 (1H, d, J = 2.5 Hz), 8.50 (1H, s), 8.60 (1H, br s).

(iv) Production of 4-(4-{[5-(2-aminoethyl)-5H-pyrrolo[3,2-d]pyrimidin-4-yl]amino}-2-chlorophenoxy)-1,3-dihydro-2H-indol-2-one dihydrochloride

tert-Butyl {2-[4-({3-chloro-4-[(2-oxo-2,3-dihydro-1H-indol-4-yl)oxy]phenyl}amino)-5H-pyrrolo[3,2-d]pyrimidin-5-yl]ethyl}carbamate (705 mg) was dissolved in ethanol (6 mL), 6N hydrochloric acid (1.1 mL) was added, and the mixture was stirred at 50°C for 13 hr. The reaction mixture was concentrated under reduced pressure, and the residue was dissolved in ethanol. The solution was concentrated under reduced pressure again, and the precipitate was collected by filtration to give the title compound (515 mg) as a white powder.
$^1$H-NMR (DMSO-$d_6$) δ: 3.20-3.34 (2H, m), 3.36 (2H, s), 5.01 (2H, t, J = 5.9 Hz), 6.41 (1H, d, J = 8.3 Hz), 6.66 (1H, d, J = 7.7 Hz), 6.74 (1H, d, J = 3.0 Hz), 7.14-7.27 (2H, m), 7.58 (1H, dd, J = 2.3 Hz, 8.9 Hz), 7.87 (1H, d, J = 2.3 Hz), 8.05 (1H, d, J = 3.0 Hz), 8.29 (3H, br s), 8.72 (1H, s), 10.04 (1H, br s), 10.58 (1H, s).

(v) Production of N-{2-[4-({3-chloro-4-[(2-oxo-2,3-dihydro-1H-indol-4-yl)oxy]phenyl}amino)-5H-pyrrolo[3,2-d]pyrimidin-5-yl]ethyl}-2-(methylsulfonyl)acetamide

A mixture of 4-(4-{[5-(2-aminoethyl)-5H-pyrrolo[3,2-d]pyrimidin-4-yl]amino}-2-chlorophenoxy)-1,3-dihydro-2H-indol-2-one dihydrochloride (149 mg) and methylsulfonylacetic acid (74 mg) was dissolved in a mixed solvent of tetrahydrofuran (0.8 mL)/N,N-dimethylformamide (0.8 mL), triethylamine (0.4 mL), 1-hydroxybenzotriazole (89.7 mg) and 1-ethyl-3-(3-dimethylaminopropyl)carbodiimide hydrochloride (126 mg) were added, and the mixture was stirred at room temperature for 5 hr. Water was added to the reaction mixture, and the mixture was extracted with ethyl acetate. The organic layer was dried over anhydrous magnesium sulfate and concentrated under reduced pressure. The residue was separated and purified by silica gel column chromatography (hexane:ethyl acetate=20:80→0:100→ethyl acetate:methanol=85:15) to give the title compound (136 mg) as a pale-orange powder.
$^1$H-NMR (DMSO-$d_6$) δ: 3.09 (3H, s), 3.38 (2H, s), 3.41-3.53 (2H, m), 4.04 (2H, s), 4.56 (2H, t, J = 6.6 Hz), 6.33 (1H, d, J = 8.3 Hz), 6.49 (1H, d, J = 3.0 Hz), 6.60 (1H, d, J = 7.6 Hz), 7.10-7.22 (2H, m), 7.62 (1H, d, J = 3.0 Hz), 7.69 (1H, dd, J = 2.2 Hz, 8.9 Hz), 7.94 (1H, d, J = 2.2 Hz), 8.33 (1H, s), 8.58-8.74 (2H, m), 10.51 (1H, br s).

Example 7

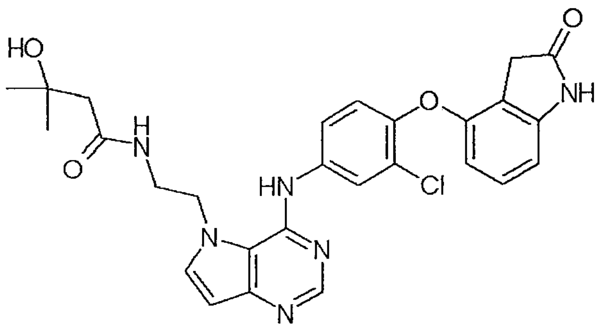

Production of N-{2-[4-({3-chloro-4-[(2-oxo-2,3-dihydro-1H-indol-4-yl)oxy]phenyl}amino)-5H-pyrrolo[3,2-d]pyrimidin-5-yl]ethyl}-3-hydroxy-3-methylbutanamide

The title compound (89.5 mg) was obtained as white crystals by reaction in the same manner as in Example 6 (v) and using 4-(4-{[5-(2-aminoethyl)-5H-pyrrolo[3,2-d]pyrimidin-4-yl]amino}-2-chlorophenoxy)-1,3-dihydro-2H-indol-2-one dihydrochloride (150 mg), 3-hydroxy-3-methylbutanoic acid (59.5 mg), triethylamine (0.4 mL), 1-hydroxybenzotriazole (87.4 mg), 1-ethyl-3-(3-dimethylaminopropyl)carbodiimide hydrochloride (123 mg) and tetrahydrofuran (0.8 mL)/N,N-dimethylformamide (0.8 mL).
$^1$H-NMR (DMSO-$d_6$) δ: 1.13 (6H, s), 2.20 (2H, s), 3.34-3.47 (2H, m), 3.37 (2H, s), 4.51 (2H, t, J = 6.6 Hz), 4.65 (1H, s), 6.33 (1H, d, J = 8.3 Hz), 6.48 (1H, d, J = 3.0 Hz), 6.60 (1H, d, J = 7.6 Hz), 7.09-7.21 (2H, m), 7.63 (1H, d, J = 3.0 Hz), 7.77 (1H, dd, J = 2.5 Hz, 8.9 Hz), 8.01 (1H, d, J = 2.5 Hz), 8.24 (1H, t, J = 5.9 Hz), 8.32 (1H, s), 8.84 (1H, s), 10.51 (1H, s).

Example 8

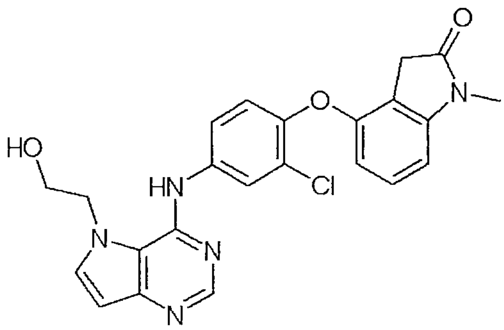

Production of 4-(2-chloro-4-{[5-(2-hydroxyethyl)-5H-pyrrolo[3,2-d]pyrimidin-4-yl]amino}phenoxy)-1-methyl-1,3-dihydro-2H-indol-2-one

(i) Production of 4-methoxy-1-methyl-1,3-dihydro-2H-indol-2-one

4-Methoxy-1,3-dihydro-2H-indol-2-one (801 mg) was dissolved in tetrahydrofuran (40 mL), iodomethane (0.46 mL), tetrabutylammonium iodide (101 mg) and finely triturated potassium hydroxide (420 mg) were added, and the mixture was stirred at room temperature for 21 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=95:5→33:67) to give the title compound (458 mg) as a pale-yellow powder.
$^1$H-NMR ($CDCl_3$) δ: 3.20 (3H, s), 3.45 (2H, s), 3.86 (3H, s), 6.50 (1H, d, J = 7.7 Hz), 6.62 (1H, d, J = 8.3 Hz), 7.20-7.30 (1H, m).

(ii) Production of 4-hydroxy-1-methyl-1,3-dihydro-2H-indol-2-one

A mixture of 4-methoxy-1-methyl-1,3-dihydro-2H-indol-2-one (600 mg) and 48% hydrobromic acid (6 mL) was stirred with heating under reflux for 60 hr. Water was added to the reaction mixture, and the mixture was extracted with a mixed solvent of ethyl acetate/tetrahydrofuran. The organic layer was washed successively with water and saturated brine, dried over anhydrous magnesium sulfate, and concentrated under reduced pressure. The precipitate was collected by filtration to give the title compound (515 mg) as an orange powder.
$^1$H-NMR ($CDCl_3$) δ: 3.20 (3H, s), 3.48 (2H, s), 5.21 (1H, s), 6.46 (1H, d, J = 8.0 Hz), 6.53 (1H, d, J = 8.0 Hz), 7.17 (1H, t, J = 8.0 Hz).

(iii) Production of 4-(2-chloro-4-nitrophenoxy)-1-methyl-1,3-dihydro-2H-indol-2-one

The title compound (97.0 mg) was obtained as white crystals by reaction in the same manner as in Example 2 (ii) and using 4-hydroxy-1-methyl-1,3-dihydro-2H-indol-2-one (502 mg), 2-chloro-1-fluoro-4-nitrobenzene (812 mg), potassium carbonate (502 mg) and N,N-dimethylformamide (8 mL).
$^1$H-NMR ($CDCl_3$) δ: 3.25 (3H, s), 3.40 (2H, s), 6.68 (1H, d, J = 8.0 Hz), 6.74 (1H, d, J = 8.0 Hz), 6.93 (1H, d, J = 9.1 Hz), 7.34 (1H, t, J = 8.0 Hz), 8.07 (1H, dd, J = 2.5 Hz, 9.1 Hz), 8.40 (1H, d, J = 2.5 Hz).

(iv) Production of 4-(4-amino-2-chlorophenoxy)-1-methyl-1,3-dihydro-2H-indol-2-one

The title compound (56.4 mg) was obtained as a pale-brown powder by reaction in the same manner as in Example 6 (ii) and using 4-(2-chloro-4-nitrophenoxy)-1-methyl-1,3-dihydro-2H-indol-2-one (92.5 mg), 5% platinum/activated carbon (16.7 mg) and methanol (5 mL)/tetrahydrofuran (3 mL).
$^1$H-NMR ($CDCl_3$) δ: 3.21 (3H, s), 3.44 (2H, s), 3.68 (2H, br s), 6.42 (1H, d, J = 8.5 Hz), 6.50-6.61 (2H, m), 6.77 (1H, d, J = 2.6 Hz), 6.90 (1H, d, J = 8.5 Hz), 7.17 (1H, t, J = 8.5 Hz).

(v) Production of 4-(2-chloro-4-{[5-(2-hydroxyethyl)-5H-pyrrolo[3,2-d]pyrimidin-4-yl]amino}phenoxy)-1-methyl-1,3-dihydro-2H-indol-2-one

A mixture of 2-(4-chloro-5H-pyrrolo[3,2-d]pyrimidin-5-yl)ethyl benzoate (55.5 mg) and 4-(4-amino-2-chlorophenoxy)-1-methyl-1,3-dihydro-2H-indol-2-one (55.4 mg) was dissolved in isopropyl alcohol (2 mL), pyridine hydrochloride (5 mg) was added, and the mixture was stirred at 70°C for 24 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=67:33→0:100) to give 2-[4-({3-chloro-4-[(1-methyl-2-oxo-2,3-dihydro-1H-indol-4-yl)oxy]phenyl}amino)-5H-pyrrolo[3,2-d]pyrimidin-5-yl]ethyl benzoate. The obtained compound was dissolved in a mixed solvent of tetrahydrofuran (1.2 mL)/isopropyl alcohol (0.6 mL), potassium carbonate (26.1 mg) and methanol (0.2 mL) were added, and the mixture was stirred at room temperature for 5.5 hr. Saturated aqueous ammonium chloride solution was added to the reaction mixture, and the mixture was extracted with ethyl acetate. The organic layer was dried over anhydrous magnesium sulfate and concentrated under reduced pressure. The residue was separated and purified by basic silica gel column chromatography (hexane:ethyl acetate=50:50→0:100→ethyl acetate:methanol=90:10) to give the title compound (20.1 mg) as a yellow powder.
$^1$H-NMR (DMSO-$d_6$) δ: 3.14 (3H, s), 3.46 (2H, s), 3.87 (2H, t, J = 4.5 Hz), 4.53 (2H, t, J = 4.5 Hz), 6.31 (1H, br s), 6.40 (1H, d, J = 8.5 Hz), 6.51 (1H, d, J = 3.0 Hz), 6.77 (1H, d, J = 7.5 Hz), 7.16-7.32 (2H, m), 7.57 (1H, dd, J = 2.6 Hz, 8.8 Hz), 7.66 (1H, d, J = 3.0 Hz), 7.96 (1H, d, J = 2.6 Hz), 8.33 (1H, s), 9.85 (1H, br s).

Example 9

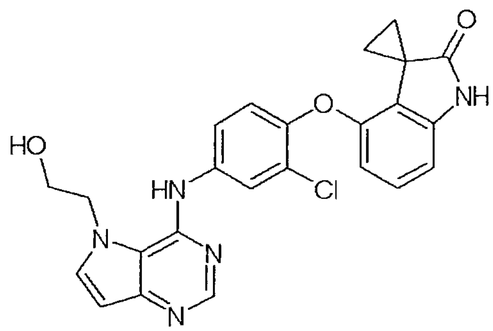

Production of 4'-(2-chloro-4-{[5-(2-hydroxyethyl)-5H-pyrrolo[3,2-d]pyrimidin-4-yl]amino}phenoxy)spiro[cyclopropane-1,3'-indol]-2'(1'H)-one

(i) Production of 4'-methoxyspiro[cyclopropane-1,3'-indol]-2'(1'H)-one

4-Methoxy-1,3-dihydro-2H-indol-2-one (1.00 g) was dissolved in tetrahydrofuran (25 mL), and the solution was cooled to -78°C. N,N,N',N'-Tetramethylethylenediamine (2.8 mL) and 1.6M n-butyllithium hexane solution (10 mL) were added, and the mixture was stirred at -78°C for 1 hr. 1,2-Dibromoethane (2.8 mL) was added to the reaction mixture, and the mixture was stirred at room temperature for 3 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=95:5→33:67) to give the title compound (287 mg) as a white powder.
$^1$H-NMR ($CDCl_3$) δ: 1.49-1.57 (2H, m), 1.95-2.08 (2H, m), 3.77 (3H, s), 6.54 (1H, d, J = 8.3 Hz), 6.61 (1H, d, J = 7.2 Hz), 7.01-7.20 (1H, m), 7.95 (1H, br s).

(ii) Production of 4'-hydroxyspiro[cyclopropane-1,3'-indol]-2'(1'H)-one

4'-Methoxyspiro[cyclopropane-1,3'-indol]-2'(1'H)-one (283 mg) was dissolved in benzotrifluoride (10 mL), and the solution was cooled to 0°C. 1N Boron tribromide dichloromethane solution (6 mL) was added, and the mixture was stirred at 0°C for 6 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=95:5→33:67) to give the title compound (140 mg) as a white powder.
$^1$H-NMR (DMSO-$d_6$) δ: 1.19-1.28 (2H, m), 1.76-1.88 (2H, m), 6.34-6.43 (2H, m), 6.92 (1H, t, J = 8.0 Hz), 9.45 (1H, s), 10.37 (1H, s).

(iii) Production of 4'-(2-chloro-4-nitrophenoxy)spiro[cyclopropane-1,3'-indol]-2'(1'H)-one

The title compound (175 mg) was obtained as a white powder by reaction in the same manner as in Example 2 (ii) and using 4'-hydroxyspiro[cyclopropane-1,3'-indol]-2'(1'H)-one (135 mg), 2-chloro-1-fluoro-4-nitrobenzene (233 mg), potassium carbonate (207 mg) and N,N-dimethylformamide (3 mL).
$^1$H-NMR ($CDCl_3$) δ: 1.60-1.70 (2H, m), 1.77-1.91 (2H, m), 6.54 (1H, d, J = 8.0 Hz), 6.85 (1H, d, J = 8.0 Hz), 6.98 (1H, d, J = 9.1 Hz), 7.20 (1H, t, J = 8.0 Hz), 7.83 (1H, br s), 8.08 (1H, dd, J = 2.5 Hz, 9.1 Hz), 8.39 (1H, d, J = 2.5 Hz).

(iv) Production of 4'-(4-amino-2-chlorophenoxy)spiro[cyclopropane-1,3'-indol]-2'(1'H)-one

The title compound (156 mg) was obtained as white crystals by reaction in the same manner as in Example 6 (ii) and using 4'-(2-chloro-4-nitrophenoxy)spiro[cyclopropane-1,3'-indol]-2'(1'H)-one (172 mg), 5% platinum/activated carbon (22.1 mg) and methanol (6 mL)/tetrahydrofuran (6 mL).
$^1$H-NMR ($CDCl_3$) δ: 1.57-1.70 (2H, m), 2.10-2.24 (2H, m), 3.68 (2H, s), 6.19 (1H, d, J = 8.5 Hz), 6.56 (1H, dd, J = 2.5 Hz, 8.5 Hz), 6.63 (1H, d, J = 7.7 Hz), 6.77 (1H, d, J = 2.5 Hz), 6.87 (1H, d, J = 8.5 Hz), 6.94 (1H, m), 7.64 (1H, br s).

(v) Production of 4'-(2-chloro-4-{[5-(2-hydroxyethyl)-5H-pyrrolo[3,2-d]pyrimidin-4-yl]amino}phenoxy)spiro[cyclopropane-1,3'-indol]-2'(1'H)-one

The title compound (172 mg) was obtained as a pale-orange powder by reaction in the same manner as in Example 2 (iv) and using 2-(4-chloro-5H-pyrrolo[3,2-d]pyrimidin-5-yl)ethyl benzoate (136 mg), 4'-(4-amino-2-chlorophenoxy)spiro[cyclopropane-1,3'-indol]-2'(1'H)-one (154 mg), isopropyl alcohol (3 mL), pyridine hydrochloride (5 mg) and 1N aqueous sodium hydroxide solution (1.5 mL).
$^1$H-NMR (DMSO-$d_6$) δ: 1.41 (2H, q, J = 3.5 Hz), 1.93 (2H, q, J = 3.5 Hz), 3.87 (2H, t, J = 4.3 Hz), 4.53 (2H, t, J = 4.3 Hz), 6.19 (1H, d, J = 8.3 Hz), 6.30 (1H, br s), 6.51 (1H, d, J = 3.0 Hz), 6.69 (1H, d, J = 8.0 Hz), 7.09 (1H, t, J = 8.0 Hz), 7.19 (1H, d, J = 8.8 Hz), 7.57 (1H, dd, J = 2.5 Hz, 8.8 Hz), 7.66 (1H, d, J = 3.0 Hz), 7.95 (1H, d, J = 2.5 Hz), 8.33 (1H, s), 9.84 (1H, br s), 10.70 (1H, br s).

Example 10

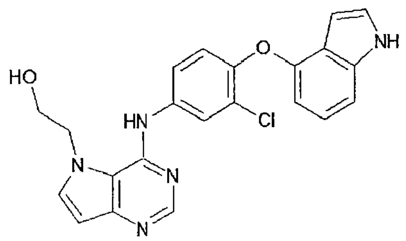

Production of 2-(4-{[3-chloro-4-(1H-indol-4-yloxy)phenyl]amino}-5H-pyrrolo[3,2-d]pyrimidin-5-yl)ethanol

(i) Production of 4-(2-chloro-4-nitrophenoxy)-1H-indole

To a solution of 1H-indol-4-ol (10.0 g) and 2-chloro-1-fluoro-4-nitrobenzene (13.2 g) in N,N-dimethylformamide (100 mL) was added potassium carbonate (15.0 g), and the mixture was stirred at room temperature for 18 hr. Saturated brine was added to the reaction system under ice-cooling, the mixture was extracted twice with ethyl acetate, and the organic layer was dried over anhydrous magnesium sulfate. After concentration under reduced pressure, the residue was separated and purified by silica gel column chromatography (eluent, hexane:ethyl acetate=1:0→1:1) to give the title compound (12.5 g) as yellow crystals.
$^1$H-NMR ($CDCl_3$) δ: 6.21-6.41 (1H, m), 6.79 (1H, d, J = 9.0 Hz), 6.87 (1H, dd, J = 0.8 Hz, 7.7 Hz), 7.13-7.29 (2H, m), 7.31-7.39 (1H, m), 7.97 (1H, dd, J = 2.7 Hz, 9.0 Hz), 8.30-8.44 (1H, m), 8.41 (1H, d, J = 2.7 Hz).

(ii) Production of 3-chloro-4-(1H-indol-4-yloxy)aniline

4-(2-Chloro-4-nitrophenoxy)-1H-indole (1.00 g) was dissolved in 15% water-containing ethanol (20 mL), reduced iron (750 mg) and calcium chloride (120 mg) were added, and the mixture was stirred at 80°C for 8 hr. The solid was removed by filtration, and the filtrate was concentrated under reduced pressure. Water was added to the residue,

and the mixture was extracted with ethyl acetate. The organic layer was washed with saturated brine, and dried over anhydrous magnesium sulfate. After concentration under reduced pressure, the residue was separated and purified by silica gel column chromatography (eluent, hexane:ethyl acetate=4:1→1:1) to give the title compound (620 mg) as a brown oil.
$^1$H-NMR ($CDCl_3$) δ: 3.63 (2H, br s), 6.40 (1H, dd, J = 1.0 Hz, 7.4 Hz), 6.50-6.64 (2H, m), 6.82 (1H, d, J = 2.8 Hz), 6.92 (1H, d, J = 8.5 Hz), 7.00-7.19 (3H, m), 8.20 (1H, br s).

(iii) Production of 2-(4-{[3-chloro-4-(1H-indol-4-yloxy)phenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (100 mg) and 3-chloro-4-(1H-indol-4-yloxy)aniline (86 mg) was dissolved in isopropyl alcohol (5 mL), pyridine hydrochloride (5 mg) was added, and the mixture was stirred at 80°C for 8 hr. The reaction mixture was cooled to room temperature, 1N aqueous sodium hydroxide solution (2 mL) was added, and the mixture was stirred at room temperature for 5 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=10:90→0:100→ethyl acetate:methanol=90:10), and crystallized from diisopropyl ether/ethyl acetate to give the title compound (120 mg) as colorless crystals.
$^1$H-NMR (DMSO-$d_6$) δ: 3.86 (2H, t, J = 4.5 Hz), 4.54 (2H, t, J = 4.5 Hz), 6.31 (1H, br s), 6.34-6.49 (2H, m), 6.92-7.08 (2H, m), 7.19 (1H, d, J = 7.9 Hz), 7.31 (1H, t, J = 2.7 Hz), 7.42-7.64 (2H, m), 7.94 (1H, d, J = 2.3 Hz), 8.29 (1H, s), 9.83 (1H, br s), 11.28 (1H, br s).
Example 11
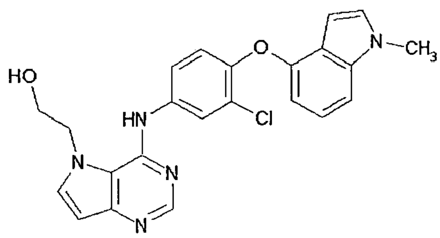

Production of 2-[4-({3-chloro-4-[(1-methyl-1H-indol-4-yl)oxy]phenyl}amino)-5H-pyrrolo[3,2-d]pyrimidin-5-yl] ethanol

(i) Production of 4-(2-chloro-4-nitrophenoxy)-1-methyl-1H-indole

The title compound (2.57 g) was obtained as yellow crystals in the same manner as in Example 10 (i) and using 4-(2-chloro-4-nitrophenoxy)-1H-indole (3.00 g), iodomethane (5 mL), potassium carbonate (3.0 g) and N,N-dimethylformamide (35 mL).
$^1$H-NMR ($CDCl_3$) δ: 3.84 (3H, s), 6.23 (1H, d, J = 3.2 Hz), 6.76 (1H, d, J = 9.2 Hz), 6.86 (1H, dd, J = 1.8 Hz, 6.7 Hz), 7.03 (1H, d, J = 3.2 Hz), 7.19-7.30 (2H, m), 7.95 (1H, dd, J = 2.7 Hz, 9.2 Hz), 8.40 (1H, d, J = 2.7 Hz).

(ii) Production of 3-chloro-4-[(1-methyl-1H-indol-4-yl)oxy]aniline

To a solution of 4-(2-chloro-4-nitrophenoxy)-1-methyl-1H-indole (1.50 g) in a mixed solvent of ethyl acetate (30 mL)/methanol (2 mL) were added 5% platinum/activated carbon (0.57 g) under nitrogen atmosphere. The reaction mixture was stirred under hydrogen atmosphere at room temperature for 3.5 hr, 5% platinum/activated carbon was filtered off, and the filtrate was concentrated under reduced pressure. The residue was separated and purified by basic silica gel column chromatography (eluent, ethyl acetate:hexane=60:40→100:0) to give the title compound (1.10 g) as a brown oil.
$^1$H-NMR ($CDCl_3$) δ: 3.63 (2H, br s), 3.79 (3H, s), 6.40 (1H, dd, J = 1.3 Hz, 7.1 Hz), 6.47-6.60 (2H, m), 6.81 (1H, d, J = 2.8 Hz), 6.91 (1H, d, J = 8.7 Hz), 6.95-7.13 (3H, m).

(iii) Production of 2-[4-({3-chloro-4-[(1-methyl-1H-indol-4-yl)oxy]phenyl}amino)-5H-pyrrolo[3,2-d]pyrimidin-5-yl]ethanol

The title compound (134 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-[(1-methyl-1H-indol-4-yl)oxy]aniline (90 mg), isopropyl alcohol (5 mL), pyridine hydrochloride (5 mg) and 1N aqueous sodium hydroxide solution (2 mL). $^1$H-NMR (DMSO-$d_6$) δ: 3.81 (3H, s), 3.87 (2H, t, J = 4.5 Hz), 4.53 (2H, t, J = 4.5 Hz), 6.32 (1H, dd, J = 0.8 Hz, 3.2 Hz), 6.40 (1H, d, J = 7.2 Hz), 6.50 (1H, d, J = 3.2 Hz), 6.93-7.13 (2H, m), 7.16-7.25 (1H, m), 7.30 (1H, d, J = 3.2 Hz), 7.51 (1H, dd, J = 2.5 Hz, 8.9 Hz), 7.64 (1H, d, J = 3.2 Hz), 7.96 (1H, d, J = 2.5 Hz), 8.32 (1H, s), 9.80 (1H, br s).

Example 12

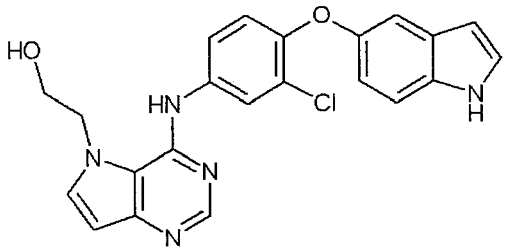

Production of 2-(4-{[3-chloro-4-(1H-indol-5-yloxy)phenyl]amino}-5H-pyrrolo[3,2-d]pyrimidin-5-yl)ethanol

(i) Production of 3-chloro-4-(1H-indol-5-yloxy)aniline

The title compound (740 mg) was obtained as a brown oil in the same manner as in Example 10 (i) and (ii) and using 1H-indol-5-ol (1.00 g), 2-chloro-1-fluoro-4-nitrobenzene (1.32 g), N,N-dimethylformamide (15 mL), potassium carbonate (1.50 g), 15% water-containing ethanol (15 mL), reduced iron (750 mg) and calcium chloride (120 mg). $^1$H-NMR ($CDCl_3$) δ: 3.63 (2H, br s), 6.48-6.58 (2H, m), 6.77-6.91 (3H, m), 7.10-7.14 (1H, m), 7.53 (1H, d, J = 8.3 Hz), 8.01 (1H, br s).

(ii) Production of 2-(4-{[3-chloro-4-(1H-indol-5-yloxy)phenyl]amino}-5H-pyrrolo[3,2-d]pyrimidin-5-yl)ethanol

The title compound (110 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-[(1H-indol-5-yl)oxy]aniline (91 mg), isopropyl alcohol (5 mL), pyridine hydrochloride (5 mg) and 1N aqueous sodium hydroxide solution (2 mL). $^1$H-NMR (DMSO-$d_6$) δ: 3.88 (2H, t, J = 4.4 Hz), 4.53 (2H, t, J = 4.4 Hz), 6.04-6.35 (1H, m), 6.40 (1H, br s), 6.50 (1H, d, J = 3.2 Hz), 6.76 (1H, dd, J = 2.2 Hz, 8.6 Hz), 6.85 (1H, d, J = 2.2 Hz), 7.12 (1H, d, J = 8.9 Hz), 7.28 (1H, t, J = 2.7 Hz), 7.44-7.57 (2H, m), 7.65 (1H, d, J = 3.2 Hz), 7.94 (1H, d, J = 2.5 Hz), 8.33 (1H, s), 9.78 (1H, br s), 10.95 (1H, br s).

Example 13

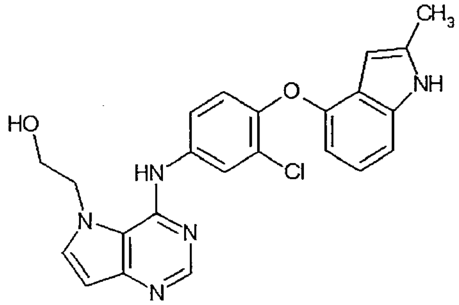

Production of 2-[4-({3-chloro-4-[(2-methyl-1H-indol-4-yl)oxy]phenyl}amino)-5H-pyrrolo[3,2-d]pyrimidin-5-yl] ethanol

(i) Production of 3-chloro-4-[(2-methyl-1H-indol-4-yl) oxy] aniline

The title compound (1.20 g) was obtained as a brown oil in the same manner as in Example 10 (i) and (ii) and using 2-methyl-1H-indol-4-ol (1.00 g), 2-chloro-1-fluoro-4-nitrobenzene (1.40 g), N,N-dimethylformamide (15 mL), potassium carbonate (1.60 g), 15% water-containing ethanol (15 mL), reduced iron (750 mg) and calcium chloride (100 mg).
$^1$H-NMR ($CDCl_3$) δ: 2. 42 (3H, s), 3. 61 (2H, br s), 6.22 (1H, s), 6.38-6.43 (1H, m), 6.53 (1H, dd, J = 3.0 Hz, 8.7 Hz), 6.81 (1H, d, J = 3.0 Hz), 6.87 (1H, d, J = 8.7 Hz), 6.92-7.06 (2H, m), 7.90 (1H, br s).

(ii) Production of 2-[4-({3-chloro-4-[(2-methyl-1H-indol-4-yl)oxy]phenyl}amino)-5H-pyrrolo[3,2-d]pyrimidin-5-yl]ethanol

The title compound (75 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-[(2-methyl-1H-indol-4-yl)oxy]aniline (90 mg), isopropyl alcohol (5 mL), pyridine hydrochloride (5 mg) and 1N aqueous sodium hydroxide solution (2 mL).
$^1$H-NMR (DMSO-$d_6$) δ: 2.35 (3H, s), 3.86 (2H, t, J = 4.5 Hz), 4.52 (2H, t, J = 4.5 Hz), 5.97 (1H, s), 6.39 (1H, d, J = 7.2 Hz), 6.49 (1H, d, J = 3.0 Hz), 6.84-7.00 (2H, m), 7.04-7.12 (1H, m), 7.46 (1H, dd, J 2.5 Hz, 8.9 Hz), 7.64 (1H, d, J = 3.0 Hz), 7.94 (1H, d, J = 2.5 Hz), 8.31 (1H, s), 9.72 (1H, br s), 11.09 (1H, s).

Example 14

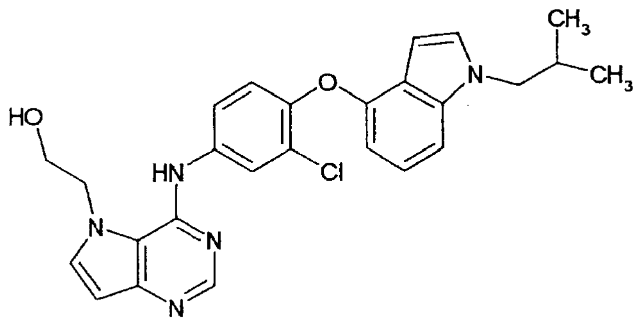

Production of 2-[4-({3-chloro-4-[(1-isobutyl-1H-indol-4-yl)oxy]phenyl}amino)-5H-pyrrolo[3,2-d]pyrimidin-5-yl] ethanol

(i) Production of 3-chloro-4-[(1-isobutyl-1H-indol-4-yl)oxy]aniline

The title compound (0.63 g) was obtained as a brown oil in the same manner as in Example 10 (i) and (ii) and using 4-(2-chloro-4-nitrophenoxy)-1H-indole (1.00 g), 1-bromo-2-methylpropane (3 mL), potassium carbonate (1.51 g), N,N-dimethylformamide (15 mL), 15% water-containing ethanol (15 mL), reduced iron (750 mg) and calcium chloride (100 mg).
$^1$H-NMR ($CDCl_3$) δ: 0.93 (6H, d, J = 6.8 Hz), 2.14-2.26 (1H, m), 3.64 (2H, br s), 3.89 (2H, d, J = 7.2 Hz), 6.33-6.41 (1H, m), 6.49-6.57 (2H, m), 6.81 (1H, d, J = 2.7 Hz), 6.91 (1H, d, J = 8.7 Hz), 6.97-7.07 (3H, m).

(ii) Production of 2-[4-({3-chloro-4-[(1-isobutyl-1H-indol-4-yl)oxy]phenyl}amino)-5H-pyrrolo[3,2-d]pyrimidin-5-yl]ethanol

The title compound (104 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-[(1-isobutyl-1H-indol-4-yl)oxy]aniline (99 mg), isopropyl alcohol (5 mL), pyridine hydrochloride (5 mg) and 1N aqueous sodium hydroxide solution (2 mL).
$^1$H-NMR (DMSO-$d_6$) δ: 0.86 (6H, d, J = 6.4 Hz), 2.07-2.21 (1H, m), 3.87 (2H, t, J = 4.5 Hz), 3.99 (2H, d, J = 7.2 Hz), 4.53 (2H, t, J = 4.5 Hz), 6.29-6.39 (2H, m), 6.50 (1H, d, J = 3.0 Hz), 6.99-7.15 (2H, m), 7.25 (1H, d, J= 8.3 Hz), 7.32 (1H, d, J = 3.0 Hz), 7.52 (1H, dd, J = 2.5 Hz, 8.9 Hz), 7.65 (1H, d, J = 3.0 Hz), 7.97 (1H, d, J = 2.5 Hz), 8.33 (1H, s), 9.79 (1H, br s).

Example 15

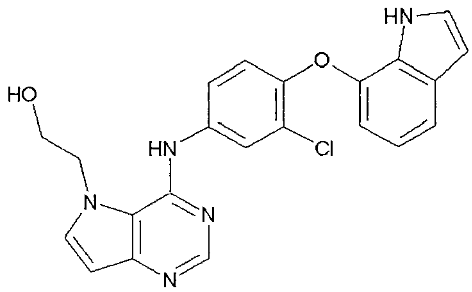

Production of 2-(4-{[3-chloro-4-(1H-indol-7-yloxy)phenyl]amino}-5H-pyrrolo[3,2-d]pyrimidin-5-yl)ethanol

(i) Production of 3-chloro-4-(1H-indol-7-yloxy)aniline

The title compound (1.11 g) was obtained as a brown oil in the same manner as in Example 10 (i) and (ii) and using 1H-indol-7-ol (1.00 g), 2-chloro-1-fluoro-4-nitrobenzene (1.34 g), N,N-dimethylformamide (15 mL), potassium carbonate (1.70 g), 15% water-containing ethanol (15 mL), reduced iron (750 mg) and calcium chloride (100 mg).
$^1$H-NMR ($CDCl_3$) δ: 3.75 (2H, br s), 6.40-6.60 (3H, m), 6.81-6.99 (4H, m), 7.29-7.32 (1H, m).

(ii) Production of 2-(4-{[3-chloro-4-(1H-indol-7-yloxy)phenyl]amino}-5H-pyrrolo[3,2-d]pyrimidin-5-yl)ethanol

The title compound (82 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-(1H-indol-7-yloxy)aniline (92 mg), isopropyl alcohol (5 mL), pyridine hydrochloride (5 mg) and 1N aqueous sodium hydroxide solution (2 mL).
$^1$H-NMR (DMSO-$d_6$) δ: 3.88 (2H, t, J = 4.5 Hz), 4.53 (2H, t, J = 4.5 Hz), 6.34 (1H, d, J = 7.6 Hz), 6.43-6.56 (2H, m), 6.89 (1H, t, J = 7.6 Hz), 7.15 (1H, d, J = 9.0 Hz), 7.23-7.39 (2H, m), 7.47-7.62 (1H, m), 7.65 (1H, d, J = 3.4 Hz), 7.99 (1H, d, J = 2.3 Hz), 8.33 (1H, s), 9.80 (1H, br s), 11.49 (1H, br s).

Example 16

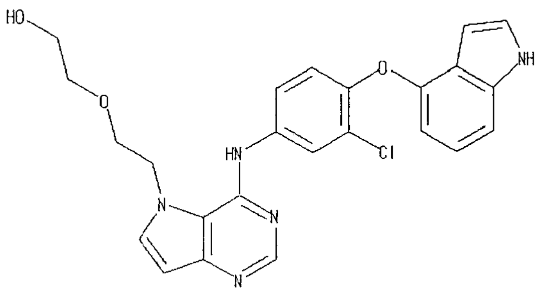

Production of 2-[2-(4-{[3-chloro-4-(1H-indol-4-yloxy)phenyl]amino}-5H-pyrrolo[3,2-d]pyrimidin-5-yl)ethoxy]ethanol

A mixture of 2-[2-(4-chloro-5H-pyrrolo[3,2-d]pyrimidin-5-yl)ethoxy]ethyl benzoate (120 mg) and 3-chloro-4-(1H-indol-4-yloxy)aniline (90 mg) was dissolved in isopropyl alcohol (5 mL), pyridine hydrochloride (5 mg) was added, and the mixture was stirred at 80°C for 8 hr. The reaction mixture was cooled to room temperature, 1N aqueous sodium hydroxide solution (2 mL) was added, and the mixture was stirred at room temperature for 5 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=10:90→0:100→ethyl acetate:methanol=90:10), and crystallized from diisopropyl ether/ethyl acetate to give the title compound (96 mg) as colorless crystals.

$^1$H-NMR (DMSO-$d_6$) δ: 3.42-3.55 (4H, m), 3.83 (2H, t, J = 4.5 Hz), 4.54-4.75 (3H, m), 6.32 (1H, br s), 6.40 (1H, d, J = 8.0 Hz), 6.50 (1H, d, J = 3.0 Hz), 6.89-7.11 (2H, m), 7.19 (1H, d, J = 8.0 Hz), 7.31 (1H, t, J = 2.7 Hz), 7.53 (1H, dd, J = 2.7 Hz, 8.7 Hz), 7.67 (1H, d, J = 3.0 Hz), 7.99 (1H, d, J = 2.3 Hz), 8.32 (1H, s), 8.88 (1H, s), 11.28 (1H, br s).

Example 17

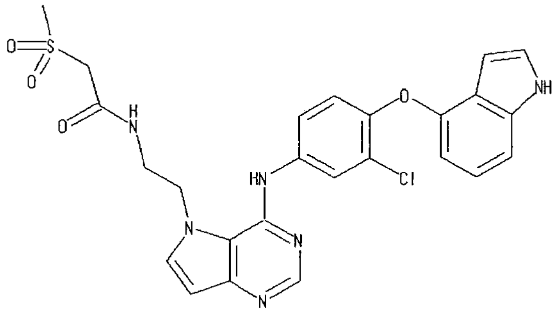

Production of N-[2-(4-{[3-chloro-4-(1H-indol-4-yloxy)phenyl]amino}-5H-pyrrolo[3,2-d]pyrimidin-5-yl)ethyl]-2-(methylsulfonyl)acetamide

A mixture of tert-butyl [2-(4-chloro-5H-pyrrolo[3,2-d]pyrimidin-5-yl)ethyl]carbamate (100 mg), 3-chloro-4-(1H-indol-4-yloxy)aniline (87 mg) and isopropyl alcohol (5 mL) was stirred at 80°C for 12 hr. Saturated aqueous sodium hydrogen carbonate solution was added to the reaction system under ice-cooling, and the mixture was extracted with ethyl acetate. The organic layer was washed with saturated brine, and dried over anhydrous magnesium sulfate. The residue was separated and purified by silica gel column chromatography (eluent, ethyl acetate:hexane=60:40→100:0) to give a crude product (150 mg). The obtained crude product (140 mg) was dissolved in tetrahydrofuran (4 mL), 4N hydrochloric acid/ethyl acetate (4 mL) was added, and the mixture was stirred at 70°C for 20 hr. The solvent was evaporated under reduced pressure, ethanol was added, and the mixture was further concentrated. Diisopropyl ether was added, and the precipitated powder was collected by filtration. A mixture of the obtained powder, methylsulfonylacetic acid (70 mg), 1-ethyl-3-(3-dimethylaminopropyl)carbodiimide hydrochloride (110 mg), 1-hydroxybenzotriazole (70 mg), triethylamine (0.35 mL) and N,N-dimethylformamide (7.0 mL) was stirred at room temperature for 16 hr. Water was added to the reaction system, and the mixture was extracted with ethyl acetate. The organic layer was washed with water and saturated brine, and dried over anhydrous magnesium sulfate. After concentration under reduced pressure, the residue was separated and purified by basic silica gel column chromatography (eluent, ethyl acetate→ethyl acetate: methanol=90:10), and crystallized from diisopropyl ether to give the title compound (76 mg) as colorless crystals.

$^1$H-NMR (DMSO-$d_6$) δ: 3.09 (3H, s), 3.46 (2H, q, J = 5.9 Hz), 4.04 (2H, s), 4.56 (2H, t, J = 5.9 Hz), 6.32 (1H, br s), 6.41 (1H, d, J = 8.0 Hz), 6.48 (1H, d, J = 2.7 Hz), 6.95-7.06 (2H, m), 7.19 (1H, d, J = 8.0 Hz), 7.31 (1H, d, J = 2.7 Hz), 7.60 (2H, br s), 7.93 (1H, s), 8.31 (1H, s), 8.52-8.75 (2H, m), 11.28 (1H, br s).

Example 18

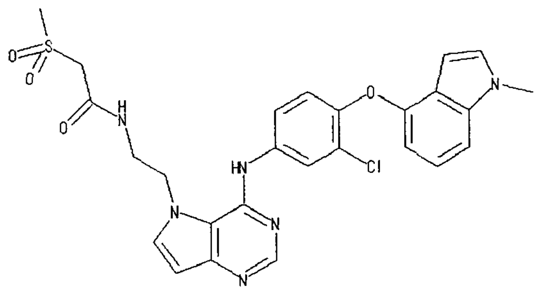

Production of N-{2-[4-({3-chloro-4-[(1-methyl-1H-indol-4-yl)oxy]phenyl}amino)-5H-pyrrolo[3,2-d]pyrimidin-5-yl]ethyl}-2-(methylsulfonyl)acetamide

A mixture of tert-butyl [2-(4-chloro-5H-pyrrolo[3,2-d]pyrimidin-5-yl)ethyl]carbamate (100 mg), 3-chloro-4-[(1-methyl-1H-indol-4-yl)oxy]aniline (90 mg) and isopropyl alcohol (5 mL) was stirred at 80°C for 12 hr. Saturated aqueous sodium hydrogen carbonate solution was added to the reaction system under ice-cooling, and the mixture was extracted with ethyl acetate. The organic layer was washed with saturated brine, and dried over anhydrous magnesium sulfate. The residue was separated and purified by silica gel column chromatography (eluent, ethyl acetate:hexane=60:40→100:0) to give a crude product (160 mg). The obtained crude product (120 mg) was dissolved in tetrahydrofuran (4 mL), 4N hydrochloric acid/ethyl acetate (4 mL) was added, and the mixture was stirred at 70°C for 20 hr. The solvent was evaporated under reduced pressure, ethanol was added, and the mixture was further concentrated. Diisopropyl ether was added, and the precipitated powder was collected by filtration. A mixture of the obtained powder, methylsulfonylacetic acid (60 mg), 1-ethyl-3-(3-dimethylaminopropyl)carbodiimide hydrochloride (100 mg), 1-hydroxybenzotriazole (70 mg), triethylamine (0.30 mL) and N,N-dimethylformamide (7.0 mL) was stirred at room temperature for 16 hr. Water was added to the reaction system, and the mixture was extracted with ethyl acetate. The organic layer was washed with water and saturated brine, and dried over anhydrous magnesium sulfate. After concentration under reduced pressure, the residue was separated and purified by basic silica gel column chromatography (eluent, ethyl acetate→ethyl acetate:methanol=90:10), and crystallized from diisopropyl ether to give the title compound (62 mg) as colorless crystals.
$^1$H-NMR (DMSO-$d_6$) δ: 3.09 (3H, s), 3.46 (2H, d, J = 5.8 Hz), 3.81 (3H, s), 4.04 (2H, s), 4.56 (2H, t, J = 5.8 Hz), 6.32 (1H, d, J = 2.8 Hz), 6.40-6.52 (2H, m), 6.98-7.14 (2H, m), 7.19-7.27 (1H, m), 7.31 (1H, d, J = 3.2 Hz), 7.53-7.66 (2H, m), 7.94 (1H, d, J = 2.3 Hz), 8.32 (1H, s), 8.57-8.74 (2H, m).
Example 19
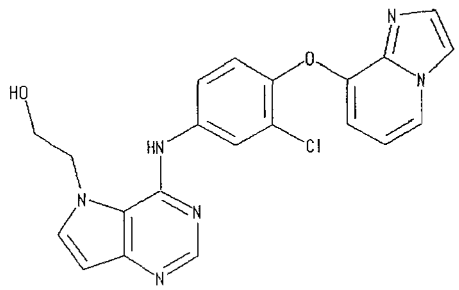

Production of 2-(4-{[3-chloro-4-(imidazo[1,2-a]pyridin-8-yloxy)phenyl]amino}-5H-pyrrolo[3,2-d]pyrimidin-5-yl)ethanol

(i) Production of 3-chloro-4-(imidazo[1,2-a]pyridin-8-yloxy)aniline

The title compound (720 mg) was obtained as a brown oil in the same manner as in Example 10 (i) and (ii) and using imidazo[1,2-a]pyridin-8-ol (1.00 g), 2-chloro-1-fluoro-4-nitrobenzene (1.40 g), N,N-dimethylformamide (15 mL), potassium carbonate (1.80 g), 15% water-containing ethanol (15 mL), reduced iron (750 mg) and calcium chloride (100 mg).
$^1$H-NMR ($CDCl_3$) δ: 3.73 (2H, br s), 6.17 (1H, d, J = 7.6 Hz), 6.53-6.66 (2H, m), 6.81 (1H, d, J = 2.7 Hz), 7.04 (1H, d, J = 8.3 Hz), 7.64 (2H, d, J = 11.7 Hz), 7.83 (1H, d, J = 6.8 Hz).

(ii) Production of 2-(4-{[3-chloro-4-(imidazo[1,2-a]pyridin-8-yloxy)phenyl]amino}-5H-pyrrolo[3,2-d]pyrimidin-5-yl)ethanol

The title compound (90 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-(imidazo[1,2-a]pyridin-8-yloxy)aniline (100 mg), isopropyl alcohol (5 mL), pyridine hydrochloride (5 mg) and 1N aqueous sodium hydroxide solution (2 mL).
$^1$H-NMR (DMSO-$d_6$) δ: 3.88 (2H, t, J = 4. 5 Hz), 4.54 (2H, t, J = 4.5 Hz), 6.43 (1H, dd, J = 0.8 Hz, 7.5 Hz), 6.51 (1H, d, J = 3.0 Hz), 6.72-6.85 (1H, m), 7.23 (1H, d, J = 8.9 Hz), 7.51-7.62 (2H, m), 7.66 (1H, d, J = 3.0 Hz), 7.98 (1H, d, J = 2.5 Hz), 8.03 (1H, d, J = 0.8 Hz), 8.25-8.38 (2H, m), 9.84 (1H, br s).
Example 20
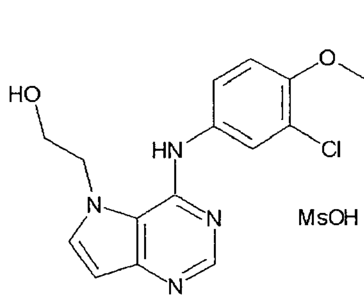

Production of 2-(4-{[3-chloro-4-(1H-indol-4-yloxy)phenyl]amino}-5H-pyrrolo[3,2-d]pyrimidin-5-yl)ethanol methanesulfonate

2-(4-{[3-Chloro-4-(1H-indol-4-yloxy)phenyl]amino}-5H-pyrrolo[3,2-d]pyrimidin-5-yl)ethanol (76 mg) was dissolved in isopropyl alcohol (1 mL), methanesulfonic acid (17 mg) was added, and the mixture was stirred at 50°C for 10 min. Diisopropyl ether (5 mL) was added, the mixture was stood at room temperature for 3 hr, and the precipitated crystals were collected by filtration and dried to give the title compound (60 mg) as pale-yellow crystals.
$^1$H-NMR (DMSO-$d_6$) δ: 2.34 (3H, s), 3.85-3.95 (2H, m), 4.68 (2H, br s), 6.19-6.29 (1H, m), 6.51 (1H, d, J = 7.2 Hz), 6.70 (1H, d, J = 3.2 Hz), 6.98-7.12 (2H, m), 7.25 (1H, d, J = 8.1 Hz), 7.33 (1H, t, J = 2.7 Hz), 7.47 (1H, dd, J = 2.5 Hz, 8.8 Hz), 7.90 (1H, d, J = 2.7 Hz), 8.01 (1H, d, J = 3.2 Hz), 8.77 (1H, s), 10.75 (1H, s), 11.34 (1H, br s).
Example 21
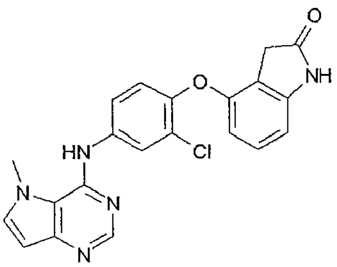

Production of 4-{2-chloro-4-[(5-methyl-5H-pyrrolo[3,2-d]pyrimidin-4-yl)amino]phenoxy}-1,3-dihydro-2H-indol-2-one

(i) Production of 4-chloro-5-methyl-5H-pyrrolo[3,2-d]pyrimidine

4-Chloro-5H-pyrrolo[3,2-d]pyrimidine (4.00 g) was dissolved in N,N-dimethylformamide (100 mL), methyl methanesulfonate (2.5 mL) and potassium carbonate (6.03 g) were added, and the mixture was stirred at room temperature for 18 hr. Aqueous ammonium chloride solution was added to the reaction mixture, and the mixture was extracted with ethyl acetate. The organic layer was washed successively with water and saturated brine, dried over anhydrous magnesium sulfate, and concentrated under reduced pressure. The residue was separated and purified by basic silica gel column chromatography (hexane:ethyl acetate=95:5→50:50) to give the title compound (3.00 g) as white crystals.
$^1$H-NMR ($CDCl_3$) δ: 4.16 (3H, s), 6.70 (1H, d, J = 3.0 Hz), 7.42 (1H, d, J = 3.0 Hz), 8.69 (1H, s).

(ii) Production of 4-{2-chloro-4-[(5-methyl-5H-pyrrolo[3,2-d]pyrimidin-4-yl)amino]phenoxy}-1,3-dihydro-2H-indol-2-one

A mixture of 4-chloro-5-methyl-5H-pyrrolo[3,2-d]pyrimidine (66.5 mg) and 4-(4-amino-2-chlorophenoxy)-1,3-dihydro-2H-indol-2-one (122 mg) was dissolved in isopropyl alcohol (3 mL), pyridine hydrochloride (5 mg) was added and the mixture was stirred at 70°C for 13 hr. Saturated aqueous sodium hydrogen carbonate solution was added to the reaction mixture, and the mixture was extracted with ethyl acetate. The organic layer was dried over anhydrous magnesium sulfate and concentrated under reduced pressure. The residue was separated and purified by silica gel column chromatography (hexane:ethyl acetate=50:50→0:100) to give the title compound (128 mg) as pale-orange

crystals.
$^1$H-NMR (DMSO-$d_6$) δ: 3.37 (2H, s), 4.15 (3H, s), 6.33 (1H, d, J = 8.0 Hz), 6.45 (1H, d, J = 3.0 Hz), 6.60 (1H, d, J = 8.0 Hz), 7.15 (1H, t, J = 8.0 Hz), 7.18 (1H, d, J = 8.8 Hz), 7.60 (1H, d, J = 3.0 Hz), 7.64 (1H, dd, J = 2.5 Hz, 8.8 Hz), 7.94 (1H, d, J = 2.5 Hz), 8.31 (1H, s), 8.57 (1H, s), 10.52 (1H, br s).

Example 22

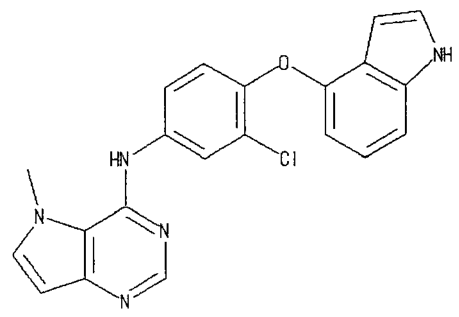

Production of N-[3-chloro-4-(1H-indol-4-yloxy)phenyl]-5-methyl-5H-pyrrolo[3,2-d]pyrimidin-4-amine

A mixture of 4-chloro-5-methyl-5H-pyrrolo[3,2-d]pyrimidine (100 mg) and 3-chloro-4-(1H-indol-4-yloxy)aniline (163 mg) was dissolved in isopropyl alcohol (5 mL), pyridine hydrochloride (5 mg) was added, and the mixture was stirred at 80°C for 8 hr. The reaction mixture was cooled to room temperature, saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=10:90-0:100→ethyl acetate:methanol=90:10), and crystallized from diisopropyl ether/ethyl acetate to give the title compound (173 mg) as colorless crystals.
$^1$H-NMR (DMSO-$d_6$) δ: 4.15 (3H, s), 6.31 (1H, br s), 6.35-6.49 (2H, m), 6.92-7.08 (2H, m), 7.19 (1H, d, J = 7.9 Hz), 7.31 (1H, t, J = 2.7 Hz), 7.42-7.64 (2H, m), 7.94 (1H, d, J = 2.3 Hz), 8.29 (1H, s), 8.52 (1H, s), 11.28 (1H, br s).

Example 23

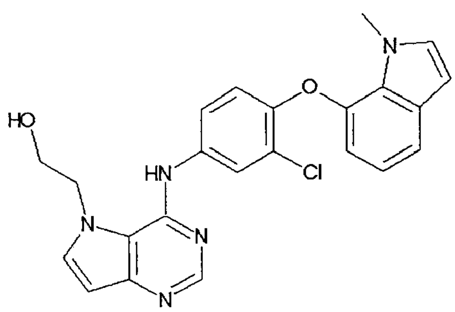

Production of 2-[4-({3-chloro-4-[(1-methyl-1H-indol-7-yl)oxy]phenyl}amino)-5H-pyrrolo[3,2-d]pyrimidin-5-yl]ethanol

(i) Production of 3-chloro-4-[(1-methyl-1H-indol-7-yl)oxy]aniline

The title compound (204 mg) was obtained as a brown oil in the same manner as in Example 10 (i) and (ii) and using 7-(2-chloro-4-nitrophenoxy)-1H-indole (500 mg), iodomethane (1 mL), potassium carbonate (700 mg), N,N-dimethylformamide (10 mL), 15% water-containing ethanol (10 mL), reduced iron (550 mg) and calcium chloride (70 mg).
$^1$H-NMR ($CDCl_3$) δ: 3.63 (2H, br s), 4.05 (3H, s), 6.44-6.60 (3H, m), 6.84-6.98 (4H, m), 7.29-7.32 (1H, m).

(ii) Production of 2-[4-({3-chloro-4-[(1-methyl-1H-indol-7-yl)oxy]phenyl}amino)-5H-pyrrolo[3,2-d]pyrimidin-5-yl]ethanol

The title compound (102 mg) was obtained as colorless crystals in the same manner as in Example 10 (iii) and using 2-(4-chloro-5H-pyrrolo[3,2-d]pyrimidin-5-yl)ethyl benzoate (100 mg), 3-chloro-4-[(1-methyl-1H-indol-7-yl)oxy]aniline (95 mg), isopropyl alcohol (5 mL), pyridine hydrochloride (5 mg) and 1N aqueous sodium hydroxide solution (2 mL).
$^1$H-NMR (DMSO-$d_6$) δ: 3.87 (2H, t, J = 4.5 Hz), 4.00 (3H, s), 4.53 (2H, t, J = 4.5 Hz), 6.32-6.54 (3H, m), 6.92 (1H, t, J = 7.8 Hz), 7.08 (1H, d, J = 8.7 Hz), 7.31 (2H, dd, J = 2.3 Hz, 5.3 Hz), 7.53 (1H, dd, J = 2.5 Hz, 8.7 Hz), 7.64 (1H, d, J = 3.0 Hz), 7.97 (1H, d, J = 2.5 Hz), 8.31 (1H, s), 9.54 - 9.97 (1H, m).
Example 24
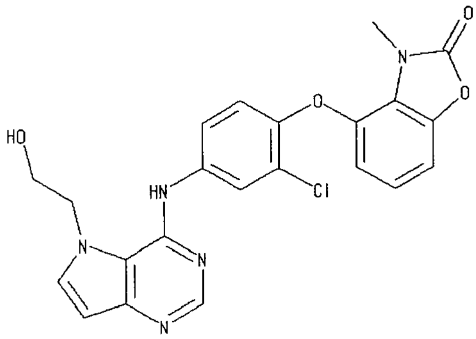

Production of 4-(2-chloro-4-{[5-(2-hydroxyethyl)-5H-pyrrolo[3,2-d]pyrimidin-4-yl]amino}phenoxy)-3-methyl-1,3-benzoxazole-2(3H)-one

(i) Production of 4-(4-amino-2-chlorophenoxy)-3-methyl-1,3-benzoxazole-2(3H)-one

The title compound (61 mg) was obtained as a brown oil in the same manner as in Example 11 (i) and (ii) and using 4-hydroxy-3-methyl-1,3-benzoxazole-2(3H)-one (200 mg), 2-chloro-1-fluoro-4-nitrobenzene (208 mg), potassium carbonate (500 mg), N,N-dimethylformamide (7 mL), ethyl acetate (8 mL), methanol (1 mL) and 5% platinum/activated carbon (0.22 g).
$^1$H-NMR ($CDCl_3$) δ: 3.67 (3H, s), 3.73 (2H, s), 6.47 (1H, dd, J = 2.7 Hz, 6.4 Hz), 6.59 (1H, dd, J = 2.7 Hz, 8.7 Hz), 6.80 (1H, d, J = 2.7 Hz) 6.88-6.96 (3H, m).

(ii) Production of 4-(2-chloro-4-{[5-(2-hydroxyethyl)-5H-pyrrolo[3,2-d]pyrimidin-4-yl]amino}phenoxy)-3-methyl-1,3-benzoxazole-2(3H)-one

The title compound (41 mg) was obtained as colorless crystals in the same manner as in Example 10 (iii) and using 2-(4-chloro-5H-pyrrolo[3,2-d]pyrimidin-5-yl)ethyl benzoate (50 mg), 4-(4-amino-2-chlorophenoxy)-3-methyl-1,3-benzoxazole-2(3H)-one (50 mg), isopropyl alcohol (5 mL), pyridine hydrochloride (5 mg) and 1N aqueous sodium hydroxide solution (2 mL).
$^1$H-NMR (DMSO-$d_6$) δ: 3.52 (3H, s), 3.87 (2H, t, J = 4.2 Hz), 4.53 (2H, t, J = 4.2 Hz), 6.29 (1H, br s), 6.51 (1H, d, J = 3.0 Hz), 6.62 (1H, dd, J = 1.0 Hz, 8.4 Hz), 7.00-7.19 (2H, m), 7.25 (1H, d, J = 8.9 Hz), 7.58 (1H, dd, J = 2.6 Hz, 8.9 Hz), 7.66 (1H, d, J = 3.0 Hz), 7.98 (1H, d, J = 2. 6 Hz), 8.33 (1H, s), 9. 85 (1H, s).
Example 25
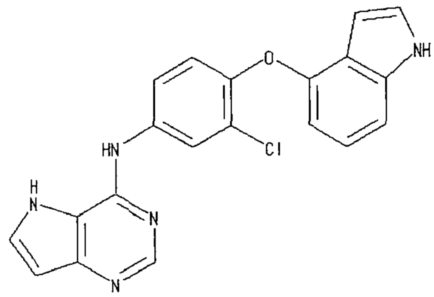

Production of N-[3-chloro-4-(1H-indol-4-yloxy)phenyl]-5H-pyrrolo[3,2-d]pyrimidin-4-amine

A mixture of 4-chloro-5H-pyrrolo[3,2-d]pyrimidine (100 mg) and 3-chloro-4-(1H-indol-4-yloxy)aniline (277 mg) was dissolved in isopropyl alcohol (5 mL), pyridine hydrochloride (5 mg) was added, and the mixture was stirred at 80°C for 8 hr. The reaction mixture was cooled to room temperature, saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=10:90→0:100→ethyl acetate:methanol=90:10), and crystallized from diisopropyl ether/ethyl acetate to give the title compound (125 mg) as colorless crystals.
$^1$H-NMR (DMSO-$d_6$) δ: 6.30 (1H, t, J = 2.1 Hz), 6.41 (1H, d, J = 7.5 Hz), 6.50 (1H, dd, J = 1.7 Hz, 2.7 Hz), 6.93-7.10 (2H, m), 7.19 (1H, d, J = 8.1 Hz), 7.31 (1H, t, J = 2.7 Hz), 7.57 (1H, dd, J = 2.7 Hz, 8.9 Hz), 7.69 (1H, t, J = 2.7 Hz), 8.32-8.44 (2H, m), 9.41 (1H, s), 11.12 (1H, br s), 11.28 (1H, br s).

Example 26

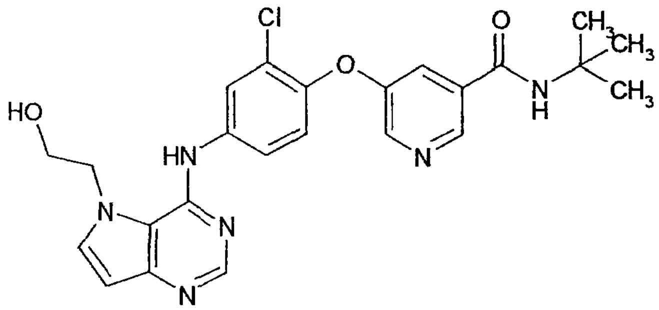

Production of N-(tert-butyl)-5-(2-chloro-4-{[5-(2-hydroxyethyl)-5H-pyrrolo[3,2-d]pyrimidin-4-yl]amino}phenoxy) nicotinamide

(i) Production of methyl 5-(2-chloro-4-nitrophenoxy)nicotinate

A mixture of methyl 5-hydroxynicotinate (2.00 g), 2-chloro-1-fluoro-4-nitrobenzene (2.03 g), potassium carbonate (2.71 g) and N,N-dimethylformamide (20 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=15:85→30:70). The object fraction was concentrated under reduced pressure to give the title compound (2.40 g) as a yellow solid.
$^1$H-NMR ($CDCl_3$) δ: 3.97 (3H, s), 7.03 (1H, d, J = 9.1 Hz), 7.91 (1H, dd, J = 1.5 Hz, 3.0 Hz), 8.15 (1H, dd, J = 2.7 Hz, 9.1 Hz), 8.44 (1H, d, J = 2.7 Hz), 8.64 (1H, d, J = 3.0 Hz), 9.10 (1H, d, J = 1.5 Hz).

(ii) Production of 5-(2-chloro-4-nitrophenoxy)nicotinic acid

Isopropyl alcohol (20 mL), tetrahydrofuran (10 mL) and 1N aqueous sodium hydroxide solution (8.8 mL) were

added to methyl 5-(2-chloro-4-nitrophenoxy)nicotinate (2.27 g) and the mixture was stirred at room temperature overnight. The reaction mixture was concentrated under reduced pressure, and 1N hydrochloric acid (8.8 mL) and water were added. The precipitated solid was collected by filtration, and washed with water to give the title compound (2.09 g) as a white powder.
$^1$H-NMR (DMSO-$d_6$) δ: 7.32 (1H, d, J = 9.1 Hz), 7.94 (1H, dd, J = 1.8 Hz, 2.8 Hz), 8.20 (1H, dd, J = 2.7 Hz, 9.1 Hz), 8.52 (1H, d, J = 2.7 Hz), 8.77 (1H, d, J = 2.8 Hz), 8.97 (1H, d, J = 1.8 Hz).

(iii) Production of N-(tert-butyl)-5-(2-chloro-4-nitrophenoxy)nicotinamide

A mixture of 5-(2-chloro-4-nitrophenoxy)nicotinic acid (589 mg), tert-butylamine (0.315 mL), 1-ethyl-3-(3-dimethylaminopropyl)carbodiimide hydrochloride (460 mg), 1-hydroxybenzotriazole (324 mg) and N,N-dimethylformamide (10 mL) was stirred at room temperature for 7 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=20:80→40:60). The object fraction was concentrated under reduced pressure to give the title compound (675 mg) as a white amorphous powder. $^1$H-NMR ($CDCl_3$) δ: 1.48 (9H, s), 5.97 (1H, br s), 7.03 (1H, d, J = 9.0 Hz), 7.74 (1H, dd, J = 1.8 Hz, 2.6 Hz), 8.14 (1H, dd, J = 2.8 Hz), 9.0 Hz), 8.42 (1H, d, J = 2.6 Hz), 8.56 (1H, d, J = 2.8 Hz), 8.73 (1H, d, J = 1.8 Hz).

(iv) Production of 5-(4-amino-2-chlorophenoxy)-N-(tert-butyl)nicotinamide

A mixture of N-(tert-butyl)-5-(2-chloro-4-nitrophenoxy)nicotinamide (675 mg), reduced iron (372 mg), calcium chloride (123 mg) and 10% water-containing ethanol (20 mL) was stirred with heating under reflux overnight. The reaction mixture was filtered to remove solid, and the filtrate was concentrated. Water was added to the residue, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=30:70→60:40). The object fraction was concentrated under reduced pressure and the residue was crystallized from diisopropyl ether to give the title compound (319 mg) as a pale-brown powder.
$^1$H-NMR ($CDCl_3$) δ: 1.46 (9H, s), 3.74 (2H, br s), 5.92 (1H, br s), 6.57 (1H, dd, J = 2.7 Hz, 8.5 Hz), 6.77 (1H, d, J = 2.7 Hz), 6.92 (1H, d, J = 8.5 Hz), 7.48 (1H, dd, J = 1.9 Hz, 2.9 Hz), 8.41 (1H, d, J = 2. 9 Hz), 8.50 (1H, d, J = 1.9 Hz).

(v) Production of N-(tert-butyl)-5-(2-chloro-4-{[5-(2-hydroxyethyl)-5H-pyrrolo[3,2-d]pyrimidin-4-yl]amino}phenoxy)nicotinamide

A mixture of 2-(4-chloro-5H-pyrrolo[3,2-d]pyrimidin-5-yl)ethyl benzoate (121 mg), 5-(4-amino-2-chlorophenoxy)-N-(tert-butyl)nicotinamide (128 mg), pyridine hydrochloride (5 mg) and isopropyl alcohol (5 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=60:40→100:0→methanol:ethyl acetate=10:90). The object fraction was concentrated under reduced pressure. Methanol (5 mL), tetrahydrofuran (1 mL) and 1N aqueous sodium hydroxide solution (0.8 mL) were added to the residue and the mixture was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure, and the residue was crystallized from ethanol/ethyl acetate to give the title compound (146 mg) as a white powder.
$^1$H-NMR (DMSO-$d_6$) δ: 1.36 (9H, s), 3.87 (2H, q, J = 4.3 Hz), 4.54 (2H, t, J = 4.5 Hz), 6.30 (1H, t, J = 4.0 Hz), 6.51 (1H, d, J = 3.2 Hz), 7.32 (1H, d, J = 8.9 Hz), 7.54 (1H, dd, J = 1.7 Hz, 2.8 Hz), 7.62 (1H, dd, J = 2.5 Hz, 8.9 Hz), 7.66 (1H, d, J = 3.2 Hz), 7.99 (1H, d, J = 2.5 Hz), 8.06 (1H, br s), 8.34 (1H, s), 8.47 (1H, d, J = 2.8 Hz), 8.73 (1H, d, J = 1.7 Hz), 9.91 (1H, br s).
Example 27
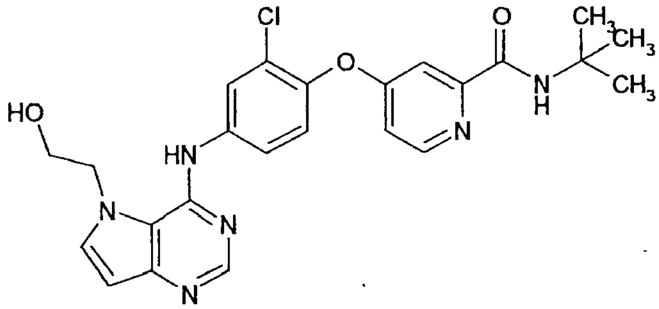

Production of N-(tert-butyl)-4-(2-chloro-4-{[5-(2-hydroxyethyl)-5H-pyrrolo[3,2-d]pyrimidin-4-yl]amino}phenoxy) pyridine-2-carboxamide

(i) Production of N-(tert-butyl)-4-(2-chloro-4-nitrophenoxy)pyridine-2-carboxamide

A mixture of 4-hydroxypyridine-2-carboxylic acid (1.39 g), tert-butylamine (3.15 mL), 1-ethyl-3-(3-dimethylaminopropyl)carbodiimide hydrochloride (2.30 g), 1-hydroxybenzotriazole (1.62 g) and N,N-dimethylformamide (20 mL) was stirred at 60°C for 4 hr. Triethylamine (1.39 mL) and tert-butylamine (3.15 mL) were added, and the mixture was stirred at 40°C overnight. The reaction mixture was concentrated under reduced pressure, water was added, and the mixture was extracted with a mixed solvent of ethyl acetate and tetrahydrofuran. The organic layer was washed with saturated brine, and dried over anhydrous sodium sulfate. The solvent was evaporated under reduced pressure and the obtained residue was dissolved in N,N-dimethylformamide (20 mL). 2-Chloro-1-fluoro-4-nitrobenzene (1.76 g) and potassium carbonate (2.76 g) were added and the mixture was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=10:90→20:80). The object fraction was concentrated under reduced pressure to give the title compound (1.79 g) as a pale-yellow amorphous solid.
$^1$H-NMR ($CDCl_3$) δ: 1.47 (9H, s), 7.07 (1H, dd, J = 2.7 Hz, 5.7 Hz), 7.27 (1H, d, J = 9.1 Hz), 7.62 (1H, d, J = 2.7 Hz), 7.97 (1H, br s), 8.21 (1H, dd, J = 2.7 Hz, 9.1 Hz), 8.42 (1H, d, J = 2.7 Hz), 8.49 (1H, d, J = 5.7 Hz).

(ii) Production of 4-(4-amino-2-chlorophenoxy)-N-(tert-butyl)pyridine-2-carboxamide

The title compound (996 mg) was obtained as a pale-orange powder in the same manner as in Example 26 (iv) and using N-(tert-butyl)-4-(2-chloro-4-nitrophenoxy)pyridine-2-carboxamide (1.77 g), reduced iron (942 mg), calcium chloride (312 mg) and 10% water-containing ethanol (50 mL).
$^1$H-NMR ($CDCl_3$) δ: 1.47 (9H, s), 3.76 (2H, br s), 6.58 (1H, dd, J = 2.7 Hz, 8.5 Hz), 6.77 (1H, d, J = 2.7 Hz), 6.92-6.97 (2H, m), 7.55 (1H, d, J = 2.3 Hz), 7.99 (1H, br s), 8.35 (1H, d, J = 5.7 Hz).

(iii) Production of N-(tert-butyl)-4-(2-chloro-4-{[5-(2-hydroxyethyl)-5H-pyrrolo[3,2-d]pyrimidin-4-yl]amino}phenoxy)pyridine-2-carboxamide

The title compound (151 mg) was obtained as a white powder in the same manner as in Example 26 (v) and using 2-(4-chloro-5H-pyrrolo[3,2-d]pyrimidin-5-yl)ethyl benzoate (121 mg), 4-(4-amino-2-chlorophenoxy)-N-(tert-butyl) pyridine-2-carboxamide (128 mg), pyridine hydrochloride (5 mg), isopropyl alcohol (5 mL), methanol (5 mL), tetrahydrofuran (1 mL) and 1N aqueous sodium hydroxide solution (0.8 mL).
$^1$H-NMR ($CDCl_3$) δ: 1.46 (9H, s), 4.13 (2H, t, J = 4.4 Hz), 4.39 (2H, t, J = 4.4 Hz), 6.14 (1H, d, J = 3.3 Hz), 7.01 (1H, d, J = 3.3 Hz), 7.03 (1H, dd, J = 2.3 Hz, 5.4 Hz), 7.10 (1H, d, J = 8.8 Hz), 7.48 (1H, dd, J = 2.5 Hz, 8.8 Hz), 7.55 (1H, d, J = 2.3 Hz), 7.82 (1H, d, J = 2.5 Hz), 8.01 (1H, br s), 8.23 (1H, s), 8.38 (1H, d, J = 5.4 Hz), 9.76 (1H, br s).
Example 28
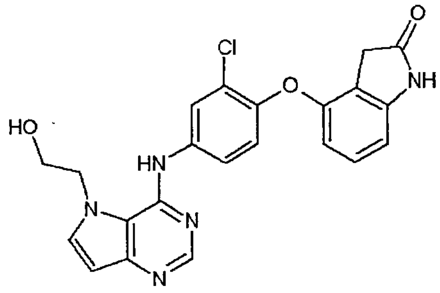

Production of 4-(2-chloro-4-{[5-(2-hydroxyethyl)-5H-pyrrolo[3,2-d]pyrimidin-4-yl]amino}phenoxy)-1,3-dihydro-2H-indol-2-one

(i) Production of 4-(2-chloro-4-nitrophenoxy)-1,3-dihydro-2H-indol-2-one

The title compound (460 mg) was obtained as a yellow powder in the same manner as in Example 26 (i) and using 4-hydroxy-1,3-dihydro-2H-indol-2-one (746 mg), 2-chloro-1-fluoro-4-nitrobenzene (878 mg), potassium carbonate (1.38 g) and N,N-dimethylformamide (10 mL).
$^1$H-NMR ($CDCl_3$) δ: 3.42 (2H, s), 6.65 (1H, d, J = 8.2 Hz), 6.78 (1H, d, J = 8.0 Hz), 6.95 (1H, d, J = 9.1 Hz), 7.23-7.31 (1H, m), 8.08 (1H, dd, J = 2.7 Hz, 9.1 Hz), 8.09 (1H, br s), 8.40 (1H, d, J = 2.7 Hz).

(ii) Production of 4-(4-amino-2-chlorophenoxy)-1,3-dihydro-2H-indol-2-one

The title compound (67 mg) was obtained as a yellow powder in the same manner as in Example 26 (iv) and using 4-(2-chloro-4-nitrophenoxy)-1,3-dihydro-2H-indol-2-one (122 mg), reduced iron (99 mg), calcium chloride (25 mg) and 10% water-containing ethanol (10 mL).
$^1$H-NMR ($CDCl_3$) δ: 3.46 (2H, s), 3.69 (2H, br s), 6.36 (1H, d, J = 7.7 Hz), 6.52-6.59 (2H, m), 6.77 (1H, d, J = 2.5 Hz), 6.90 (1H, d, J = 8.5 Hz), 7.09 (1H, d, J = 8.1 Hz), 7.65 (1H, br s).

(iii) Production of 4-(2-chloro-4-{[5-(2-hydroxyethyl)-5H-pyrrolo[3,2-d]pyrimidin-4-yl]amino}phenoxy)-1,3-dihydro-2H-indol-2-one

A mixture of 2-(4-chloro-5H-pyrrolo[3,2-d]pyrimidin-5-yl)ethyl benzoate (91 mg), 4-(4-amino-2-chlorophenoxy)-1,3-dihydro-2H-indol-2-one (67 mg) and isopropyl alcohol (3 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. Methanol (10 mL) and potassium carbonate (41 mg) were added to the residue and the mixture was stirred at room temperature overnight. Water was added to the reaction mixture, and the mixture was extracted with a mixed solvent of ethyl acetate and tetrahydrofuran. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80), and basic silica gel column chromatography (eluent, methanol:ethyl acetate=0:100→20:80). The object fraction was concentrated under reduced pressure. The residue was filtered with ethyl acetate to give the title compound (29 mg) as a white powder.
$^1$H-NMR (DMSO-$d_6$) δ: 3.37 (2H, s), 3.87 (2H, q, J = 4.4 Hz), 4.53 (2H, t, J = 4.3 Hz), 6.26-6.34 (2H, m), 6.50 (1H, d, J = 3.0 Hz), 6.59 (1H, d, J = 7.4 Hz), 7.13 (1H, t, J = 8.1 Hz), 7.20 (1H, d, J = 8.8 Hz), 7.56 (1H, dd, J = 2.6 Hz, 8.8 Hz), 7.65 (1H, d, J = 3.0 Hz), 7.95 (1H, d, J = 2.6 Hz), 8.32 (1H, s), 9.84 (1H, br s), 10.51 (1H, br s).
Example 29
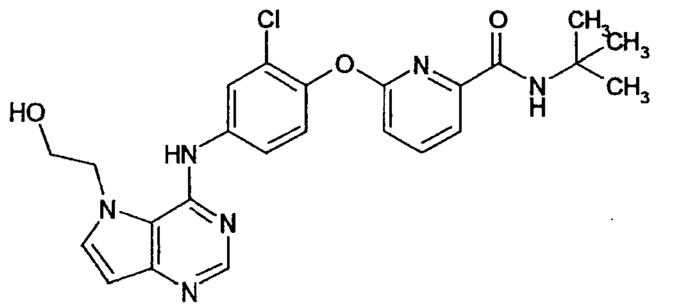

Production of N-(tert-butyl)-6-(2-chloro-4-{[5-(2-hydroxyethyl)-5H-pyrrolo[3,2-d]pyrimidin-4-yl]amino}phenoxy) pyridine-2-carboxamide

(i) Production of N-(tert-butyl)-6-(2-chloro-4-nitrophenoxy)pyridine-2-carboxamide

A mixture of 6-hydroxypicoline acid (1.39 g), tert-butylamine (3.15 mL), 1-ethyl-3-(3-dimethylaminopropyl)carbodiimide hydrochloride (2.30 g), 1-hydroxybenzotriazole (1.62 g), triethylamine (1.67 mL) and N,N-dimethylformamide (20 mL) was stirred at room temperature for 3 days. The reaction mixture was concentrated under reduced pressure, water was added, and the mixture was extracted with a mixed solvent of ethyl acetate and tetrahydrofuran. The organic layer was washed with saturated brine, and dried over anhydrous sodium sulfate. The solvent was evaporated under reduced pressure and the obtained residue was dissolved in N,N-dimethylformamide (20 mL). 2-Chloro-1-fluoro-4-nitrobenzene (1.40 g) and potassium carbonate (2.21 g) were added and the mixture was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=10:90→20:80). The object fraction was concentrated under reduced pressure to give the title compound (2.25 g) as a pale-yellow oil.
$^1$H-NMR ($CDCl_3$) δ: 1.33 (9H, s), 7.19 (1H, dd, J = 1.9 Hz, 7.1 Hz), 7.20 (1H, br s), 7.39 (1H, d, J = 8.9 Hz), 7.90-7.99 (2H, m), 8.21 (1H, dd, J = 2.7 Hz, 8.9 Hz), 8.43 (1H, d, J = 2.7 Hz).

(ii) Production of 6-(4-amino-2-chlorophenoxy)-N-(tert-butyl)pyridine-2-carboxamide

The title compound (1.59 g) was obtained as a yellow-orange solid in the same manner as in Example 26 (iv) and using N-(tert-butyl)-6-(2-chloro-4-nitrophenoxy)pyridine-2-carboxamide (2.24 g), reduced iron (1.19 g), calcium chloride (395 mg) and 10% water-containing ethanol (50 mL).
$^1$H-NMR ($CDCl_3$) δ: 1.37 (9H, s), 3.73 (2H, br s), 6.62 (1H, dd, J = 2.6 Hz, 8.7 Hz), 6.81 (1H, d, J = 2.6 Hz), 6.95 (1H, dd, J = 1.5 Hz, 7.5 Hz), 7.01 (1H, d, J = 8.7 Hz), 7.54 (1H, br s), 7.76-7.86 (2H, m).

(iii) Production of N-(tert-butyl)-6-(2-chloro-4-{[5-(2-hydroxyethyl)-5H-pyrrolo[3,2-d]pyrimidin-4-yl]amino}phenoxy)pyridine-2-carboxamide

The title compound (138 mg) was obtained as a white powder in the same manner as in Example 26 (v) and using 2-(4-chloro-5H-pyrrolo[3,2-d]pyrimidin-5-yl)ethyl benzoate (121 mg), 6-(4-amino-2-chlorophenoxy)-N-(tert-butyl) pyridine-2-carboxamide (128 mg), pyridine hydrochloride (5 mg), isopropyl alcohol (5 mL), methanol (5 mL), tetrahydrofuran (1 mL) and 1N aqueous sodium hydroxide solution (0.8 mL).
$^1$H-NMR ($CDCl_3$) δ: 1.38 (9H, s), 4.18 (2H, t, J = 4.3 Hz), 4.39 (2H, t, J = 4.3 Hz), 6.14 (1H, d, J = 3.2 Hz), 6.42 (1H, br s), 6.97 (1H, d, J = 3.2 Hz), 7.01 (1H, dd, J = 1.9 Hz, 7.2 Hz), 7.22 (1H, d, J = 8.7 Hz), 7.53-7.62 (2H, m), 7.78-7.90 (3H, m), 8.25 (1H, s), 9.65 (1H, br s).
Example 30
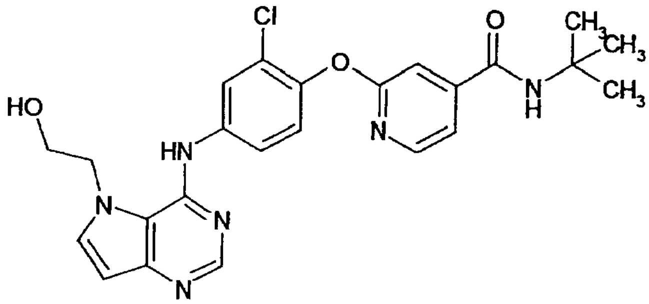

Production of N-(tert-butyl)-2-(2-chloro-4-{[5-(2-hydroxyethyl)-5H-pyrrolo[3,2-d]pyrimidin-4-yl]amino}phenoxy) isonicotinamide

(i) Production of N-(tert-butyl)-2-(2-chloro-4-nitrophenoxy)isonicotinamide

The title compound (503 mg) was obtained as a yellow powder in the same manner as in Example 29 (i) and using 2-hydroxyisonicotinic acid (1.39 g), tert-butylamine (3.15 mL), 1-ethyl-3-(3-dimethylaminopropyl)carbodiimide hydrochloride (2.30 g), 1-hydroxybenzotriazole (1.62 g), triethylamine (1.67 mL), N,N-dimethylformamide (20 mL), 2-chloro-1-fluoro-4-nitrobenzene (1.23 g), potassium carbonate (1.93 g) and N,N-dimethylformamide (20 mL).
$^1$H-NMR ($CDCl_3$) δ: 1.41 (9H, s), 6.30 (1H, br s), 6.73 (1H, dd, J = 1.7 Hz, 7.2 Hz), 6.95 (1H, m), 7.21 (1H, d, J = 7.2 Hz), 7.59 (1H, d, J = 8.7 Hz), 8.29 (1H, dd, J = 2.4 Hz, 8.7 Hz), 8.46 (1H, d, J = 2.4 Hz).

(ii) Production of 2-(4-amino-2-chlorophenoxy)-N-(tert-butyl)isonicotinamide

The title compound (300 mg) was obtained as an orange powder in the same manner as in Example 26 (iv) and using N-(tert-butyl)-2-(2-chloro-4-nitrophenoxy)isonicotinamide (500 mg), reduced iron (444 mg), calcium chloride (88 mg) and 10% water-containing ethanol (20 mL).
$^1$H-NMR ($CDCl_3$) δ: 1.41 (9H, s), 3.95 (2H, br s), 6.23 (1H, br s), 6.57-6.64 (2H, m), 6.78 (1H, d, J = 2.5 Hz), 6.86 (1H, d, J = 1.9 Hz), 7.04 (1H, d, J = 8.5 Hz), 7.21 (1H, d, J = 7.1 Hz).

(iii) Production of N-(tert-butyl)-2-(2-chloro-4-{[5-(2-hydroxyethyl)-5H-pyrrolo[3,2-d]pyrimidin-4-yl]amino}phenoxy)isonicotinamide

The title compound (80 mg) was obtained as a white powder in the same manner as in Example 26 (v) and using 2-(4-chloro-5H-pyrrolo[3,2-d]pyrimidin-5-yl)ethyl benzoate (121 mg), 2-(4-amino-2-chlorophenoxy)-N-(tert-butyl) isonicotinamide (128 mg), pyridine hydrochloride (5 mg), isopropyl alcohol (5 mL), methanol (5 mL), tetrahydrofuran (1 mL) and 1N aqueous sodium hydroxide solution (0.8 mL).
$^1$H-NMR (DMSO-$d_6$) δ: 1.37 (9H, s), 3.83-3.92 (2H, m), 4.54 (2H, t, J = 4.5 Hz), 6.32 (1H, t, J = 3.8 Hz), 6.53 (1H, d, J = 3.0 Hz), 6.56 (1H, dd, J = 1. 6 Hz, 7.1 Hz), 6.85 (1H, d, J = 1.6 Hz), 7.42 (1H, d, J = 8.5 Hz), 7.60-7.71 (3H, m), 7.97 (1H, d, J = 2.2 Hz), 8.04 (1H, br s), 8.37 (1H, s), 10.03 (1H, br s).
Example 31
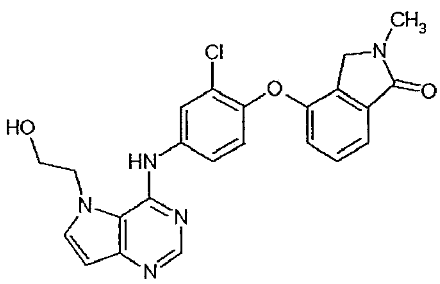

Production of 4-(2-chloro-4-{[5-(2-hydroxyethyl)-5H-pyrrolo[3,2-d]pyrimidin-4-yl]amino}phenoxy)-2-methylisoindolin-1-one

(i) Production of methyl 3-hydroxy-2-methylbenzoate

To a solution of 3-hydroxy-2-methylbenzoic acid (5.05 g) in methanol (100 mL) was added conc. sulfuric acid (2 mL) and the mixture was heated under reflux overnight. The reaction mixture was concentrated under reduced pressure, water was added, and the mixture was extracted with ethyl acetate. The organic layer was washed successively with saturated aqueous sodium hydrogen carbonate solution and saturated brine, and dried over anhydrous magnesium sulfate. The solvent was evaporated under reduced pressure and the obtained residue was subjected to silica gel column chromatography (eluent, ethyl acetate:hexane=10:90→20:80). The object fraction was concentrated under reduced pressure. The residue was filtered with hexane to give the title compound (4.18 g) as a white powder.
$^1$H-NMR ($CDCl_3$) δ: 2.45 (3H, s), 3.89 (3H, s), 5.11 (1H, s), 6.93 (1H, d, J = 7.8 Hz), 7.10 (1H, t, J = 7.8 Hz), 7.41 (1H, dd, J = 1.4 Hz, 7.8 Hz).

(ii) Production of methyl 3-(2-chloro-4-nitrophenoxy)-2-methylbenzoate

The title compound (7.72 g) was obtained as a pale-yellow powder in the same manner as in Example 26 (i) and using methyl 3-hydroxy-2-methylbenzoate (4.15 g), 2-chloro-1-fluoro-4-nitrobenzene (4.39 g), potassium carbonate (6.91 g) and N,N-dimethylformamide (50 mL).
$^1$H-NMR ($CDCl_3$) δ: 2.40 (3H, s), 3.93 (3H, s), 6.63 (1H, d, J = 9.1 Hz), 7.16 (1H, dd, J = 1.4 Hz, 8.0 Hz), 7.33 (1H, t, J = 7.8 Hz), 7.82 (1H, dd, J = 1.4 Hz, 8.0 Hz), 8.01 (1H, dd, J = 2.7 Hz, 9.1 Hz), 8.39 (1H, d, J = 2.7 Hz).

(iii) Production of methyl 2-(bromomethyl)-3-(2-chloro-4-nitrophenoxy)benzoate

To a solution of methyl 3-(2-chloro-4-nitrophenoxy)-2-methylbenzoate (4.82 g) in benzotrifluoride (50 mL) were added N-bromosuccinimide (3.20 g) and 2,2'-azobis(isobutyronitrile) (123 mg) and the mixture was stirred at 100°C overnight. The reaction mixture was filtered to remove solid, and the filtrate was washed successively with 1N aqueous sodium hydroxide solution and saturated brine, and dried over anhydrous magnesium sulfate. The solvent was evaporated under reduced pressure and the obtained residue was subjected to silica gel column chromatography (eluent, ethyl acetate:hexane=15:85→25:75). The object fraction was concentrated under reduced pressure and the residue was crystallized from ethyl acetate-hexane to give the title compound (4.21 g) as a white powder.
$^1$H-NMR ($CDCl_3$) δ: 3.98 (3H, s), 5.05 (2H, s), 6.92 (1H, d, J = 9.1 Hz), 7.08 (1H, dd, J = 1.2 Hz, 8.1 Hz), 7.42 (1H, t, J = 8.0 Hz), 7.86 (1H, dd, J = 1.2 Hz, 7.8 Hz), 8.08 (1H, dd, J = 2.6 Hz, 9.1 Hz), 8.41 (1H, d, J = 2.6 Hz).

(iv) Production of 4-(2-chloro-4-nitrophenoxy)-2-methylisoindolin-1-one

To a solution of methyl 2-(bromomethyl)-3-(2-chloro-4-nitrophenoxy)benzoate (601 mg) in acetonitrile (10 mL) was added 2M methylamine/tetrahydrofuran solution (3 mL) and the mixture was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=30:70→60:40).
The object fraction was concentrated under reduced pressure. The residue was filtered with diisopropyl ether to give the title compound (278 mg) as a pale-yellow powder.
$^1$H-NMR ($CDCl_3$) δ: 3.19 (3H, s), 4.32 (2H, s), 6.96 (1H, d, J = 9.0 Hz), 7.14 (1H, d, J = 7.9 Hz), 7.53 (1H, t, J = 7.7 Hz), 7.75 (1H, d, J = 7.3 Hz), 8.10 (1H, dd, J = 2.5 Hz, 9.0 Hz), 8.42 (1H, d, J = 2.5 Hz).

(v) Production of 4-(4-amino-2-chlorophenoxy)-2-methylisoindolin-1-one

The title compound (168 mg) was obtained as a colorless solid in the same manner as in Example 26 (iv) and using 4-(2-chloro-4-nitrophenoxy)-2-methylisoindolin-1-one (255 mg), reduced iron (199 mg), calcium chloride (49 mg) and 10% water-containing ethanol (10 mL).
$^1$H-NMR ($CDCl_3$) δ: 3.20 (3H, s), 3.73 (2H, br s), 4.37 (2H, s), 6.58 (1H, dd, J = 2.7 Hz, 8.7 Hz), 6.75-6.81 (2H, m), 6.92 (1H, d, J = 8.7 Hz), 7.32 (1H, t, J = 7.7 Hz), 7.51 (1H, d, J = 7.7 Hz).

(vi) Production of 4-(2-chloro-4-{[5-(2-hydroxyethyl)-5H-pyrrolo[3,2-d]pyrimidin-4-yl]amino}phenoxy)-2-methylisoindolin-1-one

A mixture of 2-(4-chloro-5H-pyrrolo[3,2-d]pyrimidin-5-yl)ethyl benzoate (151 mg), 4-(4-amino-2-chlorophenoxy)-2-methylisoindolin-1-one (144 mg) and isopropyl alcohol (5 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. Methanol (5 mL), tetrahydrofuran (1 mL) and 1N aqueous sodium hydroxide solution (1 mL) were added to the residue and the mixture was stirred at room temperature for 3 hr. Water was added to the reaction mixture, and the mixture was extracted with a mixed solvent of ethyl acetate and tetrahydrofuran. The organic layer was washed with saturated brine, and dried over anhydrous magnesium sulfate. The solvent was evaporated under reduced pressure and the obtained residue was subjected to basic silica gel column chromatography (eluent, methanol:ethyl acetate=5:95→35:65). The object fraction was concentrated under reduced pressure, and the residue was crystallized from ethanol/ethyl acetate to give the title compound (101 mg) as a white powder.
$^1$H-NMR (DMSO-$d_6$) δ: 3.09 (3H, s), 3.88 (2H, t, J = 4.5 Hz), 4.48 (2H, s), 4.54 (2H, t, J = 4.5 Hz), 6.31 (1H, br s), 6.52 (1H, d, J = 3.2 Hz), 6.90 (1H, dd, J = 1.9 Hz, 7.2 Hz), 7.29 (1H, d, J = 8.8 Hz), 7.40-7.49 (2H, m), 7.61 (1H, dd, J = 2.6 Hz, 8.8 Hz), 7.66 (1H, d, J = 3.2 Hz), 8.00 (1H, d, J = 2.6 Hz), 8.35 (1H, s), 9.89 (1H, br s).

Example 32

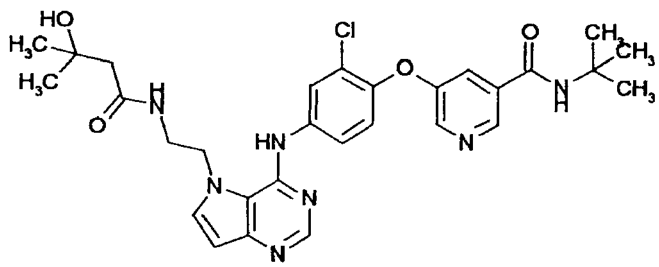

Production of N-(tert-butyl)-5-{2-chloro-4-[(5-{2-[(3-hydroxy-3-methylbutanoyl)amino]ethyl}-5H-pyrrolo[3,2-d]pyrimidin-4-yl)amino]phenoxy}nicotinamide

A mixture of tert-butyl [2-(4-chloro-5H-pyrrolo[3,2-d]pyrimidin-5-yl)ethyl]carbamate (121 mg), 5-(4-amino-2-chlorophenoxy)-N-(tert-butyl)nicotinamide (119 mg), pyridine hydrochloride (5 mg) and isopropyl alcohol (5 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. Ethanol (2 mL) and 4N hydrochloric acid/ethyl acetate (2 mL) were added to the residue, and the mixture was stirred at 60°C for 3 hr. The reaction mixture was concentrated under reduced pressure. To a solution of the residue in N,N-dimethylformamide (5 mL) were added 3-hydroxy-3-methylbutanoic acid (71 mg), 1-ethyl-3-(3-dimethylaminopropyl)carbodiimide hydrochloride (115 mg), 1-hydroxybenzotriazole (81 mg) and triethylamine (0.223 mL), and the mixture was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to basic silica gel column chromatography (eluent, methanol:ethyl acetate=0:100→2.0:80) The object fraction was concentrated under reduced pressure, and the residue was crystallized from ethyl acetate to give the title compound (137 mg) as a white powder.
$^1$H-NMR (DMSO-$d_6$) δ: 1.13 (6H, s), 1.36 (9H, s), 2.20 (2H, s), 3.41 (2H, q, J = 6.5 Hz), 4.52 (2H, t, J = 7.0 Hz), 4.66 (1H, s), 6.49 (1H, d, J = 3.2 Hz), 7.28 (1H, d, J = 8.9 Hz), 7.56 (1H, dd, J = 1.7 Hz, 2.8 Hz), 7.64 (1H, d, J = 3.2 Hz), 7.83 (1H, dd, J = 2.5 Hz, 8.9 Hz), 8.03-8.09 (2H, m), 8.24 (1H, t, J = 5.7 Hz), 8.33 (1H, s), 8.47 (1H, d, J = 2.8 Hz), 8.73 (1H, d, J = 1.7 Hz), 8.90 (1H, brs).

Example 33

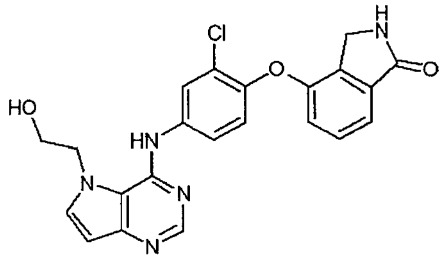

Production of (4-(2-chloro-4-{[5-(2-hydroxyethyl)-5H-pyrrolo[3,2-d]pyrimidin-4-yl]amino}phenoxy)isoindolin-1-one

(i) Production of 4-(2-chloro-4-nitrophenoxy)isoindolin-1-one

A mixture of methyl 2-(bromomethyl)-3-(2-chloro-4-nitrophenoxy)benzoate (601 mg), 28% aqueous ammonia (3 mL), tetrahydrofuran (27 mL) and methanol (1 mL) was stirred at room temperature overnight. The reaction mixture was concentrated under reduced pressure, and water, ethyl acetate and diethyl ether were added. The precipitated solid was collected by filtration, and washed successively with water and diethyl ether to give the title compound (500 mg) as a white powder.
$^1$H-NMR (DMSO-$d_6$) δ: 4.28 (2H, s), 7.16 (1H, d, J = 9.1 Hz), 7.32 (1H, dd, J = 1.4 Hz, 7.1 Hz), 7.54-7.65 (2H, m), 8.17 (1H, dd, J = 2.7 Hz, 9.1 Hz), 8.49 (1H, d, J = 2.7 Hz), 8.74 (1H, br s).

(ii) Production of 4-(4-amino-2-chlorophenoxy)isoindolin-1-one

To a solution of 4-(2-chloro-4-nitrophenoxy)isoindolin-1-one (948 mg) in ethyl acetate (20 mL)/tetrahydrofuran (20 mL)/methanol (20 mL) was added 5% platinum/activated carbon (25 mg) under hydrogen stream, and the mixture was stirred at room temperature overnight. The catalyst was filtered off, the filtrate was concentrated and the obtained residue was subjected to silica gel column chromatography (eluent, ethyl acetate:hexane=80:20→100:0→methanol: ethyl acetate=20:80). The object fraction was concentrated under reduced pressure. The residue was filtered with ethanol/ethyl acetate to give the title compound (390 mg) as a pale-brown powder.
$^1$H-NMR ($CDCl_3$) δ: 3.73 (2H, br s), 4.45 (2H, s), 6.58 (1H, dd, J = 2.7 Hz, 8.7 Hz), 6.75-6.84 (3H, m), 6.94 (1H, d, J = 8.7 Hz), 7.35 (1H, t, J = 7.8 Hz), 7.54 (1H, d, J = 7.7 Hz).

(iii) Production of 4-(2-chloro-4-{[5-(2-hydroxyethyl)-5H-pyrrolo[3,2-d]pyrimidin-4-yl]amino}phenoxy)isoindolin-1-one

A mixture of 2-(4-chloro-5H-pyrrolo[3,2-d]pyrimidin-5-yl)ethyl benzoate (151 mg), 4-(4-amino-2-chlorophenoxy) isoindolin-1-one (137 mg) and isopropyl alcohol (3 mL) was stirred at 80°C overnight. Saturated aqueous sodium hydrogen carbonate solution and ethyl acetate were added to the reaction mixture. The precipitated solid was collected by filtration, and washed with water and ethyl acetate. Methanol (5 mL), tetrahydrofuran (1 mL) and 1N aqueous sodium hydroxide solution (1 mL) were added to the obtained solid, and the mixture was stirred at room temperature overnight. The reaction mixture was concentrated under reduced pressure and water and ethyl acetate were added. The precipitated solid was collected by filtration and washed successively with water and ethyl acetate to give the title compound (213 mg) as a white powder.
$^1$H-NMR (DMSO-$d_6$) δ: 3.88 (2H, t, J = 4.5 Hz), 4.35 (2H, s), 4.53 (2H, t, J = 4.5 Hz), 6.32 (1H, br s), 6.51 (1H, d, J = 3.0 Hz), 6.89 (1H, dd, J = 1.8 Hz, 7.0 Hz), 7.30 (1H, d, J = 8.7 Hz), 7.39-7.48 (2H, m), 7.60 (1H, dd, J = 2.5 Hz, 8.7 Hz), 7.66 (1H, d, J = 3.0 Hz), 7.99 (1H, d, J = 2.5 Hz), 8.34 (1H, s), 8.68 (1H, br s), 9.88 (1H, br s).
Example 34
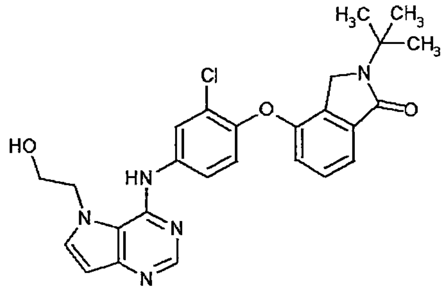

Production of 2-tert-butyl-4-(2-chloro-4-{[5-(2-hydroxyethyl)-5H-pyrrolo[3,2-d]pyrimidin-4-yl]amino}phenoxy) isoindolin-1-one

(i) Production of 2-tert-butyl-4-(2-chloro-4-nitrophenoxy)isoindolin-1-one

A mixture of methyl 2-(bromomethyl)-3-(2-chloro-4-nitrophenoxy)benzoate (801 mg), tert-butylamine (0.315 mL), potassium carbonate (415 mg) and acetonitrile (10 mL) was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure, the obtained residue was dissolved in toluene (5 mL), and the solution was stirred at 100°C for 6 hr. The reaction mixture was subjected to silica gel column chromatography (eluent, ethyl acetate:hexane=15:85→35:65). The object fraction was concentrated under reduced pressure to give the title compound (492 mg) as a white powder.
$^1$H-NMR ($CDCl_3$) δ: 1.56 (9H, s), 4.41 (2H, s), 6.96 (1H, d, J = 9.1 Hz), 7.06 (1H, d, J = 8.0 Hz), 7.48 (1H, t, J = 7.6 Hz), 7.69 (1H, d, J = 7.4 Hz), 8.10 (1H, dd, J = 2.7 Hz, 9.1 Hz), 8.41 (1H, d, J = 2.7 Hz).

(ii) Production of 4-(4-amino-2-chlorophenoxy)-2-tert-butylisoindolin-1-one

The title compound (313 mg) was obtained as a white powder in the same manner as in Example 33 (ii) and using 2-tert-butyl-4-(2-chloro-4-nitrophenoxy)isoindolin-1-one (451 mg), ethyl acetate (5 mL) and 5% platinum/activated carbon (22 mg).
$^1$H-NMR ($CDCl_3$) δ: 1.58 (9H, s), 3.74 (2H, br s), 4.49 (2H, s), 6.58 (1H, dd, J = 2.7 Hz, 8.7 Hz), 6.70 (1H, dd, J = 0.8 Hz, 8.0 Hz), 6.79 (1H, d, J = 2.7 Hz), 6.92 (1H, d, J = 8.7 Hz), 7.28 (1H, t, J = 8.0 Hz), 7.45 (1H, dd, J = 0.8 Hz, 7.4 Hz).

(iii) Production of 2-tert-butyl-4-(2-chloro-4-{[5-(2-hydroxyethyl)-5H-pyrrolo[3,2-d]pyrimidin-4-yl]amino}phenoxy)isoindolin-1-one

The title compound (215 mg) was obtained as a white powder in the same manner as in Example 31 (vi) and using 2-(4-chloro-5H-pyrrolo[3,2-d]pyrimidin-5-yl)ethyl benzoate (151 mg), 4-(4-amino-2-chlorophenoxy)-2-tert-butylisoindolin-1-one (165 mg), isopropyl alcohol (3 mL), methanol (5 mL), tetrahydrofuran (1 mL) and 1N aqueous sodium hydroxide solution (1 mL).
$^1$H-NMR (DMSO-$d_6$) δ: 1.52 (9H, s), 3.88 (2H, t, J = 4.5 Hz), 4.53 (2H, t, J = 4.5 Hz), 4.63 (2H, s), 6.30 (1H, br s), 6.51 (1H, d, J = 3.0 Hz), 6.79 (1H, dd, J = 0.8 Hz, 8.0 Hz), 7.29 (1H, d, J = 8.8 Hz), 7.31-7.36 (1H, m), 7.37-7.44 (1H, m), 7.61 (1H, dd, J = 2.6 Hz, 8.8 Hz), 7.66 (1H, d, J = 3.0 Hz), 7.99 (1H, d, J = 2.6 Hz), 8.34 (1H, s), 9.88 (1H, br s).
Example 35
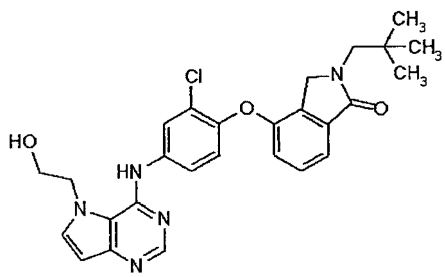

Production of 4-(2-chloro-4-{[5-(2-hydroxyethyl)-5H-pyrrolo[3,2-d]pyrimidin-4-yl]amino}phenoxy)-2-(2,2-dimethylpropyl)isoindolin-1-one

(i) Production of 4-(2-chloro-4-nitrophenoxy)-2-(2,2-dimethylpropyl)isoindolin-1-one

A mixture of methyl 2-(bromomethyl)-3-(2-chloro-4-nitrophenoxy)benzoate (601 mg), neopentylamine (261 mg), potassium carbonate (415 mg) and acetonitrile (10 mL) was stirred at room temperature for 6 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=15:85→30:70). The object fraction was concentrated under reduced pressure to give the title compound (467 mg) as a pale-yellow solid.
$^1$H-NMR ($CDCl_3$) δ: 1.02 (9H, s), 3.38 (2H, s), 4.46 (2H, s), 6.97 (1H, d, J = 9.1 Hz), 7.10 (1H, dd, J = 0.8 Hz, 8.0 Hz), 7.51 (1H, t, J = 7.8 Hz), 7.75 (1H, dd, J = 0.8 Hz, 7.4 Hz), 8.10 (1H, dd, J = 2.7 Hz, 9.1 Hz), 8.42 (1H, d, J = 2.7 Hz).

(ii) Production of 4-(4-amino-2-chlorophenoxy)-2-(2,2-dimethylpropyl)isoindolin-1-one

The title compound (313 mg) was obtained as a white powder in the same manner as in Example 33 (ii) and using 4-(2-chloro-4-nitrophenoxy)-2-(2,2-dimethylpropyl)isoindolin-1-one (467 mg), ethyl acetate (10 mL) and 5% platinum/activated carbon (30 mg).
$^1$H-NMR ($CDCl_3$) δ: 1.04 (9H, s), 3.39 (2H, s), 3.74 (2H, br s), 4.53 (2H, s), 6.58 (1H, dd, J = 2.7 Hz, 8.5 Hz), 6.75 (1H, d, J = 8.0 Hz), 6.79 (1H, d, J = 2.7 Hz), 6.93 (1H, d, J = 8.5 Hz), 7.31 (1H, t, J = 7.8 Hz), 7.52 (1H, d, J = 7.4 Hz).

(iii) Production of 4-(2-chloro-4-{[5-(2-hydroxyethyl)-5H-pyrrolo[3,2-d]pyrimidin-4-yl]amino}phenoxy)-2-(2,2-dimethylpropyl)isoindolin-1-one

The title compound (203 mg) was obtained as a white powder in the same manner as in Example 31 (vi) and using 2-(4-chloro-5H-pyrrolo[3,2-d]pyrimidin-5-yl)ethyl benzoate (151 mg), 4-(4-amino-2-chlorophenoxy)-2-(2,2-dimethylpropyl)isoindolin-1-one (172 mg), isopropyl alcohol (3 mL), methanol (5 mL), tetrahydrofuran (1 mL) and 1N aqueous sodium hydroxide solution (1 mL).
$^1$H-NMR (DMSO-$d_6$) δ: 0.97 (9H, s), 3.34 (2H, s), 3.83-3.93 (2H, m), 4.53 (2H, t, J = 4.4 Hz), 4.59 (2H, s), 6.31 (1H, br s), 6.51 (1H, d, J = 3.2 Hz), 6.87 (1H, dd, J = 2. 5 Hz, 6.6 Hz), 7.32 (1H, d, J = 8.9 Hz), 7.40-7.49 (2H, m), 7.60 (1H, dd, J = 2.5 Hz, 8.9 Hz), 7.66 (1H, d, J = 3.2 Hz), 7.99 (1H, d, J = 2.5 Hz), 8.34 (1H, s), 9.88 (1H, br s).
Example 36
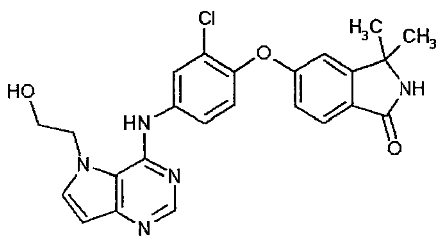

Production of 5-(2-chloro-4-{[5-(2-hydroxyethyl)-5H-pyrrolo[3,2-d]pyrimidin-4-yl]amino}phenoxy)-3,3-dimethylisoindolin-1-one

(i) Production of 5-hydroxy-3,3-dimethylisoindolin-1-one

A mixture of 5-methoxy-3,3-dimethylisoindolin-1-one (1.20 g) and 48% hydrobromic acid (12 mL) was stirred at 90°C for 17 hr and then at 100°C for 50 hr. The mixture was allowed to cool to room temperature, ethyl acetate and saturated brine were added, and the mixture was extracted 6 times with ethyl acetate. The organic layer was dried over anhydrous magnesium sulfate and concentrated under reduced pressure. The residue was separated and purified by silica gel column chromatography (eluent, ethyl acetate:hexane=80:20→100:0) to give the title compound (1.36 g) as an orange amorphous form.
$^1$H-NMR (DMSO-$d_6$) δ: 1.38 (6H, s), 6.79 (1H, d, J = 8.0 Hz), 6.85 (1H, s), 7.38 (1H, d, J = 8.0 Hz), 8.26 (1H, s), 10.08 (1H, br s).

(ii) Production of 5-(2-chloro-4-nitrophenoxy)-3,3-dimethylisoindolin-1-one

To a solution of 2-chloro-1-fluoro-4-nitrobenzene (1.28 g) and 5-hydroxy-3,3-dimethylisoindolin-1-one (1.11 g) in N,N-dimethylformamide (15 mL) was added potassium carbonate (1.04 g) under ice-cooling, and the mixture was stirred at room temperature for 8 hr. Saturated brine was added to the reaction system under ice-cooling, and the mixture was extracted twice with ethyl acetate/tetrahydrofuran, and the organic layer was dried over anhydrous magnesium sulfate, and concentrated under reduced pressure. The obtained crystals were recrystallized from ethyl acetate/tetrahydrofuran to give the title compound (877 mg) as colorless crystals.
$^1$H-NMR ($CDCl_3$) δ: 1.56 (6H, s), 6.20 (1H, br s), 7.02 (1H, d, J = 9.3 Hz), 7.05-7.10 (2H, m), 7.84 (1H, dd, J = 1.2 Hz, 7.8 Hz), 8.11 (1H, dd, J = 3.0 Hz, 9.3 Hz), 8.41 (1H, d, J = 2.4 Hz).

(iii) Production of 5-(4-amino-2-chlorophenoxy)-3,3-dimethylisoindolin-1-one

To a solution of 5-(2-chloro-4-nitrophenoxy)-3,3-dimethylisoindolin-1-one (333 mg) in ethyl acetate (5 mL)/ methanol (20 mL) was added 5% platinum/activated carbon (55 mg) under nitrogen atmosphere. The reaction mixture was stirred at room temperature for 1 hr under hydrogen atmosphere, and 5% platinum/activated carbon was filtered off. The filtrate was concentrated under reduced pressure to give the title compound (301 mg) as a pale-pink powder.
$^1$H-NMR ($CDCl_3$) δ: 1.50 (6H, s), 3.74 (2H, br s), 6.07 (1H, br s), 6.60 (1H, dd, J = 2.7 Hz, 8.7 Hz), 6.80 (1H, d, J = 2.7 Hz), 6.85-6.90 (2H, m), 6.94 (1H, d, J = 8.7 Hz), 7.69 (1H, d, J = 8.1 Hz).

(iv) Production of 5-(2-chloro-4-{[5-(2-hydroxyethyl)-5H-pyrrolo[3,2-d]pyrimidin-4-yl]amino}phenoxy)-3,3-dimethylisoindolin-1-one

A mixture of 2-(4-chloro-5H-pyrrolo[3,2-d]pyrimidin-5-yl)ethyl benzoate (272 mg), 5-(4-amino-2-chlorophenoxy)-3,3-dimethylisoindolin-1-one (301 mg) and isopropyl alcohol (20 mL) was stirred at 80°C for 16 hr, and then at 90°C for 2 hr. Under ice-cooling, saturated aqueous sodium hydrogen carbonate solution was added to the reaction system, and the mixture was extracted with ethyl acetate. The extract was washed with saturated brine, dried over anhydrous magnesium sulfate and concentrated under reduced pressure. The residue was separated and purified by silica gel column chromatography (eluent, ethyl acetate:hexane=80:20→100:0→ethyl acetate:methanol=98:2). 1N Aqueous sodium hydroxide solution (2.0 mL) and tetrahydrofuran (4.0 mL) were added to the obtained compound, and the mixture was stirred at room temperature for 7 hr. The reaction system was neutralized with 1N hydrochloric acid, and saturated aqueous sodium hydrogen carbonate solution and saturated brine were added. The mixture was extracted with ethyl acetate, and the organic layer was dried over anhydrous magnesium sulfate and concentrated under reduced pressure. The obtained crystals were recrystallized from ethyl acetate/methanol to give the title compound (184 mg) as pale-yellow crystals.
$^1$H-NMR (DMSO-$d_6$) δ: 1.42 (6H, s), 3.80-3.90 (2H, m), 4.50-4.60 (2H, m), 6.32 (1H, br s), 6.51 (1H, d, J = 3.0 Hz), 6.80-6.90 (1H, m), 7.20-7.25 (1H, m), 7.28 (1H, d, J = 9.0 Hz), 7.56 (1H, d, J = 8.4 Hz), 7.55-7.70 (2H, m), 7.99 (1H, s), 8.34 (1H, s), 8.52 (1H, s), 9.90 (1H, br s).
Example 37
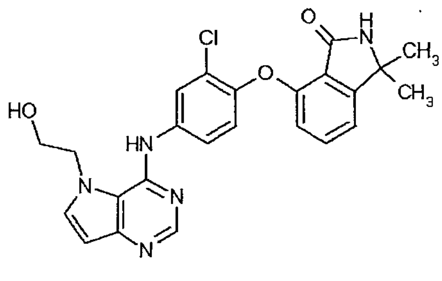

Production of 7-(2-chloro-4-{[5-(2-hydroxyethyl)-5H-pyrrolo[3,2-d]pyrimidin-4-yl]amino}phenoxy)-3,3-dimethylisoindolin-1-one

(i) Production of 7-hydroxy-3,3-dimethylisoindolin-1-one

The title compound (0.34 g) was obtained as a white solid by reaction in the same manner as in Example 36

(i) and using 7-methoxy-3,3-dimethylisoindolin-1-one (1.85 g) and 48% hydrobromic acid (20 mL).
$^1H$-NMR ($CDCl_3$) δ: 1.54 (6H, s), 6.22 (1H, br s), 6.83 (1H, d, J = 7.8 Hz), 6.84 (1H, d, J = 7.8 Hz), 7.42 (1H, t, J = 7.8 Hz), 8.50 (1H, br s).

(ii) Production of 7-(2-chloro-4-nitrophenoxy)-3,3-dimethylisoindolin-1-one

The title compound (473 mg) was obtained as colorless crystals by reaction in the same manner as in Example 36 (ii) and using 2-chloro-1-fluoro-4-nitrobenzene (390 mg), 7-hydroxy-3,3-dimethylisoindolin-1-one (0.34 g), potassium carbonate (318 mg) and N,N-dimethylformamide (4.0 mL).
$^1H$-NMR ($CDCl_3$) δ: 1.57 (6H, s), 5.99 (1H, br s), 6.85 (1H, d, J = 9.0 Hz), 7.00 (1H, d, J = 7.8 Hz), 7.25-7.30 (1H, m), 7.60 (1H, t, J = 7.8 Hz), 8.03 (1H, dd, J = 2.7 Hz, 9.0 Hz), 8.39 (1H, d, J = 2.7 Hz).

(iii) Production of 7-(4-amino-2-chlorophenoxy)-3,3-dimethylisoindolin-1-one

The title compound (176 mg) was obtained as a pale-pink amorphous by reaction in the same manner as in Example 36 (iii) and using 7-(2-chloro-4-nitrophenoxy)-3,3-dimethylisoindolin-1-one (183 mg), 5% platinum/activated carbon (31 mg) and ethyl acetate (5 mL)/methanol (10 mL).
$^1H$-NMR ($CDCl_3$) δ: 1.55 (6H, s), 3.69 (2H, br s), 5.92 (1H, br s), 6.49 (1H, d, J = 8.1 Hz), 6.57 (1H, dd, J = 2.7 Hz, 8.4 Hz), 6.77 (1H, d, J = 2.7 Hz), 6.98 (1H, d, J = 7.2 Hz), 7.00 (1H, d, J = 8.4 Hz), 7.35 (1H, t, J = 8.0 Hz).

(iv) Production of 7-(2-chloro-4-{[5-(2-hydroxyethyl)-5H-pyrrolo[3,2-d]pyrimidin-4-yl]amino}phenoxy)-3,3-dimethylisoindolin-1-one

The title compound (112 mg) was obtained as pale-yellow crystals by reaction in the same manner as in Example 36 (iv) and using 2-(4-chloro-5H-pyrrolo[3,2-d]pyrimidin-5-yl)ethyl benzoate (151 mg), 7-(4-amino-2-chlorophenoxy)-3,3-dimethylisoindolin-1-one (167 mg), isopropyl alcohol (10 mL), 1N aqueous sodium hydroxide solution (2.0 mL) and tetrahydrofuran (4.0 mL).
$^1H$-NMR (DMSO-$d_6$) δ: 1.45 (6H, s), 3.80-3.90 (2H, m), 4.50-4.60 (2H, m), 6.30 (1H, br s), 6.50 (1H, d, J = 3.0 Hz), 6.55 (1H, d, J = 8.4 Hz), 7.15 (1H, d, J = 8.7 Hz), 7.29 (1H, d, J = 7.2 Hz), 7.45-7.60 (2H, m), 7.64 (1H, d, J = 3.0 Hz), 7.96 (1H, d, J = 2.4 Hz), 8.32 (1H, s), 8.51 (1H, s), 9.80 (1H, br s).
Example 38
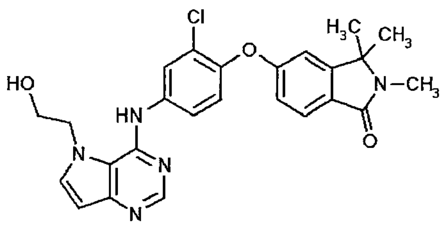

Production of 5-(2-chloro-4-{[5-(2-hydroxyethyl)-5H-pyrrolo[3,2-d]pyrimidin-4-yl]amino}phenoxy)-2,3,3-trimethylisoindolin-1-one

(i) Production of 5-(2-chloro-4-nitrophenoxy)-2,3,3-trimethylisoindolin-1-one

To a solution of 5-(2-chloro-4-nitrophenoxy)-3,3-dimethylisoindolin-1-one (333 mg) in N,N-dimethylformamide (6.0 mL) was added sodium hydride (44 mg, 60% in oil) under ice-cooling. After stirring at room temperature for 20 min, methyl iodide (0.25 mL) was added under ice-cooling, and the mixture was stirred at room temperature for 1.5 hr. Under ice-cooling, water (12 mL) was added, and the precipitate was collected by filtration and washed with water and diethyl ether to give the title compound (322 mg) as colorless crystals.
$^1H$-NMR ($CDCl_3$) δ: 1.47 (6H, s), 3.04 (3H, s), 6.99 (1H, d, J = 9.0 Hz), 7.05-7.10 (2H, m), 7.86 (1H, d, J = 9.0 Hz), 8.10 (1H, dd, J = 2.7 Hz, 9.0 Hz), 8.41 (1H, d, J = 2.7 Hz).

(ii) Production of 5-(4-amino-2-chlorophenoxy)-2,3,3-trimethylisoindolin-1-one

The title compound (170 mg) was obtained as a white amorphous by reaction in the same manner as in Example

36(iii) and using 5-(2-chloro-4-nitrophenoxy)-2,3,3-trimethylisoindolin-1-one (173 mg), 5% platinum/activated carbon (29 mg) and ethyl acetate (5 mL)/methanol (10 mL).
$^1$H-NMR ($CDCl_3$) δ: 1.42 (6H, s), 3.00 (3H, s), 3.72 (2H, br s), 6.59 (1H, dd, J = 3.0 Hz, 8.7 Hz), 6.80 (1H, d, J = 3.0 Hz), 6.85-6.90 (2H, m), 6.93 (1H, d, J = 8.7 Hz), 7.70 (1H, d, J = 8.7 Hz).

(iii) Production of 5-(2-chloro-4-{[5-(2-hydroxyethyl)-5H-pyrrolo[3,2-d]pyrimidin-4-yl]amino}phenoxy)-2,3,3-trimethylisoindolin-1-one

A mixture of 2-(4-chloro-5H-pyrrolo[3,2-d]pyrimidin-5-yl)ethyl benzoate (151 mg), 5-(4-amino-2-chlorophenoxy)-2,3,3-trimethylisoindolin-1-one (158 mg) and isopropyl alcohol (10 mL) was stirred at 80°C for 15 hr, pyridine hydrochloride (58 mg) was added, and the mixture was stirred at 80°C for 6 hr. Under ice-cooling, saturated aqueous sodium hydrogen carbonate solution was added to the reaction system, and the mixture was extracted with ethyl acetate. The extract was washed with saturated brine, and the organic layer was dried over anhydrous magnesium sulfate and concentrated under reduced pressure. The residue was separated and purified by silica gel column chromatography (eluent, ethyl acetate:methanol=100:0→98:2→95:5). 1N Aqueous sodium hydroxide solution (2.0 mL) and tetrahydrofuran (4.0 mL) were added to the obtained compound, and the mixture was stirred at room temperature for 9.5 hr. The reaction system was neutralized with 1N hydrochloric acid, and saturated aqueous sodium hydrogen carbonate solution and saturated brine were added. The mixture was extracted with ethyl acetate, and the organic layer was dried over anhydrous magnesium sulfate and concentrated under reduced pressure. The obtained crystals were recrystallized from ethyl acetate/diisopropyl ether to give the title compound (119 mg) as colorless crystals.
$^1$H-NMR (DMSO-$d_6$) δ: 1.42 (6H, s), 2.90 (3H, s), 3.85-3.90 (2H, m), 4.50-4.60 (2H, m), 6.32 (1H, br s), 6.51 (1H, d, J = 3.0 Hz), 6.86 (1H, d, J = 8.1 Hz), 7.29 (1H, d, J = 8.7 Hz), 7.30-7.35 (1H, m), 7.55-7.70 (3H, m), 8.00 (1H, d, J = 2.1 Hz), 8.34 (1H, s), 9.89 (1H, br s).
Example 39
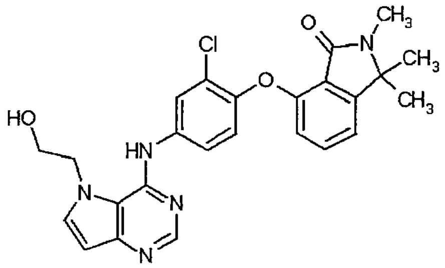

Production of 7-(2-chloro-4-{[5-(2-hydroxyethyl)-5H-pyrrolo[3,2-d]pyrimidin-4-yl]amino}phenoxy)-2,3,3-trimethylisoindolin-1-one

(i) Production of 7-(2-chloro-4-nitrophenoxy)-2,3,3-trimethylisoindolin-1-one

The title compound (246 mg) was obtained as pale-yellow crystals by reaction in the same manner as in Example 38(i) and using 7-(2-chloro-4-nitrophenoxy)-3,3-dimethylisoindolin-1-one (284 mg), 60% sodium hydride in oil (38 mg), methyl iodide (0.21 mL) and N,N-dimethylformamide (5.0 mL).
$^1$H-NMR ($CDCl_3$) δ: 1.48 (6H, s), 2.93 (3H, s), 6.73 (1H, d, J = 9.0 Hz), 7.09 (1H, d, J = 7.8 Hz), 7.34 (1H, d, J = 7.8 Hz), 7.60 (1H, t, J = 7.8 Hz), 7.99 (1H, dd, J = 2.7 Hz, 9.0 Hz), 8.39 (1H, d, J = 2.7 Hz).

(ii) Production of 7-(4-amino-2-chlorophenoxy)-2,3,3-trimethylisoindolin-1-one

The title compound (175 mg) was obtained as a white amorphous by reaction in the same manner as in Example 36(iii) and using 7-(2-chloro-4-nitrophenoxy)-2,3,3-trimethylisoindolin-1-one (173 mg), 5% platinum/activated carbon (29 mg) and ethyl acetate (5 mL)/methanol (10 mL).
$^1$H-NMR ($CDCl_3$) δ: 1.46 (6H, s), 3.01 (3H, s), 3.67 (2H, br s), 6.51 (1H, d, J = 8.1 Hz), 6.56 (1H, dd, J = 2.7 Hz, 8.4 Hz), 6.77 (1H, d, J = 2.7 Hz), 6.95 (1H, d, J = 8.4 Hz), 7.01 (1H, d, J = 7.5 Hz), 7.32 (1H, t, J = 7.5 Hz).

(iii) Production of 7-(2-chloro-4-{[5-(2-hydroxyethyl)-5H-pyrrolo[3,2-d]pyrimidin-4-yl]amino}phenoxy)-2,3,3-trimethylisoindolin-1-one

The title compound (94 mg) was obtained as pale-yellow crystals by reaction in the same manner as in Example 38(iii) and using 2-(4-chloro-5H-pyrrolo[3,2-d]pyrimidin-5-yl)ethyl benzoate (151 mg), 7-(4-amino-2-chlorophenoxy)-2,3,3-trimethylisoindolin-1-one (158 mg), pyridine hydrochloride (5.4 mg), isopropyl alcohol (10 mL), 1N aqueous sodium hydroxide solution (2.0 mL) and tetrahydrofuran (4.0 mL).
$^1$H-NMR (DMSO-$d_6$) δ: 1.45 (6H, s), 2.89 (3H, s), 3.85-3.90 (2H, m), 4.50-4.60 (2H, m), 6.30 (1H, br s), 6.50 (1H, d, J = 3.3 Hz), 6.59 (1H, d, J = 7.8 Hz), 7.14 (1H, d, J = 9.0 Hz), 7.37 (1H, d, J = 7.8 Hz), 7.50 (1H, t, J = 7.8 Hz), 7.56 (1H, dd, J = 2.7 Hz, 9.0 Hz), 7.64 (1H, d, J = 3.0 Hz), 7.96 (1H, d, J = 2.4 Hz), 8.32 (1H, s), 9.80 (1H, br s).
Example 40
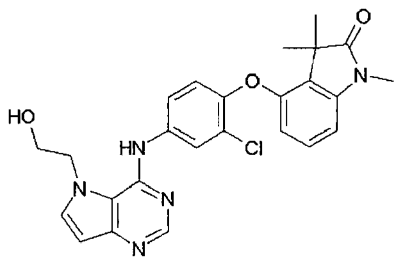

Production of 4-(2-chloro-4-{[5-(2-hydroxyethyl)-5H-pyrrolo[3,2-d]pyrimidin-4-yl]amino}phenoxy)-1,3,3-trimethyl-1,3-dihydro-2H-indol-2-one

(i) Production of 4-methoxy-1,3,3-trimethyl-1,3-dihydro-2H-indol-2-one

The title compound (629 mg) was obtained as an orange oil by reaction in the same manner as in Example 9 (i) and using 4-methoxy-1,3-dihydro-2H-indol-2-one (799 mg), 1.6M n-butyllithiumhexane solution (12 mL), N,N,N',N'-tetramethylethylenediamine (3 mL), iodomethane (3 mL) and tetrahydrofuran (25 mL).
$^1$H-NMR ($CDCl_3$) δ: 1.43 (6H, s), 3.19 (3H, s), 3.85 (3H, s), 6.50 (1H, d, J = 7.7 Hz), 6.61 (1H, d, J = 8.3 Hz), 7.17-7.24 (1H, m).

(ii) Production of 4-hydroxy-1,3,3-trimethyl-1,3-dihydro-2H-indol-2-one

The title compound (437 mg) was obtained as a pale-orange powder by reaction in the same manner as in Example 8(ii) and using 4-methoxy-1,3,3-trimethyl-1,3-dihydro-2H-indol-2-one (625 mg) and 48% hydrobromic acid (7 mL).
$^1$H-NMR ($CDCl_3$) δ: 1.48 (6H, s), 3.20 (3H, s), 5.09 (1H, s), 6.43-6.51 (2H, m), 7.12 (1H, t, J = 8.0 Hz).

(iii) Production of 4-(2-chloro-4-nitrophenoxy)-1,3,3-trimethyl-1,3-dihydro-2H-indol-2-one

The title compound (784 mg) was obtained as pale-yellow crystals by reaction in the same manner as in Example 2(ii) and using 4-hydroxy-1,3,3-trimethyl-1,3-dihydro-2H-indol-2-one (433 mg), 2-chloro-1-fluoro-4-nitrobenzene (600 mg), potassium carbonate (600 mg) and N,N-dimethylformamide (10 mL).
$^1$H-NMR ($CDCl_3$) δ: 1.42 (6H, s), 3.25 (3H, s), 6.58 (1H, d, J = 8.3 Hz), 6.76 (1H, d, J = 8.0 Hz), 6.95 (1H, d, J = 9.1 Hz), 7.26-7.34 (1H, m), 8.07 (1H, dd, J = 2.8 Hz, 9.1 Hz), 8.40 (1H, d, J = 2.8 Hz).

(iv) Production of 4-(4-amino-2-chlorophenoxy)-1,3,3-trimethyl-1,3-dihydro-2H-indol-2-one

The title compound (556 mg) was obtained as white crystals by reaction in the same manner as in Example 6 (ii) and using 4-(2-chloro-4-nitrophenoxy)-1,3,3-trimethyl-1,3-dihydro-2H-indol-2-one (781 mg), 5% platinum/activated carbon (77.0 mg) and methanol (15 mL)/ethyl acetate (15 mL). $^1$H-NMR (DMSO-$d_6$) δ: 1.41 (6H, s), 3.14 (3H, s), 5.34 (2H, s), 6.13 (1H, d, J = 7.7 Hz), 6.56 (1H, dd, J = 2.6 Hz, 8.7 Hz), 6.68-6.76 (2H, m), 6.90 (1H, d, J = 8.7 Hz), 7.06-7.23 (1H, m).

(v) Production of 4-(2-chloro-4-{[5-(2-hydroxyethyl)-5H-pyrrolo[3,2-d]pyrimidin-4-yl]amino}phenoxy)-1,3,3-trimethyl-1,3-dihydro-2H-indol-2-one

The title compound (95.1 mg) was obtained as white crystals by reaction in the same manner as in Example 2 (iv) and using 2-(4-chloro-5H-pyrrolo[3,2-d]pyrimidin-5-yl)ethyl benzoate (120 mg), 4-(4-amino-2-chlorophenoxy)-1,3,3-trimethyl-1,3-dihydro-2H-indol-2-one (138 mg), pyridine hydrochloride (5 mg), isopropyl alcohol (4 mL) and 1N aqueous sodium hydroxide solution (1.5 mL).
$^1$H-NMR (DMSO-$d_6$) δ: 1.42 (6H, s), 3.15 (3H, s), 3.86 (2H, t, J = 4.6 Hz), 4.52 (2H, t, J = 4.6 Hz), 6.28 (1H, d, J = 8.2 Hz), 6.49 (1H, d, J = 3.0 Hz), 6.79 (1H, d, J = 7.7 Hz), 7.16 (1H, d, J = 8.8 Hz), 7.18-7.26 (1H, m), 7.56 (1H, dd, J = 2.5 Hz, 8.8 Hz), 7.64 (1H, d, J = 3.0 Hz), 7.95 (1H, d, J = 2.5 Hz), 8.31 (1H, s), 9.84 (1H, br s).
Example 41
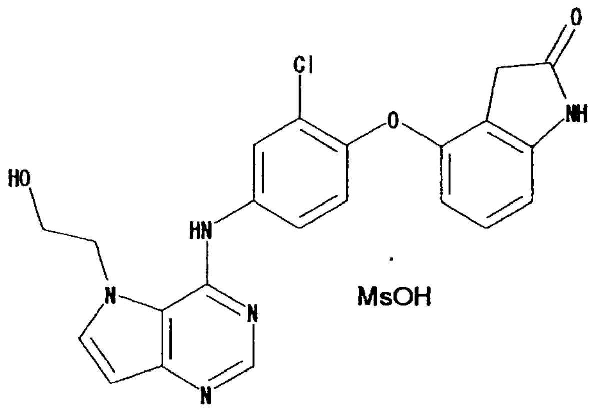

Production of 4-(2-chloro-4-{[5-(2-hydroxyethyl)-5H-pyrrolo[3,2-d]pyrimidin-4-yl]amino}phenoxy)-1,3-dihydro-2H-indol-2-one methanesulfonate

4-(2-Chloro-4-{[5-(2-hydroxyethyl)-5H-pyrrolo[3,2-d]pyrimidin-4-yl]amino}phenoxy)-1,3-dihydro-2H-indol-2-one (36 mg) was dissolved in isopropyl alcohol (0.5 mL), methanesulfonic acid (10 mg) was added, and the mixture was stirred at 50°C for 10 min. Diisopropyl ether (3 mL) was added, and the mixture was stood at room temperature for 3 hr. The precipitated crystals were collected by filtration and dried to give the title compound (31 mg) as colorless crystals.
$^1$H-NMR (DMSO-$d_6$) δ: 2.32 (3H, s), 3.35 (2H, s), 3.91 (2H, t, J = 4.5 Hz), 4.69 (2H, t, J = 4.5 Hz), 6.39 (1H, d, J = 7.9 Hz), 6.56-6.76 (2H, m), 7.10-7.32 (2H, m), 7.56 (1H, dd, J = 2.7 Hz, 8.7 Hz), 7.91 (1H, d, J = 2.3 Hz), 8.03 (1H, d, J = 2.7 Hz), 8.79 (1H, s), 10.56 (1H, s), 10.80 (1H, s), 15.01 (1H, br s).
Example 42
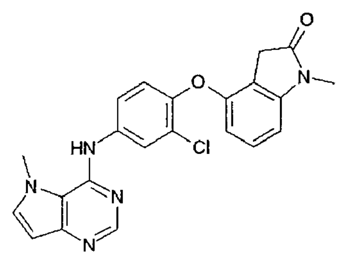

Production of 4-{2-chloro-4-[(5-methyl-5H-pyrrolo[3,2-d]pyrimidin-4-yl)amino]phenoxy}-1-methyl-1,3-dihydro-2H-indol-2-one

The title compound (44.0 mg) was obtained as white crystals by reaction in the same manner as in Example 21(ii) and using 4-chloro-5-methyl-5H-pyrrolo[3,2-d]pyrimidine (27.1 mg), 4-(4-amino-2-chlorophenoxy)-1-methyl-1,3-dihydro-2H-indol-2-one (47.8 mg), pyridine hydrochloride (5 mg) and isopropyl alcohol (2 mL).
$^1$H-NMR (DMSO-$d_6$) δ: 3.14 (3H, s), 3.45 (2H, s), 4.15 (3H, s), 6.41 (1H, d, J = 8.0 Hz), 6.45 (1H, d, J = 3.0 Hz), 6.78 (1H, d, J = 8.0 Hz), 7.18 (1H, d, J = 9.0 Hz), 7.26 (1H, t, J = 8.0 Hz), 7.59 (1H, d, J = 3.0 Hz), 7.64 (1H, dd, J = 2.7 Hz, 9.0 Hz), 7.94 (1H, d, J = 2.7 Hz), 8.31 (1H, s), 8.57 (1H, s).

Example 43

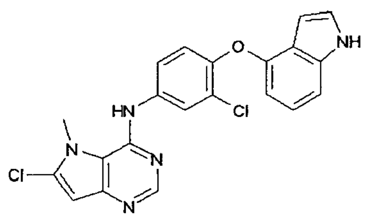

Production of 6-chloro-N-[3-chloro-4-(1H-indol-4-yloxy)phenyl]-5-methyl-5H-pyrrolo[3,2-d]pyrimidin-4-amine

(i) Production of 4,6-dichloro-5-methyl-5H-pyrrolo[3,2-d]pyrimidine

Diisopropylamine (0.9 mL) was dissolved in tetrahydrofuran (25 mL). The mixture was cooled to 0°C, 1.6M n-butyllithium hexane solution (3.6 mL) was added dropwise, and the mixture was stirred at 0°C for 1 hr. The reaction mixture was cooled to -78°C, 4-chloro-5-methyl-5H-pyrrolo[3,2-d]pyrimidine (801 mg) was added, and the mixture was stirred at -78°C for 1 hr. p-Toluenesulfonyl chloride (999 mg) was added to the reaction mixture, and the mixture was stirred at -78°C for 2.5 hr. Saturated aqueous ammonium chloride solution was added to the reaction mixture, and the mixture was extracted with ethyl acetate. The organic layer was washed with saturated brine, dried over anhydrous magnesium sulfate and concentrated under reduced pressure. The residue was separated and purified by basic silica gel column chromatography (hexane:ethyl acetate=95:5→50:50) to give the title compound (642 mg) as a white powder.
$^1$H-NMR ($CDCl_3$) δ: 4.13 (3H, s), 6.72 (1H, s), 8.67 (1H, s).

(ii) Production of 6-chloro-N-[3-chloro-4-(1H-indol-4-yloxy)phenyl]-5-methyl-5H-pyrrolo[3,2-d]pyrimidin-4-amine

A mixture of 4,6-dichloro-5-methyl-5H-pyrrolo[3,2-d]pyrimidine (81.4 mg) and 3-chloro-4-(1H-indol-4-yloxy)aniline (112 mg) was dissolved in isopropyl alcohol (4 mL), and the solution was stirred at 70°C for 24 hr. Saturated aqueous sodium hydrogen carbonate solution was added to the reaction mixture, and the mixture was extracted with ethyl acetate. The organic layer was washed with saturated brine, dried over anhydrous magnesium sulfate and concentrated under reduced pressure. The residue was separated and purified by silica gel column chromatography (hexane:ethyl acetate=80:20→20:80) to give the title compound (144 mg) as a white powder.
$^1$H-NMR (DMSO-$d_6$) δ: 4.05 (3H, s), 6.27-6.34 (1H, m), 6.41 (1H, d, J = 8.0 Hz), 6.67 (1H, s), 6.96-7.07 (2H, m), 7.19 (1H, d, J = 8.3 Hz), 7.31 (1H, t, J = 2.7 Hz), 7.51 (1H, d, J = 8.7 Hz), 7.88 (1H, s), 8.31 (1H, s), 8.70 (1H, br s), 11.28 (1H, br s).

Example 44

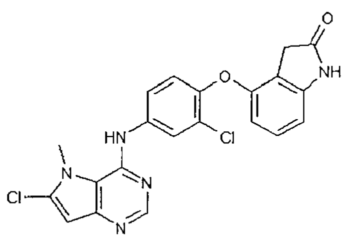

Production of 4-{2-chloro-4-[(6-chloro-5-methyl-5H-pyrrolo[3,2-d]pyrimidin-4-yl)amino]phenoxy}-1,3-dihydro-2H-indol-2-one

The title compound (82.0 mg) was obtained as pale-orange crystals by reaction in the same manner as in

Example 21(ii) and using 4,6-dichloro-5-methyl-5H-pyrrolo[3,2-d]pyrimidine (65.6 mg), 4-(4-amino-2-chlorophenoxy)-1,3-dihydro-2H-indol-2-one (94.8 mg), pyridine hydrochloride (5 mg) and isopropyl alcohol (3 mL).
$^1$H-NMR ($CDCl_3$) δ: 3.36 (2H, s), 4.05 (3H, s), 6.33 (1H, d, J = 8.0 Hz), 6.60 (1H, d, J = 7.6 Hz), 6.68 (1H, s), 7.11-7.22 (2H, m), 7.59 (1H, dd, J = 1.5 Hz, 8.7 Hz), 7.88 (1H, br s), 8.33 (1H, s), 8.75 (1H, br s), 10.52 (1H, br s).

Example 45

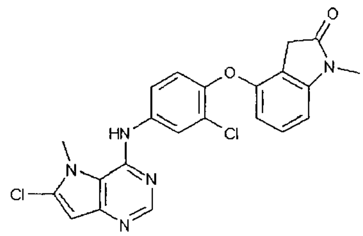

Production of 4-{2-chloro-4-[(6-chloro-5-methyl-5H-pyrrolo[3,2-d]pyrimidin-4-yl)amino]phenoxy}-1-methyl-1,3-dihydro-2H-indol-2-one

The title compound (36.8 mg) was obtained as white crystals by reaction in the same manner as in Example 21(ii) and using 4,6-dichloro-5-methyl-5H-pyrrolo[3,2-d]pyrimidine (30.4 mg), 4-(4-amino-2-chlorophenoxy)-1-methyl-1,3-dihydro-2H-indol-2-one (45.0 mg), pyridine hydrochloride (5 mg) and isopropyl alcohol (2 mL).
$^1$H-NMR ($CDCl_3$) δ:3.14 (3H, s), 3.45 (2H, s), 4.05 (3H, s), 6.42 (1H, d, J = 8.0 Hz), 6.68 (1H, s), 6.78 (1H, d, J = 8.0 Hz), 7.18 (1H, d, J = 9.0 Hz), 7.26 (1H, t, J = 8.0 Hz), 7.60 (1H, dd, J = 2.0 Hz, 9.0 Hz), 7.89 (1H, d, J = 2.0 Hz), 8.34 (1H, s), 8.75 (1H, s).

Example 46

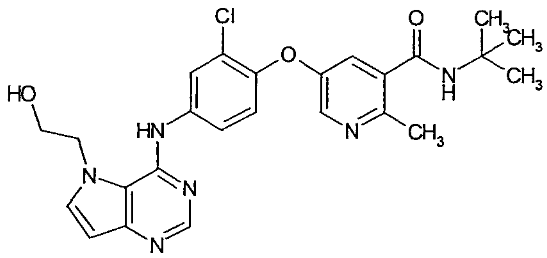

Production of N-(tert-butyl)-5-(2-chloro-4-{[5-(2-hydroxyethyl)-5H-pyrrolo[3,2-d]pyrimidin-4-yl]amino}phenoxy)-2-methylnicotinamide

(i) Production of methyl 2-methylnicotinate 1-oxide

A mixture of methyl 2-methylnicotinate (9.07 g), m-chloroperbenzoic acid (14.8 g) and toluene (100 mL) was stirred under ice-cooling for 1 hr and at room temperature overnight. The reaction mixture was subjected to silica gel column chromatography (eluent, ethyl acetate:hexane=50:50→100:0→methanol:ethyl acetate=20:80).
The object fraction was concentrated under reduced pressure to give the title compound (9.68 g) as a colorless solid.
$^1$H-NMR ($CDCl_3$) δ: 2.79 (3H, s), 3.95 (3H, s), 7.20 (1H, t, J = 7.2 Hz), 7.71 (1H, d, J = 7.5 Hz), 8.40 (1H, d, J = 6.0 Hz).

(ii) Production of methyl 5-(acetyloxy)-2-methylnicotinate

A mixture of methyl 2-methylnicotinate 1-oxide (9.07 g) and acetic anhydride (50 mL) was stirred at 110°C for 1 hr. The reaction mixture was concentrated under reduced pressure, saturated aqueous sodium hydrogen carbonate solution was added to the residue, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=25:75→50:

50). The object fraction was concentrated under reduced pressure to give the title compound (2.03 g) as a brown oil.
$^1$H-NMR ($CDCl_3$) δ: 2.34 (3H, s), 2.83 (3H, s), 3.92 (3H, s), 8.00 (1H, d, J = 2.8 Hz), 8.44 (1H, d, J = 2.8 Hz).

(iii) Production of methyl 5-(2-chloro-4-nitrophenoxy)-2-methylnicotinate

A mixture of methyl 5-(acetyloxy)-2-methylnicotinate (502 mg), potassium carbonate (332 mg) and methanol (10 mL) was stirred at room temperature for 1 hr. The reaction mixture was concentrated under reduced pressure, 2-chloro-1-fluoro-4-nitrobenzene (506 mg), potassium carbonate (332 mg) and N,N-dimethylformamide (10 mL) were added to the residue, and the mixture was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=15:85→25:75). The object fraction was concentrated under reduced pressure. The residue was filtered with ethyl acetate and diisopropyl ether to give the title compound (630 mg) as a colorless powder.
$^1$H-NMR ($CDCl_3$) δ: 2.87 (3H, s), 3.92 (3H, s), 6.94 (1H, d, J = 9.1 Hz), 7.90 (1H, d, J = 2.8 Hz), 8.11 (1H, dd, J = 2.8 Hz, 9.1 Hz), 8.42 (1H, d, J = 2.8 Hz), 8.50 (1H, d, J = 2.8 Hz).

(iv) Production of N-(tert-butyl)-5-(2-chloro-4-nitrophenoxy)-2-methylnicotinamide

Isopropyl alcohol (20 mL) was added to methyl 5-(2-chloro-4-nitrophenoxy)-2-methylnicotinate (581 mg), tetrahydrofuran (10 mL) and 1N aqueous sodium hydroxide solution (1.98 mL) were added, and the mixture was stirred at room temperature overnight. The reaction mixture was concentrated under reduced pressure, and 1N hydrochloric acid (1.98 mL) and water were added. The precipitated solid was collected by filtration, and washed with water to give 5-(2-chloro-4-nitrophenoxy)-2-methylnicotinic acid (576 mg) as a white powder. A mixture of 5-(2-chloro-4-nitrophenoxy)-2-methylnicotinic acid (525 mg), tert-butylamine (0.268 mL), 1-ethyl-3-(3-dimethylaminopropyl)carbodiimide hydrochloride (391 mg), 1-hydroxybenzotriazole (276 mg) and N,N-dimethylformamide (10 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=25:75→50:50). The object fraction was concentrated under reduced pressure to give the title compound (614 mg) as a colorless oil.
$^1$H-NMR ($CDCl_3$) δ: 1.47 (9H, s), 2.68 (3H, s), 5.56 (1H, br s), 6.93 (1H, d, J = 9.1 Hz), 7.37 (1H, d, J = 2.7 Hz), 8.10 (1H, dd, J = 2.6 Hz, 9.1 Hz), 8.38 (1H, d, J = 2.7 Hz), 8.40 (1H, d, J = 2.6 Hz).

(v) Production of 5-(4-amino-2-chlorophenoxy)-N-(tert-butyl)-2-methylnicotinamide

To a solution of N-(tert-butyl)-5-(2-chloro-4-nitrophenoxy)-2-methylnicotinamide (614 mg) in ethyl acetate (10 mL) was added 5% platinum/activated carbon (30 mg) under hydrogen stream, and the mixture was stirred at room temperature overnight. The catalyst was filtered off, the filtrate was concentrated and the obtained residue was subjected tosilica gel column chromatography (eluent, ethyl acetate:hexane=25:75→50:50). The object fraction was concentrated under reduced pressure, and the residue was crystallized from ethyl acetate/diisopropyl ether to give the title compound (493 mg) as a pale-yellow powder.
$^1$H-NMR ($CDCl_3$) δ: 1.45 (9H, s), 2.58 (3H, s), 3.72 (2H, br s), 5.52 (1H, br s), 6.57 (1H, dd, J = 2.8 Hz, 8.7 Hz), 6.78 (1H, d, J = 2.8 Hz), 6.89 (1H, d, J = 8.7 Hz), 7.11 (1H, d, J = 2.9 Hz), 8.20 (1H, d, J = 2.9 Hz).

(vi) Production of N-(tert-butyl)-5-(2-chloro-4-{[5-(2-hydroxyethyl)-5H-pyrrolo[3,2-d]pyrimidin-4-yl]amino}phenoxy)-2-methylnicotinamide

A mixture of 2-(4-chloro-5H-pyrrolo[3,2-d]pyrimidin-5-yl)ethyl benzoate (121 mg), 5-(4-amino-2-chlorophenoxy)-N-(tert-butyl)-2-methylnicotinamide (134 mg) and isopropyl alcohol (5 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=50:50→100:0→methanol:ethyl acetate=15:85). The object fraction was concentrated under reduced pressure. Methanol (5 mL), tetrahydrofuran (1 mL) and 1N aqueous sodium hydroxide solution (0.8 mL) were added to the residue and the mixture was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure and the obtained

residue was subjected to basic silica gel column chromatography (eluent, methanol:ethyl acetate=1:99→15:85). The object fraction was concentrated under reduced pressure, and the residue was crystallized from ethyl acetate/diisopropyl ether to give the title compound (116 mg) as a white powder.
$^1$H-NMR (DMSO-$d_6$) δ: 1.34 (9H, s), 2.45 (3H, s), 3.87 (2H, q, J = 4.3 Hz), 4.53 (2H, t, J = 4.6 Hz), 6.29 (1H, t, J = 4.2 Hz), 6.51 (1H, d, J = 3.2 Hz), 7.14 (1H, d, J = 2.8 Hz), 7.24 (1H, d, J = 8.9 Hz), 7.60 (1H, dd, J = 2.5 Hz, 8.9 Hz), 7.66 (1H, d, J = 3.2 Hz), 7.98 (1H, d, J = 2.5 Hz), 8.07 (1H, br s), 8.22 (1H, d, J = 2.8 Hz), 8.34 (1H, s), 9.87 (1H, br s).

Example 47

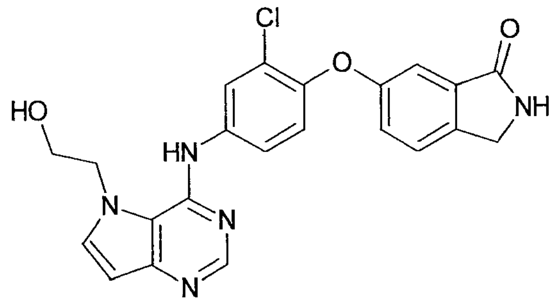

Production of 6-(2-chloro-4-{[5-(2-hydroxyethyl)-5H-pyrrolo[3,2-d]pyrimidin-4-yl]amino}phenoxy)isoindolin-1-one

(i) Production of 5-amino-2-methylbenzoic acid

To a solution of 2-methyl-5-nitrobenzoic acid (9.95 g) in ethyl acetate (100 mL)/tetrahydrofuran (100 mL) was added 10% palladium/activated carbon (50% water-containing product, 1.0 g) under hydrogen stream, and the mixture was stirred at room temperature overnight. The catalyst was filtered off, and the filtrate was concentrated. The obtained residue was filtered with ethyl acetate/hexane to give the title compound (8.17 g) as a colorless powder.
$^1$H-NMR (DMSO-$d_6$) δ: 2.32 (3H, s), 5.05 (2H, br s), 6.63 (1H, dd, J = 2.5 Hz, 8.1 Hz), 6.91 (1H, d, J = 8.1 Hz), 7.07 (1H, d, J = 2.5 Hz).

(ii) Production of methyl 5-hydroxy-2-methylbenzoate

5-Amino-2-methylbenzoic acid (907 mg) was dissolved in 50% sulfuric acid (50 g) with heating, and the solution was cooled to -10°C. Water (65 mL) was added, and a solution of sodium nitrite (455 mg) in water (5 mL) was added dropwise. Water (5 mL) was added and the mixture was stirred for 10 min, at 0°C for 1 hr and at 100°C for 1.5 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was dissolved in methanol (10 mL). Con. sulfuric acid (0.2 mL) was added, and the mixture was heated under reflux overnight. The reaction mixture was concentrated under reduced pressure, water was added, and the mixture was extracted with ethyl acetate. The organic layer was washed successively with saturated aqueous sodium hydrogen carbonate solution and saturated brine, and dried over anhydrous magnesium sulfate. The solvent was evaporated under reduced pressure and the obtained residue was subjected to silica gel column chromatography (eluent, ethyl acetate:hexane=10:90→20:80). The object fraction was concentrated under reduced pressure. The residue was filtered with hexane to give the title compound (820 mg) as an orange powder.
$^1$H-NMR ($CDCl_3$) δ: 2.50 (3H, s), 3.88 (3H, s), 5.75 (1H, s), 6.92 (1H, dd, J = 2.8 Hz, 8.3 Hz), 7.10 (1H, d, J = 8.3 Hz), 7.44 (1H, d, J = 2.8 Hz).

(iii) Production of methyl 5-(2-chloro-4-nitrophenoxy)-2-methylbenzoate

A mixture of methyl 5-hydroxy-2-methylbenzoate (4.99 g), 2-chloro-1-fluoro-4-nitrobenzene (5.27 g), potassium carbonate (6.22 g) and N,N-dimethylformamide (50 mL) was stirred at room temperature overnight. The reaction mixture was concentrated under reduced pressure, water was added, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=50:50). The object fraction was concentrated under reduced pressure,

and the residue was crystallized from ethyl acetate/hexane to give the title compound (8.87 g) as a pale-yellow solid.
$^1$H-NMR ($CDCl_3$) δ: 2. 63 (3H, s), 3.89 (3H, s), 6.86 (1H, d, J = 9.1 Hz), 7.15 (1H, dd, J = 2.7 Hz, 8.3 Hz), 7.33 (1H, d, J = 8.3 Hz), 7.64 (1H, d, J = 2.7 Hz), 8.06 (1H, dd, J = 2.7 Hz, 9.1 Hz), 8.39 (1H, d, J = 2.7 Hz).

(iv) Production of methyl 2-(bromomethyl)-5-(2-chloro-4-nitrophenoxy)benzoate

To a solution of methyl 5-(2-chloro-4-nitrophenoxy)-2-methylbenzoate (4.82 g) in benzotrifluoride (80 mL) were added N-bromosuccinimide (3.20 g) and 2,2'-azobis(isobutyronitrile) (123 mg), and the mixture was stirred at 100°C overnight. N-Bromosuccinimide (1.07 g) was added to the reaction mixture, and the mixture was stirred at 100°C overnight. 10% Aqueous sodium carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=10:90→20:80). The object fraction was concentrated under reduced pressure. The residue was filtered with diethyl ether/diisopropyl ether. The filtrate was evaporated under reduced pressure and the obtained residue was subjected to silica gel column chromatography (eluent, ethyl acetate:hexane=5:95→10:90). The object fraction was concentrated under reduced pressure to give the title compound (2.24 g) as a pale-yellow solid. $^1$H-NMR ($CDCl_3$) δ: 3.94 (3H, s), 4.97 (2H, s), 6.98 (1H, d, J = 9.0 Hz), 7.21 (1H, dd, J = 2.7 Hz, 8.3 Hz), 7.55 (1H, d, J = 8.3 Hz), 7.65 (1H, d, J = 2.7 Hz), 8.11 (1H, dd, J = 2.8 Hz, 9.0 Hz), 8.41 (1H, d, J = 2.8 Hz).

(v) Production of 6-(2-chloro-4-nitrophenoxy)isoindolin-1-one

A mixture of methyl 2-(bromomethyl)-5-(2-chloro-4-nitrophenoxy)benzoate (601 mg), 28% aqueous ammonia (3 mL), tetrahydrofuran (27 mL) and methanol (3 mL) was stirred at room temperature overnight. The reaction mixture was concentrated under reduced pressure, and water was added. The precipitated solid was collected by filtration, and washed successively with water and diisopropyl ether to give the title compound (440 mg) as a white powder.
$^1$H-NMR ($CDCl_3$) δ: 4.50 (2H, s), 6.90 (1H, br s), 6.97 (1H, d, J = 9.1 Hz), 7.34 (1H, dd, J = 2.3 Hz, 8.3 Hz), 7.48 (1H, d, J = 2.3 Hz),7.56 (1H, d, J = 8.3 Hz), 8.09 (1H, dd, J = 2.7 Hz, 9.1 Hz), 8.41 (1H, d, J = 2.7 Hz).

(vi) Production of 6-(4-amino-2-chlorophenoxy)isoindolin-1-one

To a solution of 6-(2-chloro-4-nitrophenoxy)isoindolin-1-one (396 mg) in tetrahydrofuran (10 mL)/methanol (10 mL) was added 5% platinum/activated carbon (40 mg), and the mixture was stirred with heating under hydrogen stream at room temperature for 2 days. The catalyst was filtered off, and the filtrate was concentrated. The obtained residue was filtered with ethanol/ethyl acetate to give the title compound (250 mg) as a pale-yellow powder.
$^1$H-NMR ($CDCl_3$) δ: 3.70 (2H, br s), 4.40 (2H, s), 6.30 (1H, br s), 6.58 (1H, dd, J = 2.8 Hz, 8.5 Hz), 6.78 (1H, d, J = 2.8 Hz), 6.93 (1H, d, J = 8.5 Hz), 7.15-7.30 (2H, m), 7.40 (1H, d, J = 8.3 Hz).

(vii) Production of 6-(2-chloro-4-{[5-(2-hydroxyethyl)-5H-pyrrolo[3,2-d]pyrimidin-4-yl]amino}phenoxy)isoindolin-1-one

A mixture of 2-(4-chloro-5H-pyrrolo[3,2-d]pyrimidin-5-yl)ethyl benzoate (151 mg), 6-(4-amino-2-chlorophenoxy) isoindolin-1-one (137 mg) and isopropyl alcohol (3 mL) was stirred at 80°C overnight. To the reaction mixture were added pyridine hydrochloride (5 mg) and 1-methyl-2-pyrrolidone (1 mL), and the mixture was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→30:70). The object fraction was concentrated under reduced pressure. Methanol (5 mL), tetrahydrofuran (1 mL) and 1N aqueous sodium hydroxide solution (1 mL) were added to the residue and the mixture was stirred at room temperature overnight. Water was added to the reaction mixture, and the mixture was extracted with a mixed solvent of ethyl acetate and tetrahydrofuran. The organic layer was washed with saturated brine, and dried over anhydrous magnesium sulfate. The solvent was evaporated under reduced pressure and the obtained residue was filtered with ethanol/ethyl acetate to give the title compound (155 mg) as a white powder.
$^1$H-NMR (DMSO-$d_6$) δ: 3.82-3.95 (2H, m), 4.34 (2H, s), 4.55 (2H, t, J = 4.4 Hz), 6.30 (1H, br s), 6.52 (1H, d, J = 3.0 Hz), 6.93 (1H, d, J = 2.4 Hz), 7.23-7.35 (2H, m), 7.53-7.71 (3H, m), 7.98 (1H, d, J = 2.4 Hz), 8.35 (1H, s), 8.64 (1H, br s), 9.89 (1H, br s).

Example 48

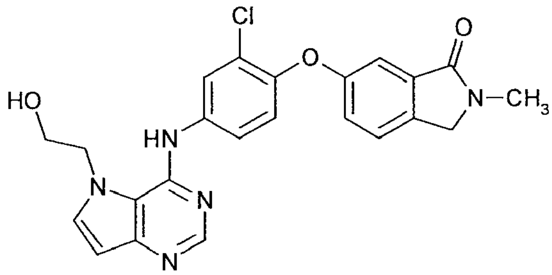

Production of 6-(2-chloro-4-{[5-(2-hydroxyethyl)-5H-pyrrolo[3,2-d]pyrimidin-4-yl]amino}phenoxy)-2-methylisoindolin-1-one

(i) Production of 6-(2-chloro-4-nitrophenoxy)-2-methylisoindolin-1-one

To a solution of methyl 2-(bromomethyl)-5-(2-chloro-4-nitrophenoxy)benzoate (601 mg) in tetrahydrofuran (10 mL) was added 2M methylamine/tetrahydrofuran solution (3 mL) and the mixture was stirred at room temperature overnight. The reaction mixture was concentrated under reduced pressure, and water and diisopropyl ether were added. The precipitated solid was collected by filtration and washed successively with water and diisopropyl ether to give the title compound (434 mg) as a pale-yellow powder.
$^1$H-NMR ($CDCl_3$) δ: 3.22 (3H, s), 4.41 (2H, s), 6.94 (1H, d, J = 9.1 Hz), 7.29 (1H, dd, J = 2.4 Hz, 8.4 Hz), 7.46 (1H, d, J = 2.4 Hz),7.51 (1H, d, J = 8.7 Hz), 8.08 (1H, dd, J = 2.7 Hz, 9.1 Hz), 8.40 (1H, d, J = 2.7 Hz).

(ii) Production of 6-(4-amino-2-chlorophenoxy)-2-methylisoindolin-1-one

To a solution of 6-(2-chloro-4-nitrophenoxy)-2-methylisoindolin-1-one (382 mg) in tetrahydrofuran (10 mL)/ methanol (10 mL) was added 5% platinum/activated carbon (38 mg) under hydrogen stream, and the mixture was stirred at room temperature for 2 days. The catalyst was filtered off, the filtrate was concentrated and the obtained residue was subjected to silica gel column chromatography (eluent, ethyl acetate:hexane=50:50→100:0). The object fraction was concentrated under reduced pressure. The residue was filtered with ethyl acetate/diisopropyl ether to give the title compound (302 mg) as a colorless powder.
$^1$H-NMR ($CDCl_3$) δ: 3.17 (3H, s), 3.71 (2H, br s), 4.31 (2H, s), 6.57 (1H, dd, J = 2.7 Hz, 8.6 Hz), 6.78 (1H, d, J = 2.7 Hz), 6.92 (1H, d, J = 8.6 Hz), 7.14-7.21 (2H, m), 7.35 (1H, d, J = 9.0 Hz).

(iii) Production of 6-(2-chloro-4-{[5-(2-hydroxyethyl)-5H-pyrrolo[3,2-d]pyrimidin-4-yl]amino}phenoxy)-2-methylisoindolin-1-one

A mixture of 2-(4-chloro-5H-pyrrolo[3,2-d]pyrimidin-5-yl)ethyl benzoate (151 mg), 6-(4-amino-2-chlorophenoxy)-2-methylisoindolin-1-one (144 mg) and isopropyl alcohol (3 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. Methanol (5 mL), tetrahydrofuran (1 mL) and 1N aqueous sodium hydroxide solution (1 mL) were added to the residue and the mixture was stirred at room temperature overnight. Water was added to the reaction mixture, and the mixture was extracted with a mixed solvent of ethyl acetate and tetrahydrofuran. The organic layer was washed with saturated brine, and dried over anhydrous magnesium sulfate. The solvent was evaporated under reduced pressure and the obtained residue was subjected to basic silica gel column chromatography (eluent, methanol:ethyl acetate=0:100→30:70). The object fraction was concentrated under reduced pressure. The residue was filtered with ethanol/ethyl acetate to give the title compound (194 mg) as a white powder.
$^1$H-NMR (DMSO-$d_6$) δ: 3.06 (3H, s), 3.83-3.93 (2H, m), 4.43 (2H, s), 4.55 (2H, t, J = 4.4 Hz), 6.30 (1H, br s), 6.52 (1H, d, J = 3.1 Hz), 6.92 (1H, d, J = 2.3 Hz), 7.24-7.29 (1H, m), 7.29 (1H, d, J = 8.9 Hz), 7.59 (1H, d, J = 8.5 Hz), 7.60-7.65 (1H, m), 7.67 (1H, d, J = 3.1 Hz), 7.98 (1H, d, J = 2.4 Hz), 8.35 (1H, s), 9.89 (1H, br s).
Example 49
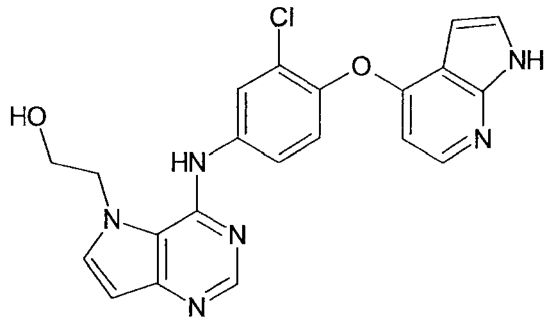

Production of 2-(4-{[3-chloro-4-(1H-pyrrolo[2,3-b]pyridin-4-yloxy)phenyl]amino}-5H-pyrrolo[3,2-d]pyrimidin-5-yl)ethanol

(i) Production of 4-chloro-1H-pyrrolo[2,3-b]pyridine

A mixture of 1H-pyrrolo[2,3-b]pyridine (8.25 g), m-chloroperbenzoic acid (18.9 g), toluene (80 mL) and ethyl acetate (120 mL) was stirred under ice-cooling for 1 hr, and at room temperature overnight. 2N Hydrochloric acid was added to the reaction mixture, and the mixture was washed with ethyl acetate. The aqueous layer was concentrated under reduced pressure to give a crude product (11.7 g) of 1H-pyrrolo[2,3-b]pyridine 7-oxide hydrochloride as an orange solid. To a mixture of the crude product (1.71 g), triethylamine (1.53 mL) and N,N-dimethylformamide (7.74 mL) was added methanesulfonyl chloride (2.32 mL) under ice-cooling, and the mixture was stirred under ice-cooling for 6 hr. Separately, to a mixture of a crude product (9.38 g) of 1H-pyrrolo[2,3-b]pyridine 7-oxide hydrochloride, triethylamine (8.43 mL) and N,N-dimethylformamide (42.6 mL) was added methanesulfonyl chloride (12.8 mL) under ice-cooling, and the mixture was stirred under ice-cooling for 6 hr. The reaction mixtures were combined, saturated aqueous sodium hydrogen carbonate solution was added, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=50:50). The object fraction was concentrated under reduced pressure. The residue was filtered with ethyl acetate/hexane to give the title compound (5.36 g) as a pale-red powder.
$^1$H-NMR ($CDCl_3$) $\delta$: 6.63 (1H, d, J = 2.4 Hz), 7.14 (1H, d, J = 5.3 Hz), 7.39-7.44 (1H, m), 8.23 (1H, d, J = 5.3 Hz), 10.97 (1H, br s).

(ii) Production of 4-(2-chloro-4-nitrophenoxy)-1H-pyrrolo[2,3-b]pyridine

A mixture of 4-chloro-1H-pyrrolo[2,3-b]pyridine (1.53 g), 2-chloro-4-nitrophenol (4.31 g) and diphenyl ether (5 mL) was stirred at 150°C overnight. 10% Aqueous sodium carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=30:70→60:40). The object fraction was concentrated under reduced pressure. The residue was filtered with diisopropyl ether to give the title compound (1.38 g) as a yellow powder.
$^1$H-NMR ($CDCl_3$) $\delta$: 6.31-6.35 (1H, m), 6.68 (1H, d, J = 5.3 Hz), 7.14 (1H, d, J = 9.1 Hz), 7.31-7.37 (1H, m), 8.13 (1H, dd, J = 2.7 Hz, 9.1 Hz), 8.31 (1H, d, J = 5.3 Hz), 8.45 (1H, d, J = 2.7 Hz), 10.80 (1H, br s).

(iii) Production of 3-chloro-4-(1H-pyrrolo[2,3-b]pyridin-4-yloxy)aniline

A mixture of 4-(2-chloro-4-nitrophenoxy)-1H-pyrrolo[2,3-b]pyridine (580 mg), reduced iron (621 mg), calcium chloride (123 mg) and 10% water-containing ethanol (30 mL) was stirred with heating under reflux for 2 days. The reaction mixture was filtered to remove solid, and the filtrate was concentrated. Water was added to the residue, and the mixture was extracted with ethyl acetate. The organic layer was washed with saturated brine, and dried over anhydrous magnesium sulfate. The solvent was evaporated under reduced pressure and the obtained residue was subjected to basic silica gel column chromatography (eluent, ethyl acetate:hexane=60:40→100:0→methanol:ethyl acetate=20:80). The object fraction was concentrated under reduced pressure. The residue was filtered with diisopropyl ether to give the title compound (243 mg) as a colorless powder.
$^1$H-NMR ($CDCl_3$) $\delta$: 3.74 (2H, br s), 6.35 (1H, d, J = 5.5 Hz), 6.43 (1H, d, J = 3.2 Hz), 6.62 (1H, dd, J = 2.8 Hz, 8.7 Hz),

6.82 (1H, d, J = 2.8 Hz), 7.04 (1H, d, J = 8.7 Hz), 7.23 (1H, d, J = 3.2 Hz), 8.14 (1H, d, J = 5.5 Hz), 10.47 (1H, br s).

(iv) Production of 2-(4-{[3-chloro-4-(1H-pyrrolo[2,3-b]pyridin-4-yloxy)phenyl]amino}-5H-pyrrolo[3,2-d]pyrimidin-5-yl)ethanol

The title compound (82 mg) was obtained as a white powder in the same manner as in Example 26 (v) and using 2-(4-chloro-5H-pyrrolo[3,2-d]pyrimidin-5-yl)ethyl benzoate (151 mg), 3-chloro-4-(1H-pyrrolo[2,3-b]pyridin-4-yloxy) aniline (130 mg), pyridine hydrochloride (5 mg), isopropyl alcohol (3 mL), methanol (5 mL), tetrahydrofuran (1 mL) and 1N aqueous sodium hydroxide solution (1 mL).
$^1$H-NMR (DMSO-$d_6$) δ: 3.89 (2H, t, J = 4.3 Hz), 4.55 (2H, t, J = 4.3 Hz), 6.26 (1H, d, J = 2.3 Hz), 6.25-6.42 (1H, m), 6.32 (1H, d, J = 5.5 Hz), 6.52 (1H, d, J = 3.0 Hz), 7.34-7.41 (2H, m), 7.61-7.71 (2H, m), 8.03 (1H, d, J = 2.4 Hz), 8.08 (1H, d, J = 5.5 Hz), 8.36 (1H, s), 9.94 (1H, br s), 11.76 (1H, br s).
Example 50
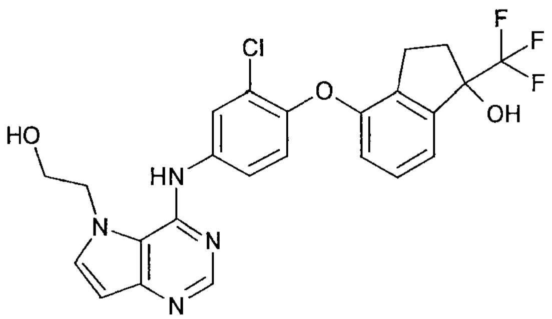

Production of 4-(2-chloro-4-{[5-(2-hydroxyethyl)-5H-pyrrolo[3,2-d]pyrimidin-4-yl]amino}phenoxy)-1-(trifluoromethyl)indan-1-ol

(i) Production of 4-(2-chloro-4-nitrophenoxy)indan-1-one

A mixture of 2-chloro-1-fluoro-4-nitrobenzene (1.18 g), 4-hydroxy-1-indanone (1.0 g) and potassium carbonate (0.94 g) in N,N-dimethylformamide (10 mL) was stirred at room temperature for 20 hr. The reaction system was acidified with water and 6N hydrochloric acid, and the mixture was extracted with ethyl acetate. The organic layer was washed with water and saturated brine, and dried over anhydrous magnesium sulfate. The residue was separated and purified by silica gel column chromatography (eluent, hexane:ethyl acetate=4:1→1:1) to give the title compound (1.93 g) as pale-yellow crystals.
$^1$H-NMR ($CDCl_3$) δ: 2.71-2.75 (2H, m), 2.99-3.03 (2H, m), 6.89 (1H, d, J = 9.0 Hz), 7.23 (1H, dd, J = 0.9, 7.8 Hz), 7.46 (1H, t, J = 7.8 Hz), 7.68 -7.70 (1H, m), 8.08 (1H, dd, J = 2.7, 9.0 Hz), 8.42 (1H, d, J = 2.7 Hz).

(ii) Production of 4-(2-chloro-4-nitrophenoxy)-1-(trifluoromethyl)indan-1-ol

To a solution of 4-(2-chloro-4-nitrophenoxy)indan-1-one (911 mg) in tetrahydrofuran (10 mL) were added trimethyl(trifluoromethyl)silane (0.89 mL) and 1M tetrabutylammonium fluoride/tetrahydrofuran solution (0.15 mL), and the mixture was stirred at room temperature overnight. Concentrated hydrochloric acid (5 mL) and water (5 mL) were added to the reaction mixture, and the mixture was stirred at room temperature for 3 hr. Water was added to the reaction mixture, and the mixture was extracted with ethyl acetate. The organic layer was washed successively with saturated aqueous sodium hydrogen carbonate solution and saturated brine, and dried over anhydrous magnesium sulfate. The solvent was evaporated under reduced pressure and the obtained residue was subjected to silica gel column chromatography (eluent, ethyl acetate:hexane=5:95→25:75). The object fraction was concentrated under reduced pressure to give the title compound (1.05 g) as a yellow oil.
$^1$H-NMR ($CDCl_3$) δ: 2.20-2.34 (1H, m), 2.60 (1H, s), 2.63-3.06 (3H, m), 6.82 (1H, d, J = 9.0 Hz), 7.07 (1H, dd, J = 1.3Hz, 7.5 Hz), 7.35 -7.51 (2H, m), 8.06 (1H, dd, J = 2.6, 9.0 Hz), 8.40 (1H, d, J = 2.6 Hz).

(iii) Production of 4-(4-amino-2-chlorophenoxy)-1-(trifluoromethyl)indan-1-ol

A mixture of 4-(2-chloro-4-nitrophenoxy)-1-(trifluoromethyl)indan-1-ol (1.05 g), reduced iron (745 mg), calcium chloride (185 mg) and 10% water-containing ethanol (20 mL) was stirred with heating under reflux overnight. The reaction mixture was filtered to remove solid, and the filtrate was concentrated. Water was added to the residue, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=20:80→40:60). The object fraction was concentrated under reduced pressure to give the title compound (243 mg) as a pale-yellow solid.
$^1$H-NMR ($CDCl_3$) δ: 2.21-2.35 (1H, m), 2.51 (1H, s), 2.64-2.75 (1H, m), 2.94-3.21 (2H, m), 3.67 (2H, br s), 6.56 (1H, dd, J = 2.7 Hz, 8.6 Hz), 6.59-6.68 (1H, m), 6.78 (1H, d, J = 2.7 Hz), 6.86 (1H, d, J = 8.6 Hz), 7.15-7.20 (2H, m).

(iv) Production of 4-(2-chloro-4-{[5-(2-hydroxyethyl)-5H-pyrrolo[3,2-d]pyrimidin-4-yl]amino}phenoxy)-1-(trifluoromethyl) indan-1-ol

A mixture of 2-(4-chloro-5H-pyrrolo[3,2-d]pyrimidin-5-yl)ethyl benzoate (151 mg), 4-(4-amino-2-chlorophenoxy)-1-(trifluoromethyl)indan-1-ol (172 mg) and isopropyl alcohol (3 mL) was stirred at 80°C overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=50:50→100:0). The object fraction was concentrated under reduced pressure. Methanol (5 mL), tetrahydrofuran (1 mL) and 1N aqueous sodium hydroxide solution (1 mL) were added to the residue and the mixture was stirred at room temperature for 3 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to basic silica gel column chromatography (eluent, methanol:ethyl acetate=0:100→20:80). The object fraction was concentrated under reduced pressure. The residue was filtered with ethanol/ethyl acetate to give the title compound (172 mg) as a white powder.
$^1$H-NMR (DMSO-$d_6$) δ: 2.15-2.31 (1H, m), 2.53-2.63 (1H, m), 2.77-3.10 (2H, m), 3.82-3.93 (2H, m), 4.53 (2H, t, J = 4.3 Hz), 6.30 (1H, br s), 6.51 (1H, d, J = 3.1 Hz), 6.66-6.75 (2H, m), 7.12-7.33 (3H, m), 7. 58 (1H, dd, J = 2.4 Hz, 8. 9 Hz), 7. 66 (1H, d, J = 3.1 Hz), 7.98 (1H, d, J = 2.4 Hz), 8.34 (1H, s), 9.85 (1H, br s).
Example 51
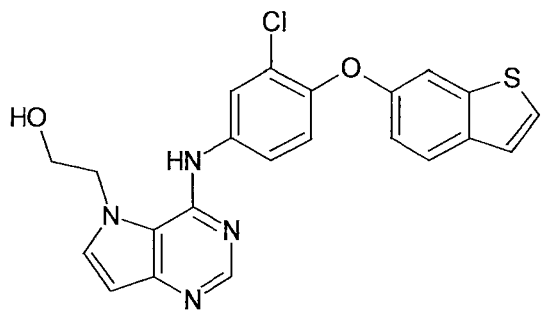

Production of 2-(4-{[4-(1-benzothiophen-6-yloxy)-3-chlorophenyl]amino}-5H-pyrrolo[3,2-d]pyrimidin-5-yl)ethanol

(i) Production of 1-benzothiophen-6-ol

To a mixture of 3-methoxybenzenethiol (14.9 mL), potassium carbonate (16.6 g) and acetone (150 mL) was added a solution of 2-bromo-1,1-diethoxyethane (16.5 mL) in acetone (20 mL), and the mixture was stirred at room temperature overnight. The reaction mixture was filtered to remove solid, and the filtrate was concentrated. Water was added to the residue, and the mixture was extracted with diethyl ether. The organic layer was washed with saturated brine, and dried over anhydrous magnesium sulfate. The solvent was evaporated under reduced pressure and the obtained residue (29.3 g) was dissolved in chlorobenzene (150 mL). The solution was added to a mixture (heated to 150°C) of diphosphoric pentaoxide (45 g) and polyphosphoric acid (150 g). The mixture was stirred at 150°C for 30 min

and allowed to cool. The supernatant was separated and washed with ethyl acetate. The organic layer was washed successively with saturated aqueous sodium hydrogen carbonate solution and saturated brine, and dried over anhydrous magnesium sulfate. The solvent was evaporated under reduced pressure and the obtained residue was subjected to silica gel column chromatography (eluent, ethyl acetate:hexane=1:99→5:95). The object fraction was concentrated under reduced pressure to give a crude product (10.98 g) of 6-methoxy-1-benzothiophene as a yellow oil. A mixture of the crude product (4.93 g), boron tribromide-methyl sulfide complex (11.3 g) and chlorobenzene (100 mL) was stirred at 130°C for 7 hr. Water was added to the reaction mixture, and the mixture was extracted with ethyl acetate. The organic layer was washed successively with saturated aqueous sodium hydrogen carbonate solution and saturated brine, and dried over anhydrous magnesium sulfate. The solvent was evaporated under reduced pressure and the obtained residue was subjected to silica gel column chromatography (eluent, ethyl acetate:hexane=5:95→15:85). The object fraction was concentrated under reduced pressure. The residue was filtered with diisopropyl ether/hexane to give the title compound (2.60 g) as a pale-gray powder.
$^1$H-NMR ($CDCl_3$) δ: 4.79 (1H, s), 6.91 (1H, dd, J = 2.3 Hz, 8.6 Hz), 7.21-7.26 (2H, m), 7.31 (1H, d, J = 2.3 Hz), 7.67 (1H, d, J = 8.6 Hz) .

(ii) Production of 6-(2-chloro-4-nitrophenoxy)-1-benzothiophene

A mixture of 1-benzothiophen-6-ol (2.55 g), 2-chloro-1-fluoro-4-nitrobenzene (2.98 g), potassium carbonate (3.52 g) and N,N-dimethylformamide (30 mL) was stirred at room temperature for 4 hr. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=1:99→10:90). The object fraction was concentrated under reduced pressure to give the title compound (5.18 g) as a pale-yellow solid.
$^1$H-NMR ($CDCl_3$) δ: 6.88 (1H, d, J = 9.2 Hz), 7.13 (1H, dd, J = 2.2 Hz, 8.7 Hz), 7.37 (1H, d, J = 5.4 Hz), 7.48 (1H, d, J = 5.4 Hz), 7.60 (1H, d, J = 2.2 Hz), 7.87 (1H, d, J = 8.7 Hz), 8.04 (1H, dd, J = 2.6 Hz, 9.2 Hz), 8.04 (1H, d, J = 2.6 Hz).

(iii) Production of 4-(1-benzothiophen-6-yloxy)-3-chloroaniline

A mixture of 6-(2-chloro-4-nitrophenoxy)-1-benzothiophene (5.18 g), reduced iron (4.22 g), calcium chloride (1.05 g) and 10% water-containing ethanol (100 mL) was stirred with heating under reflux overnight. The reaction mixture was filtered to remove solid, and the filtrate was concentrated. Water was added to the residue, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=10:90→30:70). The object fraction was concentrated under reduced pressure, and the residue was crystallized from diisopropyl ether/hexane to give the title compound (2.93 g) as a colorless powder.
$^1$H-NMR ($CDCl_3$) δ: 3.68 (2H, br s), 6.58 (1H, dd, J = 2.8 Hz, 8.5 Hz), 6.81 (1H, d, J = 2.8 Hz), 6.93 (1H, d, J = 8.5 Hz), 7.05 (1H, dd, J = 2.3 Hz, 8.7 Hz), 7.24-7.27 (2H, m), 7.29 (1H, d, J = 5.7 Hz), 7.72 (1H, d, J = 8.7 Hz).

(iv) Production of 2-(4-{[4-(1-benzothiophen-6-yloxy)-3-chlorophenyl]amino}-5H-pyrrolo[3,2-d]pyrimidin-5-yl)ethanol

The title compound (164 mg) was obtained as a white powder in the same manner as in Example 50 (iv) and using 2-(4-chloro-5H-pyrrolo[3,2-d]pyrimidin-5-yl)ethyl benzoate (151 mg), 4-(1-benzothiophen-6-yloxy)-3-chloroaniline (110 mg), isopropyl alcohol (5 mL), methanol (5 mL), tetrahydrofuran (1 mL) and 1N aqueous sodium hydroxide solution (0.8 mL). $^1$H-NMR (DMSO-$d_6$) δ: 3.88 (2H, t, J = 4.6 Hz), 4.54 (2H, t, J = 4.6 Hz), 6.30 (1H, br s), 6.51 (1H, d, J = 3.2 Hz), 7.08 (1H, dd, J = 2.3 Hz, 8.7 Hz), 7.22 (1H, d, J = 8.8 Hz), 7.42 (1H, d, J = 5.5 Hz), 7.52 (1H, d, J = 2.3 Hz), 7.59 (1H, dd, J = 2.6 Hz, 8.8 Hz), 7.63-7.68 (2H, m), 7.87 (1H, d, J = 8.7 Hz), 7.98 (1H, d, J = 2.6 Hz), 8.34 (1H, s), 9.85 (1H, br s).
Example 52
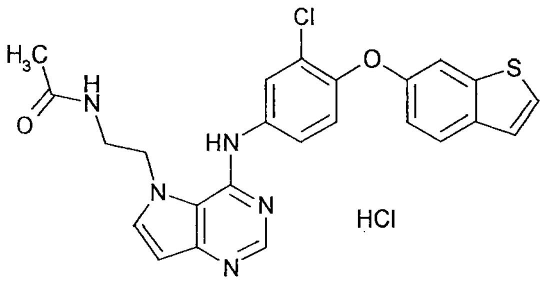

Production of N-[2-(4-{[4-(1-benzothiophen-6-yloxy)-3-chlorophenyl]amino}-5H-pyrrolo[3,2-d]pyrimidin-5-yl)ethyl]acetamide hydrochloride

(i) Production of tert-butyl [2-(4-{[4-(1-benzothiophen-6-yloxy)-3-chlorophenyl]amino}-5H-pyrrolo[3,2-d]pyrimidin-5-yl)ethyl]carbamate hydrochloride

A mixture of tert-butyl [2-(4-chloro-5H-pyrrolo[3,2-d]pyrimidin-5-yl)ethyl]carbamate (3.26 g), 4-(1-benzothiophen-6-yloxy)-3-chloroaniline (2.92 g) and isopropyl alcohol (50 mL) was stirred at 80°C overnight. Ethyl acetate was added to the reaction mixture and the precipitate was collected by filtration to give the title compound (820 mg) as a pale-yellow powder.
$^1$H-NMR (DMSO-$d_6$) δ: 1.26 (9H, s), 3.33 (2H, q, J = 5.9 Hz), 4.69 (2H, t, J = 5.8 Hz), 6.68 (1H, d, J = 3.0 Hz), 7.09-7.18 (1H, m), 7.13 (1H, dd, J = 2.4 Hz, 8.7 Hz), 7.22 (1H, d, J = 8.9 Hz), 7.46 (1H, d, J = 5.5 Hz), 7.59-7.67 (1H, m), 7.62 (1H, d, J = 2.4 Hz), 7.70 (1H, d, J = 5.5 Hz), 7.87-7.97 (2H, m), 7.92 (1H, d, J = 8.7 Hz), 8.73 (1H, s), 9.98 (1H, br s).

(ii) Production of N-[2-(4-{[4-(1-benzothiophen-6-yloxy)-3-chlorophenyl]amino}-5H-pyrrolo[3,2-d]pyrimidin-5-yl)ethyl]acetamide hydrochloride

A mixture of tert-butyl [2-(4-{[4-(1-benzothiophen-6-yloxy)-3-chlorophenyl]amino}-5H-pyrrolo[3,2-d]pyrimidin-5-yl)ethyl]carbamate hydrochloride (229 mg), 4N hydrochloric acid/ethyl acetate (3 mL) and ethanol (1 mL) was stirred at 80°C for 2 hr. The reaction mixture was concentrated under reduced pressure. Ethyl acetate (10 mL) and saturated aqueous sodium hydrogen carbonate solution (20 mL) were added to the residue and the mixture was vigorously stirred. A solution of acetic anhydride (0.048 mL) in ethyl acetate (2 mL) was added to the mixture, and the mixture was stirred at room temperature for 1 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. The residue was dissolved in ethanol (3 mL) and 1N hydrochloric acid/ethyl acetate (0.5 mL) was added. The reaction mixture was concentrated under reduced pressure and the residue was crystallized from isopropyl alcohol/ethyl acetate to give the title compound (179 mg) as a white powder. $^1$H-NMR (DMSO-$d_6$) δ: 1.80 (3H, s), 3.44 (2H, q, J = 6.5 Hz), 4.65 (2H, t, J = 6.9 Hz), 6.68 (1H, d, J = 3.0 Hz), 7.14 (1H, dd, J = 2.4 Hz, 8.7 Hz), 7.19 (1H, d, J = 8.9 Hz), 7.46 (1H, dd, J = 0.7 Hz, 5.4 Hz), 7.60 (1H, dd, J = 2.5 Hz, 8.9 Hz), 7.67 (1H, d, J = 2.4 Hz), 7.71 (1H, d, J = 5.4 Hz), 7.92 (1H, d, J = 2.5 Hz), 7.93 (1H, d, J = 8.7 Hz), 7.99 (1H, d, J = 3.0 Hz), 8.45 (1H, t, J = 5.7 Hz), 8.72 (1H, s), 10.24 (1H, br s).

Example 53

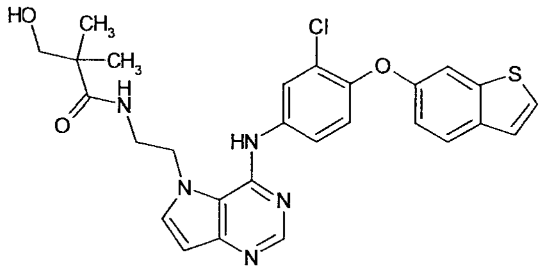

Production of N-[2-(4-{[4-(1-benzothiophen-6-yloxy)-3-chlorophenyl]amino}-5H-pyrrolo[3,2-d]pyrimidin-5-yl)ethyl]-3-hydroxy-2,2-dimethylpropanamide

A mixture of tert-butyl [2-(4-{[4-(1-benzothiophen-6-yloxy)-3-chlorophenyl]amino}-5H-pyrrolo[3,2-d]pyrimidin-5-yl)ethyl]carbamate hydrochloride (229 mg), 4N hydrochloric acid/ethyl acetate (3 mL) and ethanol (1 mL) was stirred at 80°C for 2 hr. The reaction mixture was concentrated under reduced pressure. To a solution of the residue in N,N-dimethylformamide (5 mL) were added 3-hydroxy-2,2-dimethylpropanoic acid (71 mg), 1-ethyl-3-(3-dimethylaminopropyl)carbodiimide hydrochloride (115 mg), 1-hydroxybenzotriazole (81 mg) and triethylamine (0.167 mL), and the mixture was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure, and the residue was crystallized from ethyl acetate/diisopropyl ether to give the title compound (176 mg) as a white powder.
$^1$H-NMR (DMSO-$d_6$) δ: 0.99 (6H, s), 3.33 (2H, d, J = 5.1 Hz), 3.41 (2H, q, J = 6.4 Hz), 4.51 (2H, t, J = 6.7 Hz), 4.84 (1H, t, J = 5.2 Hz), 6.49 (1H, d, J = 3.1 Hz), 7.10 (1H, dd, J = 2.3 Hz, 8.7 Hz), 7.18 (1H, d, J = 8.9 Hz), 7.43 (1H, dd, J = 0.6 Hz, 5.5 Hz), 7.55 (1H, d, J = 2.3 Hz), 7.60 (1H, d, J = 3.1 Hz), 7.66 (1H, d, J = 5.5 Hz), 7.81-7.91 (3H, m), 8.08 (1H, d, J = 2.6 Hz), 8.34 (1H, s), 8.88 (1H, br s).
Example 54
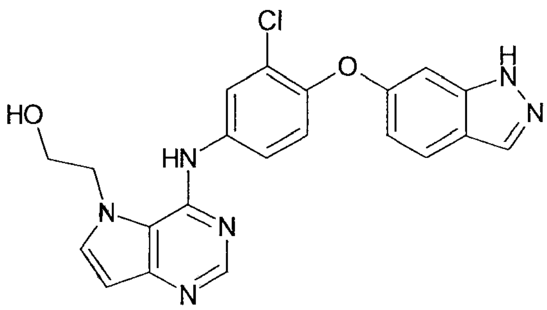

Production of 2-(4-{[3-chloro-4-(1H-indazol-6-yloxy)phenyl]amino}-5H-pyrrolo[3,2-d]pyrimidin-5-yl)ethanol

(i) Production of 1H-indazol-6-ol

1H-Indazol-6-amine (10 g) was dissolved in 47% sulfuric acid (40 g) and water (40 mL) with heating, and the solution was cooled to -10°C. 47% Sulfuric acid (40 g) and water (40 mL) were added, and a solution of sodium nitrite (5.69 g) in water (16 mL) was added dropwise. Water (5 mL) was added and the mixture was stirred for 10 min and then at room temperature for 30 min. Boric acid (6.96 g) was added, and the mixture was stirred at 110°C for 1 hr. Aqueous ammonia was added to the reaction mixture, and the precipitate was collected by filtration. The precipitate was subjected to silica gel column chromatography (eluent, ethyl acetate:hexane=50:50→100:0). The object fraction was concentrated under reduced pressure. The residue was filtered with ethyl acetate to give the title compound (2.56 g) as a vermillion powder.
$^1$H-NMR (DMSO-$d_6$) δ: 6.63 (1H, dd, J = 2.1 Hz, 8.6 Hz), 6.74-6.78 (1H, m), 7.52 (1H, d, J = 8.6 Hz), 7.83-7.87 (1H, m),

9.52 (1H, s), 12.55 (1H, br s).

(ii) Production of 6-(2-chloro-4-nitrophenoxy)-1H-indazole

A mixture of 1H-indazol-6-ol (1.34 g), 2-chloro-1-fluoro-4-nitrobenzene (1.76 g), potassium carbonate (2.07 g) and N,N-dimethylformamide (20 mL) was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=10:90→50:50). The object fraction was concentrated under reduced pressure to give the title compound (1.19 g) as a yellow solid.
$^1$H-NMR (DMSO-$d_6$) δ: 6.97 (1H, dd, J = 2.1 Hz, 8.7 Hz), 7.07 (1H, d, J = 9.1 Hz), 7.30-7.35 (1H, m), 7.89 (1H, d, J = 8.7 Hz), 8.12-8.20 (2H, m), 8.48 (1H, d, J = 2.6 Hz), 13.18 (1H, br s).

(iii) Production of 3-chloro-4-(1H-indazol-6-yloxy)aniline

A mixture of 6-(2-chloro-4-nitrophenoxy)-1H-indazole (1.16 g), reduced iron (992 mg), calcium chloride (247 mg) and 10% water-containing ethanol (30 mL) was stirred with heating under reflux for 5 hr. The reaction mixture was filtered to remove solid, and the filtrate was concentrated. Water was added to the residue, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, ethyl acetate:hexane=40:60→70:30). The object fraction was concentrated under reduced pressure to give the title compound (919 mg) as a yellow-orange amorphous solid.
$^1$H-NMR ($CDCl_3$) δ: 3.73 (2H, br s), 6.57 (1H, dd, J = 2.7 Hz, 8.6 Hz), 6.72-6.76 (1H, m), 6.79 (1H, d, J = 2.7 Hz), 6.91 (1H, dd, J = 2.1 Hz, 8.8 Hz), 6.93 (1H, d, J = 8.6 Hz), 7.65 (1H, dd, J = 0.5 Hz, 8.8 Hz), 7.99 (1H, d, J = 0.9 Hz), 10.56 (1H, br s).

(iv) Production of 2-(4-{[3-chloro-4-(1H-indazol-6-yloxy)phenyl]amino}-5H-pyrrolo[3,2-d]pyrimidin-5-yl)ethanol

The title compound (107 mg) was obtained as a pale-yellow powder in the same manner as in Example 50 (iv) and using 2-(4-chloro-5H-pyrrolo[3,2-d]pyrimidin-5-yl)ethyl benzoate (151 mg), 3-chloro-4-(1H-indazol-6-yloxy)aniline (104 mg), isopropyl alcohol (5 mL), methanol (5 mL), tetrahydrofuran (1 mL) and 1N aqueous sodium hydroxide solution (0.8 mL).
$^1$H-NMR (DMSO-$d_6$) δ: 3.84-3.93 (2H, m), 4.55 (2H, t, J = 4.6 Hz), 6.30 (1H, br s), 6.52 (1H, d, J = 3.1 Hz), 6.75-6.78 (1H, m), 6.90 (1H, dd, J = 2.3 Hz, 8.7 Hz), 7.28 (1H, d, J = 8.8 Hz), 7.61 (1H, dd, J = 2.6 Hz, 8.8 Hz), 7.67 (1H, d, J = 3.1 Hz), 7.76 (1H, d, J = 8.7 Hz), 7.99 (1H, d, J = 2.6 Hz), 8.01-8.03 (1H, m), 8.35 (1H, s), 9.87 (1H, br s), 12.80 (1H, br s).
Example 55
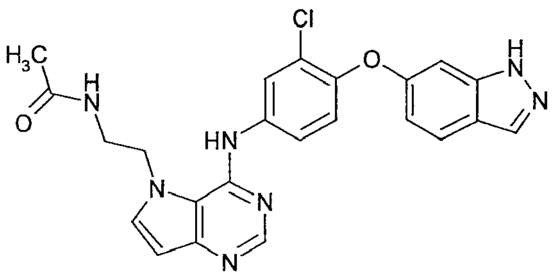

Production of N-[2-(4-{[3-chloro-4-(1H-indazol-6-yloxy)phenyl]amino}-5H-pyrrolo[3,2-d]pyrimidin-5-yl)ethyl] acetamide

(i) Production of 5-(2-aminoethyl)-N-[3-chloro-4-(1H-indazol-6-yloxy)phenyl]-5H-pyrrolo[3,2-d]pyrimidin-4-amine dihydrochloride

A mixture of tert-butyl [2-(4-chloro-5H-pyrrolo[3,2-d]pyrimidin-5-yl)ethyl]carbamate (594 mg), 3-chloro-4-(1H-indazol-6-yloxy)aniline (519 mg) and isopropyl alcohol (10 mL) was stirred at 80°C overnight. Saturated aqueous sodium hydrogen carbonate solution was added to the reaction mixture, and the mixture was extracted with ethyl acetate. The

organic layer was washed with saturated brine, and dried over anhydrous magnesium sulfate. The solvent was evaporated under reduced pressure and the obtained residue was subjected to silica gel column chromatography (eluent, methanol: ethyl acetate=0:100→10:90). The object fraction was concentrated under reduced pressure. 4N Hydrochloric acid/ethyl acetate (15 mL) and ethanol (15 mL) were added to the residue (915 mg), and the mixture was stirred at 80°C for 4 hr. Ethyl acetate was added to the reaction mixture and the precipitate was collected by filtration to give the title compound (874 mg) as a pale-yellow powder.
$^1$H-NMR (DMSO-$d_6$) δ: 3.23-3.38 (2H, m), 5.07 (2H, t, J = 6.2 Hz), 6.76 (1H, d, J = 3.2 Hz), 6.88-6.95 (2H, m), 7.28 (1H, d, J = 8.8 Hz), 7.62 (1H, dd, J = 2.5 Hz, 8.8 Hz), 7.81 (1H, dd, J = 0.8 Hz, 8.6 Hz), 7.92 (1H, d, J = 2.5 Hz), 8.06 (1H, d, J = 1.1 Hz), 8.10 (1H, d, J = 3.2 Hz), 8.37 (3H, br s), 8.77 (1H, s), 10.26 (1H, br s).

(ii) Production of N-[2-(4-{[3-chloro-4-(1H-indazol-6-yloxy)phenyl]amino}-5H-pyrrolo[3,2-d]pyrimidin-5-yl)ethyl]acetamide

Ethyl acetate (10 mL) and saturated aqueous sodium hydrogen carbonate solution (10 mL) were added to 5-(2-aminoethyl)-N-[3-chloro-4-(1H-indazol-6-yloxy)phenyl]-5H-pyrrolo[3,2-d]pyrimidin-4-amine dihydrochloride (197 mg) and the mixture was stirred vigorously. A solution of acetic anhydride (0.057 mL) in ethyl acetate (2 mL) was added to the mixture, and the mixture was stirred at room temperature for 1 hr. Water was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. The residue was crystallized from isopropyl alcohol/ethyl acetate to give the title compound (142 mg) as a white powder.
$^1$H-NMR (DMSO-$d_6$) δ: 1.81 (3H, s), 3.37 (2H, q, J = 6.4 Hz), 4.52 (2H, t, J = 6.9 Hz), 6.51 (1H, d, J = 3.1 Hz), 6.77-6.80 (1H, m), 6.92 (1H, dd, J = 2.1 Hz, 8.8 Hz), 7.26 (1H, d, J = 8.8 Hz), 7.65 (1H, d, J = 3.1 Hz), 7.75 (1H, dd, J = 2.6 Hz, 8.8 Hz), 7.77 (1H, d, J = 8.8 Hz), 8.01-8.05 (2H, m), 8.28 (1H, t, J = 5.6 Hz), 8.34 (1H, s), 8.81 (1H, br s), 12.82 (1H, br s).
Example 56
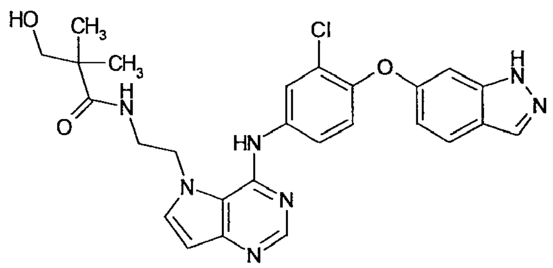

Production of N-[2-(4-{[3-chloro-4-(1H-indazol-6-yloxy)phenyl]amino}-5H-pyrrolo[3,2-d]pyrimidin-5-yl)ethyl]-3-hydroxy-2,2-dimethylpropanamide

A mixture of 5-(2-aminoethyl)-N-[3-chloro-4-(1H-indazol-6-yloxy)phenyl]-5H-pyrrolo[3,2-d]pyrimidin-4-amine dihydrochloride (197 mg), 3-hydroxy-2,2-dimethylpropanoic acid (71 mg), 1-ethyl-3-(3-dimethylaminopropyl)carbodiimide hydrochloride (115 mg), 1-hydroxybenzotriazole (81 mg), triethylamine (0.167 mL) and N,N-dimethylformamide (5 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure, and the residue was crystallized from ethyl acetate/diisopropyl ether to give the title compound (130 mg) as a white powder.
$^1$H-NMR (DMSO-$d_6$) δ: 0.99 (6H, s), 3.31-3.36 (2H, m), 3.41 (2H, q, J = 6.4 Hz), 4.51 (2H, t, J = 6.8 Hz), 4.84 (1H, t, J = 5.3 Hz), 6.49 (1H, d, J = 3.0 Hz), 6.77-6.81 (1H, m), 6.91 (1H, dd, J = 2.2 Hz, 8.8 Hz), 7.25 (1H, d, J = 8.9 Hz), 7.60 (1H, d, J = 3.0 Hz), 7.77 (1H, d, J = 8.8 Hz), 7.82-7.90 (2H, m), 8.01-8.04 (1H, m), 8.09 (1H, d, J = 2.6 Hz), 8.35 (1H, s), 8.91 (1H, br s), 12.81 (1H, br s).
Example 57
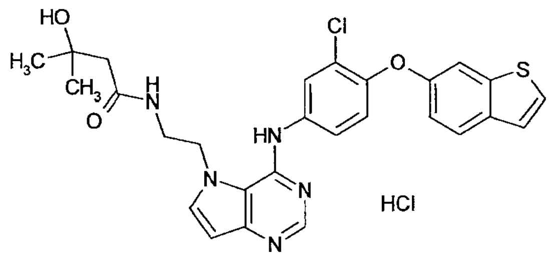

Production of N-[2-(4-{[4-(1-benzothiophen-6-yloxy)-3-chlorophenyl]amino}-5H-pyrrolo[3,2-d]pyrimidin-5-yl) ethyl]-3-hydroxy-3-methylbutanamide hydrochloride

(i) Production of 5-(2-aminoethyl)-N-[4-(1-benzothiophen-6-yloxy)-3-chlorophenyl]-5H-pyrrolo[3,2-d]pyrimidin-4-amine dihydrochloride

A mixture of tert-butyl [2-(4-{[4-(1-benzothiophen-6-yloxy)-3-chlorophenyl]amino}-5H-pyrrolo[3,2-d]pyrimidin-5-yl)ethyl]carbamate hydrochloride (5.04 g), 4N hydrochloric acid/ethyl acetate (50 mL) and ethanol (20 mL) was stirred at 80°C for 2 hr. Ethyl acetate was added to the reaction mixture and the precipitate was collected by filtration to give the title compound (4.34 g) as a yellow powder.
$^1$H-NMR (DMSO-$d_6$) δ: 3.24-3.37 (2H, m), 5.06 (2H, t, J = 6.2 Hz), 6.75 (1H, d, J = 3.1 Hz), 7.13 (1H, dd, J = 2.3 Hz, 8.7 Hz), 7.19 (1H, d, J = 8.7 Hz), 7.46 (1H, dd, J = 0.7 Hz, 5.4 Hz), 7.59 (1H, dd, J = 2.5 Hz, 8.7 Hz), 7.65 (1H, d, J = 2.3 Hz), 7.71 (1H, d, J = 5.4 Hz), 7.90 (1H, d, J = 2.5 Hz), 7.93 (1H, d, J = 8.7 Hz), 8.09 (1H, d, J = 3.1 Hz), 8.38 (3H, br s), 8.73 (1H, s), 10.20 (1H, br s).

(ii)N-[2-(4-{[4-(1-benzothiophen-6-yloxy)-3-chlorophenyl]amino}-5H-pyrrolo[3,2-d]pyrimidin-5-yl)ethyl]-3-hydroxy-3-methylbutanamide hydrochloride

A mixture of 5-(2-aminoethyl)-N-[4-(1-benzothiophen-6-yloxy)-3-chlorophenyl]-5H-pyrrolo[3,2-d]pyrimidin-4-amine dihydrochloride (204 mg), 3-hydroxy-3-methylbutanoic acid (71 mg), 1-ethyl-3-(3-dimethylaminopropyl)carbodiimide hydrochloride (115 mg), 1-hydroxybenzotriazole (81 mg), triethylamine (0.167 mL) and N,N-dimethylformamide (5 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→20:80). The object fraction was concentrated under reduced pressure. The residue was dissolved in ethyl acetate (2 mL)/ethanol (2 mL), and 1N hydrochloric acid/ ethyl acetate (0.5 mL) was added. The reaction mixture was concentrated under reduced pressure and the residue was crystallized from ethanol/ethyl acetate to give the title compound (220 mg) as a white powder.
$^1$H-NMR (DMSO-$d_6$) δ: 1.12 (6H, s), 2.20 (2H, s), 3.43-3.56 (2H, m), 4.65 (2H, t, J = 6.8 Hz), 6.67 (1H, d, J = 3.2 Hz), 7.13 (1H, dd, J = 2.3 Hz, 8.7 Hz), 7.19 (1H, d, J = 8.9 Hz), 7.46 (1H, dd, J = 0.8 Hz, 5.5 Hz), 7.62-7.68 (2H, m), 7.71 (1H, d, J = 5.5 Hz), 7.90-7.95 (2H, m), 7.98 (1H, d, J = 3.2 Hz), 8.38 (1H, t, J = 5.8 Hz), 8.72 (1H, s), 10.25 (1H, br s).
Example 58
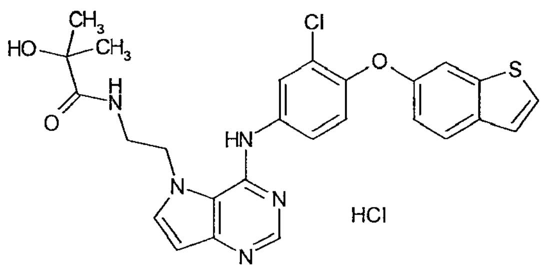

Production of N-[2-(4-{[4-(1-benzothiophen-6-yloxy)-3-chlorophenyl]amino}-5H-pyrrolo[3,2-d]pyrimidin-5-yl)ethyl]-2-hydroxy-2-methylpropanamide hydrochloride

The title compound (191 mg) was obtained as a white powder in the same manner as in Example 57 (ii) and using 5-(2-aminoethyl)-N-[4-(1-benzothiophen-6-yloxy)-3-chlorophenyl]-5H-pyrrolo[3,2-d]pyrimidin-4-amine dihydrochloride (204 mg), 2-hydroxy-2-methylpropanoic acid (62 mg), 1-ethyl-3-(3-dimethylaminopropyl)carbodiimide hydrochloride (115 mg), 1-hydroxybenzotriazole (81 mg), triethylamine (0.167 mL), N,N-dimethylformamide (5 mL), ethyl acetate (2 mL)/ethanol (2 mL) and 1N hydrochloric acid/ethyl acetate (0.5 mL).
$^1$H-NMR (DMSO-$d_6$) δ: 1.12 (6H, s), 3.46-3.56 (2H, m), 4.70 (2H, t, J = 6.1 Hz), 6.65 (1H, d, J = 3.2 Hz), 7.14 (1H, dd, J = 2.3 Hz, 8.7 Hz), 7.20 (1H, d, J = 8.7 Hz), 7.46 (1H, dd, J = 0.8 Hz, 5.4 Hz), 7.62 (1H, dd, J = 2.5 Hz, 8.7 Hz), 7.66 (1H, d, J = 2.3 Hz), 7.71 (1H, d, J = 5.4 Hz), 7.89-7.96 (3H, m), 8.05 (1H, t, J = 5.9 Hz), 8.71 (1H, s), 10.04 (1H, br s).
Example 59
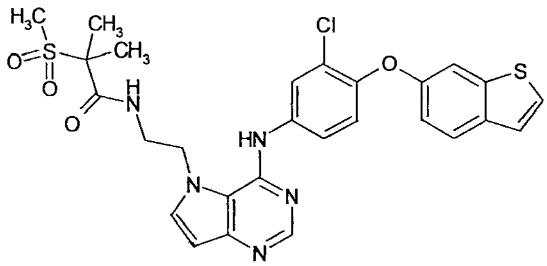

Production of N-[2-(4-{[4-(1-benzothiophen-6-yloxy)-3-chlorophenyl]amino}-5H-pyrrolo[3,2-d]pyrimidin-5-yl)ethyl]-2-methyl-2-(methylsulfonyl)propanamide

A mixture of 5-(2-aminoethyl)-N-[4-(1-benzothiophen-6-yloxy)-3-chlorophenyl]-5H-pyrrolo[3,2-d]pyrimidin-4-amine dihydrochloride (204 mg), 2-methyl-2-(methylsulfonyl)propanoic acid (100 mg), 1-ethyl-3-(3-dimethylaminopropyl) carbodiimide hydrochloride (115 mg), 1-hydroxybenzotriazole (81 mg), triethylamine (0.167 mL) and N,N-dimethylformamide (5 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→15:85). The object fraction was concentrated under reduced pressure, and the residue was crystallized from ethyl acetate/diisopropyl ether to give the title compound (211 mg) as a white powder.
$^1$H-NMR (DMSO-$d_6$) δ: 1.41 (6H, s), 2.96 (3H, s), 3.47 (2H, q, J = 5.6 Hz), 4.58 (2H, t, J = 6.3 Hz), 6.48 (1H, d, J = 3.1 Hz), 7.09 (1H, dd, J = 2.4 Hz, 8.7 Hz), 7.19 (1H, d, J = 8.9 Hz), 7.43 (1H, dd, J = 0.8 Hz, 5.5 Hz), 7.54 (1H, d, J = 2.4 Hz), 7.57 (1H, d, J = 3.1 Hz), 7.66 (1H, d, J = 5.5 Hz), 7.72 (1H, dd, J = 2.6 Hz, 8.9 Hz), 7.88 (1H, d, J = 8.7 Hz), 7.98 (1H, d, J = 2.6 Hz), 8.20 (1H, t, J = 5.5 Hz), 8.34 (1H, s), 8.66 (1H, br s).
Example 60
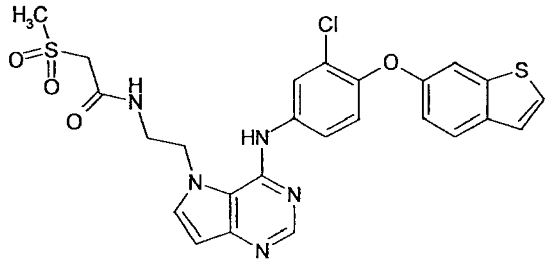

Production of N-[2-(4-{[4-(1-benzothiophen-6-yloxy)-3-chlorophenyl]amino}-5H-pyrrolo[3,2-d]pyrimidin-5-yl)ethyl]-2-(methylsulfonyl)acetamide

The title compound (198 mg) was obtained as a white powder in the same manner as in Example 59 and using 5-(2-aminoethyl)-N-[4-(1-benzothiophen-6-yloxy)-3-chlorophenyl]-5H-pyrrolo[3,2-d]pyrimidin-4-amine dihydrochloride (204 mg), methylsulfonylacetic acid (83 mg), 1-ethyl-3-(3-dimethylaminopropyl)carbodiimide hydrochloride (115 mg), 1-hydroxybenzotriazole (81 mg), triethylamine (0.167 mL), N,N-dimethylformamide (5 mL).
$^1$H-NMR (DMSO-$d_6$) δ: 3.10 (3H, s), 3.46 (2H, q, J = 6.2 Hz), 4.05 (2H, s), 4.57 (2H, t, J = 6.5 Hz), 6.50 (1H, d, J = 3.2 Hz), 7.09 (1H, dd, J = 2.4 Hz, 8.7 Hz), 7.19 (1H, d, J = 8.9 Hz), 7.43 (1H, dd, J = 0.6 Hz, 5.4 Hz), 7.55 (1H, d, J = 2.4 Hz), 7.63 (1H, d, J = 3.2 Hz), 7.66 (1H, d, J = 5.4 Hz), 7.71 (1H, dd, J = 2.5 Hz, 8.9 Hz), 7.88 (1H, d, J = 8.7 Hz), 7.96 (1H, d, J = 2.5 Hz), 8.34 (1H, s), 8.63-8.71 (1H, m), 8.67 (1H, br s).
Example 61
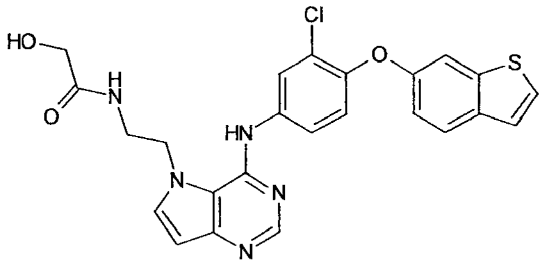

Production of N-[2-(4-{[4-(1-benzothiophen-6-yloxy)-3-chlorophenyl]amino}-5H-pyrrolo[3,2-d]pyrimidin-5-yl)ethyl]-2-hydroxyacetamide

The title compound (146 mg) was obtained as a white powder in the same manner as in Example 59 and using 5-(2-aminoethyl)-N-[4-(1-benzothiophen-6-yloxy)-3-chlorophenyl]-5H-pyrrolo[3,2-d]pyrimidin-4-amine dihydrochloride (204 mg), hydroxyacetic acid (46 mg), 1-ethyl-3-(3-dimethylaminopropyl)carbodiimide hydrochloride (115 mg), 1-hydroxybenzotriazole (81 mg), triethylamine (0.167 mL), N,N-dimethylformamide (5 mL).
$^1$H-NMR (DMSO-$d_6$) δ: 3.47 (2H, q, J = 6.7 Hz), 3.79 (2H, d, J = 5.6 Hz), 4.55 (2H, t, J = 6.9 Hz), 5.55 (1H, t, J = 5.6 Hz), 6.49 (1H, d, J = 3.1 Hz), 7.10 (1H, dd, J = 2.4 Hz, 8.7 Hz), 7.18 (1H, d, J = 8.9 Hz), 7.43 (1H, dd, J = 0.6 Hz, 5.5 Hz), 7.55 (1H, d, J = 2.4 Hz), 7.61 (1H, d, J = 3.1 Hz), 7.66 (1H, d, J = 5.5 Hz), 7.69 (1H, dd, J = 2.6 Hz, 8.9 Hz), 7.88 (1H, d, J = 8.7 Hz), 7.99 (1H, d, J = 2.6 Hz), 8.13 (1H, t, J = 5.7 Hz), 8.32 (1H, s), 8.71 (1H, br s).
Example 62
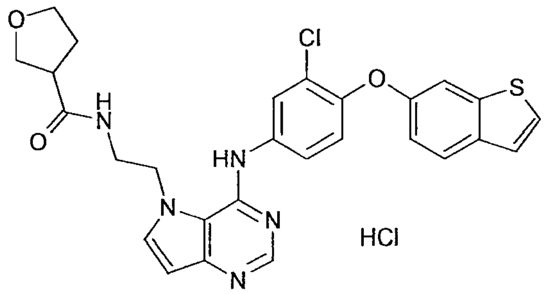

Production of N-[2-(4-{[4-(1-benzothiophen-6-yloxy)-3-chlorophenyl]amino}-5H-pyrrolo[3,2-d]pyrimidin-5-yl)ethyl]tetrahydrofuran-3-carboxamide hydrochloride

The title compound (207 mg) was obtained as a white powder in the same manner as in Example 57 (ii) and using 5-(2-aminoethyl)-N-[4-(1-benzothiophen-6-yloxy)-3-chlorophenyl]-5H-pyrrolo[3,2-d]pyrimidin-4-amine dihydrochloride (204 mg), tetrahydrofuran-3-carboxylic acid (70 mg), 1-ethyl-3-(3-dimethylaminopropyl)carbodiimide hydrochloride (115 mg), 1-hydroxybenzotriazole (81 mg), triethylamine (0.167 mL), N,N-dimethylformamide (5 mL), ethyl acetate (2 mL)/ethanol (2 mL), 1N hydrochloric acid/ethyl acetate (0.5 mL).
$^1$H-NMR (DMSO-$d_6$) δ: 1.76-1.96 (2H, m), 2.79-2.92 (1H, m), 3.39-3.78 (6H, m), 4.72 (2H, t, J = 6.3 Hz), 6.68 (1H, d, J = 3.2 Hz), 7.14 (1H, dd, J = 2.3 Hz, 8.7 Hz), 7.21 (1H, d, J = 8.9 Hz), 7.45 (1H, dd, J = 0.6 Hz, 5.4 Hz), 7.62-7.68 (2H, m), 7.71 (1H, d, J = 5.4 Hz), 7.90-7.98 (3H, m), 8.38 (1H, t, J = 5.6 Hz), 8.74 (1H, s), 10.16 (1H, br s).

Example 63

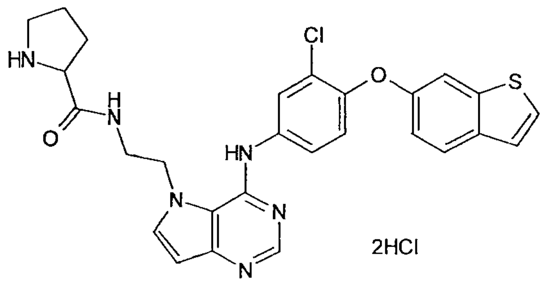

Production of N-[2-(4-{[4-(1-benzothiophen-6-yloxy)-3-chlorophenyl]amino}-5H-pyrrolo[3,2-d]pyrimidin-5-yl)ethyl]prolinamide dihydrochloride

A mixture of 5-(2-aminoethyl)-N-[4-(1-benzothiophen-6-yloxy)-3-chlorophenyl]-5H-pyrrolo[3,2-d]pyrimidin-4-amine dihydrochloride (407 mg), 1-(tert-butoxycarbonyl)proline (258 mg), 1-ethyl-3-(3-dimethylaminopropyl)carbodiimide hydrochloride (230 mg), 1-hydroxybenzotriazole (162 mg), triethylamine (0.335 mL) and N,N-dimethylformamide (10 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, ethyl acetate:hexane=50:50→100:0→methanol:ethyl acetate=10:90). The object fraction was concentrated under reduced pressure. The residue was crystallized from ethyl acetate/diisopropyl ether to give tert-butyl 2-({[2-(4-{[4-(1-benzothiophen-6-yloxy)-3-chlorophenyl]amino}-5H-pyrrolo[3,2-d]pyrimidin-5-yl)ethyl]amino}carbonyl)pyrrolidine-1-carboxylate (493 mg) as a white powder. To tert-butyl 2-({[2-(4-{[4-(1-benzothiophen-6-yloxy)-3-chlorophenyl]amino}-5H-pyrrolo[3,2-d]pyrimidin-5-yl)ethyl]amino}carbonyl)pyrrolidine-1-carboxylate (412 mg) were added concentrated hydrochloric acid (3 mL) and ethanol (9 mL), and the mixture was stirred at 60°C for 2 hr. The reaction mixture was concentrated under reduced pressure, and the residue was crystallized from ethanol/ethyl acetate to give the title compound (368 mg) as a pale-yellow powder.
$^1$H-NMR (DMSO-$d_6$) δ: 1.55-1.92 (3H, m), 2.08-2.24 (1H, m), 3.06-3.19 (2H, m), 3.35-3.79 (2H, m), 3.98-4.13 (1H, m), 4.70-5.00 (2H, m), 6.67 (1H, d, J = 3.1 Hz), 7.14 (1H, dd, J = 2.4 Hz, 8.7 Hz), 7.20 (1H, d, J = 8.9 Hz), 7.46 (1H, dd, J = 0.6 Hz, 5.5 Hz), 7.62 (1H, dd, J = 2.5 Hz, 8.9 Hz), 7.65 (1H, d, J = 2.4 Hz), 7.71 (1H, d, J = 5.5 Hz), 7.92 (1H, d, J = 2.5 Hz), 7.93 (1H, d, J = 8.7 Hz), 7.98 (1H, d, J = 3.1 Hz), 8.37-8.53 (1H, m), 8.73 (1H, s), 8.92 (1H, t, J = 5.6 Hz), 9.87-10.03 (1H, m), 10.15 (1H, br s).

Example 64

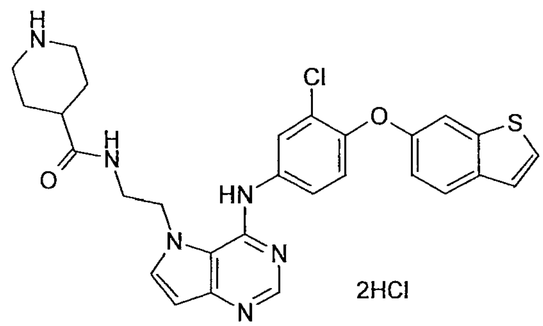

Production of N-[2-(4-{[4-(1-benzothiophen-6-yloxy)-3-chlorophenyl]amino}-5H-pyrrolo[3,2-d]pyrimidin-5-yl)ethyl]piperidine-4-carboxamide dihydrochloride

A mixture of 5-(2-aminoethyl)-N-[4-(1-benzothiophen-6-yloxy)-3-chlorophenyl]-5H-pyrrolo[3,2-d]pyrimidin-4-amine dihydrochloride (407 mg), 1-(tert-butoxycarbonyl)piperidine-4-carboxylic acid (275 mg), 1-ethyl-3-(3-dimethylaminopropyl)carbodiimide hydrochloride (230 mg), 1-hydroxybenzotriazole (162 mg), triethylamine (0.335 mL) and N,N-dimethylformamide (10 mL) was stirred at room temperature overnight. Water was added to the reaction mixture, and the mixture was extracted with ethyl acetate. The organic layer was washed successively with water and saturated brine, and dried over anhydrous magnesium sulfate. The solvent was evaporated under reduced pressure and the obtained residue was subjected to silica gel column chromatography (eluent, methanol:ethyl acetate=0:100→10:90). The object fraction was concentrated under reduced pressure. Concentrated hydrochloric acid (3 mL) and ethanol (9 mL) were added to the residue, and the mixture was stirred at 60°C for 3 hr. The reaction mixture was concentrated under reduced pressure, and the residue was crystallized from ethanol/ethyl acetate to give the title compound (432 mg) as a pale-yellow powder.
$^1$H-NMR (DMSO-$d_6$) δ: 1.52-1.81 (4H, m), 2.24-2.42 (1H, m), 2.71-2.92 (2H, m), 3.13-3.27 (2H, m), 3.32-3.62 (2H, m), 4.71 (2H, t, J = 6.0 Hz), 6.66 (1H, d, J = 3.2 Hz), 7.14 (1H, dd, J = 2.3 Hz, 8.7 Hz), 7.20 (1H, d, J = 8.8 Hz), 7.46 (1H, d, J = 5.5 Hz), 7.63 (1H, dd, J = 2.6 Hz, 8.8 Hz), 7.66 (1H, d, J = 2.3 Hz), 7.71 (1H, d, J = 5.5 Hz), 7.90-7.96 (3H, m), 8.36 (1H, t, J = 5.6 Hz), 8.55-8.76 (1H, m), 8.72 (1H, s), 8.87-9.05 (1H, m), 10.11 (1H, br s).
Example 65
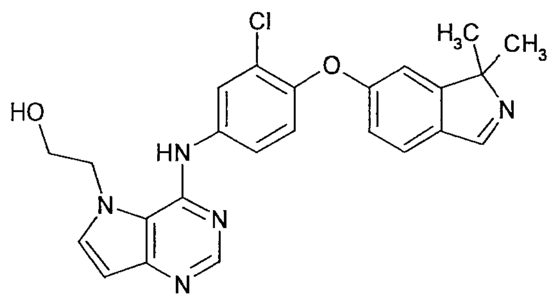

Production of 2-[4-({3-chloro-4-[(1,1-dimethyl-1H-isoindol-6-yl)oxy]phenyl}amino)-5H-pyrrolo[3,2-d]pyrimidin-5-yl]ethanol

(i) Production of 6-methoxy-1,1-dimethylisoindoline

To a solution of 5-methoxy-3,3-dimethylisoindolin-1-one (750 mg) in tetrahydrofuran (30 mL) was added lithium aluminum hydride (223 mg), and the mixture was stirred for 21 hr. Under ice-cooling, water (0.25 mL), 1N aqueous sodium hydroxide solution (0.25 mL) and water (0.75 mL) were added, and the insoluble substance was filtered off. The filtrate was concentrated to give the title compound (654 mg) as a pale-red oil.
$^1$H-NMR ($CDCl_3$) δ: 1.41 (6H, s), 3.81 (3H, s), 4.11 (2H, s), 6.68 (1H, d, J = 2.4 Hz), 6.73 (1H, dd, J = 2.4 Hz, 8.1Hz), 7.09 (1H, d, J = 8.1 Hz).

(ii) Production of 6-methoxy-1,1-dimethyl-1H-isoindole

To a solution of 6-methoxy-1,1-dimethylisoindoline (654 mg) in ethyl acetate (20 mL) was added manganese dioxide (3.21 g), and the mixture was stirred at room temperature for 18 hr. Manganese dioxide is filtered off, and the filtrate was concentrated. The residue was separated and purified by silica gel column chromatography (eluent, ethyl acetate:hexane=70:30→100:0) to give the title compound (474 mg) as a yellow oil.
$^1$H-NMR ($CDCl_3$) δ: 1.45 (6H, s), 3.87 (3H, s), 6.86 (1H, dd, J = 2.4 Hz, 8.4 Hz), 6.95 (1H, d, J = 2.4 Hz), 7.43 (1H, d, J = 8.4 Hz), 8.28 (1H, s).

(iii) Production of 1,1-dimethyl-1H-isoindol-6-ol

A mixture of 6-methoxy-1,1-dimethyl-1H-isoindole (452 mg) and 48% hydrobromic acid (8 mL) was stirred at 100°C for 2 days. The mixture was allowed to cool to room temperature, ethyl acetate and saturated brine were added,

and the mixture was extracted twice with ethyl acetate. The organic layer was dried over anhydrous magnesium sulfate and concentrated under reduced pressure. The obtained crystals were recrystallized from ethyl acetate to give the title compound (265 mg) as pale-yellow crystals.
$^1$H-NMR ($CDCl_3$) δ: 1.47 (6H, s), 6.83 (1H, dd, J = 2.1 Hz, 7.8 Hz), 6.93 (1H, d, J = 2.1 Hz), 7.42 (1H, d, J = 7.8 Hz), 8.31 (1H, s).

(iv) Production of 6-(2-chloro-4-nitrophenoxy)-1,1-dimethyl-1H-isoindole

To a solution of 2-chloro-1-fluoro-4-nitrobenzene (315 mg) and 1,1-dimethyl-1H-isoindol-6-ol (250 mg) in N,N-dimethylformamide (4 mL) was added potassium carbonate (257 mg) under ice-cooling, and the mixture was stirred at room temperature for 4 hr. Saturated brine was added to the reaction system under ice-cooling, and the mixture was extracted with ethyl acetate. The organic layer was dried over anhydrous magnesium sulfate and concentrated under reduced pressure. The residue was separated and purified by silica gel column chromatography (eluent, ethyl acetate: hexane=50:50→85:15), and the obtained crystals were recrystallized from hexane/diisopropyl ether to give the title compound (372 mg) as colorless crystals.
$^1$H-NMR ($CDCl_3$) δ: 1.48 (6H, s), 6.95 (1H, d, J = 9.0 Hz), 7.04 (1H, dd, J = 2.4 Hz, 8.4 Hz), 7.15-7.20 (1H, m), 7.57 (1H, d, J = 7.5 Hz), 8.08 (1H, dd, J = 3.0 Hz, 9.0 Hz), 8.35-8.40 (1H, m), 8.40 (1H, d, J = 2.4 Hz).

(v) Production of 3-chloro-4-[(1,1-dimethyl-1H-isoindol-6-yl)oxy]aniline

To a solution of 6-(2-chloro-4-nitrophenoxy)-1,1-dimethyl-1H-isoindole (125 mg) in ethanol (10 mL)/water (1 mL) was added calcium chloride (22 mg), the mixture was stirred at 90°C, and reduced iron (132 mg) was added. The reaction mixture was stirred at 90°C for 2 hr, and the insoluble substance was filtered off. The filtrate was concentrated under reduced pressure, and ethyl acetate and saturated brine were added. The organic layer was extracted, dried over anhydrous magnesium sulfate, and concentrated under reduced pressure. The residue was separated and purified by silica gel column chromatography (eluent, ethyl acetate:hexane=80:20→100:0) to give the title compound (88 mg) as a white powder.
$^1$H-NMR ($CDCl_3$) δ: 1.42 (6H, s), 3.70 (2H, s), 6.59 (1H, dd, J = 2.4 Hz, 8.7 Hz), 6.80-6.85 (2H, m), 6.90-6.95 (2H, m), 7.41 (1H, d, J = 8.4 Hz), 8.29 (1H, s).

(vi) Production of 2-[4-({3-chloro-4-[(1,1-dimethyl-1H-isoindol-6-yl)oxy]phenyl}amino)-5H-pyrrolo[3,2-d]pyrimidin-5-yl] ethanol

A solution of 2-(4-chloro-5H-pyrrolo[3,2-d]pyrimidin-5-yl)ethyl benzoate (91 mg), 3-chloro-4-[(1,1-dimethyl-1H-isoindol-6-yl)oxy]aniline (87 mg) and pyridine hydrochloride (5 mg) in isopropyl alcohol (8 mL) was stirred at 80°C for 2 days. Under ice-cooling, saturated aqueous sodium hydrogen carbonate solution was added to the reaction system, and the mixture was extracted with ethyl acetate. The extract was washed with saturated brine, dried over anhydrous magnesium sulfate, and concentrated under reduced pressure, and the residue was separated and purified by silica gel column chromatography (eluent, ethyl acetate:methanol=100:0→95:5→90:10). 1N Aqueous sodium hydroxide solution (2.0 mL) and tetrahydrofuran (4.0 mL) were added to the obtained compound, and the mixture was stirred at room temperature for 7 hr. The reaction system was neutralized with 1N hydrochloric acid, and saturated aqueous sodium hydrogen carbonate solution and saturated brine were added. The mixture was extracted with ethyl acetate. The organic layer was dried over anhydrous magnesium sulfate and concentrated under reduced pressure. The obtained crystals were recrystallized from ethyl acetate/diisopropyl ether to give the title compound (73 mg) as pale-yellow crystals.
$^1$H-NMR ($CDCl_3$) δ: 1.40 (6H, s), 4.15-4.20 (2H, m), 4.40-4.45 (2H, m), 6.28 (1H, d, J = 3.0 Hz), 6.90-7.00 (2H, m), 7.05-7.15 (2H, m), 7.44 (1H, d, J = 8.1 Hz), 7.50-7.60 (1H, m), 7.85 (1H, d, J = 2.1 Hz), 8.23 (1H, s), 8.32 (1H, s), 9.66 (1H, s).
Example 66
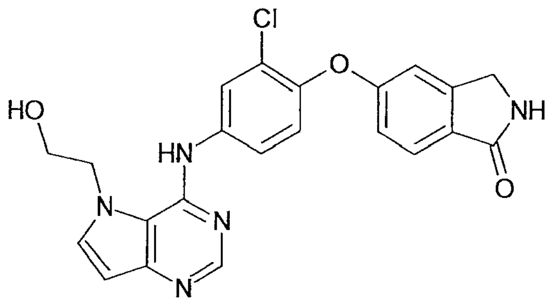

Production of 5-(2-chloro-4-{[5-(2-hydroxyethyl)-5H-pyrrolo[3,2-d]pyrimidin-4-yl]amino}phenoxy)isoindolin-1-one

(i) Production of 5-methoxyisoindolin-1-one

To a solution of methyl 4-methoxy-2-methylbenzoate (2.71 g) in benzotrifluoride (50 mL) were added N-bromosuccinimide (2.67 g) and 2,2-azobis(isobutyronitrile) (246 mg), and the mixture was stirred at 80°C for 16 hr. 0.1N Aqueous sodium hydroxide solution was added to the reaction system under ice-cooling, and the mixture was extracted with ethyl acetate. The organic layer was washed with saturated brine, dried over anhydrous magnesium sulfate, and concentrated under reduced pressure. The residue was separated and purified by silica gel column chromatography (eluent, ethyl acetate:hexane=20:80→50:50). The obtained compound was dissolved in tetrahydrofuran (10 mL)/methanol (5 mL), 28% aqueous ammonia (5.0 mL) was added, and the mixture was stirred at room temperature for 3 days. The reaction mixture was concentrated, saturated brine was added, and the mixture was extracted 3 times with ethyl acetate. The organic layer was dried over anhydrous magnesium sulfate and concentrated under reduced pressure, and the residue was recrystallized from ethyl acetate to give the title compound (0.95 g) as a white powder.
$^1$H-NMR ($CDCl_3$) δ: 3.88 (3H, s), 4.41 (2H, s), 6.39 (1H, br s), 6.90-7.05 (2H, m), 7.77 (1H, d, J = 8.4 Hz).

(ii) Production of 5-hydroxyisoindolin-1-one

The title compound (385 mg) was obtained as colorless crystals by reaction in the same manner as in Example 65(iii) and using 5-methoxyisoindolin-1-one (0.95 g) and 48% hydrobromic acid (15 mL).
$^1$H-NMR (95%$CDCl_3$+5%DMSO-$d_6$) δ: 4.32 (2H, s), 6.85-6.95 (2H, m), 7.30-7.45 (1H, br s), 7.62 (1H, d, J = 8.7 Hz).

(iii) Production of 5-(2-chloro-4-nitrophenoxy)isoindolin-1-one

The title compound (578 mg) was obtained as a pale-yellow powder by reaction in the same manner as in Example 65(iv) and using 5-hydroxyisoindolin-1-one (385 mg), 2-chloro-1-fluoro-4-nitrobenzene (525 mg), potassium carbonate (428 mg), N,N-dimethylformamide (10 mL).
$^1$H-NMR ($CDCl_3$) δ: 4.46 (2H, s), 6.47 (1H, br s), 7.03 (1H, d, J = 9.0 Hz), 7.10-7.20 (2H, m), 7.91 (1H, d, J = 8.4 Hz), 8.11 (1H, dd, J = 2.7 Hz, 9.0 Hz), 8.41 (1H, d, J = 2.7 Hz).

(iv) Production of 5-(4-amino-2-chlorophenoxy)isoindolin-1-one

To a solution of 5-(2-chloro-4-nitrophenoxy)isoindolin-1-one (152 mg) in ethyl acetate (5 mL)/methanol (10 mL) was added 5% platinum/activated carbon (25 mg) under nitrogen atmosphere. The reaction mixture was stirred under hydrogen atmosphere at room temperature for 1 hr, and 5% platinum/activated carbon was filtered off. The filtrate was concentrated under reduced pressure to give the title compound (141 mg) as a white amorphous form.
$^1$H-NMR ($CDCl_3$) δ: 3.73 (2H, br s), 4.37 (2H, s), 6.16 (1H, br s), 6.60 (1H, dd, J = 2.7 Hz, 8.4 Hz), 6.80 (1H, d, J = 2.7 Hz), 6.85-6.90 (1H, m), 6.94 (1H, d, J = 8.4 Hz), 6.95-7.00 (1H, m), 7.77 (1H, d, J = 8.4 Hz).

(v) Production of 5-(2-chloro-4-{[5-(2-hydroxyethyl)-5H-pyrrolo[3,2-d]pyrimidin-4-yl]amino}phenoxy)isoindolin-1-one

The title compound (118 mg) was obtained as colorless crystals by reaction in the same manner as in Example 65(vi) and using 2-(4-chloro-5H-pyrrolo[3,2-d]pyrimidin-5-yl)ethyl benzoate (151 mg), 5-(4-amino-2-chlorophenoxy)isoindolin-1-one (137 mg), pyridine hydrochloride (5 mg), isopropyl alcohol (10 mL), 1N aqueous sodium hydroxide solution

(2.0 mL), tetrahydrofuran (4.0 mL) and methanol (1.0 mL).
$^1$H-NMR (DMSO-$d_6$) δ: 3.85-3.90 (2H, m), 4.31 (2H, s), 4.50-4.60 (2H, m), 6.32 (1H, br s), 6.51 (1H, d, J = 3.3 Hz), 7.00-7.10 (2H, m), 7.30 (1H, d, J = 9.0 Hz), 7.60-7.70 (3H, m), 7.95-8.00 (1H, m), 8.34 (1H, s), 8.41 (1H, s), 9.89 (1H, br s).

Example 67

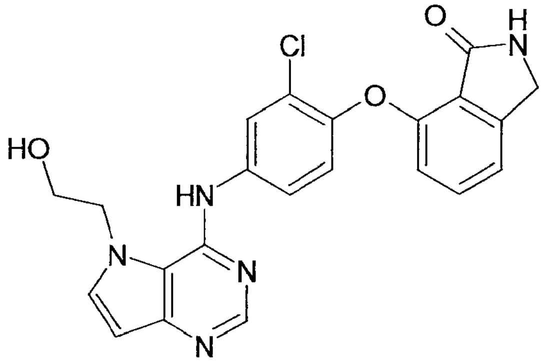

Production of 7-(2-chloro-4-{[5-(2-hydroxyethyl)-5H-pyrrolo[3,2-d]pyrimidin-4-yl]amino}phenoxy)isoindolin-1-one

(i) Production of 4-bromo-7-methoxyisoindolin-1-one

The title compound (2.53 g) was obtained as a white powder by reaction in the same manner as in Example 66(i) and using ethyl 2-methoxy-6-methylbenzoate (5.08 g), N-bromosuccinimide (9.79 g), 2,2-azobis (isobutyronitrile) (430 mg), benzotrifluoride (100 mL), 28% aqueous ammonia (4.0 mL) and tetrahydrofuran (10 mL)/methanol (4 mL).
$^1$H-NMR ($CDCl_3$) δ: 3.97 (3H, s), 4.29 (2H, s), 6.47 (1H, br s), 6.84 (1H, d, J = 8.7 Hz), 7.59 (1H, d, J = 8.7 Hz).

(ii) Production of 4-bromo-7-hydroxyisoindolin-1-one

The title compound (0.96 g) was obtained as pale-yellow crystals by reaction in the same manner as in Example 65(iii) and using 4-bromo-7-methoxyisoindolin-1-one (1.44 g) and 48% hydrobromic acid (25 mL).
$^1$H-NMR (95%$CDCl_3$+5%DMSO-$d_6$) δ: 4.28 (2H, s), 6.77 (1H, d, J = 8.7 Hz), 7.46 (1H, d, J = 8.7 Hz), 8.13 (1H, br s).

(iii) Production of 7-hydroxyisoindolin-1-one

To a solution of 4-bromo-7-hydroxyisoindolin-1-one (228 mg) in methanol (10 mL) was added 10% palladium/activated carbon (38 mg) under nitrogen atmosphere. The reaction mixture was stirred under hydrogen atmosphere at room temperature for 2 hr, and 10% palladium/activated carbon was filtered off. The filtrate was concentrated under reduced pressure to give the title compound (149 mg) as a yellow powder.
$^1$H-NMR (95%$CDCl_3$+5%DMSO-$d_6$) δ: 4.37 (2H, s), 6.79 (1H, d, J = 8.4 Hz), 6.94 (1H, d, J = 7.5 Hz), 7.30-7.45 (1H, m), 8.20 (1H, br s).

(iv) Production of 7-(2-chloro-4-nitrophenoxy)isoindolin-1-one

The title compound (177 mg) was obtained as a pale-brown powder by reaction in the same manner as in Example 65(iv) and using 7-hydroxyisoindolin-1-one (149 mg), 2-chloro-1-fluoro-4-nitrobenzene (203 mg), potassium carbonate (166 mg) and N,N-dimethylformamide (4.0 mL).
$^1$H-NMR (95%$CDCl_3$+5%DMSO-$d_6$) δ: 4.43 (2H, s), 6.82 (1H, d, J = 9.3 Hz), 7.06 (1H, d, J = 7.8 Hz), 7.41 (1H, d, J = 7.8 Hz), 7.55-7.70 (1H, m), 7.95-8.10 (2H, m), 8.34 (1H, d, J = 2.7 Hz).

(v) Production of 7-(4-amino-2-chlorophenoxy)isoindolin-1-one

The title compound (120 mg) was obtained as a pale-yellow amorphous by reaction in the same manner as in Example 66(iv) and using 7-(2-chloro-4-nitrophenoxy)isoindolin-1-one (122 mg), 5% platinum/activated carbon (20 mg) and ethyl acetate (5 mL)/methanol (10 mL).

$^1$H-NMR ($CDCl_3$) δ: 3.70 (2H, br s), 4.43 (2H, s), 6.37 (1H, br s), 6.52 (1H, d, J = 8.7 Hz), 6.55-6.60 (1H, m), 6.78 (1H, d, J = 2.4 Hz), 7.01 (1H, d, J = 8.1 Hz), 7.06 (1H, d, J = 7.5 Hz), 7.36 (1H, t, J = 8.1 Hz).

(vi) Production of 7-(2-chloro-4-{[5-(2-hydroxyethyl)-5H-pyrrolo[3,2-d]pyrimidin-4-yl]amino}phenoxy)isoindolin-1-one

The title compound (63 mg) was obtained as pale-yellow crystals by reaction in the same manner as in Example 65(vi) and using 2-(4-chloro-5H-pyrrolo[3,2-d]pyrimidin-5-yl)ethyl benzoate (121 mg), 7-(4-amino-2-chlorophenoxy)isoindolin-1-one (110 mg), pyridine hydrochloride (5 mg), isopropyl alcohol (10 mL), 1N aqueous sodium hydroxide solution (2.0 mL) and tetrahydrofuran (4.0 mL)/methanol (1.0 mL).
$^1$H-NMR (DMSO-$d_6$) δ: 3.80-3.90 (2H, m), 4.37 (2H, s), 4.50-4.60 (2H, m), 6.30 (1H, br s), 6.50 (1H, d, J = 3.0 Hz), 6.59 (1H, d, J = 9.0 Hz), 7.14 (1H, d, J = 9.0 Hz), 7.20-7.30 (1H, m), 7.48 (1H, t, J = 7.2 Hz), 7.55-7.60 (1H, m), 7.65 (1H, d, J = 3.0 Hz), 7.95-8.00 (1H, m), 8.33 (1H, s), 8.42 (1H, s), 9.84 (1H, br s).
Example 68
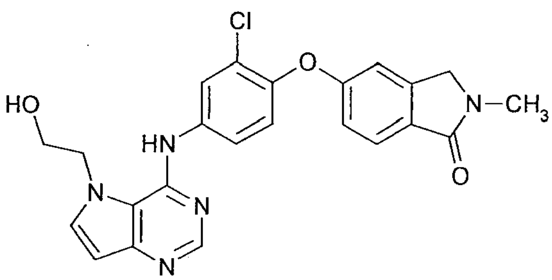

Production of 5-(2-chloro-4-{[5-(2-hydroxyethyl)-5H-pyrrolo[3,2-d]pyrimidin-4-yl]amino}phenoxy)-2-methylisoindolin-1-one

(i) Production of 5-(2-chloro-4-nitrophenoxy)-2-methylisoindolin-1-one

To a suspension of 5-(2-chloro-4-nitrophenoxy)isoindolin-1-one (229 mg) in N,N-dimethylformamide (4.0 mL) was added sodium hydride (33 mg, 60% in oil) under ice-cooling. After stirring at room temperature for 15 min, methyl iodide (0.19 mL) was added under ice-cooling, and the mixture was stirred at room temperature for 2 hr. Under ice-cooling, water (8 mL) was added, and the precipitate was collected by filtration and washed with water and diethyl ether to give the title compound (208 mg) as a yellow powder.
$^1$H-NMR ($CDCl_3$) δ: 3.21 (3H, s), 4.38 (2H, s), 6.99 (1H, d, J = 9.0 Hz), 7.10-7.20 (2H, m), 7.87 (1H, d, J = 8.1 Hz), 8.10 (1H, dd, J = 2.7 Hz, 9.0 Hz), 8.40 (1H, d, J = 2.7 Hz).

(ii) Production of 5-(4-amino-2-chlorophenoxy)-2-methylisoindolin-1-one

The title compound (143 mg) was obtained as a yellow amorphous by reaction in the same manner as in Example 66(iv) and using 5-(2-chloro-4-nitrophenoxy)-2-methylisoindolin-1-one (159 mg), 5% platinum/activated carbon (27 mg), ethyl acetate (5 mL)/methanol (10 mL).
$^1$H-NMR ($CDCl_3$) δ: 3.16 (3H, s), 3.72 (2H, br s), 4.28 (2H, s), 6.60 (1H, dd, J = 2.7 Hz, 8.7 Hz), 6.79 (1H, d, J = 2.7 Hz), 6.85-6.90 (1H, m), 6.90-7.00 (2H, m), 7.73 (1H, d, J = 8.1 Hz).

(iii) Production of 5-(2-chloro-4-{[5-(2-hydroxyethyl)-5H-pyrrolo[3,2-d]pyrimidin-4-yl]amino}phenoxy)-2-methylisoindolin-1-one

The title compound (111 mg) was obtained as pale-yellow crystals by reaction in the same manner as in Example 65(vi) and using 2-(4-chloro-5H-pyrrolo[3,2-d]pyrimidin-5-yl)ethyl benzoate (151 mg), 5-(4-amino-2-chlorophenoxy)-2-methylisoindolin-1-one (143 mg), pyridine hydrochloride (5 mg), isopropyl alcohol (10 mL), 1N aqueous sodium hydroxide solution (2.0 mL) and tetrahydrofuran (4.0 mL)/methanol (1.0 mL).
$^1$H-NMR (DMSO-$d_6$) δ: 3.03 (3H, s), 3.80-3.90 (2H, m), 4.39 (2H, s), 4.50-4.60 (2H, m), 6.30 (1H, br s), 6.45-6.55 (1H, m), 6.95-7.10 (2H, m), 7.29 (1H, d, J = 9.0 Hz), 7.55-7.70 (3H, m), 7.95-8.00 (1H, m), 8.33 (1H, s), 9.90 (1H, br s).
Example 69
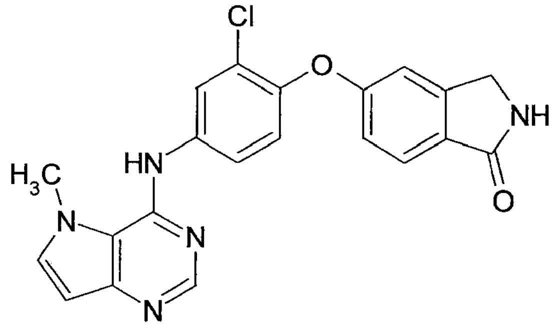

Production of 5-{2-chloro-4-[(5-methyl-5H-pyrrolo[3,2-d]pyrimidin-4-yl)amino]phenoxy}isoindolin-1-one

A mixture of 4-chloro-5-methyl-5H-pyrrolo[3,2-d]pyrimidine (84 mg), 5-(4-amino-2-chlorophenoxy)isoindolin-1-one (132 mg) and pyridine hydrochloride (5 mg) in isopropyl alcohol (10 mL) was stirred at 80°C for 22 hr. Under ice-cooling, saturated aqueous sodium hydrogen carbonate solution was added to the reaction system. The mixture was extracted with ethyl acetate, and the organic layer was washed with saturated brine, dried over anhydrous magnesium sulfate, and concentrated under reduced pressure. The obtained crystals were recrystallized from ethyl acetate/tetrahydrofuran/methanol to give the title compound (112 mg) as pale-yellow crystals.
$^1$H-NMR (DMSO-$d_6$) δ: 4.15 (3H, s), 4.32 (2H, s), 6.46 (1H, d, J = 2.4 Hz), 7.00-7.10 (2H, m), 7.28 (1H, d, J = 8.7 Hz), 7.55-7.65 (1H, m), 7.66 (1H, d, J = 9.3 Hz), 7.70-7.75 (1H, m), 7.95-8.00 (1H, m), 8.32 (1H, s), 8.42 (1H, s), 8.63 (1H, s).
Example 70
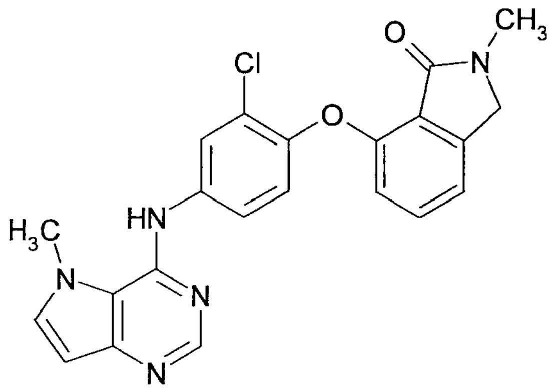

Production of 7-{2-chloro-4-[(5-methyl-5H-pyrrolo[3,2-d]pyrimidin-4-yl)amino]phenoxy}-2-methylisoindolin-1-one

The title compound (170 mg) was obtained as yellow crystals by reaction in the same manner as in Example 69 and using 4-chloro-5-methyl-5H-pyrrolo[3,2-d]pyrimidine (84 mg), 7-(4-amino-2-chlorophenoxy)-2-methylisoindolin-1-one (144 mg), pyridine hydrochloride (5 mg) and isopropyl alcohol (10 mL).
$^1$H-NMR (DMSO-$d_6$) δ: 3.03 (3H, s), 4.15 (3H, s), 4.46 (2H, s), 6.44 (1H, d, J = 3.0 Hz), 6.64 (1H, d, J = 7.5 Hz), 7.08 (1H, d, J = 9.0 Hz), 7.27 (1H, d, J = 7.5 Hz), 7.49 (1H, t, J = 7.5 Hz), 7.58 (1H, d, J = 3.0 Hz), 7.62 (1H, dd, J = 2.7 Hz, 9.0 Hz), 7.93 (1H, d, J = 2.7 Hz), 8.30 (1H, s), 8.56 (1H, s).
Example 71
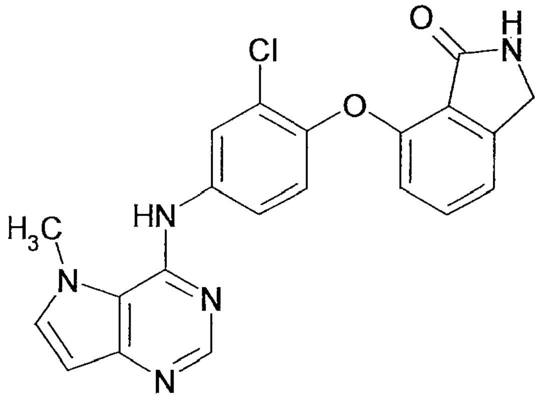

Production of 7-{2-chloro-4-[(5-methyl-5H-pyrrolo[3,2-d]pyrimidin-4-yl)amino]phenoxy}isoindolin-1-one

The title compound (110 mg) was obtained as pale-yellow crystals by reaction in the same manner as in Example 69 and using 4-chloro-5-methyl-5H-pyrrolo[3,2-d]pyrimidine (84 mg), 7-(4-amino-2-chlorophenoxy)isoindolin-1-one (137 mg), pyridine hydrochloride (5 mg) and isopropyl alcohol (10 mL). $^1$H-NMR (DMSO-$d_6$) δ: 4.15 (3H, s), 4.37 (2H, s), 6.44 (1H, d, J = 3.0 Hz), 6.61 (1H, d, J = 7.5 Hz), 7.10 (1H, d, J = 9.0 Hz), 7.25 (1H, d, J = 7.5 Hz), 7.49 (1H, t, J = 7.5 Hz), 7.58 (1H, d, J = 2.4 Hz), 7.63 (1H, dd, J = 2.4 Hz, 9.0 Hz), 7.93 (1H, d, J = 3.0 Hz), 8.30 (1H, s), 8.42 (1H, s), 8.56 (1H, s).
Example 72
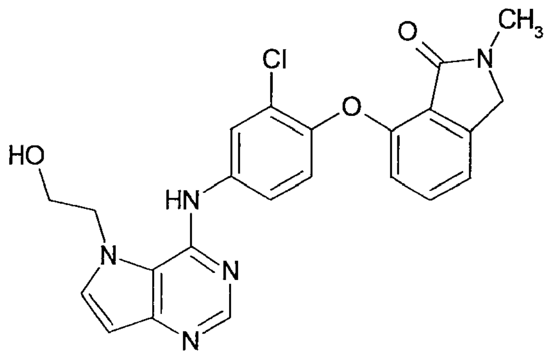

Production of 7-(2-chloro-4-{[5-(2-hydroxyethyl)-5H-pyrrolo[3,2-d]pyrimidin-4-yl]amino}phenoxy)-2-methylisoindolin-1-one

(i) Production of 4-bromo-7-methoxy-2-methylisoindolin-1-one

The title compound (1.54 g) was obtained as a pale-yellow amorphous by reaction in the same manner as in Example 68(i) and using 4-bromo-7-methoxyisoindolin-1-one (968 mg), 60% sodium hydride in oil (176 mg), methyl iodide (1.0 mL) and N,N-dimethylformamide (20 mL).
$^1$H-NMR ($CDCl_3$) δ: 3.17 (3H, s), 3.95 (3H, s), 4.25 (2H, s), 6.81 (1H, d, J = 8.7 Hz), 7.53 (1H, d, J = 8.7 Hz).

(ii) Production of 4-bromo-7-hydroxy-2-methylisoindolin-1-one

The title compound (0.53 g) was obtained as a pale-violet powder by reaction in the same manner as in Example 65(iii) and using 4-bromo-7-methoxy-2-methylisoindolin-1-one (1.28 g) and 48% hydrobromic acid (17 mL).
$^1$H-NMR ($CDCl_3$) δ: 3.17 (3H, s), 4.28 (2H, s), 6.78 (1H, d, J = 9.0 Hz), 7.45 (1H, d, J = 9.0 Hz), 8.45 (1H, br s).

(iii) Production of 7-hydroxy-2-methylisoindolin-1-one

The title compound (561 mg) was obtained as a yellow powder by reaction in the same manner as in Example 67(iii) and using 4-bromo-7-hydroxy-2-methylisoindolin-1-one (525 mg), 10% palladium/activated carbon (88 mg) and methanol (20 mL). $^1$H-NMR (95%$CDCl_3$+5%DMSO-$d_6$) δ: 3.15 (3H, s), 4.39 (2H, s), 6.82 (1H, d, J = 7.8 Hz), 6.93 (1H, d, J = 7.8 Hz), 7.38 (1H, t, J = 7.8 Hz).

(iv) Production of 7-(2-chloro-4-nitrophenoxy)-2-methylisoindolin-1-one

The title compound (539 mg) was obtained as a pale-violet powder by reaction in the same manner as in Example 65(iv) and using 7-hydroxy-2-methylisoindolin-1-one (354 mg), 2-chloro-1-fluoro-4-nitrobenzene (441 mg), potassium carbonate (480 mg) and N,N-dimethylformamide (8.0 mL).
$^1$H-NMR (DMSO-$d_6$) δ: 2.96 (3H, s), 4.50 (2H, s), 6.78 (1H, d, J = 9.3 Hz), 7.26 (1H, d, J = 7.8 Hz), 7.55 (1H, d, J = 7.8 Hz), 7.70 (1H, t, J = 7.8 Hz), 8.07 (1H, dd, J = 3.0 Hz, 9.3 Hz), 8.44 (1H, d, J = 3.0 Hz).

(v) Production of 7-(4-amino-2-chlorophenoxy)-2-methylisoindolin-1-one

The title compound (291 mg) was obtained as a pale-yellow powder by reaction in the same manner as in Example 66(iv) and using 7-(2-chloro-4-nitrophenoxy)-2-methylisoindolin-1-one (319 mg), 5% platinum/activated carbon (53 mg) and ethyl acetate (10 mL)/methanol (20 mL). $^1$H-NMR ($CDCl_3$) δ: 3.17 (3H, s), 3.68 (2H, br s), 4.35 (2H, s), 6.53 (1H, d, J = 7.8 Hz), 6.56 (1H, dd, J = 2.7 Hz, 8.7 Hz), 6.77 (1H, d, J = 2.7 Hz), 6.97 (1H, d, J = 8.7 Hz), 7.03 (1H, d, J = 7.8 Hz), 7.31 (1H, t, J = 7.8 Hz).

(vi) Production of 7-(2-chloro-4-{[5-(2-hydroxyethyl)-5H-pyrrolo[3,2-d]pyrimidin-4-yl]amino}phenoxy)-2-methylisoindolin-1-one

The title compound (134 mg) was obtained as pale-yellow crystals by reaction in the same manner as in Example 65(vi) and using 2-(4-chloro-5H-pyrrolo[3,2-d]pyrimidin-5-yl)ethyl benzoate (151 mg), 7-(4-amino-2-chlorophenoxy)-2-methylisoindolin-1-one (144 mg), pyridine hydrochloride (5 mg), isopropyl alcohol (10 mL), 1N aqueous sodium hydroxide solution (2.0 mL) and tetrahydrofuran (4.0 mL)/methanol (2.0 mL).
$^1$H-NMR (DMSO-$d_6$) δ: 3.03 (3H, s), 3.80-3.90 (2H, m), 4.46 (2H, s), 4.50-4.60 (2H, m), 6.29 (1H, br s), 6.50 (1H, d, J = 3.0 Hz), 6.62 (1H, d, J = 7.8 Hz), 7.11 (1H, d, J = 9.0 Hz), 7.26 (1H, d, J = 7.8 Hz), 7.48 (1H, t, J = 7.8 Hz), 7.56 (1H, dd, J = 2.7 Hz, 9.0 Hz), 7.65 (1H, d, J = 3.0 Hz), 7.95 (1H, d, J = 2.7 Hz), 8.32 (1H, s), 9.84 (1H, s).
Example 73
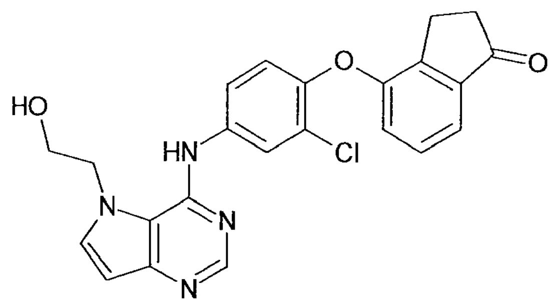

Production of 4-(2-chloro-4-{[5-(2-hydroxyethyl)-5H-pyrrolo[3,2-d]pyrimidin-4-yl]amino}phenoxy)indan-1-one

(i) Production of 4-(2-chloro-4-nitrophenoxy)indan-1-one

A mixture of 2-chloro-1-fluoro-4-nitrobenzene (1.18 g), 4-hydroxy-1-indanone (1.0 g) and potassium carbonate (0.94 g) in N,N-dimethylformamide (10 mL) was stirred at room temperature for 20 hr. The reaction system was acidified with water and 6N hydrochloric acid, and the mixture was extracted with ethyl acetate. The organic layer was washed with water and saturated brine, dried over anhydrous magnesium sulfate, and concentrated under reduced pressure, and the residue was separated and purified by silica gel column chromatography (eluent, hexane:ethyl acetate=4:1→1:1) to give the title compound (1.93 g) as pale-yellow crystals.

$^1$H-NMR ($CDCl_3$) δ: 2.71-2.75 (2H, m), 2.99-3.03 (2H, m), 6.89 (1H, d, J = 9.0 Hz), 7.23 (1H, dd, J = 0.9 Hz, 7.8 Hz), 7.46 (1H, t, J = 7.8 Hz), 7.68 -7.70 (1H, m), 8.08 (1H, dd, J = 2.7 Hz, 9.0 Hz), 8.42 (1H, d, J = 2.7 Hz).

(ii) Production of 4-(4-amino-2-chlorophenoxy)indan-1-one

A mixture of 4-(2-chloro-4-nitrophenoxy)indan-1-one (1.93 g), reduced iron (1.77 g) and calcium chloride(0.35 g) in 15% water-containing ethanol (60 mL) was heated under reflux for 6 hr. The insoluble substance was filtered off, and the filtrate was concentrated under reduced pressure. Water was added to the residue, and the mixture was extracted with ethyl acetate. The organic layer was washed with water and saturated brine, dried over anhydrous magnesium sulfate and concentrated under reduced pressure. The residue was separated and purified by silica gel column chromatography (eluent, hexane:ethyl acetate=3:1→1:3) to give the title compound (1.34 g) as a pale-yellow solid.
$^1$H-NMR ($CDCl_3$) δ: 2.71-2.75 (2H, m), 3.14-3.18 (2H, m), 3.71 (2H, br s), 6.59 (1H, dd, J = 2.7 Hz, 8.7 Hz), 6.79-6.82 (2H, m), 6.91 (1H, d, J = 8.7 Hz), 7.22-7.27 (1H, m), 7.42-7.45 (1H, m).

(iii) Production of 4-(2-chloro-4-{[5-(2-hydroxyethyl)-5H-pyrrolo[3,2-d]pyrimidin-4-yl]amino}phenoxy)indan-1-one

A solution of 2-(4-chloro-5H-pyrrolo[3,2-d]pyrimidin-5-yl)ethyl benzoate (200 mg) and 4-(4-amino-2-chlorophenoxy)indan-1-one (217 mg) in isopropyl alcohol (2.0 mL) was stirred at 80°C for 4 days. The reaction mixture was cooled to room temperature, and 1N aqueous sodium hydroxide solution (2.0 mL) was added. After stirring at room temperature for 1 hr, water was added, and the mixture was extracted with ethyl acetate. The organic layer was washed with water and saturated brine, dried over anhydrous magnesium sulfate, and concentrated under reduced pressure. The residue was separated and purified by silica gel column chromatography (eluent, ethyl acetate→ethyl acetate: methanol=85:15) to give the title compound (211 mg) as pale-yellow crystals.
$^1$H-NMR (DMSO-$d_6$) δ: 2.66-2.73 (2H, m), 3.03-3.11 (2H, m), 3.83-3.90 (2H, m), 4.46-4.55 (2H, m), 6.50 (1H, d, J = 3.0 Hz), 6.95 (1H, t, J = 4.2 Hz), 7.25 (1H, d, J = 9.0 Hz), 7.37-7.43 (2H, m), 7.57-7.63 (1H, m), 7.66 (1H, d, J = 3.0 Hz), 7.99 (1H, d, J = 2.1 Hz), 8.33 (1H, s).
Example 74
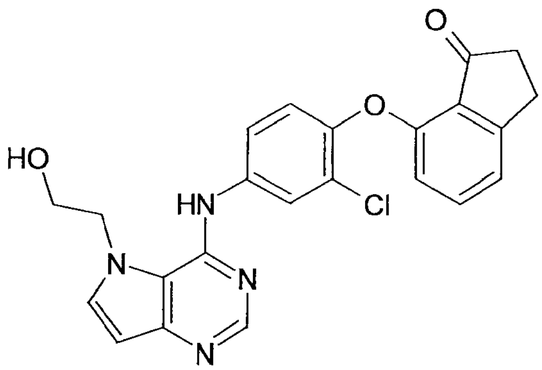

Production of 7-(2-chloro-4-{[5-(2-hydroxyethyl)-5H-pyrrolo[3,2-d]pyrimidin-4-yl]amino}phenoxy)indan-1-one

(i) Production of 7-(2-chloro-4-nitrophenoxy)indan-1-one

The title compound (1.09 g) was obtained as yellow crystals by reaction in the same manner as in Example 73(i) and using 2-chloro-1-fluoro-4-nitrobenzene (1.13 g), 7-hydroxy-1-indanone (0.95 g), potassium carbonate (0.97 g) and N,N-dimethylformamide (10 mL).
$^1$H-NMR ($CDCl_3$) δ: 2.65-2.70 (2H, m), 3.17-3.21 (2H, m), 6.77 (1H, d, J = 9.0 Hz), 6.92 (1H, dd, J = 0.9 Hz, 7.8 Hz), 7.37 (1H, dd, J = 0.9 Hz, 7.8 Hz), 7.62 (1H, t, J = 7.8 Hz), 8.01 (1H, dd, J = 2.7 Hz, 9.0 Hz), 8.40 (1H, d, J = 2.7 Hz).

(ii) Production of 7-(4-amino-2-chlorophenoxy)indan-1-one

The title compound (1.31 g) was obtained as an ocher solid by reaction in the same manner as in Example 73 (ii) and using 7-(2-chloro-4-nitrophenoxy)indan-1-one (1.90 g), reduced iron (1.80 g), calcium chloride (0.36 g) and 15%

water-containing ethanol (60 mL).
$^1$H-NMR ($CDCl_3$) δ: 2.72-2.76 (2H, m), 3.11-3.15 (2H, m), 3.70 (2H, br s), 6.38-6.41 (1H, m), 6.58 (1H, dd, J = 2.7 Hz, 8.4 Hz), 6.77 (1H, d, J = 2.7 Hz), 6.97 (1H, d, J = 8.9 Hz), 7.04-7.07 (1H, m), 7.34-7.40 (1H, m).

(iii) Production of 7-(2-chloro-4-{[5-(2-hydroxyethyl)-5H-pyrrolo[3,2-d]pyrimidin-4-yl]amino}phenoxy)indan-1-one

The title compound (66 mg) was obtained as pale-yellow crystals by reaction in the same manner as in Example 73 (iii) and using 2-(4-chloro-5H-pyrrolo[3,2-d]pyrimidin-5-yl)ethyl benzoate (100 mg), 7-(4-amino-2-chlorophenoxy)indan-1-one (109 mg), isopropyl alcohol (2.0 mL) and 1N aqueous sodium hydroxide solution (2.0 mL).
$^1$H-NMR ($CDCl_3$) δ: 2.64 (2H, t, J = 6.0 Hz), 3.11 (2H, t, J = 6.0 Hz), 3.87 (2H, t, J = 4.4 Hz), 4.53 (2H, t, J = 4.4 Hz), 6.16-6.43 (1H, m), 6.46-6.50 (2H, m), 7.18 (1H, d, J = 8.7 Hz), 7.22 (1H, d, J = 7.8 Hz), 7.51-7.65 (3H, m), 7.97 (1H, d, J = 2.1 Hz), 8.33 (1H, s), 9.71-10.05 (1H, m).
Example 75
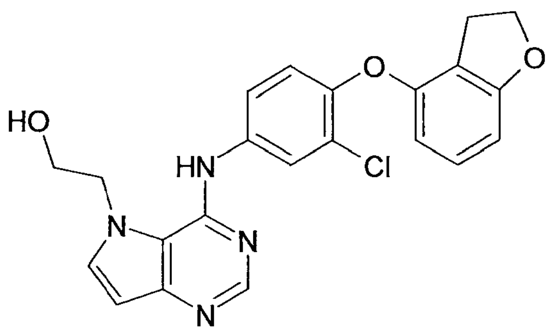

Production of 2-(4-{[3-chloro-4-(2,3-dihydro-1-benzofuran-4-yloxy)phenyl]amino}-5H-pyrrolo[3,2-d]pyrimidin-5-yl)ethanol

(i) Production of 4-(2-chloro-4-nitrophenoxy)-2,3-dihydro-1-benzofuran

The title compound (1.07 g) was obtained as a white solid by reaction in the same manner as in Example 73 (i) and using 2-chloro-1-fluoro-4-nitrobenzene (1.05 g), 4-hydroxy-2,3-dihydro-1-benzofuran (977 mg), potassium carbonate (0.98 g) and N,N-dimethylformamide (10 mL).
$^1$H-NMR ($CDCl_3$) δ: 3.06 (2H, t, J = 8.7 Hz), 4.60 (2H, t, J = 8.7 Hz), 6.53 (1H, d, J = 8.1 Hz), 6.71 (1H, d, J = 8.1 Hz), 6.88 (1H, d, J = 9.0 Hz), 7.16 (1H, d, J = 8.1 Hz), 8.05 (1H, dd, J = 2.7 Hz, 9.0 Hz), 8.37 (1H, d, J = 2.7 Hz).

(ii) Production of 3-chloro-4-(2,3-dihydro-1-benzofuran-4-yloxy)aniline

The title compound (920.2 g) was obtained as a pale-yellow solid by reaction in the same manner as in Example 73 (ii) and using 4-(2-chloro-4-nitrophenoxy)-2,3-dihydro-1-benzofuran (1.20 g), reduced iron (1.15 g), calcium chloride (0.23 g) and 15% water-containing ethanol (40 mL).
$^1$H-NMR ($CDCl_3$) δ: 3.16 (2H, t, J = 8.7 Hz), 3.65 (2H, br s), 4.59 (2H, t, J = 8.7 Hz), 6.19 (1H, dd, J = 0.6 Hz, 8.4 Hz), 6.50 (1H, d, J = 8.1 Hz), 6.39 (1H, dd, J = 2.7 Hz, 8.9 Hz), 6.76 (1H, d, J = 2.7 Hz), 6.87 (1H, d, J = 8.9 Hz), 6.95-7.01 (1H, m).

(iii) Production of 2-(4-{[3-chloro-4-(2,3-dihydro-1-benzofuran-4-yloxy)phenyl]amino}-5H-pyrrolo[3,2-d]pyrimidin-5-yl) ethanol

The title compound (120 g) was obtained as pale-yellow crystals by reaction in the same manner as in Example 73 (iii) and using 2-(4-chloro-5H-pyrrolo[3,2-d]pyrimidin-5-yl)ethyl benzoate (100 mg), 3-chloro-4-(2,3-dihydro-1-benzofuran-4-yloxy)aniline (104 mg), isopropyl alcohol (3.0 mL) and 1N aqueous sodium hydroxide solution (2.0 mL).
$^1$H-NMR ($DMSO-d_6$) δ: 3.10 (2H, t, J = 9.0 Hz), 3.83-3.90 (2H, m), 4.47-4.60 (4H, m), 6.20 (1H, d, J = 8.1 Hz), 6.50 (1H, d, J = 3.0 Hz), 6.53 (1H, d, J = 7.5 Hz), 7.05 (1H, t, J = 8.1 Hz), 7.16 (1H, d, J = 8.7 Hz), 7.53-7.57 (1H, m), 7.64 (1H, d, J = 3.0 Hz), 7.92-7.98 (1H, m), 8.32 (1H, s), 9.65-10.08 (1H, m).
Example 76
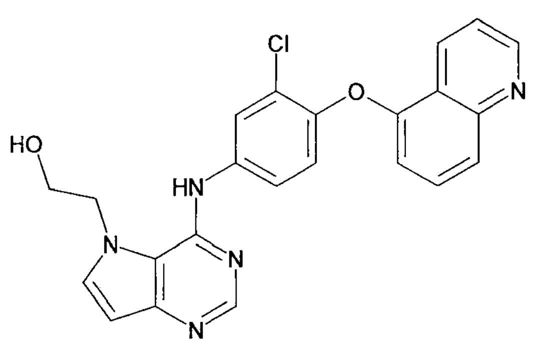

Production of 2-(4-{[3-chloro-4-(quinolin-5-yloxy)phenyl]amino}-5H-pyrrolo[3,2-d]pyrimidin-5-yl)ethanol

(i) Production of 5-(2-chloro-4-nitrophenoxy)quinoline

2-Chloro-1-fluoro-4-nitrobenzene (3 g) and quinolin-5-ol (2.48 g) were dissolved in N,N-dimethylformamide (17 mL). Potassium carbonate (3.54 g) was added, and the mixture was stirred at room temperature for 16 hr. The mixture was partitioned between ethyl acetate (200 mL) and water (100 mL). The organic layer was washed with saturated brine (80 mL), dried over anhydrous magnesium sulfate, and concentrated under reduced pressure. The residual solid was triturated with diisopropyl ether, collected by filtration and dried under reduced pressure to give the title compound (4.75 g) as a pale-yellow powder.
$^1$H-NMR (DMSO-$d_6$) δ: 7.06 (1H, d, J= 9.0 Hz), 7.34 (1H, d, J= 8.0 Hz), 7.61 (1H, dd, J= 4.0 Hz, 9.0 Hz), 7.82 (1H, t, J= 9.0 Hz), 7.99 (1H, dd, J= 1.0 Hz, 9.0 Hz), 8.13 (1H, dd, J= 3.0 Hz, 9.0 Hz), 8.34 (1H, m), 8.54 (1H, d, J= 3.0 Hz), 9.01 (1H, m).

(ii) Production of 3-chloro-4-(quinolin-5-yloxy)aniline

5-(2-Chloro-4-nitrophenoxy)quinoline (4.5 g) was suspended in ethanol (150 mL)/water (17 mL), calcium chloride (1.1 g) was added, and the mixture was stirred with heating at 90°C for 5 min. Reduced iron (5.57 g) was added, and the mixture was stirred with heating at 90°C for 16 hr. The reaction mixture was cooled to room temperature, and filtered through celite, and the filtrate was concentrated under reduced pressure. The residue was diluted with ethyl acetate/tetrahydrofuran (1:1, 200 mL), and the mixture was washed with water (100 mL). The organic layer was dried over anhydrous magnesium sulfate and concentrated under reduced pressure. The residual solid was triturated with diisopropyl ether, collected by filtration and dried under reduced pressure to give the title compound (2.88 g) as a pale-brown powder.
$^1$H-NMR (DMSO-$d_6$) δ: 3.73 (2H, br s), 6.50-6.70 (2H, m), 6.83 (1H, d, J= 3.0 Hz), 6.97 (1H, d, J= 9.0 Hz), 7.40-7.60 (2H, m), 7.77 (1H, d, J= 9.0 Hz), 8.73 (1H, m), 8.94 (1H, dd, J= 2.0 Hz, 4.5 Hz).

(iii) Production of 2-(4-{[3-chloro-4-(quinolin-5-yloxy)phenyl]amino}-5H-pyrrolo[3,2-d]pyrimidin-5-yl)ethanol

3-Chloro-4-(quinolin-5-yloxy)aniline (136 mg) and 2-(4-chloro-5H-pyrrolo[3,2-d]pyrimidin-5-yl)ethyl benzoate (151 mg) were dissolved in isopropyl alcohol (1.5 mL), pyridine hydrochloride (5.8 mg) was added, and the mixture was stirred at 80°C for 16 hr. The reaction mixture was cooled to room temperature, and the reaction mixture was partitioned between ethyl acetate (80 mL) and saturated aqueous sodium hydrogen carbonate solution (50 mL). The organic layer was dried over anhydrous magnesium sulfate and concentrated under reduced pressure. The residue was separated and purified by silica gel column chromatography (hexane:ethyl acetate=70:30→0:100), and the object fraction was concentrated under reduced pressure. The obtained oil residue was dissolved in methanol (2.18 mL), 1N aqueous sodium hydroxide solution (0.5 mL) was added, and the mixture was stirred at room temperature for 2 hr. 1N Hydrochloric acid (0.5 mL) was added, and the mixture was diluted with ethyl acetate (40 mL)/tetrahydrofuran (40 mL) and partitioned with saturated brine (50 mL). The organic layer was dried over anhydrous magnesium sulfate and concentrated under reduced pressure. The residue was separated and purified by silica gel column chromatography (ethyl acetate:methanol=100:0→85:15) to give the title compound (112 mg) as white crystals.
$^1$H-NMR (DMSO-$d_6$)δ: 3.89 (2H, m), 4.54 (2H, m), 6.33 (1H, m), 6.52 (1H, d, J= 3.0 Hz), 6.52 (1H, d, J= 3.0 Hz), 6.75 (1H, d, J= 7.5 Hz), 7.33 (1H, d, J= 9.0 Hz), 7.60-7.70 (4H, m), 7.76 (1H, d, J= 9.0 Hz), 8.03 (1H, d, J= 3.0 Hz), 8.35 (1H, s), 8.68 (1H, d, J= 8.0 Hz), 8.98 (1H, d, J= 4.0 Hz), 9.91 (1H, br s).
Example 77

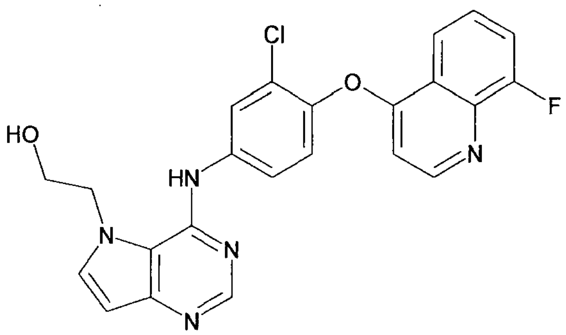

Production of 2-[4-({3-chloro-4-[(8-fluoroquinolin-4-yl)oxy]phenyl}amino)-5H-pyrrolo[3,2-d]pyrimidin-5-yl]ethanol

(i) Production of 4-(2-chloro-4-nitrophenoxy)-8-fluoroquinoline

2-Chloro-1-fluoro-4-nitrobenzene (2.15 g) and 8-fluoroquinolin-4-ol (2.0 g) were dissolved in N,N-dimethylformamide (12 mL). Potassium carbonate (2.54 g) was added, and the mixture was stirred at room temperature for 16 hr. The mixture was partitioned between ethyl acetate (100 mL) and water (80 mL), and the organic layer was dried over anhydrous magnesium sulfate and concentrated under reduced pressure. The residue was separated and purified by silica gel column chromatography (hexane:ethyl acetate=90:10→20:80) to give the title compound (3.27 g) as a white powder.
$^1$H-NMR (DMSO-$d_6$) δ: 6.96 (1H, d, J= 5.0 Hz), 7.60-7.80 (3H, m), 8.00-8.10 (1H, m), 8.30-8.40 (1H, m), 8.61 (1H, d, J= 3.0 Hz), 8.83 (1H, d, J= 5.0 Hz).

(ii) Production of 3-chloro-4-[(8-fluoroquinolin-4-yl)oxy]aniline

4-(2-Chloro-4-nitrophenoxy)-8-fluoroquinoline (3.0 g) was suspended in ethanol (94 mL)/water (10 mL), calcium chloride (690 mg) was added, and the mixture was stirred with heating at 90°C for 5 min. Reduced iron (3.49 g) was added, and the mixture was stirred with heating at 90°C for 16 hr. The reaction mixture was cooled to room temperature, and filtered through celite, and the filtrate was concentrated under reduced pressure. The residue was diluted with ethyl acetate (100 mL), and the mixture was washed with water (80 mL). The organic layer was dried over anhydrous magnesium sulfate and concentrated under reduced pressure. The residue was separated and purified by silica gel column chromatography (hexane:ethyl acetate=90:10→20:80) to give the title compound (2.30 g) as a pale-orange powder.
$^1$H-NMR ($CDCl_3$) δ: 3.85 (2H, br s), 6.47 (1H, d, J= 5.0 Hz), 6.64 (1H, dd, J= 3.0 Hz, 9.0 Hz), 6.83 (1H, d, J= 3.0 Hz), 7.03 (1H, d, J= 9.0 Hz), 7.40-7.60 (2H, m), 8.10-8.20 (1H, m), 8.69 (1H, d, J= 5.0 Hz).

(iii) Production of 2-[4-({3-chloro-4-[(8-fluoroquinolin-4-yl)oxy]phenyl}amino)-5H-pyrrolo[3,2-d]pyrimidin-5-yl]ethanol

3-Chloro-4-[(8-fluoroquinolin-4-yl)oxy]aniline (198 mg) and 2-(4-chloro-5H-pyrrolo[3,2-d]pyrimidin-5-yl)ethyl benzoate (207 mg) were dissolved in isopropyl alcohol (10 mL), pyridine hydrochloride (7.9 mg) was added, and the mixture was stirred at 80°C for 16 hr. The reaction mixture was cooled to room temperature, and partitioned between ethyl acetate (80 mL)/tetrahydrofuran (30 mL)/saturated aqueous sodium hydrogen carbonate solution (50 mL). The organic layer was dried over anhydrous magnesium sulfate and concentrated under reduced pressure. The residue was separated and purified by silica gel column chromatography (hexane:ethyl acetate=70:30→0:100), and the object fraction was concentrated under reduced pressure. The obtained oil residue was dissolved in methanol (8.99 mL), 1N aqueous sodium hydroxide solution (0.686 mL) was added, and the mixture was stirred at room temperature for 2 hr. 1N Hydrochloric acid (0.686 mL) was added, and the mixture was diluted with tetrahydrofuran (80 mL) and partitioned with saturated brine (30 mL). The organic layer was dried over anhydrous magnesium sulfate and concentrated under reduced pressure. The residue was separated and purified by silica gel column chromatography (ethyl acetate:methanol=100:0→85:15) to give the title compound (98 mg) as white crystals.
$^1$H-NMR (DMSO-$d_6$)δ: 3.90 (2H, q, J= 5.0 Hz), 4.56 (2H, t, J= 5.0 Hz), 6.36 (1H, m), 6.53 (1H, d, J= 3.0 Hz), 6.66 (1H, d, J= 5.0 Hz), 7.52 (1H, d, J= 9.0 Hz), 7.60-7.80 (4H, m), 8.08 (1H, d, J= 3.0 Hz), 8.10-8.30 (1H, m), 8.37 (1H, s), 8.75

(1H, d, J= 5.0 Hz), 10.01 (1H, br s).

Example 78

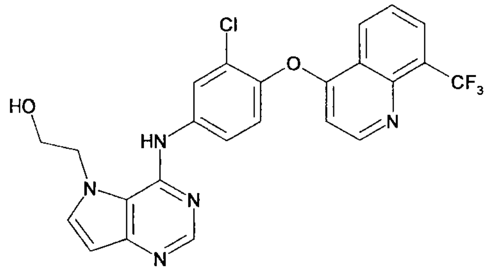

Production of 2-{4-[(3-chloro-4-{[8-(trifluoromethyl)quinolin-4-yl]oxy}phenyl)amino]-5H-pyrrolo[3,2-d]pyrimidin-5-yl}ethanol

(i) Production of 4-(2-chloro-4-nitrophenoxy)-8-(trifluoromethyl)quinoline

2-Chloro-1-fluoro-4-nitrobenzene (2.47 g) and 8-(trifluoromethyl)quinolin-4-ol (3.0 g) were dissolved in N,N-dimethylformamide (14 mL). Potassium carbonate (2.92 g) was added, and the mixture was stirred at room temperature for 16 hr. The mixture was partitioned between ethyl acetate (180 mL) and water (80 mL), and the organic layer was dried over anhydrous magnesium sulfate and concentrated under reduced pressure. The residue was separated and purified by silica gel column chromatography (hexane:ethyl acetate=90:10→50:50) to give the title compound (4.20 g) as a white powder.
$^1$H-NMR ($CDCl_3$) δ: 6.65 (1H, d, J= 5.0 Hz), 7.35 (1H, d, J= 9.0 Hz), 7.69 (1H, t, J= 8.0 Hz), 8.18 (1H, d, J= 7.0 Hz), 8.25 (1H, dd, J= 3.0 Hz, 9.0 Hz), 8.49 (1H, d, J= 2.5 Hz), 8.53 (1H, d, J= 8.0 Hz), 8.92 (1H, d, J= 5.0 Hz).

(ii) Production of 3-chloro-4-{[8-(trifluoromethyl)quinolin-4-yl]oxy}aniline

4-(2-Chloro-4-nitrophenoxy)-8-(trifluoromethyl)quinoline was suspended in ethanol (114 mL)/water (13 mL), calcium chloride (840 mg) was added, and the mixture was stirred with heating at 90°C for 5 min. Reduced iron (4.23 g) was added, and the mixture was stirred with heating at 90°C for 16 hr. The mixture was cooled to room temperature and filtered through celite, and the filtrate was concentrated under reduced pressure. The residue was diluted with ethyl acetate (100 mL), and the mixture was washed with water (80 mL). The organic layer was dried over anhydrous magnesium sulfate and concentrated under reduced pressure. The residue was separated and purified by silica gel column chromatography (hexane:ethyl acetate=90:10→20:80) to give the title compound (3.34 g) as a dark-red oil.
$^1$H-NMR ($CDCl_3$) δ: 3.87 (2H, br s), 6.52 (1H, d, J= 5.0 Hz), 6.64 (1H, dd, J= 3.0 Hz, 9.0 Hz), 6.82 (1H, d, J= 3.0 Hz), 7.02 (1H, d, J= 8.0 Hz), 7.62 (1H, t, J= 8.0 Hz), 8.11 (1H, d, J= 7.5 Hz), 8.64 (1H, d, J= 8.0 Hz), 8.80 (1H, d, J= 5.0 Hz).

(iii) Production of 2-{4-[(3-chloro-4-{[8-(trifluoromethyl)quinolin-4-yl]oxy}phenyl)amino]-5H-pyrrolo[3,2-d]pyrimidin-5-yl}ethanol

3-Chloro-4-{[8-(trifluoromethyl)quinolin-4-yl]oxy}aniline (250 mg) and 2-(4-chloro-5H-pyrrolo[3,2-d]pyrimidin-5-yl)ethyl benzoate (223 mg) were dissolved in isopropyl alcohol (10 mL), pyridine hydrochloride (8.5 mg) was added, and the mixture was stirred at 80°C for 16 hr. The reaction mixture was cooled to room temperature, and partitioned between ethyl acetate (40 mL)/tetrahydrofuran (40 mL)/saturated aqueous sodium hydrogen carbonate solution (50 mL). The organic layer was dried over anhydrous magnesium sulfate and concentrated under reduced pressure. The residue was separated and purified by silica gel column chromatography (hexane:ethyl acetate=70:30→0:100), and the object fraction was concentrated under reduced pressure. The obtained oil residue was dissolved in methanol (3.2 mL), 1N aqueous sodium hydroxide solution (0.738 mL) was added, and the mixture was stirred at room temperature for 2 hr. 1N Hydrochloric acid (0.738 mL) was added, and the mixture was diluted with tetrahydrofuran (80 mL) and partitioned with saturated brine (50 mL). The organic layer was dried over anhydrous magnesium sulfate and concentrated under reduced pressure. The residue was separated and purified by silica gel column chromatography (ethyl acetate:methanol=100:0→85:15) to give the title compound (261 mg) as white crystals.
$^1$H-NMR (DMSO-$d_6$)δ: 3.90 (2H, t, J= 4.5 Hz), 4.56 (2H, t, J= 4.5 Hz), 6.36 (1H, m), 6.53 (1H, d, J= 3.0 Hz), 6.71 (1H, d, J= 4.5 Hz), 7.53 (1H, d, J= 7.5 Hz), 7.60-7.80 (2H, m), 7.83 (1H, d, J= 7.5 Hz), 8.09 (1H, d, J= 2.0 Hz), 8.28 (1H, d,

J= 7.5 Hz), 8.37 (1H, s), 8.70 (1H, d, J= 9.0 Hz), 8.84 (1H, d, J= 4.5 Hz), 10.01 (1H, br s).

Example 79

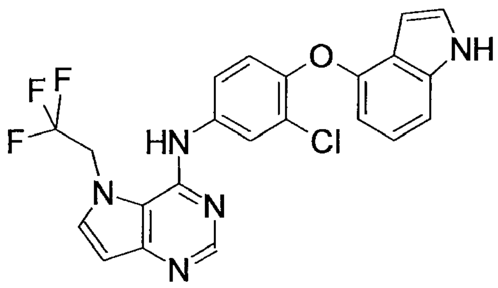

Production of N-[3-chloro-4-(1H-indol-4-yloxy)phenyl]-5-(2,2,2-trifluoroethyl)-5H-pyrrolo[3,2-d]pyrimidin-4-amine

A mixture of 4-chloro-5-(2,2,2-trifluoroethyl)-5H-pyrrolo[3,2-d]pyrimidine (70 mg) and 3-chloro-4-(1H-indol-4-yloxy)aniline (80 mg) was dissolved in isopropyl alcohol (5.0 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane: ethyl acetate=50:50→0:100) to give the title compound (110 mg) as a yellow powder.

$^1$H-NMR (DMSO-$d_6$) δ: 5.50-5. 65 (2H, m), 6.31 (1H, t, J = 2.0 Hz), 6.41 (1H, d, J = 7.0 Hz), 6.48 (1H, d, J = 3.2 Hz), 6.90-7.09 (2H, m), 7.18 (1H, d, J = 8.1 Hz), 7.20-7.38 (1H, m), 7.50 (1H, dd, J = 2.0 Hz, 8.1 Hz), 7.67 (1H, d, J = 3.2 Hz), 7.89 (1H, d, J = 2.5 Hz), 8.30 (1H, s), 8.49 (1H, s), 11.27 (1H, br s).

Example 80

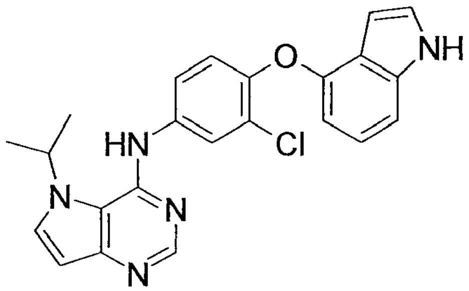

Production of N-[3-chloro-4-(1H-indol-4-yloxy)phenyl]-5-isopropyl-5H-pyrrolo[3,2-d]pyrimidin-4-amine

A mixture of 4-chloro-5-isopropyl-5H-pyrrolo[3,2-d]pyrimidine (150 mg) and 3-chloro-4-(1H-indol-4-yloxy)aniline (240 mg) was dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane: ethyl acetate=50:50→0:100) to give the title compound (220 mg) as a white powder.

$^1$H-NMR (DMSO-$d_6$) δ: 1.45 (6H, d, J = 6.6 Hz), 5.18 (1H, br s), 6.32 (1H, d, J = 2.0 Hz), 6.41 (1H, d, J = 7.2 Hz), 6.54 (1H, d, J = 3.2 Hz), 6.90-7.09 (2H, m), 7.19 (1H, d, J = 8.1 Hz), 7.26-7.35 (1H, m), 7.46 (1H, dd, J = 2.0, 8.1 Hz), 7.74-7.93 (2H, m), 8.31 (1H, s), 8.61 (1H, s), 11.28 (1H, br s).

Example 81

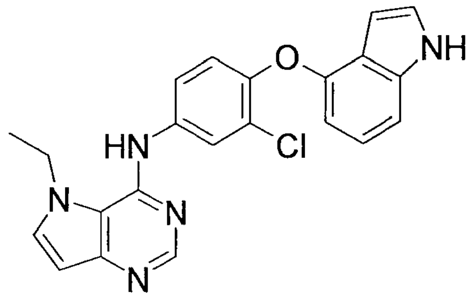

Production of N-[3-chloro-4-(1H-indol-4-yloxy)phenyl]-5-ethyl-5H-pyrrolo[3,2-d]pyrimidin-4-amine

A mixture of 4-chloro-5-ethyl-5H-pyrrolo[3,2-d]pyrimidine (108 mg) and 3-chloro-4-(1H-indol-4-yloxy)aniline (154 mg) was dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was diluted with saturated brine, dried over anhydrous magnesium sulfate, and concentrated under reduced pressure. The residue was separated and purified by silica gel column chromatography (hexane:ethyl acetate=50:50→0:100) to give the title compound (162 mg) as a white powder.
$^1$H-NMR (DMSO-$d_6$) δ: 1.31 (3H, t, J = 7.2 Hz), 4.54 (2H, q, J = 7.2 Hz), 6.32 (1H, t, J = 2.1 Hz), 6.41 (1H, d, J = 7.0 Hz), 6.48 (1H, d, J = 3.2 Hz), 6.90-7.08 (2H, m), 7.19 (1H, d, J = 8.1 Hz), 7.26-7.36 (1H, m), 7.52 (1H, dd, J = 2.1 Hz, 8.1 Hz), 7.68 (1H, d, J = 3.2 Hz), 7.88 (1H, d, J = 2.5 Hz), 8.31 (1H, s), 8.52 (1H, s), 11.29 (1H, br s).
Example 82
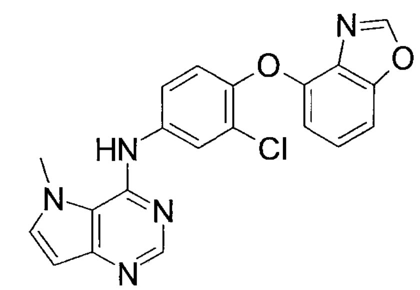

Production of N-[4-(1,3-benzoxazol-4-yloxy)-3-chlorophenyl]-5-methyl-5H-pyrrolo[3,2-d]pyrimidin-4-amine

(i) Production of 4-(2-chloro-4-nitrophenoxy)-1,3-benzoxazole

1,3-Benzoxazol-4-ol (1.0 g) was dissolved in N,N-dimethylformamide (20 mL), sodium hydride (296 mg) was added, and the mixture was stirred at room temperature for 1 hr. 2-Chloro-1-fluoro-4-nitrobenzene (1.3 g) was added, and the mixture was stirred for 1 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=0:100→100:0) to give the title compound (620 mg) as a white powder.
$^1$H-NMR (DMSO-$d_6$) δ: 6.12 (1H, d, J = 7.7 Hz), 6.49-6.51 (2H, m), 6.59-6.76 (2H, m), 6.90 (1H, d, J = 8.8 Hz), 7.02-7.25 (1H, m).

(ii) Production of N-[4-(1,3-benzoxazol-4-yloxy)-3-chlorophenyl]-5-methyl-5H-pyrrolo[3,2-d]pyrimidin-4-amine

4-(2-Chloro-4-nitrophenoxy)-1,3-benzoxazole (502 mg) was dissolved in a mixed solvent of methanol (10 mL) /tetrahydrofuran (10 mL), 5% platinum/activated carbon (250 mg) was added, and the mixture was stirred under hydrogen atmosphere for 1 hr. The reaction mixture was filtered through celite, and the filtrate was concentrated under reduced

pressure. A mixture of the residue and 4-chloro-5-methyl-5H-pyrrolo[3,2-d]pyrimidine (257 mg) was dissolved in isopropyl alcohol (15 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (162 mg) as a pale-purple powder.
$^1$H-NMR (DMSO-$d_6$) δ: 4.16 (3H, s), 6.46 (1H, d, J = 3.0 Hz), 6.54 (2H, s), 6.73-6.79 (1H, m), 7.19 (1H, d, J = 8.8 Hz), 7.39 (1H, t, J = 8.2 Hz), 7.49-7.57 (1H, m), 7.57-7.68 (2H, m), 8.32 (1H, s), 8.74 (1H, s).

Example 83

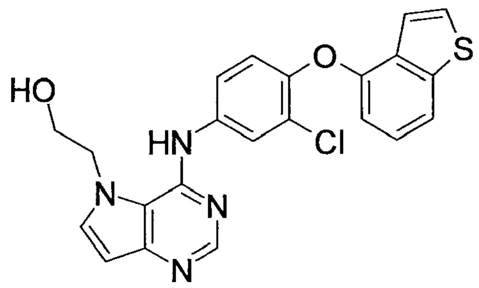

Production of 2-(4-{[4-(1-benzothiophen-4-yloxy)-3-chlorophenyl]amino}-5H-pyrrolo[3,2-d]pyrimidin-5-yl)ethanol

(i) Production of 4-(2-chloro-4-nitrophenoxy)-1-benzothiophene

To a solution of 2-chloro-1-fluoro-4-nitrobenzene (5.8 g) and 1-benzothiophen-4-ol (5.0 g) in N,N-dimethylformamide (50 mL) was added potassium carbonate (5.1 g), and the mixture was stirred at room temperature for 48 hr. Water was added to the reaction mixture, and the mixture was extracted with ethyl acetate. The organic layer was washed with saturated brine, and dried over anhydrous magnesium sulfate. Anhydrous magnesium sulfate was filtered off and the filtrate was concentrated under reduced pressure. The obtained residue was separated and purified by silica gel chromatography (hexane:ethyl acetate=19:1→4:1) to give the title compound (7.8 g) as a white powder.
$^1$H-NMR (DMSO-$d_6$) δ: 6.94 (1H, d, J = 9.1 Hz), 7.18 (1H, d, J = 8.1 Hz), 7.25 (1H, dd, J = 0.8 Hz, 5.7 Hz), 7.48 (1H, t, J = 8.1 Hz), 7.85 (1H, d, J = 5.7 Hz), 8.01 (1H, d, J = 8.1 Hz), 8.14 (1H, dd, J = 2.7 Hz, 9.1 Hz), 8.52 (1H, d, J = 2.7 Hz).

(ii) Production of 4-(1-benzothiophen-4-yloxy)-3-chloroaniline

4-(2-Chloro-4-nitrophenoxy)-1-benzothiophene (5.2 g) was dissolved in ethanol (80 mL)/1-methyl-2-pyrrolidone (15 mL), reduced iron (5.0 g) and 1N hydrochloric acid (12 mL) were added and the mixture was heated under reflux at 110°C for 1 hr. The reaction mixture was cooled to room temperature, 1N aqueous sodium hydroxide solution (18 mL) was added, and the reaction mixture was filtered through celite. The obtained filtrate was diluted with ethyl acetate, washed with water and saturated brine, and dried over anhydrous magnesium sulfate. Anhydrous magnesium sulfate was filtered off and the filtrate was concentrated under reduced pressure. The obtained residue was separated and purified by silica gel chromatography (hexane:ethyl acetate=49:1→1:1) to give the title compound (4.1 g) as a pale-purple powder.
$^1$H-NMR (DMSO-$d_6$) δ: 5.37 (2H, s), 6.44 (1H, d, J = 8.0 Hz), 6.60 (1H, dd, J = 2.6 Hz, 8.7 Hz), 6.78 (1H, d, J = 2.6 Hz), 6.98 (1H, d, J = 8.7 Hz), 7.23 (1H, t, J = 8.0 Hz), 7.56 (1H, dd, J = 0.8 Hz, 5.5 Hz), 7. 65 (1H, d, J = 8.0 Hz), 7. 74 (1H, d, J = 5.5 Hz).

(iii) Production of 2-(4-{[4-(1-benzothiophen-4-yloxy)-3-chlorophenyl]amino}-5H-pyrrolo[3,2-d]pyrimidin-5-yl)ethanol

The title compound (90 mg) was obtained as white crystals by reaction in the same manner as in Example 2 (iv) and using 2-(4-chloro-5H-pyrrolo[3,2-d]pyrimidin-5-yl)ethyl benzoate (100 mg), 4-(1-benzothiophen-4-yloxy)-3-chloroaniline (115 mg), pyridine hydrochloride (5 mg), isopropyl alcohol (5 mL) and 1N aqueous sodium hydroxide solution (1.5 mL).
$^1$H-NMR (DMSO-$d_6$) δ: 3.88 (2H, br s), 4.54 (2H, br s), 6.51 (1H, d, J = 3.0 Hz), 6.62 (1H, d, J = 7.1 Hz), 7.22 (1H, d, J

= 8.8 Hz), 7.31 (1H, t, J = 7.9 Hz), 7.48-7.84 (5H, m), 8.00 (1H, d, J = 2.4 Hz), 8.34 (1H, s), 9.85 (1H, br s).

Example 84

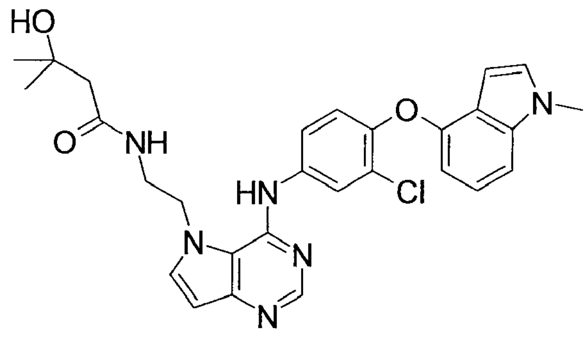

Production of N-{2-[4-({3-chloro-4-[(1-methyl-1H-indol-4-yl)oxy]phenyl}amino)-5H-pyrrolo[3,2-d]pyrimidin-5-yl]ethyl}-3-hydroxy-3-methylbutanamide

The title compound (85 mg) was obtained as white crystals by reaction in the same manner as in Example 55 and using tert-butyl [2-(4-chloro-5H-pyrrolo[3,2-d]pyrimidin-5-yl)ethyl]carbamate (100 mg), 3-chloro-4-[(1-methyl-1H-indol-4-yl)oxy]aniline (90 mg), isopropyl alcohol (5 mL), 4N hydrochloric acid (5 mL), ethanol (30 mL), 3-hydroxy-3-methylbutanoic acid (60 mg), 1-ethyl-3-(3-dimethylaminopropyl)carbodiimide hydrochloride (170 mg), 1-hydroxybenzotriazole (50 mg), triethylamine (0.75 mL) and N,N-dimethylformamide (8.0 mL).
$^1$H-NMR (DMSO-$d_6$) δ: 1.13 (6H, s), 2.20 (2H, s), 3.38-3.48 (2H, m), 3.81 (3H, s), 4.44-4.59 (2H, m), 4.67 (1H, s), 6.33 (1H, d, J = 3.0 Hz), 6.38-6.53 (2H, m), 7.01 (1H, d, J = 8.8 Hz), 7.09 (1H, t, J = 8.0 Hz), 7.23 (1H, d, J = 8.0 Hz), 7.30 (1H, d, J = 3.0 Hz), 7.56-7.77 (2H, m), 8.00 (1H, d, J = 2.0 Hz), 8.14-8.40 (2H, m), 8.82 (1H, s).

Example 85

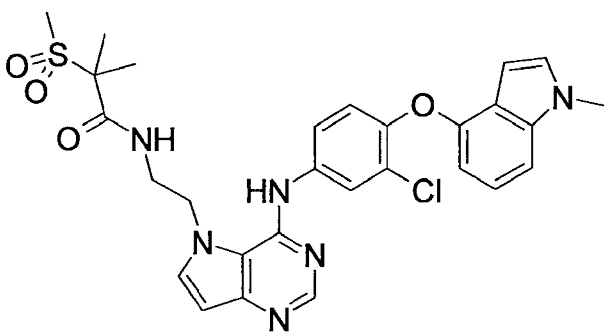

Production of N-{2-[4-({3-chloro-4-[(1-methyl-1H-indol-4-yl)oxy]phenyl}amino)-5H-pyrrolo[3,2-d]pyrimidin-5-yl]ethyl}-2-methyl-2-(methylsulfonyl)propanamide

The title compound (93 mg) was obtained as white crystals by reaction in the same manner as in Example 18 and using tert-butyl [2-(4-chloro-5H-pyrrolo[3,2-d]pyrimidin-5-yl)ethyl]carbamate (100 mg), 3-chloro-4-[(1-methyl-1H-indol-4-yl)oxy]aniline (90 mg), isopropyl alcohol (5 mL), 4N hydrochloric acid (5 mL), ethanol (30 mL), 2-methyl-2-(methylsulfonyl)propanoic acid (80 mg), 1-ethyl-3-(3-dimethylaminopropyl)carbodiimide hydrochloride (200 mg), 1-hydroxybenzotriazole (50 mg), triethylamine (0.80 mL) and N,N-dimethylformamide (9.0 mL).
$^1$H-NMR (DMSO-$d_6$) δ: 1.41 (6H, s), 2.96 (3H, s), 3.41-3.54 (2H, m), 3.81 (3H, s), 4.47-4.64 (2H, m), 6.32 (1H, d, J = 2.4 Hz), 6.37-6.51 (2H, m), 6.98-7.16 (2H, m), 7.23 (1H, d, J = 8.7 Hz), 7.31 (1H, d, J = 2.8 Hz), 7.55 (1H, d, J = 2.8 Hz), 7.63 (1H, dd, J = 2.4 Hz, 8.7 Hz), 7.96 (1H, d, J = 2.4 Hz), 8.13-8.28 (1H, m), 8.31 (1H, s), 8.61 (1H, s).

Example 86

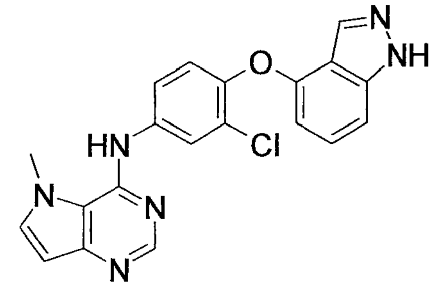

Production of N-[3-chloro-4-(1H-indazol-4-yloxy)phenyl]-5-methyl-5H-pyrrolo[3,2-d]pyrimidin-4-amine

(i) Production of 3-chloro-4-(1H-indazol-4-yloxy)aniline

The reaction in the same manner as in Example 6 (i) was performed using 1H-indazol-4-ol (1.0 g), 2-chloro-1-fluoro-4-nitrobenzene (1.3 g), potassium carbonate (2.0 g) and N,N-dimethylformamide (20 mL). The reaction in the same manner as in Example 6 (ii) was performed using the obtained compound, tetrahydrofuran (15 mL) and 5% platinum/activated carbon (150 mg) to give the title compound (190 mg) as a yellow powder.
$^1$H-NMR (DMSO-$d_6$) δ: 5.39 (2H, s), 6.19 (1H, dd, J = 2.0, 6.3 Hz), 6.60 (1H, dd, J = 2.6 Hz, 8.7 Hz), 6.77 (1H, d, J = 2.6 Hz), 7.01 (1H, d, J = 8.7 Hz), 7.13-7.28 (2H, m), 7.86 (1H, s), 13.16 (1H, br s).

(ii) Production of N-[3-chloro-4-(1H-indazol-4-yloxy)phenyl]-5-methyl-5H-pyrrolo[3,2-d]pyrimidin-4-amine

A mixture of 4-chloro-5-methyl-5H-pyrrolo[3,2-d]pyrimidine (100 mg) and 3-chloro-4-(1H-indazol-4-yloxy)aniline (156 mg) was dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane: ethyl acetate=50:50→0:100) to give the title compound (135 mg) as a white powder.
$^1$H-NMR (DMSO-$d_6$) δ: 4.16 (3H, s), 6.29-6.38 (1H, m), 6.46 (1H, d, J = 3.0 Hz), 7.16-7.38 (3H, m), 7.60 (1H, d, J = 3.0 Hz), 7.69 (1H, dd, J = 2.4 Hz, 8.8 Hz), 7.93 (1H, s), 7.99 (1H, d, J = 2.4 Hz), 8.33 (1H, s), 8.62 (1H, br s), 13.24 (1H, br s).
Example 87
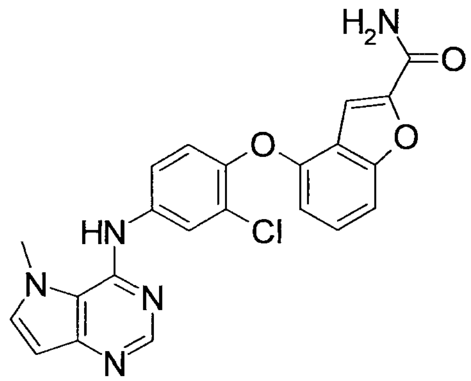

Production of 4-{2-chloro-4-[(5-methyl-5H-pyrrolo[3,2-d]pyrimidin-4-yl)amino]phenoxy}-1-benzofuran-2-carboxamide

(i) Production of 4-(2-chloro-4-nitrophenoxy)-1-benzofuran-2-carboxamide

To a solution of 2-chloro-1-fluoro-4-nitrobenzene (1.8 g) and 4-hydroxy-1-benzofuran-2-carboxamide (1.4 g) in N,N-dimethylformamide (30 mL) was added potassium carbonate (3.1 g), and the mixture was stirred at room temperature for 4 hr. Water was added to the reaction mixture, and the mixture was extracted with ethyl acetate. The organic layer was washed with saturated brine, and dried over anhydrous magnesium sulfate. Anhydrous magnesium sulfate was filtered off and the filtrate was concentrated under reduced pressure. The obtained residue was separated and purified by silica gel chromatography (hexane:ethyl acetate=19:1→4:1) to give the title compound (2.0 g) as a white powder.
$^1$H-NMR (DMSO-$d_6$) δ: 7.09 (1H, d, J = 9.2 Hz), 7.11-7.16 (1H, m), 7.36 (1H, d, J = 0.9 Hz), 7.55 (1H, t, J = 8.1 Hz), 7.61-7.67 (1H, m), 7.71 (1H, br s), 8.12 (1H, br s), 8.17 (1H, dd, J = 2.7 Hz, 9.2 Hz), 8.54 (1H, d, J = 2.7 Hz).

(ii) Production of 4-{2-chloro-4-[(5-methyl-5H-pyrrolo[3,2-d]pyrimidin-4-yl)amino]phenoxy}-1-benzofuran-2-carboxamide

4-(2-Chloro-4-nitrophenoxy)-1-benzofuran-2-carboxamide (150 mg) was dissolved in a mixed solvent of methanol (5 mL)/tetrahydrofuran (5 mL), 5% platinum/activated carbon (50 mg) was added, and the mixture was stirred under hydrogen atmosphere for 1 hr. The reaction mixture was filtered through celite, and the filtrate was concentrated under reduced pressure. The residue and 4-chloro-5-methyl-5H-pyrrolo[3,2-d]pyrimidine (65 mg) were dissolved in isopropyl alcohol (5 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (85 mg) as a yellow powder.
$^1$H-NMR (DMSO-$d_6$) δ: 4.16 (3H, s), 6.46 (1H, d, J = 3.0 Hz), 6.60-6.68 (1H, m), 7.26 (1H, d, J = 8.8 Hz), 7.37-7.43 (2H, m), 7.49 (1H, s), 7.61 (1H, d, J = 3.0 Hz), 7.68 (2H, dd, J = 2.4 Hz, 8.8 Hz), 8.01 (1H, d, J = 2.4 Hz), 8.14 (1H, br s), 8.33 (1H, s), 8.62 (1H, s).
Example 88
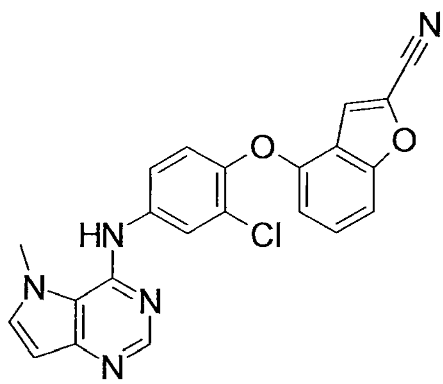

Production of 4-{2-chloro-4-[(5-methyl-5H-pyrrolo[3,2-d]pyrimidin-4-yl)amino]phenoxy}-1-benzofuran-2-carbonitrile

(i) Production of 4-(4-amino-2-chlorophenoxy)-1-benzofuran-2-carbonitrile

4-(2-Chloro-4-nitrophenoxy)-1-benzofuran-2-carboxamide (1.5 g) was dissolved in phosphorus oxychloride (8.0 mL), and the solution was stirred with heating under reflux for 1 hr. The reaction mixture was cooled to 0°C, water and saturated aqueous sodium hydrogen carbonate solution were added, and the mixture was extracted with ethyl acetate. The organic layer was washed with saturated brine, dried over anhydrous magnesium sulfate, and concentrated under reduced pressure. The residue was dissolved in ethanol (20 mL), reduced iron (1.0 g), calcium chloride (500 mg) and water (1.0 mL) were added, and the mixture was stirred with heating under reflux at 110°C for 1 hr. The reaction mixture was cooled to room temperature, and saturated aqueous sodium hydrogen carbonate solution was added. The reaction mixture was filtered through celite, and the obtained filtrate was diluted with ethyl acetate, washed with water and saturated brine, and dried over anhydrous magnesium sulfate. Anhydrous magnesium sulfate was filtered off and the filtrate was concentrated under reduced pressure. The obtained residue was separated and purified by silica gel chromatography (hexane:ethyl acetate=50:1→1:1) to give the title compound (720 mg) as a pale-purple powder.

$^1$H-NMR (DMSO-$d_6$) δ: 5.45 (2H, br s), 6.43-6.50 (1H, m), 6.60 (1H, dd, J = 2.6 Hz, 8.7 Hz), 6.77 (1H, d, J = 2.6 Hz), 7.04 (1H, d, J = 8.7 Hz), 7.31-7.53 (2H, m), 8.16 (1H, d, J = 0.9 Hz).

(ii) Production of 4-{2-chloro-4-[(5-methyl-5H-pyrrolo[3,2-d]pyrimidin-4-yl)amino]phenoxy}-1-benzofuran-2-carbonitrile

A mixture of 4-chloro-5-methyl-5H-pyrrolo[3,2-d]pyrimidine (100 mg) and 4-(4-amino-2-chlorophenoxy)-1-benzofuran-2-carbonitrile (170 mg) was dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (145 mg) as a white powder.
$^1$H-NMR (DMSO-$d_6$) δ: 4.16 (3H, s), 6.47 (1H, d, J = 3.0 Hz), 6.60 (1H, dd, J = 1.1 Hz, 7.5 Hz), 7.35 (1H, d, J = 8.8 Hz), 7.43-7.57 (2H, m), 7.61 (1H, d, J = 3.0 Hz), 7.72 (1H, dd, J = 2.2 Hz, 8.8 Hz), 8.01 (1H, d, J = 2.2 Hz), 8.24 (1H, d, J = 1.1 Hz), 8.33 (1H, s), 8.65 (1H, s).

Example 89

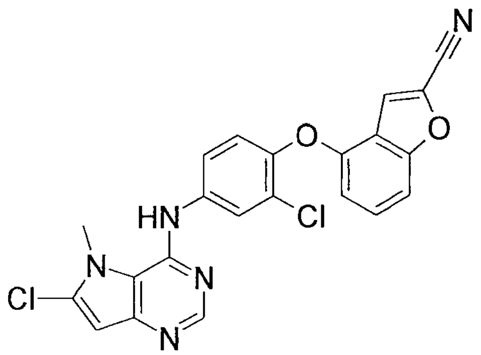

Production of 4-{2-chloro-4-[(6-chloro-5-methyl-5H-pyrrolo[3,2-d]pyrimidin-4-yl)amino]phenoxy}-1-benzofuran-2-carbonitrile

A mixture of 4,6-dichloro-5-methyl-5H-pyrrolo[3,2-d]pyrimidine (100 mg) and 4-(4-amino-2-chlorophenoxy)-1-benzofuran-2-carbonitrile (155 mg) were dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (105 mg) as a white powder.
$^1$H-NMR (DMSO-$d_6$) δ: 4.06 (3H, s), 6.60 (1H, dd, J = 1.1 Hz, 7.3 Hz), 6.70 (1H, s), 7.35 (1H, d, J = 8.8 Hz), 7.43-7.58 (2H, m), 7.62-7.71 (1H, m), 7.95 (1H, s), 8.24 (1H, d, J = 0.7 Hz), 8.36 (1H, s), 8.84 (1H, s).

Example 90

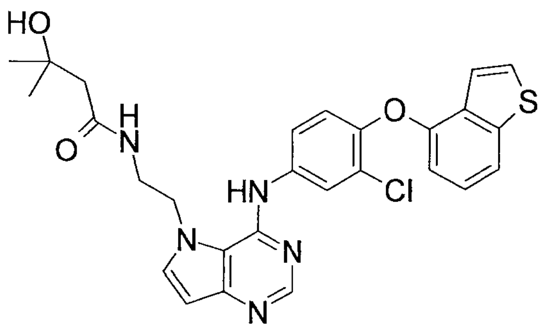

Production of N-[2-(4-{[4-(1-benzothiophen-4-yloxy)-3-chlorophenyl]amino}-5H-pyrrolo[3,2-d]pyrimidin-5-yl)ethyl]-3-hydroxy-3-methylbutanamide

(i) Production of 5-(2-aminoethyl)-N-[4-(1-benzothiophen-4-yloxy)-3-chlorophenyl]-5H-pyrrolo[3,2-d]pyrimidin-4-amine dihydrochloride

The title compound (410 mg) was obtained as a yellow powder by reaction in the same manner as in Example 55 (i) and using tert-butyl [2-(4-chloro-5H-pyrrolo[3,2-d]pyrimidin-5-yl)ethyl]carbamate (400 mg), 4-(1-benzothiophen-4-yloxy)-3-chloroaniline (420 mg), isopropyl alcohol (10 mL), ethanol (10 mL) and 4N hydrochloric acid (5.0 mL).
$^1$H-NMR (DMSO-$d_6$) δ: 3.21-3.36 (2H, m), 5.05 (2H, t, J = 6.2 Hz), 6.71-6.82 (2H, m), 7.17 (1H, d, J = 8.9 Hz), 7.38 (1H, t, J = 8.0 Hz), 7.46 (1H, dd, J = 0.8 Hz, 5.5 Hz), 7.59 (1H, dd, J = 2.5 Hz, 8.9 Hz), 7.78-7.85 (2H, m), 7.93 (1H, d, J = 2.5 Hz), 8.09 (1H, d, J = 3.2 Hz), 8.34-8.42 (3H, m), 8.73 (1H, s), 10.19 (1H, br s).

(ii) Production of N-[2-(4-{[4-(1-benzothiophen-4-yloxy)-3-chlorophenyl]amino}-5H-pyrrolo[3,2-d]pyrimidin-5-yl)ethyl]-3-hydroxy-3-methylbutanamide

A mixture of 5-(2-aminoethyl)-N-[4-(1-benzothiophen-4-yloxy)-3-chlorophenyl]-5H-pyrrolo[3,2-d]pyrimidin-4-amine dihydrochloride (120 mg) and 3-hydroxy-3-methylbutanoic acid (80 mg) were dissolved in N,N-dimethylformamide (3.0 mL), triethylamine (0.9 mL), 1-hydroxybenzotriazole (50 mg), 1-ethyl-3-(3-dimethylaminopropyl)carbodiimide hydrochloride (250 mg) were added, and the mixture was stirred at room temperature for 5 hr. Water was added to the reaction mixture, and the mixture was extracted with ethyl acetate. The organic layer was dried over anhydrous magnesium sulfate and concentrated under reduced pressure. The residue was separated and purified by silica gel column chromatography (hexane:ethyl acetate=20:80→0:100→ethyl acetate:methanol=85:15) to give the title compound (100 mg) as a white powder.
$^1$H-NMR (DMSO-$d_6$) δ: 1.13 (6H, s), 2.20 (2H, s), 3.37-3.49 (2H, m), 4.44-4.58 (2H, m), 4.67 (1H, s), 6.49 (1H, d, J = 3.2 Hz), 6.65 (1H, d, J = 7.3 Hz), 7.17 (1H, d, J = 8.8 Hz), 7.32 (1H, t, J = 7.3 Hz), 7.53 (1H, dd, J = 0.7 Hz, 5.4 Hz), 7.64 (1H, d, J = 2.4 Hz), 7.70-7.86 (3H, m), 8.05 (1H, d, J = 2.4 Hz), 8.19-8.36 (2H, m), 8.87 (1H, s).
Example 91
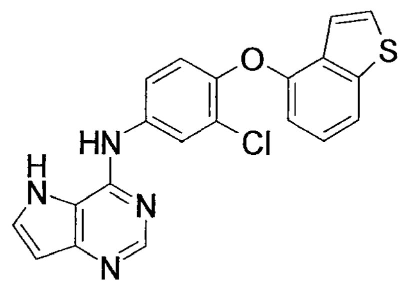

Production of N-[4-(1-benzothiophen-4-yloxy)-3-chlorophenyl]-5H-pyrrolo[3,2-d]pyrimidin-4-amine

The title compound (91 mg) was obtained as a white powder in the same manner as in Example 88 (ii) and using 4-chloro-5H-pyrrolo[3,2-d]pyrimidine (50 mg), 4-(1-benzothiophen-4-yloxy)-3-chloroaniline (90 mg) and isopropyl alcohol (7 mL). $^1$H-NMR (DMSO-$d_6$) δ: 6.51 (1H, d, J = 1.8 Hz), 6.65 (1H, d, J = 7.3 Hz), 7.22 (1H, d, J = 8.8 Hz), 7.32 (1H, t, J = 7.3 Hz), 7.52 (1H, dd, J = 0.7 Hz, 5.4 Hz), 7.61-7.71 (2H, m), 7.73-7.83 (2H, m), 8.38-8.45 (2H, m), 9.50 (1H, s), 11.19 (1H, d, J = 1.8 Hz).
Example 92
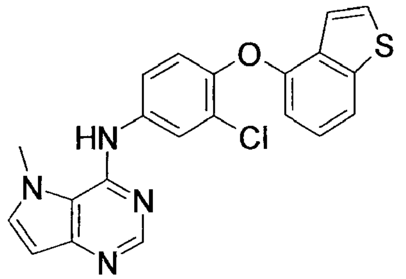

Production of N-[4-(1-benzothiophen-4-yloxy)-3-chlorophenyl]-5-methyl-5H-pyrrolo[3,2-d]pyrimidin-4-amine

The title compound (48 mg) was obtained as a white powder in the same manner as in Example 88 (ii) and using 4-chloro-5-methyl-5H-pyrrolo[3,2-d]pyrimidine (50 mg), 4-(1-benzothiophen-4-yloxy)-3-chloroaniline (90 mg) and isopropyl alcohol (5 mL).
$^1$H-NMR (DMSO-$d_6$) δ: 4.16 (3H, s), 6.45 (1H, d, J = 3.0 Hz), 6.61-6.67 (1H, m), 7.18 (1H, d, J = 8.8 Hz), 7.32 (1H, t, J = 8.0 Hz), 7.53 (1H, dd, J = 0.8 Hz, 5.5 Hz), 7.58-7.68 (2H, m), 7.73-7.82 (2H, m), 7.98 (1H, d, J = 2.6 Hz), 8.31 (1H, s), 8.59 (1H, s).
Example 93
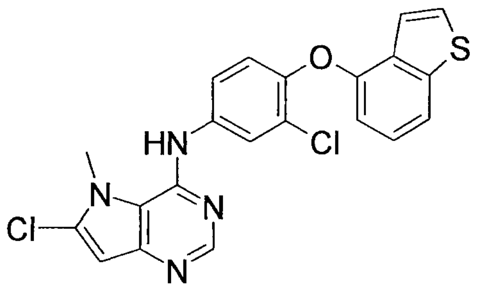

Production of N-[4-(1-benzothiophen-4-yloxy)-3-chlorophenyl]-6-chloro-5-methyl-5H-pyrrolo[3,2-d]pyrimidin-4-amine

The title compound (60 mg) was obtained as a white powder in the same manner as in Example 88 (ii) and using 4,6-dichloro-5-methyl-5H-pyrrolo[3,2-d]pyrimidine (50 mg), 4-(1-benzothiophen-4-yloxy)-3-chloroaniline (85 mg) and isopropyl alcohol (5 mL).
$^1$H-NMR (DMSO-$d_6$) δ: 4.06 (3H, s), 6.62-6.71 (2H, m), 7.18 (1H, d, J = 8.6 Hz), 7.32 (1H, t, J = 8.1 Hz), 7.52 (1H, dd, J = 0.7 Hz, 5.4 Hz), 7.57-7.64 (1H, m), 7.72-7.82 (2H, m), 7.93 (1H, s), 8.34 (1H, s), 8.77 (1H, s).
Example 94
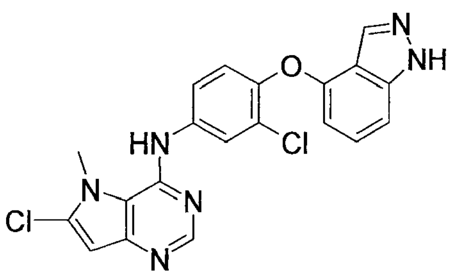

Production of 6-chloro-N-[3-chloro-4-(1H-indazol-4-yloxy)phenyl]-5-methyl-5H-pyrrolo[3,2-d]pyrimidin-4-amine

The title compound (31 mg) was obtained as a white powder in the same manner as in Example 88 (ii) and using 4,6-dichloro-5-methyl-5H-pyrrolo[3,2-d]pyrimidine (50 mg), 3-chloro-4-(1H-indazol-4-yloxy)aniline (90 mg) and isopropyl alcohol (7 mL).
$^1$H-NMR (DMSO-$d_6$) δ: 4.07 (3H, s), 6.27-6.39 (1H, m), 6.69 (1H, s), 7.19-7.35 (3H, m), 7.52-7.71 (1H, m), 7.93 (2H, s), 8.34 (1H, s), 8.80 (1H, s), 13.25 (1H, s).
Example 95
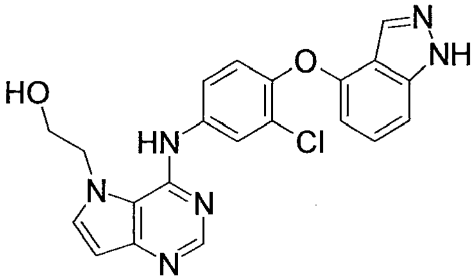

Production of 2-(4-{[3-chloro-4-(1H-indazol-4-yloxy)phenyl]amino}-5H-pyrrolo[3,2-d]pyrimidin-5-yl)ethanol

The title compound (27 mg) was obtained as a white powder by reaction in the same manner as in Example 2 (iv) and using 2-(4-chloro-5H-pyrrolo[3,2-d]pyrimidin-5-yl)ethyl benzoate (50 mg), 3-chloro-4-(1H-indazol-4-yloxy)aniline (45 mg), isopropyl alcohol (5 mL) and 1N aqueous sodium hydroxide solution (2.0 mL).
$^1$H-NMR (DMSO-$d_6$) δ: 3.89 (2H, br s), 4.55 (2H, br s), 6.25-6.40 (1H, m), 6.71 (1H, s), 7.15-7.35 (3H, m), 7.50-7.74 (1H, m), 7.92 (2H, s), 8.33 (1H, s), 8.79 (1H, s), 13.26 (1H, s).
Example 96
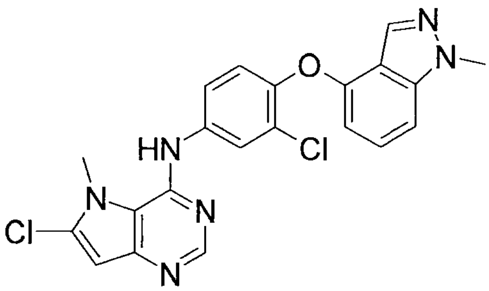

Production of 6-chloro-N-{3-chloro-4-[(1-methyl-1H-indazol-4-yl)oxy]phenyl}-5-methyl-5H-pyrrolo[3,2-d]pyrimidin-4-amine

The title compound (131 mg) was obtained as a white powder in the same manner as in Example 88 (ii) and using 4,6-dichloro-5-methyl-5H-pyrrolo[3,2-d]pyrimidine (74 mg), 3-chloro-4-[(1-methyl-1H-indazol-4-yl)oxy]aniline (100 mg) and isopropyl alcohol (5 mL).
$^1$H-NMR (DMSO-$d_6$) δ: 4.06 (3H, s), 4.08 (3H, s), 6.36 (1H, dd, J = 1.1, 6.9 Hz), 6.72 (1H, s), 7.25-7.39 (3H, m), 7.64 (1H, dd, J = 2.4 Hz, 8.8 Hz), 7.88-7.97 (2H, m), 8.39 (1H, s), 8.94 (1H, s).
Example 97
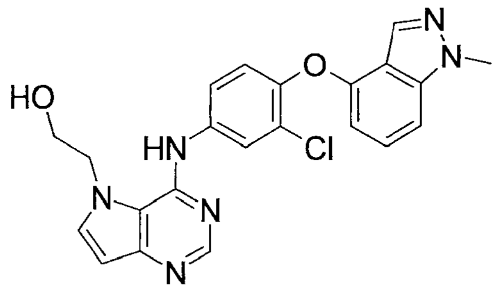

Production of 2-[4-({3-chloro-4-[(1-methyl-1H-indazol-4-yl)oxy]phenyl}amino)-5H-pyrrolo[3,2-d]pyrimidin-5-yl]ethanol

The title compound (90 mg) was obtained as a white powder by reaction in the same manner as in Example 2(iv) and using 2-(4-chloro-5H-pyrrolo[3,2-d]pyrimidin-5-yl)ethyl benzoate (110 mg), 3-chloro-4-[(1-methyl-1H-indazol-4-yl)oxy]aniline (100 mg), isopropyl alcohol (5 mL) and 1N aqueous sodium hydroxide solution (3.0 mL).
$^1$H-NMR (DMSO-$d_6$) δ: 3.89 (2H, br s), 4.06 (3H, s), 4.55 (2H, t, J = 4.6 Hz), 6.33 (2H, dd, J = 0.7, 7.0 Hz), 6.52 (1H, d, J = 3.0 Hz), 7.24-7.38 (3H, m), 7.57-7.70 (2H, m), 7.92 (1H, d, J = 0.7 Hz), 8.01 (1H, d, J = 2.4 Hz), 8.36 (1H, s), 9.92 (1H, s).

Example 98

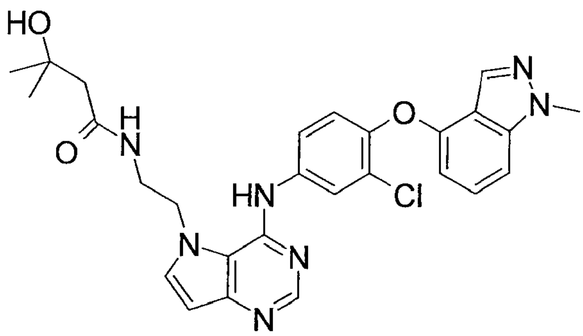

Production of N-{2-[4-({3-chloro-4-[(1-methyl-1H-indazol-4-yl)oxy]phenyl}amino)-5H-pyrrolo[3,2-d]pyrimidin-5-yl]ethyl}-3-hydroxy-3-methylbutanamide

(i) Production of 5-(2-aminoethyl)-N-{3-chloro-4-[(1-methyl-1H-indazol-4-yl)oxy]phenyl}-5H-pyrrolo[3,2-d]pyrimidin-4-amine dihydrochloride

The title compound (540 mg) was obtained as a yellow powder by reaction in the same manner as in Example 55(i) and using tert-butyl [2-(4-chloro-5H-pyrrolo[3,2-d]pyrimidin-5-yl)ethyl]carbamate (357 mg), 3-chloro-4-[(1-methyl-1H-indazol-4-yl)oxy]aniline (330 mg), isopropyl alcohol (15 mL), ethanol (15 mL) and 4N hydrochloric acid (5.0 mL).
$^1$H-NMR (DMSO-$d_6$) δ: 3.32 (2H, br s), 4.07 (3H, s), 5.08 (2H, t, J = 6.1 Hz), 6.35-6.50 (1H, m), 6.76 (1H, d, J = 3.2 Hz), 7.24-7.47 (3H, m), 7.65 (1H, dd, J = 2.5, 8.9 Hz), 7.85-7.98 (2H, m), 8.10 (1H, d, J = 3.2 Hz), 8.40 (3H, br s), 8.75 (1H, s), 10.26 (1H, br s).

(ii) Production of N-{2-[4-({3-chloro-4-[(1-methyl-1H-indazol-4-yl)oxy]phenyl}amino)-5H-pyrrolo[3,2-d]pyrimidin-5-yl]ethyl}-3-hydroxy-3-methylbutanamide

The title compound (155 mg) was obtained as a white powder by reaction in the same manner as in Example 90(ii) and using 5-(2-aminoethyl)-N-{3-chloro-4-[(1-methyl-1H-indazol-4-yl)oxy]phenyl}-5H-pyrrolo[3,2-d]pyrimidin-4-amine dihydrochloride (200 mg), 3-hydroxy-3-methylbutanoic acid (240 mg), N,N-dimethylformamide (20 mL), triethylamine (0.83 mL), 1-hydroxybenzotriazole (10 mg) and 1-ethyl-3-(3-dimethylaminopropyl)carbodiimide hydrochloride (330 mg).
$^1$H-NMR (DMSO-$d_6$) δ: 1.13 (6H, s), 2.20 (2H, s), 3.37-3.47 (2H, m), 4.06 (3H, s), 4.49-4.58 (2H, m), 4.67 (1H, s), 6.34

(1H, dd, J = 1.0, 7.0 Hz), 6.50 (1H, d, J = 3.2 Hz), 7.23-7.38 (3H, m), 7.64 (1H, d, J = 3.2 Hz), 7.82 (1H, dd, J = 2.3, 8.9 Hz), 7.93 (1H, d, J = 0.7 Hz), 8.06 (1H, d, J = 2.3 Hz), 8.20-8.28 (1H, m), 8.34 (1H, s), 8.89 (1H, s).

Example 99

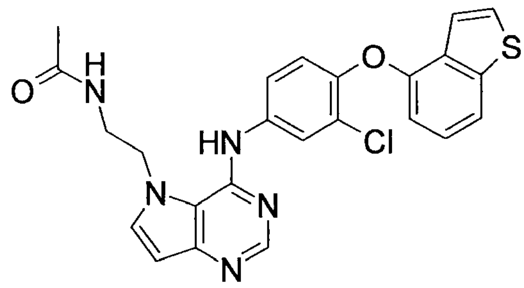

Production of N-[2-(4-{[4-(1-benzothiophen-4-yloxy)-3-chlorophenyl]amino}-5H-pyrrolo[3,2-d]pyrimidin-5-yl)ethyl]acetamide

The title compound (41 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (90 mg), acetic acid (0.15 mL), N,N-dimethylformamide (6 mL), triethylamine (0.57 mL), 1-hydroxybenzotriazole (10 mg) and 1-ethyl-3-(3-dimethylaminopropyl)carbodiimide hydrochloride (220 mg).
$^1$H-NMR (DMSO-$d_6$) δ: 1.79 (3H, s), 3.34-3.42 (2H, m), 4.44-4.57 (2H, m), 6.50 (1H, d, J = 3.2 Hz), 6.65 (1H, d, J = 7.9 Hz), 7.18 (1H, d, J = 8.8 Hz), 7.33 (1H, t, J = 7.7 Hz), 7.53 (1H, dd, J = 0.7, 5.4 Hz), 7.62-7.84 (4H, m), 8.04 (1H, d, J = 2.4 Hz), 8.21-8.29 (1H, m), 8.33 (1H, s), 8.78 (1H, s).

Example 100

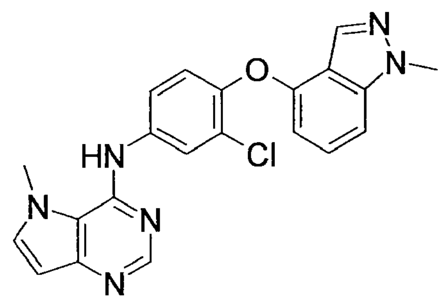

Production of N-{3-chloro-4-[(1-methyl-1H-indazol-4-yl)oxy]phenyl}-5-methyl-5H-pyrrolo[3,2-d]pyrimidin-4-amine

The title compound (63 mg) was obtained as a white powder in the same manner as in Example 88 (ii) and using 4-chloro-5-methyl-5H-pyrrolo[3,2-d]pyrimidine (50 mg), 3-chloro-4-[(1-methyl-1H-indazol-4-yl)oxy]aniline (95 mg) and isopropyl alcohol (10 mL).
$^1$H-NMR (DMSO-$d_6$) δ: 4.06 (3H, s), 4.16 (3H, s), 6.34 (1H, dd, J = 1.1, 6.9 Hz), 6.46 (1H, d, J = 3.0 Hz), 6.55 (1H, s), 7.26-7.37 (2H, m), 7.61 (1H, d, J = 3.0 Hz), 7.69 (1H, dd, J = 2.6, 8.8 Hz), 7.92 (1H, d, J = 0.7 Hz), 7.99 (1H, d, J = 2.6 Hz), 8.33 (1H, s), 8.62 (1H, s).

Example 101

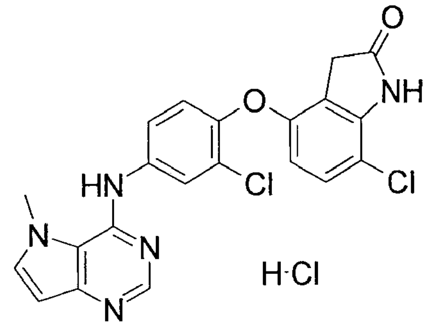

Production of 7-chloro-4-{2-chloro-4-[(5-methyl-5H-pyrrolo[3,2-d]pyrimidin-4-yl)amino]phenoxy}-1,3-dihydro-2H-indol-2-one hydrochloride

(i) Production of 7-chloro-4-(2-chloro-4-nitrophenoxy)-1,3-dihydro-2H-indol-2-one

To a solution of 2-chloro-1-fluoro-4-nitrobenzene (1.06 g) and 7-chloro-4-hydroxy-1,3-dihydro-2H-indol-2-one (720 mg) in N,N-dimethylformamide (15 mL) was added potassium carbonate (542 mg), and the mixture was stirred at room temperature for 4 hr. Water was added to the reaction mixture, and the mixture was extracted with ethyl acetate. The organic layer was washed with saturated brine, dried over anhydrous magnesium sulfate, and concentrated under reduced pressure. The obtained residue was separated and purified by silica gel chromatography (hexane:ethyl acetate=20:1→4:1) to give the title compound (170 mg) as a brown oil.
$^1$H-NMR (DMSO-$d_6$) δ: 3.25 (2H, s), 6.75 (1H, d, J = 8.5 Hz), 6.89 (1H, d, J = 8.5 Hz), 7.29 (1H, d, J = 9.1 Hz), 7.50 (1H, t, J = 8.5 Hz), 8.24 (1H, dd, J = 2.6, 9.1 Hz), 8.53 (1H, d, J = 2.6 Hz).

(ii) Production of 4-(4-amino-2-chlorophenoxy)-7-chloro-1,3-dihydro-2H-indol-2-one

7-Chloro-4-(2-chloro-4-nitrophenoxy)-1,3-dihydro-2H-indol-2-one (150 mg) was dissolved in ethyl acetate (10 mL), 5% platinum/activated carbon (40 mg) was added, and the mixture was stirred at under hydrogen atmosphere for 1 hr. The reaction mixture was filtered through celite, and the filtrate was concentrated under reduced pressure. The residue was separated and purified by silica gel column chromatography (hexane:ethyl acetate=50:50→0:100) to give the title compound (115 mg) as a brown oil.
$^1$H-NMR (DMSO-$d_6$) δ: 3.45 (2H, br s), 5.36 (2H, s), 6.19 (1H, d, J = 9.1 Hz), 6. 54 (1H, dd, J = 2.6, 8.7 Hz), 6.70 (1H, d, J = 2.6 Hz), 6.92 (1H, d, J = 8.7 Hz), 7.14 (1H, d, J = 9.1 Hz), 10.84 (1H, br s).

(iii) Production of 7-chloro-4-{2-chloro-4-[(5-methyl-5H-pyrrolo[3,2-d]pyrimidin-4-yl)amino]phenoxy}-1,3-dihydro-2H-indol-2-one hydrochloride

The title compound (47 mg) was obtained as a white powder in the same manner as in Example 88 (ii) and using 4-chloro-5-methyl-5H-pyrrolo[3,2-d]pyrimidine (70 mg), 4-(4-amino-2-chlorophenoxy)-7-chloro-1,3-dihydro-2H-indol-2-one (130 mg) and isopropyl alcohol (5 mL).
$^1$H-NMR (DMSO-$d_6$) δ: 3.50 (2H, s), 4.24 (3H, s), 6.42 ( 1H, d, J = 8.8 Hz), 6.59 (1H, d, J = 3.2 Hz), 7.10-7.30 (2H, m), 7.59 (1H, dd, J = 2.6, 8.8 Hz), 7.89 (2H, t, J = 2.6 Hz), 8.63 (1H, s), 9.50 (1H, br s), 10.95 (1H, s).
Example 102
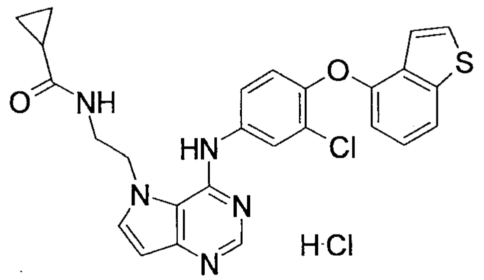

Production of N-[2-(4-{[4-(1-benzothiophen-4-yloxy)-3-chlorophenyl]amino}-5H-pyrrolo[3,2-d]pyrimidin-5-yl)ethyl]cyclopropanecarboxamide hydrochloride

The title compound (110 mg) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (150 mg), cyclopropanecarboxylic acid (70 mg), N,N-dimethylformamide (7 mL), triethylamine (0.9 mL), 1-hydroxybenzotriazole (50 mg) and 1-ethyl-3-(3-dimethylaminopropyl)carbodiimide hydrochloride (350 mg), and treatment with 4N hydrochloric acid/ethyl acetate (1 mL).
$^1$H-NMR (DMSO-$d_6$) δ: 0.52-0.70 (4H, m), 1.44-1.60 (1H, m), 3.43-3.50 (2H, m), 4.68 (2H, t, J = 6.6 Hz), 6.68 (1H, d, J = 3.2 Hz), 6.77 (1H, d, J = 7.7 Hz), 7.18 (1H, d, J = 8.8 Hz), 7.38 (1H, t, J = 7.7 Hz), 7.47 (1H, d, J = 5.4 Hz), 7.64 (1H, dd, J = 2.7, 8.8 Hz), 7.78-7.90 (2H, m), 7.97 (2H, dd, J = 2.7, 8.8 Hz), 8.60 (1H, t, J = 5.5 Hz), 8.72 (1H, s), 10.20 (1H, br s).
Example 103
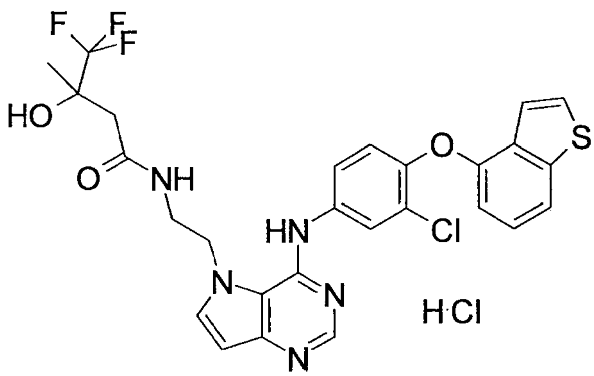

Production of N-[2-(4-{[4-(1-benzothiophen-4-yloxy)-3-chlorophenyl]amino}-5H-pyrrolo[3,2-d]pyrimidin-5-yl)ethyl]-4,4,4-trifluoro-3-hydroxy-3-methylbutanamide hydrochloride

The title compound (100 mg) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (150 mg), 4,4,4-trifluoro-3-hydroxy-3-methylbutanoic acid (80 mg), N,N-dimethylformamide (7 mL), triethylamine (0.9 mL), 1-hydroxybenzotriazole (50 mg) and 1-ethyl-3-(3-dimethylaminopropyl)carbodiimide hydrochloride (400 mg), and treatment with 4N hydrochloric acid/ethyl acetate (1 mL).
$^1$H-NMR (DMSO-$d_6$) δ: 1.33 (3H, s), 2.45 (2H, s), 3.47-3.55 (2H, m), 4.67 (2H, br s), 6.55 (1H, s), 6.63-6.80 (2H, m), 7.18 (1H, d, J = 8.8 Hz), 7.32-7.51 (2H, m), 7.64 (1H, dd, J = 2.4, 8.8 Hz), 7.78-7.86 (2H, m), 7.92-8.01 (2H, m), 8.47-8.60 (1H, m), 8.73 (1H, s), 10.10 (1H, br s).
Example 104
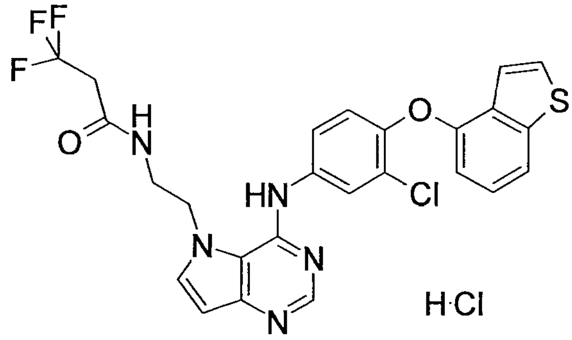

Production of N-[2-(4-{[4-(1-benzothiophen-4-yloxy)-3-chlorophenyl]amino}-5H-pyrrolo[3,2-d]pyrimidin-5-yl)ethyl]-3,3,3-trifluoropropanamide hydrochloride

The title compound (100 mg) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (150 mg), 3,3,3-trifluoropropanoic acid (80 mg), N,N-dimethylformamide (7 mL), triethylamine (0.9 mL), 1-hydroxybenzotriazole (50 mg) and 1-ethyl-3-(3-dimethylaminopropyl)carbodiimide hydrochloride (400 mg), and treatment with 4N hydrochloric acid/ethyl acetate (1 mL).
$^1$H-NMR (DMSO-$d_6$) δ: 2.99 (2H, br s), 3.50 (2H, br s), 4.66-4.78 (2H, m), 6.06 (1H, s), 6.67 (1H, d, J = 3.0 Hz), 6.74-6.81 (1H, m), 7.11-7.26 (1H, m), 7.31-7.50 (2H, m), 7.60 (1H, dd, J = 2.8, 8.7 Hz), 7.77-7.87 (2H, m), 7.93 (2H, d, J = 2.8 Hz), 8.59 (1H, s), 8.74 (1H, s), 9.93 (1H, s).
Example 105
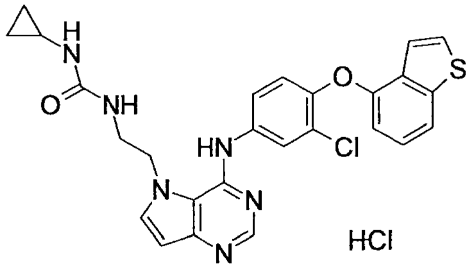

Production of N-[2-(4-{[4-(1-benzothiophen-4-yloxy)-3-chlorophenyl]amino}-5H-pyrrolo[3,2-d]pyrimidin-5-yl)ethyl]-N'-cyclopropylurea hydrochloride

A mixture of cyclopropanamine (51 mg) and 4-nitrophenyl chlorocarbonate (181 mg) was dissolved in tetrahydrofuran (25 mL), triethylamine (91 mg) was added, and the mixture was stirred at room temperature for 3 hr. 5-(2-Aminoethyl)-N-[4-(1-benzothiophen-4-yloxy)-3-chlorophenyl]-5H-pyrrolo[3,2-d]pyrimidin-4-amine dihydrochloride (300 mg) and triethylamine (300 mg) were added to the reaction mixture, and the mixture was stirred with heating under reflux for 1 hr. The reaction mixture was cooled to 0°C, water and saturated aqueous sodium hydrogen carbonate solution were added, and the mixture was extracted with ethyl acetate. The organic layer was washed with saturated brine, dried over anhydrous magnesium sulfate, and concentrated under reduced pressure. The residue was separated and purified by silica gel column chromatography (ethyl acetate:methanol=100:0→90:10), and the obtained residue was treated with hydrochloric acid (5 mL) to give the title compound (52 mg) as a pale-orange powder.
$^1$H-NMR (DMSO-$d_6$) δ: 0.32 (2H, br s), 0.56 (2H, d, J = 6.4 Hz), 2.23-2.41 (1H, m), 3.42-3.57 (2H, m), 4.60 (2H, br s), 6.04 (1H, br s), 6.56-6.82 (4H, m), 7.15 (1H, d, J = 8.8 Hz), 7.31-7.49 (2H, m), 7.59-7.71 (1H, m), 7.79-7.85 (2H, m), 7.99 (2H, d, J = 2.0 Hz), 8.72 (1H, s), 10.59 (1H, s).
Example 106
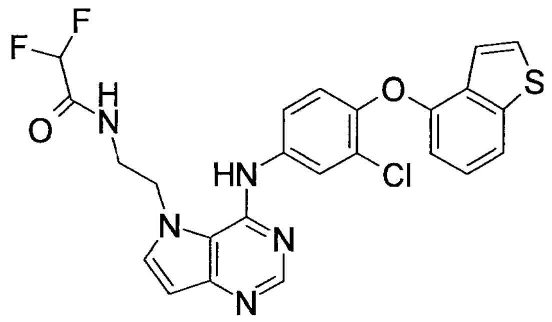

Production of N-[2-(4-{[4-(1-benzothiophen-4-yloxy)-3-chlorophenyl]amino}-5H-pyrrolo[3,2-d]pyrimidin-5-yl)ethyl]-2,2-difluoroacetamide

The title compound (1.5 g) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (2.0 g), difluoroacetic acid (1.0 g), N,N-dimethylformamide (50 mL), triethylamine (7.0 mL), 1-hydroxybenzotriazole (100 mg) and 1-ethyl-3-(3-dimethylaminopropyl)carbodiimide hydrochloride (3.5 g).
$^1$H-NMR (DMSO-$d_6$) δ: 3.50 (2H, br s), 4.63 (2H, t, J = 6.2 Hz), 6.12 (1H, t, J = 57.9 Hz), 6.49 (1H, d, J = 2.8 Hz), 6.66 (1H, d, J = 7.2 Hz), 7.18 (1H, d, J = 8.9 Hz), 7.33 (1H, t, J = 7.9 Hz), 7.49-7.69 (3H, m), 7.73-7.84 (2H, m), 7.94 (1H, br s), 8.34 (1H, s), 8.60 (1H, br s), 8.91 (1H, br s).
Example 107
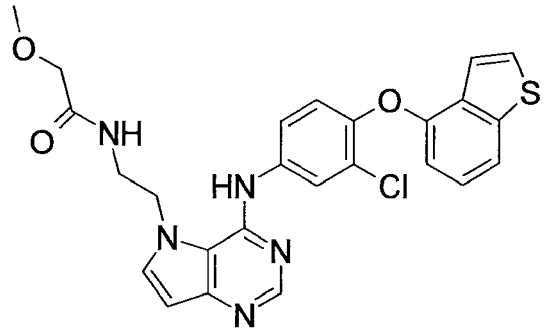

Production of N-[2-(4-{[4-(1-benzothiophen-4-yloxy)-3-chlorophenyl]amino}-5H-pyrrolo[3,2-d]pyrimidin-5-yl)ethyl]-2-methoxyacetamide

The title compound (54 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (100 mg), methoxyacetic acid (0.25 mL), N,N-dimethylformamide (10 mL), triethylamine (1.0 mL), 1-hydroxybenzotriazole (10 mg) and 1-ethyl-3-(3-dimethylaminopropyl)carbodiimide hydrochloride (420 mg).
$^1$H-NMR (DMSO-$d_6$) δ: 3.23 (3H, s), 3.42-3.51 (2H, m), 3.74 (2H, s), 4.57 (2H, t, J = 6.6 Hz), 6.49 (1H, d, J = 2.8 Hz), 6.66 (1H, d, J = 7.2 Hz), 7.18 (1H, d, J = 8.9 Hz), 7.33 (1H, t, J = 8.0 Hz), 7.53 (1H, dd, J = 0.8, 5.5 Hz), 7.60 (1H, d, J = 2.8 Hz), 7.65-7.82 (3H, m), 8.01 (1H, d, J = 2.3 Hz), 8.10 (1H, br s), 8.32 (1H, s), 8.67 (1H, s).
Example 108
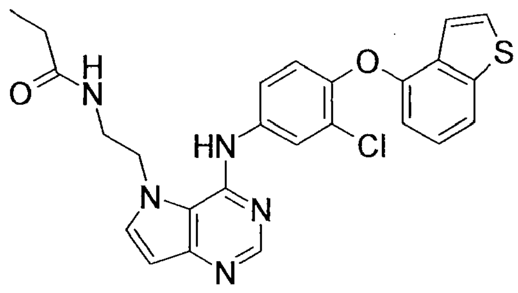

Production of N-[2-(4-{[4-(1-benzothiophen-4-yloxy)-3-chlorophenyl]amino}-5H-pyrrolo[3,2-d]pyrimidin-5-yl)ethyl]propanamide

The title compound (140 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (200 mg), propionic acid (0.32 mL), N,N-dimethylformamide (30 mL), triethylamine (2.0 mL), 1-hydroxybenzotriazole (20 mg) and 1-ethyl-3-(3-dimethylaminopropyl)carbodiimide hydrochloride (560 mg).
$^1$H-NMR (DMSO-$d_6$) δ: 0.90-1.00 (3H, m), 2.06 (2H, q, J = 7.6 Hz), 3.36-3.46 (2H, m), 4.52 (2H, t, J = 6.8 Hz), 6.50 (1H, d, J = 3.0 Hz), 6.65 (1H, d, J = 7.2 Hz), 7.18 (1H, d, J = 8.9 Hz), 7.32 (1H, t, J = 7.9 Hz), 7.49-7.54 (1H, m), 7.63 (1H, d, J = 3.0 Hz), 7.71-7.83 (3H, m), 8.07 (1H, d, J = 2.5 Hz), 8.17 (1H, br s), 8.33 (1H, s), 8.81 (1H, s).
Example 109
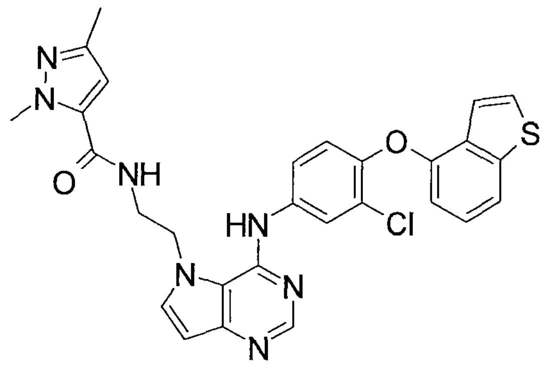

Production of N-[2-(4-{[4-(1-benzothiophen-4-yloxy)-3-chlorophenyl]amino}-5H-pyrrolo[3,2-d]pyrimidin-5-yl)ethyl]-1,3-dimethyl-1H-pyrazole-5-carboxamide

The title compound (125 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (120 mg), 1,3-dimethyl-1H-pyrazole-5-carboxylic acid (92 mg), N,N-dimethylformamide (30 mL), triethylamine (2.0 mL), 1-hydroxybenzotriazole (20 mg) and 1-ethyl-3-(3-dimethylaminopropyl)carbodiimide hydrochloride (400 mg).
$^1$H-NMR (DMSO-$d_6$) δ: 2.11 (3H, s), 3.52 (2H, br s), 3.84 (3H, s), 4.65 (2H, br s), 6.43 (1H, s), 6.47-6.55 (1H, m), 6.66 (1H, d, J = 7.7 Hz), 7.15 (1H, d, J = 8.9 Hz), 7.27-7.40 (1H, m), 7.54 (1H, d, J = 5.3 Hz), 7.61-7.83 (4H, m), 7.94 (1H, s), 8.33 (1H, s), 8.59 (1H, br s), 8.70 (1H, s).
Example 110
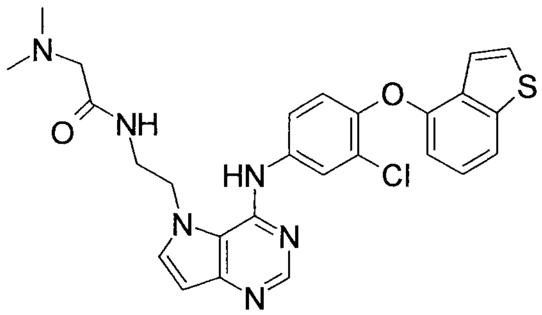

Production of N-[2-(4-{[4-(1-benzothiophen-4-yloxy)-3-chlorophenyl]amino}-5H-pyrrolo[3,2-d]pyrimidin-5-yl)ethyl]-$N^2$, $N^2$-dimethylglycinamide

The title compound (65 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (120 mg), N,N-dimethylglycine (60 mg), N,N-dimethylformamide (10 mL), triethylamine (2.0 mL), 1-hydroxybenzotriazole (10 mg) and 1-ethyl-3-(3-dimethylaminopropyl)carbodiimide hydrochloride (330 mg).
$^1$H-NMR (DMSO-$d_6$) δ: 2.12 (6H, s), 2.81 (2H, s), 3.47 (2H, t, J = 6.2 Hz), 4.56 (2H, t, J = 6.2 Hz), 6.48 (1H, d, J = 2.8 Hz), 6.66 (1H, d, J = 7.2 Hz), 7.18 (1H, d, J = 8.9 Hz), 7.33 (1H, t, J = 7.9 Hz), 7.53 (1H, dd, J = 0.8, 5.7 Hz), 7.57-7.85 (4H, m), 7.95-8.16 (2H, m), 8.32 (1H, s), 8.72 (1H, s).
Example 111
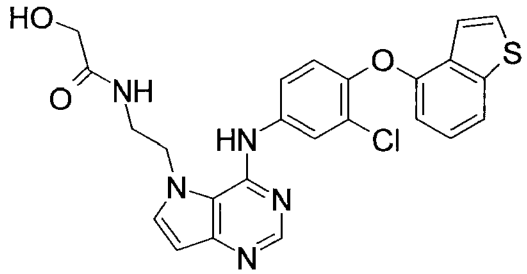

Production of N-[2-(4-{[4-(1-benzothiophen-4-yloxy)-3-chlorophenyl]amino}-5H-pyrrolo[3,2-d]pyrimidin-5-yl)ethyl]-2-hydroxyacetamide

The title compound (72 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (120 mg), hydroxyacetic acid (70 mg), N,N-dimethylformamide (10 mL), triethylamine (1.0 mL), 1-hydroxybenzotriazole (10 mg) and 1-ethyl-3-(3-dimethylaminopropyl)carbodiimide hydrochloride (300 mg).
$^1$H-NMR (DMSO-$d_6$) δ: 3.41-3.53 (2H, m), 3.78 (2H, br s), 4.49-4.59 (2H, m), 5.57 (1H, br s), 6.49 (1H, d, J = 3.0 Hz), 6.66 (1H, d, J = 7.4 Hz), 7.17 (1H, d, J = 8.7 Hz), 7.33 (1H, t, J = 7.9 Hz), 7.51-7.82 (5H, m), 8.02 (1H, d, J = 2.5 Hz), 8.14 (1H, br s), 8.32 (1H, s), 8.72 (1H, s).
Example 112
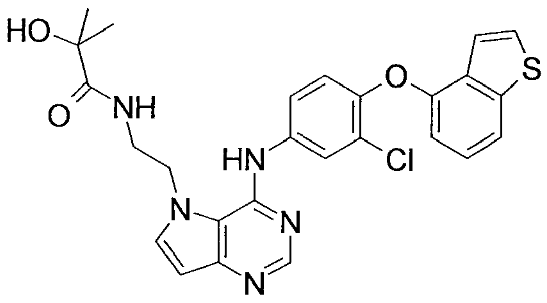

Production of N-[2-(4-{[4-(1-benzothiophen-4-yloxy)-3-chlorophenyl]amino}-5H-pyrrolo[3,2-d]pyrimidin-5-yl)ethyl]-2-hydroxy-2-methylpropanamide

The title compound (101 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (120 mg), 2-hydroxy-2-methylpropanoic acid (80 mg), N,N-dimethylformamide (15 mL), triethylamine (1.0 mL), 1-hydroxybenzotriazole (10 mg) and 1-ethyl-3-(3-dimethylaminopropyl)carbodiimide hydrochloride (300 mg). $^{1}$H-NMR (DMSO-$d_6$) δ: 1.17 (6H, s), 3.42-3.51 (2H, m), 4.54 (2H, br s), 5.39 (1H, br s), 6.48 (1H, d, J = 3.0 Hz), 6.66 (1H, d, J = 7.7 Hz), 7.18 (1H, d, J = 8.9 Hz), 7.33 (1H, t, J = 7.9 Hz), 7.46-7.60 (2H, m), 7.68-7.85 (3H, m), 8.03 (2H, d, J = 2.5 Hz), 8.32 (1H, s), 8.75 (1H, s).

Example 113

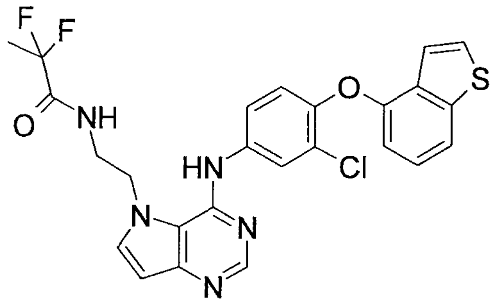

Production of N-[2-(4-{[4-(1-benzothiophen-4-yloxy)-3-chlorophenyl]amino}-5H-pyrrolo[3,2-d]pyrimidin-5-yl)ethyl]-2,2-difluoropropanamide

The title compound (82 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (200 mg), 2,2-difluoropropanoic acid (75 mg), N,N-dimethylformamide (20 mL), triethylamine (1.2 mL), 1-hydroxybenzotriazole (15 mg) and 1-ethyl-3-(3-dimethylaminopropyl)carbodiimide hydrochloride (350 mg). $^{1}$H-NMR (DMSO-$d_6$) δ: 1.57 (3H, t, J = 21.0 Hz), 3.44-3.53 (2H, m), 4.64 (2H, t, J = 6.0 Hz), 6.49 (1H, d, J = 2.8 Hz), 6.65 (1H, d, J = 7.4 Hz), 7.19 (1H, d, J = 8.7 Hz), 7.33 (1H, t, J = 8.0 Hz), 7.46-7.69 (3H, m), 7.72-7.84 (2H, m), 7.94 (1H, d, J = 1.1 Hz), 8.34 (1H, s), 8.60 (1H, br s), 8.75 (1H, br s).

Example 114

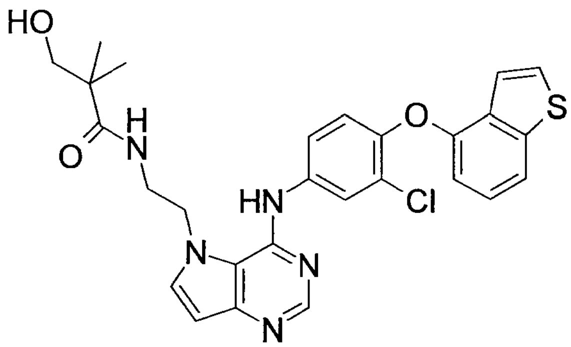

Production of N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide

The title compound (3.2 g) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (5.0 g), 3-hydroxy-2,2-dimethylpropanoic acid (1.9 g), N,N-dimethylformamide (200 mL), triethylamine (10 mL), 1-hydroxybenzotriazole (1.0 g) and 1-ethyl-3-(3-dimethylaminopropyl)carbodiimide hydrochloride (11 g).
$^1$H-NMR (DMSO-$d_6$) δ: 0.99 (6H, s), 3.30-3.33 (2H, m), 3.37-3.47 (2H, m), 4.51 (2H, t, J = 6.8 Hz), 4.84 (1H, s), 6.48 (1H, d, J = 3.0 Hz), 6.66 (1H, d, J = 7.3 Hz), 7.18 (1H, d, J = 8.8 Hz), 7.32 (1H, t, J = 7.9 Hz), 7.54 (1H, dd, J = 0.7, 5.4 Hz), 7.60 (1H, d, J = 3.0 Hz), 7.72-7.92 (4H, m), 8.11 (1H, d, J = 2.2 Hz), 8.33 (1H, s), 8.89 (1H, s).
Example 115
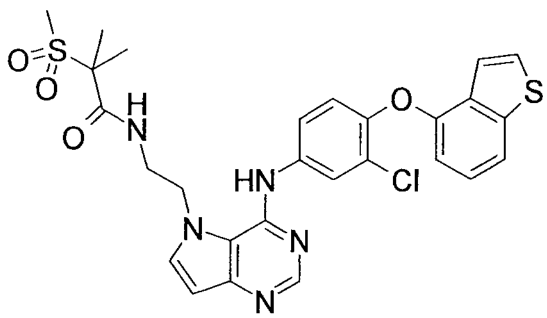

Production of N-[2-(4-{[4-(1-benzothiophen-4-yloxy)-3-chlorophenyl]amino}-5H-pyrrolo[3,2-d]pyrimidin-5-yl)ethyl]-2-methyl-2-(methylsulfonyl)propanamide

The title compound (106 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (150 mg), 2-methyl-2-(methylsulfonyl)propanoic acid (100 mg), N,N-dimethylformamide (20 mL), triethylamine (0.5 mL), 1-hydroxybenzotriazole (50 mg) and 1-ethyl-3-(3-dimethylaminopropyl)carbodiimide hydrochloride (200 mg).
$^1$H-NMR (DMSO-$d_6$) δ: 1.41 (6H, s), 2.96 (3H, s), 3.42-3.55 (2H, m), 4.58 (2H, t, J = 6.2 Hz), 6.48 (1H, d, J = 3.0 Hz), 6.65 (1H, d, J = 7.4 Hz), 7.19 (1H, d, J = 8.9 Hz), 7.33 (1H, t, J = 8.0 Hz), 7.48-7.61 (2H, m), 7.68-7.85 (3H, m), 8.01 (1H, d, J = 2.5 Hz), 8.21 (1H, s), 8.34 (1H, s), 8.67 (1H, s).
Example 116
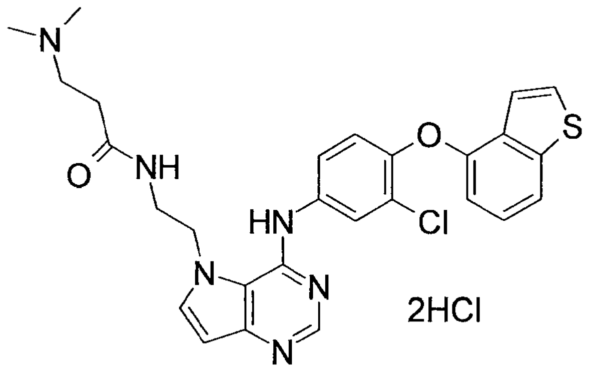

Production of N-[2-(4-{[4-(1-benzothiophen-4-yloxy)-3-chlorophenyl]amino}-5H-pyrrolo[3,2-d]pyrimidin-5-yl)ethyl]-$N^3$,$N^3$-dimethyl-β-alaninamide dihydrochloride

The title compound (1.1 g) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (2.0 g), N,N-dimethyl-β-alanine (1.0 g), N,N-dimethylformamide (100 mL), triethylamine (4.0 mL), 1-hydroxybenzotriazole (200 mg) and 1-ethyl-3-(3-dimethylaminopropyl)carbodiimide hydrochloride (3.0 g), and treatment with 4N hydrochloric acid/ethyl acetate (5 mL). $^1$H-NMR (DMSO-$d_6$) δ: 2.57-2.62 (2H, m), 2.67 (3H, s), 2.68 (3H, s), 2.93-3.06 (2H, m), 3.10-3.22 (2H, m), 3.48-3.63 (2H, m), 4.69-4.83 (2H, m), 6.69 (1H, d, J = 3.2 Hz), 6.78 (1H, d, J = 7.2 Hz), 7.19 (1H, d, J = 8.8 Hz), 7.30-7.51 (2H, m), 7.66 (1H, dd, J = 2.8, 8.8 Hz), 7.77-7.88 (2H, m), 8.00 (2H, dd, J = 2.8, 4.3 Hz), 8.58-8.77 (2H, m), 10.23 (2H, br s), 10.56 (1H, br s).

Example 117

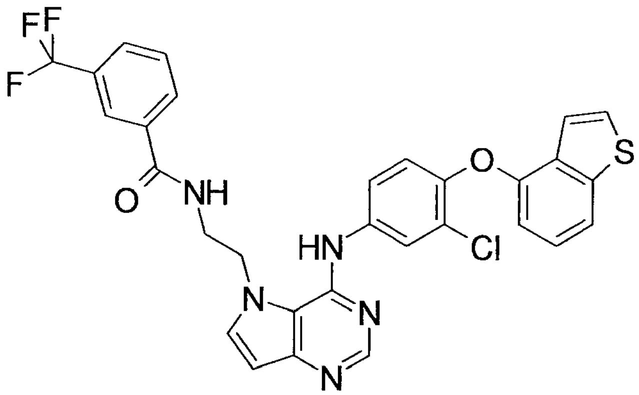

Production of N-[2-(4-{[4-(1-benzothiophen-4-yloxy)-3-chlorophenyl]amino}-5H-pyrrolo[3,2-d]pyrimidin-5-yl)ethyl]-3-(trifluoromethyl)benzamide

5-(2-Aminoethyl)-N-[4-(1-benzothiophen-4-yloxy)-3-chlorophenyl]-5H-pyrrolo[3,2-d]pyrimidin-4-amine dihydrochloride (100 mg) was dissolved in N,N-dimethylformamide (10 mL), 3-(trifluoromethyl)benzoyl chloride (0.2 mL) and triethylamine (0.2 mL) were added at 0°C, and the mixture was stirred for 30 min. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (39 mg) as a white powder.
$^1$H-NMR (DMSO-$d_6$) δ: 3.59 (2H, t, J = 5.9 Hz), 4.71 (2H, t, J = 5.9 Hz), 6.52 (1H, d, J = 3.0 Hz), 6.61 (1H, d, J = 8.0 Hz), 7.11 (1H, d, J = 8.9 Hz), 7.33 (1H, t, J = 8.0 Hz), 7.53 (1H, dd, J = 0.8, 5.5 Hz), 7.59-7.84 (5H, m), 7.86-8.07 (4H, m), 8.33 (1H, s), 8.67 (1H, s), 8.92 (1H, t, J = 5.6 Hz).

Example 118

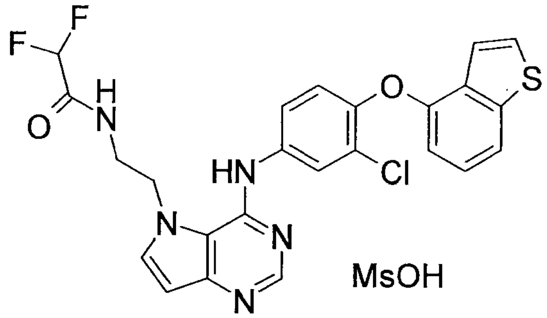

Production of N-[2-(4-{[4-(1-benzothiophen-4-yloxy)-3-chlorophenyl]amino}-5H-pyrrolo[3,2-d]pyrimidin-5-yl)ethyl]-2,2-difluoroacetamide methanesulfonate

N-[2-(4-{[4-(1-Benzothiophen-4-yloxy)-3-chlorophenyl]amino}-5H-pyrrolo[3,2-d]pyrimidin-5-yl)ethyl]-2,2-difluoroacetamide (150 mg) was dissolved in ethyl acetate (10 mL), and methanesulfonic acid (30 mg) was added. Diethyl ether (1 mL) was added, and the precipitated crystals were collected by filtration to give the title compound (160 mg) as a white powder. $^1$H-NMR (DMSO-$d_6$) δ: 2.34 (3H, s), 3.54-3.60 (2H, m), 4.67-4.84 (2H, m), 6.14 (1H, t, J = 53.4 Hz), 6.69 (1H, d, J = 2.8 Hz), 6.79 (1H, d, J = 7.7 Hz), 7.19 (1H, d, J = 8.9 Hz), 7.32-7.62 (3H, m), 7.79-7.98 (4H, m), 8.69-8.78 (1H, m), 8.93 (1H, t, J = 5.6 Hz), 9.78 (1H, br s).

Example 119

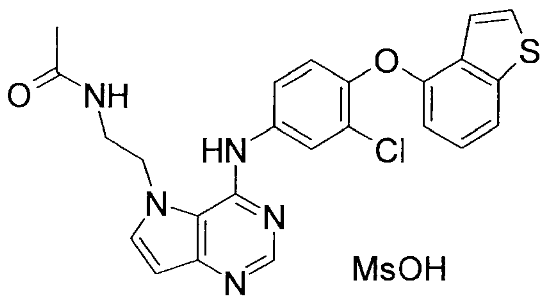

Production of N-[2-(4-{[4-(1-benzothiophen-4-yloxy)-3-chlorophenyl]amino}-5H-pyrrolo[3,2-d]pyrimidin-5-yl)ethyl]acetamide methanesulfonate

N-[2-(4-{[4-(1-Benzothiophen-4-yloxy)-3-chlorophenyl]amino}-5H-pyrrolo[3,2-d]pyrimidin-5-yl)ethyl]acetamide (50 mg) was dissolved in ethyl acetate (10 mL), and methanesulfonic acid (10 mg) was added. Diethyl ether (2 mL) was added, and the precipitated crystals were collected by filtration to give the title compound (40 mg) as a white powder.

$^1$H-NMR (DMSO-$d_6$) δ: 1.81 (3H, s), 2.31 (3H, s), 3.38-3.46 (2H, m), 4.61 (2H, t, J = 7.0 Hz), 6.67 (1H, d, J = 3.0 Hz), 6.71-6.83 (1H, m), 7.17 (1H, d, J = 8.9 Hz), 7.28-7.51 (2H, m), 7.59 (1H, dd, J = 2.8, 8.9 Hz), 7.75-7.88 (2H, m), 7.95 (2H, dd, J = 2.8, 13.2 Hz), 8.39 (1H, t, J = 5.6 Hz), 8.72 (1H, s), 10.10 (1H, br s).

Example 120

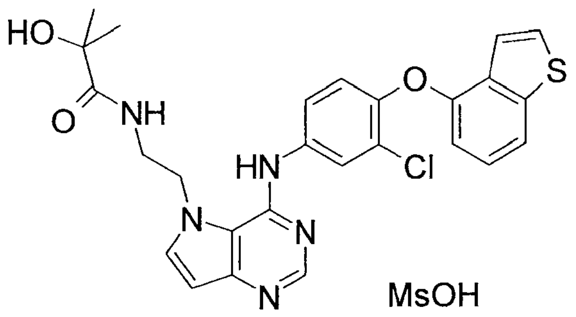

Production of N-[2-(4-{[4-(1-benzothiophen-4-yloxy)-3-chlorophenyl]amino}-5H-pyrrolo[3,2-d]pyrimidin-5-yl)ethyl]-2-hydroxy-2-methylpropanamide methanesulfonate

N-[2-(4-{[4-(1-Benzothiophen-4-yloxy)-3-chlorophenyl]amino}-5H-pyrrolo[3,2-d]pyrimidin-5-yl)ethyl]acetamide (700 mg) was dissolved in ethanol (5 mL), and methanesulfonic acid (130 mg) was added. Ethyl acetate (5 mL) was added, and the precipitated crystals were collected by filtration to give the title compound (750 mg) as a white powder. $^1$H-NMR (DMSO-$d_6$) δ: 1.12 (6H, s), 2.31 (3H, s), 3.47-3.57 (2H, m), 4.66 (2H, t, J = 6.3 Hz), 6.64 (1H, d, J = 3.2 Hz), 6.79 (1H, d, J = 7.2 Hz), 7.19 (1H, d, J = 8.9 Hz), 7.33-7.51 (2H, m), 7.60 (1H, dd, J = 2.5, 8.9 Hz), 7.72-8.00 (4H, m), 8.06 (1H, t, J = 5.9 Hz), 8.71 (1H, s), 9.90 (1H, br s).

Example 121

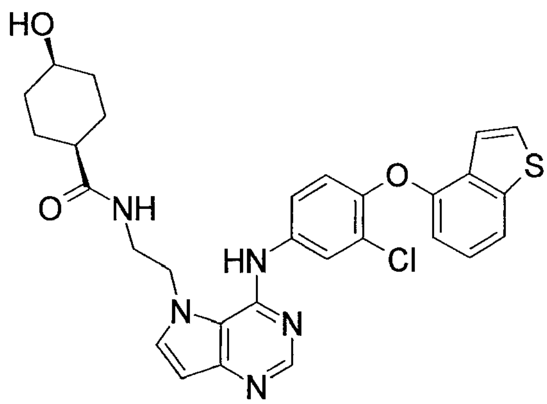

Production of cis-N-[2-(4-{[4-(1-benzothiophen-4-yloxy)-3-chlorophenyl]amino}-5H-pyrrolo[3,2-d]pyrimidin-5-yl)ethyl]-4-hydroxycyclohexanecarboxamide

The title compound (120 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (140 mg), cis-4-hydroxycyclohexanecarboxylic acid (70 mg), N,N-dimethylformamide (15 mL), triethylamine (1.2 mL), 1-hydroxybenzotriazole (20 mg) and 1-ethyl-3-(3-dimethylaminopropyl)carbodiimide hydrochloride (400 mg).
$^1$H-NMR (DMSO-$d_6$) δ: 1.21-1.46 (4H, m), 1.51-1.78 (4H, m), 1.95-2.13 (1H, m), 3.33-3.46 (2H, m), 3.65-3.74 (1H, m), 4.27 (1H, d, J = 3.0 Hz), 4.41-4.61 (2H, m), 6.49 (1H, d, J = 3.0 Hz), 6.65 (1H, d, J = 7.4 Hz), 7.18 (1H, d, J = 8.9 Hz), 7.32 (1H, t, J = 8.0 Hz), 7.51-7.58 (1H, m), 7.60 (1H, d, J = 3.0 Hz), 7.70-7.85 (3H, m), 7.95-8.10 (2H, m), 8.33 (1H, s), 8.83 (1H, s).

Example 122

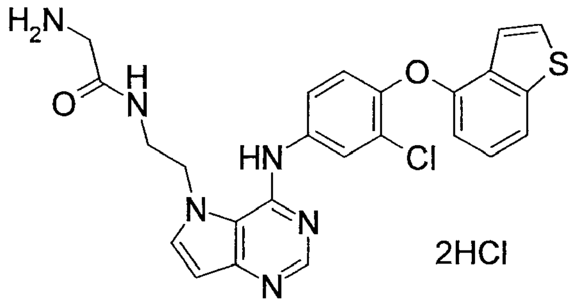

Production of N-[2-(4-{[4-(1-benzothiophen-4-yloxy)-3-chlorophenyl]amino}-5H-pyrrolo[3,2-d]pyrimidin-5-yl)ethyl]glycinamide dihydrochloride

The title compound (118 mg) was obtained as a yellow powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (200 mg), N-(tert-butoxycarbonyl)glycine (100 mg), N,N-dimethylformamide (15 mL), triethylamine (1.0 mL), 1-hydroxybenzotriazole (20 mg) and 1-ethyl-3-(3-dimethylaminopropyl)carbodiimide hydrochloride (400 g), and treatment with 4N hydrochloric acid/ethyl acetate (5 mL).
$^1$H-NMR (DMSO-$d_6$) δ: 3.43-3.48 (2H, m), 3.49-3.60 (2H, m), 4.76-4.86 (2H, m), 6.68 (1H, d, J = 3.2 Hz), 6.79 (1H, d, J = 7.2 Hz), 7.17 (1H, d, J = 8.9 Hz), 7.28-7.53 (2H, m), 7.61 (1H, dd, J = 2.5, 8.9 Hz), 7.76-7.88 (2H, m), 7.98 (2H, dd, J = 2.5, 9.3 Hz), 8.14 (3H, br s), 8.72 (1H, s), 8.83 (1H, t, J = 5.7 Hz), 10.10 (1H, br s).
Example 123
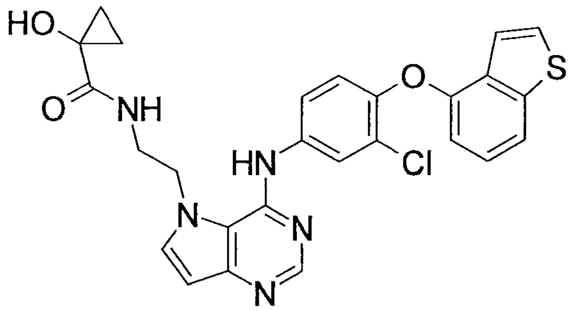

Production of N-[2-(4-{[4-(1-benzothiophen-4-yloxy)-3-chlorophenyl]amino}-5H-pyrrolo[3,2-d]pyrimidin-5-yl)ethyl]-1-hydroxycyclopropanecarboxamide

The title compound (105 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (200 mg), 1-hydroxycyclopropanecarboxylic acid (150 mg), N,N-dimethylformamide (15 mL), triethylamine (1.0 mL), 1-hydroxybenzotriazole (20 mg) and 1-ethyl-3-(3-dimethylaminopropyl)carbodiimide hydrochloride (400 mg).
$^1$H-NMR (DMSO-$d_6$) δ: 0.75-0.89 (2H, m), 0.92-1.05 (2H, m), 3.43-3.54 (2H, m), 4.55 (2H, t, J = 6.9 Hz), 6.27 (1H, s), 6.50 (1H, d, J = 3.0 Hz), 6.65 (1H, d, J = 7.9 Hz), 7.17 (1H, d, J = 8.9 Hz), 7.32 (1H, t, J = 7.9 Hz), 7.53 (1H, dd, J = 0.8, 5.5 Hz), 7.62 (1H, d, J = 3.0 Hz), 7.69-7.85 (3H, m), 8.02 (1H, d, J = 2.5 Hz), 8.25-8.41 (2H, m), 8.78 (1H, s).
Example 124
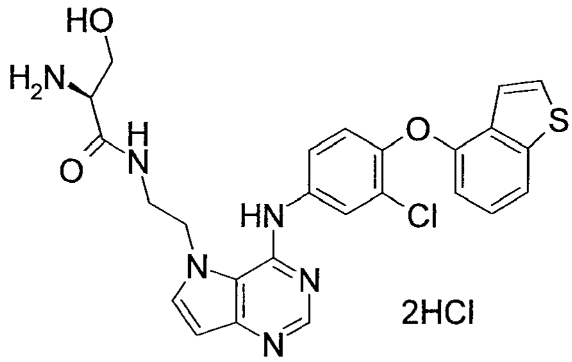

Production of N-[2-(4-{[4-(1-benzothiophen-4-yloxy)-3-chlorophenyl]amino}-5H-pyrrolo[3,2-d]pyrimidin-5-yl)ethyl]-L-serinamide dihydrochloride

The title compound (72 mg) was obtained as a pale-yellow powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (150 mg), N-(tert-butoxycarbonyl)serine (100 mg), N,N-dimethylformamide (15 mL), triethylamine (1.0 mL), 1-hydroxybenzotriazole (10 mg) and 1-ethyl-3-(3-dimethylaminopropyl)carbodiimide hydrochloride (350 mg), and treatment with 4N hydrochloric acid/ethyl acetate (5 mL).
$^1$H-NMR (DMSO-$d_6$) δ: 3.42-3.58 (2H, m), 3.58-3.84 (3H, m), 4.75 (2H, br s), 5.50 (1H, br s), 6.64 (1H, br s), 6.78 (1H, d, J = 7.9 Hz), 7.17 (1H, d, J = 8.7 Hz), 7.30-7.42 (1H, m), 7.47 (1H, d, J = 5.3 Hz), 7.51-7.65 (1H, m), 7.73-7.98 (4H, m), 8.13 (3H, br s), 8.72 (2H, br s), 9.86 (1H, br s).
Example 125
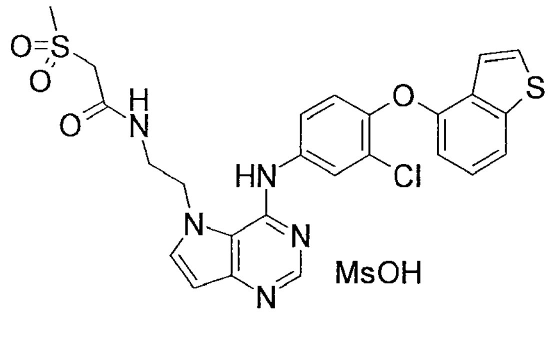

Production of N-[2-(4-{[4-(1-benzothiophen-4-yloxy)-3-chlorophenyl]amino}-5H-pyrrolo[3,2-d]pyrimidin-5-yl)ethyl]-2-(methylsulfonyl)acetamide methanesulfonate

The title compound (750 mg) was obtained as a pale-green powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (100 mg), (methylsulfonyl)acetic acid (50 mg), N,N-dimethylformamide (10 mL), triethylamine (4.5 mL), 1-hydroxybenzotriazole (100 mg) and 1-ethyl-3-(3-dimethylaminopropyl)carbodiimide hydrochloride (2.5 g), and treatment with methanesulfonic acid (150 mg).
$^1$H-NMR (DMSO-$d_6$) δ: 2.31 (3H, s), 3.06 (3H, s), 3.51-3.60 (2H, m), 4.06 (2H, s), 4.68 (2H, br s), 6.67 (1H, d, J = 3.0 Hz), 6.78 (1H, d, J = 7.7 Hz), 7.18 (1H, d, J = 8.7 Hz), 7.29-7.52 (2H, m), 7.59 (1H, dd, J = 2.5, 8.7 Hz), 7.76-7.88 (2H, m), 7.94 (2H, dd, J = 2.5, 6.1 Hz), 8.64-8.72 (1H, m), 8.75 (1H, s), 9.89 (1H, s).
Example 126
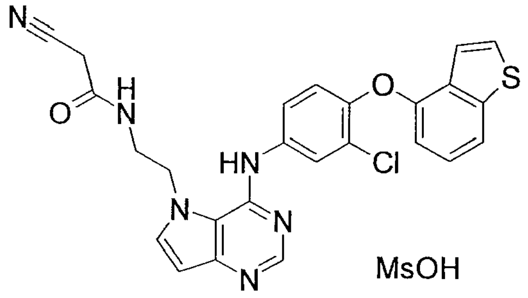

Production of N-[2-(4-{[4-(1-benzothiophen-4-yloxy)-3-chlorophenyl]amino}-5H-pyrrolo[3,2-d]pyrimidin-5-yl)ethyl]-2-cyanoacetamide methanesulfonate

The title compound (220 mg) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (300 mg), cyanoacetic acid (100 mg), N,N-dimethylformamide(50 mL), triethylamine(1.5 mL), 1-hydroxybenzotriazole(50 mg), 1-ethyl-3-(3-dimethylaminopropyl)carbodiimide hydrochloride (500 mg), and treatment with methanesulfonic acid (50 mg).
$^1$H-NMR (DMSO-$d_6$) δ: 2.33 (3H, s), 3.47-3.55 (2H, m), 3.57 (2H, s), 4.70 (2H, t, J = 6.1 Hz), 6.68 (1H, d, J = 3.0 Hz), 6.79 (1H, d, J = 7.4 Hz), 7.18 (1H, d, J = 8.9 Hz), 7.38 (1H, t, J = 8.0 Hz), 7.46 (1H, dd, J = 0.8, 5.5 Hz), 7.58 (1H, dd, J = 2.5, 8.9 Hz), 7.76-7.88 (2H, m), 7.89-8.01 (2H, m), 8.48 (1H, t, J = 5.5 Hz), 8.75 (1H, s), 9.80 (1H, br s).

Example 127

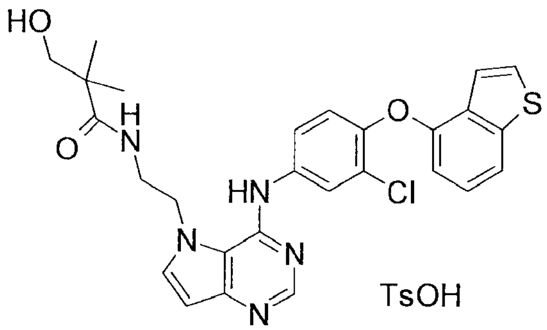

Production of N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide tosylate

N-[2-(4-{[4-(1-Benzothiophen-4-yloxy)-3-chlorophenyl]amino}-5H-pyrrolo[3,2-d]pyrimidin-5-yl)ethyl]-3-hydroxy-2,2-dimethylpropanamide (750 mg) was dissolved in ethanol (30 mL), and tosylic acid monohydrate (280 mg) was added. Ethyl acetate (5 mL) was added, and precipitated crystals were collected by filtration to give the title compound (810 mg) as a white powder. $^1$H-NMR (DMSO-$d_6$) δ: 0.96 (6H, s), 2.28 (3H, s), 3.30 (2H, s), 3.43-3.55 (2H, m), 4.55-4.70 (2H, m), 6.65 (1H, d, J = 3.0 Hz), 6.78 (1H, d, J = 7.4 Hz), 7.11 (2H, d, J = 7.7 Hz), 7.19 (1H, d, J = 8.9 Hz), 7.38 (1H, t, J = 7.7 Hz), 7.45-7.54 (3H, m), 7.66 (1H, dd, J = 2.5, 8.9 Hz), 7.73-8.07 (5H, m), 8.74 (1H, s), 10.11 (1H, br s).

Example 128

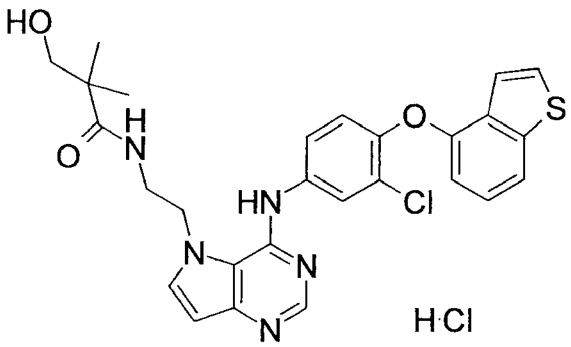

Production of N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide hydrochloride

N-[2-(4-{[4-(1-Benzothiophen-4-yloxy)-3-chlorophenyl]amino}-5H-pyrrolo[3,2-d]pyrimidin-5-yl)ethyl]-3-hydroxy-2,2-dimethylpropanamide (500 mg) was dissolved in ethanol (15 mL), and 4N hydrochloric acid/ethyl acetate (20 mL) was added. Ethyl acetate (5 mL) was added, and precipitated crystals were collected by filtration to give the title compound (495 mg) as a white powder.
$^1$H-NMR (DMSO-$d_6$) δ: 0.95 (6H, s), 3.29 (2H, s), 3.44-3.54 (2H, m), 4.66 (2H, t, J = 6.3 Hz), 6.65 (1H, d, J = 3.2 Hz), 6.78 (1H, d, J = 7.7 Hz), 7.19 (1H, d, J = 8.9 Hz), 7.38 (1H, t, J = 7.7 Hz), 7.47 (1H, d, J = 5.5 Hz), 7.68 (1H, dd, J = 2.6, 8.9 Hz), 7.73-7.95 (4H, m), 8.00 (1H, d, J = 2.6 Hz), 8.72 (1H, s), 10.18 (1H, br s).
Example 129
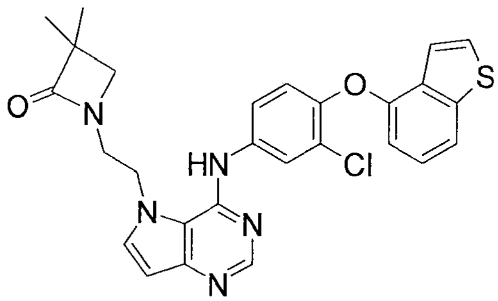

Production of 1-[2-(4-{[4-(1-benzothiophen-4-yloxy)-3-chlorophenyl]amino}-5H-pyrrolo[3,2-d]pyrimidin-5-yl)ethyl]-3,3-dimethylazetidin-2-one

The reaction in the same manner as in Example 90 (ii) was performed using 5-(2-aminoethyl)-N-[4-(1-benzothiophen-4-yloxy)-3-chlorophenyl]-5H-pyrrolo[3,2-d]pyrimidin-4-amine dihydrochloride (200 mg), 3-bromo-2,2-dimethylpropanoic acid (150 mg), N,N-dimethylformamide (15 mL), triethylamine (1.0 mL), 1-hydroxybenzotriazole (20 mg) and 1-ethyl-3-(3-dimethylaminopropyl)carbodiimide hydrochloride (400 mg). The obtained compound was dissolved in ethanol (100 mL), sodium methanethiolate (50 mg) was added, and the mixture was stirred for 1 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (81 mg) as a white powder.
$^1$H-NMR (DMSO-$d_6$) δ: 0.99 (6H, s), 2.70 (2H, s), 3.42-3.53 (2H, m), 4.65 (2H, t, J = 5.7 Hz), 6.47-6.58 (1H, m), 6.65 (1H, d, J = 7.4 Hz), 7.18 (1H, d, J = 8.7 Hz), 7.33 (1H, t, J = 8.0 Hz), 7.47-7.65 (2H, m), 7.65-7.82 (3H, m), 7.91 (1H, br s), 8.34 (1H, s), 8.70 (1H, s).
Example 130
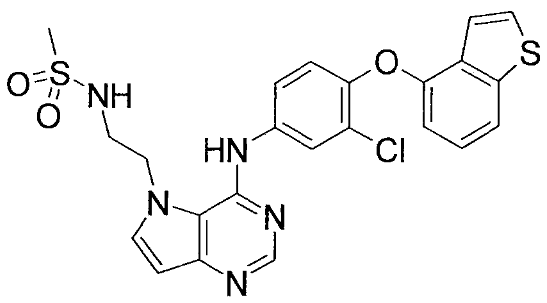

Production of N-[2-(4-{[4-(1-benzothiophen-4-yloxy)-3-chlorophenyl]amino}-5H-pyrrolo[3,2-d]pyrimidin-5-yl)ethyl]methanesulfonamide

5-(2-Aminoethyl)-N-[4-(1-benzothiophen-4-yloxy)-3-chlorophenyl]-5H-pyrrolo[3,2-d]pyrimidin-4-amine dihydrochloride (200 mg) was dissolved in tetrahydrofuran (20 mL), methanesulfonyl chloride (0.2 mL) and pyridine (1.0 mL) were added at 0°C, and the mixture was stirred for 5 min. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (110 mg) as a white powder.
$^1$H-NMR (DMSO-$d_6$) δ: 2.80 (3H, s), 3.34-3.40 (2H, m), 4.60 (2H, t, J = 6.0 Hz), 6.52 (1H, d, J = 2.8 Hz), 6.65 (1H, d, J = 7.4 Hz), 7.18 (1H, d, J = 8.9 Hz), 7.33 (1H, t, J = 7.9 Hz), 7.47-7.71 (3H, m), 7.71-7.85 (2H, m), 7.92 (1H, br s), 8.34 (1H, br s), 8.56 (1H, br s).
Example 131
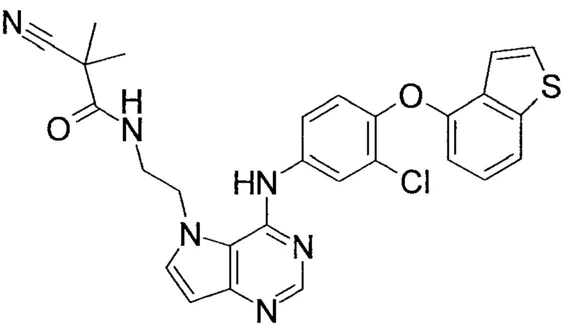

Production of N-[2-(4-{[4-(1-benzothiophen-4-yloxy)-3-chlorophenyl]amino}-5H-pyrrolo[3,2-d]pyrimidin-5-yl)ethyl]-2-cyano-2-methylpropanamide

The title compound (562 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (900 mg), 2-cyano-2-methylpropanoic acid (2.0 g), N,N-dimethylformamide (200 mL), triethylamine (4.0 mL), 1-hydroxybenzotriazole (200 mg) and 1-ethyl-3-(3-dimethylaminopropyl)carbodiimide hydrochloride (2.5 g).
$^1$H-NMR (DMSO-$d_6$) δ: 1.38 (6H, s), 3.40-3.52 (2H, m), 4.61 (2H, t, J = 5.9 Hz), 6.49 (1H, d, J = 3.0 Hz), 6.66 (1H, d, J = 7.4 Hz), 7.19 (1H, d, J = 8.9 Hz), 7.32 (1H, t, J = 8.0 Hz), 7.49-7.60 (2H, m), 7.68 (1H, dd, J = 2.2 Hz, 8.9 Hz), 7.73-7.84 (2H, m), 7.99 (1H, d, J = 2.2 Hz), 8.27 (1H, br s), 8.35 (1H, s), 8.62 (1H, s).
Example 132
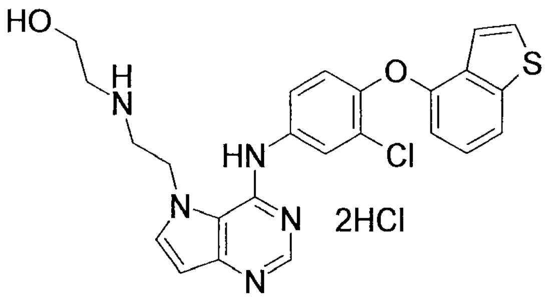

Production of 2-{[2-(4-{[4-(1-benzothiophen-4-yloxy)-3-chlorophenyl]amino}-5H-pyrrolo[3,2-d]pyrimidin-5-yl)ethyl]amino}ethanol dihydrochloride

5-(2-Aminoethyl)-N-[4-(1-benzothiophen-4-yloxy)-3-chlorophenyl]-5H-pyrrolo[3,2-d]pyrimidin-4-amine dihydrochloride (500 mg) was dissolved in N,N-dimethylformamide (40 mL), triethylamine (1.0 mL) was added and the mixture was stirred for 10 min. Hydroxyacetoaldehyde (60 mg), acetic acid (4.0 mL) and molecular sieves 4A (500 mg) were added to the reaction mixture, and the mixture was stirred for 1 hr. Sodium triacetoxyborohydride (220 mg) was added to the reaction mixture, and the mixture was stirred for 1 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100), and treated with 4N hydrochloric acid/ethyl acetate (5 mL) to give the title compound (115 mg) as a brown powder.
$^1$H-NMR (DMSO-$d_6$) δ: 2.96-3.22 (4H, m), 3.39-3.71 (4H, m), 5.01-5.12 (1H, m), 6.69-6.81 (2H, m), 7.17 (1H, d, J = 8.9 Hz), 7.38 (1H, t, J = 7.9 Hz), 7.47 (1H, d, J = 0.8 Hz), 7.50-7.63 (1H, m), 7.70-7.94 (3H, m), 7.95 (1H, s), 8.63-8.75 (1H, m), 9.07 (1H, br s), 9.88 (1H, br s).

Example 133

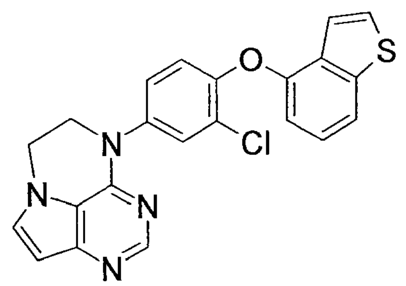

Production of 4-[4-(1-benzothiophen-4-yloxy)-3-chlorophenyl]-5,6-dihydro-4H-pyrrolo[3,2,1-de]pteridine

(i) Production of 4-chloro-5-(2-chloroethyl)-5H-pyrrolo[3,2-d]pyrimidine

To a solution of 4-chloro-5H-pyrrolo[3,2-d]pyrimidine (1.0 g), 1-bromo-2-chloroethane (930 mg) in N,N-dimethylformamide (25 mL) was added potassium carbonate (1.5 g), and the mixture was stirred at room temperature for 2 hr. Saturated brine was added to the reaction system under ice-cooling, and the mixture was extracted twice with ethyl acetate. The organic layer was dried over anhydrous magnesium sulfate and concentrated under reduced pressure. The residue was separated and purified by silica gel column chromatography (eluent, hexane:ethyl acetate=1:0→1:1) to give the title compound (806 mg) as yellow crystals.
$^1$H-NMR (DMSO-$d_6$) δ: 3.97-4.06 (2H, m), 4.65-4.76 (2H, m), 6.51 (1H, d, J = 3.0 Hz), 7.72 (1H, d, J = 3.0 Hz), 8.74 (1H, s).

(ii) Production of 4-[4-(1-benzothiophen-4-yloxy)-3-chlorophenyl]-5,6-dihydro-4H-pyrrolo[3,2,1-de]pteridine

A mixture of 4-chloro-5-(2-chloroethyl)-5H-pyrrolo[3,2-d]pyrimidine (150 mg), 4-(1-benzothiophen-4-yloxy)-3-chloroaniline (270 mg) and isopropyl alcohol (5 mL) was stirred at 80°C for 2 hr. 1-Methyl-2-pyrrolidone (5 mL) and

potassium carbonate (100 mg) were added to the reaction mixture, and the mixture was stirred at 100°C for 2 hr. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to silica gel column chromatography (eluent, hexane:ethyl acetate=50:50→0:100). The object fraction was concentrated under reduced pressure to give the title compound (46 mg) as yellow crystals.
$^1$H-NMR (DMSO-$d_6$) δ: 4.22-4.29 (2H, m), 4.37-4.49 (2H, m), 6.49 (1H, d, J = 2.8 Hz), 6.74 (1H, d, J = 7.2 Hz), 7.19 (1H, d, J = 8.9 Hz), 7.36 (1H, t, J = 8.0 Hz), 7.46-7.53 (1H, m), 7.56 (1H, dd, J = 2.6 Hz, 8.9 Hz), 7.64 (1H, d, J = 2.8 Hz), 7.78-7.85 (2H, m), 7.88 (1H, d, J = 2.6 Hz), 8.29 (1H, s).

Example 134

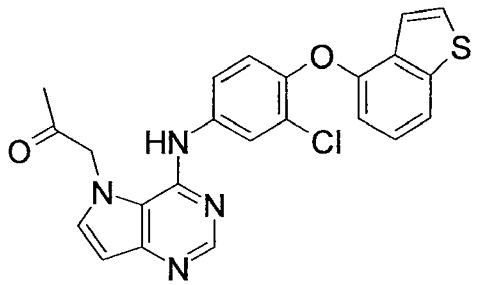

Production of 1-(4-{[4-(1-benzothiophen-4-yloxy)-3-chlorophenyl]amino}-5H-pyrrolo[3,2-d]pyrimidin-5-yl)acetone

(i) Production of 1-(4-chloro-5H-pyrrolo[3,2-d]pyrimidin-5-yl)acetone

To a solution of 4-chloro-5H-pyrrolo[3,2-d]pyrimidine (1.0 g), 1-bromoacetone (890 mg) in N,N-dimethylformamide (25 mL) was added potassium carbonate (1.5 g), and the mixture was stirred at room temperature for 2 hr. Saturated brine was added to the reaction system under ice-cooling, and the mixture was extracted twice with ethyl acetate. The organic layer was dried over anhydrous magnesium sulfate and concentrated under reduced pressure. The residue was separated and purified by silica gel column chromatography (eluent, hexane:ethyl acetate=1:0→1:1) to give the title compound (406 mg) as yellow crystals.
$^1$H-NMR (DMSO-$d_6$) δ: 2.15 (3H, s), 5.30 (2H, br s), 6.51-6.68 (1H, m), 7.78 (1H, d, J = 3.0 Hz), 8.72 (1H, s).

(ii) Production of 1-(4-{[4-(1-benzothiophen-4-yloxy)-3-chlorophenyl]amino}-5H-pyrrolo[3,2-d]pyrimidin-5-yl)acetone

A mixture of 1-(4-chloro-5H-pyrrolo[3,2-d]pyrimidin-5-yl)acetone (100 mg) and 4-(1-benzothiophen-4-yloxy)-3-chloroaniline (132 mg) was dissolved in isopropyl alcohol (10 mL), and the solution was stirred at 80°C for 2.5 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (hexane:ethyl acetate=50:50→0:100) to give the title compound (65 mg) as a yellow powder.
$^1$H-NMR (DMSO-$d_6$) δ: 2.14 (3H, s), 5.56 (2H, s), 6.55 (1H, br s), 6.64 (1H, d, J = 7.9 Hz), 7.15 (1H, d, J = 8.7 Hz), 7.32 (1H, t, J = 7.9 Hz), 7.44-7.60 (3H, m), 7.70-7.86 (3H, m), 8.36 (1H, s), 8.50 (1H, s).

Example 135

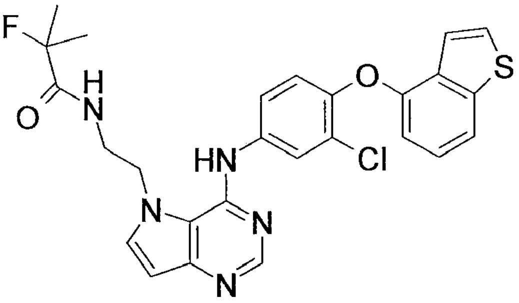

Production of N-[2-(4-{[4-(1-benzothiophen-4-yloxy)-3-chlorophenyl]amino}-5H-pyrrolo[3,2-d]pyrimidin-5-yl)ethyl]-2-fluoro-2-methylpropanamide

The title compound (65 mg) was obtained as a white powder by reaction in the same manner as in Example 90(ii) and using 5-(2-aminoethyl)-N-[4-(1-benzothiophen-4-yloxy)-3-chlorophenyl]-5H-pyrrolo[3,2-d]pyrimidin-4-amine dihydrochloride (100 mg), 2-fluoro-2-methylpropanoic acid (50 mg), N,N-dimethylformamide (10 mL), triethylamine (1.0 mL), 1-hydroxybenzotriazole (10 mg) and 1-ethyl-3-(3-dimethylaminopropyl)carbodiimide hydrochloride (200 mg).
$^1$H-NMR (DMSO-$d_6$) δ: 1.29 (3H, s), 1.37 (3H, s), 3.42-3.53 (2H, m), 4.60 (2H, br s), 6.48 (1H, d, J = 2.5 Hz), 6.65 (1H, d, J = 7.7 Hz), 7.19 (1H, d, J = 8.9 Hz), 7.33 (1H, t, J = 7.9 Hz), 7.48-7.59 (2H, m), 7.62-7.71 (1H, m), 7.71-7.84 (2H, m), 7.97 (1H, s), 8.18-8.28 (1H, m), 8.32 (1H, s), 8.63 (1H, s).

Example 136

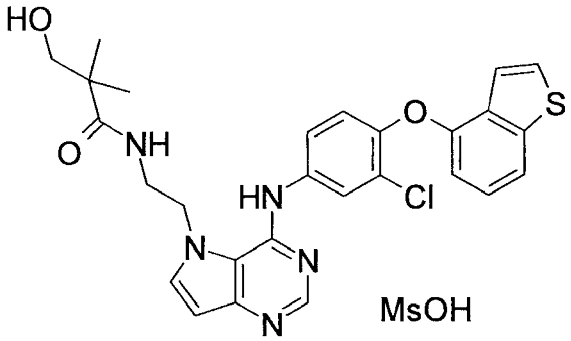

Production of N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide methanesulfonate

N-[2-(4-{[4-(1-Benzothiophen-4-yloxy)-3-chlorophenyl]amino}-5H-pyrrolo[3,2-d]pyrimidin-5-yl)ethyl]-3-hydroxy-2,2-dimethylpropanamide (1.0 g) was dissolved in ethanol (20 mL) and methanesulfonic acid (185 mg) was added. Ethyl acetate (5 mL) was added, and precipitated crystals were collected by filtration to give the title compound (920 mg) as a white powder.
$^1$H-NMR (DMSO-$d_6$) δ: 0.96 (6H, s), 2.32 (3H, s), 3.30 (2H, s), 3.42-3.54 (2H, m), 4.63 (2H, t, J = 6.6 Hz), 6.66 (1H, d, J = 3.0 Hz), 6.78 (1H, d, J = 7.2 Hz), 7.19 (1H, d, J = 8.8 Hz), 7.38 (1H, t, J = 7.9 Hz), 7.46 (1H, dd, J = 0.8 Hz, 5.5 Hz), 7.66 (1H, dd, J = 2.5 Hz, 8.8 Hz), 7.78-8.04 (5H, m), 8.75 (1H, s), 10.14 (1H, s).

Example 137

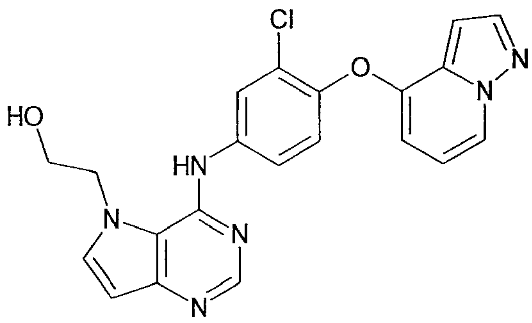

Production of 2-(4-{[3-chloro-4-(pyrazolo[1,5-a]pyridin-4-yloxy)phenyl]amino}-5H-pyrrolo[3,2-d]pyrimidin-5-yl) ethanol

(i) Production of 4-(2-chloro-4-nitrophenoxy)pyrazolo[1,5-a]pyridine

Pyrazolo[1,5-a]pyridin-4-ol (557 mg) was dissolved in N,N-dimethylformamide (10 mL), potassium carbonate (574 mg) and 2-chloro-1-fluoro-4-nitrobenzene (728 mg) were added, and the mixture was stirred at room temperature for 6 hr and then heated to 50°C. Two hours later, the mixture was partitioned between ethyl acetate (80 mL) and water (80 mL), and the organic layer was dried over anhydrous magnesium sulfate and concentrated under reduced pressure. The residue was separated and purified by silica gel column chromatography (hexane:ethyl acetate=95:5→40:60) to give the title compound (995 mg) as a pale-yellow oil.
$^1$H-NMR ($CDCl_3$) δ: 6.46 (1H, d, J= 3.3 Hz), 6.72-6.79 (2H, m), 6.99 (1H, d, J= 9.3 Hz), 7.96 (1H, d, J= 2.1 Hz), 8.08 (1H, dd, J= 2.7 Hz, 9.3 Hz), 8.41-8.44 (2H, m).

(ii) Production of 3-chloro-4-(pyrazolo[1,5-a]pyridin-4-yloxy)aniline

A mixture of 4-(2-chloro-4-nitrophenoxy)pyrazolo[1,5-a]pyridine (995 mg), reduced iron (559 mg), calcium chloride (222 mg), water (5 mL) and ethanol (10 mL) was heated to 90°C and stirred for 2 hr. The reaction mixture was cooled to room temperature and filtered through celite. The celite was washed with ethanol (100 mL). The filtrate was concentrated under reduced pressure, the residue was partitioned between ethyl acetate and water, and the organic layer was dried over anhydrous magnesium sulfate and concentrated under reduced pressure. The residue was separated and purified by silica gel column chromatography (hexane:ethyl acetate=97:3→30:70) to give the title compound (995 mg) as a pale-yellow oil.
$^1$H-NMR ($CDCl_3$)δ: 3.73 (2H, br s), 6.17 (1H, dd, J= 0.6 Hz, 7.5 Hz), 6.55-6.62 (2H, m), 6.72 (1H, dd, J= 0.9 Hz, 2.4 Hz), 6.81 (1H, d, J= 2.7 Hz), 6.99 (1H, d, J= 8.7 Hz).

(iii) Production of 2-(4-{[3-chloro-4-(pyrazolo[1,5-a]pyridin-4-yloxy)phenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (151 mg), 3-chloro-4-(pyrazolo[1,5-a]pyridin-4-yloxy)aniline (130 mg), isopropyl alcohol (5 mL) and pyridine hydrochloride (5 mg) was stirred at 80°C overnight. The reaction mixture was cooled to room temperature, and poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=90:10→0:100) to give a coupling compound (229 mg). Tetrahydrofuran (1 mL), methanol (1 mL) and 1N aqueous sodium hydroxide solution (1 mL) were added to the obtained coupling compound and the mixture was stirred for 15 min. The reaction mixture was poured into saturated aqueous sodium hydrogen carbonate solution, and the mixture was extracted with ethyl acetate. The extract was dried over anhydrous magnesium sulfate, and concentrated under reduced pressure, and the residue was crystallized from hexane/ethyl acetate to give the title compound (157 mg) as white crystals.
$^1$H-NMR ($CDCl_3$) δ: 1.26 (1H, br s), 4.17-4.19 (2H, m), 4.43 (2H, t, J= 4.5 Hz), 6.24 (1H, d, J= 2.7 Hz), 6.32 (1H, d, J= 7.2 Hz), 6.62 (1H, t, J= 7.2 Hz), 6.74 (1H, dd, J= 0.9 Hz, 2.4 Hz), 7.03 (1H, d, J= 3.0 Hz), 7.16 (1H, d, J= 9.0 Hz), 7.53 (1H, dd, J= 2.7 Hz, 8.7 Hz), 7.86 (1H, d, J= 2.7 Hz), 7.96 (1H, d, J= 2.4 Hz), 8.24 (1H, d, J= 7.2 Hz), 8.31 (1H, s), 9.51 (1H, s).
Example 138

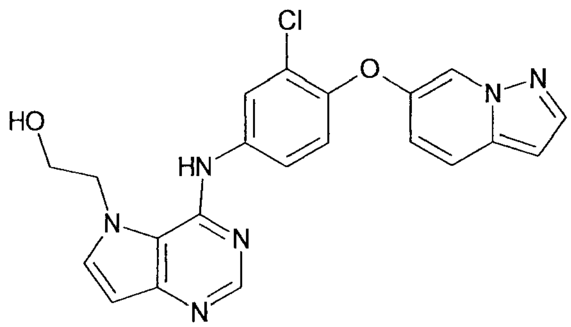

Production of 2-(4-{[3-chloro-4-(pyrazolo[1,5-a]pyridin-6-yloxy)phenyl]amino}-5H-pyrrolo[3,2-d]pyrimidin-5-yl) ethanol

(i) Production of 6-(2-chloro-4-nitrophenoxy)pyrazolo[1,5-a]pyridine

The title compound (1.55 g) was obtained as a pale-yellow solid by operation in the same manner as in Example 137 (i) and using pyrazolo[1,5-a]pyridin-6-ol (840 mg), N,N-dimethylformamide (12 mL), potassium carbonate (865 mg) and 2-chloro-1-fluoro-4-nitrobenzene (1.1 g).
$^1$H-NMR ($CDCl_3$) δ: 6.62-6.64 (1H, m), 6.95-7.00 (2H, m), 7.63 (1H, d, J= 9.0 Hz), 8.01 (1H, d, J= 2.1 Hz), 8.08 (1H, dd, J= 2.7 Hz, 9.3 Hz), 8.41-8.45 (2H, m).

(ii) Production of 3-chloro-4-(pyrazolo[1,5-a]pyridin-6-yloxy)aniline

The title compound (1.2 g) was obtained as a pale-yellow solid by operation in the same manner as in Example 137 (ii) and using 6-(2-chloro-4-nitrophenoxy)pyrazolo[1,5-a]pyridine (1.53 g), reduced iron (886 mg), calcium chloride (353 mg), water (7.5 mL) and ethanol (15 mL).
$^1$H-NMR ($CDCl_3$)δ: 3.70 (2H, br s), 6.49 (1H, d, J= 2.1 Hz), 6.57 (1H, dd, J= 3.0 Hz, J= 8.7 Hz), 6.80 (1H, d, J= 2.4 Hz), 6.93 (1H, d, J= 8.7 Hz), 7.04 (1H, dd, J= 2.4 Hz, 9.9 Hz), 7.48 (1H, d, J= 9.6 Hz), 7.86 (1H, d, J= 2.4 Hz), 8.00-8.01 (1H, m).

(iii) Production of 2-(4-{[3-chloro-4-(pyrazolo[1,5-a]pyridin-6-yloxy)phenyl]amino}-5H-pyrrolo[3,2-d]pyrimidin-5-yl)ethanol

The title compound (156 mg) was obtained as white crystals in the same manner as in Example 137 (iii) and using 3-chloro-4-(pyrazolo[1,5-a]pyridin-6-yloxy)aniline (130 mg), 2-(4-chloro-5H-pyrrolo[3,2-d]pyrimidin-5-yl)ethyl benzoate (151 mg), isopropyl alcohol (5 mL) and pyridine hydrochloride (5 mg).
$^1$H-NMR ($CDCl_3$) δ: 1.25 (1H, br s), 4.16-4.18 (2H, m), 4.41 (2H, t, J= 4.8 Hz), 6.21 (1H, d, J= 3.0 Hz), 6.53-6.64 (1H, m), 7.02-7.11 (3H, m), 7.46-7.54 (2H, m), 7.81 (1H, d, J= 2.4 Hz), 7.90 (1H, d, J= 2.1 Hz), 8.13-8.14 (1H, m), 8.29 (1H, s), 9.50 (1H, br s).
Example 139
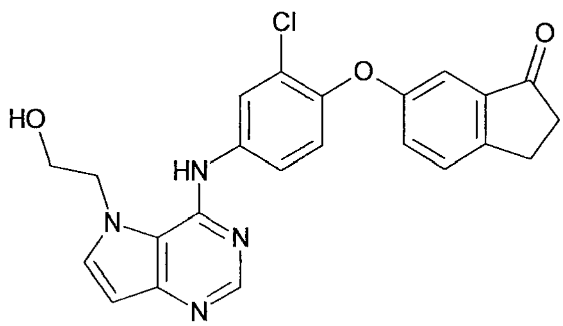

Production of 6-(2-chloro-4-{[5-(2-hydroxyethyl)-5H-pyrrolo[3,2-d]pyrimidin-4-yl]amino}phenoxy)indan-1-one

(i) Production of 6-(2-chloro-4-nitrophenoxy)indan-1-one

A mixture of 6-hydroxyindan-1-one (5.0 g), N,N-dimethylformamide (60 mL), potassium carbonate (4.66 g) and 2-chloro-1-fluoro-4-nitrobenzene (5.92 g) was stirred at room temperature for 60 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was washed with ethyl acetate-hexane to give the title compound (8.45 g) as a white solid.
$^1$H-NMR ($CDCl_3$) δ: 2.76-2.80 (2H, m), 3.16-3.20 (2H, m), 6.94 (1H, d, J= 9.0 Hz), 7.35-7.38 (2H, m), 7.55-7.58 (1H, m), 8.08 (1H, dd, J= 2.7 Hz, 9.3 Hz), 8.40 (1H, d, J= 2.4 Hz).

(ii) Production of 6-(4-amino-2-chlorophenoxy)indan-1-one

A mixture of 6-(2-chloro-4-nitrophenoxy)indan-1-one (8.44 g), reduced iron (4.66 g), calcium chloride (1.85 g), water (37.5 mL) and ethanol (75 mL) was heated to 90°C, and the mixture was stirred for 5 hr. The reaction mixture was cooled to room temperature and filtered through celite. The celite was successively washed with ethanol (150 mL) and ethyl acetate (150 mL). The filtrate was concentrated under reduced pressure, and the residue was washed with ethyl acetate to give the title compound (5.27 g) as a pale-yellow solid.
$^1$H-NMR ($CDCl_3$)δ: 2.68-2.72 (2H, m), 3.07-3.11 (2H, m), 3.70 (2H, br s), 6.58 (1H, dd, J= 3.0 Hz, 8.7 Hz), 6.78 (1H, d, J= 3.0 Hz), 6.91 (1H, d, J= 8.7 Hz), 7.03 (1H, d, J= 2.7 Hz), 7.26-7.31 (1H, m), 7.40-7.43 (1H, m).

(iii) Production of 6-(2-chloro-4-{[5-(2-hydroxyethyl)-5H-pyrrolo[3,2-d]pyrimidin-4-yl]amino}phenoxy)indan-1-one

A mixture of 2-(4-chloro-5H-pyrrolo[3,2-d]pyrimidin-5-yl)ethyl benzoate (302 mg), 6-(4-amino-2-chlorophenoxy) indan-1-one (274 mg), isopropyl alcohol (10 mL) and pyridine hydrochloride (5 mg) was heated to 80°C and stirred overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a coupling compound. Tetrahydrofuran (2 mL), methanol (2 mL) and 1N aqueous sodium hydroxide solution (2 mL) were added to the obtained coupling compound and the mixture was stirred for 15 min. The reaction mixture was poured into water, and the mixture was extracted with ethyl acetate. The extract was dried over anhydrous magnesium sulfate and concentrated under reduced pressure, and the residue was crystallized from hexane/ethyl acetate to give the title compound (267 mg) as white crystals.
$^1$H-NMR ($CDCl_3$) δ: 2.72 (2H, t, J= 5.4 Hz), 3.12 (2H, t, J= 6.0 Hz), 4.16-4.18 (2H, m), 4.40-4.43 (2H, m), 6.22 (1H, d, J= 3.0 Hz), 7.02-7.12 (3H, m), 7.35-7.50 (3H, m), 7.80 (1H, d, J= 2.7 Hz), 8.29 (1H, s), 9.51 (1H, s).
Example 140
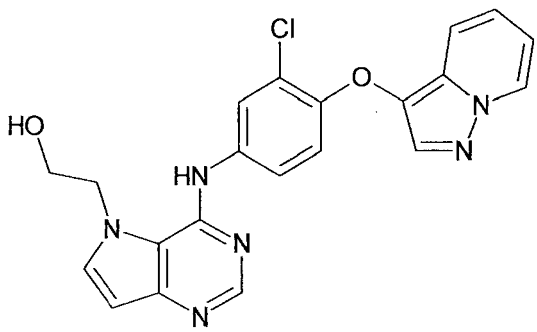

Production of 2-(4-{[3-chloro-4-(pyrazolo[1,5-a]pyridin-3-yloxy)phenyl]amino}-5H-pyrrolo[3,2-d]pyrimidin-5-yl) ethanol

(i) Production of 3-chloro-4-(pyrazolo[1,5-a]pyridin-3-yloxy)aniline

A mixture of pyrazolo[1,5-a]pyridin-3-ol (3.69 g), N,N-dimethylformamide (50 mL), potassium carbonate (3.80

g) and 2-chloro-1-fluoro-4-nitrobenzene (4.83 g) was stirred at room temperature overnight. The reaction mixture was poured into saturated aqueous sodium hydrogen carbonate solution, and the mixture was extracted with ethyl acetate. The extract was washed with saturated aqueous sodium hydrogen carbonate solution, dried over anhydrous magnesium sulfate, and concentrated under reduced pressure. Reduced iron (4.66 g), calcium chloride (1.85 g), water (37.5 mL) and ethanol (75 mL) were added to the residue, and the mixture was heated to 80°C and stirred for 16 hr. The reaction mixture was cooled to room temperature and filtered through celite. The celite was successively washed with ethanol and methanol. The filtrate was concentrated under reduced pressure, and the residue was partitioned between ethyl acetate and water. The organic layer was dried over anhydrous magnesium sulfate and concentrated under reduced pressure. The residue was separated and purified by silica gel column chromatography (hexane:ethyl acetate=95:5→25:75) to give the title compound (4.80 g) as an orange solid.
$^1$H-NMR ($CDCl_3$)δ: 3.58 (2H, s), 6.46 (1H, dd, J= 2.7 Hz, 8.7 Hz), 6.68-6.79 (3H, m), 6.98-7.04 (1H, m), 7.37-7.41 (1H, m), 7.77 (1H, s), 8.32 (1H, d, J= 7.2 Hz).

(ii) Production of 2-(4-{[3-chloro-4-(pyrazolo[1,5-a]pyridin-3-yloxy)phenyl]amino}-5H-pyrrolo[3,2-d]pyrimidin-5-yl)ethanol

The title compound (184 mg) was obtained as white crystals by operation in the same manner as in Example 139 (iii) and using 3-chloro-4-(pyrazolo[1,5-a]pyridin-3-yloxy)aniline (130 mg), 2-(4-chloro-5H-pyrrolo[3,2-d]pyrimidin-5-yl)ethyl benzoate (151 mg), isopropyl alcohol (5 mL) and pyridine hydrochloride (5 mg).
$^1$H-NMR ($CDCl_3$) δ: 4.09-4.16 (2H, m), 4.35-4.38 (2H, m), 6.15 (1H, d, J= 3.0 Hz), 6.75 (1H, t, J= 6.6 Hz), 6.87 (1H, d, J= 8.7 Hz), 6.96 (1H, d, J= 3.0 Hz), 7.06-7.09 (1H, m), 7.33 (1H, dd, J= 2.7 Hz, 9.0 Hz), 7.43-7.46 (1H, m), 7.76 (1H, d, J= 2.4 Hz), 7.85 (1H, s), 8.22 (1H, s), 8.36 (1H, d, J= 7.2 Hz), 9.36 (1H, s).
Example 141
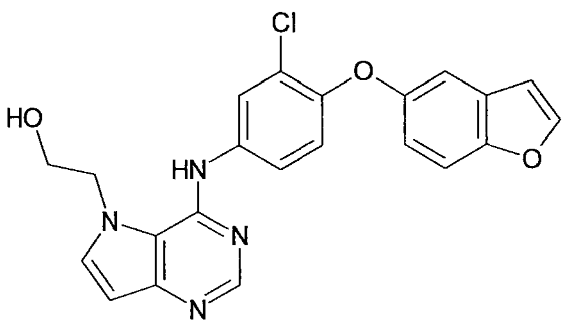

Production of 2-(4-{[4-(1-benzofuran-5-yloxy)-3-chlorophenyl]amino}-5H-pyrrolo[3,2-d]pyrimidin-5-yl)ethanol

(i) Production of 5-(2-chloro-4-nitrophenoxy)-1-benzofuran

1-Benzofuran-5-ol (766 mg) was dissolved in N,N-dimethylformamide (10 mL), potassium carbonate (789 mg) and 2-chloro-1-fluoro-4-nitrobenzene (1.0 g) were added, and the mixture was stirred at room temperature for 60 hr. The reaction mixture was partitioned between ethyl acetate and saturated aqueous sodium hydrogen carbonate solution, and the organic layer was dried over anhydrous magnesium sulfate and concentrated under reduced pressure. The residue was separated and purified by silica gel column chromatography (hexane:ethyl acetate=98:2→80:20) to give the title compound (1.56 g) as a white solid.
$^1$H-NMR ($CDCl_3$) δ: 6.78-6.82 (2H, m), 7.05 (1H, dd, J= 2.4 Hz, 8.7 Hz), 7.33 (1H, d, J= 2.4 Hz), 7.57 (1H, d, J= 8.7 Hz), 7.72 (1H, d, J= 2.4 Hz), 8.01 (1H, dd, J= 2.7 Hz, 9.0 Hz), 8.39 (1H, d, J= 3.0 Hz).

(ii) Production of 4-(1-benzofuran-5-yloxy)-3-chloroaniline

A mixture of 5-(2-chloro-4-nitrophenoxy)-1-benzofuran (1.64 g), reduced iron (949 mg), calcium chloride (377 mg), water (7.5 mL) and ethanol (15 mL) was heated to 80°C and stirred overnight. The mixture was cooled to room temperature and filtered through celite. The celite was successively washed with ethanol and ethyl acetate. The filtrate was concentrated under reduced pressure, residue was partitioned between ethyl acetate and water, and the organic layer was dried over anhydrous magnesium sulfate and concentrated under reduced pressure. The residue was separated

and purified by silica gel column chromatography (hexane:ethyl acetate=98:2→60:40) to give the title compound (1.05 g) as a pale-yellow solid.
$^1$H-NMR ($CDCl_3$)δ: 3.64 (2H, br s), 6.55 (1H, dd, J= 2.7 Hz, 8.7 Hz), 6.67 (1H, dd, J= 0.9 Hz, 2.1 Hz), 6.80 (1H, d, J= 2.7 Hz), 6.86 (1H, d, J= 8.7 Hz), 6.95 (1H, dd, J= 2.7 Hz, 9.0 Hz), 7.02 (1H, d, J= 2.4 Hz), 7.41 (1H, d, J= 9.0 Hz), 7.60 (1H, d, J= 2.4 Hz).

(iii) Production of 2-(4-{[4-(1-benzofuran-5-yloxy)-3-chlorophenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (151 mg), 4-(1-benzofuran-5-yloxy)-3-chloroaniline (130 mg), isopropyl alcohol (5 mL) and pyridine hydrochloride (5 mg) was stirred at 80°C overnight. The reaction mixture was cooled to room temperature, and poured into saturated aqueous sodium hydrogen carbonate solution, and the mixture was extracted with ethyl acetate. The extract was dried over anhydrous magnesium sulfate and concentrated under reduced pressure. The residue was crystallized from toluene, and washed with toluene-hexane. Tetrahydrofuran (1 mL), methanol (1 mL) and 1N aqueous sodium hydroxide solution (1 mL) were added to the obtained coupling compound and the mixture was stirred for 20 min. The reaction mixture was poured into saturated aqueous sodium hydrogen carbonate solution, and the mixture was extracted with ethyl acetate. The extract was dried over anhydrous magnesium sulfate and concentrated under reduced pressure, and the residue was crystallized from hexane/ethyl acetate to give the title compound (154 mg) as white crystals.
$^1$H-NMR (DMSO-$d_6$) δ: 3.85-3.89 (2H, m), 4.52-4.55 (2H, m), 6.29 (1H, br s), 6.51 (1H, d, J= 3.0 Hz), 6.92-6.93 (1H, m), 7.01 (1H, dd, J= 2.7 Hz, 9.0 Hz), 7.13-7.16 (2H, m), 7.53-7.66 (3H, m), 7.97-8.01 (2H, m), 8.33 (1H, s), 9.81 (1H, br s).
Example 142
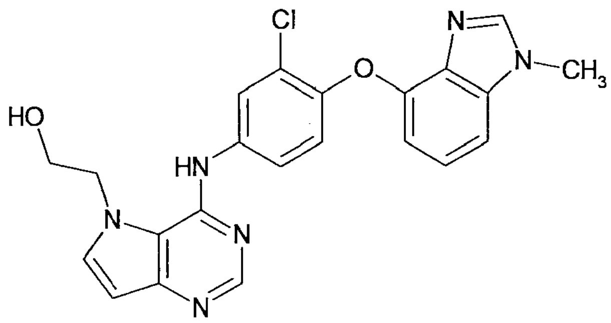

Production of 2-[4-({3-chloro-4-[(1-methyl-1H-benzimidazol-4-yl)oxy]phenyl}amino)-5H-pyrrolo[3,2-d]pyrimidin-5-yl]ethanol

(i) Production of 3-chloro-4-[(1-methyl-1H-benzimidazol-4-yl)oxy]aniline

A mixture of 1-methyl-1H-benzimidazol-4-ol (6.84 g), N,N-dimethylformamide (80 mL), potassium carbonate (6.39 g) and 2-chloro-1-fluoro-4-nitrobenzene (8.11 g) was stirred at room temperature overnight. The reaction mixture was poured into saturated aqueous sodium hydrogen carbonate solution, and the mixture was extracted with ethyl acetate. The extract was washed with saturated aqueous sodium hydrogen carbonate solution, dried over anhydrous magnesium sulfate, and concentrated under reduced pressure. The residue was washed with toluene-hexane (1:1) to give a coupling compound (9.78 g). Reduced iron (5.40 g), calcium chloride (2.14 g), water (50 mL) and ethanol (100 mL) were added to the obtained coupling compound, and the mixture was heated to 80°C and stirred for 16 hr. The reaction mixture was cooled to room temperature and filtered through celite. The celite was successively washed with ethanol and ethyl acetate. The filtrate was concentrated under reduced pressure, and the residue was partitioned between ethyl acetate-water was added. The organic layer was dried over anhydrous magnesium sulfate and concentrated under reduced pressure. The residue was separated and purified by silica gel column chromatography (ethyl acetate:methanol=100:0→75:25) to give the title compound (5.72 g) as a brown solid.
$^1$H-NMR (DMSO-$d_6$)δ: 3.83 (3H, s), 5.28 (2H, br s), 6.31 (1H, d, J= 6.9 Hz), 6.53 (1H, dd, J= 2.7 Hz, 8.7 Hz), 6.73 (1H, d, J= 2.7 Hz), 6.86 (1H, d, J= 8.7 Hz), 7.10 (1H, t, J= 7.8 Hz), 7.21 (1H, d, J= 6.9 Hz) 8.12 (1H, s).

(ii) Production of 2-[4-({3-chloro-4-[(1-methyl-1H-benzimidazol-4-yl)oxy]phenyl}amino)-5H-pyrrolo[3,2-d]pyrimidin-5-yl] ethanol

The title compound (107 mg) was obtained as white crystals by operation in the same manner as in Example 139 (iii) and using 3-chloro-4-[(1-methyl-1H-benzimidazol-4-yl)oxy]aniline (137 mg), 2-(4-chloro-5H-pyrrolo[3,2-d]pyrimidin-5-yl)ethyl benzoate (151 mg), isopropyl alcohol (5 mL) and pyridine hydrochloride (25 mg).
$^1$H-NMR (DMSO-$d_6$) δ: 3.86 (5H, m), 4.51-4.54 (2H, m), 6.27 (1H, br s), 6.50 (1H, d, J= 3.0 Hz), 6.59 (1H, t, J= 6.9 Hz), 7.03 (1H, d, J= 9.0 Hz), 7.20 (1H, d, J= 8.1 Hz), 7.34 (1H, d, J= 7.8 Hz), 7.49 (1H, dd, J= 2.7 Hz, 9.0 Hz), 7.65 (1H, d, J= 3.0 Hz), 7.93 (1H, d, J= 2.4 Hz), 8.16 (1H, s), 8.32 (1H, s), 9.77 (1H, br s).
Example 143
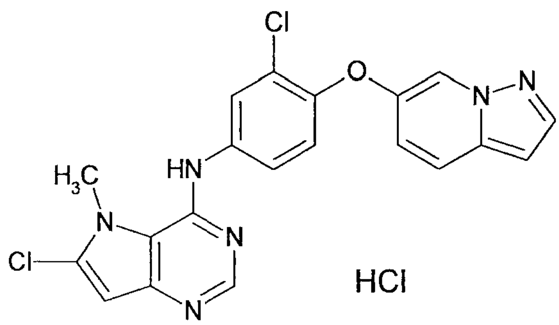

Production of 6-chloro-N-[3-chloro-4-(pyrazolo[1,5-a]pyridin-6-yloxy)phenyl]-5-methyl-5H-pyrrolo[3,2-d]pyrimidin-4-amine hydrochloride

A mixture of 4,6-dichloro-5-methyl-5H-pyrrolo[3,2-d]pyrimidine (101 mg), 3-chloro-4-(pyrazolo[1,5-a]pyridin-6-yloxy)aniline (130 mg), isopropyl alcohol (5 mL) and pyridine hydrochloride (5 mg) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a coupling compound. The obtained coupling compound was dissolved in methanol (3 mL), and 4N hydrochloric acid/ethyl acetate solution (1 mL) was added. The mixture was concentrated under reduced pressure, and the residue was triturated with isopropyl alcohol to give the title compound (175 mg) as a pale-yellow solid.
$^1$H-NMR (DMSO-$d_6$) δ: 4.17 (3H, s), 6.69 (1H, m), 6.90 (1H, s), 7.15-7.23 (2H, m), 7.55 (1H, dd, J= 2.4 Hz, 8.7 Hz), 7.81 (1H, d, J= 9.6 Hz), 7.88 (1H, d, J= 2.4 Hz), 8.01 (1H, d, J= 2.4 Hz), 8.60 (1H, m), 8.67 (1H, s), 9.96 (1H, br s).
Example 144
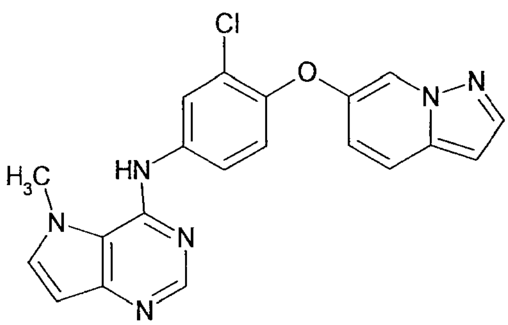

Production of N-[3-chloro-4-(pyrazolo[1,5-a]pyridin-6-yloxy)phenyl]-5-methyl-5H-pyrrolo[3,2-d]pyrimidin-4-amine

A mixture of 4-chloro-5-methyl-5H-pyrrolo[3,2-d]pyrimidine (84 mg), 3-chloro-4-(pyrazolo[1,5-a]pyridin-6-yloxy) aniline (130 mg), isopropyl alcohol (5 mL) and pyridine hydrochloride (5 mg) was stirred at 80°C overnight. The reaction

mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100), and crystallized from ethyl acetate to give the title compound (116 mg) as a white solid.
$^1$H-NMR (DMSO-$d_6$) δ: 4.15 (3H, s), 6.45 (1H, d, J= 3.3 Hz), 6.66 (1H, m), 7.13-7.22 (2H, m), 7.59-7.64 (2H, m), 7.77 (1H, d, J= 9.6 Hz), 7.95-7.98 (2H, m), 8.31 (1H, s), 8.43 (1H, m), 8.58 (1H, br s).
Example 145
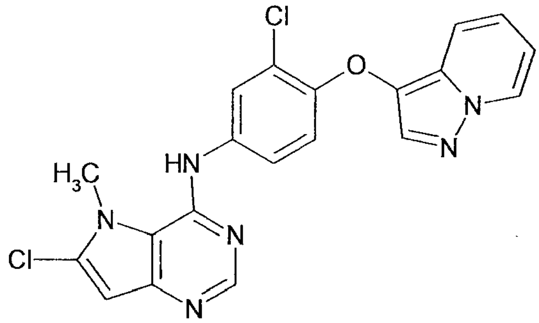

Production of 6-chloro-N-[3-chloro-4-(pyrazolo[1,5-a]pyridin-3-yloxy)phenyl]-5-methyl-5H-pyrrolo[3,2-d]pyrimidin-4-amine

A mixture of 4,6-dichloro-5-methyl-5H-pyrrolo[3,2-d]pyrimidine (101 mg), 3-chloro-4-(pyrazolo[1,5-a]pyridin-3-yloxy)aniline (130 mg), isopropyl alcohol (5 mL) and pyridine hydrochloride (5 mg) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=90:10→0:100), and crystallized from isopropyl alcohol to give the title compound (170 mg) as a gray solid.
$^1$H-NMR (DMSO-$d_6$) δ: 4.03 (3H, s), 6.66 (1H, s), 6.89-6.96 (2H, m), 7.17-7.23 (1H, m), 7.42-7.46 (2H, m), 7.85 (1H, m), 8.07 (1H, s), 8.29 (1H, m), 8.63-8.66 (2H, m).
Example 146
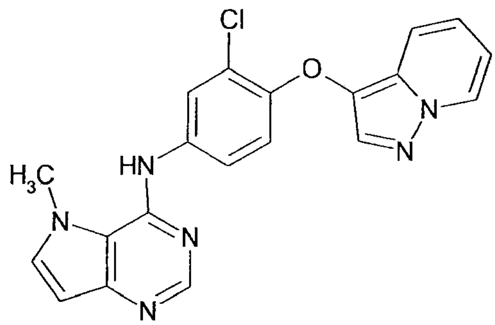

Production of N-[3-chloro-4-(pyrazolo[1,5-a]pyridin-3-yloxy)phenyl]-5-methyl-5H-pyrrolo[3,2-d]pyrimidin-4-amine

A mixture of 4-chloro-5-methyl-5H-pyrrolo[3,2-d]pyrimidine (84 mg), 3-chloro-4-(pyrazolo[1,5-a]pyridin-3-yloxy) aniline (130 mg), isopropyl alcohol (5 mL) and pyridine hydrochloride (5 mg) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→90:10), and

crystallized from methanol/isopropyl alcohol to give the title compound (164 mg) as a pale-pink solid.
$^1$H-NMR (DMSO-$d_6$) δ: 4.13 (3H, s), 6.43 (1H, d, J= 3.3 Hz), 6.88-6.97 (2H, m), 7.17-7.23 (1H, m), 7.43-7.50 (2H, m), 7.57 (1H, d, J= 3.0 Hz), 7.90 (1H, d, J= 2.4 Hz), 8.07 (1H, s), 8.26 (1H, s), 8.47 (1H, br s), 8.64 (1H, d, J= 7.2 Hz).

Example 147

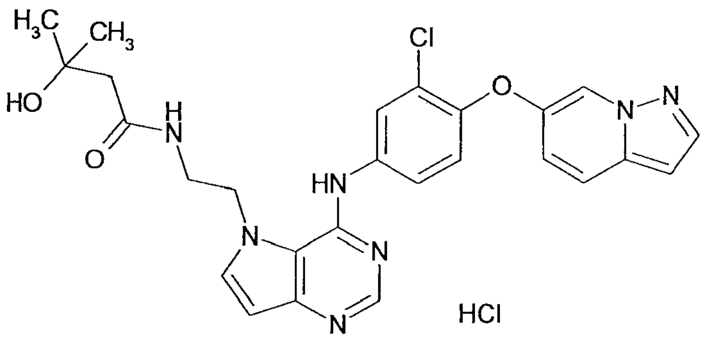

HCl

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-3-methylbutanamide hydrochloride

A mixture of tert-butyl [2-(4-chloro-5H-pyrrolo[3,2-d]pyrimidin-5-yl)ethyl]carbamate (148 mg), 3-chloro-4-(pyrazolo[1,5-a]pyridin-6-yloxy)aniline (130 mg), isopropyl alcohol (5 mL) and pyridine hydrochloride (5 mg) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was dissolved in methanol (4 mL), 4N hydrochloric acid/ethyl acetate solution (2 mL) was added, and the mixture was heated to 60°C and stirred for 1 hr. The reaction mixture was poured into saturated aqueous sodium hydrogen carbonate solution, and the mixture was extracted with ethyl acetate. The extract was dried over anhydrous magnesium sulfate and concentrated under reduced pressure. 3-Hydroxy-3-methylbutanoic acid (65 mg), 1-hydroxybenzotriazole (74 mg), 1-ethyl-3-(3-dimethylaminopropyl)carbodiimide hydrochloride (105 mg) and N,N-dimethylformamide (5 mL) were added to the residue, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→80:20) to give a coupling compound. The obtained coupling compound was dissolved in methanol (3 mL), 4N hydrochloric acid/ethyl acetate solution (1 mL) was added, and the mixture was concentrated under reduced pressure. The residue was crystallized from isopropyl alcohol/ethyl acetate to give the title compound (145 mg) as a white solid.
$^1$H-NMR (DMSO-$d_6$) δ: 1.11 (6H, s), 2.20 (2H, s), 3.47-3.51 (2H, m), 4.63-4.67 (2H, m), 6.67-6.70 (2H, m), 7.16-7.23 (2H, m), 7.61 (1H, dd, J= 2.1 Hz, 8.7 Hz), 7.81 (1H, d, J= 9.9 Hz), 7.92 (1H, d, J= 2.1 Hz), 7.97-8.02 (2H, m), 8.39 (1H, m), 8.60 (1H, m), 8.73 (1H, s), 10.32 (1H, br s).

Example 148

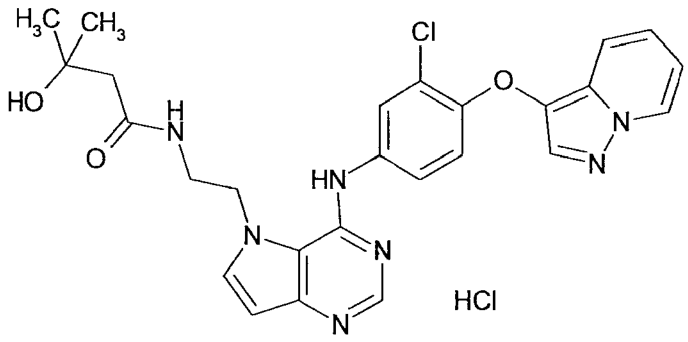

Production of N-[2-(4-{[3-chloro-4-(pyrazolo[1,5-a]pyridin-3-yloxy)phenyl]amino}-5H-pyrrolo[3,2-d]pyrimidin-5-yl)ethyl]-3-hydroxy-3-methylbutanamide hydrochloride

The title compound (127 mg) was obtained as a solid in the same manner as in Example 147 and using 3-chloro-4-(pyrazolo[1,5-a]pyridin-3-yloxy)aniline (130 mg), 2-(4-chloro-5H-pyrrolo[3,2-d]pyrimidin-5-yl)ethyl benzoate (151 mg), isopropyl alcohol (5 mL) and pyridine hydrochloride (5 mg). $^1$H-NMR (DMSO-$d_6$) δ: 1.11 (6H, s), 2.19 (2H, s), 3.45-3.48 (2H, m), 4.60-4.65 (2H, m), 6.65 (1H, d, J= 3.0 Hz), 6.91-6.99 (2H, m), 7.20-7.25 (1H, m), 7.45-7.51 (2H, m), 7.87 (1H, d, J= 2.4 Hz), 7.96 (1H, d, J= 2.7 Hz), 8.14 (1H, s), 8.36 (1H, m), 8.66-8.68 (2H, m), 10.23 (1H, br s).

Example 149

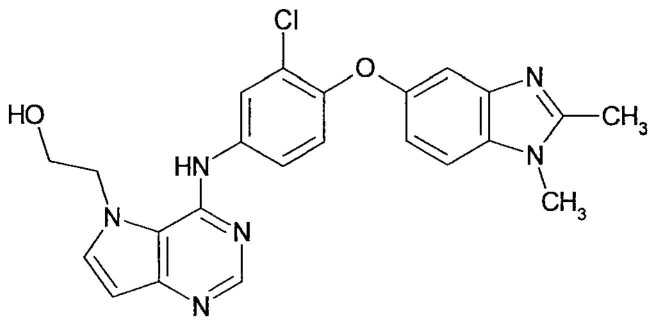

Production of 2-[4-({3-chloro-4-[(1,2-dimethyl-1H-benzimidazol-5-yl)oxy]phenyl}amino)-5H-pyrrolo[3,2-d]pyrimidin-5-yl]ethanol

(i) Production of 3-chloro-4-[(1,2-dimethyl-1H-benzimidazol-5-yl)oxy]aniline

A mixture of 1,2-dimethyl-1H-benzimidazol-5-ol (811 mg), N,N-dimethylformamide (10 mL), potassium carbonate (691 mg) and 2-chloro-1-fluoro-4-nitrobenzene (878 mg) was stirred at room temperature for 5 days. The reaction mixture was poured into water, and the mixture was extracted with ethyl acetate. The extract was washed with saturated aqueous sodium hydrogen carbonate solution, dried over anhydrous magnesium sulfate, and concentrated under reduced pressure. The residue was separated and purified by silica gel column chromatography (ethyl acetate:methanol=100:0→80:20) to give a coupling compound (998 mg). Reduced iron (526 mg), calcium chloride (209 mg), water (6 mL) and ethanol (12 mL) were added to the obtained coupling compound, and the mixture was heated to 80°C and stirred overnight. The reaction mixture was cooled to room temperature and filtered through celite. The celite was washed with ethanol. The filtrate was concentrated under reduced pressure, and ethyl acetate, water and a small amount of methanol were added to the residue, and the mixture was partitioned. The organic layer was dried over anhydrous magnesium sulfate and concentrated under reduced pressure. The residue was separated and purified by silica gel column chromatography (ethyl acetate:methanol=100:0→90:10) to give the title compound (5.72 g) as a gray solid. $^1$H-NMR ($CDCl_3$)δ: 2.57 (3H, s), 3.61 (2H, br s), 3.70 (3H, s), 6.53 (1H, dd, J= 3.0 Hz, 8.7 Hz), 6.78-6.85 (2H, m), 6.94 (1H, dd, J= 2.1 Hz, 8.7 Hz), 7.16-7.19 (2H, m).

(ii) Production of 2-[4-({3-chloro-4-[(1,2-dimethyl-1H-benzimidazol-5-yl)oxy]phenyl}amino)-5H-pyrrolo[3,2-d]pyrimidin-5-yl]ethanol

A mixture of 2-(4-chloro-5H-pyrrolo[3,2-d]pyrimidin-5-yl)ethyl benzoate (151 mg), 3-chloro-4-[(1,2-dimethyl-1H-benzimidazol-5-yl)oxy]aniline (144 mg), isopropyl alcohol (5 mL) and pyridine hydrochloride (100 mg) was heated to 80°C and stirred overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→80:20) to give a coupling compound. Tetrahydrofuran (2 mL), methanol (2 mL), 1N aqueous sodium hydroxide solution (1 mL) were added to the obtained coupling compound and the mixture was stirred for 1 hr. The reaction mixture was poured into saturated aqueous sodium hydrogen carbonate solution, and the mixture was extracted with ethyl acetate. The extract was dried over anhydrous magnesium sulfate and concentrated under reduced pressure, and the residue was crystallized from methanol/isopropyl alcohol to give the title compound (135 mg) as white crystals.
$^1$H-NMR (DMSO-$d_6$) δ: 2.50 (3H, s), 3.73 (3H, s), 3.85-3.88 (2H, m), 4.51-4.54 (2H, m), 6.26 (1H, br s), 6.50 (1H, d, J= 3.0 Hz), 6.90 (1H, dd, J= 2.4 Hz, 8.7 Hz), 7.01-7.04 (2H, m), 7.46-7.52 (2H, m), 7.64 (1H, d, J= 3.0 Hz), 7.93 (1H, d, J= 2.7 Hz), 8.31 (1H, s), 9.74 (1H, br s).
Example 150
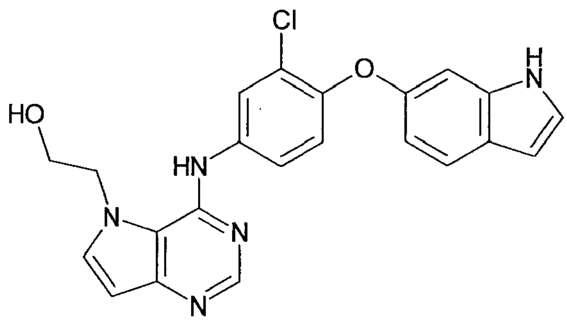

Production of nyl]amino}-5H-pyrrolo[3,2-d]pyrimidin-5-yl)ethanol

(i) Production of 3-chloro-4-(1H-indol-6-yloxy)aniline

A mixture of 1H-indol-6-ol (500 mg), N,N-dimethylformamide (10 mL), potassium carbonate (520 mg) and 2-chloro-1-fluoro-4-nitrobenzene (660 mg) was stirred at room temperature for 5 days. The reaction mixture was poured into water, and the mixture was extracted with ethyl acetate. The extract was washed with saturated aqueous sodium hydrogen carbonate solution, dried over anhydrous magnesium sulfate, and concentrated under reduced pressure. The residue was separated and purified by silica gel column chromatography (hexane:ethyl acetate=95:5→70:30) to give a coupling compound (1.05 g). Reduced iron (609 mg), calcium chloride (242 mg), water (7.5 mL) and ethanol (15 mL) were added to the obtained coupling compound, and the mixture was heated to 80°C and stirred overnight. The reaction mixture was cooled to room temperature and filtered through celite. The celite was washed with ethanol. The filtrate was concentrated under reduced pressure, and the residue was partitioned with ethyl acetate, water and a small amount of methanol. The organic layer was dried over anhydrous magnesium sulfate and concentrated under reduced pressure. The residue was separated and purified by silica gel column chromatography (hexane:ethyl acetate=97:3→70:30) to give the title compound (422 mg) as an orange solid.
$^1$H-NMR ($CDCl_3$)δ: 3.63 (2H, br s), 6.49-6.57 (2H, m), 6.80-6.89 (4H, m), 7.12-7.14 (1H, m), 7.53 (1H, d, J= 8.1 Hz), 8.01 (1H, br s).

(ii) Production of 2-(4-{[3-chloro-4-(1H-indol-6-yloxy)phenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (151 mg), 3-chloro-4-(1H-indol-6-yloxy) aniline (129 mg), isopropyl alcohol (5 mL) and pyridine hydrochloride (5 mg) was heated to 80°C and stirred overnight. The reaction mixture was poured into saturated aqueous sodium hydrogen carbonate solution, and the mixture was

extracted with ethyl acetate. The extract was dried over anhydrous magnesium sulfate and concentrated under reduced pressure. The residue was separated and purified by silica gel column chromatography (ethyl acetate:methanol=90:10→0:100) to give a coupling compound. Tetrahydrofuran (2 mL), methanol (2 mL) and 1N aqueous sodium hydroxide solution (1 mL) were added to the obtained coupling compound and the mixture was stirred for 1 hr. The reaction mixture was poured into saturated aqueous sodium hydrogen carbonate solution, and the mixture was extracted with ethyl acetate. The extract was dried over anhydrous magnesium sulfate and concentrated under reduced pressure, and the residue was crystallized from ethyl acetate to give the title compound (135 mg) as pale-yellow crystals.
$^1$H-NMR (DMSO-$d_6$) δ: 3.86-3.89 (2H, m), 4.52-4.55 (2H, m), 6.27 (1H, br s), 6.40-6.41 (1H, m), 6.50 (1H, d, J= 3.3 Hz), 6.76 (1H, dd, J= 2.1 Hz, 8.4 Hz), 6.84-6.85 (1H, m), 7.12 (1H, d, J= 8.7 Hz), 7.28 (1H, t, J= 3.0 Hz), 7.51-7.55 (2H, m), 7.65 (1H, d, J= 3.0 Hz), 7.94 (1H, d, J= 2.4 Hz), 8.33 (1H, s), 9.79 (1H, br s), 10.95 (1H, br s).

Example 151

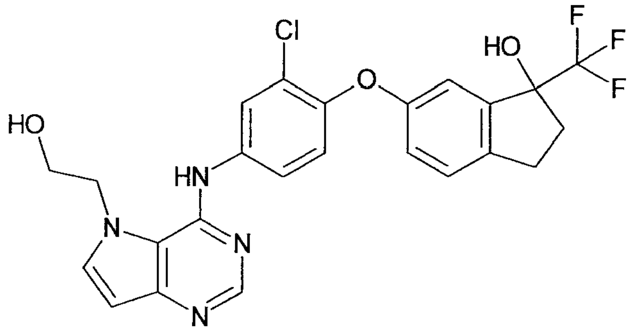

Production of 6-(2-chloro-4-{[5-(2-hydroxyethyl)-5H-pyrrolo[3,2-d]pyrimidin-4-yl]amino}phenoxy)-1-(trifluoromethyl)indan-1-ol

(i) Production of 6-(2-chloro-4-nitrophenoxy)-1-(trifluoromethyl)indan-1-ol

A mixture of 6-(2-chloro-4-nitrophenoxy)indan-1-one (3.04 g), trimethyl(trifluoromethyl)silane (1.71 g) and tetrahydrofuran (70 mL) was cooled to 0°C in an ice bath, and stirring was started. 1M tetrabutylammonium fluoride/tetrahydrofuran (2 mL) was added, the reaction mixture was allowed to warm to room temperature, and stirred overnight. Trimethyl(trifluoromethyl)silane (1.71 g) was added, and the mixture was stirred for 3 hr and 6N hydrochloric acid (40 mL) was added. One hr later, ethyl acetate was added for partitioning, and the obtained organic layer was washed with saturated aqueous sodium chloride solution, dried over anhydrous magnesium sulfate, and concentrated under reduced pressure. The residue was separated and purified by silica gel column chromatography (hexane:ethyl acetate=97:3→75:15) to give the title compound (2.95 g) as a pale-brown oil.
$^1$H-NMR ($CDCl_3$) δ: 2.29-2.34 (1H, m), 2.50 (1H, s), 2.70-2.79 (1H, m), 2.98-3.17 (2H, m), 6.87 (1H, d, J= 9.0 Hz), 7.10 (1H, dd, J= 2.4 Hz, 8.4 Hz), 7.23 (1H, m), 7.36 (1H, d, J= 8.1 Hz), 8.06 (1H, dd, J= 2.7 Hz, 9.0 Hz), 8.39 (1H, d, J= 2.7 Hz).

(ii) Production of 6-(4-amino-2-chlorophenoxy)-1-(trifluoromethyl)indan-1-ol

6-(2-Chloro-4-nitrophenoxy)-1-(trifluoromethyl)indan-1-ol (2.93 g), reduced iron (1.31 g), calcium chloride (522 mg), water (12.5 mL) and ethanol (25 mL) were mixed, and the mixture was heated to 80°C and stirred overnight. The reaction mixture was cooled to room temperature and filtered through celite. The celite was washed with ethanol. The filtrate was concentrated under reduced pressure, and the residue was partitioned with ethyl acetate, water and a small amount of methanol. The organic layer was dried over anhydrous magnesium sulfate and concentrated under reduced pressure. The residue was separated and purified by silica gel column chromatography (hexane:ethyl acetate=97:3→70:30) to give the title compound (1.71 g) as a yellow oil.
$^1$H-NMR ($CDCl_3$)δ: 2.22-2.30 (1H, m), 2.42 (1H, br s), 2.63-2.72 (1H, m), 2.88-3.07 (2H, m), 3.67 (2H, br s), 6.57 (1H, dd, J= 3.0 Hz, 8.7 Hz), 6.79 (1H, d, J= 2.7 Hz), 6.87-6.92 (2H, m), 7.00 (1H, m), 7.18 (1H, d, J= 8.4 Hz).

(iii) Production of 6-(2-chloro-4-{[5-(2-hydroxyethyl)-5H-pyrrolo[3,2-d]pyrimidin-4-yl]amino}phenoxy)-1-(trifluoromethyl) indan-1-ol

A mixture of 2-(4-chloro-5H-pyrrolo[3,2-d]pyrimidin-5-yl)ethyl benzoate (151 mg), 6-(4-amino-2-chlorophenoxy)-1-(trifluoromethyl)indan-1-ol (172 mg), isopropyl alcohol (5 mL) and pyridine hydrochloride (5 mg) was heated to 80°C and stirred overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane: ethyl acetate=95:5→0:100) to give a coupling compound. Tetrahydrofuran (2 mL), methanol (2 mL) and 1N aqueous sodium hydroxide solution (1 mL) were added to the obtained coupling compound and the mixture was stirred for 1 hr. The reaction mixture was poured into saturated aqueous sodium hydrogen carbonate solution, and the mixture was extracted with ethyl acetate. The extract was dried over anhydrous magnesium sulfate and concentrated under reduced pressure, and the residue was crystallized from isopropyl alcohol to give the title compound (136 mg) as pale-pink crystals. $^1$H-NMR (DMSO-$d_6$) δ: 2.12-2.25 (1H, m), 2.50-2.58 (1H, m), 2.76-3.03 (2H, m), 3.86-3.89 (2H, m), 4.52-4.54 (2H, m), 6.29 (1H, br s), 6.51 (1H, d, J= 3.0 Hz), 6.68 (1H, s), 6.92-6.98 (2H, m), 7.19 (1H, d, J= 9.0 Hz), 7.30 (1H, d, J= 8.4 Hz), 7.59 (1H, dd, J= 2.4 Hz, 8.7 Hz), 7.66 (1H, d, J= 3.0 Hz), 7.97 (1H, d, J= 2.4 Hz), 8.34 (1H, s), 9.85 (1H, br s).

Example 152

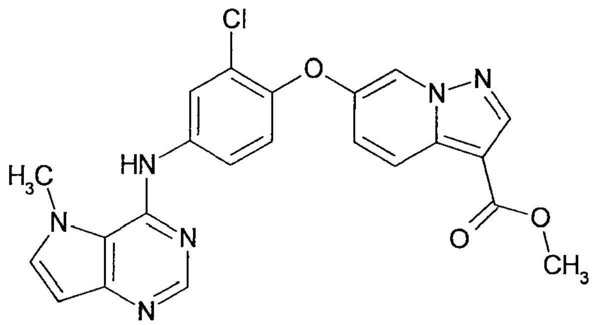

Production of methyl 6-{2-chloro-4-[(5-methyl-5H-pyrrolo[3,2-d]pyrimidin-4-yl)amino]phenoxy}pyrazolo[1,5-a] pyridine-3-carboxylate

(i) Production of methyl 6-hydroxypyrazolo[1,5-a]pyridine-3-carboxylate

A mixture of methyl 6-(benzyloxy)pyrazolo[1,5-a]pyridine-3-carboxylate (1.39 g), 10% palladium/activated carbon (140 mg) and methanol (30 mL) was stirred under hydrogen atmosphere (up to 1 pressure), and then at room temperature for 3 hr. The reaction mixture was filtered through celite. The celite was successively washed with methanol and ethyl acetate. The filtrate was concentrated under reduced pressure to give the title compound (928 mg) as a gray solid.
$^1$H-NMR ($CDCl_3$)δ: 3.80 (3H, s), 7.32 (1H, dd, J= 2.1 Hz, 9.3 Hz), 7.94 (1H, d, J= 9.3 Hz), 8.25-8.29 (2H, m), 10.13 (1H, br s).

(ii) Production of methyl 6-(2-chloro-4-nitrophenoxy)pyrazolo[1,5-a]pyridine-3-carboxylate

Methyl 6-hydroxypyrazolo[1,5-a]pyridine-3-carboxylate (928 mg) was dissolved in N,N-dimethylformamide (10 mL), potassium carbonate (668 mg) and 2-chloro-1-fluoro-4-nitrobenzene (848 mg) were added, and the mixture was stirred at room temperature for 12 hr. The reaction mixture was partitioned between ethyl acetate and water, and the organic layer was dried over anhydrous magnesium sulfate and concentrated under reduced pressure. The residue was washed with hexane to give the title compound (1.33 g) as a pale-yellow solid.
$^1$H-NMR ($CDCl_3$) δ: 3.94 (3H, s), 7.00 (1H, d, J= 6.3 Hz), 7.26-7.31 (1H, m), 8.12 (1H, dd, J= 2.7 Hz, 9.0 Hz), 8.26 (1H, d, J= 9.6 Hz), 8.43-8.45 (3H, m).

(iii) Production of methyl 6-(4-amino-2-chlorophenoxy)pyrazolo[1,5-a]pyridine-3-carboxylate

A mixture of methyl 6-(2-chloro-4-nitrophenoxy)pyrazolo[1,5-a]pyridine-3-carboxylate (695 mg), reduced iron

(559 mg), calcium chloride (222 mg), water (2 mL) and methanol (10 mL) was heated to 70°C and stirred for 16 hr. The mixture was cooled to room temperature and filtered through celite. The celite was successively washed with methanol and ethyl acetate. The filtrate was concentrated under reduced pressure, and the residue was partitioned between ethyl acetate and water, and the organic layer was dried over anhydrous magnesium sulfate and concentrated under reduced pressure. The residue was separated and purified by silica gel column chromatography (hexane:ethyl acetate=95:5→0:100) to give the title compound (495 mg) as a pale-yellow solid.
$^1$H-NMR (DMSO-$d_6$)δ: 3.82 (3H, s), 5.42 (2H, br s), 6.57 (1H, dd, J= 2.7 Hz, J= 8.7 Hz), 6.75 (1H, d, J= 2.4 Hz), 7.03 (1H, d, J= 8.7 Hz), 7.45 (1H, dd, J= 1.8 Hz, 9.3 Hz), 8.06 (1H, d, J= 9.6 Hz), 8.31 (1H, m), 8.41 (1H, s).

(iv) Production of methyl 6-{2-chloro-4-[(5-methyl-5H-pyrrolo[3,2-d]pyrimidin-4-yl)amino]phenoxy}pyrazolo[1,5-a]pyridine-3-carboxylate

A mixture of 4-chloro-5-methyl-5H-pyrrolo[3,2-d]pyrimidine (84 mg), methyl 6-(4-amino-2-chlorophenoxy)pyrazolo[1,5-a]pyridine-3-carboxylate (159 mg) and 1-methyl-2-pyrrolidone (5 mL) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100), and triturated with ethyl acetate/hexane to give the title compound (132 mg) as a white solid.
$^1$H-NMR (DMSO-$d_6$) δ: 3.84 (3H, s), 4.15 (3H, s), 6.46 (1H, d, J= 3.0 Hz), 7.27 (1H, d, J= 9.0 Hz), 7.51-7.67 (3H, m), 7.98 (1H, m), 8.12 (1H, d, J= 6.6 Hz), 8.32 (1H, s), 8.46 (1H, s), 8.60 (1H, s), 8.67 (1H, m).

Example 153

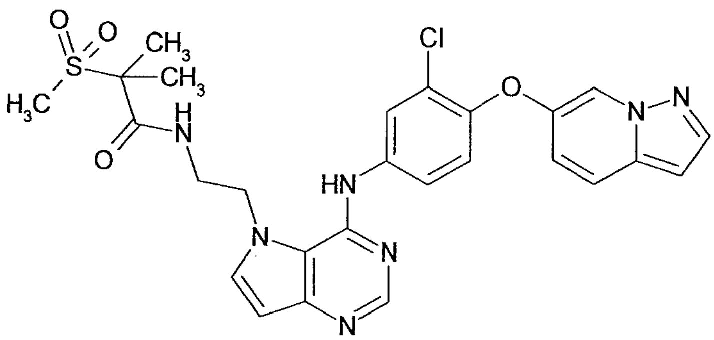

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-2-methyl-2-(methylsulfonyl)propanamide

(i) Production of 5-(2-aminoethyl)-N-[3-chloro-4-(pyrazolo[1,5-a]pyridin-6-yloxy)phenyl]-5H-pyrrolo[3,2-d]pyrimidin-4-amine dihydrochloride

A mixture of tert-butyl [2-(4-chloro-5H-pyrrolo[3,2-d]pyrimidin-5-yl)ethyl]carbamate (742 mg), 3-chloro-4-(pyrazolo[1,5-a]pyridin-6-yloxy)aniline (650 mg), isopropyl alcohol (15 mL) and pyridine hydrochloride (20 mg) was stirred at 80°C overnight. The reaction mixture was concentrated under reduced pressure, methanol (6 mL) and 4N hydrochloric acid/ethyl acetate solution (6 mL) were added, and the mixture was heated to 50°C and stirred for 2 hr. After cooling to room temperature, the precipitate was collected by filtration to give the title compound (1.23 g) as a white solid.
$^1$H-NMR (DMSO-$d_6$) δ: 3.27-3.32 (2H, m), 5.04 (2H, m), 6.69 (1H, m), 6.75 (1H, d, J= 3.0 Hz), 7.16-7.23 (2H, m), 7.55 (1H, dd, J= 2.4 Hz, 8.7 Hz), 7.81 (1H, d, J= 9.0 Hz), 7.88 (1H, d, J= 2.4 Hz), 8.01 (1H, d, J= 2.1 Hz), 8.09 (1H, d, J= 3.6 Hz), 8.33 (3H, br s), 8.60 (1H, m), 8.74 (1H, s), 10.20 (1H, br s).

(ii) Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-2-methyl-2-(methylsulfonyl)propanamide

5-(2-Aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL) and N,N-dimethylformamide (5 mL) were mixed, and stirring thereof was started at room temperature. 2-Methyl-2-(methylsulfonyl)propanoic acid (60 mg), 1-hydroxybenzotriazole (49 mg) and 1-ethyl-3-(3-dimethylaminopropyl)carbodiimide hydrochloride (69 mg) were successively added, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→90:10), and triturated with hexane/ethyl acetate to give the title compound (60 mg) as a white solid.
$^1$H-NMR (DMSO-$d_6$) δ: 1.41 (6H, s), 2.96 (3H, s), 3.47 (2H, m), 4.57 (2H, m), 6.48 (1H, d, J= 3.0 Hz), 6.66 (1H, dd, J= 0.9 Hz, 2.4 Hz), 7.13-7.22 (2H, m), 7.57 (1H, d, J= 3.3 Hz), 7.69 (1H, dd, J= 2.7 Hz, 9.0 Hz), 7.77 (1H, d, J= 9.6 Hz), 7.98 (2H, m), 8.21 (1H, m), 8.33 (1H, s), 8.44 (1H, m), 8.66 (1H, br s).

Example 154

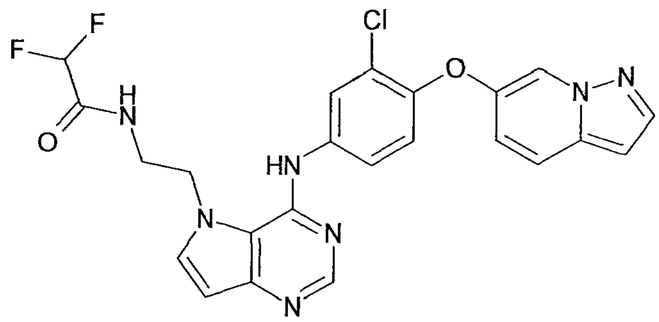

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-2,2-difluoroacetamide

The title compound (93 mg) was obtained as a white solid by operation in the same manner as in Example 153 (ii) and using 5-(2-aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL), N,N-dimethylformamide (5 mL), 1-hydroxybenzotriazole (49 mg), 1-ethyl-3-(3-dimethylaminopropyl)carbodiimide hydrochloride (69 mg) and difluoroacetic acid (35 mg).
$^1$H-NMR (DMSO-$d_6$) δ: 3.48 (2H, m), 4.62 (2H, t, J= 6.0 Hz), 6.12 (1H, t, J= 53.7 Hz), 6.48 (1H, m), 6.66 (1H, m), 7.14-7.22 (2H, m), 7.57 (2H, m), 7.77 (1H, d, J= 9.3 Hz), 7.91 (1H, m), 7.98 (1H, d, J= 2.4 Hz), 8.32 (1H, m), 8.43 (1H, m), 8.59 (1H, br s), 8.91 (1H, br s).
Example 155
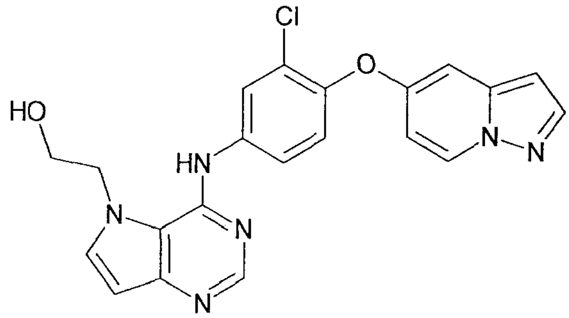

Production of 2-(4-{[3-chloro-4-(pyrazolo[1,5-a]pyridin-5-yloxy)phenyl]amino}-5H-pyrrolo[3,2-d]pyrimidin-5-yl) ethanol

(i) Production of 3-chloro-4-(pyrazolo[1,5-a]pyridin-5-yloxy)aniline

A mixture of pyrazolo[1,5-a]pyridin-5-ol (224 mg), N,N-dimethylformamide (5 mL), potassium carbonate (231 mg) and 2-chloro-1-fluoro-4-nitrobenzene (293 mg) was stirred at room temperature overnight. The reaction mixture was poured into water, and the mixture was extracted with ethyl acetate. The extract was washed with saturated aqueous sodium hydrogen carbonate solution, dried over anhydrous magnesium sulfate, and concentrated under reduced pressure. The residue was separated and purified by silica gel column chromatography (hexane:ethyl acetate=95:5→60:40) to give a coupling compound (401 mg). Reduced iron (386 mg), calcium chloride (154 mg), water (2 mL) and ethanol (10 mL) were added to the obtained coupling compound, and the mixture was heated to 80°C and stirred overnight. The reaction mixture was cooled to room temperature and filtered through celite. The celite was successively washed with ethanol and ethyl acetate. The filtrate was concentrated under reduced pressure, and the residue was partitioned with ethyl acetate, water and a small amount of methanol. The organic layer was dried over anhydrous magnesium sulfate and concentrated under reduced pressure. The residue was separated and purified successively by silica gel column chromatography (hexane:ethyl acetate=95:5→50:50) and basic silica gel column chromatography (hexane:ethyl acetate=97:3→60:40) to give the title compound (254 mg) as a brown oil.
$^1$H-NMR ($CDCl_3$)δ: 3.74 (2H, br s), 6.26 (1H, m), 6.57-6.63 (3H, m), 6.81 (1H, d, J= 3.0 Hz), 6.98 (1H, d, J= 8.7 Hz), 7.86 (1H, d, J= 2.1 Hz), 8.37 (1H, d, J= 7.5 Hz).

(ii) Production of 2-(4-{[3-chloro-4-(pyrazolo[1,5-a]pyridin-5-yloxy)phenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (295 mg), 3-chloro-4-(pyrazolo[1,5-a]pyridin-5-yloxy)aniline (254 mg), isopropyl alcohol (9 mL) and pyridine hydrochloride (10 mg) was heated to 80°C and stirred overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a coupling compound. Tetrahydrofuran (2 mL), methanol (2 mL) and 1N aqueous sodium hydroxide solution (1 mL) were added to the obtained coupling compound and the mixture was stirred for 1 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was purified by high performance liquid chromatography (ODS, 0.1%trifluoroacetic acid containing water: acetonitrile=92:8→0:100), and a fraction containing the object product was poured into saturated aqueous sodium hydrogen carbonate solution, and the mixture was extracted with ethyl acetate. The extract was dried over anhydrous magnesium sulfate and concentrated under reduced pressure, and the residue was crystallized from ethyl acetate to give the title compound (96 mg) as pale-yellow crystals.
$^1$H-NMR (DMSO-$d_6$) δ: 3.89 (2H, t, J= 4.8 Hz), 4.54 (2H, m), 6.33 (1H, br s), 6.43 (1H, d, J= 1.5 Hz), 6.52 (1H, d, J= 3.0 Hz), 6.75-6.78 (2H, m), 7.38 (1H, d, J= 9.0 Hz), 7.63-7.68 (2H, m), 7.92 (1H, d, J= 2.1 Hz), 8.01 (1H, d, J= 2.4 Hz), 8.36 (1H, s), 8.68 (1H, d, J= 6.9 Hz), 9.93 (1H, br s).
Example 156
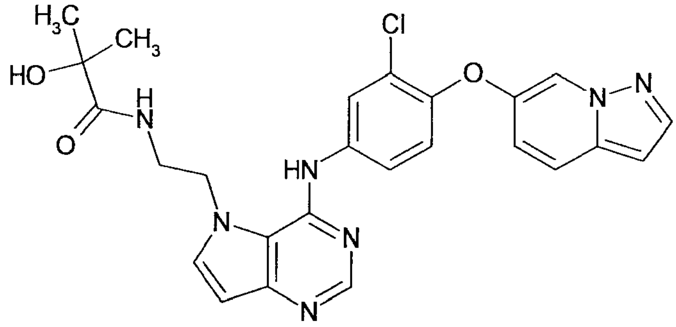

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-2-hydroxy-2-methylpropanamide

The title compound (70 mg) was obtained as a white solid by operation in the same manner as in Example 153 (ii) and using 5-(2-aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL) and N,N-dimethylformamide (5 mL), 1-hydroxybenzotriazole (49 mg), 1-ethyl-3-(3-dimethylaminopropyl)carbodiimide hydrochloride (69 mg) and 2-hydroxy-2-methylpropanoic acid (37 mg).
$^1$H-NMR (DMSO-$d_6$) δ: 1.17 (6H, s), 3.43 (2H, m), 4.53 (2H, m), 5.38 (1H, s), 6.47 (1H, d, J= 3.0 Hz), 6.66 (1H, d, J= 1.8 Hz), 7.14-7.21 (2H, m), 7.57 (1H, m), 7.70-7.79 (2H, m), 7.97-8.05 (3H, m), 8.31 (1H, s), 8.45 (1H, br s), 8.74 (1H, br s).
Example 157
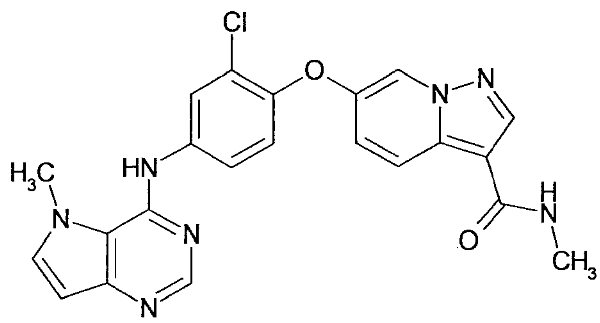

Production of 6-{2-chloro-4-[(5-methyl-5H-pyrrolo[3,2-d]pyrimidin-4-yl)amino]phenoxy}-N-methylpyrazolo[1,5-a]pyridine-3-carboxamide

A mixture of methyl 6-{2-chloro-4-[(5-methyl-5H-pyrrolo[3,2-d]pyrimidin-4-yl)amino]phenoxy}pyrazolo[1,5-a]pyridine-3-carboxylate (93 mg), 8N aqueous sodium hydroxide solution (0.25 mL) and methanol (5 mL) was heated to 70°C and stirred overnight. The reaction mixture was concentrated under reduced pressure, and partitioned with 6N hydrochloric acid (0.333 mL), water (15 mL) and ethyl acetate/tetrahydrofuran (2:1, 200 mL). The aqueous layer was extracted twice with ethyl acetate/tetrahydrofuran (2:1, 200 mL). The combined organic layers were dried over anhydrous magnesium sulfate, and concentrated under reduced pressure. The residue was mixed with methylamine hydrochloride (20 mg), triethylamine (43 μL) and N,N-dimethylformamide (5 mL) and stirring of the mixture was started at room temperature. 1-Hydroxybenzotriazole (49 mg) and 1-ethyl-3-(3-dimethylaminopropyl)carbodiimide hydrochloride (69 mg) were successively added, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→66:34) to give the title compound (40 mg) as a white solid.
$^1$H-NMR (DMSO-$d_6$) δ: 2.79 (3H, d, J= 4.5 Hz), 4.15 (3H, s), 6.46 (1H, d, J= 3.0 Hz), 7.24 (1H, d, J= 8.7 Hz), 7.38 (1H, dd, J= 2.1 Hz, 9.6 Hz), 7.60-7.66 (2H, m), 7.97 (1H, m), 8.18-8.31 (3H, m), 8.47-8.59 (3H, m).
Example 158

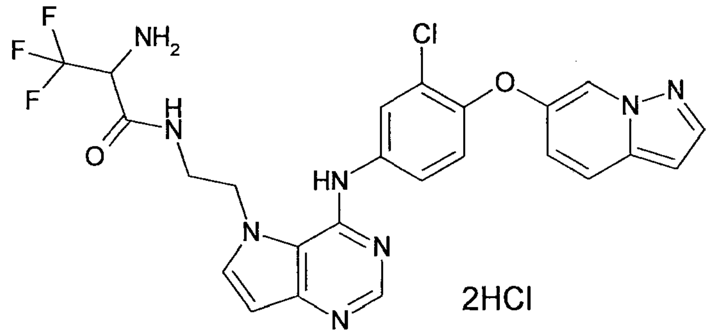

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-3,3,3-trifluoroalaninamide dihydrochloride

5-(2-Aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL) and N,N-dimethylformamide (5 mL) were mixed, and stirring was started at room temperature. N-(tert-Butoxycarbonyl)-3,3,3-trifluoroalanine (75 mg), 1-hydroxybenzotriazole (49 mg) and 1-ethyl-3-(3-dimethylaminopropyl)carbodiimide hydrochloride (69 mg) were successively added, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. Ethyl acetate was added to the residue and the precipitate was collected by filtration and dissolved in methanol (5 mL). 4N Hydrochloric acid/ethyl acetate solution (3 mL) was added, and the mixture was stirred at room temperature for 2 hr. The reaction mixture was concentrated under reduced pressure, and the residue was triturated with ethanol/ethyl acetate to give the title compound (113 mg) as a pale-yellow solid.
$^1$H-NMR (DMSO-$d_6$) δ: 3.40-3.85 (2H, m), 4.69-4.95 (5H, m), 6.65-6.70 (2H, m), 7.16-7.24 (2H, m), 7.55 (1H, dd, J= 2.7 Hz, 9.0 Hz), 7.82 (1H, d, J= 9.6 Hz), 7.89 (1H, m), 8.01 (2H, m), 8.60 (1H, m), 8.73 (1H, s), 9.18 (1H, br s), 10.69 (1H, br s).
Example 159
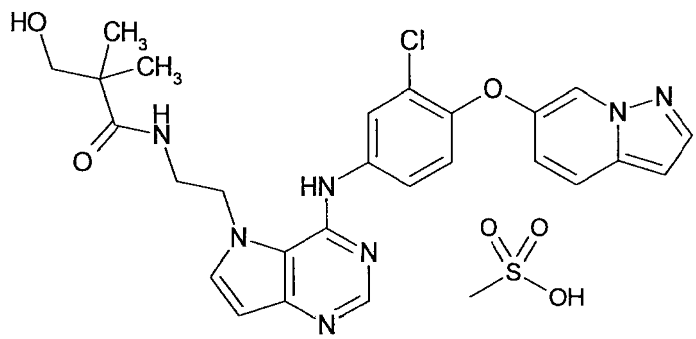

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-2,2-dimethylpropanamide methanesulfonate

5-(2-Aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL) and N,N-dimethylformamide (5 mL) were mixed, and stirring was started at room temperature. 3-Hydroxy-2,2-dimethylpropanoic acid (43 mg), 1-hydroxybenzotriazole (49 mg) and 1-ethyl-3-(3-dimethylaminopropyl)carbodiimide hydrochloride (69 mg) were successively added, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl

acetate:methanol=100:0→80:20) to give a coupling compound (113 mg). The obtained coupling compound was dissolved in ethanol (2 mL), methanesulfonic acid (15.5 μL) was added, and the mixture was concentrated under reduced pressure, and the residue was triturated with ethanol/ethyl acetate to give the title compound (125 mg) as a white solid.
$^1$H-NMR (DMSO-$d_6$) δ: 0.96 (6H, s), 2.30 (3H, s), 3.29 (2H, s), 3.41-3.50 (3H, m), 4.59-4.63 (2H, m), 6.65 (1H, d, J= 3.0 Hz), 6.70 (1H, dd, J= 0.6 Hz, 2.4 Hz), 7.16-7.24 (2H, m), 7.63 (1H, dd, J= 2.7 Hz, 8.7 Hz), 7.80-7.95 (4H, m), 8.01 (1H, d, J= 2.7 Hz), 8.61 (1H, m), 8.72 (1H, s), 10.09 (1H, br s).

Example 160

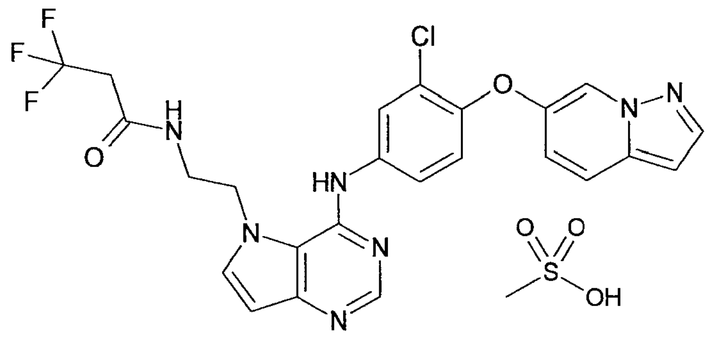

Production of N-[2-(4-{[3-chloro-4-(pyrazolo[1,5-a]pyridin-6-yloxy)phenyl]amino}-5H-pyrrolo[3,2-d]pyrimidin-5-yl)ethyl]-3,3,3-trifluoropropanamide methanesulfonate

The title compound (129 mg) was obtained as a white solid by operation in the same manner as in Example 159 and using 5-(2-aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL), N,N-dimethylformamide (5 mL), 1-hydroxybenzotriazole (49 mg), 1-ethyl-3-(3-dimethylaminopropyl)carbodiimide hydrochloride (69 mg) and 3,3,3-trifluoropropanoic acid (46 mg).
$^1$H-NMR (DMSO-$d_6$) δ: 2.30 (3H, s), 3.15-3.27 (2H, m), 3.51 (2H, m), 4.68 (2H, m), 6.66-6.70 (2H, m), 7.16-7.24 (2H, m), 7.56 (1H, dd, J= 2.4 Hz, 8.7 Hz), 7.82 (1H, d, J= 9.9 Hz), 7.88 (1H, d, J= 2.4 Hz), 7.93 (1H, m), 8.01 (1H, d, J= 2.4 Hz), 8.52 (1H, m), 8.61 (1H, m), 8.73 (1H, m), 9.85 (1H, br s).

Example 161

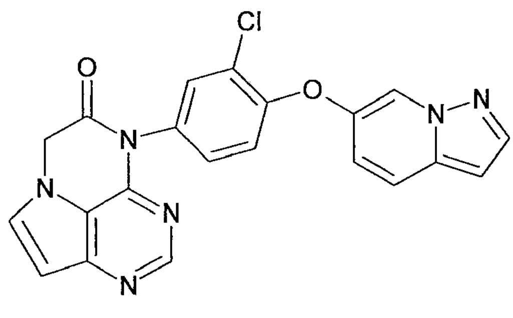

Production of 4-[3-chloro-4-(pyrazolo[1,5-a]pyridin-6-yloxy)phenyl]-4H-pyrrolo[3,2,1-de]pteridin-5(6H)-one

A mixture of ethyl (4-chloro-5H-pyrrolo[3,2-d]pyrimidin-5-yl)acetate (201 mg), 3-chloro-4-(pyrazolo[1,5-a]pyridin-6-yloxy)aniline (218 mg), 1-methyl-2-pyrrolidone (5 mL) and pyridine hydrochloride (5 mg) was stirred at 80°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a purified product (390 mg). Tetrahydrofuran (2 mL), methanol (2 mL), 1N aqueous sodium hydroxide solution (1 mL) were added to the obtained purified product and the mixture was stirred at room temperature for 4 hr. 1N Hydrochloric acid (1 mL) was added, and the reaction mixture was concentrated under reduced pressure. Triethylamine (141 μL), (3,4-trans)-3-azidopiperidin-4-ol hydrochloride (153 mg) and N,N-dimethylformamide (5 mL)

were added to the residue, and the mixture was stirred at room temperature for 5 min. 1-Hydroxybenzotriazole (50 mg) and 1-ethyl-3-(3-dimethylaminopropyl)carbodiimide hydrochloride (70 mg) were successively added, and the mixture was stirred at room temperature overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give the title compound (85 mg) as a white solid.
$^1$H-NMR (DMSO-$d_6$) δ: 5.32 (2H, s), 6.67-6.72 (2H, m), 7.18 (1H, d, J= 8.7 Hz), 7.24 (1H, dd, J= 2.1 Hz, 9.6 Hz), 7.36 (1H, dd, J= 2.4 Hz, 8.7 Hz), 7.70 (1H, d, J= 2.4 Hz), 7.79-7.85 (2H, m), 8.04 (1H, d, J= 2.4 Hz), 8.43 (1H, s), 8.79 (1H, m).
Example 162
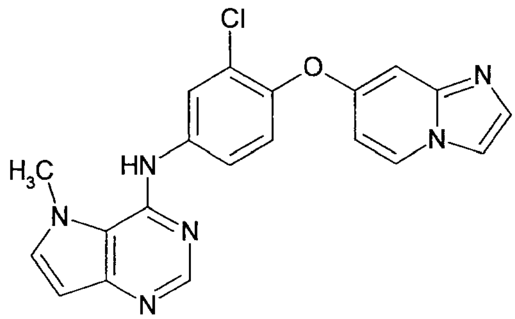

Production of N-[3-chloro-4-(imidazo[1,2-a]pyridin-7-yloxy)phenyl]-5-methyl-5H-pyrrolo[3,2-d]pyrimidin-4-amine

(i) Production of 7-(benzyloxy)imidazo[1,2-a]pyridine

A mixture of 4-(benzyloxy)pyridin-2-amine (1.01 g), sodium hydrogen carbonate (1.68 g), water (40 mL) and 1,2-dichloroethane (40 mL) was stirred at room temperature, during which 40% aqueous chloroacetoaldehyde solution (2.31 g) was added. The mixture was stirred at room temperature for 2 hr, and the organic layer was separated. The aqueous layer was extracted twice with ethyl acetate. The combined organic layers were dried over anhydrous magnesium sulfate, and concentrated under reduced pressure. The residue was separated and purified by silica gel column chromatography (hexane:ethyl acetate=95:5→0:100) to give the title compound (913 mg) as a pale-brown solid. The solid was used for the next step without further purification.

(ii) Production of 7-(2-chloro-4-nitrophenoxy)imidazo[1,2-a]pyridine

A mixture of 7-(benzyloxy)imidazo[1,2-a]pyridine (913 mg), 10% palladium/activated carbon and methanol (15 mL) was stirred under hydrogen atmosphere (up to 1 pressure) at room temperature for 1 hr. The reaction mixture was filtered through celite. The celite was washed with methanol. The filtrate was concentrated under reduced pressure, N, N-dimethylformamide (5 mL), potassium carbonate (563 mg) and 2-chloro-1-fluoro-4-nitrobenzene (714 mg) were added to the residue, and the mixture was stirred at room temperature for 12 hr. The reaction mixture was partitioned between ethyl acetate and saturated aqueous sodium hydrogen carbonate solution, and the organic layer was dried over anhydrous magnesium sulfate and concentrated under reduced pressure. The residue was separated and purified by silica gel column chromatography (hexane:ethyl acetate=95:5→0:100) to give the title compound (1.33 g) as a yellow solid.
$^1$H-NMR ($CDCl_3$) δ: 6.69 (1H, dd, J= 2.4 Hz, 7.5 Hz), 7.10-7.16 (2H, m), 7.59 (1H, s), 7.63 (1H, d, J= 1.2 Hz), 8.12-8.19 (2H, m), 8.43 (1H, d, J= 2.7 Hz).

(iii) Production of 3-chloro-4-(imidazo[1,2-a]pyridin-7-yloxy)aniline

7-(2-Chloro-4-nitrophenoxy)imidazo[1,2-a]pyridine (754 mg), reduced iron (726 mg), 2N hydrochloric acid (5.2 mL) and ethanol (26 mL) were mixed, and the mixture was heated to 80°C and stirred for 3 hr. The reaction mixture was cooled to 50°C and filtered through celite. The celite was washed with ethanol. The filtrate was concentrated under reduced pressure, and the residue was partitioned between ethyl acetate and water. The organic layer was dried over anhydrous magnesium sulfate and concentrated under reduced pressure. The residue was separated and purified by silica gel column chromatography (ethyl acetate:methanol=100:0→80:20) to give the title compound (558 mg) as a pale-yellow solid.

$^1$H-NMR ($CDCl_3$)δ: 3.74 (2H, br s), 6.61 (1H, dd, J= 3.0 Hz, 8.7 Hz), 6.68-6.71 (2H, m), 6.80 (1H, d, J= 2.7 Hz), 6.98 (1H, d, J= 8.7 Hz), 7.46-7.50 (2H, m), 8.02 (1H, d, J= 8.1 Hz).

(iv) Production of N-[3-chloro-4-(imidazo[1,2-a]pyridin-7-yloxy)phenyl]-5-methyl-5H-pyrrolo[3,2-d]pyrimidin-4-amine

A mixture of 4-chloro-5-methyl-5H-pyrrolo[3,2-d]pyrimidine (84 mg), 3-chloro-4-(imidazo[1,2-a]pyridin-7-yloxy) aniline (130 mg), pyridine hydrochloride (116 mg) and isopropyl alcohol (5 mL) was stirred at 70°C for 5 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified successively by basic silica gel column chromatography (ethyl acetate:methanol=100:0→90:10) and silica gel column chromatography (ethyl acetate:methanol=100:0→85:15), and triturated with ethyl acetate/hexane to give the title compound (83 mg) as a white solid.
$^1$H-NMR (DMSO-$d_6$) δ: 4.16 (3H, s), 6.47 (1H, d, J= 3.0 Hz), 6.61 (1H, m), 6.83 (1H, dd, J= 2.7 Hz, 7.5 Hz), 7.34 (1H, d, J= 9.0 Hz), 7.46 (1H, d, J= 1.2 Hz), 7.61 (1H, d, J= 3.0 Hz), 7.72 (1H, dd, J= 2.4 Hz, 8.7 Hz), 7.86 (1H, m), 8.00 (1H, d, J= 2.4 Hz), 8.34 (1H, s), 8.56 (1H, d, J= 7.8 Hz), 8.65 (1H, br s).

Example 163

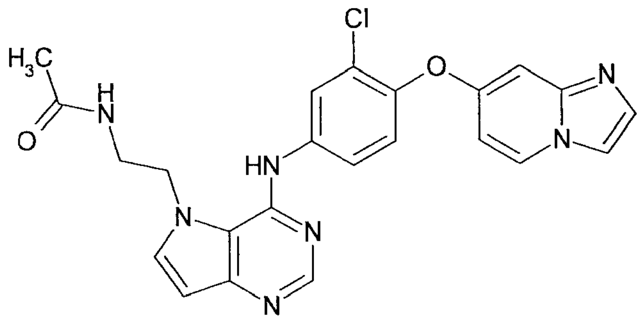

Production of N-[2-(4-{[3-chloro-4-(imidazo[1,2-a]pyridin-7-yloxy)phenyl]amino}-5H-pyrrolo[3,2-d]pyrimidin-5-yl)ethyl] acetamide

A mixture of tert-butyl [2-(4-chloro-5H-pyrrolo[3,2-d]pyrimidin-5-yl)ethyl]carbamate (489 mg), 3-chloro-4-(imidazo[1,2-a]pyridin-7-yloxy)aniline (428 mg), isopropyl alcohol (10 mL) and pyridine hydrochloride (231 mg) was stirred at 70°C for 6 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a purified product (326 mg). Methanol (5 mL) and 4N hydrochloric acid/ethyl acetate solution (2 mL) were added to the purified product, and the mixture was heated to 50°C and stirred for 1.5 hr. The reaction mixture was concentrated under reduced pressure, ethyl acetate was added, and the precipitate (296 mg) was collected by filtration. A mixture of the collected product, triethylamine (315 μL) and N,N-dimethylformamide (10 mL) were stirred at room temperature for 5 min. Acetic acid (50 mg), 1-hydroxybenzotriazole (114 mg) and 1-ethyl-3-(3-dimethylaminopropyl)carbodiimide hydrochloride (161 mg) were successively added, and the mixture was stirred at room temperature for 5 days. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→70:30), and crystallized from ethyl acetate to give the title compound (111 mg) as a pale-yellow solid.
$^1$H-NMR (DMSO-$d_6$) δ: 1.80 (3H, s), 3.32-3.37 (2H, m), 4.49-4.54 (2H, m), 6.52 (1H, d, J= 3.0 Hz), 6.62 (1H, d, J= 2.4 Hz), 6.83 (1H, dd, J= 2.4 Hz, 7.5 Hz), 7.34 (1H, d, J= 11.7 Hz), 7.46 (1H, d, J= 1.5 Hz), 7. 65 (1H, d, J= 3.3 Hz), 7.79 (1H, dd, J= 2.4 Hz, 9.0 Hz), 7.86 (1H, s), 8.06 (1H, d, J= 2.4 Hz), 8.27 (1H, m), 8.35 (1H, s), 8.57 (1H, d, J= 7.8 Hz), 8.84 (1H, br s).

Example 164

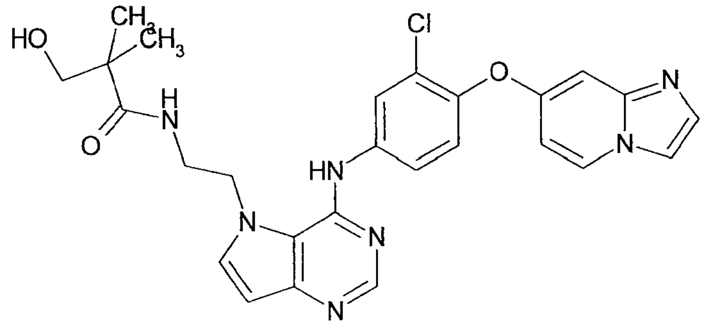

Production of N-[2-(4-{[3-chloro-4-(imidazo[1,2-a]pyridin-7-yloxy)phenyl]amino}-5H-pyrrolo[3,2-d]pyrimidin-5-yl)ethyl]-3-hydroxy-2,2-dimethylpropanamide

A mixture of tert-butyl [2-(4-chloro-5H-pyrrolo[3,2-d]pyrimidin-5-yl)ethyl]carbamate (162 mg), 3-chloro-4-(imidazo[1,2-a]pyridin-7-yloxy)aniline (142 mg), isopropyl alcohol (5 mL) and pyridine hydrochloride (115 mg) was stirred at 70°C for 6 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a purified product. Methanol (5 mL) and 4N hydrochloric acid/ethyl acetate solution (2 mL) were added to the purified product, and the mixture was heated to 50°C and stirred for 1.5 hr. The reaction mixture was concentrated under reduced pressure, ethyl acetate was added, and the precipitate (129 mg) was collected by filtration. A mixture of the collected product, triethylamine (142 μL) and N,N-dimethylformamide (5 mL) was stirred at room temperature for 5 min. 3-Hydroxy-2,2-dimethylpropanoic acid (35 mg), 1-hydroxybenzotriazole (41 mg) and 1-ethyl-3-(3-dimethylaminopropyl)carbodiimide hydrochloride (58 mg) were successively added, and the mixture was stirred at room temperature for 5 days. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→70:30), and crystallized from ethyl acetate to give the title compound (47 mg) as a white solid. $^1$H-NMR (DMSO-$d_6$) δ: 0.98 (6H, s), 3.30 (2H, m), 3.36-3.43 (2H, m), 4.50-4.52 (2H, m), 4.84 (1H, t, J= 5.4 Hz), 6.49 (1H, d, J= 3.3 Hz), 6.62 (1H, d, J= 2.4 Hz), 6.83 (1H, dd, J= 2.7 Hz, 7.5 Hz), 7.34 (1H, d, J= 8.7 Hz), 7.46 (1H, d, J= 1.2 Hz), 7.61 (1H, d, J= 3.3 Hz), 7.86-7.92 (3H, m), 8.12 (1H, d, J= 2.7 Hz), 8.35 (1H, s), 8.57 (1H, d, J= 7.5 Hz), 8.94 (1H, br s).

Example 165

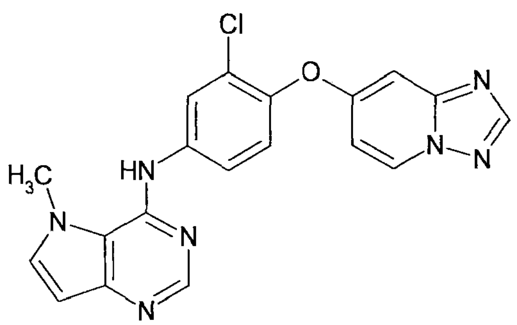

Production of N-[3-chloro-4-([1,2,4]triazolo[1,5-a]pyridin-7-yloxy)phenyl]-5-methyl-5H-pyrrolo[3,2-d]pyrimidin-4-amine

(i) Production of [1,2,4]triazolo[1,5-a]pyridin-7-ol

A mixture of 4-(benzyloxy)pyridin-2-amine (1.01 g), N,N-dimethylformamide dimethylacetal (0.67 mL) and ethanol (10 mL) was heated to 80°C and stirred for 1.5 hr. The reaction mixture was concentrated under reduced pressure, and the residue was dissolved in methanol (5 mL), and ice-cooled to 0°C. Pyridine (0.81 mL) and hydroxylamine-O-sulfonic acid (622 mg) were added, and the mixture was stirred at room temperature overnight. The reaction mixture was concentrated under reduced pressure, and partitioned between ethyl acetate/tetrahydrofuran (2:1) and saturated aqueous sodium hydrogen carbonate solution. The organic layer was dried over anhydrous magnesium sulfate and concentrated under reduced pressure. 10% Palladium/activated carbon (200 mg) and methanol (10 mL) were added to the residue, and the mixture was stirred under hydrogen atmosphere (up to 1 pressure) at room temperature for 1 hr. The reaction mixture was filtered through celite. The celite was washed with methanol. The filtrate was concentrated under reduced pressure, and the residue was separated and purified by silica gel column chromatography (hexane:ethyl acetate=95:5→0:100) to give the title compound (155 mg) as a white solid.
$^1$H-NMR (DMSO-$d_6$) δ: 6.73 (1H, dd, J= 2.1 Hz, 7.2 Hz), 6.89 (1H, m), 8.24 (1H, s), 8.70 (1H, dd, J= 0.6 Hz, 7.2 Hz), 10.83 (1H, br s).

(ii) Production of 3-chloro-4-([1,2,4]triazolo[1,5-a]pyridin-7-yloxy) aniline

N,N-Dimethylformamide (10 mL), potassium carbonate (159 mg) and 2-chloro-1-fluoro-4-nitrobenzene (202 mg) were added to [1,2,4]triazolo[1,5-a]pyridin-7-ol (155 mg), and the mixture was stirred at room temperature overnight. The reaction mixture was partitioned between ethyl acetate and saturated aqueous sodium hydrogen carbonate solution, and the organic layer was dried over anhydrous magnesium sulfate and concentrated under reduced pressure. Reduced iron (321 mg), 1N hydrochloric acid (3.0 mL) and ethanol (15 mL) were added to the residue, and the mixture was heated to 75°C and stirred for 5 hr. The reaction mixture was cooled to 50°C, 8N sodium hydroxide (0.4 mL) was added, and the mixture was filtered through celite. The celite was washed with ethanol and the filtrate was concentrated under reduced pressure. The residue was partitioned between ethyl acetate and saturated aqueous sodium hydrogen carbonate solution. The organic layer was dried over anhydrous magnesium sulfate and concentrated under reduced pressure. The residue was separated and purified by silica gel column chromatography (hexane:ethyl acetate=95:5→0:100) to give the title compound (186 mg) as a white solid.
$^1$H-NMR ($CDCl_3$)δ: 5.50 (2H, br s), 6.62 (1H, dd, J= 2.7 Hz, 8.7 Hz), 6.71 (1H, d, J= 1.8 Hz), 6.78 (1H, d, J= 2.7 Hz), 6.97 (1H, dd, J= 2.7 Hz, 7.5 Hz), 7.07 (1H, d, J= 8.7 Hz), 8.37 (1H, s), 8.89 (1H, d, J= 8.4 Hz).

(iii) Production of N-[3-chloro-4-([1,2,4]triazolo[1,5-a]pyridin-7-yloxy)phenyl]-5-methyl-5H-pyrrolo[3,2-d]pyrimidin-4-amine

A mixture of 4-chloro-5-methyl-5H-pyrrolo[3,2-d]pyrimidine (50 mg), 3-chloro-4-([1,2,4]triazolo[1,5-a]pyridin-7-yloxy)aniline (74 mg), pyridine hydrochloride (38 mg) and isopropyl alcohol (5 mL) was stirred at 70°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was triturated with ethyl acetate/methanol to give the title compound (79 mg) as a white solid.
$^1$H-NMR (DMSO-$d_6$) δ: 4.17 (3H, s), 6.48 (1H, d, J= 3.3 Hz), 6.90 (1H, d, J= 2.4 Hz), 7.06 (1H, dd, J= 3.0 Hz, 7.5 Hz), 7.42 (1H, d, J= 8.7 Hz), 7.63 (1H, d, J= 3.0 Hz), 7.76 (1H, dd, J= 2.7 Hz, 8.7 Hz), 8.03 (1H, d, J= 2.4 Hz), 8.35 (1H, s), 8.41 (1H, s), 8.69 (1H, br s), 8.96 (1H, d, J= 6.3 Hz).
Example 166
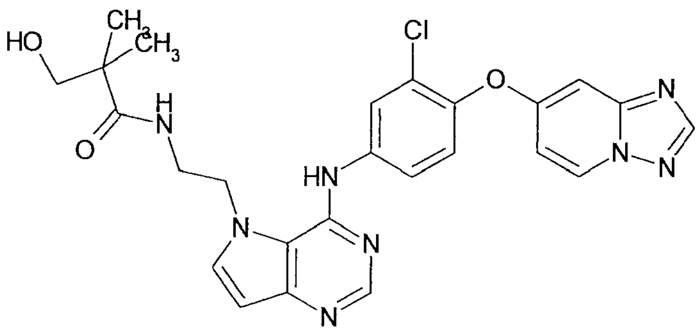

Production of N-[2-(4-{[3-chloro-4-([1,2,4]triazolo[1,5-a]pyridin-7-yloxy)phenyl]amino}-5H-pyrrolo[3,2-d]pyrimidin-5-yl) ethyl]-3-hydroxy-2,2-dimethylpropanamide

A mixture of tert-butyl [2-(4-chloro-5H-pyrrolo[3,2-d]pyrimidin-5-yl)ethyl]carbamate (122 mg), 3-chloro-4-([1,2,4]triazolo[1,5-a]pyridin-7-yloxy)aniline (108 mg), isopropyl alcohol (5 mL) and pyridine hydrochloride (51 mg) was stirred at 70°C overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give a purified product. Methanol (5 mL) and 4N hydrochloric acid/ethyl acetate solution (2 mL) were added to the purified product, and the mixture was stirred at room temperature for 4 hr. The reaction mixture was concentrated under reduced pressure, ethyl acetate was added, and the precipitate (139 mg) was collected by filtration. A mixture of the collected product, triethylamine (169 μL) and N,N-dimethylformamide (5 mL) was stirred at room temperature for 5 min. 3-Hydroxy-2,2-dimethylpropanoic acid (47 mg), 1-hydroxybenzotriazole (54 mg) and 1-ethyl-3-(3-dimethylaminopropyl)carbodiimide hydrochloride (77 mg) were successively added, and the mixture was stirred at room temperature for 4 hr. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→80:20), and crystallized from ethyl acetate to give the title compound (109 mg) as a white solid.
$^1$H-NMR (DMSO-$d_6$) δ: 0.99 (6H, s), 3.31-3.44 (4H, m), 4.50-4.54 (2H, m), 4.84 (1H, t, J= 5.1 Hz), 6.50 (1H, d, J= 3.0 Hz), 6.90 (1H, m), 7.06 (1H, dd, J= 2.7 Hz, 7.2 Hz), 7.42 (1H, d, J= 9.0 Hz), 7.62 (1H, d, J= 3.3 Hz), 7.85-7.88 (1H, m), 7.95 (1H, dd, J= 2.4 Hz, 9.0 Hz), 8.16 (1H, d, J= 2.7 Hz), 8.39 (1H, s), 8.41 (1H, s), 8.95-8.97 (2H, m).

Example 167

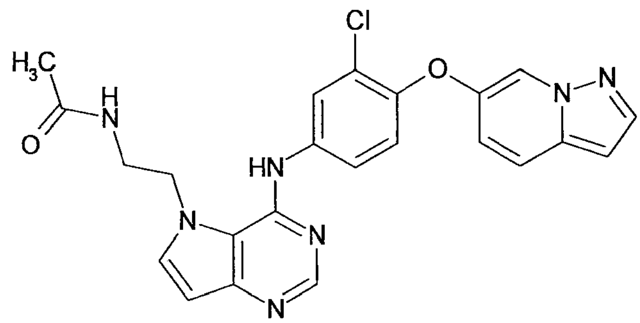

Production of N-[2-(4-{[3-chloro-4-(pyrazolo[1,5-a]pyridin-6-yloxy)phenyl]amino}-5H-pyrrolo[3,2-d]pyrimidin-5-yl)ethyl] acetamide

The title compound (29 mg) was obtained as a white solid by operation in the same manner as in Example 153 (ii) and using 5-(2-aminoethyl)-N-[3-chloro-4-(pyrazolo[1,5-a]pyridin-6-yloxy)phenyl]-5H-pyrrolo[3,2-d]pyrimidin-4-amine dihydrochloride (148 mg), triethylamine (84 μL), N,N-dimethylformamide (5 mL), 1-hydroxybenzotriazole (49 mg), 1-ethyl-3-(3-dimethylaminopropyl)carbodiimide hydrochloride (69 mg) and acetic acid (22 mg).
$^1$H-NMR (DMSO-$d_6$) δ: 1.79 (3H, s), 3.32-3.39 (2H, m), 4.50 (2H, m), 6.50 (1H, t, J= 3.3 Hz), 6.66-6.67 (1H, m), 7.14-7.22 (2H, m), 7.63-7.71 (2H, m), 7.77 (1H, d, J= 9.0 Hz), 7.97-8.01 (2H, m), 8.25-8.32 (2H, m), 8.44 (1H, m), 8.78 (1H, br s).

Example 168

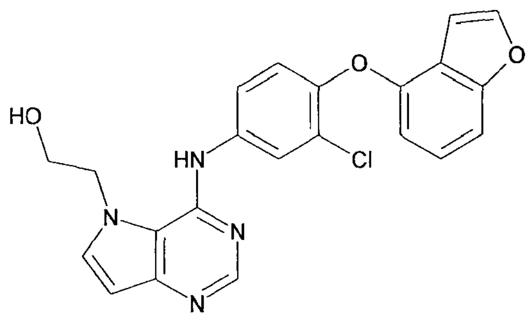

Production of 2-(4-{[4-(1-benzofuran-4-yloxy)-3-chlorophenyl]amino}-5H-pyrrolo[3,2-d]pyrimidin-5-yl)ethanol

(i) Production of 4-(2-chloro-4-nitrophenoxy)-1-benzofuran

A mixture of 1-benzofuran-4-ol (10.5 g), 2-chloro-1-fluoro-4-nitrobenzene (16.5 g), potassium carbonate (16.1 g) and N,N-dimethylformamide (150 mL) was stirred at room temperature for 3 hr. Water was added to the reaction mixture, and the mixture was extracted with ethyl acetate. The organic layer was washed successively with water and saturated brine, dried over anhydrous magnesium sulfate, and concentrated under reduced pressure. The residue was separated and purified by silica gel column chromatography (eluent, hexane→hexane:ethyl acetate=9:1), and crystallized from hexane/diisopropyl ether to give the title compound (16.9 g) as pale-yellow crystals.
$^1$H-NMR ($CDCl_3$) δ: 6.60 (1H, dd, J = 0.8 Hz, 2.3 Hz), 6.84 (1H, d, J = 9.1 Hz), 6.96 (1H, dd, J = 0.8 Hz, 8.0 Hz), 7.34 (1H, t, J = 8.0 Hz), 7.41-7.51 (1H, m), 7.58-7.65 (1H, m), 8.02 (1H, dd, J = 2.7 Hz, 9.1 Hz), 8.42 (1H, d, J = 2.7 Hz).

(ii) Production of 4-(1-benzofuran-4-yloxy)-3-chloroaniline

1N Hydrochloric acid (7 mL) was added to a mixture of 4-(2-chloro-4-nitrophenoxy)-1-benzofuran (2.01 g), reduced iron (2.00 g) and ethanol (70 mL), and the mixture was stirred with heating under reflux for 15 hr. The reaction mixture was cooled to room temperature, 1N aqueous sodium hydroxide solution (8 mL) was added, and the mixture was filtered through celite. The filtrate was concentrated under reduced pressure, and ethyl acetate was added to the residue. This solution was washed successively with water and saturated brine, dried over anhydrous magnesium sulfate, and concentrated under reduced pressure. The residue was separated and purified by basic silica gel column chromatography (eluent, hexane:ethyl acetate=49:1→1:2) to give the title compound (1.75 g) as a purple oil.
$^1$H-NMR ($CDCl_3$) δ: 3.68 (2H, br s), 6.53 (1H, dd, J = 1.0 Hz, 8.0 Hz), 6.57 (1H, dd, J = 8.6 Hz, 2.7 Hz), 6.76 (1H, dd, J = 1.0 Hz, 2.2 Hz), 6.81 (1H, d, J = 2.7 Hz), 6.93 (1H, d, J = 8.6 Hz), 7.14 (1H, t, J = 8.0 Hz), 7.21 (1H, td, J = 1.0 Hz, 8.0 Hz), 7.55 (1H, d, J = 2.2 Hz).

(iii) Production of 2-(4-{[4-(1-benzofuran-4-yloxy)-3-chlorophenyl]amino}-5H-pyrrolo[3,2-d]pyrimidin-5-yl)ethanol

A mixture of 2-(4-chloro-5H-pyrrolo[3,2-d]pyrimidin-5-yl)ethyl benzoate (130 mg), 4-(1-benzofuran-4-yloxy)-3-chloroaniline (124 mg) and isopropyl alcohol (2 mL) was stirred at 80°C for 14 hr. The reaction mixture was cooled to room temperature, 1N aqueous sodium hydroxide solution (1.5 mL) was added, and the mixture was stirred at room temperature for 2 hr. Aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, hexane:ethyl acetate=1:1→ethyl acetate) and crystallized from diisopropyl ether/ethyl acetate to give the title compound (120 mg) as white crystals.
$^1$H-NMR (DMSO-$d_6$) δ: 3.88 (2H, t, J = 4.5 Hz), 4.54 (2H, t, J = 4.5 Hz), 6.31 (1H, br s), 6.51 (1H, d, J = 3.0 Hz), 6.58 (1H, dd, J = 0.9 Hz, 8.0 Hz), 6.86 (1H, dd, J = 0.9 Hz, 2.3 Hz), 7.21-7.30 (2H, m), 7.33-7.40 (1H, m), 7.59 (1H, dd, J = 2.6 Hz, 8. 9 Hz), 7. 66 (1H, d, J = 3. 0 Hz), 7.92-8.09 (2H, m), 8.34 (1H, s), 9.87 (1H, br s).
Example 169
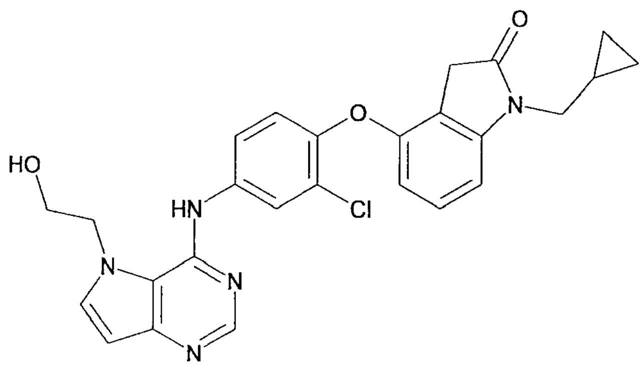

Production of 4-(2-chloro-4-{[5-(2-hydroxyethyl)-5H-pyrrolo[3,2-d]pyrimidin-4-yl]amino}phenoxy)-1-(cyclopropylmethyl)-1,3-dihydro-2H-indol-2-one

(i) Production of 4-(2-chloro-4-nitrophenoxy)-1-(cyclopropylmethyl)-1H-indole

A mixture of 4-(2-chloro-4-nitrophenoxy)-1H-indole (805 mg), (bromomethyl)cyclopropane (0.4 mL), potassium carbonate (691 mg) and N,N-dimethylformamide (10 mL) was stirred at room temperature for 3 days. Saturated aqueous ammonium chloride solution was added to the reaction mixture, and the mixture was extracted with ethyl acetate. The organic layer was washed successively with water and saturated brine, dried over anhydrous magnesium sulfate, and concentrated under reduced pressure. The residue was separated and purified by silica gel column chromatography (eluent, hexane:ethyl acetate=49:1→4:1) to give the title compound (748 mg) as a yellow powder.
$^1$H-NMR ($CDCl_3$) δ: 0.34-0.44 (2H, m), 0.61-0.73 (2H, m), 1.22-1.38 (1H, m), 4.01 (2H, d, J = 6.8 Hz), 6.25 (1H, d, J = 3.4 Hz), 6.80 (1H, d, J = 9.1 Hz), 6.82-6.88 (1H, m), 7.16-7.35 (3H, m), 7.96 (1H, dd, J = 2.7Hz, 9.1 Hz), 8.40 (1H, d, J = 2.7 Hz).

(ii) Production of 3-bromo-4-(2-chloro-4-nitrophenoxy)-1-(cyclopropylmethyl)-1H-indole

To a solution of 4-(2-chloro-4-nitrophenoxy)-1-(cyclopropylmethyl)-1H-indole (742 mg) in a mixed solvent of tert-butanol (25 mL) and tetrahydrofuran (2.5 mL) was added N-bromosuccinimide (439 mg), and the mixture was stirred at room temperature for 1 hr. Saturated aqueous sodium hydrogen carbonate solution was added to the reaction mixture, and the mixture was extracted with ethyl acetate. The organic layer was washed successively with water and saturated brine, dried over anhydrous magnesium sulfate, and concentrated under reduced pressure. The residue was separated and purified by silica gel column chromatography (eluent, hexane:ethyl acetate=19:1→2:1) to give the title compound (611 mg) as a yellow powder.
$^1$H-NMR ($CDCl_3$) δ: 0.31-0.38 (2H, m), 0.62-0.80 (2H, m), 1.18-1.39 (1H, m), 3.98 (2H, d, J = 7.0 Hz), 6.61 (1H, d, J = 9.0 Hz), 6.80-6.93 (1H, m), 7.17-7.41 (3H, m), 7.97 (1H, dd, J = 2.6 Hz, 9.0 Hz), 8.40 (1H, d, J = 2.6 Hz).

(iii) Production of 4-(2-chloro-4-nitrophenoxy)-1-(cyclopropylmethyl)-1,3-dihydro-2H-indol-2-one

To a solution of 3-bromo-4-(2-chloro-4-nitrophenoxy)-1-(cyclopropylmethyl)-1H-indole (298 mg) in tetrahydrofuran (4 mL) was added 6N hydrochloric acid (1 mL), and the mixture was stirred at 70°C for 23 hr. Saturated aqueous sodium hydrogen carbonate solution was added to the reaction mixture, and the mixture was extracted with a mixed solvent of ethyl acetate/tetrahydrofuran. The organic layer was washed with saturated brine, dried over anhydrous magnesium sulfate, and concentrated under reduced pressure. The precipitate was collected by filtration to give the title compound (215 mg) as a white powder.
$^1$H-NMR ($CDCl_3$) δ: 0.37-0.46 (2H, m), 0.51-0.62 (2H, m), 1.09-1.31 (1H, m), 3.41 (2H, s), 3.62 (2H, d, J = 7.2 Hz), 6.67 (1H, d, J = 8.0 Hz), 6.82 (1H, d, J = 8.0 Hz), 6.96 (1H, d, J = 9.1 Hz), 7.33 (1H, t, J = 8.0 Hz), 8.08 (1H, dd, J = 2.5 Hz, 9.1 Hz), 8.40 (1H, d, J = 2.5 Hz).

(iv) Production of 4-(4-amino-2-chlorophenoxy)-1-(cyclopropylmethyl)-1,3-dihydro-2H-indol-2-one

To a solution of 4-(2-chloro-4-nitrophenoxy)-1-(cyclopropylmethyl)-1,3-dihydro-2H-indol-2-one (213 mg) in a mixed solvent of methanol (7 mL) and tetrahydrofuran (7 mL) was added 5% platinum/activated carbon (21.8 mg) was added, and the mixture was stirred at room temperature for 3.5 hr under hydrogen atmosphere. The reaction mixture was filtered through celite, and the filtrate was concentrated under reduced pressure. The precipitate was collected by filtration to give the title compound (162 mg) as a brown powder.
$^1$H-NMR ($CDCl_3$) δ: 0.34-0.44 (2H, m), 0.48-0.59 (2H, m), 1.08-1.29 (1H, m), 3.45 (2H, s), 3.59 (2H, d, J = 6.8 Hz), 3.68 (2H, s), 6.40 (1H, d, J = 8.0 Hz), 6.56 (1H, dd, J = 2.6 Hz, 8.7 Hz), 6.62 (1H, d, J = 8.0 Hz), 6.78 (1H, d, J = 2.6 Hz), 6.91 (1H, d, J = 8.7 Hz), 7.16 (1H, t, J = 8.0 Hz).

(v) Production of 4-(2-chloro-4-{[5-(2-hydroxyethyl)-5H-pyrrolo[3,2-d]pyrimidin-4-yl]amino}phenoxy)-1-(cyclopropylmethyl)-1,3-dihydro-2H-indol-2-one

A mixture of 2-(4-chloro-5H-pyrrolo[3,2-d]pyrimidin-5-yl)ethyl benzoate (129 mg), 4-(4-amino-2-chlorophenoxy)-1-(cyclopropylmethyl)-1,3-dihydro-2H-indol-2-one (159 mg), pyridine hydrochloride (3 mg) and isopropyl alcohol (5 mL) was stirred at 70°C for 22 hr. Saturated aqueous sodium hydrogen carbonate solution was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:2→ethyl acetate→ethyl acetate:methanol=19:1) to give 2-{4-[(3-chloro-4-{[1-(cyclopropylmethyl)-2-oxo-2,3-dihydro-1H-indol-4-yl]oxy}phenyl)amino]-5H-pyrrolo[3,2-d]pyrimidin-5-yl}ethyl benzoate (236 mg). The obtained compound was dissolved in a mixed solvent of tetrahydrofuran (4 mL) and isopropyl

alcohol (2 mL), and potassium carbonate (61.6 mg) was added. Methanol (0.4 mL) was added dropwise to the reaction mixture, and the mixture was stirred at room temperature for 3 hr. Saturated aqueous ammonium chloride solution was added to the reaction mixture, and the mixture was extracted with a mixed solvent of ethyl acetate/tetrahydrofuran. The organic layer was washed with saturated brine, dried over anhydrous magnesium sulfate, and concentrated under reduced pressure. The residue was separated and purified by silica gel column chromatography (eluent, hexane:ethyl acetate=1:9→ethyl acetate→ethyl acetate:methanol=9:1) to give the title compound (62.8 mg) as pale-yellow crystals.
$^1$H-NMR (DMSO-$d_6$) δ: 0.30-0.39 (2H, m), 0.42-0.53 (2H, m), 1.06-1.24 (1H, m), 3.50 (2H, s), 3.57 (2H, d, J = 7.0 Hz), 3.87 (2H, t, J = 4.5 Hz), 4.53 (2H, t, J = 4.5 Hz), 6.30 (1H, br s), 6.36 (1H, d, J = 8.0 Hz), 6.51 (1H, d, J = 3.0 Hz), 6.88 (1H, d, J = 8.0 Hz), 7.18-7.30 (2H, m), 7.58 (1H, dd, J = 2.5 Hz, 8.9 Hz), 7.66 (1H, d, J = 3.0 Hz), 7.97 (1H, d, J = 2.5 Hz), 8.33 (1H, s), 9.86 (1H, br s).

Example 170

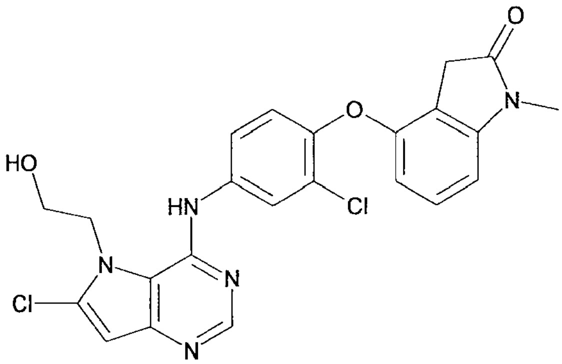

Production of 4-(2-chloro-4-{[6-chloro-5-(2-hydroxyethyl)-5H-pyrrolo[3,2-d]pyrimidin-4-yl]amino}phenoxy)-1-methyl-1,3-dihydro-2H-indol-2-one

A mixture of 5-(2-{[tert-butyl(dimethyl)silyl]oxy}ethyl)-4,6-dichloro-5H-pyrrolo[3,2-d]pyrimidine (129 mg), 4-(4-amino-2-chlorophenoxy)-1-methyl-1,3-dihydro-2H-indol-2-one (114 mg), pyridine hydrochloride (3 mg) and isopropyl alcohol (5 mL) was stirred at 70°C for 23 hr. Saturated aqueous sodium hydrogen carbonate solution was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=2:1→1:4) to give 4-(4-{[5-(2-{[tert-butyl(dimethyl)silyl]oxy}ethyl)-6-chloro-5H-pyrrolo[3,2-d]pyrimidin-4-yl]amino}-2-chlorophenoxy)-1-methyl-1,3-dihydro-2H-indol-2-one. The obtained compound was dissolved in tetrahydrofuran (4 mL), a solution (0.38 mL) of 1N tetrabutylammonium fluoride in tetrahydrofuran was added dropwise, and the mixture was stirred at room temperature for 1 hr. Saturated aqueous ammonium chloride solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, hexane:ethyl acetate=1:1→ethyl acetate) to give the title compound (68.8 mg) as orange crystals.
$^1$H-NMR (DMSO-$d_6$) δ: 3.14 (3H, s), 3.46 (2H, s), 3.84-3.94 (2H, m), 4.54 (2H, t, J = 4.4 Hz), 6.40 (1H, d, J = 8.0 Hz), 6.48 (1H, t, J = 4.4 Hz), 6.73 (1H, s), 6.78 (1H, d, J = 8.0 Hz), 7.14-7.32 (2H, m), 7.57 (1H, dd, J = 2.5 Hz, 8.9 Hz), 7.96 (1H, d, J = 2.5 Hz), 8.37 (1H, s), 9.93 (1H, s).

Example 171

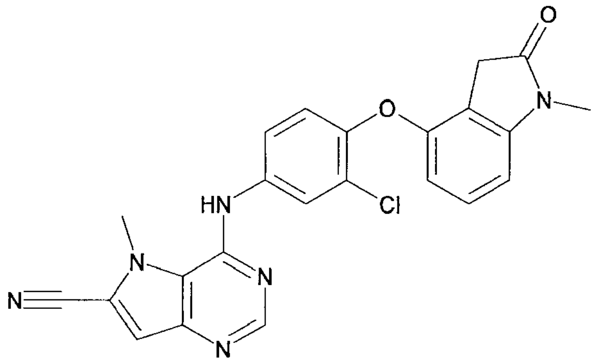

Production of 4-({3-chloro-4-[(1-methyl-2-oxo-2,3-dihydro-1H-indol-4-yl)oxy]phenyl}amino)-5-methyl-5H-pyrrolo[3,2-d]pyrimidine-6-carbonitrile

A mixture of 4-chloro-5-methyl-5H-pyrrolo[3,2-d]pyrimidine-6-carbonitrile (59.7 mg), 4-(4-amino-2-chlorophenoxy)-1-methyl-1,3-dihydro-2H-indol-2-one (93.3 mg), pyridine hydrochloride (3 mg), isopropyl alcohol (4 mL) and 1-methyl-2-pyrrolidone (2 mL) was stirred at 80°C for 5 hr. Saturated aqueous sodium hydrogen carbonate solution was added to the reaction mixture, and the mixture was extracted with a mixed solvent of ethyl acetate/tetrahydrofuran. The organic layer was washed successively with water and saturated brine, dried over anhydrous magnesium sulfate, and concentrated under reduced pressure. The residue was separated and purified by silica gel column chromatography (eluent, hexane:ethyl acetate=2:1→ethyl acetate) to give the title compound (108 mg) as white crystals.
$^1$H-NMR ($CDCl_3$) δ: 3.23 (3H, s), 3.45 (2H, s), 4.30 (3H, s), 6.55 (1H, d, J = 8.0 Hz), 6.62 (1H, d, J = 8.0 Hz), 6.88 (1H, s), 7.07 (1H, d, J = 8.8 Hz), 7.18-7.28 (2H, m), 7.46 (1H, dd, J = 2.8 Hz, 8.8 Hz), 7.83 (1H, d, J = 2.8 Hz), 8.57 (1H, s).
Example 172
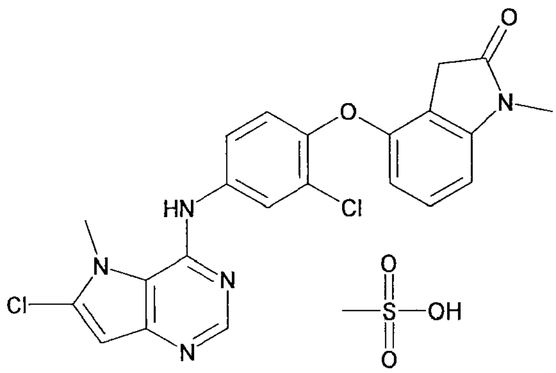

Production of 4-{2-chloro-4-[(6-chloro-5-methyl-5H-pyrrolo[3,2-d]pyrimidin-4-yl)amino]phenoxy}-1-methyl-1,3-dihydro-2H-indol-2-one methanesulfonate

4-{2-Chloro-4-[(6-chloro-5-methyl-5H-pyrrolo[3,2-d]pyrimidin-4-yl)amino]phenoxy}-1-methyl-1,3-dihydro-2H-indol-2-one was obtained by reaction in the same manner as in Example 171 and using 4,6-dichloro-5-methyl-5H-pyrrolo[3,2-d]pyrimidine (80 mg), 4-(4-amino-2-chlorophenoxy)-1-methyl-1,3-dihydro-2H-indol-2-one (120 mg), pyridine hydrochloride (3 mg) and isopropyl alcohol (5 mL). The obtained compound was dissolved in a mixed solvent of tetrahydrofuran (4 mL), ethanol (2 mL) and ethyl acetate (2 mL), and methanesulfonic acid (30 μL) were added at 70°C, and the mixture was stirred at room temperature for 4 hr. The reaction mixture was concentrated under reduced pressure, and the precipitate was collected by filtration to give the title compound (125 mg) as white crystals.
$^1$H-NMR (DMSO-$d_6$) δ: 2.30 (3H, s), 3.14 (3H, s), 3.44 (2H, s), 4.13 (3H, s), 6.48 (1H, d, J = 8.0 Hz), 6.83 (1H, d, J = 8.0 Hz), 6.89 (1H, s), 7.22 (1H, d, J = 8.8 Hz), 7.30 (1H, t, J = 8.0 Hz), 7.54 (1H, dd, J = 2.6 Hz, 8.8 Hz), 7.84 (1H, d, J = 2.6 Hz), 8.65 (1H, s), 9.70 (1H, br s).
Example 173
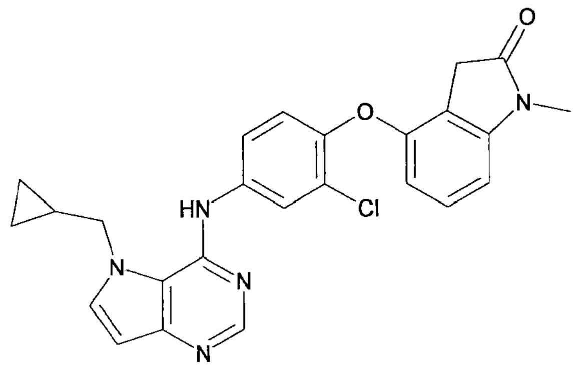

Production of 4-(2-chloro-4-{[5-(cyclopropylmethyl)-5H-pyrrolo[3,2-d]pyrimidin-4-yl]amino}phenoxy)-1-methyl-1,3-dihydro-2H-indol-2-one

(i) Production of 4-chloro-5-(cyclopropylmethyl)-5H-pyrrolo[3,2-d]pyrimidine

A mixture of 4-chloro-5H-pyrrolo[3,2-d]pyrimidine (2.54 g), (bromomethyl)cyclopropane (2 mL), cesium carbonate (8.11 g) and N,N-dimethylformamide (50 mL) was stirred at room temperature for 2 hr. Water was added to the reaction mixture, and the mixture was extracted with ethyl acetate. The organic layer was washed successively with water and saturated brine, dried over anhydrous magnesium sulfate, and concentrated under reduced pressure. The residue was separated and purified by silica gel column chromatography (eluent, hexane:ethyl acetate=19:1→1:1) to give the title compound (2.50 g) as orange crystals.
$^1$H-NMR ($CDCl_3$) δ: 0.33-0.50 (2H, m), 0.59-0.77 (2H, m), 1.23-1.47 (1H, m), 4.37 (2H, d, J = 6.9 Hz), 6.73 (1H, d, J = 3.0 Hz), 7.60 (1H, d, J = 3.0 Hz), 8.69 (1H, s).

(ii) Production of 4-(2-chloro-4-{[5-(cyclopropylmethyl)-5H-pyrrolo[3,2-d]pyrimidin-4-yl]amino}phenoxy)-1-methyl-1,3-dihydro-2H-indol-2-one

The title compound (94.6 mg) was obtained as pale-yellow crystals by reaction in the same manner as in Example 171 and using 4-chloro-5-(cyclopropylmethyl)-5H-pyrrolo[3,2-d]pyrimidine (60.0 mg), 4-(4-amino-2-chlorophenoxy)-1-methyl-1,3-dihydro-2H-indol-2-one (88.3 mg), pyridine hydrochloride (2 mg) and isopropyl alcohol (4 mL). $^1$H-NMR (DMSO-$d_6$) δ: 0.28-0.40 (2H, m), 0.41-0.53 (2H, m), 1.09-1.26 (1H, m), 3.14 (3H, s), 3.47 (2H, s), 4.39 (2H, d, J = 7.2 Hz), 6.42 (1H, d, J = 8.0 Hz), 6.50 (1H, d, J = 3.0 Hz), 6.78 (1H, d, J = 8.0 Hz), 7.18 (1H, d, J = 8.9 Hz), 7.26 (1H, t, J = 8.0 Hz), 7.58 (1H, dd, J = 2.1 Hz, 8.9 Hz), 7.74 (1H, d, J = 3.0 Hz), 7.88 (1H, d, J = 2.1 Hz), 8.33 (1H, s), 8.65 (1H, s).
Example 174
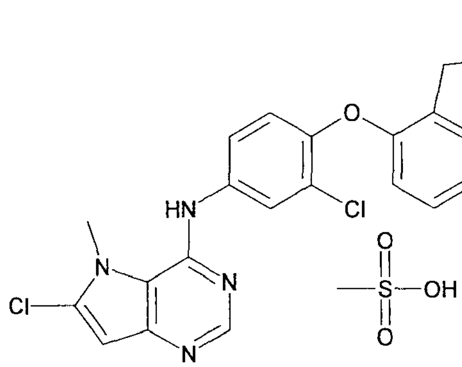

Production of 4-{2-chloro-4-[(6-chloro-5-methyl-5H-pyrrolo[3,2-d]pyrimidin-4-yl)amino]phenoxy}-1,3-dihydro-2H-indol-2-one methanesulfonate

The title compound (142 mg) was obtained as pale-orange crystals by reaction in the same manner as in

Example 172 and using 4,6-dichloro-5-methyl-5H-pyrrolo[3,2-d]pyrimidine (80.5 mg), 4-(4-amino-2-chlorophenoxy)-1,3-dihydro-2H-indol-2-one (113 mg), pyridine hydrochloride (3 mg), isopropyl alcohol (5 mL), methanesulfonic acid (30 μL), tetrahydrofuran (4 mL), ethanol (2 mL) and ethyl acetate (2 mL).
$^1$H-NMR (DMSO-$d_6$) δ: 2.31 (3H, s), 3.35 (2H, s), 4.14 (3H, s), 6.41 (1H, d, J = 8.0 Hz), 6.66 (1H, d, J = 8.0 Hz), 6.90 (1H, s), 7.20 (1H, t, J = 8.0 Hz), 7.22 (1H, d, J = 8.9 Hz), 7.54 (1H, dd, J = 2.6 Hz, 8.9 Hz), 7.84 (1H, d, J = 2.6 Hz), 8.67 (1H, s), 9.73 (1H, br s), 10.56 (1H, s).

Example 175

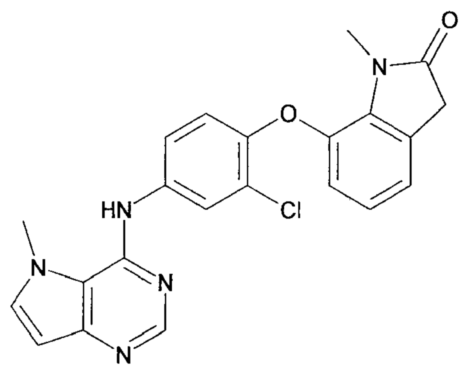

Production of 7-{2-chloro-4-[(5-methyl-5H-pyrrolo[3,2-d]pyrimidin-4-yl)amino]phenoxy}-1-methyl-1,3-dihydro-2H-indol-2-one

(i) Production of 7-(2-chloro-4-nitrophenoxy)-1H-indole

The title compound (3.08 g) was obtained as yellow crystals by reaction in the same manner as in Example 168(i) and using 2-chloro-1-fluoro-4-nitrobenzene (2.60 g), 7-hydroxyindole (2.05 g), potassium carbonate (3.05 g) and N,N-dimethylformamide (30 mL).
$^1$H-NMR ($CDCl_3$) δ: 6.62-6.66 (1H, m), 6.88 (1H, d, J = 8.0 Hz), 6.90 (1H, d, J = 9.1 Hz), 7.12 (1H, t, J = 8.0 Hz), 7.21-7.25 (1H, m), 7.56 (1H, d, J = 8.0 Hz), 8.00 (1H, dd, J = 2.8 Hz, 9.1 Hz), 8.25 (1H, br s), 8.41 (1H, d, J = 2.8 Hz).

(ii) Production of 7-(2-chloro-4-nitrophenoxy)-1-methyl-1H-indole

The title compound (1.00 g) was obtained as a yellow powder by reaction in the same manner as in Example 169(i) and using 7-(2-chloro-4-nitrophenoxy)-1H-indole (1.00 g), iodomethane (0.35 mL), potassium carbonate (814 mg) and N,N-dimethylformamide (12 mL).
$^1$H-NMR ($CDCl_3$) δ: 3.81 (3H, s), 6.52 (1H, d, J = 3.0 Hz), 6.78-6.87 (2H, m), 6.99 (1H, d, J = 3.0 Hz), 7.07 (1H, t, J = 8.0 Hz), 7.52 (1H, d, J = 8.0 Hz), 8.01 (1H, dd, J = 2.8 Hz, 9.1 Hz), 8.41 (1H, d, J = 2.8 Hz).

(iii) Production of 3-bromo-7-(2-chloro-4-nitrophenoxy)-1-methyl-1H-indole

The title compound (671 mg) was obtained as a brown oil by reaction in the same manner as in Example 169 (ii) and using 7-(2-chloro-4-nitrophenoxy)-1-methyl-1H-indole (995 mg), N-bromosuccinimide (647 mg), tert-butanol (30 mL) and tetrahydrofuran (10 mL).
$^1$H-NMR ($CDCl_3$) δ: 3.81 (3H, s), 6.82 (1H, d, J = 9.1 Hz), 6.88 (1H, d, J = 8.0 Hz), 7.03 (1H, s), 7.16 (1H, t, J = 8.0 Hz), 7.48 (1H, dd, J = 1.0 Hz, 8.0 Hz), 8.02 (1H, dd, J = 2.8 Hz, 9.1 Hz), 8.41 (1H, d, J = 2.8 Hz).

(iv) Production of 7-(2-chloro-4-nitrophenoxy)-1-methyl-1,3-dihydro-2H-indol-2-one

The title compound (308 mg) was obtained as an orange powder by reaction in the same manner as in Example 169(iii) and using 3-bromo-7-(2-chloro-4-nitrophenoxy)-1-methyl-1H-indole (668 mg), 6N hydrochloric acid (3 mL) and tetrahydrofuran (10 mL).
$^1$H-NMR ($CDCl_3$) δ: 3.28 (3H, s), 3.61 (2H, s), 6.87 (1H, d, J = 9.0 Hz), 6.91-6.96 (1H, m), 7.07 (1H, t, J = 7.7 Hz), 7.16-7.22 (1H, m), 8.08 (1H, dd, J = 2.6 Hz, 9.0 Hz), 8.42 (1H, d, J = 2.6 Hz).

(v) Production of 7-(4-amino-2-chlorophenoxy)-1-methyl-1,3-dihydro-2H-indol-2-one

The title compound (245 mg) was obtained as a violet powder by reaction in the same manner as in Example 169(iv) and using 7-(2-chloro-4-nitrophenoxy)-1-methyl-1,3-dihydro-2H-indol-2-one (303 mg), 5% platinum/activated carbon (31.4 mg), methanol (10 mL) and tetrahydrofuran (10 mL).
$^1$H-NMR ($CDCl_3$) δ: 3.51 (3H, s), 3.56 (2H, s), 3.67 (2H, br s), 6.56 (1H, dd, J = 2.6 Hz, 8.7 Hz), 6.60-6.67 (1H, m), 6.77-6.87 (2H, m), 6.90 (1H, d, J = 8.1 Hz), 6.93-6.99 (1H, m).

(vi) Production of 7-{2-chloro-4-[(5-methyl-5H-pyrrolo[3,2-d]pyrimidin-4-yl)amino]phenoxy}-1-methyl-1,3-dihydro-2H-indol-2-one

The title compound (73.1 mg) was obtained as pale-orange crystals by reaction in the same manner as in Example 171 and using 4-chloro-5-methyl-5H-pyrrolo[3,2-d]pyrimidine (41.7 mg), 7-(4-amino-2-chlorophenoxy)-1-methyl-1,3-dihydro-2H-indol-2-one (80.0 mg), pyridine hydrochloride (2 mg) and isopropyl alcohol (3 mL).
$^1$H-NMR (DMSO-$d_6$) δ: 3.32 (3H, s), 3.64 (2H, s), 4.14 (3H, s), 6.45 (1H, d, J = 3.0 Hz), 6.76 (1H, dd, J = 1.0 Hz, 8.1 Hz), 6.94-7.16 (3H, m), 7.54-7.64 (2H, m), 7.93 (1H, d, J = 2.3 Hz), 8.29 (1H, s), 8.54 (1H, s).
Example 176
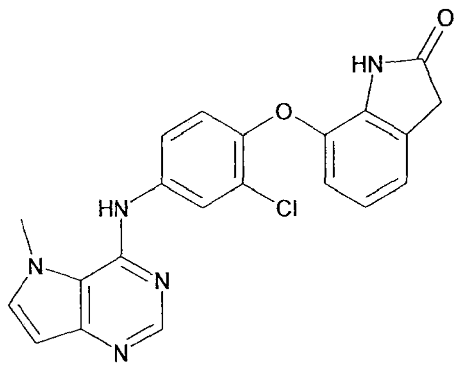

Production of 7-{2-chloro-4-[(5-methyl-5H-pyrrolo[3,2-d]pyrimidin-4-yl)amino]phenoxy}-1,3-dihydro-2H-indol-2-one

(i) Production of 3-bromo-7-(2-chloro-4-nitrophenoxy)-1H-indole

The title compound (530 mg) was obtained as a yellow powder by reaction in the same manner as in Example 169(ii) and using 7-(2-chloro-4-nitrophenoxy)-1H-indole (701 mg), N-bromosuccinimide (479 mg), tert-butanol (15 mL) and tetrahydrofuran (2.5 mL).
$^1$H-NMR ($CDCl_3$) δ: 6.88-6.96 (2H, m), 7.21 (1H, t, J = 8.0 Hz), 7.25-7.30 (1H, m), 7.51 (1H, d, J = 8.0 Hz), 8.03 (1H, dd, J = 2.6 Hz, 9.1 Hz), 8.35 (1H, br s), 8.42 (1H, d, J = 2.6 Hz).

(ii) Production of 7-(2-chloro-4-nitrophenoxy)-1,3-dihydro-2H-indol-2-one

The title compound (334 mg) was obtained as an orange powder by reaction in the same manner as in Example 169(iii) and using 3-bromo-7-(2-chloro-4-nitrophenoxy)-1H-indole (526 mg), 6N hydrochloric acid (3 mL) and tetrahydrofuran (10 mL).
$^1$H-NMR ($CDCl_3$) δ: 3.63 (2H, s), 6.88-6.98 (2H, m), 7.06 (1H, t, J = 7.8 Hz), 7.12-7.20 (1H, m), 7.60 (1H, br s), 8.08 (1H, dd, J = 2.8 Hz, 9.1 Hz), 8.41 (1H, d, J = 2.8 Hz).

(iii) Production of 7-(4-amino-2-chlorophenoxy)-1,3-dihydro-2H-indol-2-one

The title compound (276 mg) was obtained as a gray powder by reaction in the same manner as in Example 169(iv) and using 7-(2-chloro-4-nitrophenoxy)-1,3-dihydro-2H-indol-2-one (332 mg), 5% platinum/activated carbon (31.3

mg), methanol (10 mL) and tetrahydrofuran (10 mL).
$^1$H-NMR ($CDCl_3$) δ: 3.58 (2H, s), 3.69 (2H, br s), 6.49-6.64 (2H, m), 6.78 (1H, d, J = 2.8 Hz), 6.83-6.97 (3H, m), 7.61 (1H, br s).

(iv) Production of 7-{2-chloro-4-[(5-methyl-5H-pyrrolo[3,2-d]pyrimidin-4-yl)amino]phenoxy}-1,3-dihydro-2H-indol-2-one

The title compound (20.0 mg) was obtained as orange crystals by reaction in the same manner as in Example 171 and using 4-chloro-5-methyl-5H-pyrrolo[3,2-d]pyrimidine (43.7 mg), 7-(4-amino-2-chlorophenoxy)-1,3-dihydro-2H-indol-2-one (79.8 mg), pyridine hydrochloride (2 mg) and isopropyl alcohol (3 mL).
$^1$H-NMR (DMSO-$d_6$) δ: 3.57 (2H, s), 4.15 (3H, s), 6.45 (1H, d, J = 3.0 Hz), 6.57 (1H, d, J = 8.0 Hz), 6.89 (1H, t, J = 8.0 Hz), 6.96-7.04 (1H, m), 7.07 (1H, d, J = 9.1 Hz), 7.49-7.71 (2H, m), 7.92 (1H, d, J = 2.7 Hz), 8.30 (1H, s), 8.55 (1H, s), 10.77 (1H, s).
Example 177
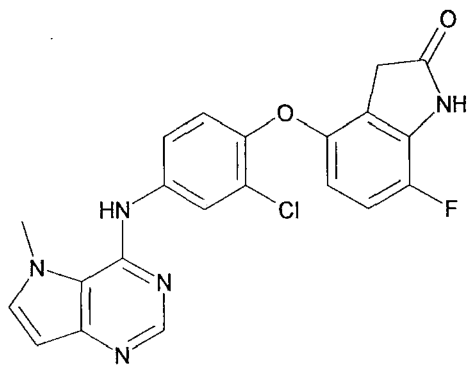

Production of 4-{2-chloro-4-[(5-methyl-5H-pyrrolo[3,2-d]pyrimidin-4-yl)amino]phenoxy}-7-fluoro-1,3-dihydro-2H-indol-2-one

(i) Production of 7-fluoro-4-methoxy-3-(methylthio)-1,3-dihydro-2H-indol-2-one

A solution of (methylthio)ethyl acetate (1.1 mL) in dichloromethane (30 mL) was cooled to -78°C, sulfuryl chloride (0.75 mL) was added dropwise, and the mixture was stirred at - 78°C for 20 min. A solution of 2-fluoro-5-methoxyaniline (1.18 g) and N,N,N',N'-tetramethyl-1,8-naphthalenediamine (1.60 g) in dichloromethane (15 mL) was added dropwise to this solution, and the mixture was stirred at -78°C for 20 min. Triethylamine (1.4 mL) was added, and the mixture was stirred at -78°C for 2 hr. Water was added to the reaction mixture, and the mixture was extracted with ethyl acetate. The organic layer was washed with saturated brine, dried over anhydrous magnesium sulfate, and concentrated under reduced pressure. The residue was dissolved in acetic acid (10 mL), and the solution was stood overnight. The mixture was concentrated under reduced pressure, and diethyl ether was added to the residue. This solution was washed successively with 1N hydrochloric acid and saturated brine, dried over anhydrous magnesium sulfate, and concentrated under reduced pressure. The residue was separated and purified by silica gel column chromatography (eluent, hexane: ethyl acetate=19:1→1:2) to give the title compound (774 mg) as a white powder.
$^1$H-NMR ($CDCl_3$) δ: 2.15 (3H, s), 3.88 (3H, s), 4.32 (1H, s), 6.51 (1H, dd, J = 3.3 Hz, 9.1 Hz), 7.00 (1H, t, J = 9.1 Hz), 7.67 (1H, br s).

(ii) Production of 7-fluoro-4-methoxy-1,3-dihydro-2H-indol-2-one

To a solution of 7-fluoro-4-methoxy-3-(methylthio)-1,3-dihydro-2H-indol-2-one (771 mg) in dichloromethane (20 mL) were added triphenylphosphine (1.05 g) and p-toluenesulfonic acid monohydrate (772 mg), and the mixture was stirred at room temperature for 14 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, hexane:ethyl acetate=19:1→1:2) to give the title compound (461 mg) as pale-yellow crystals.
$^1$H-NMR ($CDCl_3$) δ: 3.50 (2H, s), 3.82 (3H, s), 6.46 (1H, dd, J = 3.3 Hz, 9.4 Hz), 6.95 (1H, t, J = 9.4 Hz), 7.87 (1H, br s).

(iii) Production of 7-fluoro-4-hydroxy-1,3-dihydro-2H-indol-2-one

A mixture of 7-fluoro-4-methoxy-1,3-dihydro-2H-indol-2-one (457 mg) and 48% hydrobromic acid (5 mL) was stirred with heating under reflux for 2.5 hr. Water was added to the reaction mixture, and the mixture was extracted with a mixed solvent of ethyl acetate/tetrahydrofuran. The organic layer was washed successively with saturated aqueous sodium hydrogen carbonate solution and saturated brine, dried over anhydrous magnesium sulfate, and concentrated under reduced pressure. The residue was separated and purified by silica gel column chromatography (eluent, hexane: ethyl acetate=4:1→1:4) to give the title compound (205 mg) as a gray powder.
$^1$H-NMR ($CDCl_3$) δ: 3.36 (2H, s), 6.35 (1H, dd, J = 3.6 Hz, 9.0 Hz), 6.89 (1H, t, J = 9.0 Hz), 9.47 (1H, s), 10.72 (1H, s).

(iv) Production of 4-(2-chloro-4-nitrophenoxy)-7-fluoro-1,3-dihydro-2H-indol-2-one

The title compound (97.8 mg) was obtained as a pale-brown powder by reaction in the same manner as in Example 168(i) and using 7-fluoro-4-hydroxy-1,3-dihydro-2H-indol-2-one (203 mg), 2-chloro-1-fluoro-4-nitrobenzene (317 mg), potassium carbonate (181 mg) and N,N-dimethylformamide (3 mL).
$^1$H-NMR ($CDCl_3$) δ: 3.45 (2H, s), 6.63 (1H, dd, J = 3.6 Hz, 9.1 Hz), 6.91 (1H, d,J = 9.1 Hz), 7.07 (1H, t, J = 9.1 Hz), 7.70 (1H, br s), 8.09 (1H, dd, J = 2.8 Hz, 9.1 Hz), 8.40 (1H, d, J = 2.8 Hz).

(v) Production of 4-(4-amino-2-chlorophenoxy)-7-fluoro-1,3-dihydro-2H-indol-2-one

The title compound (55.7 mg) was obtained as a yellow powder by reaction in the same manner as in Example 169(iv) and using 4-(2-chloro-4-nitrophenoxy)-7-fluoro-1,3-dihydro-2H-indol-2-one (95.0 mg), 5% platinum/activated carbon (10.2 mg), methanol (5 mL) and tetrahydrofuran (1 mL).
$^1$H-NMR ($CDCl_3$) δ: 3.40 (2H, s), 5.33 (2H, s), 6.15 (1H, dd, J = 3.6 Hz, 9.1 Hz), 6.52 (1H, dd, J = 2.8 Hz, 8.7 Hz), 6.69 (1H, d, J = 2.8 Hz), 6.89 (1H, d, J = 8.7 Hz), 7.00 (1H, t, J = 9.1 Hz), 10.93 (1H, s).

(vi) Production of 4-{2-chloro-4-[(5-methyl-5H-pyrrolo[3,2-d]pyrimidin-4-yl)amino]phenoxy}-7-fluoro-1,3-dihydro-2H-indol-2-one

The title compound (38.7 mg) was obtained as orange crystals by reaction in the same manner as in Example 171 and using 4-chloro-5-methyl-5H-pyrrolo[3,2-d]pyrimidine (28.1 mg), 4-(4-amino-2-chlorophenoxy)-7-fluoro-1,3-dihydro-2H-indol-2-one (52.0 mg), pyridine hydrochloride (2 mg) and isopropyl alcohol (3 mL).
$^1$H-NMR (DMSO-$d_6$) δ: 3.46 (2H, s), 4.14 (3H, s), 6.34 (1H, dd, J = 3.6 Hz, 9.1 Hz), 6.44 (1H, d, J = 3.0 Hz), 7.08 (1H, t, J = 9.1 Hz), 7.14 (1H, d, J = 8.8 Hz), 7.55 -7.66 (2H, m), 7.92 (1H, d, J = 2.5 Hz), 8.29 (1H, s), 8.55 (1H, s), 11.01 (1H, br s).
Example 178
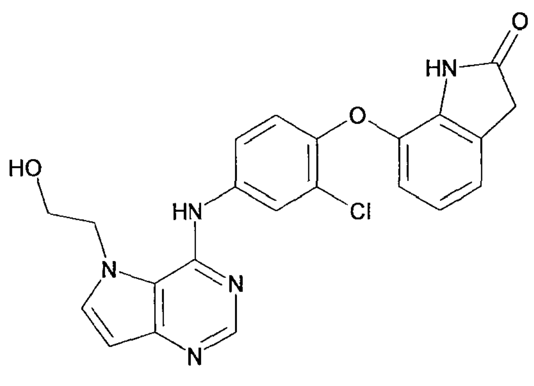

Production of 7-(2-chloro-4-{[5-(2-hydroxyethyl)-5H-pyrrolo[3,2-d]pyrimidin-4-yl]amino}phenoxy)-1,3-dihydro-2H-indol-2-one

The title compound (72.8 mg) was obtained as pale-yellow crystals by reaction in the same manner as in Example 169(v) and using 2-(4-chloro-5H-pyrrolo[3,2-d]pyrimidin-5-yl)ethyl benzoate (101 mg), 7-(4-amino-2-chlorophenoxy)-1,3-dihydro-2H-indol-2-one (101 mg), pyridine hydrochloride (3 mg), isopropyl alcohol (5 mL), potassium carbonate (50.3 mg), tetrahydrofuran (3 mL), isopropyl alcohol (1.5 mL) and methanol (0.3 mL).
$^1$H-NMR (DMSO-$d_6$) δ: 3.57 (2H, s), 3.87 (2H, t, J = 4.5 Hz), 4.53 (2H, t, J = 4.5 Hz), 6.29 (1H, br s), 6.50 (1H, d, J = 3.2 Hz), 6.54 (1H, dd, J = 0.9 Hz, 8.0 Hz), 6.82-6.93 (1H, m), 6.99 (1H, dd, J = 0.9 Hz, 8.0 Hz), 7.12 (1H, d, J = 8.9 Hz), 7.54 (1H, dd, J = 2.6 Hz, 8.9 Hz), 7.66 (1H, d, J = 3.2 Hz), 7.94 (1H, d, J = 2.6 Hz), 8.32 (1H, s), 9.82 (1H, br s), 10.77 (1H, s).

Example 179

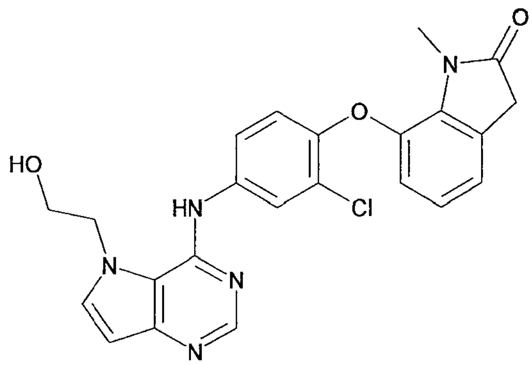

Production of 7-(2-chloro-4-{[5-(2-hydroxyethyl)-5H-pyrrolo[3,2-d]pyrimidin-4-yl]amino}phenoxy)-1-methyl-1,3-dihydro-2H-indol-2-one

The title compound (70.6 mg) was obtained as white crystals by reaction in the same manner as in Example 169(v) and using 2-(4-chloro-5H-pyrrolo[3,2-d]pyrimidin-5-yl)ethyl benzoate (100 mg), 7-(4-amino-2-chlorophenoxy)-1-methyl-1,3-dihydro-2H-indol-2-one (102 mg), pyridine hydrochloride (3 mg), isopropyl alcohol (5 mL), potassium carbonate (61.8 mg), tetrahydrofuran (3 mL), isopropyl alcohol (1.5 mL) and methanol (0.3 mL).
$^1$H-NMR (DMSO-$d_6$) δ: 3.33 (3H, s), 3.64 (2H, s), 3.86 (2H, t, J = 4.6 Hz), 4.52 (2H, t, J = 4.7 Hz), 6.26 (1H, br s), 6.49 (1H, d, J = 3.0 Hz), 6.69-6.77 (1H, m), 6.97 (1H, t, J = 7.7 Hz), 7.03-7.13 (2H, m), 7.52 (1H, dd, J = 2.6 Hz, 8.9 Hz), 7.64 (1H, d, J = 3.0 Hz), 7.93 (1H, d, J = 2.6 Hz), 8.30 (1H, s), 9.77 (1H, br s).

Example 180

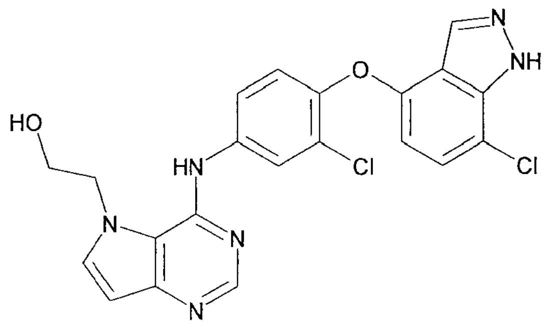

Production of 2-[4-({3-chloro-4-[(7-chloro-1H-indazol-4-yl)oxy]phenyl}amino)-5H-pyrrolo[3,2-d]pyrimidin-5-yl]ethanol

(i) Production of 7-chloro-4-methoxy-1H-indazole

To a solution of 3-chloro-2-fluoro-6-methoxybenzaldehyde (1.00 g) in pyridine (15 mL) was added hydrazine monohydrate (2.7 mL), and the mixture was stirred at 100°C for 13 hr. Water was added to the reaction mixture, and the mixture was extracted with ethyl acetate. The organic layer was washed successively with 1N hydrochloric acid and saturated brine, dried over anhydrous magnesium sulfate, and concentrated under reduced pressure. The residue was separated and purified by silica gel column chromatography (eluent, hexane:ethyl acetate=19:1→1:1) to give the title

compound (758 mg) as white crystals.
$^1$H-NMR ($CDCl_3$) δ: 3.96 (3H, s), 6.44 (1H, d, J = 8.0 Hz), 7.26 (1H, d, 8.0 Hz), 8.17 (1H, s), 10.14 (1H, br s).

(ii) Production of 7-chloro-1H-indazol-4-ol

A mixture of 7-chloro-4-methoxy-1H-indazole (657 mg) and 48% hydrobromic acid (10 mL) was stirred with heating under reflux for 1 day. Water was added to the reaction mixture, and the mixture was extracted with ethyl acetate. The organic layer was washed successively with saturated aqueous sodium hydrogen carbonate solution and saturated brine, dried over anhydrous magnesium sulfate, and concentrated under reduced pressure. The precipitate was collected by filtration to give the title compound (561 mg) as a brown powder.
$^1$H-NMR ($CDCl_3$) δ: 6.39 (1H, d, J = 7.9 Hz), 7.18 (1H, d, 7.9 Hz), 8.13 (1H, s), 10.35 (1H, br s), 13.35 (1H, br s).

(iii) Production of 7-chloro-4-(2-chloro-4-nitrophenoxy)-1H-indazole

The title compound (486 mg) was obtained as a yellow powder by reaction in the same manner as in Example 168(i) and using 7-chloro-1H-indazol-4-ol (620 mg), 2-chloro-1-fluoro-4-nitrobenzene (670 mg), potassium carbonate (603 mg) and N,N-dimethylformamide (8 mL).
$^1$H-NMR ($CDCl_3$) δ: 6.69 (1H, d, J = 8.3 Hz), 6.99 (1H, d, 9.1 Hz), 7.35 (1H, d, J = 8.3 Hz), 7.98 (1H, s), 8.07 (1H, dd, J = 2.5 Hz, 9.1 Hz), 8.43 (1H, d, J = 2.5 Hz), 10.38 (1H, br s).

(iv) Production of 3-chloro-4-[(7-chloro-1H-indazol-4-yl)oxy]aniline

To a mixture of 7-chloro-4-(2-chloro-4-nitrophenoxy)-1H-indazole (480 mg), ethanol (15 mL) and water (1.5 mL) were added calcium chloride (83.8 mg) and reduced iron (417 mg), and the mixture was stirred at 90°C for 13 hr. The reaction mixture was filtered through celite, and the filtrate was concentrated under reduced pressure. Ethyl acetate was added to the residue, and the mixture was washed successively with water and saturated brine, dried over anhydrous magnesium sulfate, and concentrated under reduced pressure. The residue was separated and purified by silica gel column chromatography (eluent, hexane:ethyl acetate=19:1→1:1) to give the title compound (310 mg) as a pale-yellow powder.
$^1$H-NMR ($CDCl_3$) δ: 3.74 (2H, s), 6.28 (1H, d, J = 8.3 Hz), 6.60 (1H, dd, J = 2.8 Hz, 8.8 Hz), 6.81 (1H, d, J = 2.8 Hz), 6.98 (1H, d, J = 8.8 Hz), 7.18 (1H, d, J = 8.3 Hz), 8.04 (1H, s), 10.19 (1H, br s).

(v) Production of 2-[4-({3-chloro-4-[(7-chloro-1H-indazol-4-yl)oxy]phenyl}amino)-5H-pyrrolo[3,2-d]pyrimidin-5-yl]ethanol

A mixture of 2-(4-chloro-5H-pyrrolo[3,2-d]pyrimidin-5-yl)ethyl benzoate (121 mg), 3-chloro-4-[(7-chloro-1H-indazol-4-yl)oxy]aniline (131 mg), pyridine hydrochloride (5 mg) and isopropyl alcohol (6 mL) was stirred at 80°C for 17.5 hr. The reaction mixture was cooled to room temperature, 1N aqueous sodium hydroxide solution (1.5 mL) was added, and the mixture was stirred at room temperature for 1 hr. Aqueous ammonium chloride solution was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:4→ethyl acetate→ethyl acetate:methanol=19:1) and crystallized from ethyl acetate to give the title compound (159 mg) as white crystals.
$^1$H-NMR (DMSO-$d_6$) δ: 3.88 (2H, t, J = 4.4 Hz), 4.55 (2H, t, J = 4.4 Hz), 6.29 (1H, d, J = 8.1 Hz), 6.36 (1H, br s), 6.52 (1H, d, J = 3.0 Hz), 7.28-7.40 (2H, m), 7.63 (1H, dd, J = 2.5 Hz, 8.9 Hz), 7.67 (1H, d, J = 3.0 Hz), 8.02 (1H, d, J = 2.5 Hz), 8.11 (1H, s), 8.35 (1H, s), 9.92 (1H, br s), 13.77 (1H, br s).
Example 181
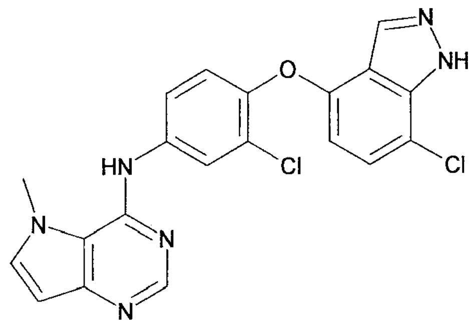

Production of N-{3-chloro-4-[(7-chloro-1H-indazol-4-yl)oxy]phenyl}-5-methyl-5H-pyrrolo[3,2-d]pyrimidin-4-amine

The title compound (69.4 mg) was obtained as pale-yellow crystals by reaction in the same manner as in Example 171 and using 4,6-dichloro-5-methyl-5H-pyrrolo[3,2-d]pyrimidine (40.8 mg), 3-chloro-4-[(7-chloro-1H-indazol-4-yl)oxy]aniline (77.7 mg), pyridine hydrochloride (1 mg) and isopropyl alcohol (3 mL).
$^1$H-NMR (DMSO-$d_6$) δ: 4.16 (3H, s), 6.30 (1H, d, J = 8.1 Hz), 6.47 (1H, d, J = 3.0 Hz), 7.27-7.39 (2H, m), 7.61 (1H, d, J = 3.0 Hz), 7.70 (1H, dd, J = 2.5 Hz, 8.9 Hz), 8.00 (1H, d, J = 2.5 Hz), 8.11 (1H, s), 8.33 (1H, s), 8.63 (1H, br s), 13.77 (1H, br s).
Example 182
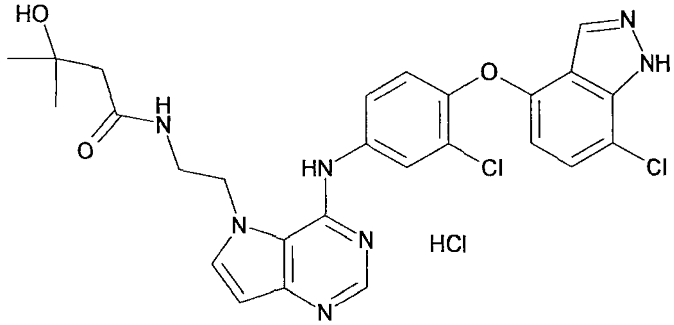

Production of N-{2-[4-({3-chloro-4-[(7-chloro-1H-indazol-4-yl)oxy]phenyl}amino)-5H-pyrrolo[3,2-d]pyrimidin-5-yl]ethyl}-3-hydroxy-3-methylbutanamide hydrochloride

(i) Production of tert-butyl {2-[4-({3-chloro-4-[(7-chloro-1H-indazol-4-yl)oxy]phenyl}amino)-5H-pyrrolo[3,2-d]pyrimidin-5-yl]ethyl}carbamate

A mixture of tert-butyl [2-(4-chloro-5H-pyrrolo[3,2-d]pyrimidin-5-yl)ethyl]carbamate (97.5 mg), 3-chloro-4-[(7-chloro-1H-indazol-4-yl)oxy]aniline (99.1 mg) and isopropyl alcohol (5 mL) was stirred at 80°C for 5 days. Saturated aqueous sodium hydrogen carbonate solution was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:1→ethyl acetate) to give the title compound (154 mg) as a pale-orange oil.
$^1$H-NMR ($CDCl_3$) δ: 1.50 (9H, s), 3.43-3.58 (2H, m), 4.42-4.55 (2H, m), 5.07 (1H, t, J = 5.6 Hz), 6.42 (1H, d, J = 8.1 Hz), 6.62 (1H, d, J = 3.2 Hz), 7.11-7.29 (3H, m), 7.93 (1H, dd, J = 2.5 Hz, 9.0 Hz), 8.07 (1H, d, J = 2.5 Hz), 8.13 (1H, s), 8.53 (1H, s), 8.64 (1H, s), 10.22 (1H, br s).

(ii) Production of 5-(2-aminoethyl)-N-{3-chloro-4-[(7-chloro-1H-indazol-4-yl)oxy]phenyl}-5H-pyrrolo[3,2-d]pyrimidin-4-amine dihydrochloride

To a solution of tert-butyl {2-[4-({3-chloro-4-[(7-chloro-1H-indazol-4-yl)oxy]phenyl}amino)-5H-pyrrolo[3,2-d]pyrimidin-5-yl]ethyl}carbamate (152 mg) in ethanol (2 mL) was added 6N hydrochloric acid (0.3 mL), and the mixture was

stirred at 50°C for 38 hr. The reaction mixture was concentrated under reduced pressure, ethanol was added to the residue, and the mixture was concentrated again under reduced pressure. The precipitate was collected by filtration to give the title compound (121 mg) as a white powder.
$^1$H-NMR (DMSO-$d_6$) δ: 3.22-3.36 (2H, m), 5.02 (2H, t, J = 5.9 Hz), 6.38 (1H, d, J = 8.1 Hz), 6.75 (1H, d, J = 3.0), 7.32-7.47 (2H, m), 7.65 (1H, dd, J = 2.4 Hz, 8.8 Hz), 7.89-8.15 (3H, m), 8.28 (3H, br s), 8.75 (1H, s), 10.12 (1H, br s), 13.84 (1H, br s).

(iii) Production of N-{2-[4-({3-chloro-4-[(7-chloro-1H-indazol-4-yl)oxy]phenyl}amino)-5H-pyrrolo[3,2-d]pyrimidin-5-yl] ethyl}-3-hydroxy-3-methylbutanamide hydrochloride

A mixture of 5-(2-aminoethyl)-N-{3-chloro-4-[(7-chloro-1H-indazol-4-yl)oxy]phenyl}-5H-pyrrolo[3,2-d]pyrimidin-4-amine dihydrochloride (111 mg), 3-hydroxy-3-methylbutanoic acid (30.8 mg), 1-hydroxybenzotriazole (43.4 mg), 1-ethyl-3-(3-dimethylaminopropyl)carbodiimide hydrochloride (70.9 mg), triethylamine (0.3 mL), tetrahydrofuran (0.6 mL) and N,N-dimethylformamide (0.6 mL) was stirred at room temperature for 18 hr. Water was added to the reaction mixture, and the mixture was extracted with ethyl acetate. The organic layer was dried over anhydrous magnesium sulfate and concentrated under reduced pressure. The residue was separated and purified by basic silica gel column chromatography (eluent, hexane:ethyl acetate=1:9→ethyl acetate→ethyl acetate:methanol=9:1) to give N-{2-[4-({3-chloro-4-[(7-chloro-1H-indazol-4-yl)oxy]phenyl}amino)-5H-pyrrolo[3,2-d]pyrimidin-5-yl]ethyl}-3-hydroxy-3-methylbutanamide. To a solution of the obtained compound in ethyl acetate (3 mL) was added 4N hydrochloric acid/ethyl acetate (0.3 mL), and the mixture was stirred at room temperature for 3 hr. The reaction mixture was concentrated under reduced pressure and the residue was crystallized from ethyl acetate/ethanol to give the title compound (74.0 mg) as pale-yellow crystals.
$^1$H-NMR (DMSO-$d_6$) δ: 1.12 (6H, s), 2.20 (2H, s), 3.44-3.56 (2H, m), 4.65 (2H, t, J = 7.4 Hz), 6.38 (1H, d,J = 8.1 Hz), 6.67 (1H, d, J = 3.0 Hz), 7.33-7.44 (2H, m), 7.72 (1H, dd, J = 2.5 Hz, 8.9 Hz), 7.93-8.03 (2H, m), 8.08 (1H, s), 8.37 (1H, t, J = 5.3 Hz), 8.73 (1H, s), 10.20 (1H, br s), 13.82 (1H, br s).
Example 183
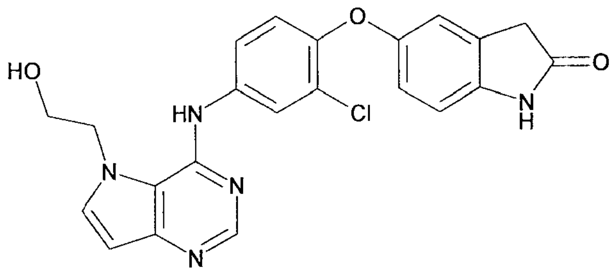

Production of 5-(2-chloro-4-{[5-(2-hydroxyethyl)-5H-pyrrolo[3,2-d]pyrimidin-4-yl]amino}phenoxy)-1,3-dihydro-2H-indol-2-one

(i) Production of 5-(2-chloro-4-nitrophenoxy)-1H-indole

The title compound (4.68 g) was obtained as yellow crystals by reaction in the same manner as in Example 168(i) and using 5-hydroxyindole (2.49 g), 2-chloro-1-fluoro-4-nitrobenzene (3.50 g), potassium carbonate (3.88 g) and N,N-dimethylformamide (30 mL).
$^1$H-NMR ($CDCl_3$) δ: 6.53-6.61 (1H, m), 6.79 (1H, d, J = 9.1 Hz), 6.96 (1H, dd, J = 2.3 Hz, 8.7 Hz), 7.32 (1H, t, J = 2.8 Hz), 7.37 (1H, d, J = 2.3 Hz), 7.45 (1H, d, J = 8.7 Hz), 7.98 (1H, dd, J = 2.7 Hz, 9.1 Hz), 8.30 (1H, br s), 8.38 (1H, d, J = 2.7 Hz).

(ii) Production of 3-bromo-5-(2-chloro-4-nitrophenoxy)-1H-indole

The title compound (1.23 g) was obtained as a yellow oil by reaction in the same manner as in Example 169 (ii) and using 5-(2-chloro-4-nitrophenoxy)-1H-indole (1.20 g), N-bromosuccinimide (815 mg), tert-butanol (25 mL) and tetrahydrofuran (5 mL).
$^1$H-NMR ($CDCl_3$) δ: 6.80 (1H, d, J = 9.0 Hz), 7.02 (1H, dd, J = 2.4 Hz, 8.8 Hz), 7.29-7.37 (2H, m), 7.45 (1H, d, J = 8.8 Hz), 8.01 (1H, dd, J = 2.7 Hz, 9.0 Hz), 8.38 (1H, br s), 8.39 (1H, d, J = 2.7 Hz).

(iii) Production of 5-(2-chloro-4-nitrophenoxy)-1,3-dihydro-2H-indol-2-one

The title compound (755 mg) was obtained as an orange powder by reaction in the same manner as in Example 169(iii) and using 3-bromo-5-(2-chloro-4-nitrophenoxy)-1H-indole (1.22 g), 6N hydrochloric acid (6 mL) and tetrahydrofuran (25 mL). $^1$H-NMR (DMSO-$d_6$) δ: 3.53 (2H, s), 6.90 (1H, d, J = 8.5 Hz), 6.94 (1H, d, J = 9.1 Hz), 7.04 (1H, dd, J = 2.3 Hz, 8.5 Hz), 7.13 (1H, d, J = 2.3 Hz), 8.16 (1H, dd, J = 2.7 Hz, 9.1 Hz), 8.45 (1H, d, J = 2.7 Hz), 10.50 (1H, s).

(iv) Production of 5-(4-amino-2-chlorophenoxy)-1,3-dihydro-2H-indol-2-one

The title compound (643 mg) was obtained as a white powder by reaction in the same manner as in Example 169(iv) and using 5-(2-chloro-4-nitrophenoxy)-1,3-dihydro-2H-indol-2-one (752 mg), 5% platinum/activated carbon (72.6 mg), methanol (20 mL) and tetrahydrofuran (20 mL).
$^1$H-NMR ($CDCl_3$) δ: 3.50 (2H, s), 3.66 (2H, br s), 6.56 (1H, dd, J = 2.8 Hz, 8.7 Hz), 6.72-6.84 (4H, m), 6.85 (1H, d, J = 8.7 Hz), 7.72 (1H, br s).

(v) Production of 5-(2-chloro-4-{[5-(2-hydroxyethyl)-5H-pyrrolo[3,2-d]pyrimidin-4-yl]amino}phenoxy)-1,3-dihydro-2H-indol-2-one

The title compound (90.4 mg) was obtained as pale-orange crystals by reaction in the same manner as in Example 169(v) and using 2-(4-chloro-5H-pyrrolo[3,2-d]pyrimidin-5-yl)ethyl benzoate (149 mg), 5-(4-amino-2-chlorophenoxy)-1,3-dihydro-2H-indol-2-one (150 mg), pyridine hydrochloride (4 mg), isopropyl alcohol (8 mL), potassium carbonate (85.9 mg), tetrahydrofuran (5 mL), isopropyl alcohol (1.5 mL) and methanol (1.5 mL).
$^1$H-NMR (DMSO-$d_6$) δ: 3.47 (2H, s), 3.86 (2H, t, J = 4.5 Hz), 4.52 (2H, t, J = 4.5 Hz), 6.27 (1H, s), 6.50 (1H, d, J = 3.0 Hz), 6.75-6.80 (2H, m), 6.88 (1H, s), 7.08 (1H, d, J = 8.7 Hz), 7.52 (1H, dd, J = 2.7 Hz, 9.1 Hz), 7.65 (1H, d, J = 3.0 Hz), 7.91 (1H, d, J = 2.7 Hz), 8.31 (1H, s), 9.77 (1H, br s), 10.32 (1H, s).
Example 184
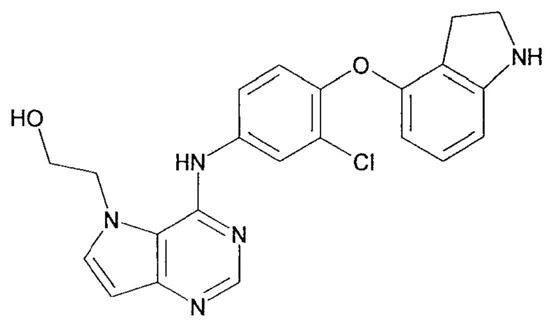

Production of 2-(4-{[3-chloro-4-(2,3-dihydro-1H-indol-4-yloxy)phenyl]amino}-5H-pyrrolo[3,2-d]pyrimidin-5-yl) ethanol

(i) Production of tert-butyl 4-(2-chloro-4-nitrophenoxy)indoline-1-carboxylate

To a solution of 4-(2-chloro-4-nitrophenoxy)-1H-indole (800 mg) in acetic acid (20 mL) was slowly added sodium cyanoborohydride (526 mg), and the mixture was stirred at room temperature for 21 hr. The reaction mixture was concentrated under reduced pressure, and the residue was dissolved in ethyl acetate. This solution was washed successively with saturated aqueous sodium hydrogen carbonate solution and saturated brine, dried over anhydrous magnesium sulfate, and concentrated under reduced pressure. The residue was dissolved in methanol (15 mL), di-tert-butyl dicarbonate (1 mL) and triethylamine (3.2 mL) were added, and the mixture was stirred at room temperature for 1 hr. The reaction mixture was concentrated under reduced pressure, and the residue was separated and purified by silica gel column chromatography (hexane→hexane:ethyl acetate=1:1) to give the title compound (626 mg) as a yellow solid.
$^1$H-NMR ($CDCl_3$) δ: 1.56 (9H, s), 2.93 (2H, t, J = 8.8 Hz), 4.00 (2H, t, J = 8.8 Hz), 6.62 (1H, d, J = 8.5 Hz), 6.82 (1H, d, J = 9.0 Hz), 7.18-7.28 (1H, m), 7.74 (1H, br s), 8.04 (1H, dd, J = 2.6 Hz, 9.0 Hz), 8.38 (1H, d, J = 2.6 Hz).

(ii) Production of tert-butyl 4-(4-amino-2-chlorophenoxy)indoline-1-carboxylate

The title compound (403 mg) was obtained as a colorless oil by reaction in the same manner as in Example 169(iv) and using tert-butyl 4-(2-chloro-4-nitrophenoxy)indoline-1-carboxylate (624 mg), 5% platinum/activated carbon (62.6 mg), ethyl acetate (15 mL) and methanol (30 mL).
$^1$H-NMR ($CDCl_3$) δ: 1.56 (9H, s), 3.08 (2H, t, J = 8.8 Hz), 3.64 (2H, br s), 4.02 (2H, t, J = 8.8 Hz), 6.26 (1H, d, J = 8.1 Hz), 6.54 (1H, dd, J = 2.8 Hz, 8.7 Hz), 6.77 (1H, d, J = 2.8 Hz), 6.85 (1H, d, J = 8.7 Hz), 7.04 (1H, t, J = 8,1 Hz), 7.55 (1H, br s).

(iii) Production of tert-butyl 4-(2-chloro-4-{[5-(2-hydroxyethyl)-5H-pyrrolo[3,2-d]pyrimidin-4-yl]amino}phenoxy)indoline-1-carboxylate

The title compound (244 mg) was obtained as white crystals by reaction in the same manner as in Example 168(iii) and using 2-(4-chloro-5H-pyrrolo[3,2-d]pyrimidin-5-yl)ethyl benzoate (201 mg), tert-butyl 4-(4-amino-2-chlorophenoxy)indoline-1-carboxylate (279 mg), isopropyl alcohol (10 mL) and 1N aqueous sodium hydroxide solution (3 mL).
$^1$H-NMR ($CDCl_3$) δ: 1.57 (9H, s), 3.10 (2H, t, J = 8.8 Hz), 4.03 (2H, t, J = 8.8 Hz), 4.15 (2H, t, J = 4.3 Hz), 4.32-4.43 (2H, m), 6.16 (1H, d, J = 3.0 Hz), 6.41 (1H, d, J = 8.3 Hz), 6.91-7.02 (2H, m), 7.10 (1H, t, J = 8.3 Hz), 7.44 (1H, dd, J = 2.5 Hz, 8.8 Hz), 7.60 (1H, br s), 7.78 (1H, d, J = 2.5 Hz), 8.25 (1H, s), 9.43 (1H, s).

(iv) Production of 2-(4-{[3-chloro-4-(2,3-dihydro-1H-indol-4-yloxy)phenyl]amino}-5H-pyrrolo[3,2-d]pyrimidin-5-yl)ethanol

To a solution of tert-butyl 4-(2-chloro-4-{[5-(2-hydroxyethyl)-5H-pyrrolo[3,2-d]pyrimidin-4-yl]amino}phenoxy) indoline-1-carboxylate (129 mg) in ethanol (2.5 mL) was added 6N hydrochloric acid (0.2 mL), and the mixture was stirred at 50°C for 19 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by basic silica gel column chromatography (eluent, hexane:ethyl acetate=1:4→ethyl acetate→ethyl acetate:methanol=19:1) and crystallized from diisopropyl ether/ethyl acetate to give the title compound (70.3 mg) as white crystals.
$^1$H-NMR (DMSO-$d_6$) δ: 2.83 (2H, t, J = 8.5 Hz), 3.45 (2H, td, J = 1.9 Hz, 8.5 Hz), 3.86 (2H, t, J = 4.5 Hz), 4.52 (2H, t, J = 4.5), 5.67 (1H, br s), 5.95 (1H, d, J = 8.0 Hz), 6.19-6.34 (2H, m), 6.50 (1H, d, J = 3.0 Hz), 6.88 (1H, t, J = 8.0 Hz), 7.05 (1H, d, J = 9.0 Hz), 7.51 (1H, dd, J = 2.7 Hz, 9.0 Hz), 7.64 (1H, d, J = 3.0 Hz), 7.91 (1H, d, J = 2.7 Hz), 8.31 (1H, s), 9.76 (1H, br s).
Example 185
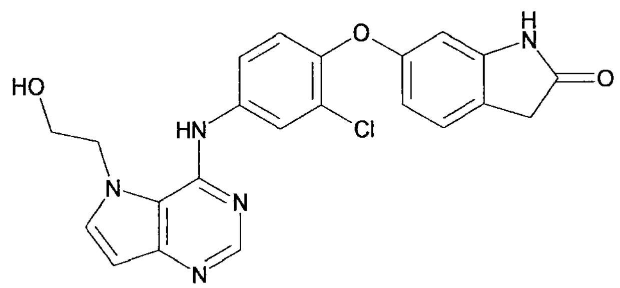

Production of 6-(2-chloro-4-{[5-(2-hydroxyethyl)-5H-pyrrolo[3,2-d]pyrimidin-4-yl]amino}phenoxy)-1,3-dihydro-2H-indol-2-one

(i) Production of 6-(2-chloro-4-nitrophenoxy)-1H-indole

The title compound (1.37 mg) was obtained as yellow crystals by reaction in the same manner as in Example 168(i) and using 6-hydroxyindole (1.17 g), 2-chloro-1-fluoro-4-nitrobenzene (1.60 g), potassium carbonate (1.45 g) and N,N-dimethylformamide (15 mL).
$^1$H-NMR ($CDCl_3$) δ: 6.57-6.62 (1H, m), 6.82 (1H, d, J = 9.1 Hz), 6.68 (1H, dd, J = 2.1 Hz, 8.7 Hz), 7.13-7.17 (1H, m), 7.27 (1H, dd, J = 2.1 Hz, 3.2 Hz), 7.67 (1H, d, J = 8.7 Hz), 7.98 (1H, dd, J = 2.8 Hz, 9.1 Hz), 8.25 (1H, br s), 8.37 (1H, d, J = 2.8 Hz).

(ii) Production of 3-bromo-6-(2-chloro-4-nitrophenoxy)-1H-indole

The title compound (721 mg) was obtained as a yellow solid by reaction in the same manner as in Example 169(ii) and using 6-(2-chloro-4-nitrophenoxy)-1H-indole (803 mg), N-bromosuccinimide (548 mg), tert-butanol (16 mL) and tetrahydrofuran (4 mL).
$^1$H-NMR ($CDCl_3$) δ: 6.81 (1H, d, J = 9.1 Hz), 6.96 (1H, dd, J = 2.0 Hz, 8.7 Hz), 7.14 (1H, d, J = 2.0 Hz), 7.28 (1H, d, J = 2.5 Hz), 7.62 (1H, d, J = 8.7 Hz), 8.00 (1H, dd, J = 2.8 Hz, 9.1 Hz), 8.30 (1H, br s), 8.38 (1H, d, J = 2.8 Hz).

(iii) Production of 6-(2-chloro-4-nitrophenoxy)-1,3-dihydro-2H-indol-2-one

The title compound (383 mg) was obtained as a brown powder by reaction in the same manner as in Example 169(iii) and using 3-bromo-6-(2-chloro-4-nitrophenoxy)-1H-indole (716 mg), 6N hydrochloric acid (4 mL) and tetrahydrofuran (15 mL). $^1$H-NMR ($CDCl_3$) δ: 3.56 (2H, s), 6.63 (1H, d, J = 2.2 Hz), 6.72 (1H, dd, J = 2.2 Hz, 8.0 Hz), 6.94 (1H, d, J = 9.0 Hz), 7.15-7.37 (1H, m), 7.78 (1H, br s), 8.07 (1H, dd, J = 2.8 Hz, 9.0 Hz), 8.39 (1H, d, J = 2.8 Hz).

(iv) Production of 6-(4-amino-2-chlorophenoxy)-1,3-dihydro-2H-indol-2-one

The title compound (208 mg) was obtained as pale-yellow crystals by reaction in the same manner as in Example 169(iv) and using 6-(2-chloro-4-nitrophenoxy)-1,3-dihydro-2H-indol-2-one (380 mg), 5% platinum/activated carbon (36.4 mg), methanol (10 mL) and tetrahydrofuran (10 mL).
$^1$H-NMR ($CDCl_3$) δ: 3.47 (2H, s), 3.69 (2H, s), 6.41 (1H, d, J = 2.3 Hz), 6.52 (1H, dd, J = 2.3 Hz, 8.0 Hz), 6.58 (1H, dd, J = 2.8 Hz, 8.7 Hz), 6.78 (1H, d, J = 2.8 Hz), 6.91 (1H, d, J = 8.7 Hz), 7.09 (1H, d, J = 8.0 Hz), 7.68 (1H, br s).

(v) Production of 6-(2-chloro-4-{[5-(2-hydroxyethyl)-5H-pyrrolo[3,2-d]pyrimidin-4-yl]amino}phenoxy)-1,3-dihydro-2H-indol-2-one

The title compound (90.2 mg) was obtained as pale-orange crystals by reaction in the same manner as in Example 169(v) and using 2-(4-chloro-5H-pyrrolo[3,2-d]pyrimidin-5-yl)ethyl benzoate (120 mg), 6-(4-amino-2-chlorophenoxy)-1,3-dihydro-2H-indol-2-one (113 mg), pyridine hydrochloride (3 mg), isopropyl alcohol (6 mL), potassium carbonate (86.2 mg), tetrahydrofuran (5 mL), isopropyl alcohol (1.5 mL) and methanol (1.5 mL).
$^1$H-NMR (DMSO-$d_6$) δ: 3.42 (2H, s), 3.87 (2H, t, J = 4.5 Hz), 4.53 (2H, t, J = 4.5 Hz), 6.29 (1H, br s), 6.31 (1H, d, J = 2.3 Hz), 6.46-6.56 (2H, m), 7.16 (1H, d, J = 8.3 Hz), 7.22 (1H, d, J = 8.7 Hz), 7.58 (1H, dd, J = 2.7 Hz, 8.7 Hz), 7.66 (1H, d, J = 3.0 Hz), 7.95 (1H, d, J = 2.7 Hz), 8.34 (1H, s), 9.85 (1H, br s), 10.29 (1H, br s).
Example 186
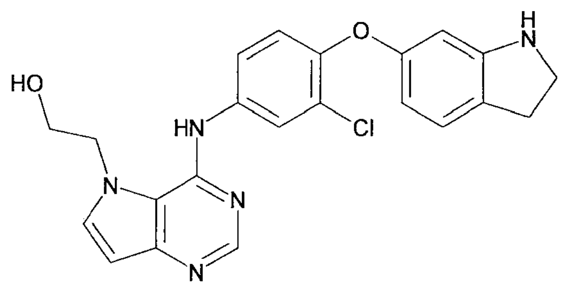

Production of 2-(4-{[3-chloro-4-(2,3-dihydro-1H-indol-6-yloxy)phenyl]amino}-5H-pyrrolo[3,2-d]pyrimidin-5-yl) ethanol

(i) Production of tert-butyl 6-(2-chloro-4-nitrophenoxy)indoline-1-carboxylate

The title compound (444 mg) was obtained as a yellow powder by reaction in the same manner as in Example 184(i) and using 6-(2-chloro-4-nitrophenoxy)-1H-indole (531 mg), acetic acid (13 mL), sodium cyanoborohydride (385 mg), di-tert-butyl dicarbonate (0.65 mL) and triethylamine (1.6 mL).
$^1$H-NMR ($CDCl_3$) δ: 1.51 (9H, s), 3.11 (2H, t, J = 9.0 Hz), 4.04 (2H, t, J = 9.0 Hz), 6.65 (1H, dd, J = 2.3 Hz, 8.0 Hz), 6.88 (1H, br s), 7.15 (1H, d, J = 8.0 Hz), 7.62 (1H, br s), 8.02 (1H, dd, J = 2.8 Hz, 8.8 Hz), 8.35 (1H, d, J = 2.8 Hz).

(ii) Production of tert-butyl 6-(4-amino-2-chlorophenoxy)indoline-1-carboxylate

The title compound (406 mg) was obtained as a white powder by reaction in the same manner as in Example 169(iv) and using tert-butyl 6-(2-chloro-4-nitrophenoxy)indoline-1-carboxylate (442 mg), 5% platinum/activated carbon (44.3 mg) and ethyl acetate (30 mL).
$^1$H-NMR ($CDCl_3$) δ: 1.47 (9H, s), 3. 02 (2H, t, J = 8.7 Hz), 3. 63 (2H, br s), 3.98 (2H, t, J = 8.7 Hz), 6.49 (1H, br s), 6.55 (1H, dd, J = 2.6 Hz, 8.7 Hz), 6.76 (1H, d, J = 2.6 Hz), 6.84-6.95 (1H, m), 7.01 (1H, d, J = 8.3 Hz), 7.53 (1H, br s).

(iii) Production of tert-butyl 6-(2-chloro-4-{[5-(2-hydroxyethyl)-5H-pyrrolo[3,2-d]pyrimidin-4-yl]amino}phenoxy)indoline-1-carboxylate

The title compound (237 mg) was obtained as pink crystals by reaction in the same manner as in Example 168 (iii) and using 2-(4-chloro-5H-pyrrolo[3,2-d]pyrimidin-5-yl)ethyl benzoate (192 mg), tert-butyl 6-(4-amino-2-chlorophenoxy)indoline-1-carboxylate (256 mg), diisopropyl alcohol (10 mL) and 1N aqueous sodium hydroxide solution (3 mL).
$^1$H-NMR (DMSO-$d_6$) δ: 1.41 (9H, br s), 2.94-3.07 (2H, m), 3.79-3.98 (4H, m), 4.53 (2H, t, J = 4.8 Hz), 6.25-6.31 (1H, m), 6.50 (1H, d, J = 3.0 Hz), 6.51-6.60 (1H, m), 7.09-7.25 (1H, m), 7.14 (1H, d, J = 8.3 Hz), 7.57 (1H, dd, J = 2.6 Hz, 8.9 Hz), 7.65 (1H, d, J = 3.0 Hz), 7.94 (1H, d, J = 2.6 Hz), 8.32 (1H, s), 9.82 (1H, s).

(iv) Production of 2-(4-{[3-chloro-4-(2,3-dihydro-1H-indol-6-yloxy)phenyl]amino}-5H-pyrrolo[3,2-d]pyrimidin-5-yl)ethanol

The title compound (79.6 mg) was obtained as white crystals by reaction in the same manner as in Example 184(iv) and using tert-butyl 6-(2-chloro-4-{[5-(2-hydroxyethyl)-5H-pyrrolo[3,2-d]pyrimidin-4-yl]amino}phenoxy)indoline-1-carboxylate (129 mg), 6N hydrochloric acid (0.2 mL) and ethanol (2.5 mL).
$^1$H-NMR (DMSO-$d_6$) δ: 2.85 (2H, t, J = 8.4 Hz), 3.42 (2H, td, J = 1.6 Hz, 8.4 Hz), 3.87 (2H, t, J = 4.5 Hz), 4.52 (2H, t, J = 4.5 Hz), 5.61 (1H, s), 6.00 (1H, d, J = 2.3 Hz), 6.07 (1H, dd, J = 2.3 Hz, 7.9 Hz), 6.27 (1H, br s), 6.50 (1H, d, J = 3.0 Hz), 6.94 (1H, d, J = 7.9 Hz), 7.11 (1H, d, J = 8.9 Hz), 7.53 (1H, dd, J = 2.5 Hz, 8.9 Hz), 7.65 (1H, d, J = 3.0 Hz), 7.90 (1H, d, J = 2.5 Hz), 8.32 (1H, s), 9.77 (1H, br s).
Example 187
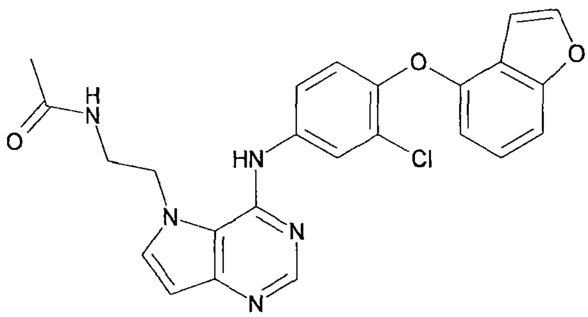

Production of N-[2-(4-{[4-(1-benzofuran-4-yloxy)-3-chlorophenyl]amino}-5H-pyrrolo[3,2-d]pyrimidin-5-yl)ethyl] acetamide

(i) Production of tert-butyl [2-(4-{[4-(1-benzofuran-4-yloxy)-3-chlorophenyl]amino}-5H-pyrrolo[3,2-d]pyrimidin-5-yl)ethyl] carbamate

The title compound (1.01 g) was obtained as a pale-yellow powder by reaction in the same manner as in Example 182(i) and using tert-butyl [2-(4-chloro-5H-pyrrolo[3,2-d]pyrimidin-5-yl)ethyl]carbamate (1.00 g), 4-(1-benzofuran-4-yloxy)-3-chloroaniline (963 mg) and isopropyl alcohol (15 mL).
$^1$H-NMR ($CDCl_3$) δ: 1.49 (9H, s), 3.42-3.57 (2H, m), 4.42-4.53 (2H, m), 5.06 (1H, t, J = 5.6 Hz), 6.60 (1H, d, J = 3.2 Hz), 6.69 (1H, dd, J = 0.9 Hz, 7.7 Hz), 6.81 (1H, dd, J = 0.9 Hz, 2.3 Hz), 7.04 (1H, d, J = 9.0 Hz), 7.14-7.32 (3H, m), 7.57 (1H, d, J = 2.3 Hz), 7.84 (1H, dd, J = 2.6 Hz, 9.0 Hz), 8.03 (1H, d, J = 2.6 Hz), 8.51 (1H, s), 8.58 (1H, br s).

(ii) Production of 5-(2-aminoethyl)-N-[4-(1-benzofuran-4-yloxy)-3-chlorophenyl]-5H-pyrrolo[3,2-d]pyrimidin-4-amine dihydrochloride

The title compound (855 mg) was obtained as a white powder by reaction in the same manner as in Example 182(ii) and using tert-butyl [2-(4-{[4-(1-benzofuran-4-yloxy)-3-chlorophenyl]amino}-5H-pyrrolo[3,2-d]pyrimidin-5-yl)ethyl] carbamate (1.00 g), 6N hydrochloric acid (1.5 mL), ethanol (15 mL) and tetrahydrofuran (15 mL).
$^1$H-NMR (DMSO-$d_6$) δ: 3.21-3.36 (2H, m), 5.02 (2H, t, J = 5.8 Hz), 6.64-6.79 (2H, m), 6.85 (1H, s), 7.22 (1H, dd, J = 1.5 Hz, 9.0 Hz), 7.32 (1H, t, J = 8.1 Hz), 7.40-7.48 (1H, m), 7.60 (1H, d, J = 9.0 Hz), 7.92 (1H, s), 7.98-8.12 (2H, m), 8.31 (3H, br s), 8.73 (1H, s), 10.08 (1H, br s).

(iii) Production of N-[2-(4-{[4-(1-benzofuran-4-yloxy)-3-chlorophenyl]amino}-5H-pyrrolo[3,2-d]pyrimidin-5-yl)ethyl]acetamide

A mixture of 5-(2-aminoethyl)-N-[4-(1-benzofuran-4-yloxy)-3-chlorophenyl]-5H-pyrrolo[3,2-d]pyrimidin-4-amine dihydrochloride (110 mg), acetic acid (20 μL), 1-hydroxybenzotriazole (51.3 mg), 1-ethyl-3-(3-dimethylaminopropyl)carbodiimide hydrochloride (71.6 mg), triethylamine (0.3 mL), tetrahydrofuran (0.6 mL) and N,N-dimethylformamide (0.6 mL) was stirred at room temperature for 18 hr. Water was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:4→ethyl acetate→ethyl acetate:methanol=9:1) and crystallized from diisopropyl ether/ethyl acetate to give the title compound (83.3 mg) as white crystals.
$^1$H-NMR (DMSO-$d_6$) δ: 1.79 (3H, s), 3.23-3.46 (2H, m), 4.51 (2H, t, J = 6.9 Hz), 6.50 (1H, d, J = 3.0 Hz), 6.60 (1H, d, J = 8.0 Hz), 6.87 (1H, dd, J = 0.9 Hz, 2.2 Hz), 7.21 (1H, d, J = 8.9 Hz), 7.27 (1H, t, J = 8.0 Hz), 7.34-7.42 (1H, m), 7.64 (1H, d, J = 3.0 Hz), 7.73 (1H, dd, J = 2.3 Hz, 8.9 Hz), 8.00 (1H, d, J = 2.3 Hz), 8.04 (1H, d, J = 2.2 Hz), 8.26 (1H, t, J = 4.9 Hz), 8.33 (1H, s), 8.78 (1H, s).
Example 188
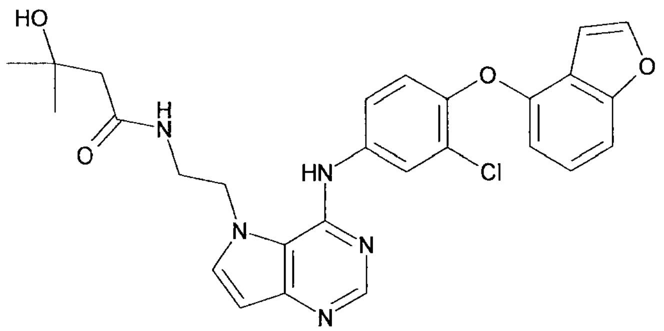

Production of N-[2-(4-{[4-(1-benzofuran-4-yloxy)-3-chlorophenyl]amino}-5H-pyrrolo[3,2-d]pyrimidin-5-yl)ethyl]-3-hydroxy-3-methylbutanamide

The title compound (87.6 mg) was obtained as white crystals by reaction in the same manner as in Example 187(iii) and using 5-(2-aminoethyl)-N-[4-(1-benzofuran-4-yloxy)-3-chlorophenyl]-5H-pyrrolo[3,2-d]pyrimidin-4-amine dihydrochloride (110 mg), 3-hydroxy-3-methylbutanoic acid (39.9 mg), 1-hydroxybenzotriazole (57.7 mg), 1-ethyl-3-(3-dimethylaminopropyl)carbodiimide hydrochloride (69.4 mg), triethylamine (0.3 mL), tetrahydrofuran (0.6 mL) and N,N-dimethylformamide (0.6 mL).
$^1$H-NMR (DMSO-$d_6$) δ: 1.13 (6H, s), 2.20 (2H, s), 3.36-3.48 (2H, m), 4.52 (2H, t, J = 7.0 Hz), 4.67 (1H, s), 6.49 (1H, d, J = 3.0 Hz), 6.60 (1H, dd, J =0.9 Hz, 8.0 Hz), 6.87 (1H, dd, J = 0.9 Hz, 2.2 Hz), 7.19 (1H, d, J = 8.9 Hz), 7.26 (1H, t, J = 8.0 Hz), 7.34-7.41 (1H, m), 7.64 (1H, d, J = 3.0 Hz), 7.79 (1H, dd, J = 2.6 Hz, 8.9 Hz), 7.99 (1H, d, J = 2.2 Hz), 8.05 (1H, d, J = 2.6 Hz), 8.26 (1H, t, J = 5.4 Hz), 8.33 (1H, s), 8.87 (1H, s).
Example 189
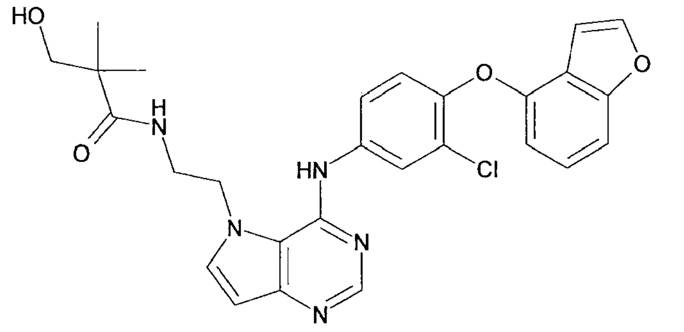

Production of N-[2-(4-{[4-(1-benzofuran-4-yloxy)-3-chlorophenyl]amino}-5H-pyrrolo[3,2-d]pyrimidin-5-yl)ethyl]-3-hydroxy-2,2-dimethylpropanamide

The title compound (100 mg) was obtained as white crystals by reaction in the same manner as in Example 187(iii) and using 5-(2-aminoethyl)-N-[4-(1-benzofuran-4-yloxy)-3-chlorophenyl]-5H-pyrrolo[3,2-d]pyrimidin-4-amine dihydrochloride (111 mg), 3-hydroxy-2,2-dimethylpropanoic acid (40.9 mg), 1-hydroxybenzotriazole (54.4 mg), 1-ethyl-3-(3-dimethylaminopropyl)carbodiimide hydrochloride (70.8 mg), triethylamine (0.3 mL), tetrahydrofuran (0.6 mL) and N,N-dimethylformamide (0.6 mL).
$^1$H-NMR (DMSO-$d_6$) δ: 0.98 (6H, s), 3.28-3.36 (2H, m), 3.36-3.46 (2H, m), 4.51 (2H, t, J = 6.8 Hz), 4.84 (1H, t, J = 5.3 Hz), 6.49 (1H, d, J = 3.0 Hz), 6.60 (1H, dd, J = 0.9 Hz, 8.0 Hz), 6.87 (1H, dd, J = 0.9 Hz, 2.3 Hz), 7.21 (1H, d, J = 8.9 Hz), 7.27 (1H, t, J = 8.0 Hz), 7.34-7.41 (1H, m), 7.60 (1H, d, J = 3.0 Hz), 7.78-7.91 (2H, m), 7.99 (1H, d, J = 2.3 Hz), 8.11 (1H, d, J = 2.5 Hz), 8.34 (1H, s), 8.89 (1H, s).

Example 190

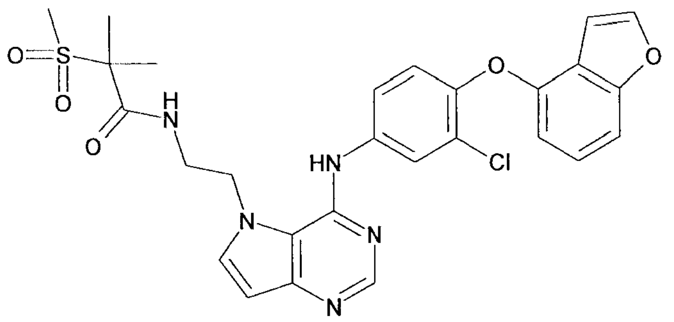

Production of N-[2-(4-{[4-(1-benzofuran-4-yloxy)-3-chlorophenyl]amino}-5H-pyrrolo[3,2-d]pyrimidin-5-yl)ethyl]-2-methyl-2-(methylsulfonyl)propanamide

The title compound (104 mg) was obtained as white crystals by reaction in the same manner as in Example 187(iii) and using 5-(2-aminoethyl)-N-[4-(1-benzofuran-4-yloxy)-3-chlorophenyl]-5H-pyrrolo[3,2-d]pyrimidin-4-amine dihydrochloride (111 mg), 2-methyl-2-(methylsulfonyl)propanoic acid (56.2 mg), 1-hydroxybenzotriazole (59.4 mg), 1-ethyl-3-(3-dimethylaminopropyl)carbodiimide hydrochloride (74.2 mg), triethylamine (0.3 mL), tetrahydrofuran (0.6 mL) and N,N-dimethylformamide (0.6 mL).
$^1$H-NMR (DMSO-$d_6$) δ: 1.41 (6H, s), 2.96 (3H, s), 3.48 (2H, q, J = 6.0 Hz), 4.58 (2H, t, J = 6.0 Hz), 6.49 (1H, d, J = 3.0 Hz), 6.60 (1H, dd, J = 0.9 Hz, 8.0 Hz), 6.86 (1H, dd, J = 0.9 Hz, 2.2 Hz), 7.22 (1H, d, J = 8.9 Hz), 7.27 (1H, t, J = 8.0 Hz), 7.34-7.43 (1H, m), 7.57 (1H, d, J = 3.0 Hz), 7.73 (1H, dd, J = 2.3 Hz, 8.9 Hz), 7.96-8.05 (2H, m), 8.22 (1H, t, J = 6.0 Hz), 8.34 (1H, s), 8.68 (1H, s).

Example 191

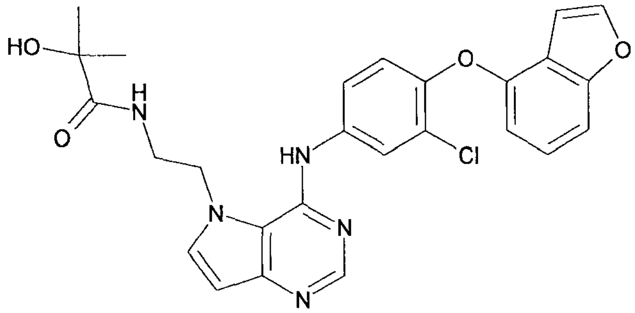

Production of N-[2-(4-{[4-(1-benzofuran-4-yloxy)-3-chlorophenyl]amino}-5H-pyrrolo[3,2-d]pyrimidin-5-yl)ethyl]-2-hydroxy-2-methylpropanamide

The title compound (73.9 mg) was obtained as white crystals by reaction in the same manner as in Example 187(iii) and using 5-(2-aminoethyl)-N-[4-(1-benzofuran-4-yloxy)-3-chlorophenyl]-5H-pyrrolo[3,2-d]pyrimidin-4-amine dihydrochloride (110 mg), 2-hydroxy-2-methylpropanoic acid (39.7 mg), 1-hydroxybenzotriazole (49.5 mg), 1-ethyl-3-(3-dimethylaminopropyl)carbodiimide hydrochloride (74.3 mg), triethylamine (0.3 mL), tetrahydrofuran (0.6 mL) and N,N-dimethylformamide (0.6 mL).
$^1$H-NMR (DMSO-$d_6$) δ: 1.17 (6H, s), 3.45 (2H, q, J = 6.5 Hz), 4.55 (2H, t, J = 6.5 Hz), 5.38 (1H, s), 6.48 (1H, d, J = 3.0 Hz), 6.61 (1H, d, J = 8.0 Hz), 6.87 (1H, dd, J = 0.9 Hz, 2.2 Hz), 7.21 (1H, d, J = 8.9 Hz), 7.27 (1H, t, J = 8.0 Hz), 7.34-7.41 (1H, m), 7.58 (1H, d, J = 3.0 Hz), 7.76 (1H, dd, J = 2.5 Hz, 8.9 Hz), 7.99 (1H, d, J = 2.2 Hz), 8.01-8.09 (2H, m), 8.32 (1H, s), 8.75 (1H,s).
Example 192
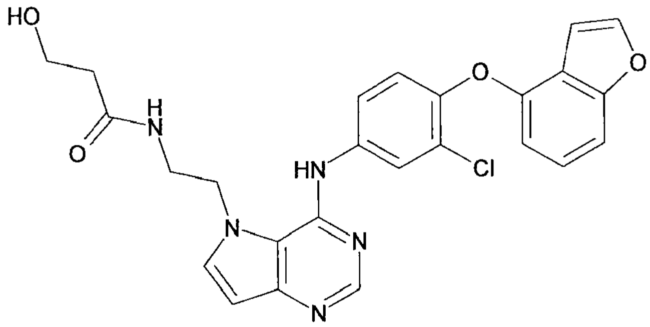

Production of N-[2-(4-{[4-(1-benzofuran-4-yloxy)-3-chlorophenyl]amino}-5H-pyrrolo[3,2-d]pyrimidin-5-yl)ethyl]-3-hydroxypropanamide

The title compound (39.4 mg) was obtained as white crystals by reaction in the same manner as in Example 187(iii) and using 5-(2-aminoethyl)-N-[4-(1-benzofuran-4-yloxy)-3-chlorophenyl]-5H-pyrrolo[3,2-d]pyrimidin-4-amine dihydrochloride (111 mg), 3-hydroxypropanoic acid (106 mg), 1-hydroxybenzotriazole (64.8 mg), 1-ethyl-3-(3-dimethylaminopropyl)carbodiimide hydrochloride (73.4 mg), triethylamine (0.3 mL), tetrahydrofuran (0.6 mL) and N,N-dimethylformamide (0.6 mL).
$^1$H-NMR (DMSO-$d_6$) δ: 2.23 (2H, t, J = 6.5 Hz), 3.34-3.44 (2H, m), 3.53-3.64 (2H, m), 4.52 (2H, t, J = 7.0 Hz), 4.59 (1H, t, J = 5.1 Hz), 6.49 (1H, d, J = 3.0 Hz), 6.60 (1H, dd, J = 0.9 Hz, 8.0 Hz), 6.88 (1H, dd, J = 0.9 Hz, 2.3 Hz), 7.21 (1H, d, J = 8.9 Hz), 7.27 (1H, t, J = 8.0 Hz), 7.34-7.42 (1H, m), 7.64 (1H, d, J = 3.0 Hz), 7.78 (1H, dd, J = 2.5 Hz, 8.9 Hz), 8.00 (1H, d, J = 2.3 Hz), 8.07 (1H, d, J = 2.5 Hz), 8.25 (1H, t, J = 5.3 Hz), 8.34 (1H, s), 8.82 (1H, s).
Example 193
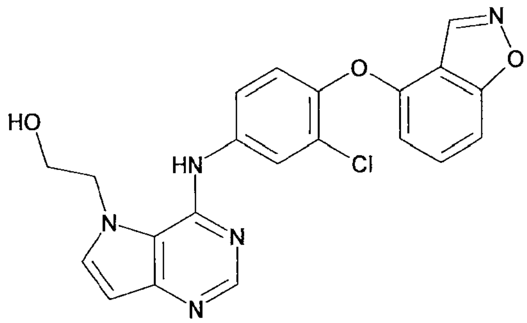

Production of 2-(4-{[4-(1,2-benzisoxazol-4-yloxy)-3-chlorophenyl]amino}-5H-pyrrolo[3,2-d]pyrimidin-5-yl)ethanol

(i) Production of 2-(2-chloro-4-nitrophenoxy)-6-methoxybenzaldehyde

The title compound (11.2 g) was obtained as yellow crystals by reaction in the same manner as in Example 168(i) and using 2-hydroxy-6-methoxybenzaldehyde (6.50 g), 2-chloro-1-fluoro-4-nitrobenzene (7.91 g), potassium carbonate (7.89 g) and N,N-dimethylformamide (100 mL).
$^1$H-NMR ($CDCl_3$) δ: 3. 99 (3H, s), 6. 64 (1H, d, J = 8.0 Hz), 6.77 (1H, d, J = 9.1 Hz), 6.94 (1H, d, J = 8.0 Hz), 7.57 (1H, t, J = 8.0 Hz), 8.03 (1H, dd, J = 2.6 Hz, 9.1 Hz), 8.40 (1H, d, J = 2.6 Hz), 10.43 (1H, s).

(ii) Production of 2-(2-chloro-4-nitrophenoxy)-6-hydroxybenzaldehyde

A solution of 2-(2-chloro-4-nitrophenoxy)-6-methoxybenzaldehyde (10.8 g) in chlorobenzene (150 mL) was cooled to 0°C, 1N boron tribromide dichloromethane solution (80 mL) was added dropwise, and the mixture was stirred at 0°C for 1.5 hr. Water was added to the reaction mixture, and the mixture was extracted with a mixed solvent of diethyl ether/ethyl acetate. The organic layer was washed with saturated brine, dried over anhydrous magnesium sulfate, and concentrated under reduced pressure. The residue was separated and purified by silica gel column chromatography (eluent, hexane:ethyl acetate=49:1→2:1) to give the title compound (8.62 g) as yellow crystals.
$^1$H-NMR ($CDCl_3$) δ: 6.29 (1H, dd, J = 0.8 Hz, 8.0 Hz), 6.79-6.85 (1H, m), 7.18 (1H, d, J = 9.0 Hz), 7.46 (1H, t, J = 8.0 Hz), 8.17 (1H, dd, J = 2.6 Hz, 9.0 Hz), 8.43 (1H, d, J = 2.6 Hz), 10.37 (1H, s), 11.80 (1H, s).

(iii) Production of 4-(2-chloro-4-nitrophenoxy)-1,2-benzisoxazole

To a mixture of 2-(2-chloro-4-nitrophenoxy)-6-hydroxybenzaldehyde (8.50), acetonitrile (75 mL) and water (75 mL) was added hydroxylamine O-sulfonic acid (3.65 g), and the mixture was stirred at room temperature for 1 hr. Sodium hydrogen carbonate (6.03 g) was slowly added to the reaction mixture, and the mixture was stirred for 1.5 hr while raising the temperature to room temperature. The reaction mixture was extracted with ethyl acetate, and the organic layer was washed with saturated brine, dried over anhydrous magnesium sulfate, and concentrated under reduced pressure. The residue was separated and purified by silica gel column chromatography (eluent, hexane:ethyl acetate=49:1→2:1) to give the title compound (5.56 g) as white crystals.
$^1$H-NMR ($CDCl_3$) δ: 6.78 (1H, dd, J = 0.9 Hz, 7.5 Hz), 7.11 (1H, d, J = 9.0 Hz), 7.45-7.61 (2H, m), 8.15 (1H, dd, J = 2.6 Hz, 9.0 Hz), 8.46 (1H, d, J = 2.6 Hz), 8.68 (1H, d, J = 0.9 Hz).

(iv) Production of 4-(1,2-benzisoxazol-4-yloxy)-3-chloroaniline

The title compound (356 mg) was obtained as a yellow oil by reaction in the same manner as in Example 168 (ii) and using 4-(2-chloro-4-nitrophenoxy)-1,2-benzisoxazole (1.00 g), reduced iron (1.16 g), 1N hydrochloric acid (4 mL) and ethanol (20 mL).
$^1$H-NMR ($CDCl_3$) δ: 3.77 (2H, br s), 6.51 (1H, dd, J = 1.0 Hz, 7.8 Hz), 6.62 (1H, dd, J = 2.8 Hz, 8.5 Hz), 6.82 (1H, d, J = 2.8 Hz), 7.01 (1H, d, J = 8.5 Hz), 7.23-7.29 (1H, m), 7.41 (1H, dd, J = 7.8 Hz, 8.4 Hz), 8.56 (1H, d, J = 1.0 Hz).

(v) Production of 2-(4-{[4-(1,2-benzisoxazol-4-yloxy)-3-chlorophenyl]amino}-5H-pyrrolo[3,2-d]pyrimidin-5-yl)ethanol

A mixture of 5-(2-{[tert-butyl(dimethyl)silyl]oxy}ethyl)-4-chloro-5H-pyrrolo[3,2-d]pyrimidine (126 mg), 4-(1,2-benzisoxazol-4-yloxy)-3-chloroaniline (117 mg) and isopropyl alcohol (2 mL) was stirred at 80°C for 15 hr. 6N Hydrochloric acid (0.2 mL) was added to the reaction mixture, and the mixture was stirred at room temperature for 30 min. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, hexane:ethyl acetate=4:1→ethyl acetate) and crystallized from ethyl acetate/ethanol to give the title compound (71.1 mg) as white crystals.
$^1$H-NMR (DMSO-$d_6$) δ: 3.84-3.94 (2H, m), 4.55 (2H, t, J = 4.4 Hz), 6.33 (1H, br s), 6.49-6.58 (2H, m), 7.43 (1H, d, J = 8.9 Hz), 7.46-7.52 (1H, m), 7.58 (1H, t, J = 8.1 Hz), 7.63-7.74 (2H, m), 8.05 (1H, d, J = 2.6 Hz), 8.36 (1H, s), 9.28 (1H, d, J = 0.9 Hz), 9.96 (1H, s).

Example 194

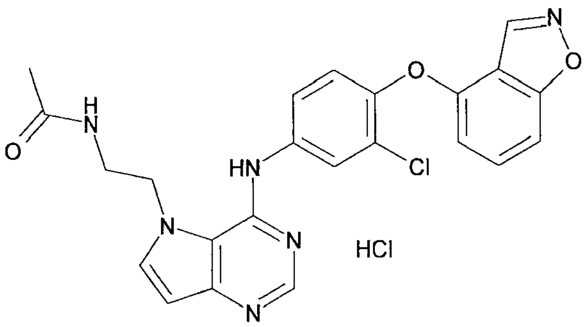

Production of N-[2-(4-{[4-(1,2-benzisoxazol-4-yloxy)-3-chlorophenyl]amino}-5H-pyrrolo[3,2-d]pyrimidin-5-yl) ethyl]acetamide hydrochloride

(i) Production of tert-butyl [2-(4-{[4-(1,2-benzisoxazol-4-yloxy)-3-chlorophenyl]amino}-5H-pyrrolo[3,2-d]pyrimidin-5-yl) ethyl]carbamate

The title compound (579 mg) was obtained as pale-yellow crystals by reaction in the same manner as in Example 182(i) and using tert-butyl [2-(4-chloro-5H-pyrrolo[3,2-d]pyrimidin-5-yl)ethyl]carbamate (424 mg), 4-(1,2-benzisoxazol-4-yloxy)-3-chloroaniline (535 mg) and isopropyl alcohol (10 mL).
$^1$H-NMR ($CDCl_3$) δ: 1.50 (9H, s), 3.44-3.58 (2H, m), 4.43-4.56 (2H, m), 5.10 (1H, t, J = 5.9 Hz), 6.62 (1H, d, J = 3.2 Hz), 6.63 (1H, dd, J = 0.9 Hz, 8.0 Hz), 7.14-7.23 (2H, m), 7.28-7.33 (1H, m), 7.44 (1H, t, J = 8.0 Hz), 7.96 (1H, dd, J = 2.6 Hz, 8.9 Hz), 8.09 (1H, d, J = 2.6 Hz), 8.53 (1H, s), 8.65 (1H, d, J = 0.9 Hz), 8.67 (1H, br s).

(ii) Production of 5-(2-aminoethyl)-N-[4-(1,2-benzisoxazol-4-yloxy)-3-chlorophenyl]-5H-pyrrolo[3,2-d]pyrimidin-4-amine dihydrochloride

The title compound (509 mg) was obtained as a white powder by reaction in the same manner as in Example 182(ii) and using tert-butyl [2-(4-{[4-(1,2-benzisoxazol-4-yloxy)-3-chlorophenyl]amino}-5H-pyrrolo[3,2-d]pyrimidin-5-yl) ethyl]carbamate (575 mg), 6N hydrochloric acid (1.5 mL), ethanol (10 mL) and tetrahydrofuran (10 mL).
$^1$H-NMR (DMSO-$d_6$) δ: 3.24-3.36 (2H, m), 5.02 (2H, t, J = 5.9 Hz), 6.59 (1H, dd, J = 1.0 Hz, 7.8 Hz), 6.75 (1H, d, J = 3.0 Hz), 7.49 (1H, d, J = 8.9 Hz), 7.52-7.57 (1H, m), 7.62 (1H, d, J = 7.8 Hz), 7.71 (1H, dd, J = 2.0 Hz, 8.9 Hz), 7.98 (1H, d, J = 2.0 Hz), 8.06 (1H, d, J = 3.0 Hz), 8.27 (3H, br s), 8.74 (1H, s), 9.28 (1H, d, J = 1.0 Hz), 10.10 (1H, br s).

(iii) Production of N-[2-(4-{[4-(1,2-benzisoxazol-4-yloxy)-3-chlorophenyl]amino}-5H-pyrrolo[3,2-d]pyrimidin-5-yl)ethyl] acetamide hydrochloride

To a mixture of 5-(2-aminoethyl)-N-[4-(1,2-benzisoxazol-4-yloxy)-3-chlorophenyl]-5H-pyrrolo[3,2-d]pyrimidin-4-amine dihydrochloride (121 mg), ethyl acetate (1.5 mL) and tetrahydrofuran (0.5 mL) were added acetic anhydride

(30 μL) and saturated aqueous sodium hydrogen carbonate solution (1.5 mL), and the mixture was stirred at room temperature for 15 min. Water was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:4→ethyl acetate→ethyl acetate:methanol=19:1), 4N hydrochloric acid/ethyl acetate (80 μL) was added to the object fraction, and the mixture was stirred at room temperature for 5 min. The reaction mixture was concentrated under reduced pressure and the residue was crystallized from ethyl acetate/ethanol to give the title compound (83.1 mg) as white crystals.
$^1$H-NMR (DMSO-$d_6$) δ: 1.81 (3H, s), 3.39-3.52 (2H, m), 4.64 (2H, t, J = 7.0 Hz), 6.61 (1H, d, J = 7.9 Hz), 6.68 (1H, d, J = 3.0 Hz), 7.49 (1H, d, J = 8.9 Hz), 7.52-7.58 (1H, m), 7.62 (1H, d, J = 7.9 Hz), 7.74 (1H, dd, J = 2.6 Hz, 8.9 Hz), 7.98 (1H, d, J = 3.0 Hz), 8.01 (1H, d, J = 2.6 Hz), 8.40 (1H, t, J = 5.8 Hz), 8.74 (1H, s), 9.28 (1H, d, J = 1.1 Hz), 10.13 (1H, br s).

Example 195

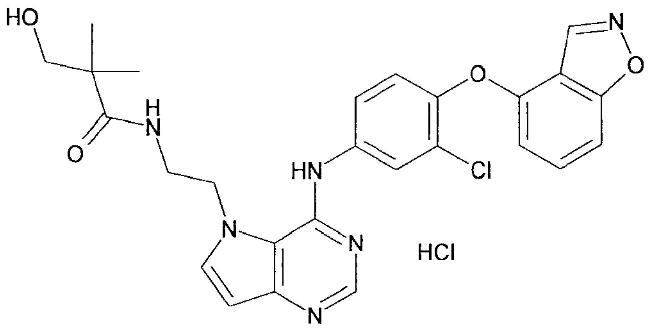

Production of N-[2-(4-{[4-(1,2-benzisoxazol-4-yloxy)-3-chlorophenyl]amino}-5H-pyrrolo[3,2-d]pyrimidin-5-yl)ethyl]-3-hydroxy-2,2-dimethylpropanamide hydrochloride

To a mixture of 5-(2-aminoethyl)-N-[4-(1,2-benzisoxazol-4-yloxy)-3-chlorophenyl]-5H-pyrrolo[3,2-d]pyrimidin-4-amine dihydrochloride (100 mg), 3-hydroxy-2,2-dimethylpropanoic acid (29.7 mg), 1-hydroxybenzotriazole (44.7 mg), 1-ethyl-3-(3-dimethylaminopropyl)carbodiimide hydrochloride (66.5 mg), tetrahydrofuran (0.6 mL) and N,N-dimethylformamide (0.6 mL) was added triethylamine (28.4 μL), and the mixture was stirred at room temperature for 18 hr. Water was added to the reaction mixture, and the mixture was extracted with ethyl acetate. The organic layer was dried over anhydrous magnesium sulfate and concentrated under reduced pressure. The residue was separated and purified by silica gel column chromatography (eluent, hexane:ethyl acetate=1:4→ethyl acetate→ethyl acetate:methanol=9:1), 4N hydrochloric acid/ethyl acetate (80 μL) was added to the object fraction, and the mixture was stirred at room temperature for 5 min. The reaction mixture was concentrated under reduced pressure and the residue was crystallized from ethyl acetate/ethanol to give the title compound (21.1 mg) as white crystals.
$^1$H-NMR (DMSO-$d_6$) δ: 0.96 (6H, s), 3.30 (2H, s), 3.44-3.55 (2H, m), 4.66 (2H, t, J = 6.6 Hz), 6.61 (1H, dd, J = 0.9 Hz, 7.7 Hz), 6.66 (1H, d, J = 3.0 Hz), 7.50 (1H, d, J = 8.7 Hz), 7.52-7.57 (1H, m), 7.62 (1H, d, J = 7.7 Hz), 7.82 (1H, dd, J = 2.5 Hz, 8.7 Hz), 7.89 (1H, t, J = 5.6 Hz), 7.93 (1H, d, J = 3.0 Hz), 8.06 (1H, d, J = 2.5 Hz), 8.74 (1H, s), 9.27 (1H, d, J = 0.9 Hz), 10.17 (1H, br s).

Example 196

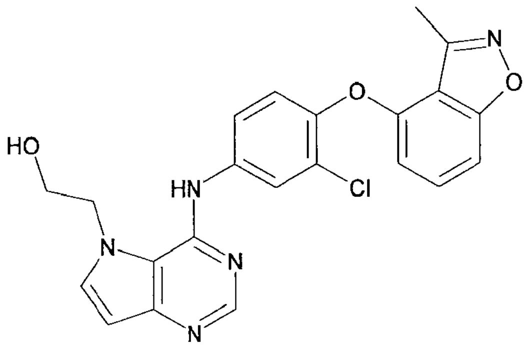

Production of 2-[4-({3-chloro-4-[(3-methyl-1,2-benzisoxazol-4-yl)oxy]phenyl}amino)-5H-pyrrolo[3,2-d]pyrimidin-5-yl]ethanol

(i) Production of 4-(2-chloro-4-nitrophenoxy)-3-methyl-1,2-benzisoxazole

The title compound (3.26 g) was obtained as a white solid by reaction in the same manner as in Example 168 (i) and using 3-methyl-1,2-benzisoxazol-4-ol (1.93 g), 2-chloro-1-fluoro-4-nitrobenzene (2.49 g), potassium carbonate (2.48 g) and N,N-dimethylformamide (40 mL).
$^1$H-NMR ($CDCl_3$) δ: 2.56 (3H, s), 6.74 (1H, dd, J = 0.7 Hz, 7.6 Hz), 7.04 (1H, d, J = 9.1 Hz), 7.42 (1H, dd, J = 0.7 Hz, 8.4 Hz), 7.52 (1H, dd, J = 7.6, 8.4 Hz), 8.13 (1H, dd, J = 2.6 Hz, 9.1 Hz), 8.45 (1H, d, J = 2. 6 Hz).

(ii) Production of 3-chloro-4-[(3-methyl-1,2-benzisoxazol-4-yl)oxy]aniline

The title compound (1.27 g) was obtained as pale-orange crystals by reaction in the same manner as in Example 168(ii) and using 4-(2-chloro-4-nitrophenoxy)-3-methyl-1,2-benzisoxazole (3.25 g), reduced iron (3.57 g), 1N hydrochloric acid (11 mL) and ethanol (100 mL).
$^1$H-NMR ($CDCl_3$) δ: 2.74 (3H, s), 3.74 (2H, br s), 6.32 (1H, dd, J = 0.6 Hz, 8.0 Hz), 6.62 (1H, dd, J = 2.6 Hz, 8.6 Hz), 6.82 (1H, d, J = 2.6 Hz), 7.00 (1H, d, J = 8.6 Hz), 7.16 (1H, dd, J = 0.6 Hz, 8.0 Hz), 7.31 (1H, t, J = 8.0 Hz).

(iii) Production of 2-[4-({3-chloro-4-[(3-methyl-1,2-benzisoxazol-4-yl)oxy]phenyl}amino)-5H-pyrrolo[3,2-d]pyrimidin-5-yl]ethanol

The title compound (65.5 mg) was obtained as white crystals by reaction in the same manner as in Example 168(iii) and using 2-(4-chloro-5H-pyrrolo[3,2-d]pyrimidin-5-yl)ethyl benzoate (100 mg), 3-chloro-4-[(3-methyl-1,2-benzisoxazol-4-yl)oxy]aniline (101 mg), isopropyl alcohol (3 mL) and 1N aqueous sodium hydroxide solution (1 mL).
$^1$H-NMR (DMSO-$d_6$) δ: 2.68 (3H, s), 3.89 (2H, t, J = 4.6 Hz), 4.54 (2H, t, J = 4.6 Hz), 6.34 (1H, br s), 6.46 (1H, d, J = 8.0 Hz), 6.52 (1H, d, J = 3.2 Hz), 7.35-7.45 (2H, m), 7.52 (1H, t, J = 8.0 Hz), 7.61-7.71 (2H, m), 8.03 (1H, d, J = 2.5 Hz), 8.35 (1H, s), 9.91 (1H, br s).
Example 197
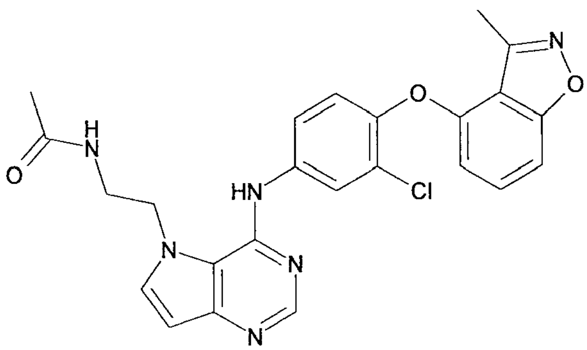

Production of N-{2-[4-({3-chloro-4-[(3-methyl-1,2-benzisoxazol-4-yl)oxy]phenyl}amino)-5H-pyrrolo[3,2-d]pyrimidin-5-yl]ethyl}acetamide

(i) Production of tert-butyl {2-[4-({3-chloro-4-[(3-methyl-1,2-benzisoxazol-4-yl)oxy]phenyl}amino)-5H-pyrrolo[3,2-d]pyrimidin-5-yl]ethyl}carbamate

The title compound (882 mg) was obtained as a white powder by reaction in the same manner as in Example 182(i) and using tert-butyl [2-(4-chloro-5H-pyrrolo[3,2-d]pyrimidin-5-yl)ethyl]carbamate (781 mg), 3-chloro-4-[(3-methyl-1,2-benzisoxazol-4-yl)oxy]aniline (608 mg) and isopropyl alcohol (10 mL).
$^1$H-NMR ($CDCl_3$) δ: 1.50 (9H, s), 2.75 (3H, s), 3.44-3.56 (2H, m), 4.42-4.55 (2H, m), 5.09 (1H, t, J = 5.6 Hz), 6.49 (1H, d, J = 8.0 Hz), 6.62 (1H, d, J = 3.0 Hz), 7.13-7.24 (3H, m), 7.36 (1H, t, J = 8.0 Hz), 7.95 (1H, dd, J = 2.6 Hz, 8.9 Hz), 8.06 (1H, d, J = 2.6 Hz), 8.52 (1H, s), 8.63 (1H, s).

(ii) Production of 5-(2-aminoethyl)-N-{3-chloro-4-[(3-methyl-1,2-benzisoxazol-4-yl)oxy]phenyl}-5H-pyrrolo[3,2-d]pyrimidin-4-amine

To a mixture of tert-butyl {2-[4-({3-chloro-4-[(3-methyl-1,2-benzisoxazol-4-yl)oxy]phenyl}amino)-5H-pyrrolo [3,2-d]pyrimidin-5-yl]ethyl}carbamate (876 mg), tetrahydrofuran (15 mL) and ethanol (15 mL) was added 4N hydrochloric acid/ethyl acetate (2 mL), and the mixture was stirred at 60°C for 5 hr. The reaction mixture was concentrated under reduced pressure and suspended in ethyl acetate. The suspension was washed successively with saturated aqueous sodium hydrogen carbonate solution and saturated brine, dried over anhydrous magnesium sulfate, and concentrated under reduced pressure. The residue was separated and purified by basic silica gel column chromatography (eluent, hexane:ethyl acetate=1:1→ethyl acetate→ethyl acetate:methanol=9:1) to give the title compound (177 mg) as a white powder.
$^1$H-NMR (DMSO-$d_6$) δ: 2.69 (3H, s), 2.99-3.14 (2H, m), 4.30-4.43 (2H, m), 6.03 (2H, br s), 6.44 (1H, d, J = 8.0 Hz), 6.50 (1H, d, J = 3.0 Hz), 7.33-7.43 (2H, m), 7.52 (1H, t, J = 8.0 Hz), 7.64 (1H, d, J = 3.0 Hz), 7.75 (1H, dd, J = 2.5 Hz, 8.8 Hz), 8.08 (1H, d, J = 2.5 Hz), 8.33 (1H, s).

(iii) Production of N-{2-[4-({3-chloro-4-[(3-methyl-1,2-benzisoxazol-4-yl)oxy]phenyl}amino)-5H-pyrrolo[3,2-d]pyrimidin-5-yl]ethyl}acetamide

To a mixture of 5-(2-aminoethyl)-N-{3-chloro-4-[(3-methyl-1,2-benzisoxazol-4-yl)oxy]phenyl}-5H-pyrrolo[3,2-d] pyrimidin-4-amine (131 mg), ethyl acetate (1.5 mL) and tetrahydrofuran (1.5 mL) were added acetic anhydride (34 μL) and saturated aqueous sodium hydrogen carbonate solution (1.5 mL), and the mixture was stirred at room temperature for 1 hr. Water was added to the reaction mixture, and the mixture was extracted with ethyl acetate. The organic layer was washed with saturated brine and anhydrous magnesium sulfate, and concentrated under reduced pressure. The residue was separated and purified by silica gel column chromatography (eluent, hexane:ethyl acetate=1:2→ethyl acetate→ethyl acetate:methanol=9:1) and crystallized from diisopropyl ether/ethyl acetate to give the title compound (103 mg) as white crystals.
$^1$H-NMR (DMSO-$d_6$) δ: 1.80 (3H, s), 2.68 (3H, s), 3.34-3.43 (2H, m), 4.52 (2H, t, J = 6.9 Hz), 6.48 (1H, d, J = 8.0 Hz), 6.51 (1H, d, J = 3.0 Hz), 7.34-7.44 (2H, m), 7.54 (1H, t, J = 8.0 Hz), 7.65 (1H, d, J = 3.0 Hz), 7.80 (1H, dd, J = 2.3 Hz, 8.7 Hz), 8.07 (1H, d, J = 2.3 Hz), 8.21-8.32 (1H, m), 8.35 (1H, s), 8.84 (1H, s).
Example 198
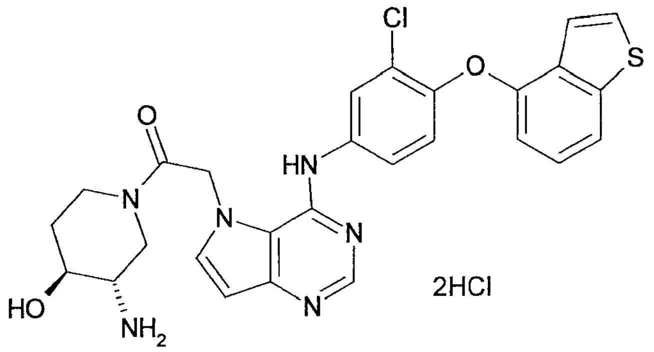

2HCl

Production of (trans)-3-amino-1-[(4-{[4-(1-benzothiophen-4-yloxy)-3-chlorophenyl]amino}-5H-pyrrolo[3,2-d]pyrimidin-5-yl)acetyl]piperidin-4-ol dihydrochloride

(i) Production of (trans)-3-azidopiperidin-4-ol hydrochloride

A mixture of tert-butyl (trans)-3-azido-4-hydroxypiperidine-1-carboxylate (3.58g, US2006/526523), ethyl acetate (30 mL) and 4N hydrochloric acid/ethyl acetate solution (25 mL) was stirred at room temperature for 6 hr. The precipitate was collected by filtration, and washed with ethyl acetate to give the title compound (2.26 g) as a white solid. The solid was used for the next step without further purification.

(ii) Production of (trans)-3-azido-1-[(4-chloro-5H-pyrrolo[3,2-d]pyrimidin-5-yl)acetyl]piperidin-4-ol

A mixture of (trans)-3-azidopiperidin-4-ol hydrochloride (305 mg), triethylamine (0.42 mL) and N,N-dimethylformamide (5 mL) was stirred at room temperature for 10 min. Chloroacetyl chloride (0.16 mL) was added, and the mixture was stirred at room temperature for 10 min and under ice-cooling for 10 min. The reaction mixture was poured into saturated aqueous sodium hydrogen carbonate solution, and the mixture was extracted with ethyl acetate. The extract was dried over anhydrous magnesium sulfate and concentrated under reduced pressure. The residue was dissolved in N,N-dimethylformamide (2 mL), 4-chloro-5H-pyrrolo[3,2-d]pyrimidine (307 mg), 60% sodium hydride (80 mg) and N,N-dimethylformamide (8 mL) were added and the mixture was stirred under ice-cooling for 20 min. After stirring under ice-cooling for 1 hr, the mixture was warmed to room temperature. After 1 hr, the reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (hexane:ethyl acetate=95:5→0:100) to give the title compound (163 mg) as a pale-yellow solid.
$^1$H-NMR ($CDCl_3$) δ: 1.56-1.71 (1H, m), 2.05-2.16 (1H, m), 2.88-3.98 (6H, m), 4.48 (1H, m), 5.28-5.43 (2H, m), 6.80 (1H, d, J = 3.0 Hz), 7.44 (1H, d, J = 3.3 Hz), 8.72 (1H, s).

(iii) Production of (trans)-3-amino-1-[(4-{[4-(1-benzothiophen-4-yloxy)-3-chlorophenyl]amino}-5H-pyrrolo[3,2-d]pyrimidin-5-yl)acetyl]piperidin-4-ol

A mixture of (trans)-3-azido-1-[(4-chloro-5H-pyrrolo[3,2-d]pyrimidin-5-yl)acetyl]piperidin-4-ol (160 mg), 4-(1-benzothiophen-4-yloxy)-3-chloroaniline (132 mg), pyridine hydrochloride (10 mg) and 1-methyl-2-pyrrolidone (5 mL) was heated to 75°C and stirred overnight. The reaction mixture was poured into saturated aqueous sodium hydrogen carbonate solution, and the mixture was extracted with ethyl acetate. The extract was dried over anhydrous magnesium sulfate and concentrated under reduced pressure. The residue was separated and purified by silica gel column chromatography (ethyl acetate:methanol=100:0→90:10). A mixture of the obtained concentrated product (301 mg), triphenylphosphine (289 mg), tetrahydrofuran (10 mL) and water (0.1 mL) was heated to 70°C, and the mixture was stirred for 3 hr. The solvent was evaporated under reduced pressure, and the residue was separated and purified by aminosilica gel column chromatography (ethyl acetate:methanol=100:0→80:20). The obtained amine was dissolved in ethanol (2 mL), and 4N hydrochloric acid/ethyl acetate solution (2 mL) was added. The solvent was evaporated under reduced pressure, the residue was treated with isopropyl alcohol/ethanol and the precipitated solid was collected by filtration to give the title compound (143 mg) as a pale-yellow solid.
$^1$H-NMR (DMSO-$d_6$) δ: 1.18-4.30 (8H, m), 5.77-6.09 (2H, m), 6.70-6.76 (2H, m), 7.17 (1H, d, J = 8.7 Hz), 7.37 (1H, t, J

= 7.8 Hz), 7.45-7.56 (2H, m), 7.81-8.01 (4H, m), 8.26-8.45 (3H, m), 9.74 (1H, s), 10.38 (1H, m).

Example 199

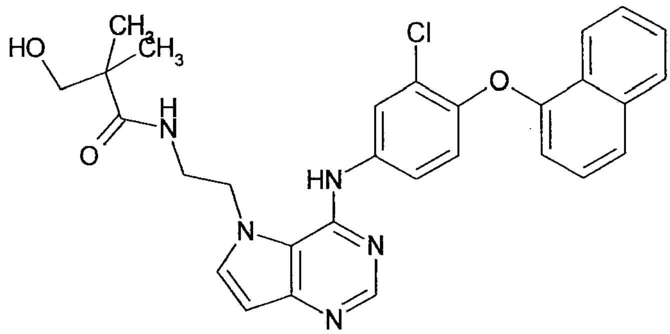

Production of N-[2-(4-{[3-chloro-4-(1-naphthyloxy)phenyl]amino}-5H-pyrrolo[3,2-d]pyrimidin-5-yl)ethyl]-3-hydroxy-2,2-dimethylpropanamide

(i) Production of 3-chloro-4-(1-naphthyloxy)aniline

N,N-Dimethylformamide (80 mL), potassium carbonate (5.53 g) and 2-chloro-1-fluoro-4-nitrobenzene (7.02 g) were added to 1-naphthol (5.77 g), and the mixture was stirred at room temperature overnight. The reaction mixture was partitioned between ethyl acetate and saturated aqueous sodium hydrogen carbonate solution. The organic layer was dried over anhydrous magnesium sulfate and concentrated under reduced pressure. Reduced iron (8.94 g), 6N hydrochloric acid (6.7 mL) and ethanol (160 mL) were added to the residue, and the mixture was heated to 70°C and stirred for 3 hr. The reaction mixture was cooled to 50°C, 8N sodium hydroxide (0.4 mL) was added, and the mixture was filtered through celite and celite was washed with ethanol. The filtrate was concentrated under reduced pressure, and the residue was partitioned between ethyl acetate and saturated aqueous sodium hydrogen carbonate solution. The organic layer was dried over anhydrous magnesium sulfate and concentrated under reduced pressure. The residue was separated and purified by silica gel column chromatography (hexane:ethyl acetate=95:5→50:50) to give the title compound (10.8 g) as a red oil.

$^1$H-NMR ($CDCl_3$) δ: 3.68 (2H, s), 6.56-6.63 (2H, m), 6.84 (1H, d, J = 3.0 Hz), 6.93 (1H, d, J = 8.7 Hz), 7.31 (1H, d, J = 7.8 Hz), 7.51-7.56 (3H, m), 7.83-7.86 (1H, m), 8.37-8.40 (1H, m).

(ii) Production of N-[2-(4-{[3-chloro-4-(1-naphthyloxy)phenyl]amino}-5H-pyrrolo[3,2-d]pyrimidin-5-yl)ethyl]-3-hydroxy-2,2-dimethylpropanamide

The title compound (191 mg) was synthesized in the same manner as in Example 166 and using tert-butyl [2-(4-chloro-5H-pyrrolo[3,2-d]pyrimidin-5-yl)ethyl]carbamate (162 mg), 3-chloro-4-(1-naphthyloxy)aniline (178 mg), isopropyl alcohol (2 mL), pyridine hydrochloride (5 mg), methanol (10 mL), 4N hydrochloric acid/ethyl acetate solution (4 mL), triethylamine (281 μL), N,N-dimethylformamide (5 mL), 3-hydroxy-2,2-dimethylpropanoic acid (89 mg), 1-hydroxybenzotriazole (101 mg) and 1-ethyl-3-(3-dimethylaminopropyl)carbodiimide hydrochloride (143 mg).

$^1$H-NMR (DMSO-$d_6$) δ: 0.99 (6H, s), 3.32-3.44 (4H, m), 4.49-4.53 (2H, m), 4.84 (1H, t, J = 5.4 Hz), 6.49 (1H, d, J = 3.0 Hz), 6.76 (1H, d, J = 7.2 Hz), 7.18 (1H, d, J = 9.0 Hz), 7.44 (1H, t, J = 8.1 Hz), 7.59-7.70 (4H, m), 7.82-7.88 (2H, m), 7.97-8.02 (1H, m), 8.11-8.12 (1H, m), 8.22-8.26 (1H, m), 8.33 (1H, s), 8.90 (1H, br s).

Example 200

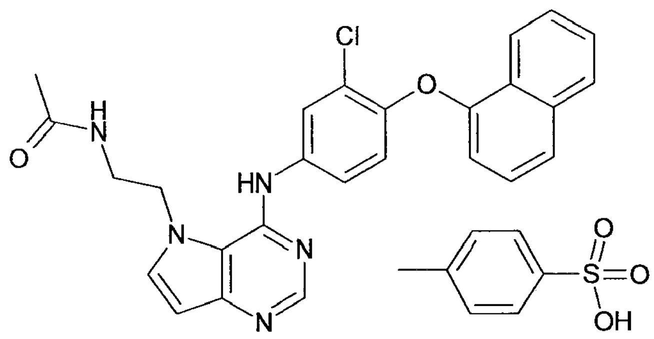

Production of N-[2-(4-{[3-chloro-4-(1-naphthyloxy)phenyl]amino}-5H-pyrrolo[3,2-d]pyrimidin-5-yl)ethyl]acetamide tosylate

A free form (234 mg) of the title compound was obtained in the same manner as in Example 166 and using tert-butyl [2-(4-chloro-5H-pyrrolo[3,2-d]pyrimidin-5-yl)ethyl]carbamate (162 mg), 3-chloro-4-(1-naphthyloxy)aniline (178 mg), isopropyl alcohol (2 mL), pyridine hydrochloride (5 mg), methanol (10 mL), 4N hydrochloric acid/ethyl acetate solution (4 mL), triethylamine (281 μL), N,N-dimethylformamide (5 mL), acetic acid (45 mg), 1-hydroxybenzotriazole (101 mg) and 1-ethyl-3-(3-dimethylaminopropyl)carbodiimide hydrochloride (143 mg). Ethyl acetate (5 mL), ethanol (3 mL) and tosylic acid monohydrate (90 mg) were added to the obtained free form, and the mixture was ultrasonicated. The precipitate was collected by filtration to give the title compound (158 mg) as a white solid.
$^1$H-NMR (DMSO-$d_6$) δ: 1.81 (3H, s), 2.29 (3H, s), 3.45 (2H, m), 4.58-4.62 (2H, m), 6.66 (1H, d, J = 3.0 Hz), 6.92 (1H, d, J = 6.9 Hz), 7.09-7.17 (3H, m), 7.46-7.66 (6H, m), 7.77 (1H, d, J = 8.4 Hz), 7.94-8.03 (3H, m), 8.14-8.17 (1H, m), 8.39 (1H, m), 8.71 (1H, s), 10.08 (1H, br s).
Example 201
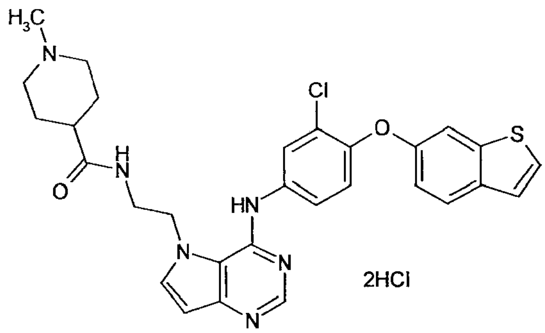

2HCl

Production of N-[2-(4-{[4-(1-benzothiophen-6-yloxy)-3-chlorophenyl]amino}-5H-pyrrolo[3,2-d]pyrimidin-5-yl)ethyl]-1-methylpiperidine-4-carboxamide dihydrochloride

Tetrahydrofuran (2 mL) was added to a mixture of N-[2-(4-{[4-(1-benzothiophen-6-yloxy)-3-chlorophenyl]amino}-5H-pyrrolo[3,2-d]pyrimidin-5-yl)ethyl]piperidine-4-carboxamide dihydrochloride (186 mg), sodium acetate (49 mg) and methanol (2 mL). A solution of formalin (37%, 49 mg) in tetrahydrofuran (3 mL) was added, and then sodium triacetoxyborohydride (76 mg) was added, and the mixture was stirred at room temperature overnight. Saturated aqueous sodium hydrogen carbonate solution was added to the reaction mixture, and the mixture was extracted with ethyl acetate. The organic layer was washed with saturated brine, and dried over anhydrous magnesium sulfate. The solvent was evaporated under reduced pressure and the obtained residue was subjected to basic silica gel column chromatography (eluent, methanol:ethyl acetate=0:100→20:80). The object fraction was concentrated under reduced pressure. The residue was dissolved in ethanol (3 mL), 1N hydrochloric acid/ethyl acetate (0.6 mL) was added. The reaction mixture was concentrated under reduced pressure and the residue was crystallized from isopropyl alcohol/ethyl acetate to give the title compound (168 mg) as a white powder.
$^1$H-NMR (DMSO-$d_6$) δ: 1.62-1.96 (4H, m), 2.20-2.36 (1H, m), 2.61-2.71 (3H, m), 2.75-3.05 (2H, m), 3.12-3.57 (4H, m), 4.64-4.82 (2H, m), 6.66 (1H, d, J = 3.0 Hz), 7.14 (1H, dd, J = 2.4 Hz, 8.6 Hz), 7.20 (1H, d, J = 8.9 Hz), 7.46 (1H, d, J =

5.5 Hz), 7.61-7.69 (2H, m), 7.71 (1H, d, J = 5.5 Hz), 7.88-8.01 (3H, m), 8.34-8.59 (1H, m), 8.72 (1H, s), 10.00-10.50 (1H, m), 10.15 (1H, br s).

Example 202

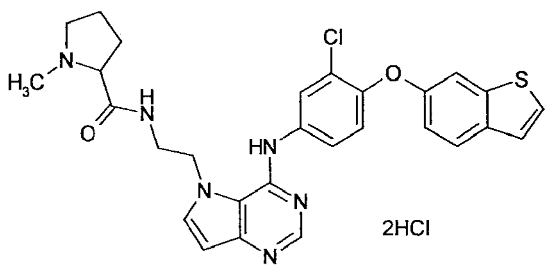

Production of N-[2-(4-{[4-(1-benzothiophen-6-yloxy)-3-chlorophenyl]amino}-5H-pyrrolo[3,2-d]pyrimidin-5-yl)ethyl]-1-methylprolinamide dihydrochloride

The title compound (160 mg) was obtained as a white powder in the same manner as in Example 201 and using N-[2-(4-{[4-(1-benzothiophen-6-yloxy)-3-chlorophenyl]amino}-5H-pyrrolo[3,2-d]pyrimidin-5-yl)ethyl]prolinamide dihydrochloride (182 mg), sodium acetate (49 mg), methanol (2 mL), tetrahydrofuran (2 mL), a solution of formalin (37%, 49 mg) in tetrahydrofuran (3 mL), sodium triacetoxyborohydride (76 mg), ethanol (3 mL) and 1N hydrochloric acid/ethyl acetate (0.6 mL).
$^1$H-NMR (DMSO-$d_6$) δ: 1.52-1.87 (2H, m), 1.89-2.11 (1H, m), 2.24-2.44 (1H, m), 2.67 (3H, br s), 2.99-3.77 (4H, m), 3.88-4.01 (1H, m), 4.78-5.00 (2H, m), 6.67 (1H, d, J = 3.1 Hz), 7.13 (1H, dd, J = 2.4 Hz, 8.6 Hz), 7.21 (1H, d, J = 8.9 Hz), 7.46 (1H, dd, J = 0.8 Hz, 5.5 Hz), 7.62 (1H, dd, J = 2.5 Hz, 8.9 Hz), 7.65 (1H, d, J = 2.4 Hz), 7.71 (1H, d, J = 5.5 Hz), 7.93 (1H, d, J = 8.6 Hz), 7.93 (1H, d, J = 2.5 Hz), 7.97 (1H, d, J = 3.1 Hz), 8.72 (1H, s), 9.16 (1H, t, J = 5.6 Hz), 9.62 (1H, br s), 10.21 (1H, br s).

Example 203

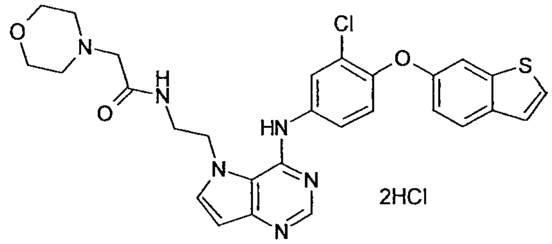

Production of N-[2-(4-{[4-(1-benzothiophen-6-yloxy)-3-chlorophenyl]amino}-5H-pyrrolo[3,2-d]pyrimidin-5-yl)ethyl]-2-(morpholin-4-yl)acetamide dihydrochloride

The title compound (234 mg) was obtained as a white powder in the same manner as in Example 57 (ii) and using 5-(2-aminoethyl)-N-[4-(1-benzothiophen-6-yloxy)-3-chlorophenyl]-5H-pyrrolo[3,2-d]pyrimidin-4-amine dihydrochloride (204 mg), (morpholin-4-yl)acetic acid hydrochloride (109 mg), 1-ethyl-3-(3-dimethylaminopropyl)carbodiimide hydrochloride (115 mg), 1-hydroxybenzotriazole (81 mg), triethylamine (0.223 mL), N,N-dimethylformamide (5 mL), ethyl acetate (2 mL)/ethanol (2 mL) and 1N hydrochloric acid/ethyl acetate (0.5 mL).
$^1$H-NMR (DMSO-$d_6$) δ: 3.08-3.25 (4H, m), 3.52-3.64 (2H, m), 3.74-3.94 (4H, m), 3.87 (2H, s), 4.87 (2H, t, J = 5.8 Hz), 6.69 (1H, d, J = 3.1 Hz), 7. 14 (1H, dd, J = 2. 5 Hz, 8.7 Hz), 7.21 (1H, d, J = 8.7 Hz), 7.45 (1H, dd, J = 0.6 Hz, 5.4 Hz), 7.62-7.69 (2H, m), 7.70 (1H, d, J = 5.4 Hz), 7.93 (1H, d, J = 8.7 Hz), 7.95 (1H, d, J = 2.5 Hz), 8.00 (1H, d, J = 3.1 Hz), 8.73 (1H, s), 9.13 (1H, t, J = 5.7 Hz), 10.31 (1H, br s).

Example 204

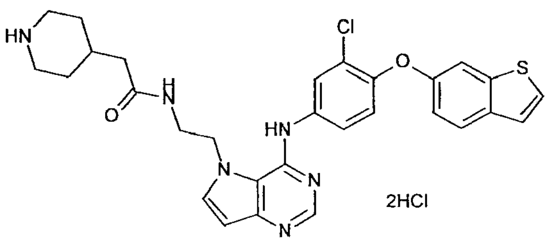

Production of N-[2-(4-{[4-(1-benzothiophen-6-yloxy)-3-chlorophenyl]amino}-5H-pyrrolo[3,2-d]pyrimidin-5-yl)ethyl]-2-(piperidin-4-yl)acetamide dihydrochloride

The title compound (458 mg) was obtained as a white powder in the same manner as in Example 64 and using 5-(2-aminoethyl)-N-[4-(1-benzothiophen-6-yloxy)-3-chlorophenyl]-5H-pyrrolo[3,2-d]pyrimidin-4-amine dihydrochloride (407 mg), [1-(tert-butoxycarbonyl)piperidin-4-yl]acetic acid (292 mg), 1-ethyl-3-(3-dimethylaminopropyl)carbodiimide hydrochloride (230 mg), 1-hydroxybenzotriazole (162 mg), triethylamine (0.335 mL), N,N-dimethylformamide (10 mL), concentrated hydrochloric acid (3 mL) and ethanol (9 mL).
$^1$H-NMR (DMSO-$d_6$) δ: 1.19-1.39 (2H, m), 1.58-1.73 (2H, m), 1.76-1.94 (1H, m), 2.02 (2H, d, J = 7.0 Hz), 2.70-2.88 (2H, m), 3.12-3.25 (2H, m), 3.32-3.60 (2H, m), 4.67 (2H, t, J = 6.5 Hz), 6.66 (1H, d, J = 3.0 Hz), 7.13 (1H, dd, J = 2.4 Hz, 8.6 Hz), 7.20 (1H, d, J = 8.9 Hz), 7.46 (1H, dd, J = 0.8 Hz, 5.5 Hz), 7.61-7.68 (2H, m), 7.71 (1H, d, J = 5.5 Hz), 7.90-7.98 (3H, m), 8.44 (1H, t, J = 5.4 Hz), 8.52-8.87 (2H, m), 8.71 (1H, s), 10.13 (1H, br s).

Example 205

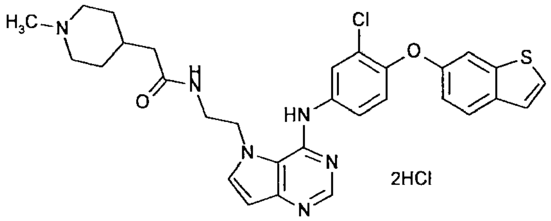

Production of N-[2-(4-{[4-(1-benzothiophen-6-yloxy)-3-chlorophenyl]amino}-5H-pyrrolo[3,2-d]pyrimidin-5-yl)ethyl]-2-(1-methylpiperidin-4-yl)acetamide dihydrochloride

The title compound (181 mg) was obtained as a white powder in the same manner as in Example 201 and using N-[2-(4-{[4-(1-benzothiophen-6-yloxy)-3-chlorophenyl]amino}-5H-pyrrolo[3,2-d]pyrimidin-5-yl)ethyl]-2-(piperidin-4-yl)acetamide dihydrochloride (190 mg), sodium acetate (49 mg), methanol (2 mL), tetrahydrofuran (2 mL), formalin (37%, 49 mg) in tetrahydrofuran (3 mL)solution, sodium triacetoxyborohydride (76 mg), ethanol (3 mL) and 1N hydrochloric acid/ethyl acetate (0.6 mL).
$^1$H-NMR (DMSO-$d_6$) δ: 1.33-1.57 (2H, m), 1.59-1.88 (3H, m), 2.01 (2H, d, J = 6.8 Hz), 2.65 (3H, d, J = 4.7 Hz), 2.76-2.94 (2H, m), 3.20-3.60 (4H, m), 4.71 (2H, t, J = 6.4 Hz), 6.68 (1H, d, J = 3.2 Hz), 7.14 (1H, dd, J = 2.4 Hz, 8.6 Hz), 7.20 (1H, d, J = 8.9 Hz), 7.46 (1H, dd, J = 0.6 Hz, 5.5 Hz), 7.62-7.69 (2H, m), 7.71 (1H, d, J = 5.5 Hz), 7.91-7.96 (2H, m), 7.97 (1H, d, J = 3.2 Hz), 8.48 (1H, t, J = 5.6 Hz), 8.72 (1H, s), 10.24 (1H, br s), 10.50 (1H, br s).

Example 206

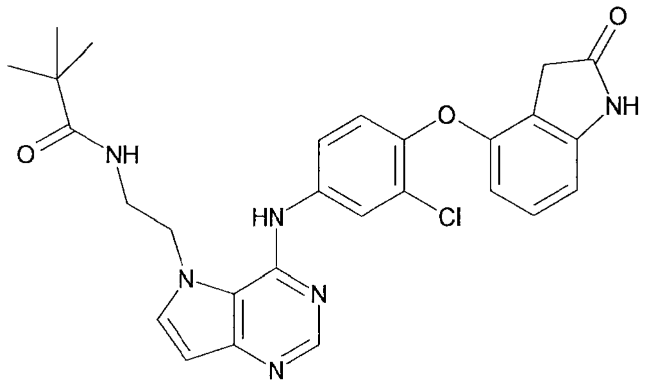

Production of N-{2-[4-({3-chloro-4-[(2-oxo-2,3-dihydro-1H-indol-4-yl)oxy]phenyl}amino)-5H-pyrrolo[3,2-d]pyrimidin-5-yl] ethyl}-2,2-dimethylpropanamide

4-(4-{[5-(2-Aminoethyl)-5H-pyrrolo[3,2-d]pyrimidin-4-yl]amino}-2-chlorophenoxy)-1,3-dihydro-2H-indol-2-one dihydrochloride (100 mg) was dissolved in N,N-dimethylformamide (25 mL), 2,2-dimethylpropanoic acid anhydride (0.7 mL) and triethylamine (1.5 mL) were added at 0°C, and the mixture was stirred at room temperature for 2 hr. Saturated aqueous sodium hydrogen carbonate solution was added to the reaction mixture, and the mixture was extracted with ethyl acetate. The organic layer was washed with saturated brine, dried over anhydrous magnesium sulfate, and concentrated under reduced pressure. The residue was separated and purified by silica gel column chromatography (eluent, methanol:ethyl acetate=0:100→20:80) to give the title compound (37 mg) as a white powder.
$^1$H-NMR (DMSO-$d_6$) δ: 1.01 (9H, s), 3.67-3.78 (2H, m), 4.50 (2H, t, J = 6.6 Hz), 6.33 (1H, d, J = 7.2 Hz), 6.48 (1H, d, J = 3.0 Hz), 6.62 (1H, d, J = 7.2 Hz), 6.93-7.26 (2H, m), 7.56 (1H, d, J = 3.0 Hz), 7.70-7.87 (2H, m), 8.01 (1H, d, J = 2.5 Hz), 8.30 (1H, s), 8.82 (1H, s), 10.55 (1H, s).

Example 207

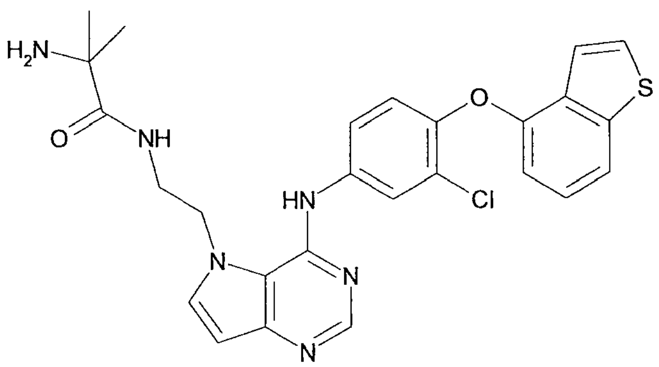

Production of N-[2-(4-{[4-(1-benzothiophen-4-yloxy)-3-chlorophenyl]amino}-5H-pyrrolo[3,2-d]pyrimidin-5-yl)ethyl]-2-methylalaninamide

The title compound (213 mg) was obtained as a white powder by reaction in the same manner as in Example 90 (ii) and using 5-(2-aminoethyl)-N-[4-(1-benzothiophen-4-yloxy)-3-chlorophenyl]-5H-pyrrolo[3,2-d]pyrimidin-4-amine dihydrochloride (300 mg), N-(tert-butoxycarbonyl)-2-methylalanine (150 mg), N,N-dimethylformamide (50 mL), triethylamine (1.5 mL), 1-hydroxybenzotriazole (20 mg) and 1-ethyl-3-(3-dimethylaminopropyl)carbodiimide hydrochloride (450 mg).
$^1$H-NMR (DMSO-$d_6$) δ: 1.11 (6H, s), 1.70-1.94 (2H, m), 3.43 (2H, t, J = 6.5 Hz), 4.53 (2H, t, J = 6.5 Hz), 6.48 (1H, d, J = 3.0 Hz), 6.65 (1H, d, J = 7.0 Hz), 7.08-7.24 (1H, m), 7.32 (1H, t, J = 8.0 Hz), 7.48-7.63 (2H, m), 7.68-7.89 (3H, m), 8.06 (1H, d, J = 2.5 Hz), 8.32 (1H, s), 8.83 (1H, s).

**Formulation Example 1** (amount per tablet)

| | |
|---|---|
| (1) Compound obtained in Example 22 | 10.0 mg |
| (2) Lactose | 60.0 mg |
| (3) Corn starch | 35.0 mg |
| (4) Gelatin | 3.0 mg |
| (5) Magnesium stearate | 2.0 mg |

A mixture of 10.0 mg of the compound obtained in Example 22, 60.0 mg of lactose and 35.0 mg of corn starch is granulated through a 1 mm-mesh sieve using 0.03 ml of a 10% by weight aqueous solution of gelatin (3.0 mg of gelatin), after which the granules are dried at 40°C and filtered again. The obtained granules are mixed with 2.0 mg of magnesium stearate and compressed. The obtained core tablets are coated with a sugar coat comprising a suspension of sucrose, titanium dioxide, talc and gum arabic and polished with beeswax to yield sugar-coated tablets.

**Formulation Example 2** (dose per tablet)

| | |
|---|---|
| (1) Compound obtained in Example 22 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

10.0 mg of the compound obtained in Example 22 and 3.0 mg of magnesium stearate are granulated using 0.07 ml of an aqueous solution of soluble starch (7.0 mg of soluble starch), after which these granules are dried and mixed with 70.0 mg of lactose and 50.0 mg of corn starch. This mixture is compressed to yield tablets.

**Experimental Example 1A** Cloning of human HER2 gene and preparation of recombinant baculovirus

Human HER2 gene was cloned by RT-PCR using total RNA prepared from MCF7 cells as a template. The primer used for RT-PCR was prepared in consideration of the information of nucleotide sequence (Genbank Accession No. M11730) of HER2 gene by adding a nucleotide sequence encoding DYKDDDD peptide and a restriction enzyme recognition sequence to a nucleotide sequence (corresponding to 2176-3918 of Genbank Accession No. M11730 as a base, 676-1255 amino acids of Genbank Accession No. NP_004439 as a protein) encoding the HER2 intracellular domain region such that the protein contains an N-terminal DYKDDDD peptide tag. The primer nucleotide sequence is shown below.

HER2-U:5'-
AATTAAGTCGACATGGACTACAAAGACGATGACGACAAGCGACGGCAGCAGAAGATCCGGAA
GTAC-3'(SEQ ID NO:1)

and
HER2-L:
5'-AATTAAGCATGCTCACACTGGCACGTCCAGACCCAGGTACTC-3'(SEQ ID NO:2)
The RT reaction was conducted using SuperScript First-Strand Synthesis System for RT-PCR (Invitrogen) and the PCR reaction was conducted using a KOD-plus kit (TOYOBO). The obtained PCR product was electrophoresed on agarose gel (1%), the DNA fragment amplified by PCR was recovered from the gel, and then digested with restriction enzymes Sal I and Sph I. The DNA treated with the restriction enzymes was electrophoresed on agarose gel (1%), and the obtained DNA fragment was recovered and ligated to plasmid pFASTBAC1 (Invitrogen) digested with restriction enzymes Sal I and Sph I to give expression plasmid pFB-HER2. The nucleotide sequence of the insertion fragment was confirmed and

found to be identical with the nucleotide sequence (2176-3918 of Genbank Accession M11730) of HER2 intracellular domain. Furthermore, using BAC-TO-BAC Baculovirus Expression System (Invitrogen), recombinant baculovirus BAC-HER2 was prepared.

**Experimental Example 1B** Preparation of HER2 intracellular domain protein

SF-21 cells were sown at $1\times10^6$ cells/mL in Sf-900II SFM medium (1 L, Invitrogen) containing 10% fetal bovine serum (trace), 50 mg/L gentamicin (Invitrogen) and 0.1% Pluronic F-68 (Invitrogen), and shaking culture was performed using a 2 L volume Erlenmeyer flask at 27°C, 100 rpm. After culturing for 24 hr, recombinant baculovirus BAC-HER2 (13.4 mL) was added, and the mixture was further cultured for 3 days. The culture medium was centrifuged at 2,000 rpm for 5 min to give virus-infected cells. The infected cells were washed with a phosphate buffered saline (Invitrogen), centrifuged under the same conditions, and the cells were preserved at -80°C. The cryopreserved cells were thawed in ice, suspended in buffer A (50 mM Tris buffer (30 mL, pH 7.4) containing 20% glycerol, 0.15 M NaCl) supplemented with Complete Protease Inhibitor (Boehringer), and ruptured 3 times with a Polytron homogenizer (Kinematica) at 20,000 rpm for 30 sec. The ruptured solution was clarified by centrifugation at 40,000 rpm for 30 min, and filtered with a 0.45 μm filter. The filtrate was passed through a column packed with Anti-FLAG M2 Affinity Gel (4 mL, Sigma-Aldrich) at a flow rate of about 0.5 mL/min. The column was washed with buffer A, and eluted with buffer A containing 100 μg/mL of FLAG peptide. The eluent was concentrated with Vivaspin 20 (Vivascience) having a molecular weight cut off of 30K. The concentrate was purified by gel filtration using Hi Load 16/60 Superdex 200 pg (GE Healthcare Bioscience) equilibrated with buffer A. The fractions containing HER2 intracellular domain were collected and cryopreserved at -80°C.

**Experimental Example 1C** Determination of HER2 kinase inhibitory activity

HER2 kinase reaction was performed using a 96 well plate. As a buffer for kinase reaction, a buffer having a composition of 50 mM Tris-HCl (pH 7.5), 5 mM $MnCl_2$, 2 mM dithiothreitol, 0.01% Tween-20 was used. The compound of the present invention was dissolved in dimethyl sulfoxide (DMSO), and diluted with the kinase reaction buffer so that DMSO concentration would be 0.1% during kinase reaction. This compound solution (10 μL) was mixed with a kinase reaction buffer (20 μL) containing HER2 intracellular domain (0.625 μg/mL) obtained in Experimental Example 1B and polypeptide substrate poly-Glu:Tyr (4:1) (Sigma Ltd., 12.5 μg/mL), and the mixture was stood at room temperature for 5 min. Then, a kinase reaction buffer (20 μL) containing 125 μM ATP and 45 μCi/mL [$\gamma$-$^{32}$P]ATP was added, and the kinase reaction was initiated with 50 μL of the final reaction mixture. A kinase reaction was performed at room temperature for 10 min, and the kinase reaction was quenched by addition of 20% TCA solution (50 μL). The mixture after completion of the reaction was stood at room temperature for 30 min, and acid insoluble fractions in the mixture after completion of the reaction were collected in a 96 well GF/C filter plate (PerkinElmer) using a cell harvester (PerkinElmer). Thereafter, the filter containing the acid insoluble fractions was washed with 3% phosphoric acid solution. After washing, the filter plate was dried at 45°C for 60 min, and 25 μL of MicroScinti 0 (PerkinElmer) was added. The radioactivity was measured using TopCount (PerkinElmer). HER2 kinase inhibitory rate (%) of the test compound was calculated by the following formula:

$$\text{Inhibitory rate (\%)} = (1 - (\text{count of test compound} - \text{blank}) \div (\text{control} - \text{blank})) \times 100$$

The count of the solution reacted without addition of the compound was used as a "control", and the count of the solution without the compound and HER2 intracellular domain was used as a "blank". The results of the inhibitory rate of the compounds are shown in Table 1.
From the foregoing, it was shown that the compounds of the present invention strongly inhibited the activity of HER2 kinase.

[Table 1]

| Example No. | inhibitory rate (%) at 1.0 μM |
|---|---|
| 10 | 99 |
| 16 | 100 |
| 26 | 100 |
| 28 | 100 |

(continued)

| | |
|---|---|
| 29 | 100 |
| 83 | 99 |
| 95 | 100 |
| 127 | 99 |
| 159 | 100 |
| 164 | 100 |
| 170 | 97 |

**Experimental Example 2A** Cloning of human EGFR gene and preparation of recombinant baculovirus

Human EGFR gene was cloned by RT-PCR using total RNA prepared from A431 cells as a template. The primer used for RT-PCR was prepared in consideration of the information of nucleotide sequence (Genbank Accession No. X00588) of EGFR gene by adding a nucleotide sequence encoding DYKDDDD peptide and a restriction enzyme recognition sequence to a nucleotide sequence (corresponding to 2191-3819 of Genbank Accession No. X00588 as a base, 669-1210 amino acids of Genbank Accession No. NP_005219 as a protein) encoding the EGFR intracellular domain region such that the protein contains an N-terminal DYKDDDD peptide tag. The primer nucleotide sequence is shown below.

EGFR-U:5'-
AATTAAGTCGACATGGACTACAAAGACGATGACGACCGAAGGCGCCACATCGTTCGGAAGCG
CACG-3'(SEQ ID NO: 3)

and
EGFR-L:5'-AATTAAGCATGCTCATGCTCCAATAAATTCACTGCTTTGTGG-3' (SEQ ID NO: 4)
The RT reaction was conducted using SuperScript First-Strand Synthesis System for RT-PCR (Invitrogen) and the PCR reaction was conducted using a KOD-plus kit (TOYOBO). The obtained PCR product was electrophoresed on agarose gel (1%), the DNA fragment amplified by PCR was recovered from the gel, and then digested with restriction enzymes Sal I and Sph I. The DNA treated with the restriction enzymes was electrophoresed on agarose gel (1%), and the obtained DNA fragment was recovered and ligated to plasmid pFASTBAC1 (Invitrogen) digested with restriction enzymes Sal I and Sph I to give expression plasmid pFB-EGFR. The nucleotide sequence of the insertion fragment was confirmed and found to be identical with the nucleotide sequence (2191-3819 of Genbank Accession X00588) of EGFR intracellular domain. Furthermore, using BAC-TO-BAC Baculovirus Expression System (Invitrogen), recombinant baculovirus BAC-EGFR was prepared.

**Experimental Example 2B** Preparation of human EGFR intracellular domain protein

SF-21 cells were sown at $1\times10^6$ cells/mL in Sf-900II SFM medium (1 L, Invitrogen) containing 10% fetal bovine serum (trace), 50 mg/L gentamicin (Invitrogen) and 0.1% Pluronic F-68 (Invitrogen), and shaking culture was performed using a 2 L volume Erlenmeyer flask at 27°C, 100 rpm. After culturing for 24 hrs, recombinant baculovirus BAC-EGFR (13.4 mL) was added, and the mixture was further cultured for 3 days. The culture medium was centrifuged at 2,000 rpm for 5 min to give virus-infected cells. The infected cells were washed with a phosphate buffered saline (Invitrogen), centrifuged under the same conditions, and the cells were preserved at -80°C. The cryopreserved cells were thawed in ice, suspended in buffer A (50 mM Tris buffer (30 mL, pH 7.4) containing 20% glycerol, 0.15 M NaCl) supplemented with Complete Protease Inhibitor (Boehringer), and ruptured 3 times with a Polytron homogenizer (Kinematica) at 20,000 rpm for 30 sec. The ruptured solution was clarified by centrifugation at 40,000 rpm for 30 min, and filtered with a 0.45 μm filter. The filtrate was passed through a column packed with Anti-FLAG M2 Affinity Gel (4 mL, Sigma-Aldrich) at a flow rate of about 0.5 mL/min. The column was washed with buffer A, and eluted with buffer A containing 100 μg/mL of FLAG peptide. The eluent was concentrated with Vivaspin 20 (Vivascience) having a molecular weight cut off of 30K. The concentrate was purified by gel filtration using Hi Load 16/60 Superdex 200 pg (GE Healthcare Bioscience) equilibrated with buffer A. The fractions containing EGFR intracellular domain were collected and cryopreserved at -80°C.

**Experimental Example 2C** Determination of EGFR kinase inhibitory activity

EGFR kinase reaction was performed using a 96 well plate. As a buffer for kinase reaction, a buffer having a composition of 50 mM Tris-HCl (pH 7.5), 5 mM $MnCl_2$, 2 mM dithiothreitol, 0.01% Tween-20 was used. The compound of the present invention was dissolved in dimethyl sulfoxide (DMSO), and diluted with the kinase reaction buffer so that DMSO concentration would be 0.1% during kinase reaction. This compound solution (10 μL) was mixed with a kinase reaction buffer (20 μL) containing EGFR intracellular domain (0.625 μg/mL) obtained in Experimental Example 2B and polypeptide substrate poly-Glu:Tyr (4:1) (Sigma Ltd., 12.5 μg/mL), and the mixture was stood at room temperature for 5 min. Then, a kinase reaction buffer (20 μL) containing 125 μM ATP and 45 μCi/mL [$\gamma$-$^{32}$P]ATP was added, and the kinase reaction was initiated with 50 μL of the final reaction mixture. A kinase reaction was performed at room temperature for 10 min, and the kinase reaction was quenched by addition of 20% TCA solution (50 μL). The mixture after completion of the reaction was stood at room temperature for 30 min, and acid insoluble fractions in the mixture after completion of the reaction were collected in a 96 well GF/C filter plate (PerkinElmer) using a cell harvester (PerkinElmer). Thereafter, the filter containing the acid insoluble fractions was washed with 3% phosphoric acid solution. After washing, the filter plate was dried at 45°C for 60 min, and 25 μL of MicroScinti 0 (PerkinElmer) was added. The radioactivity was measured using TopCount (PerkinElmer). EGFR kinase inhibitory rate (%) of the test compound was calculated by the following formula:

Inhibitory rate (%)=(1-(count of test compound - blank)÷(control - blank))×100

The count of the solution reacted without addition of the compound was used as a "control", and the count of the solution without the compound and EGFR intracellular domain was used as a "blank". The results of the inhibitory rate of the compounds are shown in Table 2.
From the foregoing, it was shown that the compounds of the present invention strongly inhibited the activity of EGFR kinase.
[Table 2]

| Example No. | inhibitory rate (%) at 1.0 μM |
|---|---|
| 6 | 100 |
| 8 | 100 |
| 16 | 99 |
| 22 | 98 |
| 53 | 98 |
| 60 | 99 |
| 99 | 98 |
| 119 | 99 |

**Experimental Example 3** Inhibitory action on breast cancer cell BT-474 proliferation in vitro

A suspension of human breast cancer cell BT-474 (100 μl (6,000 cells)) were plated in a 96-well microplate and cultured in an incubator (37°C, 5% carbon dioxide). On the following day, 100 μl of a solution of each test compound previously diluted serially in 2-fold, was added, and the cells were cultured for 5 days. After the culture medium containing the test compound was removed, the cells were washed and fixed with 50% trichloroacetic acid, after which a 0.4% (w/v) SRB dye solution (dissolved in 1% acetic acid) was added to stain as well as fix the cell protein (Skehan et al., Journal of the National Cancer Institute, Vol. 82, pp. 1107-1112, 1990). After washing with a 1% acetic acid solution, 100 μl of extract solution (10 mM, Tris buffer) was added to extract the dye and the absorbance was measured at a wavelength of 550 nm to quantify the amount of cells as protein content. Taking as 100% the protein content for the control group, which received no test compound solution, the ratio of the residual protein content for each treatment group was determined, and the compound concentration required to achieve 50% suppression of the residual cell content relative to the control ($IC_{50}$ value) was calculated. The results are shown in Table 3.
[Table 3]

| Example No. | $IC_{50}$ (nM) |
|---|---|
| 6 | <100 |
| 8 | <100 |
| 10 | <100 |
| 22 | <100 |
| 26 | <100 |
| 28 | <100 |
| 86 | <100 |
| 95 | <100 |
| 114 | <100 |
| 147 | <100 |
| 166 | <100 |

## Industrial Applicability

According to the present invention, pyrrolo[3,2-d]pyrimidine compound, a production method thereof and use thereof are provided. These fused pyrimidine compounds have a superior tyrosine kinase inhibitory action, are highly safe, and are sufficiently satisfactory as pharmaceutical products.

This application is based on JP2006/335158 filed in Japan, the contents of which are incorporated in full herein by this reference.

## Claims

**1.** A compound represented by the formula:

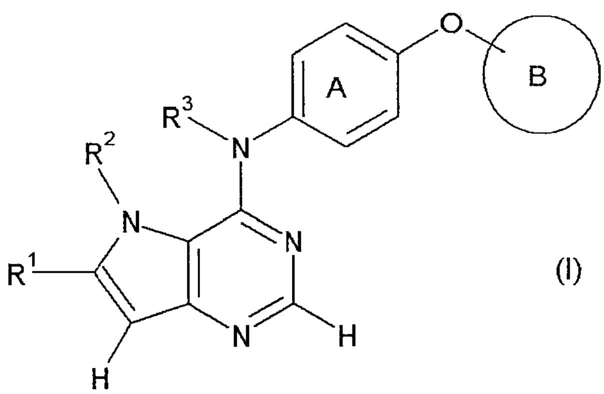

(I)

wherein
$R^1$ is a hydrogen atom, a halogen atom, or an optionally substituted group bonded via a carbon atom, a nitrogen atom or an oxygen atom;
$R^2$ is a hydrogen atom, or an optionally substituted group bonded via a carbon atom or a sulfur atom, or

$R^1$ and $R^2$, or $R^2$ and $R^3$ are optionally bonded to each other to form an optionally substituted ring structure;
$R^3$ is a hydrogen atom or an optionally substituted aliphatic hydrocarbon group, or
$R^3$ is optionally bonded to the carbon atom on ring A to form an optionally substituted ring structure;
ring A is an optionally substituted benzene ring; and
ring B is

(i) an optionally substituted fused ring, or
(ii) a pyridine ring having optionally substituted carbamoyl (the pyridine ring is optionally further substituted),

or a salt thereof.

**2.** The compound of claim 1, wherein $R^1$ is a hydrogen atom, a halogen atom or cyano.

**3.** The compound of claim 1, wherein $R^2$ is a hydrogen atom or optionally substituted alkyl.

**4.** The compound of claim 1, wherein $R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) $-O-(CH_2)_n-OH$,
(c) $-NR^5-(CH_2)_n-OH$,
(d) $-NR^5-CO-(C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) $-NR^5-CO-(CH_2)_n-SO_2-C_{1-4}$ alkyl,
(f) $-NR^5-CO-$(a 5- or 6-membered heterocycle optionally substituted by 1 or 2 $C_{1-4}$ alkyl),
(g) $-NR^5CO-(CH_2)_n-$(a 6-membered heterocycle optionally substituted by $C_{1-4}$ alkyl),
(h) $-NR^5-CO-(C_{3-6}$ cycloalkyl optionally substituted by substituent(s) selected from hydroxy and cyano),
(i) $-NR^5-CO-(C_{6-10}$ aryl optionally substituted by $C_{1-4}$ alkyl optionally substituted by 1 to 3 halogen atoms),
(j) $-NR^5-CO-NR^{5'}-C_{3-6}$ cycloalkyl,
(k) $-NR^5-SO_2-C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo, wherein n is an integer of 1 to 4, $R^5$ and $R^{5'}$ are each a hydrogen atom or $C_{1-4}$ alkyl, and $-(CH_2)_n-$ is optionally substituted by $C_{1-4}$ alkyl.

**5.** The compound of claim 1, wherein $R^3$ is a hydrogen atom.

**6.** The compound of claim 1, wherein ring A is a benzene ring optionally substituted by 1 or 2 substituents selected from the group consisting of (1) a halogen atom and (2) $C_{1-4}$ alkyl.

**7.** The compound of claim 1, wherein ring B is

(1) a fused ring optionally substituted by substituent(s) selected from the group consisting of

(a) a halogen atom,
(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and

(i) $C_{1-6}$ alkoxy-carbonyl, or

(2) a pyridine ring having carbamoyl optionally substituted by $C_{1-6}$ alkyl (the pyridine ring is optionally further substituted by $C_{1-6}$ alkyl).

**8.** The compound of claim 1, wherein
$R^1$ is a hydrogen atom, a halogen atom or cyano;
$R^2$ is

(1) a hydrogen atom, or
(2) $C_{1-6}$ alkyl optionally substituted by 1 to 3 substituents selected from the group consisting of

(a) $C_{3-6}$ cycloalkyl,
(b) -O-$(CH_2)_n$-OH,
(c) -$NR^5$-$(CH_2)_n$-OH,
(d) -$NR^5$-CO-($C_{1-4}$ alkyl optionally substituted by 1 to 4 substituents selected from hydroxy, a halogen atom, cyano, $C_{1-4}$ alkoxy, amino and di-$C_{1-4}$ alkylamino),
(e) -$NR^5$-CO-$(CH_2)_n$-$SO_2$-$C_{1-4}$ alkyl,
(f) -$NR^5$-CO-(a 5- or 6-membered heterocycle optionally substituted by 1 or 2 $C_{1-4}$ alkyl),
(g) -$NR^5$-CO-$(CH_2)_n$-(a 6-membered heterocycle optionally substituted by $C_{1-4}$ alkyl),
(h) -$NR^5$-CO-($C_{3-6}$ cycloalkyl optionally substituted by substituent(s) selected from hydroxy and cyano),
(i) -$NR^5$-CO- ($C_{6-10}$ aryl optionally substituted by $C_{1-4}$ alkyl optionally substituted by 1 to 3 halogen atoms),
(j) -$NR^5$-CO-$NR^{5'}$-$C_{3-6}$ cycloalkyl,
(k) -$NR^5$-$SO_2$-$C_{1-4}$ alkyl,
(l) $C_{1-4}$ alkyl-carbonyl,
(m) (a 5- or 6-membered heterocycle optionally substituted by 1 or 2 substituents selected from hydroxyl and amino)-carbonyl,
(n) hydroxy,
(o) a halogen atom, and
(p) 3- to 6-membered cyclic amino optionally substituted by 1 to 3 substituents selected from $C_{1-4}$ alkyl and oxo, wherein n is an integer of 1 to 4, $R^5$ and $R^{5'}$ are each a hydrogen atom or $C_{1-4}$ alkyl, and -$(CH_2)_n$- is optionally substituted by $C_{1-4}$ alkyl;

$R^3$ is a hydrogen atom;
ring A is a benzene ring optionally substituted by 1 or 2 substituents selected from the group consisting of (1) a halogen atom and (2) $C_{1-4}$ alkyl; and
ring B is
(1) a fused ring optionally substituted by substituent(s) selected from the group consisting of

(a) a halogen atom,
(b) cyano,
(c) $C_{1-6}$ alkyl optionally substituted by a halogen atom or $C_{3-6}$ cycloalkyl,
(d) oxo,
(e) $C_{2-4}$ alkylene,
(f) hydroxy,
(g) carbamoyl,
(h) $C_{1-6}$ alkyl-carbamoyl, and
(i) $C_{1-6}$ alkoxy-carbonyl, or

(2) a pyridine ring having carbamoyl optionally substituted by $C_{1-6}$ alkyl (the pyridine ring is optionally further substituted by $C_{1-6}$ alkyl) .

**9.** N-{2-[4-({3-Chloro-4-[(2-oxo-2,3-dihydro-1H-indol-4-yl)oxy]phenyl}amino)-5H-pyrrolo[3,2-d]pyrimidin-5-yl]ethyl}-2-(methylsulfonyl)acetamide;
4-(2-chloro-4-{[5-(2-hydroxyethyl)-5H-pyrrolo[3,2-d]pyrimidin-4-yl]amino}phenoxy)-1-methyl-1,3-dihydro-2H-indol-2-one;
2-(4-{[3-chloro-4-(1H-indol-4-yloxy)phenyl]amino}-5H-pyrrolo[3,2-d]pyrimidin-5-yl)ethanol;
N-[3-chloro-4-(1H-indol-4-yloxy)phenyl]-5-methyl-5H-pyrrolo[3,2-d]pyrimidin-4-amine;

4-(2-chloro-4-{[5-(2-hydroxyethyl)-5H-pyrrolo[3,2-d]pyrimidin-4-yl]amino}phenoxy)-1,3-dihydro-2H-indol-2-one;
N-[2-(4-{[4-(1-benzothiophen-6-yloxy)-3-chlorophenyl]amino}-5H-pyrrolo[3,2-d]pyrimidin-5-yl)ethyl]-3-hydroxy-2,2-dimethylpropanamide;
N-[2-(4-{[4-(1-benzothiophen-6-yloxy)-3-chlorophenyl]amino}-5H-pyrrolo[3,2-d]pyrimidin-5-yl)ethyl]-2-(methylsulfonyl)acetamide;
2-(4-{[4-(1-benzothiophen-4-yloxy)-3-chlorophenyl]amino}-5H-pyrrolo[3,2-d]pyrimidin-5-yl)ethanol;
N-[3-chloro-4-(1H-indazol-4-yloxy)phenyl]-5-methyl-5H-pyrrolo[3,2-d]pyrimidin-4-amine;
2-(4-{[3-chloro-4-(1H-indazol-4-yloxy)phenyl]amino}-5H-pyrrolo[3,2-d]pyrimidin-5-yl)ethanol;
N-[2-(4-{[4-(1-benzothiophen-4-yloxy)-3-chlorophenyl]amino}-5H-pyrrolo[3,2-d]pyrimidin-5-yl)ethyl]acetamide;
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide;
N-[2-(4-{[4-(1-benzothiophen-4-yloxy)-3-chlorophenyl]amino}-5H-pyrrolo[3,2-d]pyrimidin-5-yl)ethyl]acetamide;
N-[2-(4-{[4-(1-benzothiophen-4-yloxy)-3-chlorophenyl]amino}-5H-pyrrolo[3,2-d]pyrimidin-5-yl)ethyl]-3-hydroxy-2,2-dimethylpropanamide;
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-3-methylbutanamide;
N-[2-(4-{[3-chloro-4-(pyrazolo[1,5-a]pyridin-6-yloxy)phenyl]amino}-5H-pyrrolo[3,2-d]pyrimidin-5-yl)ethyl]-3-hydroxy-2,2-dimethylpropanamide;
N-[2-(4-{[3-chloro-4-(imidazo[1,2-a]pyridin-7-yloxy)phenyl]amino}-5H-pyrrolo[3,2-d]pyrimidin-5-yl)ethyl]-3-hydroxy-2,2-dimethylpropanamide;
N-[2-(4-{[3-chloro-4-([1,2,4]triazolo[1,5-a]pyridin-7-yloxy)phenyl]amino}-5H-pyrrolo[3,2-d]pyrimidin-5-yl)ethyl]-3-hydroxy-2,2-dimethylpropanamide;
4-(2-chloro-4-{[6-chloro-5-(2-hydroxyethyl)-5H-pyrrolo[3,2-d]pyrimidin-4-yl]amino}phenoxy)-1-methyl-1,3-dihydro-2H-indol-2-one; or
N-[2-(4-{[4-(1-benzothiophen-4-yloxy)-3-chlorophenyl]amino}-5H-pyrrolo[3,2-d]pyrimidin-5-yl)ethyl]-2-methylalaninamide or a salt thereof.

**10.** A prodrug of the compound of claim 1.

**11.** A pharmaceutical agent comprising the compound of claim 1 or a salt thereof, or a prodrug thereof.

**12.** The pharmaceutical agent of claim 11, which is a tyrosine kinase inhibitor.

**13.** The pharmaceutical agent of claim 11, which is an agent for the prophylaxis or treatment of cancer.

**14.** The pharmaceutical agent of claim 11, which is an agent for the prophylaxis or treatment of breast cancer, ovarian cancer, colorectal cancer, small intestinal cancer, gastric cancer, esophagus cancer, prostate cancer, lung cancer, pancreatic cancer or kidney cancer.

**15.** A method for the prophylaxis or treatment of cancer in a mammal, which comprises administering an effective amount of the compound of claim 1 or a salt thereof, or a prodrug thereof, to the mammal.

**16.** Use of the compound of claim 1 or a salt thereof, or a prodrug thereof, for the production of an agent for the prophylaxis or treatment of cancer.

**INTERNATIONAL SEARCH REPORT**

International application No.
PCT/JP2007/073879

A. CLASSIFICATION OF SUBJECT MATTER
*C07D519/00*(2006.01)i, *A61K31/519*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D519/00, A61K31/519, A61P35/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), CAOLD(STN), REGISTRY(STN), MEDLINE(STN), BIOSIS(STN), EMBASE(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2007/064045 A1 (TAKEDA PHARMACEUTICAL CO., LTD.),<br>07 June, 2007 (07.06.07),<br>Full text; particularly, examples, Claims<br>(Family: none) | 1-14,16 |
| X | WO 2005/118588 A1 (Takeda Chemical Industries, Ltd.),<br>15 December, 2005 (15.12.05),<br>Full text; particularly, page 84, lines 14 to 20; page 89, lines 7 to 13; pages 97 to 98, 193; examples; Claims<br>& AU 2005250285 A & CA 2569016 A<br>& EP 1752457 A1 & CN 1993362 A<br>& US 2007/244132 A1 & NO 2006006015 A | 1-14,16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 January, 2008 (24.01.08) | Date of mailing of the international search report<br>05 February, 2008 (05.02.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/073879

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-508875 A (Pfizer Inc.), 02 September, 1998 (02.09.98), Full text; particularly, Claims; examples & EP 831829 A1 & WO 1996/040142 A1 & CA 2223081 A & AP 637 A & NO 962386 A & NZ 286755 A & PL 314641 A & AU 5479196 A & SG 45483 A & FI 974443 A & BR 9602695 A & CN 1141298 A & HU 9601559 A & IL 118487 A & CZ 9601641 A & HR 960269 A & SI 9600184 A & SK 72996 A & TR 961053 A & ZA 9604725 A & OA 10460 A & MA 26409 A & AR 2746 A | 1-14,16 |
| A | SIZOVA, O.S. et al., Pyrrolo[3,2-d]pyrimidine derivatives. IV. Synthesis, antibacterial and antitumor activity of 2,4,7- substituted pyrrolo[3,2-d]pyrimidines, Khim.-Farm. Zh., 1982, Vol.16/No.11, p.1338-1343 | 1-14,16 |
| A | JP 2002-535391 A (Pfizer Products Inc.), 22 October, 2002 (22.10.02), Full text; particularly, Claims; examples & US 6284764 B1 & EP 1147093 A1 & WO 2000/044728 A1 & NO 20023671 A & BR 9916980 A & BG 105842 A & HR 20010542 A & CA 2358998 A & SK 10182001 A & HU 203425 A & NZ 511707 A & HK 1043795 A & IS 5949 A & CN 1333758 A & CZ 20012638 A & TW 519541 B & ID 29276 A & AU 775163 B & MA 26712 A & OA 11752 A & DZ 2963 A | 1-14,16 |

Form PCT/ISA/210 (continuation of second sheet) (April 2007)

**INTERNATIONAL SEARCH REPORT**

International application No.
PCT/JP2007/073879

**Box No. II Observations where certain claims were found unsearchable (Continuation of item 2 of first sheet)**

This international search report has not been established in respect of certain claims under Article 17(2)(a) for the following reasons:

1. [X] Claims Nos.: 15
because they relate to subject matter not required to be searched by this Authority, namely:
Claim 15 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9713771 A
- WO 9802437 A
- WO 0044728 A
- WO 0202552 A
- WO 0198277 A
- WO 03049740 A
- WO 03050108 A
- WO 03053446 A
- WO 9803648 A
- WO 0177107 A
- WO 03031442 A
- WO 2005118588 A
- WO 9640142 A
- WO 9823613 A
- EP 1348707 A
- US 2006526523 A
- JP 2006335158 A


### Non-patent literature cited in the description


- *Khim.-Farm. Zh.,* 1982, vol. 16, 1338-1343
- *Collect. Czech. Chem. Commun.,* 2003, vol. 68, 779-791
- *Bioorganic & Medicinal Chemistry Letters,* 2003, vol. 13, 2989-2992
- *The Journal of Organic Chemistry,* 1956, vol. 21, 833-836
- *Khim. -Farm.Zh.,* 1982, vol. 16, 1338-1343
- Design of Molecules. IYAKUHIN no KAIHATSU. HIROKAWA SHOTEN, 1990, vol. 7, 163-198
- Genbank. M11730
- Genbank. NP_004439
- Genbank. X00588
- Genbank. NP_005219
- **Skehan et al.** *Journal of the National Cancer Institute,* 1990, vol. 82, 1107-1112

SEQUENCE LISTING

<110> Takeda Pharmaceutical Company Limited

<120> FUSED HETEROCYCLIC COMPOUND

<130> 091154

<150> JP2006-335158
<151> 2006-12-12

<160> 2

<170> PatentIn version 3.3

<210> 1
<211> 66
<212> DNA
<213> Artificial

<220>
<223> primer for cloning human HER2 gene

<400> 1
aattaagtcg acatggacta caaagacgat gacgacaagc gacggcagca gaagatccgg 60
aagtac 66

<210> 2
<211> 42
<212> DNA
<213> Artificial

<220>
<223> primer for cloning human HER2 gene

<400> 2
aattaagcat gctcacactg gcacgtccag acccaggtac tc 42